(19) Europäisches Patentamt
European Patent Office
Office européen des brevets

(11) EP 2 546 262 A1

(12) EUROPEAN PATENT APPLICATION

(43) Date of publication:
16.01.2013 Bulletin 2013/03

(51) Int Cl.:
*C07K 14/415* (2006.01) *C12N 15/82* (2006.01)

(21) Application number: 12185025.9

(22) Date of filing: 02.08.2007

(84) Designated Contracting States:
AT BE BG CH CY CZ DE DK EE ES FI FR GB GR HU IE IS IT LI LT LU LV MC MT NL PL PT RO SE SI SK TR

(30) Priority:
12.10.2006 US 851258 P
01.12.2006 US 868095 P
12.10.2006 US 851250 P
12.10.2006 US 851265 P
17.11.2006 US 859717 P
10.08.2006 US 836804 P
06.10.2006 EP 06121928
27.10.2006 EP 06123066
31.10.2006 EP 06123237
05.10.2006 EP 06121856
02.08.2006 EP 06118347
20.09.2006 EP 06120994

(62) Document number(s) of the earlier application(s) in accordance with Art. 76 EPC:
07788181.1 / 2 054 516

(71) Applicant: CropDesign N.V.
9052 Zwijnaarde (BE)

(72) Inventor: Mironov, Vladimir
7052 Trondheim (NO)

(74) Representative: Hennin, Caroline Marie Odile
BASF SE
Global Intellectual Property
Carl-Bosch-Strasse 38
67056 Ludwigshafen (DE)

Remarks:
This application was filed on 19-09-2012 as a divisional application to the application mentioned under INID code 62.

# (54) Modifying the content of storage compounds in seeds by expression of a Class II HD-Zip transcription factor

(57) The present invention relates generally to the field of molecular biology and concerns a method for enhancing various economically important characteristics in plants. More specifically, the present invention concerns a method for improving yield-related traits, such as enhanced yield and/or enhanced growth, or modified content of storage compounds in plants by modulating expression in a plant of a nucleic acid encoding a Class II Homeodomain-leucine zipper (HD-Zip) polypeptide. The present invention also concerns plants having modulated expression of a nucleic acid encoding a Class II-HD-Zip polypeptide, which plants have improved characteristics relative to control plants.

EP 2 546 262 A1

## Description

The present invention relates generally to the field of molecular biology and concerns a method for improving various plant characteristics by modulating expression in a plant of a nucleic acid encoding a GRP (Growth Related Protein). The present invention also concerns plants having modulated expression of a nucleic acid encoding a GRP polypeptide, which plants have improved characteristics relative to corresponding wild type plants or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increasing yield (seed yield and/or biomass) in plants may be through modification of the inherent growth mechanisms of a plant, such as the cell cycle or various signalling pathways involved in plant growth or in defense mechanisms.

It has now been found that various characteristics may be improved in plants by modulating expression in a plant of a nucleic acid encoding a GRP polypeptide in a plant. The GRP polypeptide may be a Class II homeodomain-leucine zipper (HD Zip) transcription factor. The improved characteristics comprise yield related traits, such as enhanced yield and/or enhanced growth, or modified content of storage compounds.

## Background

HD Zip

The study and genetic manipulation of plants has a long history that began even before the famed studies of Gregor Mendel. In perfecting this science, scientists have accomplished modification of particular traits in plants ranging from potato tubers having increased starch content to oilseed plants such as canola and sunflower having increased or altered fatty acid content. With the increased consumption and use of plant oils, the modification of seed oil content and seed oil levels has become increasingly widespread (e.g. Töpfer et al., 1995, Science 268: 681-686). Manipulation of biosynthetic pathways in transgenic plants provides a number of opportunities for molecular biologists and plant biochemists to affect plant metabolism giving rise to the production of specific higher-value products. The seed oil production or composition has been altered in numerous traditional oilseed plants such as soybean (U.S. Patent No. 5,955,650), canola (U.S. Patent No. 5,955,650), sunflower (U.S. Patent No. 6,084,164), rapeseed (Töpfer et al., 1995, Science 268: 681-686), and non-traditional oil seed plants such as tobacco (Cahoon et al., 1992, Proc. Natl. Acad. Sci. USA 89: 11184-11188).

Plant seed oils comprise both neutral and polar lipids (See Table 1). The neutral lipids contain primarily triacylglycerol, which is the main storage lipid that accumulates in oil bodies in seeds. The polar lipids are mainly found in the various membranes of the seed cells, e.g. the microsomal, plastidial, and mitochondrial membranes, and the cell membrane. The neutral and polar lipids contain several common fatty acids (See Table 2) and a range of less common fatty acids. The fatty acid composition of membrane lipids is highly regulated and only a select number of fatty acids are found in membrane lipids. On the other hand, a large number of unusual fatty acids can be incorporated into the neutral storage lipids in seeds of many plant species (Van de Loo F.J. et al., 1993, Unusual Fatty Acids in Lipid Metabolism in Plants pp. 91-126, editor TS Moore Jr. CRC Press; Millar et al., 2000, Trends Plant Sci. 5:95-101).

**Table 1:** plant lipid classes

| | |
|---|---|
| Neutral lipids | Triacylglycerol (TAG) |
| | Diacylglycerol (DAG) |
| | Monoacylglycerol (MAG) |
| Polar lipids | Monogalactosyldiacylglycerol (MGDG) |
| | Digalactosyldiacylglycerol (DGDG) |
| | Phosphatidylglycerol (PG) |
| | Phosphatidylcholine (PC) |
| | Phosphatidylethanolamine (PE) |
| | Phosphatidylinositol (PI) |
| | Phosphatidylserine (PS) |
| | Sulfoquinovosyldiacylglycerol |

**Table 2:** common plant fatty acids

| | |
|---|---|
| 16:0 | palmitic acid |
| 16:1 | palmitoleic acid |
| 16:3 | hiragonic acid |
| 18:0 | stearic acid |
| 18:1 | oleic acid |
| 18:2 | linoleic acid |
| 18:3 | linolenic acid |
| □-18:3 | gamma-linoleic acid* |
| 20:0 | arachidic acid |

(continued)

| | |
|---|---|
| 20:1 | eicosenoic acid |
| 22:6 | docosahexanoic acid (DHA)* |
| 20:2 | eicosadienoic acid |
| 20:4 | arachidonic acid (AA)* |
| 20:5 | eicosapentaenoic acid (EPA)* |
| 22:1 | erucic acid |

In Table 2, the fatty acids denoted with an asterisk do not normally occur in plant seed oils, but their production in transgenic plant seed oil is of importance in plant biotechnology.

The primary sites of fatty acid biosynthesis in plants are the plastids. Fatty acid biosynthesis begins with the conversion of acetyl-CoA to malonyl-CoA by acetyl-CoA carboxylase (ACCase). The malonyl moiety is then transferred to an acyl carrier protein (ACP) by the malonyl-CoA:ACP transacylase. The enzyme beta-ketoacyl-ACP-synthase III (KAS III) catalyzes the initial condensation reaction of fatty acid biosynthesis, in which after decarboxylation of malonyl-ACP, the resulting carbanion is transferred to acetyl-CoA by a nucleophilic attack of the carbonyl-carbon, resulting in the formation of 3-ketobutyryl-ACP. The reaction cycle is completed by a reduction, a dehydration and again a reduction yielding butyric acid. This reaction cycle is repeated (with KAS I or KAS II catalyzing the condensation reaction) until the acyl-group reach a chain length of usually 16 to 18 carbon atoms. These acyl-ACPs can be desaturated by the stearoyl-ACP desaturase, used as substrates for plastidial acyltransferases in the formation of lipids through what has been referred to as the prokaryotic pathway, or exported to the cytosol after cleavage from ACP through the action of thioesterases. In the cytosol they enter the acyl-CoA pool and can be used for the synthesis of lipids through what has been referred to as the eukaryotic pathway in the endoplasmic reticulum.

Lipid synthesis through both the prokaryotic and eukaryotic pathways occurs through the successive acylation of glycerol-3-phosphate, catalyzed by glycerol-3-phosphate acyltransferases (GPAT) and lysophosphatidic acid acyltransferase (LPAAT) (Browse et al., 1986, Biochemical J. 235:25-31; Ohlrogge & Browse, 1995,5 Plant Cell 7:957-970). The resulting phosphatidic acid (PA) is the precursor for other polar membrane lipids such as monogalactosyldiacylglycerol (MGD), digalactosyldiacylglycerol (DGD), phosphatidylglycerol (PG) and sulfoquinovosyldiacylglycerol (SQD) in the plastid and phosphatidylcholine (PC), phosphatidylethanolamine (PE), phosphatidylinositol (PI) and phosphatidylserine (PS) in the endoplasmic reticulum. The polar lipids are also the sites of further modification of the acyl-chain such as desaturation, acetylenation, and hydroxylation. In the endoplasmic reticulum, PA is also the intermediate in the biosynthesis of triacylglycerol (TAG), the major component of neutral lipids and hence of seed oil. Furthermore, alternative pathways for the biosynthesis of TAGS can exist (i.e. transacylation through the action of phosphatidylcholine:diacylglycerol acyltransferase) (Voelker, 1996, Genetic Engineering ed.: Setlow 18: 111-113; Shanklin & Cahoon, 1998, Annu. Rev. Plant Physiol. Plant Mol. Biol. 49:611-641; Frentzen, 1998, Lipids 100:161-166; Millar et al., 2000, Trends Plant Sci. 5:95-101). The reverse reaction, the breakdown of triacylglycerol to diacylglycerol and fatty acids is catalyzed by lipases. Such a breakdown can be seen towards the end of seed development resulting in a certain reduction in seed oil. (Buchanan et al., 2000).

Storage lipids in seeds are synthesized from carbohydrate-derived precursors. Plants have a complete glycolytic pathway in the cytosol (Plaxton, 1996, Annu. Rev. Plant Physiol. Plant Mol. Biol. 47: 185-214), and it has been shown that a complete pathway also exists in the plastids of rapeseed (Kang & Rawsthorne, 1994, Plant J. 6:795-805). Sucrose is the primary source of carbon and energy, transported from the leaves into the developing seeds. During the storage phase of seeds, sucrose is converted in the cytosol to provide the metabolic precursors glucose-6-phosphate and pyruvate. These are transported into the plastids and converted into acetyl-CoA that serves as the primary precursor for the synthesis of fatty acids. Acetyl-CoA in the plastids is the central precursor for lipid biosynthesis. Acetyl-CoA can be formed in the plastids by different reactions, and the exact contribution of each reaction is still being debated (Ohlrogge & Browse, 1995, Plant Cell 7: 957-970). It is accepted, however, that a large part of the acetyl-CoA is derived from glucose-6-phospate and pyruvate that are imported from the cytoplasm into the plastids. Sucrose is produced in the source organs (leaves, or anywhere that photosynthesis occurs) and is transported to the developing seeds, also termed sink organs. In the developing seeds, sucrose is the precursor for all the storage compounds, i.e. starch, lipids, and partly the seed storage proteins. Therefore, it is clear that carbohydrate metabolism in which sucrose plays a central role is very important to the accumulation of seed storage compounds.

Although lipid and fatty acid content of seed oil can be modified by the traditional methods of plant breeding, the advent of recombinant DNA technology has allowed for easier manipulation of the seed oil content of a plant, and in some cases, has allowed for the alteration of seed oils in ways that could not be accomplished by breeding alone (See, e.g., Töpfer et al., 1995, Science 268:681-686). For example, introduction of a $\Delta^{12}$-hydroxylase nucleic acid sequence into transgenic tobacco resulted in the formation of a novel fatty acid, ricinoleic acid, into the tobacco seed oil (Van de Loo et al., 1995, Proc. Natl. Acad. Sci USA 92: 6743-6747). Tobacco plants have also been engineered to produce low levels of petroselinic acid by the introduction and expression of an acyl-ACP desaturase from coriander (Cahoon et al., 1992, Proc. Natl. Acad. Sci USA 89: 11184-11188).

The modification of seed oil content in plants has significant medical, nutritional, and economic ramifications. With regard to the medical ramifications, the long chain fatty acids (C18 and longer) found in many seed oils have been linked to reductions in hypercholesterolemia and other clinical disorders related to coronary heart disease (Brenner, 1976, Adv. Exp. Med. Biol. 83: 85-101). Therefore, consumption of a plant having increased levels of these types of fatty acids may reduce the risk of heart disease. Enhanced levels of seed oil content are also useful in increasing the large-scale production of seed oils and thereby reducing the cost of these oils.

As mentioned earlier, several desaturase nucleic acids such as the $\Delta^{6}$-desaturase nucleic acid, $\Delta^{12}$-desaturase nucleic acid and acyl-ACP desaturase nucleic acid have been cloned and demonstrated to encode enzymes required for fatty acid synthesis in various plant species. Oleosin nucleic acid sequences from species such as Brassica, soybean, carrot, pine, and *Arabidopsis thaliana* have also been cloned and determined to encode proteins associated with the phospholipid monolayer membrane of oil bodies in those plants.

It has now been found that nucleic acid sequences encoding Class II homeodomain-leucine zipper (HD-Zip) transcription factors are useful in modifying the content of storage compounds in seeds.

Transcription factors are usually defined as proteins that show sequence-specific DNA binding and that are capable of activating and/or repressing transcription. The *Arabidopsis* genome codes for at least 1533 transcriptional regulators, which account for ~5.9% of its estimated total number of genes. About 45% of these transcription factors are reported to be from families specific to plants (Riechmann et al., 2000 (Science Vol. 290, 2105-2109)). One example of such a plant-specific family of transcription factors is the family of HD-Zip transcription factors.

Homeobox genes are transcription factors present in all eukaryotes and constitute a gene family of at least 89 members in *Arabidopsis thaliana.* They are characterized by the presence of a homeodomain, which usually consists of 60 conserved amino acid residues that form a helix-loop-helix-turn-helix structure that binds DNA. This DNA binding site is usually pseudopalindromic. Homeobox genes play crucial and diverse roles in many aspects of development, including the early development of animal embryos, the specification of cell types in yeast, and the initiation and maintenance of the shoot apical meristem in flowering plants (Sakakibara et al. Mol. Biol. Evol. 18(4): 491-502, 2001).

Numerous angiosperm homeobox genes have been isolated and sorted into seven distinct groups based on their amino acid similarities. These include the KNOX, BELL, HD-PHD-finger, HAT1, HAT 2, GL2, and ATHB8 groups. Genes in the latter four groups also encode a leucine zipper motif adjacent to the C-terminus of the homeodomain and collectively form the homeodomain-leucine zipper (HD-Zip) gene family. Aso et al., Mol. Biol. Evol. 16(4): 544-552, 1999. Of the at least 89 members comprising the homeobox gene family in *Arabidopsis thaliana,* at least 47 comprise both a homeodomain and a leucine zipper. Although the combination of a homeodomain and a leucine zipper motif is unique to plants, it has also been encountered in moss in addition to vascular plants (Sakakibara et al. (2001) Mol Biol Evol 18 (4): 491-502).

Homeodomain leucine zipper (HD-Zip) proteins constitute a family of transcription factors characterized by the presence of a DNA-binding domain (HD) and an adjacent leucine zipper (Zip) motif. The leucine zipper, adjacent to the C-terminal end of the homeodomain, consists of several heptad repeats (at least four) in which a leucine (occasionally a valine or an isoleucine) typically appears every seventh amino acid. The leucine zipper is important for protein dimerisation. This dimerisation is a prerequisite for DNA binding (Sessa et al. (1993) EMBO J 12(9): 3507-3517), and may proceed between two identical HD-Zip proteins (homodimer) or between two different HD-Zip proteins (heterodimer).

The group of angiosperm homeobox genes HAT1, HAT2, GL2, and ATHB8 groups have been renamed the HD-Zip I-IV subfamilies. The combination of a homeodomain and a leucine zipper motif is unique to higher plants, suggesting that the HD-Zip genes may be involved in the regulation of developmental processes specific to plants. Aso et al., Mol. Biol. Evol. 16(4): 544-552, 1999. The functions of the HD-Zip genes are diverse among the different subfamilies. HD-Zip I and II genes are likely involved in signal transduction networks of light, dehydration-induced ABA, or auxin. These signal transduction networks are related to the general growth regulation of plants. The overexpression of sense or antisense HD-Zip I or II mRNA usually alters growth rate and development. Most members of the HD-Zip III subfamily play roles in cell differentiation in the stele. HD-Zip IV genes are related to the differentiation of the outermost cell layer. Sakakibara et al. Mol. Biol. Evol. 18(4): 491-502, 2001.

Different HD-Zip proteins have been shown to either activate or repress transcription. In *Arabidopsis,* the class I HD-Zip ATHB1, -5, -6, and -16 were shown to act as transcriptional activators in transient expression assays on *Arabidopsis* leaves using a reporter gene, luciferase (Henriksson et al. (2005) Plant Phys 139: 509-518). Two rice class I HDZip proteins, Oshox4 and Oshox5, acted as activators in transient expression assays on rice cell suspension cultures using another reporter gene (glucuronidase; Meijer et al. (2000) Mol Gen Genet 263:12-21). In contrast, two rice class II HD-Zip proteins, Oshox1 and Oshox3, acted as transcriptional repressors in the same experiments (Meijer et al. (1997) Plant J 11: 263-276; Meijer *et al.* (2000) supra).

The Class II HD-Zip gene from *Arabidopsis thaliana,* HAT4, also known as ATHB-2, has been reported to act as a regulator of shade-avoidance responses (Morelli and Ruberti TIPS Vol. 7 No. 9, September 2002).

Summary

Surprisingly, it has now been found that modulating expression of a nucleic acid encoding a GRP polypeptide gives plants having improved characteristics relative to control plants.

In an aspect of the present invention, it has now been found that nucleic acids encoding Class II HD-Zip transcription factors are useful in modifying the content of storage compounds in seeds. The present invention therefore provides a method for modifying the content of storage compounds in seeds relative to control plants by modulating expression in a plant of a nucleic acid encoding a Class II HD-Zip transcription factor. The present invention also provides nucleic acid sequences and constructs useful in performing such methods. The invention further provides seeds having a modified content of storage compounds relative to control plants, which seeds have modulated expression of a nucleic acid encoding a Class II HD-Zip transcription factor. The present invention provides a method for modifying the content of storage compounds in seeds relative to control plants, comprising modulating expression in a plant of a nucleic acid encoding a Class II HD-Zip transcription factor.

**Definitions**

Polypeptide(s)/Protein(s)

The terms "polypeptide" and "protein" are used interchangeably herein and refer to amino acids in a polymeric form of any length, linked together by peptide bonds.

Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break $\alpha$-helical structures or $\beta$-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 3 below).

**Table 3:** Examples of conserved amino acid substitutions

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

Motif/Consensus sequence/Signature

The term "motif' or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1 °C per % base mismatch. The Tm may be calculated using the following equations, depending on the types of hybrids:

1) DNA-DNA hybrids (Meinkoth and Wahl, Anal. Biochem., 138: 267-284, 1984):

$$T_m = 81.5°C + 16.6 x \log_{10}[Na^+]^a + 0.41 x \%[G/C^b] - 500 x [L^c]^{-1} - 0.61 x \% \text{ formamide}$$

2) DNA-RNA or RNA-RNA hybrids:

$$Tm = 79.8 + 18.5 (\log_{10}[Na^+]^a) + 0.58 (\%G/C^b) + 11.8 (\%G/C^b)^2 - 820/L^c$$

3) oligo-DNA or oligo-RNA[d] hybrids:

For <20 nucleotides: $T_m = 2 (l_n)$
For 20-35 nucleotides: $T_m = 22 + 1.46 (l_n)$

a or for other monovalent cation, but only accurate in the 0.01-0.4 M range.
b only accurate for %GC in the 30% to 75% range.
c L = length of duplex in base pairs.

d oligo, oligonucleotide; $l_n$, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell.

Operably linked

The term "operably linked" as used herein refers to a functional linkage between the promoter sequence and the gene of interest, such that the promoter sequence is able to initiate transcription of the gene of interest.

Constitutive promoter

A "constitutive promoter" refers to a promoter that is transcriptionally active during most, but not necessarily all, phases of growth and development and under most environmental conditions, in at least one cell, tissue or organ. Table 4a below gives examples of constitutive promoters.

**Table 4a:** Examples of constitutive promoters

| Gene Source | Reference |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |

(continued)

| Gene Source | Reference |
|---|---|
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters are shown in Tables 4b to 4e below. Further examples of seed-specific promoters are given in Qing Qu and Takaiwa (Plant Biotechnol. J. 2, 113-125, 2004), which disclosure is incorporated by reference herein as if fully set forth.

**Table 4b:** Examples of seed-specific promoters

| Gene source | Reference |
|---|---|
| seed-specific genes | Simon et al., Plant Mol. Biol. 5: 191, 1985; |
| | Scofield et al., J. Biol. Chem. 262: 12202, 1987.; |

(continued)

| Gene source | Reference |
|---|---|
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat $\alpha$, $\beta$, Y-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice $\alpha$-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice $\alpha$-globulin REB/OHP-1 | Nakase et al. Plant Mol. Biol. 33: 513-522, 1997 |
| rice ADP-glucose pyrophosphorylase | Trans Res 6:157-68, 1997 |
| maize ESR gene family | Plant J 12:235-46, 1997 |
| sorghum $\alpha$-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136, rice alanine aminotransferase | unpublished |
| PRO0147, trypsin inhibitor ITR1 (barley) | unpublished |
| PRO0151, rice WSI18 | WO 2004/070039 |
| PRO0175, rice RAB21 | WO 2004/070039 |
| PRO005 | WO 2004/070039 |
| PRO0095 | WO 2004/070039 |
| $\alpha$-amylase (Amy32b) | Lanahan et al, Plant Cell 4:203-211, 1992; Skriver et al, Proc Natl Acad Sci USA 88:7266-7270, 1991 |
| cathepsin $\beta$-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |

(continued)

| Gene source | Reference |
|---|---|
| Maize B-Peru | Selinger et al., Genetics 149;1125-38,1998 |

**Table 4c:** examples of endosperm-specific promoters

| Gene source | Reference |
|---|---|
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley Itr1 promoter | Diaz et al. (1995) Mol Gen Genet 248(5):592-8 |
| barley B1, C, D, hordein | Cho et al. (1999) Theor Appl Genet 98:1253-62;<br>Muller et al. (1993) Plant J 4:343-55;<br>Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

**Table 4d:** Examples of embryo specific promoters:

| Gene source | Reference |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |
| PRO0095 | WO 2004/070039 |

**Table 4e:** Examples of aleurone-specific promoters:

| Gene source | Reference |
|---|---|
| α-amylase (Amy32b) | Lanahan et al, Plant Cell 4:203-211, 1992;<br>Skriver et al, Proc Natl Acad Sci USA 88:7266-7270, 1991 |

(continued)

| Gene source | Reference |
|---|---|
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts.

Another example of a tissue-specific promoter is a meristem-specific promoter, which is transcriptionally active predominantly in meristematic tissue, substantially to the exclusion of any other parts of a plant, whilst still allowing for any leaky expression in these other plant parts.

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., W09322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Decreased expression

Reference herein to "decreased epression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an $\alpha$-anomeric nucleic acid sequence. An $\alpha$-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation (s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via Agrobacterium-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for Agrobacterium-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis* (*Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

T-DNA activation tagging

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8).

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters. The term "yield" of a plant may relate to vegetative biomass (root and/or shoot biomass), to reproductive organs, and/or to propagules (such as seeds) of that plant.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following: a) an increase in seed biomass (total seed weight) which may be on an individual seed basis and/or per plant and/or per square meter; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Triticosecale rimpaui, Triticum* spp. *(e.g. Triticum aestivum, Triticum durum, Triticum turgidum, Triticum hybernum, Triticum macha, Triticum sativum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

**Detailed description of the invention**

Detailed description for the Class II HD-Zip transcription factors

It has now been found that nucleic acids encoding Class II HD-Zip transcription factors are useful in modifying the content of storage compounds in seeds. The present invention therefore provides a method for modifying the content of storage compounds in seeds relative to control plants by modulating expression in a plant of a nucleic acid encoding a Class II HD-Zip transcription factor. The present invention also provides nucleic acid sequences and constructs useful in performing such methods. The invention further provides seeds having a modified content of storage compounds relative to control plants, which seeds have modulated expression of a nucleic acid encoding a Class II HD-Zip transcription factor.

The present invention provides a method for modifying the content of storage compounds in seeds relative to control plants, comprising modulating expression in a plant of a nucleic acid encoding a Class II HD-Zip transcription factor.

A preferred method for modulating (preferably, increasing) expression of a nucleic acid encoding a Class II HD-Zip transcription factor is by introducing and expressing in a plant a nucleic acid encoding a Class II HD-Zip transcription factor as will now be defined.

A "Class II HD-Zip transcription factor" is taken to mean a polypeptide comprising the following: (i) a homeodomain box; (ii) a leucine zipper; and (iii) Motifs I, II and III given below (in any order).

**Motif I (SEQ ID NO: 279)**

RKKLRL, or Motif I with one or more conservative amino acid substitution at any position, and/or Motif I with one or two non-conservative change(s) at any position; and

**Motif II (SEQ ID NO: 280)**

TKLKQTEVDCEFLRRCCENLTEEN, or Motif II with one or more conservative amino acid substitution at any position, and/or a motif having in increasing order of preference at least 50%, 55%, 60%, 65%, 70%, 75%, 80%, 85%, 90%, 95% or more sequence identity to Motif II; and

**Motif III (SEQ ID NO: 281)**

TLTMCPSCER, or Motif III with one or more conservative amino acid substitution at any position, and/or Motif III with one, two or three non-conservative change(s) at any position.

Any reference herein to a "nucleic acid encoding a Class II HD-Zip transcription factor" or to a Class II HD-Zip-encoding nucleic acid" is taken to mean a nucleic acid encoding a Class II HD-Zip transcription factor as defined hereinabove, such nucleic acids being useful in performing the methods of the invention.

As mentioned, a preferred method for modulating (preferably, increasing) expression of a nucleic acid encoding a Class II HD-Zip transcription factor is by introducing and expressing in a plant a nucleic acid encoding a Class II HD-Zip transcription factor.

The present invention is illustrated by transforming plants with the nucleic acid sequence represented by SEQ ID NO: 229, encoding the polypeptide sequence of SEQ ID NO: 230. However, performance of the invention is not restricted to these sequences; the methods of the invention may advantageously be performed using any Class II HD-Zip transcription factor-encoding nucleic acid or Class II HD-Zip transcription factor as defined herein.

For example, nucleic acids encoding orthologues or paralogues of an amino acid sequence represented by SEQ ID NO: 230 may be useful in performing the methods of the invention. Examples of such orthologues and paralogues are provided in Table A of Example 1.

Orthologues and paralogues encompass evolutionary concepts used to describe the ancestral relationships of genes. Paralogues are genes within the same species that have originated through duplication of an ancestral gene and orthologues are genes from different organisms that have originated through speciation.

Orthologues and paralogues may easily be found by performing a so-called reciprocal blast search. Typically this involves a first BLAST involving BLASTing a query sequence (for example, SEQ ID NO: 229 or SEQ ID NO: 230) against any sequence database, such as the publicly available NCBI database which may be found at: http://www.ncbi.nlm.nih.gov. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 229 or SEQ ID NO: 230, the second BLAST would therefore be against *Oryza* sequences). The results of the first and second BLASTs are then compared. A paralogue is identified if a high-ranking hit from the first blast is from the same species as from which the query sequence is derived, a BLAST back then ideally results in the query sequence being among the highest hits; an orthologue is identified if a high-ranking hit in the first BLAST is not from the same species as from which the query sequence is derived, and preferably results upon BLAST back in the query sequence being among the highest hits. High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Nucleic acids encoding homologues of an amino acid sequence represented by SEQ ID NO: 230, or nucleic acids encoding homologues of any of the amino acid sequences given in Table A, may also be useful in performing the methods of the invention.

Also useful in the methods of the invention are nucleic acids encoding derivatives of any one of the amino acids given in Table A or derivatives of orthologues or paralogues of any of the amino acid sequences given in Table A. "Derivatives" include peptides, oligopeptides, polypeptides which may, compared to the amino acid sequence of the naturally-occurring form of the protein, such as the one presented in SEQ ID NO: 230, comprise substitutions of amino acids with non-naturally occurring amino acid residues, or additions of non-naturally occurring amino acid residues.

Homologues (or homologous proteins, encompassing orthologues and paralogues) may readily be identified using routine techniques well known in the art, such as by sequence alignment. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the alignment of two complete sequences that maximizes the number of matches and minimizes the number of gaps. The BLAST algorithm (Altschul et al. (1990) J Mol Biol 215: 403-410) calculates percent sequence identity and performs a statistical analysis of the similarity between the two sequences. The software for performing BLAST analysis is publicly available through the National Centre for Biotechnology Information. Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 4, 29, 2003). Minor manual editing may be performed to optimise alignment between conserved motifs, as would be apparent to a person skilled in the art. Furthermore, instead of using full-length sequences for the identification of homologues, specific domains may be used as well.

The sequence identity values, which are indicated below as a percentage were determined over the entire nucleic acid or amino acid sequence using ClustalW and default parameters.

Preferably, the polypeptides encoded by the nucleic acids useful in the methods of the present invention (such as any of the polypeptide sequences given in Table A) have, in increasing order of preference, at least 40%, 41 %, 42%, 43%, 44%, 45%, 46%, 47%, 48%, 49%, 50%, 51 %, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identity to the polypeptide of SEQ ID NO: 230.

According to the present invention, there is provided a method for modifying the content of storage compounds in seeds relative to control plants, comprising modulating expression in a plant of a nucleic acid encoding a Class II HD-Zip transcription factor represented by SEQ ID NO: 2 or an orthologue, paralogue or homologue thereof.

A preferred method for modulating (preferably, increasing) expression of a nucleic acid encoding a Class II HD-Zip transcription factor is by introducing and expressing in a plant a nucleic acid encoding a Class II HD-Zip transcription factor represented by SEQ ID NO: 230 or an orthologue, paralogue or homologue thereof.

The orthologues, paralogues and homologues described above fall under the definition of a Class II HD-Zip transcription factor, i.e. meaning that the orthologues, paralogues and homologues are polypeptides comprising the following: (i) a homeodomain box; (ii) a leucine zipper; and (iii) Motifs I, II and III described herein.

The various domains and motifs may be used to help identify sequences useful in the methods of the invention. Specialist databases also exist for the identification of domains, for example, SMART (Schultz et al. (1998) Proc. Natl. Acad. Sci. USA 95, 5857-5864; Letunic et al. (2002) Nucleic Acids Res 30, 242-244, InterPro (Mulder et al., (2003) Nucl. Acids. Res. 31, 315-318, Prosite (Bucher and Bairoch (1994), A generalized profile syntax for biomolecular sequences motifs and its function in automatic sequence interpretation. (in) ISMB-94; Proceedings 2nd International Conference on Intelligent Systems for Molecular Biology. Altman R., Brutlag D., Karp P., Lathrop R., Searls D., Eds., pp53-61, AAAIPress, Menlo Park; Hulo et al., Nucl. Acids. Res. 32:D134-D137, (2004)), or Pfam (Bateman et al., NucleicAcids Research 30(1): 276-280 (2002)). Domains and motifs may also be identified using routine techniques, such as by sequence alignment as described herein.

Nucleic acids useful in the methods of the invention need not be full-length, since performance of the methods of the invention does not rely on the use of full length nucleic acids. Examples include portions of the nucleic acid sequence represented by SEQ ID NO: 229 or portions of any one of the nucleic acid sequences given in Table A.

According to the present invention, there is provided a method for modifying the content of storage compounds in seeds relative to control plants, comprising modulating expression in a plant of a portion of a nucleic acid sequence represented by SEQ ID NO: 229, or comprising modulating expression in a plant of a portion of any one of the nucleic acid sequences given in Table A, or comprising modulating expression in a plant of a portion of a nucleic acid sequence encoding an orthologue, paralogue or homologue of any of the amino acid sequences given in Table A.

A preferred method for modulating (preferably, increasing) expression in a plant of such a portion is by introducing and expressing in a plant a portion of a nucleic acid sequence given in Table A, or comprising introducing and expressing in a plant a portion of a nucleic acid sequence encoding an orthologue, paralogue or homologue of any of the polypeptide sequences given in Table A.

Portions useful in the methods of the invention, include portions of sufficient length to encode a polypeptide falling under the definition of a Class II HD-Zip transcription factor, i.e. meaning that the polypeptide comprises the following: (i) a homeodomain box; (ii) a leucine zipper; and (iii) Motifs I, II and III described herein. Furthermore, such portions have substantially the same biological activity as Class II HD-Zip transcription factors.

Preferably, the portion is at least 500 consecutive nucleotides in length, preferably at least 750 consecutive nucleotides in length, more preferably at least 1,250 consecutive nucleotides in length, the consecutive nucleotides being of SEQ ID NO: 229, or of any one of the nucleic acid sequences given in Table A, or of any nucleic acid encoding an orthologue, paralogue or homologue of any one of the amino acid sequences given in Table A. Most preferably the portion is a portion of the nucleic acid of SEQ ID NO: 229.

A portion as defined herein may be prepared, for example, by making one or more deletions to the nucleic acid in question. The portions may be used in isolated form or they may be fused to other coding (or non coding) sequences in order to, for example, produce a protein that combines several activities. When fused to other coding sequences, the resultant polypeptide produced upon translation may be bigger than that predicted for the portion.

Another nucleic acid useful in the methods of the invention is a nucleic acid capable of hybridising, under reduced stringency conditions, preferably under medium stringency conditions, more preferably under stringent conditions, with a nucleic acid represented by SEQ ID NO: 229, or capable of hybridising, under reduced stringency conditions, preferably under medium stringency conditions, more preferably under stringent conditions, with a nucleic acid sequence given in Table A, or capable of hybridising under reduced stringency conditions, preferably under medium stringency conditions, more preferably under stringent conditions with a nucleic acid encoding an orthologue, paralogue or homologue of a polypeptide sequence given in Table A.

According to the present invention, there is provided a method for modifying the content of storage compounds in seeds relative to control plants, comprising modulating expression in a plant of a nucleic acid capable of hybridising to a nucleic acid represented by SEQ ID NO: 229, or comprising modulating expression in a plant of a nucleic acid capable of hybridising to a nucleic acid sequence given in Table A, or comprising modulating expression in a plant of a nucleic acid capable of hybridising to a nucleic acid sequence encoding an orthologue, paralogue or homologue of any of the polypeptide sequences given in Table A. Most preferably the hybridising sequence is capable of hybridising to a nucleic acid as represented by SEQ ID NO: 229. The hybridising sequence is preferably capable of hybridising under reduced stringency conditions, preferably under medium stringency conditions, more preferably under stringent conditions.

A preferred method for modulating (preferably, increasing) expression in a plant of such a hybridising sequence is by introducing and expressing in a plant a nucleic acid capable of hybridising to a nucleic acid sequence given in Table A, or comprising introducing and expressing in a plant of a nucleic acid sequence capable of hybridising to a nucleic acid sequence encoding an orthologue, paralogue or homologue of any one of the amino acid sequences given in Table A. Most preferably the hybridising sequence is capable of hybridising to a nucleic acid as represented by SEQ ID NO: 229. The hybridising sequence is preferably capable of hybridising under reduced stringency conditions, preferably under medium stringency conditions, more preferably under stringent conditions.

Hybridising sequences useful in the methods of the invention, include nucleic acids of sufficient length to encode a polypeptide falling under the definition of a Class II HD-Zip transcription factor, i.e. meaning that the polypeptide comprises the following: (i) a homeodomain box; (ii) a leucine zipper; and (iii) Motifs I, II and III described herein. Furthermore, such hybridising sequences have substantially the same biological activity as the Class II HD-Zip transcription factors.

The hybridising sequence is typically at least 500 consecutive nucleotides in length, preferably at least 750 consecutive nucleotides in length, more preferably at least 1,250 consecutive nucleotides in length, the consecutive nucleotides being of any nucleic acid capable of hybridising to a nucleic acid sequence given in Table A, or of any nucleic acid encoding an orthologue, paralogue or homologue of any one of the amino acid sequences given in Table A. Most preferably the consecutive nucleotides are of a nucleic acid capable of hybridising to a nucleic acid represented by SEQ ID NO: 229 or to a portion thereof.

Another nucleic acid useful in the methods of the invention is a splice variant of SEQ ID NO: 229, or a splice variant of any of the nucleic acid sequences given in Table A, or a splice variant of a nucleic acid encoding an orthologue, paralogue or homologue of any one of the amino acid sequences given in Table A.

According to the present invention, there is provided a method for modifying the content of storage compounds in seeds relative to control plants, comprising modulating expression in a plant of a splice variant of a nucleic acid represented by SEQ ID NO: 229, or comprising modulating expression in a plant of a splice variant of nucleic acid sequence given in Table A, or comprising modulating expression in a plant of a splice variant of a nucleic acid sequence encoding an orthologue, paralogue or homologue of any one of the amino acid sequences given in Table A.

A preferred method for modulating (preferably, increasing) expression in a plant of such a splice variant is by introducing and expressing in a plant a splice variant of a nucleic acid sequence given in Table A, or comprising introducing and expressing in a plant a splice variant of a nucleic acid sequence encoding an orthologue, paralogue or homologue of any one of the amino acid sequences given in Table A.

Splice variants useful in the methods of the invention, include nucleic acids of sufficient length to encode a polypeptide falling under the definition of a Class II HD-Zip transcription factor, i.e. meaning that the polypeptide comprises the following: (i) a homeodomain box; (ii) a leucine zipper; and (iii) Motifs I, II and III described herein. Furthermore, such splice variants have substantially the same biological activity as the Class II HD-Zip transcription factors.

Another nucleic acid useful in performing the methods of the invention is an allelic variant of SEQ ID NO: 229, or an allelic variant of any of the nucleic acid sequences given in Table A, or an allelic variant of a nucleic acid encoding an orthologue, paralogue or homologue of any one of the amino acid sequences given in Table A. Allelic variants exist in nature, and encompassed within the methods of the present invention is the use of these natural alleles. According to the present invention, there is provided a method for modifying the content of storage compounds in seeds relative to control plants, comprising modulating expression in a plant of an allelic variant of a nucleic acid represented by SEQ ID NO: 229, or comprising modulating expression in a plant of an allelic variant of nucleic acid sequence given in Table A, or comprising modulating expression in a plant of an allelic variant of a nucleic acid sequence encoding an orthologue, paralogue or homologue of any one of the amino acid sequences given in Table A.

A preferred method for modulating (preferably, increasing) expression in a plant of such an allelic variant is by introducing and expressing in a plant an allelic variant of a nucleic acid sequence given in Table A, or comprising introducing and expressing in a plant an allelic variant of a nucleic acid sequence encoding an orthologue, paralogue or homologue of any one of the amino acid sequences given in Table A.

Allelic variants useful in the methods of the invention, include nucleic acids of sufficient length to encode a polypeptide falling under the definition of a Class II HD-Zip transcription factor, i.e. meaning that the polypeptide comprises the following: (i) a homeodomain box; (ii) a leucine zipper; and (iii) Motifs I, II and III described herein. Furthermore, such allelic variants have substantially the same biological activity as the Class II HD-Zip transcription factors.

A further nucleic acid useful in the methods of the invention is a nucleic acid obtained by gene shuffling. Gene shuffling or directed evolution may also be used to generate variants of any one of the nucleic acids given in Table A, or variants of nucleic acids encoding orthologues, paralogues or homologues of any one of the amino acid sequences given in Table A.

According to the present invention, there is provided a method for modifying the content of storage compounds in seeds relative to control plants, comprising modulating expression in a plant of a variant of a nucleic acid represented by SEQ ID NO: 229, or comprising modulating expression in a plant of a variant of any one of the nucleic acid sequences given in Table A, or comprising modulating expression in a plant a variant of a nucleic acid encoding an orthologue, paralogue or homologue of any of the amino acid sequences given in Table A, which variant nucleic acid is obtained by gene shuffling.

A preferred method for modulating (preferably, increasing) expression in a plant of such a variant obtained by gene shuffling is by introducing and expressing in a plant a variant of any one of the nucleic acid sequences given in Table A, or comprising introducing and expressing in a plant a variant of a nucleic acid sequence encoding an orthologue, paralogue or homologue of any of the amino acid sequences given in Table A, which variant nucleic acid is obtained by gene shuffling.

Such variants obtained by gene shuffling useful in the methods of the invention, include nucleic acids of sufficient length to encode a polypeptide falling under the definition of a Class II HD-Zip transcription factor, i.e. meaning that the polypeptide comprising the following: (i) a homeodomain box; (ii) a leucine zipper; and (iii) Motifs I, II and III described herein.

Furthermore, nucleic acids useful in the methods of the invention may also be obtained by site-directed mutagenesis. Several methods are available to achieve site-directed mutagenesis, the most common being PCR-based methods (Current Protocols in Molecular Biology. Wiley Eds.).

Class II HD-Zip transcription factors exhibit the general biological activity of transcription factors (at least in their native form) and typically have DNA-binding activity and an activation domain. A person skilled in the art may easily determine the presence of an activation domain and DNA-binding activity using routine tools and techniques. Sessa et al., 1997 (J Mol Biol 274(3): 303-309) studied the DNA-binding properties of the ATHB-1 and ATHB-2 (= HAT4) HD-Zip (HD-Zip-1 and -2) domains and found that they interact with DNA as homodimers and recognize two distinct 9 bp pseudopalindromic sequences, CAAT(A/T)ATTG (BS-1) and CAAT(G/C)ATTG (BS-2), respectively. From a mutational analysis of the HD-Zip-2 domain, they determined that conserved amino acid residues of helix 3, Val47 and Asn51, and Arg55 are essential for the DNA-binding activity of the HD-Zip-2 domain. They also report that the preferential recognition of a G/C base-pair at the central position by the HD-Zip-2 domain is abolished either by the replacement of Arg55 with lysine or by the substitution of Glu46 and Thr56 with the corresponding residues of the HD-Zip-1 domain (alanine and tryptophan, respectively).

According to a preferred feature of the invention, the modulated expression is increased expression. Methods for increasing expression of nucleic acids or genes, or gene products, are well documented in the art.

Another method for modulating expression in a plant of a nucleic acid encoding a Class 11 HD-Zip transcription factor comprises the reduction or substantial elimination of expression in a plant of an endogenous gene encoding a Class II HD-Zip transcription factor. Reference herein to "reduction or substantial elimination" is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants. Methods for decreasing expression are described above in the "definitions" sections.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from SEQ ID NO: 229, or from any of the nucleic acid sequences given in Table A, or from any nucleic acid capable of encoding an orthologue, paralogue or homologue of any one of the amino acid sequences given in Table A. A nucleic acid sequence encoding a (functional) polypeptide is not a requirement for the various methods discussed herein for the reduction or substantial elimination of expression of an endogenous gene.

Nucleic acids suitable for use in the methods of the invention may be derived from any natural or artificial source. The nucleic acid may be modified from its native form in composition and/or genomic environment through deliberate human manipulation. Preferably the nucleic acid is from a plant, further preferably from a dicotyledonous plant, further preferably from the family Brassicaceae, more preferably from *Arabidopsis thaliana.*

A preferred method for modulating (preferably, increasing) expression of a nucleic acid encoding a Class II HD-Zip transcription factor is by introducing and expressing in a plant a nucleic acid encoding a Class II HD-Zip transcription factor; however the effects of performing the method, i.e. the modified content of seed storage compounds, may also be achieved using other well known techniques. One such technique is T-DNA activation tagging. The effects of the invention may also be reproduced using the technique of TILLING. The effects of the invention may also be reproduced using homologous recombination, which allows introduction in a genome of a selected nucleic acid at a defined selected position.

Performance of the methods of the invention gives plants having seeds with a modified content of seed storage compounds relative to the seeds of control plants. The modified content of seed storage compounds refers to a modified content of one or more of lipids, oil, fatty acids, starch, sugar and proteins relative to that of control plants. Preferably, modulation of expression in a plant of a nucleic acid encoding a Class II HD-Zip transcription factor gives plants with seeds having increased oil content relative to the seeds of control plants. In such a case, the modulated expression is typically overexpression in a plant of a nucleic acid encoding a Class II HD-Zip transcription factor. Preferably, modulation of expression in a plant of a nucleic acid encoding a Class II HD-Zip transcription factor gives plants with seeds having increased protein content relative to the seeds of control plants. In such a case, the modulated expression is typically the reduction or substantial elimination of expression of an endogenous Class II HD-Zip transcription factor-encoding gene.

The present invention also encompasses plants or parts thereof (particularly seeds) obtainable by the methods according to the present invention. The plants or parts thereof comprise a nucleic acid transgene (comprising any one of the nucleic acid sequences described above as being useful in the methods of the invention).

The invention also provides genetic constructs and vectors to facilitate introduction and/or expression in a plant of the nucleic acid sequences described above as being useful in the methods of the invention.

More particularly, there is provided a gene construct comprising:

(i) A nucleic acid encoding a Class II HD-Zip transcription factor as defined hereinabove;
(ii) One or more control sequences operably linked to the nucleic acid of (i).

Constructs useful in the methods according to the present invention may be constructed using recombinant DNA technology well known to persons skilled in the art. The gene constructs may be inserted into vectors, which may be commercially available, suitable for transforming into plants and suitable for expression of the gene of interest in the transformed cells. The invention therefore provides use of a gene construct as defined hereinabove in the methods of the invention.

Plants are transformed with a vector comprising the sequence of interest. The skilled artisan is well aware of the genetic elements that must be present in the vector in order to successfully transform, select and propagate host cells containing the sequence of interest. The sequence of interest is operably linked to one or more control sequences (at least to a promoter). The terms "regulatory element", "control sequence" and "promoter" are all used interchangeably herein and are defined above.

Advantageously, any type of promoter, whether natural or synthetic, may be used to drive expression of the nucleic acid sequence. See the "Definitions" section herein for definitions of various promoter types.

According to a preferred feature of the invention, the nucleic acid of interest is operably linked to a constitutive promoter. A constitutive promoter is transcriptionally active during most but not necessarily all phases of growth and development and is substantially ubiquitously expressed. The constitutive promoter is preferably a GOS2 promoter, more preferably the constitutive promoter is a rice GOS2 promoter, further preferably the constitutive promoter is represented by a nucleic acid sequence substantially similar to SEQ ID NO: 56 or SEQ ID NO: 282, most preferably the constitutive promoter is as represented by SEQ ID NO: 56 or SEQ ID NO: 282. Examples of other constitutive promoters which may also be used to perform the methods of the invention are shown above.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The invention also provides a method for the production of transgenic plants having modified content of storage compounds in seeds relative to control plants, comprising introduction and expression in a plant of a nucleic acid sequence represented by SEQ ID NO: 229, or comprising introduction and expression in a plant of a nucleic acid sequence given in Table A, or comprising introduction and expression in a plant of a nucleic acid sequence encoding an orthologue, paralogue or homologue of any of the amino acid sequences given in Table A, or comprising introduction and expression in a plant of any of the nucleic acids defined herein as being useful in the methods of the invention.

More specifically, the present invention provides a method for the production of transgenic plants having modified content of storage compounds in seeds relative to control plants, which method comprises:

(i) introducing and expressing in a plant, plant part or plant cell a nucleic acid sequence represented by SEQ ID NO: 229, or comprising introducing and expressing in a plant a nucleic acid sequence given in Table A, or comprising introducing and expressing in a plant a nucleic acid sequence encoding an orthologue, paralogue or homologue of any of the amino acid sequences given in Table A; and
(ii) cultivating the plant cell under conditions promoting plant growth and development; and
(iii) harvest of seeds from the plant of (ii); and optionally
(iv) extraction of any one or more of lipids, oils, fatty acids, starch, sugar or protein from the seeds of (iii).

The harvested seeds may be processed to extract particular seed storage compounds, such as lipids, oils fatty acids, starch, sugar or protein. In particular, the seeds are used for oil extraction. The oils may be extracted from processed or unprocessed seeds.

The nucleic acid may be introduced directly into a plant cell or into the plant itself (including introduction into a tissue, organ or any other part of a plant). According to a preferred feature of the present invention, the nucleic acid is preferably introduced into a plant by transformation.

The term "transformation" as referred to herein is as defined above. Plant tissue capable of subsequent clonal propagation, whether by organogenesis or embryogenesis, may be transformed with a genetic construct of the present invention and a whole plant regenerated from there. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

Transformation of plant species is now a fairly routine technique. Advantageously, any of several transformation methods may be used to introduce the gene of interest into a suitable ancestor cell. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al. (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al. (1986) Mol. Gen Genet 202: 179-185); DNA or RNA-coated particle bombardment (Klein TM et al. (1987) Nature 327: 70) infection with (non-integrative) viruses and the like. Transgenic rice plants are preferably produced via *Agrobacterium*-mediated transformation using any of the well known methods for rice transformation, such as described in any of the following: published European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, or quantitative PCR, all techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing any one of the nucleic acids described above as being useful in the methods of the invention. Preferred host cells according to the invention are plant cells.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

According to a further preferred embodiment of the present invention, the plant is a crop plant typically cultivated for oil production. Examples of such crop plants for oil production include rapeseed, canola, linseed, soybean, sunflower, maize, oat, rye, barley, wheat, pepper, tagetes, cotton, oil palm, coconut palm, flax, castor, peanut, olive, avocado, sesame, jatropha.

The invention also extends to harvestable parts of a plant, particularly seeds, but also leaves, fruits, flowers, stems, rhizomes, tubers, roots and bulbs. The invention furthermore relates to products derived, preferably directly derived, from a harvestable part of such a plant, such as dry pellets or powders, oil, fat and fatty acids, starch or proteins. A particular product of interest derived from a harvestable part of a plant is oil.

The present invention also encompasses use of any of the nucleic acids mentioned herein as being useful in the methods of the invention in modifying seed storage content relative to control plants. Particularly useful is the nucleic acid represented by SEQ ID NO: 229, and any of the nucleic acid sequences given in Table A, and any nucleic acid sequence encoding an orthologue, paralogue or homologue of any of the polypeptide sequences given in Table A. The present invention also encompasses use of a polypeptide sequence represented by SEQ ID NO: 230, and use of a polypeptide sequences given in Table A in modifying seed storage content relative to control plants.

These nucleic acids or the encoded polypeptides may find use in breeding programmes in which a DNA marker is identified which may be genetically linked to a Class II HD-Zip transcription factor-encoding gene. The nucleic acids/ genes, or the polypeptides themselves may be used to define a molecular marker. This DNA or protein marker may then be used in breeding programmes to select plants having modified content of seed storage compounds.

Allelic variants may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

The nucleic acids may also be used as probes for genetically and physically mapping the genes that they are a part of, and as markers for traits linked to those genes. Such information may be useful in plant breeding in order to develop lines with desired phenotypes. Such use of requires only a nucleic acid sequence of at least 15 nucleotides in length. The nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence *in situ* hybridisation (FISH) mapping (Trask (1991) Trends Genet. 7:149-154). Although current methods of FISH mapping favor use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11: 95-96), polymorphism of PCR-amplified fragments (CAPS; Sheffield et al. (1993) Genomics 16:325-332), allele-specific ligation (Landegren et al. (1988) Science 241: 1077-1080), nucleotide extension reactions (Sokolov (1990) Nucleic Acid Res. 18: 3671), Radiation Hybrid Mapping (Walter et al. (1997) Nat. Genet. 7: 22-28) and Happy Mapping (Dear and Cook (1989) Nucleic Acid Res. 17: 6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having modified content of seed storage compounds, as described hereinbefore. These traits may also be combined with other economically advantageous traits, such as yield-enhancing traits, tolerance to other abiotic and biotic stresses, traits modifying various architectural features and/or biochemical and/or physiological features.

## Description of figures

The present invention will now be described with reference to the following figures in which:

**Figure 1** shows a section of a HD-Zip phylogenetic tree showing Class II HD-Zip members.
**Figure 2** shows an alignment of several Class II HD-Zip polypeptide sequences.

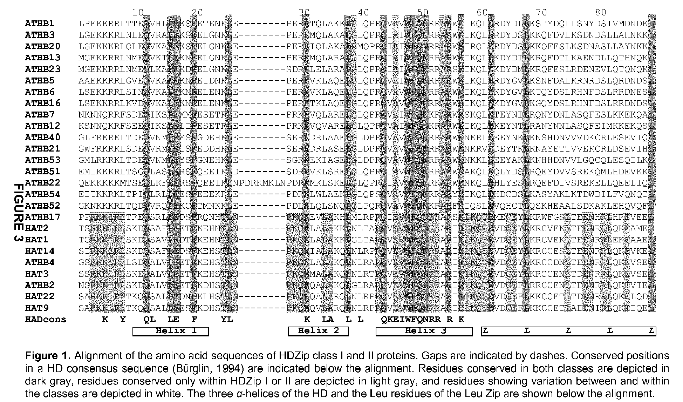

**Figure 3** is an alignment taken from Henrikson et al., 2005 (Plant Physiol, Vol. 139, pp. 509-518). The Leu Zip domains in all HDZip I and II proteins are in identical positions, C terminal to the HD. The HDZip domains of HDZip I and II proteins are similar to each other in sequence, although a number of amino acid positions distinguish HDZip I from II. The amino acid at position 46 is invariant within the HDZip I and II, but distinct between the classes. Several other amino acids, e.g. the ones at positions 6, 25, 29, 30, 58, and 61, are invariable within the HDZip II and differ from HDZip I amino acids, which show variation at these positions.
**Figure 4** shows a binary vector for increased expression in *Oryza sativa* of the *Arabidopsis thaliana* Class II HD-Zip transcription factor (HAT4)-encoding nucleic acid under the control of a GOS2 promoter.

## Examples

The present invention will now be described with reference to the following examples, which are by way of illustration alone. The following examples are not intended to completely define or otherwise limit the scope of the invention.
DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

<u>Example 1: Identification of sequences related to SEQ ID NO: 229 and SEQ ID NO: 230</u>

Sequences (full length cDNA, ESTs or genomic) related to SEQ ID NO: 229 and SEQ ID NO: 230 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). This program was used to find regions of local similarity between sequences by comparing nucleic acid or polypeptide sequences to sequence databases and by calculating the statistical significance of matches. The polypeptide encoded by SEQ ID NO: 229 was used for the TBLASTN algorithm, with default settings and with the filter to ignore low complexity sequences set off. The output of the analysis was viewed by pairwise comparison, and ranked according to the probability score (E-value), where the score reflects the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length.
Table A provides a list of nucleic acid sequences related to the nucleic acid sequence as represented by SEQ ID NO: 229.

**Table A:** Nucleic acid and polypeptide sequences related to the nucleic acid sequence of SEQ ID NO: 229 and SEQ ID NO: 230 which are useful in performing the methods of the present invention.

| Name | Source organism | Nucleic acid SEQ ID NO: | Poly-peptide SEQ ID NO: | Database accession number | Status |
|---|---|---|---|---|---|
| HAT4 | *Arabidopsis thaliana* | 229 | 230 | AT4G16780 | Full length |

(continued)

| Name | Source organism | Nucleic acid SEQ ID NO: | Poly-peptide SEQ ID NO: | Database accession number | Status |
|---|---|---|---|---|---|
| LD | *Arabidopsis thaliana* | 231 | 232 | NP_192165.1 | Full length |
| | *Oryza sativa* | 233 | 234 | Os03g47022 | Full length |
| | *Oryza sativa* | 235 | 236 | Os03g12860 | Full length |
| HAT22 | *Oryza sativa* | 237 | 238 | Os10g01470 | Full length |
| HAT14 | *Oryza sativa* | 239 | 240 | Os08g36220 | Full length |
| | *Oryza sativa* | 241 | 242 | Os09g27450 | Full length |
| HAT3 | *Oryza sativa* | 243 | 244 | Os06g04850 | Full length |
| S1 HDL2 | *Oryza sativa* | 245 | 246 | Os06g04870 | Full length |
| HD-ZIP | *Oryza sativa* | 247 | 248 | Os10g41230 | Full length |
| | *Arabidopsis thaliana* | 249 | 250 | NP_174025.3 | Full length |
| HAT14 | *Arabidopsis thaliana* | 251 | 252 | NP_974743.1 | Full length |
| HAT4 | *Arabidopsis thaliana* | 253 | 254 | NP_193411.1 | Full length |
| HAT1 | *Arabidopsis thaliana* | 255 | 256 | NP_193476.1 | Full length |
| HAT2 | *Arabidopsis thaliana* | 257 | 258 | NP_199548.1 | Full length |
| HB-4 | *Arabidopsis thaliana* | 259 | 260 | NP_182018.1 | Full length |
| HAT3 | *Arabidopsis thaliana* | 261 | 262 | NP_191598.1 | Full length |
| | *Oryza sativa* | 263 | 264 | Os04g46350 | Full length |
| | *Oryza sativa* | 265 | 266 | Os10g23090 | Full length |
| | *Oryza sativa* | 267 | 268 | Os10g23090 | Full length |
| | *Oryza sativa* | 269 | 270 | Os03g08960 | Full length |
| | *Oryza sativa* | 271 | 272 | Os08g37580 | Full length |
| | *Oryza sativa* | 273 | 274 | Os09g29460 | Full length |
| | *Oryza sativa* | 275 | 276 | Os10g39720 | Full length |
| | *Oryza sativa* | 277 | 278 | Os05g09630 | Full length |

Example 2: Alignment of relevant polypeptide sequences

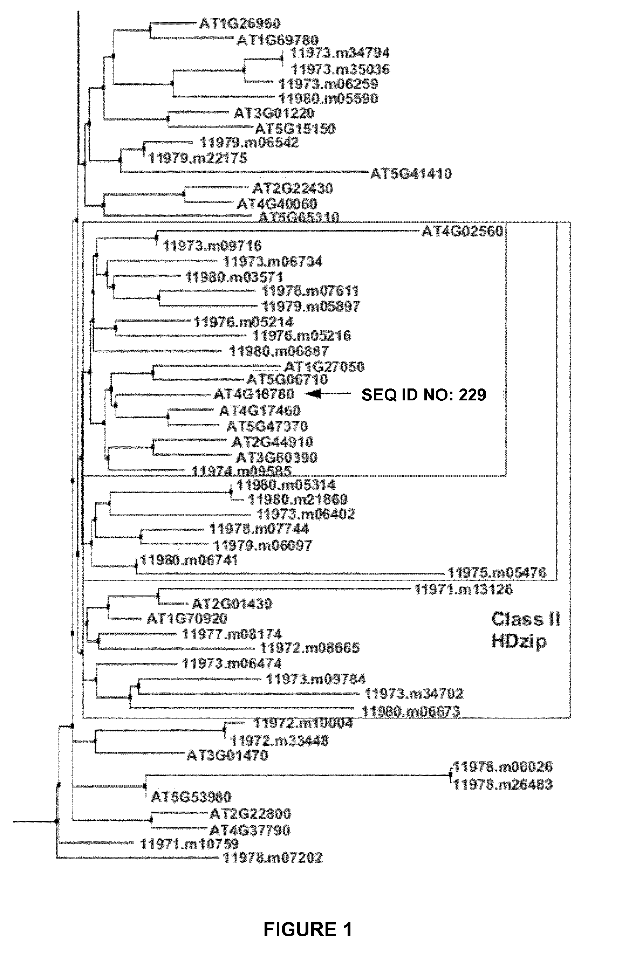

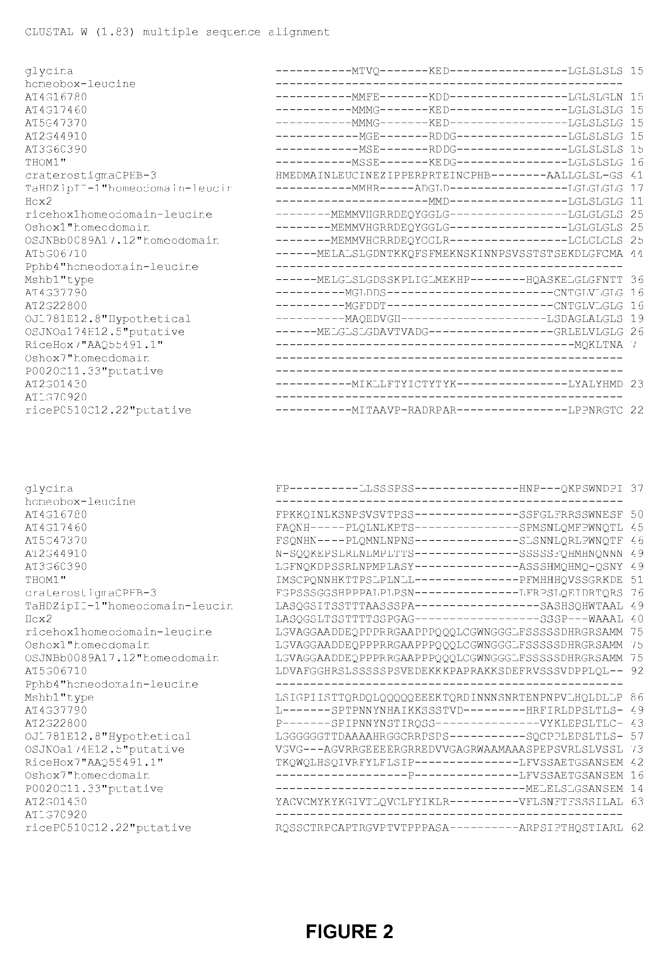

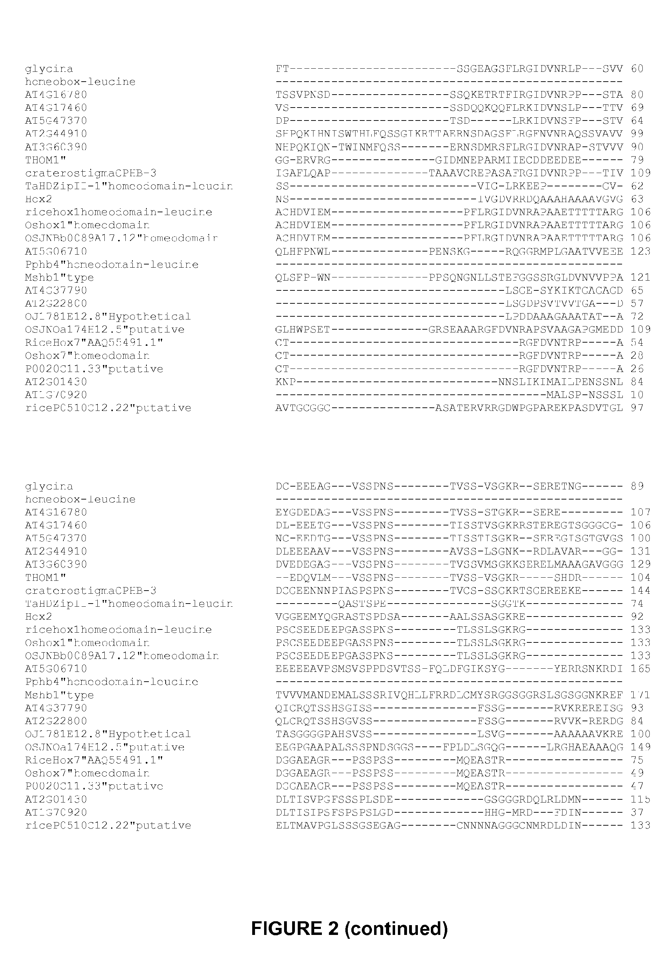

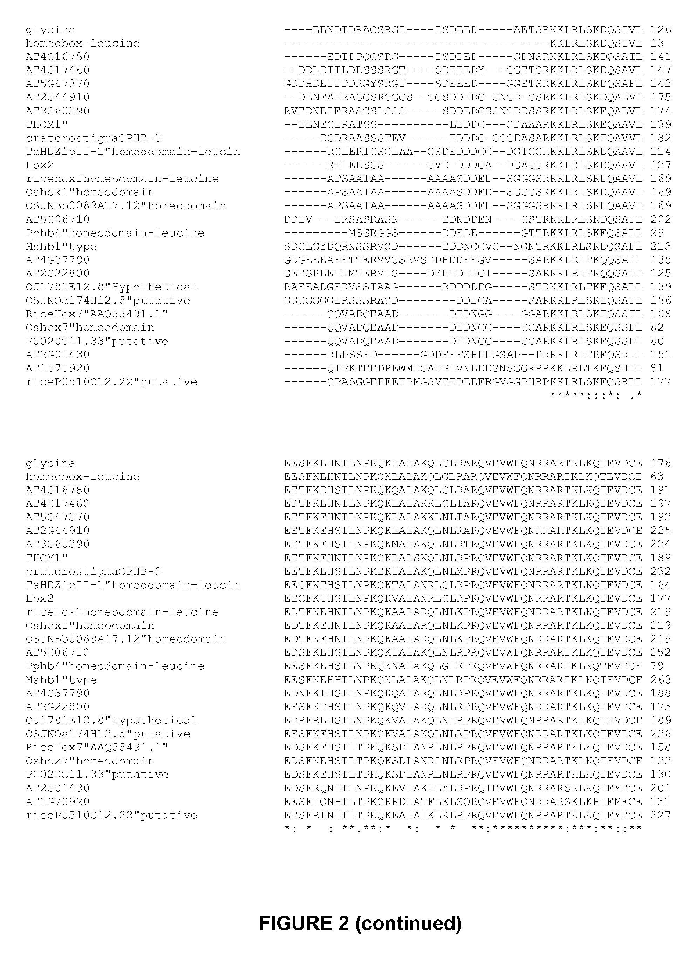

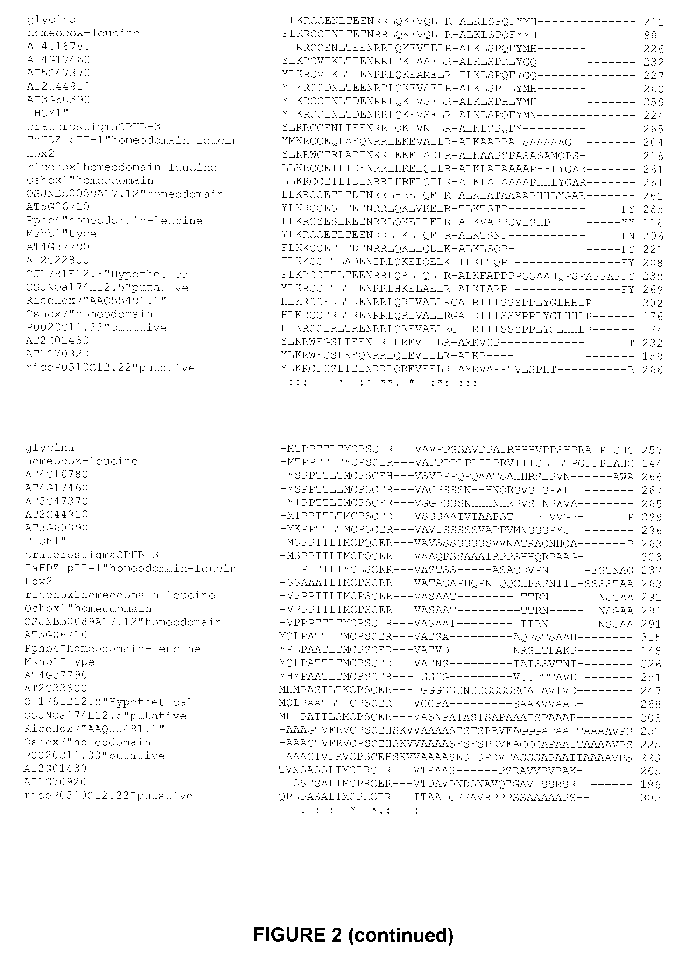

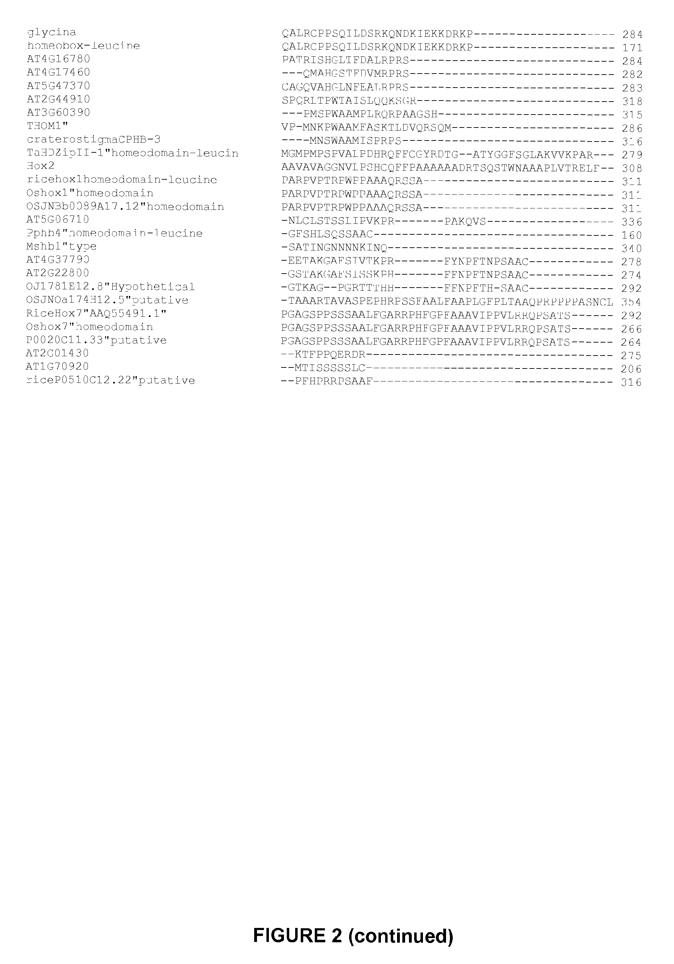

AlignX from the Vector NTI (Invitrogen) is based on the popular Clustal algorithm of progressive alignment (Thompson et al. (1997) Nucleic Acids Res 25:4876-4882; Chenna et al. (2003). Nucleic Acids Res 31:3497-3500). A phylogenetic tree was constructed using a neighbour-joining clustering algorithm (see Figure 1). Default values are for the gap open penalty of 10, for the gap extension penalty of 0,1 and the selected weight matrix is Blosum 62 (if polypeptides are aligned). The result of the multiple sequence alignment using is shown in Figure 2.

Example 3: Identification of domains comprised in polypeptide sequences useful in performing the methods of the invention

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Interpro is hosted at the European Bioinformatics Institute in the United Kingdom.

The results of the InterPro scan of the polypeptide sequence as represented by SEQ ID NO: 230 are presented in Table B.

**Table B:** InterPro scan results of the polypeptide sequence as represented by SEQ ID NO: 230

| Database | Accession number | Accession name |
|---|---|---|
| InterPro | IPR006712 | HDZIP |
| InterPro | IPR001356 | homeobox |
| InterPro | IPR003106 | HALZ |
| ProDom | PDAOK5J1 | homeobox PPHB4 DNA binding |
| ProDom | PD728753 | DNA binding |
| ProDom | PD064887 | DNA binding |
| PIR superfamily | PIR PS00001 | asn glycosylation motif |
| PIR superfamily | PS00004 | cAMP phosphorylation site |
| PIR superfamily | PS00005 | PKC phosphorylation site |
| PIR superfamily | PS00006 | CK2 phsophorylation site |
| PIR superfamily | PS00008 | myristoylation |
| PIR superfamily | PS00009 | amidation |
| PIR superfamily | PS00027 | homeobox |
| PIR superfamily | PS00029 | leucine zipper |
| Pfam | PF04618 | HDZIP |
| Pfam | PF00046 | homeobox |
| Pfam | PF02183 | HALZ |

Example 4: Cloning of nucleic acid sequence represented by SEQ ID NO: 229

The Arabidopsis thaliana HAT4-encoding gene was amplified by PCR using as a template an Arabidopsis thaliana cDNA library (Invitrogen, Paisley, UK). After reverse transcription of RNA extracted from seedlings, the cDNAs were cloned into pCMV Sport 6.0. Average insert size of the bank was 1.6 kb and the original number of clones was of the order of 1.67x107 cfu. Original titer was determined to be 3.34 x106 cfu/ml after first amplification of 6x1010 cfu/ml. After plasmid extraction, 200 ng of template was used in a 50 μl PCR mix. The primers used were:

Forward (SEQ ID NO: 283)
ggggacaagtttgtacaaaaaagcaggcttcacaatgatgttcgagaaagacgatctg

Reverse (SEQ ID NO: 284)
ggggaccactttgtacaagaaagctgggtttaggacctaggacgaagagcgt

which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone".

Plasmid pDONR201 was purchased from Invitrogen, as part of the Gateway® technology.

Example 5: Expression vector construction using the nucleic acid sequence as represented by SEQ ID NO: 229

The entry clone was subsequently used in an LR reaction with a destination vector used for *Oryza sativa* transformation. This vector contains as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 282) for constitutive expression was located upstream of this Gateway cassette.

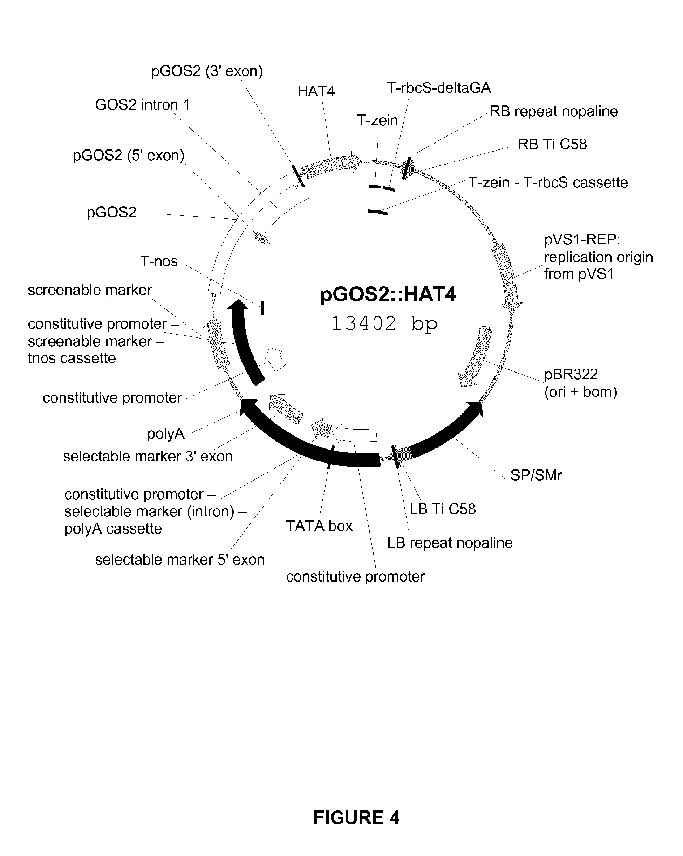

After the LR recombination step, the resulting expression vector (Figure 4) was transformed into *Agrobacterium* strain LBA4044 according to methods well known in the art.

Example 6: Plant transformation

*Rice transformation*

The Agrobacterium containing the expression vector was used to transform Oryza sativa plants. Mature dry seeds of the rice japonica cultivar Nipponbare were dehusked. Sterilization was carried out by incubating for one minute in 70% ethanol, followed by 30 minutes in 0.2%HgCl2, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

Agrobacterium strain LBA4404 containing the expression vector was used for cocultivation. Agrobacterium was inoculated on AB medium with the appropriate antibiotics and cultured for 3 days at 28°C. The bacteria were then collected and suspended in liquid co-cultivation medium to a density (OD600) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges 1996, Chan et al. 1993, Hiei et al. 1994).

Example 7: Phenotypic evaluation procedure

7.1 Evaluation setup

Approximately 35 independent T0 rice transformants were generated. The primary transformants were transferred from a tissue culture chamber to a greenhouse for growing and harvest of T1 seed. Eight events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%.

Four T1 events were further evaluated in the T2 generation following the same evaluation procedure as for the T1 generation but with more individuals per event.

*Drought screen*

Plants from T2 seeds are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level was reached again. The plants are then retransferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

Non-destructive oil measurements from rice seeds was measured using the Oxford QP20+ pulsed NMR. Whole seeds were used without dehusking.

7.2 Statistical analysis: F-test

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and to verify an overall effect of the gene, also known as a global gene effect. The threshold for significance for a true global gene effect was set at a 5% probability level for the F-test. A significant F-test value points to a gene effect, meaning that it is not only the mere presence or position of the gene that is causing the differences in phenotype.

7.3 Results

The best line showed an 11 % increase in oil content compared to control plants, with an average increase in oil content of 6% over all lines and a p value from the F-test of $<0.0001$

Example 8: Transformation of Corn

Transformation of maize (Zea mays) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for tansformation, but other genotypes can be used successfully as well. Ears are harvested from corn plant approximately 11 days after pollination (DAP) when the length of the immature embryo is about 1 to 1.2 mm. Immature embryos are cocultivated with Agrobacterium tumefaciens containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

Example 9: Transformation of Wheat

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with Agrobacterium tumefaciens containing the expression vector, and transgenic plants are recovered through organogenesis. After incubation with Agrobacterium, the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

Example 10: Transformation of Soybean

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for in vitro sowing. The hypocotyl, the radicle and one cotyledon are excised from seven-day old young seedlings. The epicotyl and the remaining cotyledon are further grown to develop axillary nodes. These axillary nodes are excised and incubated with Agrobacterium tumefaciens containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

Example 11: Transformation of Rapeseed/Canola

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with Agrobacterium (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with Agrobacterium, the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

Example 12: Transformation of Alfalfa

A regenerating clone of alfalfa (Medicago sativa) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of Agrobacterium tumefaciens C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

## Claims

1. Method for modifying the content of storage compounds in seeds relative to control plants, comprising modulating expression in a plant of a nucleic acid encoding a Class II HD-Zip transcription factor.

2. Method according to claim 1, wherein said Class II HD-Zip transcription factor comprises the following: (i) a homeodomain box; (ii) a leucine zipper; and (iii) Motifs I, II and III in any order

   - Motif I: RKKLRL, or Motif I with one or more conservative amino acid substitution at any position, and/or Motif I with one or two non-conservative change(s) at any position; and
   - Motif II:TKLKQTEVDCEFLRRCCENLTEEN, or Motif II with one or more conservative amino acid substitution at any position, and/or a motif having in increasing order of preference at least 50%, 55%, 60%, 65%, 70%, 75%, 80%, 85%, 90%, 95% or more sequence identity to Motif II; and
- Motif III: TLTMCPSCER, or Motif III with one or more conservative amino acid substitution at any position, and/or Motif III with one, two or three non-conservative change(s) at any position.

**3.** Method according to claim 1 or 2, wherein said nucleic acid encoding a Class II HD-Zip transcription factor is any one of the nucleic acid sequences given in Table A or a portion thereof, or a sequence capable of hybridising with any one of the nucleic acids given in Table A.

**4.** Method according to any one of claims 1 to 3, wherein said modulated expression is effected by introducing and expressing in a plant a nucleic acid encoding a Class II HD-Zip transcription factor.

**5.** Method according to any preceding claim, wherein said modified content of storage compounds comprises seeds having a modified content of one or more of lipids, oil, fatty acids, starch, sugar and proteins relative to that of control plants.

**6.** Method according to claim 5, wherein said modified content is increased oil content relative to the seeds of control plants.

**7.** Method according to any one of claims 4 to 6, wherein said nucleic acid is operably linked to a constitutive promoter, preferably to a GOS2 promoter.

**8.** Method according to any preceding claim, wherein said nucleic acid encoding a encoding a Class II HD-Zip transcription factor is of plant origin, preferably from a dicotyledonous plant, further preferably from the family Brassicaceae, more preferably from *Arabidopsis thaliana.*

**9.** Plant or part thereof including seeds obtainable by a method according to any preceding claim, wherein said plant or part thereof comprises a nucleic acid encoding a encoding a Class II HD-Zip transcription factor.

**10.** Construct comprising:

(i) A nucleic acid encoding a Class II HD-Zip transcription factor as defined hereinabove;
(ii) One or more control sequences operably linked to the nucleic acid of (i).

**11.** Construct according to claim 10, wherein said one or more control sequences is at least a constitutive promoter, preferably a GOS2 promoter.

**12.** Construct according to claim 10 or 11, wherein said nucleic acid encoding a Class II HD-Zip transcription factor is any one of the nucleic acid sequences given in Table A or any nucleic acid sequence encoding an orthologue, paralogue or homologue of any of the polypeptide sequences given in Table A.

**13.** Use of a construct according to any one of claims 10 to 12 for making plants having modified seed storage content relative to control plants.

**14.** Plant, plant part or plant cell transformed with a construct according to any one of claims 10 to 12.

**15.** Method for the production of a transgenic plant having modified content of storage compounds in seeds relative to control plants, which method comprises:

(i) introducing and expressing in a plant, plant part or plant cell a nucleic acid encoding a Class II HD-Zip transcription factor, preferably a nucleic acid sequence given in Table A, or comprising introducing and expressing in a plant a nucleic acid sequence encoding an orthologue, paralogue or homologue of any of the amino acid sequences given in Table A; and
(ii) cultivating the plant cell under conditions promoting plant growth and development; and
(iii) harvesting seeds from the plant of (ii); and optionally
(iv) extraction of any one or more of lipids, oils, fatty acids, starch, sugar or protein from the seeds of (iii).

**16.** Transgenic plant having modified content of storage compounds in seeds relative to control plants, said modified content resulting from increased expression of a nucleic acid encoding a Class II HD-Zip transcription factor.

**17.** Transgenic plant according to claim 9, 14 or 16, wherein said plant is a crop plant cultivated for oil production, preferably rapeseed, canola, linseed, soybean, sunflower, maize, oat, rye, barley, wheat, pepper, tagetes, cotton, oil palm, coconut palm, flax, castor, peanut, olive, avocado, sesame, jatropha.

**18.** Harvestable parts of a plant according to claim 17, wherein said harvestable parts are seeds.

**19.** Products derived from a plant according to claim 17 and/or from harvestable parts of a plant according to claim 18, preferably wherein said products are one or more of lipids, oil, fatty acids, starch, sugar and proteins.

**20.** Use of a nucleic acid encoding a Class II HD-Zip transcription factor in modifying the content of storage compounds in seeds relative to control plants.

**21.** Use according to claim 20, wherein said Class II HD-Zip transcription factor is a nucleic acid sequence given in Table A, or is a nucleic acid sequence encoding an orthologue, paralogue or homologue of any of the amino acid sequences given in Table A.

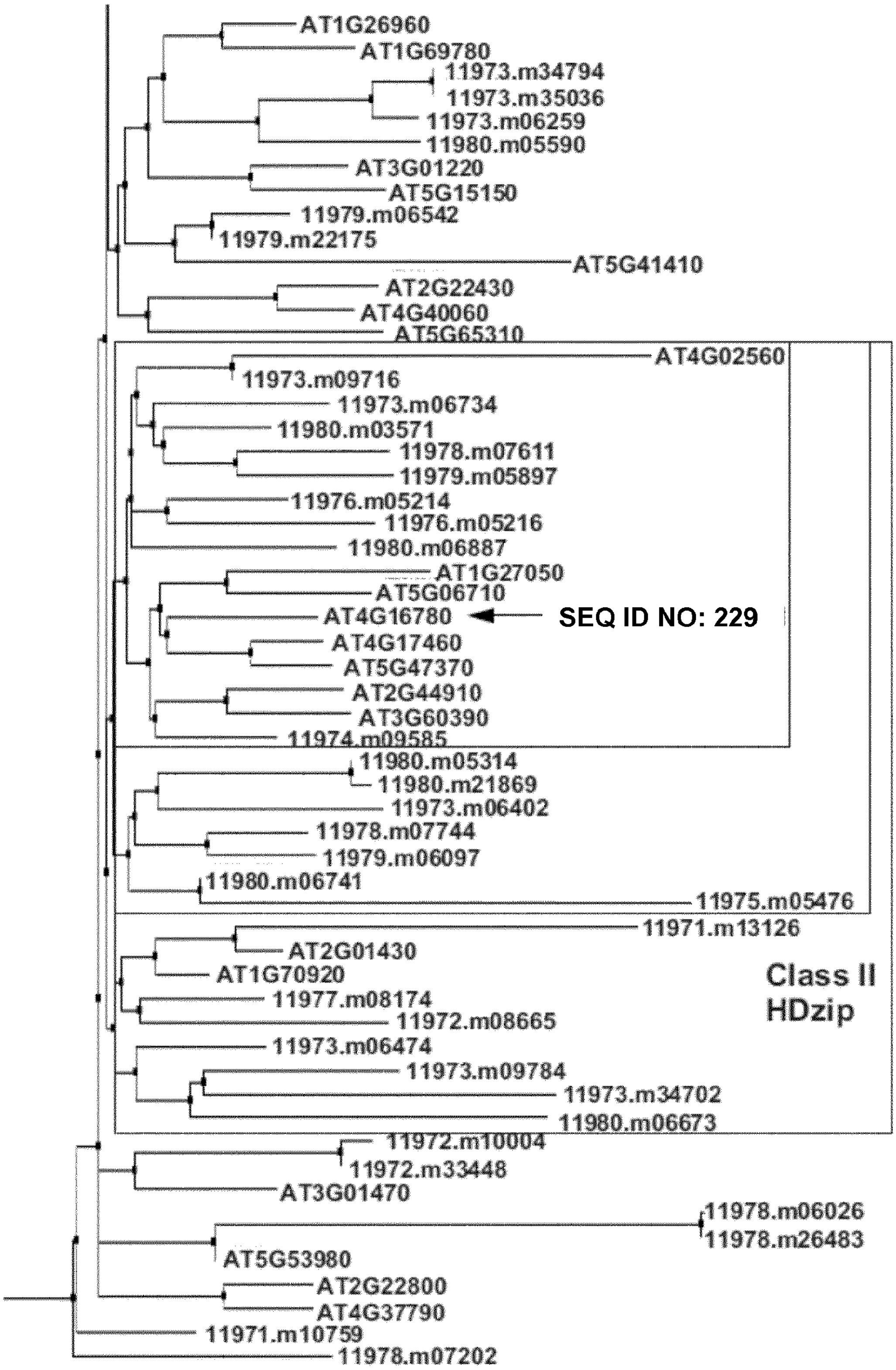
AT1G26960
AT1G69780
11973.m34794
11973.m35036
11973.m06259
11980.m05590
AT3G01220
AT5G15150
11979.m06542
11979.m22175
AT5G41410
AT2G22430
AT4G40060
AT5G65310
AT4G02560
11973.m09716
11973.m06734
11980.m03571
11978.m07611
11979.m05897
11976.m05214
11976.m05216
11980.m06887
AT1G27050
AT5G06710
AT4G16780
SEQ ID NO: 229
AT4G17460
AT5G47370
AT2G44910
AT3G60390
11974.m09585
11980.m05314
11980.m21869
11973.m06402
11978.m07744
11979.m06097
11980.m06741
11975.m05476
11971.m13126
AT2G01430
AT1G70920
Class II
HDzip
11977.m08174
11972.m08665
11973.m06474
11973.m09784
11973.m34702
11980.m06673
11972.m10004
11972.m33448
AT3G01470
11978.m06026
11978.m26483
AT5G53980
AT2G22800
AT4G37790
11971.m10759
11978.m07202

FIGURE 1

```
CLUSTAL W (1.83) multiple sequence alignment


glycina                          -----------MTVQ-------KED-----------------LGLSLSLS 15
homeobox-leucine                 --------------------------------------------------
AT4G16780                        -----------MMFE-------KDD-----------------LGLSLGLN 15
AT4G17460                        -----------MMMG-------KED-----------------LGLSLSLG 15
AT5G47370                        -----------MMMG-------KED-----------------LGLSLSLG 15
AT2G44910                        ------------MGE-------RDDG----------------LGLSLSLG 15
AT3G60390                        ------------MSE-------RDDG----------------LGLSLSLS 15
THOM1"                           -----------MSSE-------KEDG----------------LGLSLSLG 16
craterostigmaCPEB-3              HMEDMAINLEUCINEZIPPERPRTEINCPHB--------AALLGLSL-GS 41
TaHDZipII-1"homeodomain-leucin   -----------MMHR-----ADGLD-----------------LGLGLGLG 17
Hox2                             ----------------------MMD-----------------LGLSLGLG 11
ricehox1homeodomain-leucine      --------MEMMVHGRRDEQYGGLG-----------------LGLGLGLS 25
Oshox1"homeodomain               --------MEMMVHGRRDEQYGGLG-----------------LGLGLGLS 25
OSJNBb0089A17.12"homeodomain     --------MEMMVHGRRDEQYGGLR-----------------LGLGLGLS 25
AT5G06710                        ------MELALSLGDNTKKQFSFMEKNSKINNPSVSSTSTSEKDLGFCMA 44
Pphb4"homeodomain-leucine        --------------------------------------------------
Mshb1"type                       ------MELGLSLGDSSKPLIGLMEKHP--------HQASKELGLGFNTT 36
AT4G37790                        ----------MGLDDS------------------------CNTGLVLGLG 16
AT2G22800                        ----------MGFDDT------------------------CNTGLVLGLG 16
OJ1781E12.8"Hypothetical         ----------MAQEDVGH---------------------LSDAGLALGLS 19
OSJNOa174E12.5"putative          ------MELGLSLGDAVTVADG------------------GRLELVLGLG 26
RiceHox7"AAQ55491.1"             -------------------------------------------MQKLTNA 7
Oshox7"homeodomain               --------------------------------------------------
P0020C11.33"putative             --------------------------------------------------
AT2G01430                        -----------MIKLLFTYICTYTYK----------------LYALYHMD 23
AT1G70920                        --------------------------------------------------
ricePC510C12.22"putative         -----------MITAAVP-RADRPAR----------------LPPNRGTC 22


glycina                          FP----------LLSSSPSS---------------HNP---QKPSWNDPI 37
homeobox-leucine                 --------------------------------------------------
AT4G16780                        FPKKQINLKSNPSVSVTPSS---------------SSFGLFRRSSWNESF 50
AT4G17460                        FAQNH-----PLQLNLKPTS---------------SPMSNLQMFPWNQTL 45
AT5G47370                        FSQNHN----PLQMNLNPNS---------------SLSNNLQRLPWNQTF 46
AT2G44910                        N-SQQKEPSLRLNLMPLTTS---------------SSSSSFQHMHNQNNN 49
AT3G60390                        LGFNQKDPSSRLNPMPLASY---------------ASSSHMQHMQ-QSNY 49
THOM1"                           IMSCPQNNHKTTPSLPLNLL---------------PFMHHHQVSSGRKDE 51
craterostigmaCPEB-3              FGPSSSGGSHPPPALPLPSN---------------LFRPSLQETDRTQRS 76
TaHDZipII-1"homeodomain-leucin   LASQGSITSSTTTAASSSPA------------------SASHSQHWTAAL 49
Hox2                             LASQGSLTSSTTTTSSPGAG------------------SSSP---WAAAL 40
ricehox1homeodomain-leucine      LGVAGGAADDEQPPPRRGAAPPPQQQLCGWNGGGLFSSSSSDHRGRSAMM 75
Oshox1"homeodomain               LGVAGGAADDEQPPPRRGAAPPPQQQLCGWNGGGLFSSSSSDHRGRSAMM 75
OSJNBb0089A17.12"homeodomain     LGVAGGAADDEQPPPRRGAAPPPQQQLCGWNGGGLFSSSSSDHRGRSAMM 75
AT5G06710                        LDVAFGGHRSLSSSSSPSVEDEKKKPAPRAKKSDEFRVSSSVDPPLQL-- 92
Pphb4"homeodomain-leucine        --------------------------------------------------
Mshb1"type                       LSIGPIISTTQRDQLQQQQQEEEKTQRDINNNSNRTENPNPVLHQLDLLP 86
AT4G37790                        L-------SPTPNNYNHAIKKSSSTVD---------HRFIRLDPSLTLS- 49
AT2G22800                        P-------SPIPNNYNSTIRQSS---------------VYKLEPSLTLC- 43
OJ1781E12.8"Hypothetical         LGGGGGGTTDAAAAHRGGCRRPSPS-----------SQCPPLEPSLTLS- 57
OSJNOa174E12.5"putative          VGVG---AGVRRGEEEERGRREDVVGAGRWAAMAAASPEPSVRLSLVSSL 73
RiceHox7"AAQ55491.1"             TKQWQLHSQIVRFYLFLSIP---------------LFVSSAETGSANSEM 42
Oshox7"homeodomain               -------------------P---------------LFVSSAETGSANSEM 16
P0020C11.33"putative             ------------------------------------MELELSLGSANSEM 14
AT2G01430                        YACVCMYKYKGIVTLQVCLFYIKLR----------VFLSNFTFSSSILAL 63
AT1G70920                        --------------------------------------------------
ricePC510C12.22"putative         RQSSCTRPCAPTRGVPTVTPPPASA----------ARPSIPTHQSTIARL 62
```

**FIGURE 2**

```
glycina                             FT------------------------SSGEAGSFLRGIDVNRLP---SVV 60
homeobox-leucine                    --------------------------------------------------
AT4G16780                           TSSVPNSD-----------------SSQKETRTFIRGIDVNRPP---STA 80
AT4G17460                           VS-----------------------SSDQQKQQFLRKIDVNSLP---TTV 69
AT5G47370                           DP-----------------------TSD------LRKIDVNSFP---STV 64
AT2G44910                           SFPQKTHNTSWTHLFQSSGTKRTTAERNSDAGSFLRGFNVNRAQSSVAVV 99
AT3G60390                           NEPQKIQN-TWINMFQSS-------ERNSDMRSFLRGIDVNRAP-STVVV 90
THOM1"                              GG-ERVRG---------------GIDMNEPARMIIECDDEEDEE------ 79
craterostigmaCPEB-3                 IGAFLQAP--------------TAAAVCREPASAFRGIDVNRPP---TIV 109
TaHDZipII-1"homeodomain-leucin      SS---------------------------VIG-LRKEEP--------CV- 62
Hox2                                NS---------------------------IVGDVRRDQAAAHAAAAVGVG 63
ricehox1homeodomain-leucine         ACHDVIEM-------------------PFLRGIDVNRAPAAETTTTTARG 106
Oshox1"homeodomain                  ACHDVIEM-------------------PFLRGIDVNRAPAAETTTTTARG 106
OSJNBb0C89A17.12"homeodomain        ACHDVIEM-------------------PFLRGIDVNRAPAAETTTTTARG 106
AT5G06710                           QLHFPNWL--------------PENSKG-----RQGGRMPLGAATVVEEE 123
Pphb4"homeodomain-leucine           --------------------------------------------------
Mshb1"type                          QLSFP-WN--------------PPSQNGNLLSTEFGGSSRGLDVNVVPPA 121
AT4G37790                           ---------------------------------LSCE-SYKIKTCACACD 65
AT2G22800                           ---------------------------------LSGDPSVTVVTGA---D 57
OJ1781E12.8"Hypothetical            ---------------------------------LPDDAAAGAAATAT--A 72
OSJNOa174E12.5"putative             GLHWPSET--------------GRSEAAARGFDVNRAPSVAAGAPGMEDD 109
RiceHox7"AAQ55491.1"                CT---------------------------------RGFDVNTRP-----A 54
Oshox7"homeodomain                  CT---------------------------------RGFDVNTRP-----A 28
P0020C11.33"putative                CT---------------------------------RGFDVNTRP-----A 26
AT2G01430                           KNP-----------------------------NNSLIKIMAILPENSSNL 84
AT1G70920                           ---------------------------------------MALSP-NSSSL 10
ricePC510C12.22"putative            AVTGCGGC---------------ASATERVRRGDWPGPAREKPASDVTGL 97
```

```
glycina                             DC-EEEAG---VSSPNS--------TVSS-VSGKR--SERETNG------ 89
homeobox-leucine                    --------------------------------------------------
AT4G16780                           EYGDEDAG---VSSPNS--------TVSS-STGKR--SERE--------- 107
AT4G17460                           DL-EEETG---VSSPNS--------TISSTVSGKRRSTEREGTSGGGCG- 106
AT5G47370                           NC-EEDTG---VSSPNS--------TISSTISGKR--SEREGTSGTGVGS 100
AT2G44910                           DLEEEAAV---VSSPNS--------AVSS-LSGNK--RDLAVAR---GG- 131
AT3G60390                           DVEDEGAG---VSSPNS--------TVSSVMSGKKSERELMAAAGAVGGG 129
THOM1"                              --EDQVLM---VSSPNS--------TVSS-VSGKR-----SHDR------ 104
craterostigmaCPEB-3                 DCCEENNNPIASPSPNS--------TVCS-SSCKRTSCEREEKE------ 144
TaHDZipII-1"homeodomain-leucin      ---------QASTSPE---------------SGGTK-------------- 74
Hox2                                VGGEEMYQGRASTSPDSA-------AALSSASGKRE-------------- 92
ricehox1homeodomain-leucine         PSCSEEDEEPGASSPNS---------TLSSLSGKRG-------------- 133
Oshox1"homeodomain                  PSCSEEDEEPGASSPNS---------TLSSLSGKRG-------------- 133
OSJNBb0C89A17.12"homeodomain        PSCSEEDEEPGASSPNS---------TLSSLSGKRG-------------- 133
AT5G06710                           EEEEEAVPSMSVSPPDSVTSS-FQLDFGIKSYG-------YERRSNKRDI 165
Pphb4"homeodomain-leucine           --------------------------------------------------
Mshb1"type                          TVVVMANDEMALSSSRIVQHLLFRRDLCMYSRGGSGGRSLSGSGGNKREF 171
AT4G37790                           QICRQTSSHSGISS---------------FSSG-------RVKREREISG 93
AT2G22800                           QLCRQTSSHSGVSS---------------FSSG-------RVVK-RERDG 84
OJ1781E12.8"Hypothetical            TASGGGGPAHSVSS---------------LSVG-------AAAAAAVKRE 100
OSJNOa174E12.5"putative             EEGPGAAPALSSSPNDSGGS----FPLDLSGQG------LRGHAEAAAQG 149
RiceHox7"AAQ55491.1"                DGGAEAGR---PSSPSS---------MQEASTR----------------- 75
Oshox7"homeodomain                  DGGAEAGR---PSSPSS---------MQEASTR----------------- 49
P0020C11.33"putative                DGGAEAGR---PSSPSS---------MQEASTR----------------- 47
AT2G01430                           DLTISVPGFSSSPLSDE-------------GSGGGRDQLRLDMN------ 115
AT1G70920                           DLTISIPSFSPSPSLGD-------------HHG-MRD---FDIN------ 37
ricePC510C12.22"putative            ELTMAVPGLSSSGSEGAG--------CNNNNAGGGCNMRDLDIN------ 133
```

# FIGURE 2 (continued)

```
glycina                               ----EENDTDRACSRGI----ISDEED-----AETSRKKLRLSKDQSIVL 126
homeobox-leucine                      -------------------------------------KKLRLSKDQSIVL 13
AT4G16780                             ------EDTDPQGSRG-----ISDDED-----GDNSRKKLRLSKDQSAIL 141
AT4G17460                             --DDLDITLDRSSSRGT----SDEEEDY---GGETCRKKLRLSKDQSAVL 147
AT5G47370                             GDDHDEITPDRGYSRGT----SDEEED----GGETSRKKLRLSKDQSAFL 142
AT2G44910                             --DENEAERASCSRGGGS--GGSDDEDG-GNGD-GSRKKLRLSKDQALVL 175
AT3G60390                             RVFDNEIERASCSTGGG-----SDDEDGSGNGDDSSRKKLRLSKEQALVL 174
THOM1"                                --EENEGERATSS----------LEDDG---GDAAARKKLRLSKEQAAVL 139
craterostigmaCPHB-3                   -----DGDRAASSSFEV------EDDDG-GGGDASARKKLRLSKEQAVVL 182
TaHDZipII-1"homeodomain-leucin        ------RGLERTGSGLAA--GSDEDDDGG--DGTGGRKKLRLSKDQAAVL 114
Hox2                                  ------RELERSGS------GVD-DDDGA--DGAGGRKKLRLSKDQAAVL 127
ricehox1homeodomain-leucine           ------APSAATAA------AAAASDDED--SGGGSRKKLRLSKDQAAVL 169
Oshox1"homeodomain                    ------APSAATAA------AAAASDDED--SGGGSRKKLRLSKDQAAVL 169
OSJNBb0089A17.12"homeodomain          ------APSAATAA------AAAASDDED--SGGGSRKKLRLSKDQAAVL 169
AT5G06710                             DDEV---ERSASRASN------EDNDDEN----GSTRKKLRLSKDQSAFL 202
Pphb4"homeodomain-leucine             ---------MSSRGGS------DDEDE------GTTRKKLRLSKEQSALL 29
Mshb1"type                            SDCEGYDQRNSSRVSD------EDDNGGVG--NGNTRKKLRLSKDQSAFL 213
AT4G37790                             GDGEEEAEETTERVVCSRVSDDHDDEEGV-----SARKKLRLTKQQSALL 138
AT2G22800                             GEESPEEEEMTERVIS----DYHEDEEGI-----SARKKLRLTKQQSALL 125
OJ1781E12.8"Hypothetical              RAEEADGERVSSTAAG------RDDDDDG-----STRKKLRLTKEQSALL 139
OSJNOa174H12.5"putative               GGGGGGGERSSSRASD--------DDEGA-----SARKKLRLSKEQSAFL 186
RiceHox7"AAQ55491.1"                  ------QQVADQEAAD-------DEDNGG----GGARKKLRLSKEQSSFL 108
Oshox7"homeodomain                    ------QQVADQEAAD-------DEDNGG----GGARKKLRLSKEQSSFL 82
PC020C11.33"putative                  ------QQVADQEAAD-------DEDNGG----GGARKKLRLSKEQSSFL 80
AT2G01430                             ------RLPSSED------GDDEEFSHDDGSAP--PRKKLRLTREQSRLL 151
AT1G70920                             ------QTPKTEEDREWMIGATPHVNEDDSNSGGRRRKKLRLTKEQSHLL 81
riceP0510C12.22"putative              ------QPASGGEEEEFPMGSVEEDEEERGVGGPHRPKKLRLSKEQSRLL 177
                                                                           *****:::*: .*


glycina                               EESFKEHNTLNPKQKLALAKQLGLRARQVEVWFQNRRARTKLKQTEVDCE 176
homeobox-leucine                      EESFKEHNTLNPKQKLALAKQLGLRARQVEVWFQNRRARTKLKQTEVDCE 63
AT4G16780                             EETFKDHSTLNPKQKQALAKQLGLRARQVEVWFQNRRARTKLKQTEVDCE 191
AT4G17460                             EDTFKEHNTLNPKQKLALAKKLGLTARQVEVWFQNRRARTKLKQTEVDCE 197
AT5G47370                             EETFKEHNTLNPKQKLALAKKLNLTARQVEVWFQNRRARTKLKQTEVDCE 192
AT2G44910                             EETFKEHSTLNPKQKLALAKQLNLRARQVEVWFQNRRARTKLKQTEVDCE 225
AT3G60390                             EETFKEHSTLNPKQKMALAKQLNLRTRQVEVWFQNRRARTKLKQTEVDCE 224
THOM1"                                EETFKEHNTLNPKQKLALSKQLNLRPRQVEVWFQNRRARTKLKQTEVDCE 189
craterostigmaCPHB-3                   EETFKEHSTLNPKEKIALAKQLNLMPRQVEVWFQNRRARTKLKQTEVDCE 232
TaHDZipII-1"homeodomain-leucin        EECFKTHSTLNPKQKTALANRLGLRPRQVEVWFQNRRARTKLKQTEVDCE 164
Hox2                                  EECFKTHSTLNPKQKVALANRLGLRPRQVEVWFQNRRARTKLKQTEVDCE 177
ricehox1homeodomain-leucine           EDTFKEHNTLNPKQKAALARQLNLKPRQVEVWFQNRRARTKLKQTEVDCE 219
Oshox1"homeodomain                    EDTFKEHNTLNPKQKAALARQLNLKPRQVEVWFQNRRARTKLKQTEVDCE 219
OSJNBb0089A17.12"homeodomain          EDTFKEHNTLNPKQKAALARQLNLKPRQVEVWFQNRRARTKLKQTEVDCE 219
AT5G06710                             EDSFKEHSTLNPKQKIALAKQLNLRPRQVEVWFQNRRARTKLKQTEVDCE 252
Pphb4"homeodomain-leucine             EESFKEHSTLNPKQKNALAKQLGLRPRQVEVWFQNRRARTKLKQTEVDCE 79
Mshb1"type                            EESFKEHHTLNPKQKLALAKQLNLRPRQVEVWFQNRRARTKLKQTEVDCE 263
AT4G37790                             EDNFKLHSTLNPKQKQALARQLNLRPRQVEVWFQNRRARTKLKQTEVDCE 188
AT2G22800                             EESFKDHSTLNPKQKQVLARQLNLRPRQVEVWFQNRRARTKLKQTEVDCE 175
OJ1781E12.8"Hypothetical              EDRFREHSTLNPKQKVALAKQLNLRPRQVEVWFQNRRARTKLKQTEVDCE 189
OSJNOa174H12.5"putative               EESFKEHSTLNPKQKVALAKQLNLRPRQVEVWFQNRRARTKLKQTEVDCE 236
RiceHox7"AAQ55491.1"                  EDSFKEHSTLTPKQKSDLANRLNLRPRQVEVWFQNRRARTKLKQTEVDCE 158
Oshox7"homeodomain                    EDSFKEHSTLTPKQKSDLANRLNLRPRQVEVWFQNRRARTKLKQTEVDCE 132
PC020C11.33"putative                  EDSFKEHSTLTPKQKSDLANRLNLRPRQVEVWFQNRRARTKLKQTEVDCE 130
AT2G01430                             EDSFRQNHTLNPKQKEVLAKHLMLRPRQIEVWFQNRRARSKLKQTEMECE 201
AT1G70920                             EESFIQNHTLTPKQKKDLATFLKLSQRQVEVWFQNRRARSKLKHTEMECE 131
riceP0510C12.22"putative              EESFRLNHTLTPKQKEALAIKLKLRPRQVEVWFQNRRARTKLKQTEMECE 227
                                      *: *  : **.**:*  *:  * *  **:**********:***:**::**
```

# FIGURE 2 (continued)

```
glycina                              FLKRCCENLTEENRRLQKEVQELR-ALKLSPQFYMH-------------- 211
homeobox-leucine                     FLKRCCENLTEENRRLQKEVQELR-ALKLSPQFYMH-------------- 98
AT4G16780                            FLRRCCENLTEENRRLQKEVTELR-ALKLSPQFYMH-------------- 226
AT4G17460                            YLKRCVEKLTEENRRLEKEAAELR-ALKLSPRLYCQ-------------- 232
AT5G47370                            YLKRCVEKLTEENRRLQKEAMELR-TLKLSPQFYGQ-------------- 227
AT2G44910                            YLKRCCDNLTEENRRLQKEVSELR-ALKLSPHLYMH-------------- 260
AT3G60390                            YLKRCCENLTDENRRLQKEVSELR-ALKLSPHLYMH-------------- 259
THOM1"                               YLKRCCENLTDENRRLQKEVSELR-ALKLSPQFYMN-------------- 224
craterostigmaCPHB-3                  YLRRCCENLTEENRRLQKEVNELR-ALKLSPQFY---------------- 265
TaHDZipII-1"homeodomain-leucin       YMKRCCEQLAEQNRRLEKEVAELR-ALKAAPPAHSAAAAAG--------- 204
Hox2                                 YLKRWCERLADENKRLEKELADLR-ALKAAPSPASASAMQPS-------- 218
ricehox1homeodomain-leucine          LLKRCCETLTDENRRLERELQELR-ALKLATAAAAPHHLYGAR------- 261
Oshox1"homeodomain                   LLKRCCETLTDENRRLERELQELR-ALKLATAAAAPHHLYGAR------- 261
OSJNBb0089A17.12"homeodomain         LLKRCCETLTDENRRLHRELQELR-ALKLATAAAAPHHLYGAR------- 261
AT5G06710                            YLKRCCESLTEENRRLQKEVKELR-TLKTSTP----------------FY 285
Pphb4"homeodomain-leucine            LLKRCYESLKEENRRLQKELLELR-AIKVAPPCVISHD----------YY 118
Mshb1"type                           YLKRCCETLTEENRRLHKELQELR-ALKTSNP----------------FN 296
AT4G37790                            FLKKCCETLTDENRRLQKELQDLK-ALKLSQP----------------FY 221
AT2G22800                            FLKKCCETLADENIRLQKEIQELK-TLKLTQP----------------FY 208
OJ1781E12.8"Hypothetical             FLKRCCETLTEENRRLQRELQELR-ALKFAPPPPSSAAHQPSPAPPAPFY 238
OSJNOa174H12.5"putative              YLKRCCETLTEENRRLHKELAELR-ALKTARP----------------FY 269
RiceHox7"AAQ55491.1"                 HLKRCCERLTRENRRLQREVAELRGALRTTTSSYPPLYGLHHLP------ 202
Oshox7"homeodomain                   HLKRCCERLTRENRRLQREVAELRGALRTTTSSYPPLYGLHHLP------ 176
P0020C11.33"putative                 HLKRCCERLTRENRRLQREVAELRGTLRTTTSSYPPLYGLEELP------ 174
AT2G01430                            YLKRWFGSLTEENHRLHREVEELR-AMKVGP-----------------T 232
AT1G70920                            YLKRWFGSLKEQNRRLQIEVEELR-ALKP--------------------- 159
riceP0510C12.22"putative             YLKRCFGSLTEENRRLQREVEELR-AMRVAPPTVLSPHT----------R 266
                                      :::    *  :* **. *  :*: :::
```

```
glycina                              -MTPPTTLTMCPSCER---VAVPPSSAVDPATREEEVPPSEPRAFPICHC 257
homeobox-leucine                     -MTPPTTLTMCPSCER---VAFPPPLPLILPRVTITCLELTPGPFPLAHG 144
AT4G16780                            -MSPPTTLTMCPSCEH---VSVPPPQPQAATSAHHRSLPVN------AWA 266
AT4G17460                            -MSPPTTLLMCPSCER---VAGPSSSN--HNQRSVSLSPWL--------- 267
AT5G47370                            -MTPPTTLIMCPSCER---VGGPSSSNHHHNHRPVSTNPWVA-------- 265
AT2G44910                            -MTPPTTLTMCPSCER---VSSSAATVTAAPSTTTTPTVVGR-------P 299
AT3G60390                            -MKPPTTLTMCPSCER---VAVTSSSSSVAPPVMNSSSPMG--------- 296
THOM1"                               -MSPPTTLTMCPQCER---VAVSSSSSSSSVVNATRAQNHQA-------P 263
craterostigmaCPHB-3                  -MSPPTTLTMCPQCER---VAAQPSSAAAIRPPSHHQRPAAG-------- 303
TaHDZipII-1"homeodomain-leucin       ---PLTTLTMCLSCKR---VASTSS-----ASACDVPN------FSTNAG 237
Hox2                                 -SSAAATLTMCPSCRR---VATAGAPHQPNHQQCHPKSNTTI-SSSSTAA 263
ricehox1homeodomain-leucine          -VPPPTTLTMCPSCER---VASAAT---------TTRN-------NSGAA 291
Oshox1"homeodomain                   -VPPPTTLTMCPSCER---VASAAT---------TTRN-------NSGAA 291
OSJNBb0089A17.12"homeodomain         -VPPPTTLTMCPSCER---VASAAT---------TTRN-------NSGAA 291
AT5G06710                            MQLPATTLTMCPSCER---VATSA---------AQPSTSAAH-------- 315
Pphb4"homeodomain-leucine            MPLPAATLTMCPSCER---VATVD---------NRSLTFAKP-------- 148
Mshb1"type                           MQLPATTLTMCPSCER---VATNS---------TATSSVTNT-------- 326
AT4G37790                            MHMPAATLTMCPSCER---LGGGG---------VGGDTTAVD-------- 251
AT2G22800                            MHMPASTLTKCPSCER---IGGGGGGNGGGGGSGATAVTVD-------- 247
OJ1781E12.8"Hypothetical             MQLPAATLTICPSCER---VGGPA---------SAAKVVAAD-------- 268
OSJNOa174H12.5"putative              MHLPATTLSMCPSCER---VASNPATASTSAPAAATSPAAAP-------- 308
RiceHox7"AAQ55491.1"                 -AAAGTVFRVCPSCEHSKVVAAAASESFSPRVFAGGGAPAAITAAAAVPS 251
Oshox7"homeodomain                   -AAAGTVFRVCPSCEHSKVVAAAASESFSPRVFAGGGAPAAITAAAAVPS 225
P0020C11.33"putative                 -AAAGTVFRVCPSCEHSKVVAAAASESFSPRVFAGGGAPAAITAAAAVPS 223
AT2G01430                            TVNSASSLTMCPRCER---VTPAAS------PSRAVVPVPAK-------- 265
AT1G70920                            --SSTSALTMCPRCER---VTDAVDNDSNAVQEGAVLSSRSR-------- 196
riceP0510C12.22"putative             QPLPASALTMCPRCER---ITAATGPPAVRPPPSSAAAAAPS-------- 305
                                        . : :  *  *.:   :
```

FIGURE 2 (continued)

| | | |
|---|---|---|
| glycina | QALRCPPSQILDSRKQNDKIEKKDRKP-------------------- | 284 |
| homeobox-leucine | QALRCPPSQILDSRKQNDKIEKKDRKP-------------------- | 171 |
| AT4G16780 | PATRISHGLTIFDALRPRS---------------------------- | 284 |
| AT4G17460 | ---QMAHGSTFDVMRPRS----------------------------- | 282 |
| AT5G47370 | CAGQVAHGLNFEALRPRS----------------------------- | 283 |
| AT2G44910 | SPQRLTPWTAISLQQKSGR---------------------------- | 318 |
| AT3G60390 | ---PMSPWAAMPLRQRPAAGSH------------------------- | 315 |
| THOM1" | VP-MNKPWAAMFASKTLDVQRSQM----------------------- | 286 |
| craterostigmaCPHB-3 | ----MNSWAAMISPRPS------------------------------ | 316 |
| TaHDZipII-1"homeodomain-leucin | MGMPMPSPVALPDHRQFFCGYRDTG--ATYGGFSGLAKVVKPAR--- | 279 |
| Hox2 | AAVAVAGGNVLPSHCQFFPAAAAAADRTSQSTWNAAAPLVTRELF-- | 308 |
| ricehox1homeodomain-leucine | PARPVPTRPWPPAAAQRSSA--------------------------- | 311 |
| Oshox1"homeodomain | PARPVPTRPWPPAAAQRSSA--------------------------- | 311 |
| OSJNBb0089A17.12"homeodomain | PARPVPTRPWPPAAAQRSSA--------------------------- | 311 |
| AT5G06710 | -NLCLSTSSLIPVKPR-------PAKQVS------------------ | 336 |
| Pphb4"homeodomain-leucine | -GFSHLSQSSAAC---------------------------------- | 160 |
| Mshb1"type | -SATINGNNNNKINQ-------------------------------- | 340 |
| AT4G37790 | -EETAKGAFSTVTKPR-------FYNPFTNPSAAC------------ | 278 |
| AT2G22800 | -GSTAKGAFSISSKPH-------FFNPFTNPSAAC------------ | 274 |
| OJ1781E12.8"Hypothetical | -GTKAG--PGRTTTHH-------FFNPFTH-SAAC------------ | 292 |
| OSJNOa174H12.5"putative | -TAAARTAVASPEPHRPSSFAALFAAPLGFPLTAAQPRPPPPASNCL | 354 |
| RiceHox7"AAQ55491.1" | PGAGSPPSSSAALFGARRPHFGPFAAAVIPPVLRRQPSATS------ | 292 |
| Oshox7"homeodomain | PGAGSPPSSSAALFGARRPHFGPFAAAVIPPVLRRQPSATS------ | 266 |
| P0020C11.33"putative | PGAGSPPSSSAALFGARRPHFGPFAAAVIPPVLRRQPSATS------ | 264 |
| AT2G01430 | --KTFPPQERDR----------------------------------- | 275 |
| AT1G70920 | --MTISSSSSLC----------------------------------- | 206 |
| riceP0510C12.22"putative | --PFHPRRPSAAF---------------------------------- | 316 |

FIGURE 2 (continued)

FIGURE 3

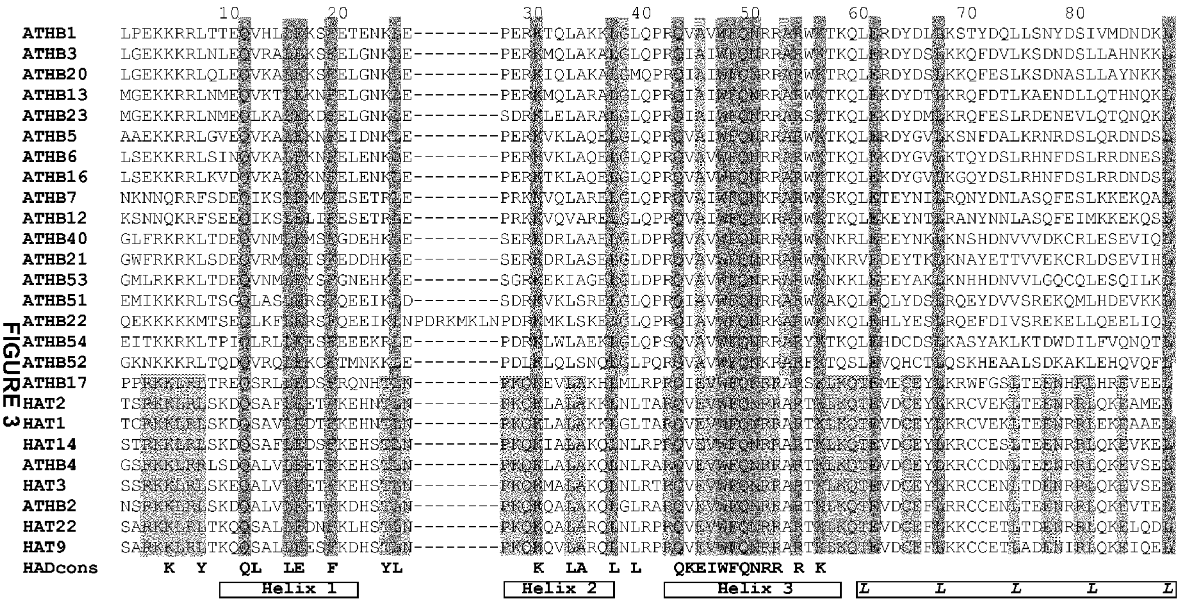


**Figure 1.** Alignment of the amino acid sequences of HDZip class I and II proteins. Gaps are indicated by dashes. Conserved positions in a HD consensus sequence (Bürglin, 1994) are indicated below the alignment. Residues conserved in both classes are depicted in dark gray, residues conserved only within HDZip I or II are depicted in light gray, and residues showing variation between and within the classes are depicted in white. The three α-helices of the HD and the Leu residues of the Leu Zip are shown below the alignment.

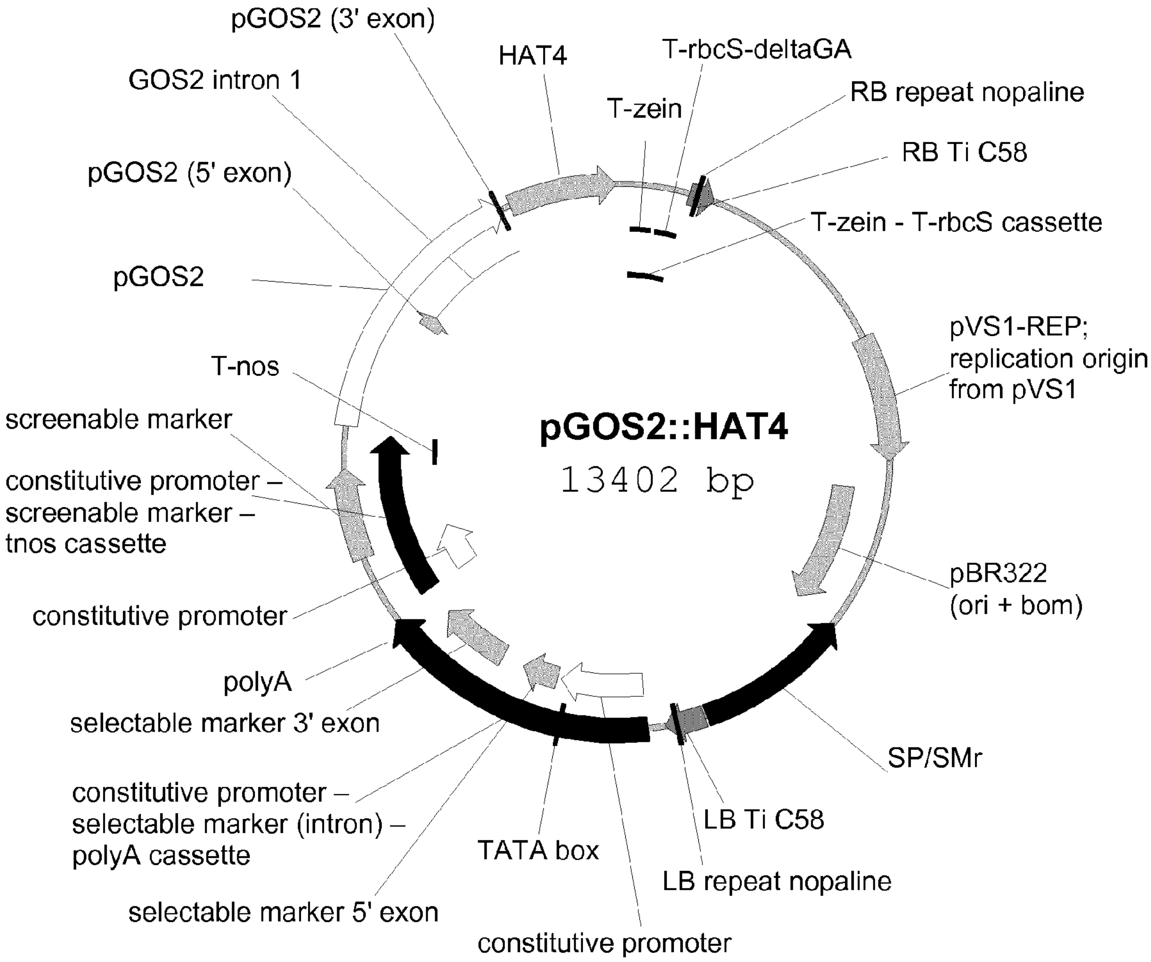
pGOS2 (3' exon)
HAT4
T-rbcS-deltaGA
GOS2 intron 1
T-zein
RB repeat nopaline
RB Ti C58
pGOS2 (5' exon)
T-zein - T-rbcS cassette
pGOS2
pVS1-REP;
replication origin
from pVS1
T-nos
screenable marker
pGOS2::HAT4
13402 bp
constitutive promoter –
screenable marker –
tnos cassette
pBR322
(ori + bom)
constitutive promoter
polyA
selectable marker 3' exon
SP/SMr
constitutive promoter –
selectable marker (intron) –
polyA cassette
LB Ti C58
TATA box
LB repeat nopaline
selectable marker 5' exon
constitutive promoter

FIGURE 4

Europäisches Patentamt
European Patent Office
Office européen des brevets

# EUROPEAN SEARCH REPORT

Application Number
EP 12 18 5025

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/076638 A2 (MENDEL BIOTECHNOLOGY INC [US]; SHERMAN BRADLEY K [US]; RIECHMANN JOSE) 10 September 2004 (2004-09-10) * page 220, line 11 - page 221, line 14 * -& DATABASE NUCLEOTIDE [Online] 5 April 2000 (2000-04-05), "Arabidopsis thaliana chromosome II section 3 of 255 of the complete sequence. Sequence from clones F10A8, F2I9", XP002687969, retrieved from NCBI Database accession no. AC005560 * gene="At2g01430" * ----- | 1,2,4,5, 7-11, 13-20 | INV. C07K14/415 C12N15/82 |
| X | SCHENA M ET AL: "THE HAT4 GENE OF ARABIDOPSIS ENCODES A DEVELOPMENTAL REGULATOR", GENES AND DEVELOPMENT, vol. 7, no. 3, 1993, pages 367-379, XP009033912, COLD SPRING HARBOR LABORATORY PRESS, PLAINVIEW, NY, US ISSN: 0890-9369 * the whole document * ----- | 9,10, 14-19 | |
| | -/-- | | TECHNICAL FIELDS SEARCHED (IPC) C07K C12N |

The present search report has been drawn up for all claims

| Place of search | Date of completion of the search | Examiner |
|---|---|---|
| Munich | 26 November 2012 | Bilang, Jürg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 12 18 5025

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MEIJER A H ET AL: "Transcriptional repression by Oshox1, a novel homeodomain leucine zipper protein from rice", THE PLANT JOURNAL, vol. 11, no. 2, 1 February 1997 (1997-02-01), pages 263-276, XP002130665, BLACKWELL SCIENTIFIC PUBLICATIONS, OXFORD, GB ISSN: 0960-7412, DOI: 10.1046/J.1365-313X.1997.11020263.X * the whole document * ----- | 9,10, 14-19 | |
| A | SCHENA M ET AL: "HD-ZIP PROTEINS: MEMBERS OF AN ARABIDOPSIS HOMEODOMAIN PROTEIN SUPERFAMILY", PROCEEDINGS OF THE NATIONAL ACADEMY OF SCIENCES OF USA,, vol. 89, May 1992 (1992-05), pages 3894-3898, XP002931606, NATIONAL ACADEMY OF SCIENCE, WASHINGTON, DC, US ISSN: 0027-8424 * the whole document * ----- | 1-21 | TECHNICAL FIELDS SEARCHED (IPC) |
| A | US 2005/278805 A1 (SHEN BO [US] ET AL) 15 December 2005 (2005-12-15) * page 1 - page 2 * ----- | 1-21 | |
| | -/-- | | |

The present search report has been drawn up for all claims

| Place of search | Date of completion of the search | Examiner |
|---|---|---|
| Munich | 26 November 2012 | Bilang, Jürg |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 18 5025

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HANSON JOHANNES ET AL: "Sugar-dependent alterations in cotyledon and leaf development in transgenic plants expressing the HDZhdip gene ATHB13", PLANT MOLECULAR BIOLOGY, vol. 45, no. 3, 1 February 2001 (2001-02-01), pages 247-262, XP002597972, SPRINGER, DORDRECHT, NL ISSN: 0167-4412, DOI: 10.1023/A:1006464907710 * the whole document * ----- | 1-21 | |
| A | MASAKI TAKESHI ET AL: "ACTIVATOR of Spo(min):: LUC1/WRINKLED1 of a Arabidopsis thaliana transactivates sugar-inducible promoters", PLANT AND CELL PHYSIOLOGY, vol. 46, no. 4, April 2005 (2005-04), pages 547-556, XP009088128, ISSN: 0032-0781 * the whole document * ----- | 1-21 | TECHNICAL FIELDS SEARCHED (IPC) |
| A | BO SHEN ET AL: "The Homeobox Gene GLABRA2 Affects Seed Oil Content in Arabidopsis", PLANT MOLECULAR BIOLOGY, vol. 60, no. 3, 1 February 2006 (2006-02-01), pages 377-387, XP019262836, KLUWER ACADEMIC PUBLISHERS, DO ISSN: 1573-5028 * page 386 * ----- -/-- | 1-21 | |

The present search report has been drawn up for all claims

| Place of search | Date of completion of the search | Examiner |
|---|---|---|
| Munich | 26 November 2012 | Bilang, Jürg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 12 18 5025

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SESSA G ET AL: "DNA-binding specificity of the homeodomain-leucine zipper domain", JOURNAL OF MOLECULAR BIOLOGY, vol. 274, no. 3, 5 December 1997 (1997-12-05), pages 303-309, XP004451564, LONDON, GB ISSN: 0022-2836 * the whole document * ----- | 1-21 | |
| A,D | TOEPFER R ET AL: "MODIFICATION OF PLANT LIPID SYNTHESIS", SCIENCE, AMERICAN ASSOCIATION FOR THE ADVANCEMENT OF SCIENCE,, US, vol. 268, 5 May 1995 (1995-05-05), pages 681-685, XP002014017, ISSN: 0036-8075 * the whole document * ----- | 1-21 | |
| A,D | SAKAKIBARA KEIKO ET AL: "Isolation of homeodomain-leucine zipper genes from the moss Physcomitrella patens and the evolution of homeodomain-leucine zipper genes in land plants", MOLECULAR BIOLOGY AND EVOLUTION, vol. 18, no. 4, April 2001 (2001-04), pages 491-502, XP002334991, THE UNIVERSITY OF CHICAGO PRESS, US ISSN: 0737-4038 * the whole document * ----- | 1-21 | TECHNICAL FIELDS SEARCHED (IPC) |
| A,D | MORELLI GIORGIO ET AL: "Light and shade in the photocontrol of Arabidopsis growth", TRENDS IN PLANT SCIENCE, vol. 7, no. 9, September 2002 (2002-09), pages 399-404, XP009088165, ISSN: 1360-1385 * the whole document * ----- | 1-21 | |

The present search report has been drawn up for all claims

| Place of search | Date of completion of the search | Examiner |
|---|---|---|
| Munich | 26 November 2012 | Bilang, Jürg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 12 18 5025

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2012

| Patent document cited in search report | Publication date | Patent family member(s) | Publication date |
|---|---|---|---|
| WO 2004076638 A2 | 10-09-2004 | AU 2004214935 A1 | 10-09-2004 |
| | | BR PI0407822 A | 14-02-2006 |
| | | CA 2516645 A1 | 10-09-2004 |
| | | EP 1601758 A2 | 07-12-2005 |
| | | US 2005086718 A1 | 21-04-2005 |
| | | US 2006242738 A1 | 26-10-2006 |
| | | WO 2004076638 A2 | 10-09-2004 |
| US 2005278805 A1 | 15-12-2005 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5955650 A
- US 6084164 A
- US 5811238 A
- US 6395547 A
- WO 2004070039 A
- WO 2004065596 A
- US 4962028 A
- WO 0114572 A
- WO 9514098 A
- WO 9412015 A
- EP 99106056 A
- US 5565350 A, Kmiec
- WO 9322443 A, Zarling
- WO 9853083 A, Grierson
- WO 9953050 A, Waterhouse
- US 4987071 A, Cech
- US 5116742 A, Cech
- WO 9400012 A, Atkins
- WO 9503404 A, Lenne
- WO 0000619 A, Lutziger
- WO 9713865 A, Prinsen
- WO 9738116 A, Scott
- WO 9836083 A
- WO 9915682 A
- WO 0015815 A
- EP 1198985 A1
- US 5164310 A


### Non-patent literature cited in the description


- **WANG et al.** *Planta,* 2003, vol. 218, 1-14
- **TÖPFER et al.** *Science,* 1995, vol. 268, 681-686
- **CAHOON et al.** *Proc. Natl. Acad. Sci. USA,* 1992, vol. 89, 11184-11188
- **VAN DE LOO F.J. et al.** Unusual Fatty Acids in Lipid Metabolism in Plants. CRC Press, 1993, 91-126
- **MILLAR et al.** *Trends Plant Sci.,* 2000, vol. 5, 95-101
- **BROWSE et al.** *Biochemical J.,* 1986, vol. 235, 25-31
- **OHLROGGE ; BROWSE.** *Plant Cell,* May 1995, vol. 7, 957-970
- **VOELKER.** Genetic Engineering. 1996, vol. 18, 111-113
- **SHANKLIN ; CAHOON.** *Annu. Rev. Plant Physiol. Plant Mol. Biol.,* 1998, vol. 49, 611-641
- **FRENTZEN.** *Lipids,* 1998, vol. 100, 161-166
- **PLAXTON.** *Annu. Rev. Plant Physiol. Plant Mol. Biol.,* 1996, vol. 47, 185-214
- **KANG ; RAWSTHORNE.** *Plant J.,* 1994, vol. 6, 795-805
- **OHLROGGE ; BROWSE.** *Plant Cell,* 1995, vol. 7, 957-970
- **VAN DE LOO et al.** *Proc. Natl. Acad. Sci USA,* 1995, vol. 92, 6743-6747
- **CAHOON et al.** *Proc. Natl. Acad. Sci USA,* 1992, vol. 89, 11184-11188
- **BRENNER.** *Adv. Exp. Med. Biol.,* 1976, vol. 83, 85-101
- **RIECHMANN et al.** *Science,* 2000, vol. 290, 2105-2109
- **SAKAKIBARA et al.** *Mol. Biol. Evol,* 2001, vol. 18 (4), 491-502
- **ASO et al.** *Mol. Biol. Evol.,* 1999, vol. 16 (4), 544-552
- **SAKAKIBARA et al.** *Mol Biol Evol,* 2001, vol. 18 (4), 491-502
- **SESSA et al.** *EMBO J,* 1993, vol. 12 (9), 3507-3517
- **SAKAKIBARA et al.** *Mol. Biol. Evol.,* 2001, vol. 18 (4), 491-502
- **HENRIKSSON et al.** *Plant Phys,* 2005, vol. 139, 509-518
- **MEIJER et al.** *Mol Gen Genet,* 2000, vol. 263, 12-21
- **MEIJER et al.** *Plant J,* 1997, vol. 11, 263-276
- **MORELLI ; RUBERTI.** *TIPS,* September 2002, vol. 7 (9
- **CREIGHTON.** Proteins. W.H. Freeman and Company, 1984
- **TERPE.** *Appl. Microbiol. Biotechnol.,* 2003, vol. 60, 523-533
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- Current Protocols in Molecular Biology. John Wiley & Sons, 1989

- **FOISSAC ; SCHIEX.** *BMC Bioinformatics,* 2005, vol. 6, 25
- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1999, vol. 39 (6), 1696
- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- **QING QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* 1987, vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* 1991, vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **TAKAIWA et al.** *Mol Gen Genet,* 1986, vol. 208, 15-22
- **COLOT et al.** *Mol Gen Genet,* 1989, vol. 216, 81-90
- **ANDERSON et al.** *NAR,* 1989, vol. 17, 461-2
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62
- **ONATE et al.** *J Biol Chem,* 1999, vol. 274 (14), 9175-82
- **VICENTE-CARBAJOSA et al.** *Plant J,* 1998, vol. 13, 629-640
- **WU et al.** *Plant Cell Physiol,* 1998, vol. 39 (8), 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46
- **DEROSE et al.** *Plant Mol Biol,* 1996, vol. 32, 1029-35

- **BUCHMAN ; BERG.** *Mol. Cell biol.,* 1988, vol. 8, 4395-4405
- **CALLIS et al.** *Genes Dev,* 1987, vol. 1, 1183-1200
- The Maize Handbook. Springer, 1994
- **GAULTIER et al.** *Nucl Ac Res,* 1987, vol. 15, 6625-6641
- **INOUE et al.** *Nucl Ac Res,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **ANGELL ; BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.J.** *Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **TRIBBLE et al.** *J. Biol. Chem.,* 2000, vol. 275, 22255-22267
- **VELMURUGAN et al.** *J. Cell Biol.,* 2000, vol. 149, 553-566
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **LSHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer. **B. JENES et al.** Transgenic Plants, Vol. 1, Engineering and Utilization. Academic Press, 1993, vol. 1, 128-143
- **POTRYKUS.** *Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- Vectors for Gene Transfer in Higher Plants. **F.F. WHITE.** Transgenic Plants, Vol. 1, Engineering and Utilization. Academic Press, 1993, vol. 1, 15-38
- **FELDMAN, KA ; MARKS MD.** *Mol Gen Genet,* 1987, vol. 208, 274-289
- **FELDMANN K.** Methods in Arabidopsis Research. Word Scientific, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol,* 2003, vol. 21, 20-28
- **HAYASHI et al.** *Science,* 1992, 1350-1353
- **REDEI GP ; KONCZ C.** Methods in Arabidopsis Research. World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **LIDA ; TERADA.** *Curr Opin Biotech,* 2004, vol. 15 (2), 132-8
- **NEEDLEMAN ; WUNSCH.** *J Mol Biol,* 1970, vol. 48, 443-453
- **ALTSCHUL et al.** *J Mol Biol,* 1990, vol. 215, 403-410
- **CAMPANELLA et al.** *BMC Bioinformatics,* 2003, vol. 4, 29
- **SCHULTZ et al.** *Proc. Natl. Acad. Sci. USA,* 1998, vol. 95, 5857-5864
- **LETUNIC et al.** *Nucleic Acids Res,* 2002, vol. 30, 242-244
- **MULDER et al.** *Nucl. Acids. Res.,* 2003, vol. 31, 315-318

- A generalized profile syntax for biomolecular sequences motifs and its function in automatic sequence interpretation. **BAIROCH.** Proceedings 2nd International Conference on Intelligent Systems for Molecular Biology. AAAIPress, 1994, 53-61
- **HULO et al.** *Nucl. Acids. Res.,* 2004, vol. 32, D134-D137
- **BATEMAN et al.** *NucleicAcids Research,* 2002, vol. 30 (1), 276-280
- Current Protocols in Molecular Biology. Wiley
- **SESSA et al.** *J Mol Biol,* 1997, vol. 274 (3), 303-309
- **ISHIDA et al.** *Nat. Biotechnol,* 1996, vol. 14 (6), 745-50
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BERNATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet.,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet,* 1997, vol. 7, 22-28
- **DEAR ; COOK.** *Nucleic Acid Res.,* 1989, vol. 17, 6795-6807
- **HENRIKSON et al.** *Plant Physiol,* 2005, vol. 139, 509-518
- **SAMBROOK.** Molecular Cloning: a laboratory manual. Cold Spring Harbor Laboratory Press, 2001
- **AUSUBEL et al.** Current Protocols in Molecular Biology. Current Protocols, 1994, vol. 1, 2
- **R.D.D. CROY.** Plant Molecular Biology Labfax. BIOS Scientific Publications Ltd (UK, 1993
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659

SEQUENCE LISTING

<110> CropDesign N.V.

<120> Plants having increased yield and method for making the same

<130> PF58401-prio

<160> 347

<170> PatentIn version 3.3

<210> 1
<211> 2151
<212> DNA
<213> Arabidopsis thaliana

<400> 1
atgtgctgtg gatcagaccg attaaaccag atcgtgtcat caagatcttc gttgccaatt 60

tctttcgagg aagataacaa tcttgttacc aacacagaca tgaatcactt aacagtcgaa 120

acagaggata cgtttgcgag cttgcttgag cttgcagcta acaacgatgt tgaaggtgta 180

aggctatcta tcgagagaga cccttcttgt gtagacgaag ctggtctctg gtacggtcgt 240

caaaaaggtt ctaaagctat ggtcaacgat tacaggactc cgttgatggt tgctgctact 300

tacggaagca ttgatgtgat caagcttatt gtttctttga ctgatgctga cgtgaaccgt 360

gcttgcggga atgatcagac cactgcgtta cactgcgctg cttctggagg agctgtgaat 420

gctatccaag ttgttaagct gcttcttgca gctggagctg atttgaatct gttggatgct 480

gaaggtcaac gagctggtga tgttattgtt gttcctccta agcttgaagg cgtgaagctg 540

atgcttcagg agcttctttc tgctgatgga tcatctactg cggagcggaa tctacgggtt 600

gtgacaaatg ttccgaatag aagctcatct ccgtgtcatt ctcctactgg agagaatggt 660

ggatcagggt ctggttcacc gctcggctct ccttttaagc tgaaatctac tgaattcaag 720

aaagagtatc cggttgatcc gtctttgcca gatatcaaga acagtatcta cgcgactgat 780

gagtttagaa tgtattcctt caaggtccgg ccttgctctc gtgcttattc acatgattgg 840

actgagtgtc cttttgttca cccgggtgaa aacgcgagga ggagagaccc gaggaagttc 900

cattacagct gcgttccttg cccggatttt aggaaaggag cttgtaggag aggagatatg 960

tgtgagtatg cgcacggtgt gtttgaatgc tggcttcatc cggctcagta caggacccgt 1020

ctttgcaaag atggaacagg ctgtgctcgg cgggtttgtt tctttgcgca tacacccgag 1080

gagcttcgac ctttgtacgc atcaactggt tcagcggttc cttcgcctag atcgaatgct 1140

gattatgcag ctgctttgag tctccttcct ggttctccat caggagtctc tgtcatgtcc 1200

ccgctttccc catcagcagc ggggaacgga atgtctcatt cgaatatggc ttggccacaa 1260

ccaaatgtcc ctgcgttgca cttaccagga agcaatctac agtcaagcag gctaaggtct 1320

tctctcaatg caagggatat cccgacggat gagttcaata tgttagcgga ttacgagcag 1380

cagcaactcc tcaacgagta ttccaatgct ctgagccgtt ctggtcggat gaaatcaatg 1440

```
cctccttcga atcttgaaga tcttttctca gcagaaggct cttcatctcc ccggttcact   1500

gattccgctt tagcttccgc ggtgttctcg cctacacaca agtcagctgt cttcaaccag   1560

ttccaacaac agcaacagca gcagcagagc atgttgtctc caatcaacac aagcttttct   1620

tcaccaaaga gcgttgacca ctcattgttt tcaggtggag gaagaatgtc tcctcggaat   1680

gttgttgaac caatatcacc catgagtgct cgggtttcca tgttggctca gtgcgtgaag   1740

caacaacaac agcaacagca gcagcagcag cagcaacatc agttccgtag ccttagctcc   1800

agagagctca gaacaaactc tagcccaatc gttggttcac cggtaaacaa caacacatgg   1860

tcatcaaaat ggggatcttc aaatggtcaa ccggattggg gaatgagctc agaagcactt   1920

ggtaagttga gatcttcgtc atcgtttgat ggtgatgagc ctgatgtgtc atgggtccag   1980

tcactggtga aggagactcc agcagaagcc aaagagaaag cagcaacatc ttcctcaggg   2040

gaacacgtga tgaagcagcc aaatccggtt gaaccggtaa tggatcatgc tgggctagaa   2100

gcttggattg agcaaatgca gctcgatcag cttgtggctc agcagaattg a            2151


<210>  2
<211>  716
<212>  PRT
<213>  Arabidopsis thaliana

<400>  2

Met Cys Cys Gly Ser Asp Arg Leu Asn Gln Ile Val Ser Ser Arg Ser
1               5                   10                  15


Ser Leu Pro Ile Ser Phe Glu Glu Asp Asn Asn Leu Val Thr Asn Thr
            20                  25                  30


Asp Met Asn His Leu Thr Val Glu Thr Glu Asp Thr Phe Ala Ser Leu
        35                  40                  45


Leu Glu Leu Ala Ala Asn Asn Asp Val Glu Gly Val Arg Leu Ser Ile
    50                  55                  60


Glu Arg Asp Pro Ser Cys Val Asp Glu Ala Gly Leu Trp Tyr Gly Arg
65                  70                  75                  80


Gln Lys Gly Ser Lys Ala Met Val Asn Asp Tyr Arg Thr Pro Leu Met
                85                  90                  95


Val Ala Ala Thr Tyr Gly Ser Ile Asp Val Ile Lys Leu Ile Val Ser
            100                 105                 110


Leu Thr Asp Ala Asp Val Asn Arg Ala Cys Gly Asn Asp Gln Thr Thr
        115                 120                 125
```

Ala Leu His Cys Ala Ala Ser Gly Gly Ala Val Asn Ala Ile Gln Val
    130                 135                 140

Val Lys Leu Leu Leu Ala Ala Gly Ala Asp Leu Asn Leu Leu Asp Ala
145                 150                 155                 160

Glu Gly Gln Arg Ala Gly Asp Val Ile Val Val Pro Pro Lys Leu Glu
                165                 170                 175

Gly Val Lys Leu Met Leu Gln Glu Leu Leu Ser Ala Asp Gly Ser Ser
            180                 185                 190

Thr Ala Glu Arg Asn Leu Arg Val Val Thr Asn Val Pro Asn Arg Ser
        195                 200                 205

Ser Ser Pro Cys His Ser Pro Thr Gly Glu Asn Gly Gly Ser Gly Ser
    210                 215                 220

Gly Ser Pro Leu Gly Ser Pro Phe Lys Leu Lys Ser Thr Glu Phe Lys
225                 230                 235                 240

Lys Glu Tyr Pro Val Asp Pro Ser Leu Pro Asp Ile Lys Asn Ser Ile
                245                 250                 255

Tyr Ala Thr Asp Glu Phe Arg Met Tyr Ser Phe Lys Val Arg Pro Cys
            260                 265                 270

Ser Arg Ala Tyr Ser His Asp Trp Thr Glu Cys Pro Phe Val His Pro
        275                 280                 285

Gly Glu Asn Ala Arg Arg Arg Asp Pro Arg Lys Phe His Tyr Ser Cys
    290                 295                 300

Val Pro Cys Pro Asp Phe Arg Lys Gly Ala Cys Arg Arg Gly Asp Met
305                 310                 315                 320

Cys Glu Tyr Ala His Gly Val Phe Glu Cys Trp Leu His Pro Ala Gln
                325                 330                 335

Tyr Arg Thr Arg Leu Cys Lys Asp Gly Thr Gly Cys Ala Arg Arg Val
            340                 345                 350

Cys Phe Phe Ala His Thr Pro Glu Glu Leu Arg Pro Leu Tyr Ala Ser
        355                 360                 365

Thr Gly Ser Ala Val Pro Ser Pro Arg Ser Asn Ala Asp Tyr Ala Ala
    370                 375                 380

Ala Leu Ser Leu Leu Pro Gly Ser Pro Ser Gly Val Ser Val Met Ser

```
385                 390                 395                 400

Pro Leu Ser Pro Ser Ala Ala Gly Asn Gly Met Ser His Ser Asn Met
                405                 410                 415

Ala Trp Pro Gln Pro Asn Val Pro Ala Leu His Leu Pro Gly Ser Asn
            420                 425                 430

Leu Gln Ser Ser Arg Leu Arg Ser Ser Leu Asn Ala Arg Asp Ile Pro
        435                 440                 445

Thr Asp Glu Phe Asn Met Leu Ala Asp Tyr Glu Gln Gln Gln Leu Leu
    450                 455                 460

Asn Glu Tyr Ser Asn Ala Leu Ser Arg Ser Gly Arg Met Lys Ser Met
465                 470                 475                 480

Pro Pro Ser Asn Leu Glu Asp Leu Phe Ser Ala Glu Gly Ser Ser Ser
                485                 490                 495

Pro Arg Phe Thr Asp Ser Ala Leu Ala Ser Ala Val Phe Ser Pro Thr
            500                 505                 510

His Lys Ser Ala Val Phe Asn Gln Phe Gln Gln Gln Gln Gln Gln Gln
        515                 520                 525

Gln Ser Met Leu Ser Pro Ile Asn Thr Ser Phe Ser Ser Pro Lys Ser
    530                 535                 540

Val Asp His Ser Leu Phe Ser Gly Gly Gly Arg Met Ser Pro Arg Asn
545                 550                 555                 560

Val Val Glu Pro Ile Ser Pro Met Ser Ala Arg Val Ser Met Leu Ala
                565                 570                 575

Gln Cys Val Lys Gln Gln Gln Gln Gln Gln Gln Gln Gln Gln Gln Gln
            580                 585                 590

His Gln Phe Arg Ser Leu Ser Ser Arg Glu Leu Arg Thr Asn Ser Ser
        595                 600                 605

Pro Ile Val Gly Ser Pro Val Asn Asn Asn Thr Trp Ser Ser Lys Trp
    610                 615                 620

Gly Ser Ser Asn Gly Gln Pro Asp Trp Gly Met Ser Ser Glu Ala Leu
625                 630                 635                 640

Gly Lys Leu Arg Ser Ser Ser Ser Phe Asp Gly Asp Glu Pro Asp Val
                645                 650                 655
```

```
Ser Trp Val Gln Ser Leu Val Lys Glu Thr Pro Ala Glu Ala Lys Glu
            660                 665                 670

Lys Ala Ala Thr Ser Ser Ser Gly Glu His Val Met Lys Gln Pro Asn
        675                 680                 685

Pro Val Glu Pro Val Met Asp His Ala Gly Leu Glu Ala Trp Ile Glu
    690                 695                 700

Gln Met Gln Leu Asp Gln Leu Val Ala Gln Gln Asn
705                 710                 715
```

<210> 3
<211> 13
<212> PRT
<213> Artificial sequence

<220>
<223> motif 1

<220>
<221> VARIANT
<222> (1)..(1)
<223> \replace = "Ala"

<220>
<221> VARIANT
<222> (7)..(7)
<223> \replace = "Thr"

<400> 3

```
Pro Cys Ser Arg Ala Tyr Ser His Asp Trp Thr Glu Cys
1               5                   10
```

<210> 4
<211> 12
<212> PRT
<213> Artificial sequence

<220>
<223> motif 2

<400> 4

```
His Pro Gly Glu Asn Ala Arg Arg Arg Asp Pro Arg
1               5                   10
```

<210> 5
<211> 19
<212> PRT
<213> Artificial sequence

<220>
<223> motif 3

```
<220>
<221>  VARIANT
<222>  (3)..(3)
<223>  \replace = "Ile"

<220>
<221>  VARIANT
<222>  (6)..(6)
<223>  \replace = "Ser"

<220>
<221>  VARIANT
<222>  (11)..(11)
<223>  \replace = "Ser"

<220>
<221>  VARIANT
<222>  (14)..(14)
<223>  \replace = "Lys"

<400>  5

His Gly Val Phe Glu Cys Trp Leu His Pro Ala Gln Tyr Arg Thr Arg
1               5                   10                  15


Leu Cys Lys



<210>  6
<211>  5
<212>  PRT
<213>  Artificial sequence

<220>
<223>  motif 4

<400>  6

Cys Phe Phe Ala His
1               5


<210>  7
<211>  54
<212>  DNA
<213>  Artificial sequence

<220>
<223>  primer: prm06717

<400>  7
ggggacaagt ttgtacaaaa aagcaggctt aaacaatgtg ctgtggatca gacc           54


<210>  8
<211>  50
<212>  DNA
<213>  Artificial sequence

<220>
<223>  primer: prm06718
```

<400> 8
ggggaccact ttgtacaaga aagctgggtg gttaggtctc tcaattctgc 50

<210> 9
<211> 981
<212> DNA
<213> Oryza sativa

<400> 9
aagaggcaag agcatccgta ttaaccagcc ttttgagact tgagagtgtg tgtgactcga 60

tccagcgtag tttcagttcg tgtgttggtg agtgattcca gccaagtttg cgatggcttc 120

tcagcaggaa cgggctagct accacgccgg cgagaccaag gcccgcgccg aggagaagac 180

ggggcgcatg atgggcacgg cgcaggagaa ggcgcgggag gccaaggaca cggcgtccga 240

cgccgcgggg cgcgcgatgg gcaggggaca cggcgccaag gaggcgacca aggagaaggc 300

gtacgagacc aaggacgcga ccaaggagaa ggcgtacgag gcaaaggacg cggcctccga 360

cgccaccggc cgcgccatgg acaagggccg cggcgccgcg ggcgccacga gggacaaggc 420

gtacgatgcc aaggacaggg cggctgacac ggcgcagtcc gccgccgacc gcgcccgcga 480

cggcgccggg cagaccggga gctacattgg acagaccgcc gaggccgcca agcagaaagc 540

ggccggcgcc gcgcagtacg ccaaggagac cgcgatcgcc ggcaaggaca agaccggcgc 600

cgtgctccag caggcagggg agcaggtgaa gagcgtggcg gtgggggcga aggacgcggt 660

gatgtacacg ctcgggatgt caggcgataa caagaacaac gccgctgccg gcaaggacac 720

cagcacctac aagcctggaa ctgggagtga ctaccagtaa tacggtagaa gaagcatgtg 780

tcgtctttgg cactgatgcc aaagtgtacg tgttgtatcc tcttttttaa gtttcagctc 840

gacttcgacg tgttcggtgt cacactttgg tttttcagtt gtgctcaact gttcatgttt 900

ctggttccat ggagggccag tgtggaggtc aatgtttaag ctttcgtttt aaaatctgat 960

aataaagttg gttaagacct g 981

<210> 10
<211> 3372
<212> DNA
<213> Eucalyptus grandis

<400> 10
ggaagacgaa gagcaacaaa ataggtctca ctccctcctc tctcctctct cctctctctt 60

ctttctctct cttctgcttc taacaaagtc tcttccttga gagacagggc tgcgtcgtcg 120

tctttctctc tcctcgctgc gagcttctga gaaagttcaa ttctttttct cttgttctct 180

ctctctaccc ttctgggtac cactgtgaag ctccggtctt ttctattttt tttttttttg 240

ggctatctgg gtctgggcaa atccatcgcg cgctctgctc tggactgaga ggccgtcagt 300

ggctttagat ctgcgacgcc tcttgcttgc tcagtgagct gggctagttc aaatcgacga 360

agaaagcatg cgctagtgat tggtgtgggt aatacactgc attcgatctc tactaagtat 420

ccccaagtat actaagatcc cgttctcagc catgagtcaa ctgaccattc agactgagga 480
cacttttgcc agcttgcttg agcttgctgc taacaacgac acagaatctt tcggacggtg 540
tgtggaacgt gatccttcga gcatagatga aattggatat tggtatggtc gccaaaaggg 600
ttcgaagcag gtggtcaata tgcaaagaac tcctcttatg gtggctgcta catatggtag 660
tgttgatgta atgagactca ttctttgcct atctgatgct gatgtgaatc gaacctgcag 720
cacagacaag agcacagccc ttcactgtgc tgcctctggt ggtgctgtga atgctgtaga 780
tgctgtgagg ctactcctgt cagctggtgc tgacccaagt ttagcagatg ctaacggtca 840
gcggcctgtg gatgttattg ttgttcctcc aaagctcctt tcaataaagt ttgctcttga 900
agagctcttg tcgaccgaag gatctgtaaa tgaacacaat ctgagagtgt ccgtagccac 960
ttccaattca acctctcccc cactttcatc ttccccggat aatggttccc cagcatctgc 1020
taattgttct tcccccaaga actcaaagtt aagtgatgcc cctgttcttt atgcatcaga 1080
aaagaaggaa tacccggtgg atccatctct tccagatatc aagaatagca tttactcaac 1140
agatgaattc cgaatgtatt cttttaaagt gcggccttgt tcacgagcgt actcgcatga 1200
ttggacggag tgccctttttg ttcatccagg ggagaatgcc cgtagaaggg atccaaggaa 1260
gttccactac agctgtgtcc cttgccctga tttccggaag ggtgcttgta gacgtggaga 1320
tatgtgtgaa tatgctcatg gtgtttttga gtgctggctc catcctgctc agtatcggac 1380
tcgattatgc aaggatggta caagttgtgc tcggagagtg tgcttctttg cccacacgga 1440
gcaagagctg cgtccattgt acgtctccac tggttctgct gttccgtctc ctcgctcgag 1500
tacctctgga gctgctgcca tggattttgc tgcagccatg agcctcttac ctggttcccc 1560
atcatcagta tccatcatgt ccccttcacc cttcactcct cccatgtctc catctgctaa 1620
tggtatttct cacccatctg ttgcctggcc ccagcaaaat gtaccaactt tgcatcttcc 1680
cggaagcaat cttcagtcca gccgcttgag atcttctctt aatgcaagag atattcctca 1740
ggaggatttt gacttgctgt cagattatga tgtgcaacag cagcagctcc taaatgagtt 1800
ttccatcctt tcacaacaat cgatgggtgc taattccttg aaccgttctg gtcggctgaa 1860
aactttgacc ccctcaaacc ttgatgatct cttctctgct gagagctcat cccctcgcta 1920
cgctgatcaa gccctggctt ctgctgtttt ttcaccaacg cacaaatctg cagtaatcaa 1980
tcaatttcag cagcagcagc agagcatgtt atcacccatc aacacaacct tctctcctaa 2040
gagtgtcgac cacccctttgt tgcaagcgtc tttcggtgtt caatctgggc gaatgtcccc 2100
tcgtaacatg gatcccatct ctcctataag ttctcgtgtg tcgatgttgg cccaacgaga 2160
gaaacagcaa cagcaattac gcagcctaag ctctcgtgaa ctcggttcca attcagccgc 2220
cattgtgggt tcccccgtgg gttcttggtc gaaatgggga gctacaaatg ggaaaccaga 2280
ctgggctgtt agtgcagatg aactaggtaa gcttcgcagg tctaattcat ttgagcttgg 2340
gaacaatggt gaggagccag atctttcatg ggttcaatcc ctcgttaaag aatctcctac 2400

```
cgagatgaaa gaaaagcttt cgtcaactct ctctggtgtt ccagcccccg ctacatccag   2460
tgaggttccg agtatcagct cgcagatgga atcggttgat cacgaagtgc taggagcatg   2520
gctccagcag atgcagctcg atccgctcgt ggctcagcaa aactaggttg ttttttttcc   2580
tacatggcct tgaggaagta gacagcggaa agtttttttt ggtaaatact atgtttttc    2640
tggaaatttt tgatgctggg ggtggggtct ggaagaagat aacaaggcag gaaaggggtc   2700
agtgaagtca ctggagaaaa ggaattcatt tttaaccatt ttatcattct attacaacag   2760
aaagtaggga aaaaaaagga agaccctctg ggttatgaag agaaattaaa cccaggctag   2820
gcgttctcct ttctaatatt tccaatttta ggtccatatt actgtcattt ccttttttgcc  2880
gtcttatcat atttcatcaa aatggaactg gggactaatg tttgttccat tctttcgctc   2940
ttctgattta tttgcaccct tggggtaaga tcaaaagaga aattatgatc attttctttt   3000
gaggatattt ttttttccca atatttgtga gaatgaaagt taagagggga tatgatgtgt   3060
ctggtgttgt agtatgaaaa accaataacc gagttcacct gttgctgctg gtggtagaag   3120
aagtggagaa gaagctatga tcctttgatg taacagtcaa tcaaacattt taataccttt   3180
atttttttgtt tcctcatgta atccatcctt tgtgattgtc ctctctctct ctctctctct  3240
ctctctctcc ctccccgtgt tctttcttca taagcgtctt gcttgtcgat ctgtaaatta   3300
ttgaaaaggg tcatggaaag ccgtgccggt gtggattctc atttttgcaa aaaaaaaaaa   3360
aaaaaaaaaa aa                                                        3372
```

<210> 11
<211> 704
<212> PRT
<213> Eucalyptus grandis

<400> 11

```
Met Ser Gln Leu Thr Ile Gln Thr Glu Asp Thr Phe Ala Ser Leu Leu
1               5                   10                  15

Glu Leu Ala Ala Asn Asn Asp Thr Glu Ser Phe Gly Arg Cys Val Glu
            20                  25                  30

Arg Asp Pro Ser Ser Ile Asp Glu Ile Gly Tyr Trp Tyr Gly Arg Gln
        35                  40                  45

Lys Gly Ser Lys Gln Val Val Asn Met Gln Arg Thr Pro Leu Met Val
    50                  55                  60

Ala Ala Thr Tyr Gly Ser Val Asp Val Met Arg Leu Ile Leu Cys Leu
65                  70                  75                  80

Ser Asp Ala Asp Val Asn Arg Thr Cys Ser Thr Asp Lys Ser Thr Ala
                85                  90                  95
```

```
Leu His Cys Ala Ala Ser Gly Gly Ala Val Asn Ala Val Asp Ala Val
            100                 105                 110

Arg Leu Leu Leu Ser Ala Gly Ala Asp Pro Ser Leu Ala Asp Ala Asn
        115                 120                 125

Gly Gln Arg Pro Val Asp Val Ile Val Val Pro Pro Lys Leu Leu Ser
    130                 135                 140

Ile Lys Phe Ala Leu Glu Glu Leu Leu Ser Thr Glu Gly Ser Val Asn
145                 150                 155                 160

Glu His Asn Leu Arg Val Ser Val Ala Thr Ser Asn Ser Thr Ser Pro
                165                 170                 175

Pro Leu Ser Ser Ser Pro Asp Asn Gly Ser Pro Ala Ser Ala Asn Cys
            180                 185                 190

Ser Ser Pro Lys Asn Ser Lys Leu Ser Asp Ala Pro Val Leu Tyr Ala
        195                 200                 205

Ser Glu Lys Lys Glu Tyr Pro Val Asp Pro Ser Leu Pro Asp Ile Lys
    210                 215                 220

Asn Ser Ile Tyr Ser Thr Asp Glu Phe Arg Met Tyr Ser Phe Lys Val
225                 230                 235                 240

Arg Pro Cys Ser Arg Ala Tyr Ser His Asp Trp Thr Glu Cys Pro Phe
                245                 250                 255

Val His Pro Gly Glu Asn Ala Arg Arg Arg Asp Pro Arg Lys Phe His
            260                 265                 270

Tyr Ser Cys Val Pro Cys Pro Asp Phe Arg Lys Gly Ala Cys Arg Arg
        275                 280                 285

Gly Asp Met Cys Glu Tyr Ala His Gly Val Phe Glu Cys Trp Leu His
    290                 295                 300

Pro Ala Gln Tyr Arg Thr Arg Leu Cys Lys Asp Gly Thr Ser Cys Ala
305                 310                 315                 320

Arg Arg Val Cys Phe Phe Ala His Thr Glu Gln Glu Leu Arg Pro Leu
                325                 330                 335

Tyr Val Ser Thr Gly Ser Ala Val Pro Ser Pro Arg Ser Ser Thr Ser
            340                 345                 350
```

Gly Ala Ala Ala Met Asp Phe Ala Ala Ala Met Ser Leu Leu Pro Gly
355 360 365

Ser Pro Ser Ser Val Ser Ile Met Ser Pro Ser Pro Phe Thr Pro Pro
370 375 380

Met Ser Pro Ser Ala Asn Gly Ile Ser His Pro Ser Val Ala Trp Pro
385 390 395 400

Gln Gln Asn Val Pro Thr Leu His Leu Pro Gly Ser Asn Leu Gln Ser
405 410 415

Ser Arg Leu Arg Ser Ser Leu Asn Ala Arg Asp Ile Pro Gln Glu Asp
420 425 430

Phe Asp Leu Leu Ser Asp Tyr Asp Val Gln Gln Gln Gln Leu Leu Asn
435 440 445

Glu Phe Ser Ile Leu Ser Gln Gln Ser Met Gly Ala Asn Ser Leu Asn
450 455 460

Arg Ser Gly Arg Leu Lys Thr Leu Thr Pro Ser Asn Leu Asp Asp Leu
465 470 475 480

Phe Ser Ala Glu Ser Ser Ser Pro Arg Tyr Ala Asp Gln Ala Leu Ala
485 490 495

Ser Ala Val Phe Ser Pro Thr His Lys Ser Ala Val Ile Asn Gln Phe
500 505 510

Gln Gln Gln Gln Gln Ser Met Leu Ser Pro Ile Asn Thr Thr Phe Ser
515 520 525

Pro Lys Ser Val Asp His Pro Leu Leu Gln Ala Ser Phe Gly Val Gln
530 535 540

Ser Gly Arg Met Ser Pro Arg Asn Met Asp Pro Ile Ser Pro Ile Ser
545 550 555 560

Ser Arg Val Ser Met Leu Ala Gln Arg Glu Lys Gln Gln Gln Gln Leu
565 570 575

Arg Ser Leu Ser Ser Arg Glu Leu Gly Ser Asn Ser Ala Ala Ile Val
580 585 590

Gly Ser Pro Val Gly Ser Trp Ser Lys Trp Gly Ala Thr Asn Gly Lys
595 600 605

Pro Asp Trp Ala Val Ser Ala Asp Glu Leu Gly Lys Leu Arg Arg Ser
    610                 615                 620

Asn Ser Phe Glu Leu Gly Asn Asn Gly Glu Glu Pro Asp Leu Ser Trp
625                 630                 635                 640

Val Gln Ser Leu Val Lys Glu Ser Pro Thr Glu Met Lys Glu Lys Leu
                645                 650                 655

Ser Ser Thr Leu Ser Gly Val Pro Ala Pro Ala Thr Ser Ser Glu Val
            660                 665                 670

Pro Ser Ile Ser Ser Gln Met Glu Ser Val Asp His Glu Val Leu Gly
        675                 680                 685

Ala Trp Leu Gln Gln Met Gln Leu Asp Pro Leu Val Ala Gln Gln Asn
    690                 695                 700

<210> 12
<211> 1860
<212> DNA
<213> Oryza sativa

<400> 12
atgggggagc ctgggggcgc cgaggcggcc gtctccgcga ggctgctcga gctggcggcc 60

gacgacaacg cggcggggct cggggagctc ctcgcggcgt ggccctccct cgccgacgag 120

cccgcgccgt ggtacacccc ggcgcggggc gcggagccgc tgaccccgct catggtcgcc 180

gccgtgtacg gctcggtggg ctgcctcgac gcgctcctct cgccgcccta cctcgtggac 240

cccaaccgcg cctcggcgtc gtcgctctcc accccgctcc acctcgccgc cgcgggcggg 300

tccgcctccg cccccgcggc ggtctcccgc ctcctcgccg ccggcgccga cccggccctc 360

ctcgaccacc tccagcgccg ggcgtccgac ctcgtcgcgc tcccgcccaa ctcgctcccg 420

ctcaagaacc acctcctctc cctcctcggc gcccgcaagg agtggcctcc cgacccctcc 480

ctccccgaca tcaagaacgg cgcctacgcc tccgacgact tcaggatgta ctcgttcaag 540

gtgcgcgcgt gctcgcgggc ctactcccat gactggacgg agtgcccctt cgtccacccc 600

ggcgagaacg cgcggcggcg cgacccgagg aagtaccact acagctgcgt gccgtgcccg 660

gagttcaaga agggggccgg gtgcaggaga ggggacatgt gcgagtacgc gcacggggtg 720

ttcgagagct ggctccaccc ggcgcagtac cggacgcgcc tctgcaagga cggcgtcggc 780

tgcgcccgcc gcgtctgctt cttcgcccac acgcccgacg agctccgccc gctctacgtc 840

tccacgggct ccgccgtgcc gtcgccgcgc ggggcgttgg agatggcggc ggcggcggcg 900

gcgatgggga tggggctgtc gtcgccgggg tcgtcgtcgt tcacgccgcc gctatcgccg 960

tcggccggcg gggcgggggg cgggggcggg ggcagcggcg gcggcggcgc gtggccgcag 1020

cagccgagcg tgccggcgct ctgcctgccc gggagcgccg ggaacctcca cctgagccgg 1080

ctgcgcacgt cgctgagcgc gcgcgacatg gccgtcgacg agctgctcgc cgcggcggcg 1140

gcggcggcgg actacgacgg cctcgtcgcc tccccgcct ccatccggtc cgcgaggggg 1200

aaggcgcttg tgccgtcaaa tctcgacgag ctcttctccg ctgagctcgc cgccgccgcg 1260

gcgtcgcgct cgccgcgcta cgccgaccaa ggcggcgccg cgttctcccc gacccgcaag 1320

gccaccgtgc tcaaccaatt ccagctgcag cagcagcata gcttgctctc gccgcgggcg 1380

gccgcggtga caccagagcc ggtctcccca atgagctccc gcctcctcgc cgcgctggcg 1440

cagcgggaga agatgcagca gcagacgctg cggagcatga gctcacggga cctcggcaac 1500

gccgcgtcgc tgctggtcgg ctcgccggtg agctcgagca tgtccaaatg gggggttcccc 1560

tccggcaacc cggactgggg cgccgacgac gaggagctcg gccgcctcaa gcgttgctcc 1620

tcgttcgagc tccggtccgg agccgccaat ggcaaccatg agcctgacct ctcatgggtc 1680

aacaccctag tgaaggagcc gacaccggag aagatgatga cgacgacatc ggcaatggat 1740

tccattggca tcttgggaca gaacacaagc cgtgatcaca tcgtcggagg cgaggatgac 1800

actgccggag tcatcagcag ctggcttgaa cagctccagc tcgatgagat ggttgtctag 1860

<210> 13
<211> 619
<212> PRT
<213> Oryza sativa

<400> 13

Met Gly Glu Pro Gly Gly Ala Glu Ala Ala Val Ser Ala Arg Leu Leu
1 5 10 15

Glu Leu Ala Ala Asp Asp Asn Ala Ala Gly Leu Gly Glu Leu Leu Ala
20 25 30

Ala Trp Pro Ser Leu Ala Asp Glu Pro Ala Pro Trp Tyr Thr Pro Ala
35 40 45

Arg Gly Ala Glu Pro Leu Thr Pro Leu Met Val Ala Ala Val Tyr Gly
50 55 60

Ser Val Gly Cys Leu Asp Ala Leu Leu Ser Pro Pro Tyr Leu Val Asp
65 70 75 80

Pro Asn Arg Ala Ser Ala Ser Ser Leu Ser Thr Pro Leu His Leu Ala
85 90 95

Ala Ala Gly Gly Ser Ala Ser Ala Pro Ala Ala Val Ser Arg Leu Leu
100 105 110

Ala Ala Gly Ala Asp Pro Ala Leu Leu Asp His Leu Gln Arg Arg Ala
115 120 125

```
Ser Asp Leu Val Ala Leu Pro Pro Asn Ser Leu Pro Leu Lys Asn His
    130                 135                 140

Leu Leu Ser Leu Leu Gly Ala Arg Lys Glu Trp Pro Pro Asp Pro Ser
145                 150                 155                 160

Leu Pro Asp Ile Lys Asn Gly Ala Tyr Ala Ser Asp Asp Phe Arg Met
                165                 170                 175

Tyr Ser Phe Lys Val Arg Ala Cys Ser Arg Ala Tyr Ser His Asp Trp
            180                 185                 190

Thr Glu Cys Pro Phe Val His Pro Gly Glu Asn Ala Arg Arg Arg Asp
        195                 200                 205

Pro Arg Lys Tyr His Tyr Ser Cys Val Pro Cys Pro Glu Phe Lys Lys
    210                 215                 220

Gly Ala Gly Cys Arg Arg Gly Asp Met Cys Glu Tyr Ala His Gly Val
225                 230                 235                 240

Phe Glu Ser Trp Leu His Pro Ala Gln Tyr Arg Thr Arg Leu Cys Lys
                245                 250                 255

Asp Gly Val Gly Cys Ala Arg Arg Val Cys Phe Phe Ala His Thr Pro
            260                 265                 270

Asp Glu Leu Arg Pro Leu Tyr Val Ser Thr Gly Ser Ala Val Pro Ser
        275                 280                 285

Pro Arg Gly Ala Leu Glu Met Ala Ala Ala Ala Ala Ala Met Gly Met
    290                 295                 300

Gly Leu Ser Ser Pro Gly Ser Ser Ser Phe Thr Pro Pro Leu Ser Pro
305                 310                 315                 320

Ser Ala Gly Gly Gly Gly Gly Gly Gly Gly Gly Ser Gly Gly Gly Gly
                325                 330                 335

Ala Trp Pro Gln Gln Pro Ser Val Pro Ala Leu Cys Leu Pro Gly Ser
            340                 345                 350

Ala Gly Asn Leu His Leu Ser Arg Leu Arg Thr Ser Leu Ser Ala Arg
        355                 360                 365

Asp Met Ala Val Asp Glu Leu Leu Ala Ala Ala Ala Ala Ala Ala Asp
    370                 375                 380
```

Tyr Asp Gly Leu Val Ala Ser Pro Ala Ser Ile Arg Ser Ala Arg Gly
385 390 395 400

Lys Ala Leu Val Pro Ser Asn Leu Asp Glu Leu Phe Ser Ala Glu Leu
405 410 415

Ala Ala Ala Ala Ala Ser Arg Ser Pro Arg Tyr Ala Asp Gln Gly Gly
420 425 430

Ala Ala Phe Ser Pro Thr Arg Lys Ala Thr Val Leu Asn Gln Phe Gln
435 440 445

Leu Gln Gln Gln His Ser Leu Leu Ser Pro Arg Ala Ala Ala Val Thr
450 455 460

Pro Glu Pro Val Ser Pro Met Ser Ser Arg Leu Leu Ala Ala Leu Ala
465 470 475 480

Gln Arg Glu Lys Met Gln Gln Gln Thr Leu Arg Ser Met Ser Ser Arg
485 490 495

Asp Leu Gly Asn Ala Ala Ser Leu Leu Val Gly Ser Pro Val Ser Ser
500 505 510

Ser Met Ser Lys Trp Gly Phe Pro Ser Gly Asn Pro Asp Trp Gly Ala
515 520 525

Asp Asp Glu Glu Leu Gly Arg Leu Lys Arg Cys Ser Ser Phe Glu Leu
530 535 540

Arg Ser Gly Ala Ala Asn Gly Asn His Glu Pro Asp Leu Ser Trp Val
545 550 555 560

Asn Thr Leu Val Lys Glu Pro Thr Pro Glu Lys Met Met Thr Thr Thr
565 570 575

Ser Ala Met Asp Ser Ile Gly Ile Leu Gly Gln Asn Thr Ser Arg Asp
580 585 590

His Ile Val Gly Gly Glu Asp Asp Thr Ala Gly Val Ile Ser Ser Trp
595 600 605

Leu Glu Gln Leu Gln Leu Asp Glu Met Val Val
610 615

<210> 14
<211> 2106
<212> DNA
<213> Medicago truncatula

<400> 14
atgaaaaatc taactgttcg tactgatgat tctttttcca gcttacttga acatgcttct 60
aacaatgatt ttgaagattt caaggtagct ctagatagtg atgcttcact tattaatgaa 120
gttggcttct ggtatgtccg tcaaaaggga tctaaccaaa ttgttcttga gcaccgaacc 180
cctttaatgg tggctgcttc ctatgggagt attgatattc taaagcttat actctcatat 240
cccgaggctg atgttaattt ctcctgtgga actgataaaa gcactgctct tcactgtgct 300
gcctcaagtg gttcagttaa tgctgttgat gctataaaat tgcttttatc agctggtgct 360
gatatcaatt ctgtggatgc taatgggaaa cgccctgtgg atgttatcgt tgttcctatt 420
gttgttcctc ataagctcga aggtgttaaa acaattcttg aagaacttct ctcagacagt 480
gcttctgaag gatctgtgga tgattgctct cttcccctgt ctcttatttc atcgagtcct 540
ggttcatctg cccctttatc atctgctgaa aatggatctc catcctctcc tgtggctccc 600
aagtttacag atacagctgt taattctaca tcagaaaaga aagagtatcc agttgaccca 660
tctcttcctg acataaaaaa cagcatgtat gccacagatg aattccgcat gtattcattc 720
aaggttcgtc cttgttctcg tgcatactct catgattgga ctgagtgtcc ttttgtgcat 780
cctggagaga atgctcgaag gagagaccct agaaagtttc actacagctg tgtgccatgc 840
cctgatttta ggaaaggggc ttgccgacgt tcggatatgt gtgaatatgc tcatggagta 900
ttcgagtgct ggctacaccc agctcagtat cggacaaggc tgtgcaaaga cggtatgggt 960
tgtaaccgaa gggtgtgctt cttcgctcac tcacctgaag agctgcgtcc gctgtatgtg 1020
tccactggtt ctgctgttcc ttcaccccga tcagctgctt ctactgctaa tgtcatggac 1080
atggctgctg ctatgagcct tttccctggt tcaccatcat caatctcttt gatgtctcaa 1140
tcaccctttg cacagcctcc tctatctcca tctgcaaatg gcaataatgc ttggccacag 1200
cccaatgtgc cagctcttca tttaccagga agcattaatc aaactagtcg tttgagatct 1260
tctcttagtg cccgtgatat gccacacgac gacttcaaca atatgttgca agactttgat 1320
gggcagcagc agatactaaa tgacttgagc tgtttctcac agccccgtcc tggtgctatt 1380
tcagttggtc gatctggccg ccctaaaaca ctaactccct caaatctgga tgatcttttt 1440
tgtgctgaga ttgcttcatc tcctaggtat tccgaccccg ctgcggcttc tgtattttcc 1500
ccaacacaca aatctgctgt cttcaaccag tttcaacagc ttcaaagctc cttatcaccc 1560
atcaacacaa atgtcatgtc tcctacaaac gtagagcatc ccctgttcca ccaggcttca 1620
tatggtctct cttctcctgg aaggatgtca ccaagaagta tggaagccct atctccaatg 1680
agttctcggc tgtcagcttt tgctcagcgt gagaaacaac agcagcagca gcaacagctg 1740
cgtagcctca gctcaagaga actcggtgct aacaatcctc tctcagctgt tgggtcccct 1800
gttaactcct ggtccaagtg gggatcatcc cctattggaa aagctgattg gtcggtaaat 1860
ccaaatgact tcggtcaaac acagagatca acttcttttg agcatggaaa caatggagaa 1920

gagcctgatg taggttgggt ccattccctt gtcaaggatc ccacacctga gaagaaagag 1980

aagcttgcag gttccggccc aattccatcc gttgaaaaga atcccaatcc tcaagcggac 2040

ggcattgatc actctgtttt gggagcttgg ctcgagcaac tgcagctgga tcaacttgta 2100

gtctag 2106

<210> 15
<211> 701
<212> PRT
<213> Medicago truncatula

<400> 15

Met Lys Asn Leu Thr Val Arg Thr Asp Asp Ser Phe Ser Ser Leu Leu
1 5 10 15

Glu His Ala Ser Asn Asn Asp Phe Glu Asp Phe Lys Val Ala Leu Asp
20 25 30

Ser Asp Ala Ser Leu Ile Asn Glu Val Gly Phe Trp Tyr Val Arg Gln
35 40 45

Lys Gly Ser Asn Gln Ile Val Leu Glu His Arg Thr Pro Leu Met Val
50 55 60

Ala Ala Ser Tyr Gly Ser Ile Asp Ile Leu Lys Leu Ile Leu Ser Tyr
65 70 75 80

Pro Glu Ala Asp Val Asn Phe Ser Cys Gly Thr Asp Lys Ser Thr Ala
85 90 95

Leu His Cys Ala Ala Ser Ser Gly Ser Val Asn Ala Val Asp Ala Ile
100 105 110

Lys Leu Leu Leu Ser Ala Gly Ala Asp Ile Asn Ser Val Asp Ala Asn
115 120 125

Gly Lys Arg Pro Val Asp Val Ile Val Val Pro Ile Val Val Pro His
130 135 140

Lys Leu Glu Gly Val Lys Thr Ile Leu Glu Glu Leu Leu Ser Asp Ser
145 150 155 160

Ala Ser Glu Gly Ser Val Asp Asp Cys Ser Leu Pro Leu Ser Leu Ile
165 170 175

Ser Ser Ser Pro Gly Ser Ser Ala Pro Leu Ser Ser Ala Glu Asn Gly
180 185 190

```
Ser Pro Ser Ser Pro Val Ala Pro Lys Phe Thr Asp Thr Ala Val Asn
        195                 200                 205

Ser Thr Ser Glu Lys Lys Glu Tyr Pro Val Asp Pro Ser Leu Pro Asp
    210                 215                 220

Ile Lys Asn Ser Met Tyr Ala Thr Asp Glu Phe Arg Met Tyr Ser Phe
225                 230                 235                 240

Lys Val Arg Pro Cys Ser Arg Ala Tyr Ser His Asp Trp Thr Glu Cys
                245                 250                 255

Pro Phe Val His Pro Gly Glu Asn Ala Arg Arg Arg Asp Pro Arg Lys
            260                 265                 270

Phe His Tyr Ser Cys Val Pro Cys Pro Asp Phe Arg Lys Gly Ala Cys
        275                 280                 285

Arg Arg Ser Asp Met Cys Glu Tyr Ala His Gly Val Phe Glu Cys Trp
    290                 295                 300

Leu His Pro Ala Gln Tyr Arg Thr Arg Leu Cys Lys Asp Gly Met Gly
305                 310                 315                 320

Cys Asn Arg Arg Val Cys Phe Phe Ala His Ser Pro Glu Glu Leu Arg
                325                 330                 335

Pro Leu Tyr Val Ser Thr Gly Ser Ala Val Pro Ser Pro Arg Ser Ala
            340                 345                 350

Ala Ser Thr Ala Asn Val Met Asp Met Ala Ala Ala Met Ser Leu Phe
        355                 360                 365

Pro Gly Ser Pro Ser Ser Ile Ser Leu Met Ser Gln Ser Pro Phe Ala
    370                 375                 380

Gln Pro Pro Leu Ser Pro Ser Ala Asn Gly Asn Asn Ala Trp Pro Gln
385                 390                 395                 400

Pro Asn Val Pro Ala Leu His Leu Pro Gly Ser Ile Asn Gln Thr Ser
                405                 410                 415

Arg Leu Arg Ser Ser Leu Ser Ala Arg Asp Met Pro His Asp Asp Phe
            420                 425                 430

Asn Asn Met Leu Gln Asp Phe Asp Gly Gln Gln Gln Ile Leu Asn Asp
        435                 440                 445

Leu Ser Cys Phe Ser Gln Pro Arg Pro Gly Ala Ile Ser Val Gly Arg
```

450 455 460

Ser Gly Arg Pro Lys Thr Leu Thr Pro Ser Asn Leu Asp Asp Leu Phe
465 470 475 480

Cys Ala Glu Ile Ala Ser Ser Pro Arg Tyr Ser Asp Pro Ala Ala Ala
485 490 495

Ser Val Phe Ser Pro Thr His Lys Ser Ala Val Phe Asn Gln Phe Gln
500 505 510

Gln Leu Gln Ser Ser Leu Ser Pro Ile Asn Thr Asn Val Met Ser Pro
515 520 525

Thr Asn Val Glu His Pro Leu Phe His Gln Ala Ser Tyr Gly Leu Ser
530 535 540

Ser Pro Gly Arg Met Ser Pro Arg Ser Met Glu Ala Leu Ser Pro Met
545 550 555 560

Ser Ser Arg Leu Ser Ala Phe Ala Gln Arg Glu Lys Gln Gln Gln Gln
565 570 575

Gln Gln Gln Leu Arg Ser Leu Ser Ser Arg Glu Leu Gly Ala Asn Asn
580 585 590

Pro Leu Ser Ala Val Gly Ser Pro Val Asn Ser Trp Ser Lys Trp Gly
595 600 605

Ser Ser Pro Ile Gly Lys Ala Asp Trp Ser Val Asn Pro Asn Asp Phe
610 615 620

Gly Gln Thr Gln Arg Ser Thr Ser Phe Glu His Gly Asn Asn Gly Glu
625 630 635 640

Glu Pro Asp Val Gly Trp Val His Ser Leu Val Lys Asp Pro Thr Pro
645 650 655

Glu Lys Lys Glu Lys Leu Ala Gly Ser Gly Pro Ile Pro Ser Val Glu
660 665 670

Lys Asn Pro Asn Pro Gln Ala Asp Gly Ile Asp His Ser Val Leu Gly
675 680 685

Ala Trp Leu Glu Gln Leu Gln Leu Asp Gln Leu Val Val
690 695 700

<210> 16
<211> 2841

<212> DNA
<213> Oryza sativa

<400> 16
atgaacggca cgccgatctc cgcgtccgcc gcggccggcg tcgacggagt cggcgcggcg 60

gtggcgctgg cggccgcgac caagaagagt gccgccgcgg cggccgccgt cgccgagatg 120

gcgaaaaccc tcaccgtcga cacggacgac gccttcgcgg ggctcctcga gctcgccgcg 180

gacgacgacg cggagggcct gcgccgcgcg ctggagcgcg ccccgcccgc cgccgcggac 240

gaggcgggcc tctggtacgg ccgccgcaag gtcctcgagc accgcacgcc gctgatggtc 300

gcggccacct atggcagcct cgcggtgctt cgcctgctgc tgtccctccc gtccgtcgat 360

gtcaatcgcc gctgtggctc cgacggcacc accgccctcc actgtgcggc gtctggtggc 420

tcgccgtctt gtgtggaggc cgtcaagctg ctgcttgctg ctggggctga tgctgatgcc 480

acggatgctt ccggatatcg tccagctgat gtgatctctg ttcctccaaa gatgtttgac 540

gccaagattg ccctccaaga tcttcttgga tgcccaaagg ctgggcatgg cgttctccgg 600

gtggtgacaa gggccgcaaa ctctatgttg tcacctgtat catcccctac agcagaagat 660

gcacgatctc catcagctgc tgtgatgatg acgacaaagt ttgcagatct tccaagggtt 720

gtgacatcgg aaaagaaaga atatccagtg gatccgtccc ttcccgatat caagaacagc 780

atctatgctt ccgatgagtt ccgcatgtac tcatttaaga tcaggccatg ctcgcgggcg 840

tactcacatg attggactga gtgcccgttt gttcacccag gggagaacgc acggcgtcgg 900

gaccctcgca agtatcacta cagctgtgtg ccatgccccg actttagaaa gggagtttgc 960

cggcgtggtg acatgtgtga atatgctcat ggcgtgttcg agtgttggct ccatccagca 1020

cagtaccgta ctcgcctttg caaggatggc acaagctgta atcgccgtgt ctgtttcttt 1080

gcgcatacaa ctgatgagct ccgaccacta tatgtttcca ctggatctgc agtaccatcc 1140

ccaagagcct cggcaacagc tacaatggag atggctgcag caatgggctt gatgcctggt 1200

tctccatcat cagtttcagc agtcatgtcc ccatttacac caccaatgtc cccttcaggc 1260

aatgggatgc ccccttcatt gggctggcag cagccaaatg ttccgacact acaccttcca 1320

ggcagcagcc ttcagtcgag ccggctccgt acctcactta gtgcaaggga tatgcctgct 1380

gatgattact ccctgatgca ggatattgat tcacagctta taaatgattt gtgctattca 1440

cgtattggtt catcaacagg aaaccacacg tctcggacca agtccctaaa tccgtcaaac 1500

ttggatgatc tcttctctgc tgagatggtc tcttccccga ggtatagtaa tgctgatcag 1560

ggtggtatgt tttcaccatc tcacaaggct gctttcctta atcagttcca gcaacagcag 1620

caggcacttc tttcaccaat caacacagtc ttctccccga agtctgtgga caaccagcag 1680

ttgccttcac actcatctct gttgcaagca tcacttggta tatcctcccc tggccgcatg 1740

tctcctcgat gtgttgaatc tgggtcccct atgaactctc atcttgctgc tgctcttgct 1800

cagcgtgaga agcaacagca gacaatgaga agtctcagtt ctcgtgatct tgggccgagt 1860

```
gctgcaagag catcaggtgt tgttggctcc cctctaagct catcatggtc aaagtgggga   1920

tcaccttcag ggacacctga ctggggtgtt aatggtgaag aattgggcaa gcttcgccgg   1980

tcatcatcgt ttgagctgag atctggtggt gatgatccag atctctcttg ggtacacaca   2040

ctggttaagg aatctccacc agagaagcaa gtcactactg ctgaatccat aaactctgtt   2100

ggaccttcac cactgatgcc tcccagtgta agcaacggtg aaggtcctag tctgaatgcc   2160

ccgctggatg ggcatgacca agctgctgtt attggagcat tgcttgaaca gatgcagctt   2220

gatcagcata ttggtagtct agcaacataa gcgctgaatg agcctggaaa gtgcaaggag   2280

ttattattct tagttaatga atttggagta attttttttcc tgttcattaa gatggtcagc   2340

aagcaaaagg atggatagct gatggtggtg attcagagat tggttttctt tactttattg   2400

aggtaaatca tatacattat tgaggttcca gtaggttgaa agattgaagt accttgattg   2460

gggtcgtttc aagaccgacc caggtagaat cgcacccccgg cagcttcaat tcatcggtca   2520

aaaatatttc cctgttttgt taattaaccc cgttaaaaaa gaagactcgt ttggtgtttc   2580

ggaattcttt tctttacctt agcggtgttt attttgttta ttatgatatt gatacttgat   2640

gtactgatgg gtataaggtt ggttaccagg catgctatag tggtatatca agtcccaaag   2700

tattcttttt ctccctttca ccatttgtcg aggatcatac tatggccttg ttttggtcag   2760

atcttgaggc ctgtataatc cttggatttg taataatgta atattgtcat tgaacttaca   2820

ttgctattgt tttgcaatcg c                                               2841
```

```
<210>  17
<211>  749
<212>  PRT
<213>  Oryza sativa

<400>  17

Met Asn Gly Thr Pro Ile Ser Ala Ser Ala Ala Ala Gly Val Asp Gly
1               5                   10                  15

Val Gly Ala Ala Val Ala Leu Ala Ala Ala Thr Lys Lys Ser Ala Ala
            20                  25                  30

Ala Ala Ala Ala Val Ala Glu Met Ala Lys Thr Leu Thr Val Asp Thr
        35                  40                  45

Asp Asp Ala Phe Ala Gly Leu Leu Glu Leu Ala Ala Asp Asp Asp Ala
    50                  55                  60

Glu Gly Leu Arg Arg Ala Leu Glu Arg Ala Pro Pro Ala Ala Ala Asp
65                  70                  75                  80

Glu Ala Gly Leu Trp Tyr Gly Arg Arg Lys Val Leu Glu His Arg Thr
                85                  90                  95
```

```
Pro Leu Met Val Ala Ala Thr Tyr Gly Ser Leu Ala Val Leu Arg Leu
            100                 105                 110

Leu Leu Ser Leu Pro Ser Val Asp Val Asn Arg Arg Cys Gly Ser Asp
        115                 120                 125

Gly Thr Thr Ala Leu His Cys Ala Ala Ser Gly Gly Ser Pro Ser Cys
    130                 135                 140

Val Glu Ala Val Lys Leu Leu Leu Ala Ala Gly Ala Asp Ala Asp Ala
145                 150                 155                 160

Thr Asp Ala Ser Gly Tyr Arg Pro Ala Asp Val Ile Ser Val Pro Pro
                165                 170                 175

Lys Met Phe Asp Ala Lys Ile Ala Leu Gln Asp Leu Leu Gly Cys Pro
            180                 185                 190

Lys Ala Gly His Gly Val Leu Arg Val Val Thr Arg Ala Ala Asn Ser
        195                 200                 205

Met Leu Ser Pro Val Ser Ser Pro Thr Ala Glu Asp Ala Arg Ser Pro
    210                 215                 220

Ser Ala Ala Val Met Met Thr Thr Lys Phe Ala Asp Leu Pro Arg Val
225                 230                 235                 240

Val Thr Ser Glu Lys Lys Glu Tyr Pro Val Asp Pro Ser Leu Pro Asp
                245                 250                 255

Ile Lys Asn Ser Ile Tyr Ala Ser Asp Glu Phe Arg Met Tyr Ser Phe
            260                 265                 270

Lys Ile Arg Pro Cys Ser Arg Ala Tyr Ser His Asp Trp Thr Glu Cys
        275                 280                 285

Pro Phe Val His Pro Gly Glu Asn Ala Arg Arg Arg Asp Pro Arg Lys
    290                 295                 300

Tyr His Tyr Ser Cys Val Pro Cys Pro Asp Phe Arg Lys Gly Val Cys
305                 310                 315                 320

Arg Arg Gly Asp Met Cys Glu Tyr Ala His Gly Val Phe Glu Cys Trp
                325                 330                 335

Leu His Pro Ala Gln Tyr Arg Thr Arg Leu Cys Lys Asp Gly Thr Ser
            340                 345                 350
```

```
Cys Asn Arg Arg Val Cys Phe Phe Ala His Thr Thr Asp Glu Leu Arg
        355                 360                 365

Pro Leu Tyr Val Ser Thr Gly Ser Ala Val Pro Ser Pro Arg Ala Ser
    370                 375                 380

Ala Thr Ala Thr Met Glu Met Ala Ala Ala Met Gly Leu Met Pro Gly
385                 390                 395                 400

Ser Pro Ser Ser Val Ser Ala Val Met Ser Pro Phe Thr Pro Pro Met
                405                 410                 415

Ser Pro Ser Gly Asn Gly Met Pro Pro Ser Leu Gly Trp Gln Gln Pro
            420                 425                 430

Asn Val Pro Thr Leu His Leu Pro Gly Ser Ser Leu Gln Ser Ser Arg
        435                 440                 445

Leu Arg Thr Ser Leu Ser Ala Arg Asp Met Pro Ala Asp Asp Tyr Ser
    450                 455                 460

Leu Met Gln Asp Ile Asp Ser Gln Leu Ile Asn Asp Leu Cys Tyr Ser
465                 470                 475                 480

Arg Ile Gly Ser Ser Thr Gly Asn His Thr Ser Arg Thr Lys Ser Leu
                485                 490                 495

Asn Pro Ser Asn Leu Asp Asp Leu Phe Ser Ala Glu Met Val Ser Ser
            500                 505                 510

Pro Arg Tyr Ser Asn Ala Asp Gln Gly Gly Met Phe Ser Pro Ser His
        515                 520                 525

Lys Ala Ala Phe Leu Asn Gln Phe Gln Gln Gln Gln Gln Ala Leu Leu
    530                 535                 540

Ser Pro Ile Asn Thr Val Phe Ser Pro Lys Ser Val Asp Asn Gln Gln
545                 550                 555                 560

Leu Pro Ser His Ser Ser Leu Leu Gln Ala Ser Leu Gly Ile Ser Ser
                565                 570                 575

Pro Gly Arg Met Ser Pro Arg Cys Val Glu Ser Gly Ser Pro Met Asn
            580                 585                 590

Ser His Leu Ala Ala Ala Leu Ala Gln Arg Glu Lys Gln Gln Gln Thr
        595                 600                 605
```

```
Met Arg Ser Leu Ser Ser Arg Asp Leu Gly Pro Ser Ala Ala Arg Ala
    610                 615                 620

Ser Gly Val Val Gly Ser Pro Leu Ser Ser Ser Trp Ser Lys Trp Gly
625                 630                 635                 640

Ser Pro Ser Gly Thr Pro Asp Trp Gly Val Asn Gly Glu Glu Leu Gly
                645                 650                 655

Lys Leu Arg Arg Ser Ser Ser Phe Glu Leu Arg Ser Gly Gly Asp Asp
            660                 665                 670

Pro Asp Leu Ser Trp Val His Thr Leu Val Lys Glu Ser Pro Pro Glu
        675                 680                 685

Lys Gln Val Thr Thr Ala Glu Ser Ile Asn Ser Val Gly Pro Ser Pro
    690                 695                 700

Leu Met Pro Pro Ser Val Ser Asn Gly Glu Gly Pro Ser Leu Asn Ala
705                 710                 715                 720

Pro Leu Asp Gly His Asp Gln Ala Ala Val Ile Gly Ala Leu Leu Glu
                725                 730                 735

Gln Met Gln Leu Asp Gln His Ile Gly Ser Leu Ala Thr
            740                 745

<210>  18
<211>  2769
<212>  DNA
<213>  Arabidopsis thaliana

<400>  18
acaccagtta ccctctcatc cgttttcgtt tttttttct ctctttcaaa aatctctcag     60

ctgaggttga tcgatcttct tcttcttctt cctcactctt tagatttgtt ccttttgcat    120

tttacacttt tggatctgaa aatgtggttc tctgtttcgc ccgttaccgt ttagattcag    180

ttctgttttt ttcttacccg atcgcttgat tcggactgtg atctttgatc ttttttcttc    240

tccagtgccg tgaaggatgt gtggtcttgc taagaagctg gatatagagg atactttgac    300

atcactgtca gaccaagaga atgaatcttt ggccaaaccc atgaatgatg ctgctgaatg    360

ggaacattcg ttttctgcct tgcttgagtt tgctgcagac aacgatgtgg aggggtttag    420

gcggcaactc tctgatgtgt cttgtatcaa ccagatgggt ctttggtaca gacggcagag    480

gtttgttaga agaatggttc ttgagcaaag aaccccgctg atggttgctt cgttatatgg    540

gagtttagat gttgtgaagt ttattctttc tttcccggaa gcggagttga atctgtcttg    600

tggtcctgat aaaagtactg ctcttcattg cgctgcttct ggtgcttctg tgaattcctt    660

ggatgttgtc aagttgcttt tgagtgtagg agcagatcct aatatccctg atgctcatgg    720
```

aaatcgtcct gttgatgttc ttgttgtgtc tccacacgct cctggtttga gaaccatcct 780
tgaagagatc ttgaagaaag acgagattat atctgaagat ctgcatgcct cgtcatctag 840
cttgggatca agtttccggt ctctctcatc atcccctgat aatggttcct cgttactctc 900
cttagattca gtatcctctc cgactaagcc acacggtact gatgtaactt tcgcatcaga 960
gaagaaagag tacccaattg atccatcatt gcctgatatc aaaagcggga tttattcaac 1020
cgatgagttt cgtatgttct cgttcaagat ccgcccatgt tctcgagcat attcccatga 1080
ctggactgaa tgtccatttg cacacccagg tgagaatgca aggagaagag acccgaggaa 1140
gtttcactat acgtgtgttc catgcccgga ttttaagaaa ggatcctgta agcaaggtga 1200
tatgtgtgaa tatgctcatg ggtttttgga atgctggcta caccctgctc agtacagaac 1260
acgattgtgc aaggacggaa tgggttgcaa ccgaagggtt tgcttctttg ctcacgcaaa 1320
tgaggagttg cgtcccttgt acccttccac aggatctgga ttgccatctc ctcgggcttc 1380
gtctgctgtt tccgcctcta ctatggacat ggcgtcagtt ttgaacatgt taccaggctc 1440
accatctgct gctcaacatt cgttcacccc accaatatct ccttctggaa atggtagtat 1500
gccccattca tcgatgggtt ggcctcagca gaacataccg gcgttgaatc ttcctggaag 1560
caatatccag ttgagtcgtc tgagatcttc tcttaacgct agagatattc cttctgagca 1620
gcttagcatg ctgcatgagt ttgaaatgca acgtcagctt gctggcgata tgcacagtcc 1680
acgctttatg aatcattccg ctcgtcctaa gacactgaac ccttcaaatc tggaggaact 1740
cttctcagct gaggttgcat ctcctcgttt ctctgatcaa cttgctgttt catctgttct 1800
atcgccttcc cacaagtccg cgcttcttaa tcagctgcag aataataagc agagcatgct 1860
ttctcctatc aagacaaatc taatgtcttc tccaaagaat gtggagcaac attctcttct 1920
gcagcaagcc tcgtcacccc gaggcggaga gcctatttcc ccaatgaatg ctcgaatgaa 1980
acagcagcta cattcacgca gcctaagctc ccgtgatttt ggatctagtc tgccccgtga 2040
tttaatgccg actgattctg gttcgccatt aagtccatgg tcaagttggg accagaccca 2100
tggaagcaag gtggattggt cagtccaatc agatgagtta ggtcggttga gaaaatctca 2160
ttccttggct aataacccaa acagggaagc agatgtttca tgggctcagc agatgttaaa 2220
agactcttca tcacctagga acggaaaccg tgttgtgaac atgaatggtg caaggccatt 2280
gactcaaggt ggttcgagtg tgaatcctca caacagtgac actcgtgaga gcgacattct 2340
tgatgcgtgg cttgaacagc tgcacctaga tcgctgagcc tcagctgcga gagagaggtt 2400
cacatttctg tgaagctgtg aaactgatga ttcgtttatt tattattcaa gaaagcaaac 2460
ggaaacaaaa gcaaactccg ggtaagcttt tttcgattct aataacccta aaaggctcag 2520
ttttttcagg cttctttctg aaatttcttt actttcttat ttttatcacc tcattaaatt 2580
aattattgta tcatctctgt tgtaacaatg gccaaagtgc gcctctatta cttcccggat 2640

ttctgattta cattttttgt atcctctcag tttgtcaatt gtttctaata tctccttcat 2700

atttgtcaaa gaacactgta tgagaaataa taacatattg tttcagctaa taagattcat 2760

tcatttcct 2769

<210> 19
<211> 706
<212> PRT
<213> Arabidopsis thaliana

<400> 19

Met Cys Gly Leu Ala Lys Lys Leu Asp Ile Glu Asp Thr Leu Thr Ser
1 5 10 15

Leu Ser Asp Gln Glu Asn Glu Ser Leu Ala Lys Pro Met Asn Asp Ala
20 25 30

Ala Glu Trp Glu His Ser Phe Ser Ala Leu Leu Glu Phe Ala Ala Asp
35 40 45

Asn Asp Val Glu Gly Phe Arg Arg Gln Leu Ser Asp Val Ser Cys Ile
50 55 60

Asn Gln Met Gly Leu Trp Tyr Arg Arg Gln Arg Phe Val Arg Arg Met
65 70 75 80

Val Leu Glu Gln Arg Thr Pro Leu Met Val Ala Ser Leu Tyr Gly Ser
85 90 95

Leu Asp Val Val Lys Phe Ile Leu Ser Phe Pro Glu Ala Glu Leu Asn
100 105 110

Leu Ser Cys Gly Pro Asp Lys Ser Thr Ala Leu His Cys Ala Ala Ser
115 120 125

Gly Ala Ser Val Asn Ser Leu Asp Val Val Lys Leu Leu Leu Ser Val
130 135 140

Gly Ala Asp Pro Asn Ile Pro Asp Ala His Gly Asn Arg Pro Val Asp
145 150 155 160

Val Leu Val Val Ser Pro His Ala Pro Gly Leu Arg Thr Ile Leu Glu
165 170 175

Glu Ile Leu Lys Lys Asp Glu Ile Ile Ser Glu Asp Leu His Ala Ser
180 185 190

Ser Ser Ser Leu Gly Ser Ser Phe Arg Ser Leu Ser Ser Ser Pro Asp
195 200 205

Asn Gly Ser Ser Leu Leu Ser Leu Asp Ser Val Ser Ser Pro Thr Lys
210 215 220

Pro His Gly Thr Asp Val Thr Phe Ala Ser Glu Lys Lys Glu Tyr Pro
225 230 235 240

Ile Asp Pro Ser Leu Pro Asp Ile Lys Ser Gly Ile Tyr Ser Thr Asp
245 250 255

Glu Phe Arg Met Phe Ser Phe Lys Ile Arg Pro Cys Ser Arg Ala Tyr
260 265 270

Ser His Asp Trp Thr Glu Cys Pro Phe Ala His Pro Gly Glu Asn Ala
275 280 285

Arg Arg Arg Asp Pro Arg Lys Phe His Tyr Thr Cys Val Pro Cys Pro
290 295 300

Asp Phe Lys Lys Gly Ser Cys Lys Gln Gly Asp Met Cys Glu Tyr Ala
305 310 315 320

His Gly Val Phe Glu Cys Trp Leu His Pro Ala Gln Tyr Arg Thr Arg
325 330 335

Leu Cys Lys Asp Gly Met Gly Cys Asn Arg Arg Val Cys Phe Phe Ala
340 345 350

His Ala Asn Glu Glu Leu Arg Pro Leu Tyr Pro Ser Thr Gly Ser Gly
355 360 365

Leu Pro Ser Pro Arg Ala Ser Ser Ala Val Ser Ala Ser Thr Met Asp
370 375 380

Met Ala Ser Val Leu Asn Met Leu Pro Gly Ser Pro Ser Ala Ala Gln
385 390 395 400

His Ser Phe Thr Pro Pro Ile Ser Pro Ser Gly Asn Gly Ser Met Pro
405 410 415

His Ser Ser Met Gly Trp Pro Gln Gln Asn Ile Pro Ala Leu Asn Leu
420 425 430

Pro Gly Ser Asn Ile Gln Leu Ser Arg Leu Arg Ser Ser Leu Asn Ala
435 440 445

Arg Asp Ile Pro Ser Glu Gln Leu Ser Met Leu His Glu Phe Glu Met
450 455 460

Gln Arg Gln Leu Ala Gly Asp Met His Ser Pro Arg Phe Met Asn His
465 470 475 480

Ser Ala Arg Pro Lys Thr Leu Asn Pro Ser Asn Leu Glu Glu Leu Phe
485 490 495

Ser Ala Glu Val Ala Ser Pro Arg Phe Ser Asp Gln Leu Ala Val Ser
500 505 510

Ser Val Leu Ser Pro Ser His Lys Ser Ala Leu Leu Asn Gln Leu Gln
515 520 525

Asn Asn Lys Gln Ser Met Leu Ser Pro Ile Lys Thr Asn Leu Met Ser
530 535 540

Ser Pro Lys Asn Val Glu Gln His Ser Leu Leu Gln Gln Ala Ser Ser
545 550 555 560

Pro Arg Gly Gly Glu Pro Ile Ser Pro Met Asn Ala Arg Met Lys Gln
565 570 575

Gln Leu His Ser Arg Ser Leu Ser Ser Arg Asp Phe Gly Ser Ser Leu
580 585 590

Pro Arg Asp Leu Met Pro Thr Asp Ser Gly Ser Pro Leu Ser Pro Trp
595 600 605

Ser Ser Trp Asp Gln Thr His Gly Ser Lys Val Asp Trp Ser Val Gln
610 615 620

Ser Asp Glu Leu Gly Arg Leu Arg Lys Ser His Ser Leu Ala Asn Asn
625 630 635 640

Pro Asn Arg Glu Ala Asp Val Ser Trp Ala Gln Gln Met Leu Lys Asp
645 650 655

Ser Ser Ser Pro Arg Asn Gly Asn Arg Val Val Asn Met Asn Gly Ala
660 665 670

Arg Pro Leu Thr Gln Gly Gly Ser Ser Val Asn Pro His Asn Ser Asp
675 680 685

Thr Arg Glu Ser Asp Ile Leu Asp Ala Trp Leu Glu Gln Leu His Leu
690 695 700

Asp Arg
705

<210> 20

<211> 2674
<212> DNA
<213> Oryza sativa

<400> 20
tgcgagtcct cctcctcttc tcgtcgccgt gctactctcg ctttctctct ctctctctct 60

cacttgttcc ccaaggcgag aagcagccgc cgccggcgag cgtcgcgggg gaggggaggg 120

aagggaggga ggagcggtgg atccgggctt gattggattg ggtcggattc gattttggat 180

caaccccgga gggcgggagc ggttgctaca gatgcgttga gctttggtta atctatccgg 240

cgagagataa tgggcgagct tgctgatctc gttgtcgtgc cgtcgcagcc gccgctcgcc 300

ggcggccggc gggacaggct ggcggcgctg ctggagctcg cggcggcgga tgatgttgat 360

gggctcaggg gggcgctcgc ggagggaggc gaggaggcgg cggagttggc tgatggggtc 420

gggctgtggt atggtcggag caaggcgtac gaggcgcgca cgccgctgat ggtggcggcg 480

acgtacggca gcgccggggt ggtctcgctg ctggtgggcc tcggcggttg cgtcgacgtc 540

aaccgtcgcc ctggagccga cggcgccacc gcgctccact gcgccgcctc cggtggctcg 600

cgcaacgccg tcgctgttgt caagctgctt ttggccgctg gcgccgatcc ggccaccccc 660

gattccgccg gccgcttccc cgccgacgtc atcctagctc ctccggcttc gccagatgcc 720

cttggcgatc tcgaggtgct cctcggccgc cgccgagcac tcgccgtggc gacctcggtg 780

gcttcaggtt cgtcatcccc tccgctctcg tcctcaccag atgagggcaa caggtcgccc 840

tcgtcgcgtt cgtcgtcgct gtctcccatc actgtggatc gtgggaagaa ggagtatccg 900

gtggatccaa ctctgccgga catcaagagc agcgtgtatg cttcggatga gttccgcatg 960

tttgcgttca aggtccggcc ctgctcccgt gcctactcac acgactggac tgagtgcccg 1020

tttgtgcacc ccggcgagaa cgcccgccgc cgtgatcccc gcaagcaccc atacactgct 1080

gtgccttgcc ccaactttcg ccggcctggt ggctgcccta gcggcgatag ctgtgagttc 1140

tcgcatggcg tgtttgagag ctggctacac ccatcacagt atcgcacaag gctctgcaag 1200

gagggagcag cttgcgcccg tcgcatttgc ttctttgccc atgatgagga tgagctccgc 1260

catgtgcctc acaacagtgg tgccggcctg ctgtctcccc gcgcttcttc atccattgat 1320

atgactgctg cagctgcgct cgggcttctt ccaggttctc ctaccagaca ctttgcaccg 1380

ccgcctgtgt caccatctgc tgggagcaat ggaggagctg ctgctgcgca ttggctccaa 1440

ggcagtaggc tgcgttcttc tttcaatgca agggatgctg ctgttgatga ccttggcatg 1500

ctcctcgaat gggaatcaca ataccttggg gcactctgcc tgccacccag cagccgcccc 1560

caaccacgcc tttcagctgg tctgagtatc aggccaacaa ttgctccatc caatcttgaa 1620

gacatgtatg cttcagacat ggcaatgtct ccgaggttcc ctaatgacca aggtcactca 1680

gtctactcac cagcccacaa atcagccctc ctcaacaagc ttcatcaaca gaagggcctc 1740

ttatcacctg ttaacaccaa cagaatgtac tccccaaggg ctcttgatcc gtcatctttg 1800

```
gcacattctc catttggtgg catgtctccc cggtcccccc gtaccatgga acctacatca   1860
cccctaagtg ctcgtgtagg agcccctgcc acacagcggc cttctgttgg ttcaccacgg   1920
aattccagtg cttggggcac cgtggggtcc ccgatgggta aggttgactg gggtgtcgat   1980
agcgaggagc tagtccgctt gagacgccct gcacaaccag ggtttggaga agatgagaca   2040
gatgtatcat gggtgcagtc actggtaagc aatgctgagc ttaatggcaa gaggggcgaa   2100
gtacaaggca tgcctggtac ttctgcattg atgaacaggc ctgacctgaa caatcagggt   2160
gacttgttgg accagacggt gatcggtgct tggcttgagc agatgcacct ggatcagaag   2220
tgatttccaa gggaagccat gaagtcccaa agtggatgaa gcctttattt tgccaaggtt   2280
atttaccaaa gaatagttgt tggtcctagt aaataataat ttattctttt taattcttga   2340
aatttttggt gggcaaagtc agagatggtg gtcaagttca acaaaacatt tggtcacaga   2400
ttggtagctg aaatcagttc cagagattgg taaacaacct cattacttgg ggtcctaact   2460
agtattcttt tgattagctc agatgagtct ttattttagt gggttaaaat tcatatgttc   2520
cccatggtta ttatgtccat gatctcttcc taacaaaaga gagattataa ttgtccattt   2580
ttcatttatc aatgaatgat tttgttaaaa caatgtaagt tacattctta atttttctc    2640
tgttcaatgg aattaccttc cttggttagt cctc                               2674
```

<210> 21
<211> 657
<212> PRT
<213> Oryza sativa

<400> 21

```
Met Gly Glu Leu Ala Asp Leu Val Val Val Pro Ser Gln Pro Pro Leu
1               5                   10                  15

Ala Gly Gly Arg Arg Asp Arg Leu Ala Ala Leu Leu Glu Leu Ala Ala
            20                  25                  30

Ala Asp Asp Val Asp Gly Leu Arg Gly Ala Leu Ala Glu Gly Gly Glu
        35                  40                  45

Glu Ala Ala Glu Leu Ala Asp Gly Val Gly Leu Trp Tyr Gly Arg Ser
    50                  55                  60

Lys Ala Tyr Glu Ala Arg Thr Pro Leu Met Val Ala Ala Thr Tyr Gly
65                  70                  75                  80

Ser Ala Gly Val Val Ser Leu Leu Val Gly Leu Gly Gly Cys Val Asp
                85                  90                  95

Val Asn Arg Arg Pro Gly Ala Asp Gly Ala Thr Ala Leu His Cys Ala
            100                 105                 110
```

```
Ala Ser Gly Gly Ser Arg Asn Ala Val Ala Val Val Lys Leu Leu Leu
        115                 120                 125

Ala Ala Gly Ala Asp Pro Ala Thr Pro Asp Ser Ala Gly Arg Phe Pro
    130                 135                 140

Ala Asp Val Ile Leu Ala Pro Pro Ala Ser Pro Asp Ala Leu Gly Asp
145                 150                 155                 160

Leu Glu Val Leu Leu Gly Arg Arg Arg Ala Leu Ala Val Ala Thr Ser
                165                 170                 175

Val Ala Ser Gly Ser Ser Ser Pro Pro Leu Ser Ser Ser Pro Asp Glu
            180                 185                 190

Gly Asn Arg Ser Pro Ser Ser Arg Ser Ser Ser Leu Ser Pro Ile Thr
        195                 200                 205

Val Asp Arg Gly Lys Lys Glu Tyr Pro Val Asp Pro Thr Leu Pro Asp
    210                 215                 220

Ile Lys Ser Ser Val Tyr Ala Ser Asp Glu Phe Arg Met Phe Ala Phe
225                 230                 235                 240

Lys Val Arg Pro Cys Ser Arg Ala Tyr Ser His Asp Trp Thr Glu Cys
                245                 250                 255

Pro Phe Val His Pro Gly Glu Asn Ala Arg Arg Arg Asp Pro Arg Lys
            260                 265                 270

His Pro Tyr Thr Ala Val Pro Cys Pro Asn Phe Arg Arg Pro Gly Gly
        275                 280                 285

Cys Pro Ser Gly Asp Ser Cys Glu Phe Ser His Gly Val Phe Glu Ser
    290                 295                 300

Trp Leu His Pro Ser Gln Tyr Arg Thr Arg Leu Cys Lys Glu Gly Ala
305                 310                 315                 320

Ala Cys Ala Arg Arg Ile Cys Phe Phe Ala His Asp Glu Asp Glu Leu
                325                 330                 335

Arg His Val Pro His Asn Ser Gly Ala Gly Leu Leu Ser Pro Arg Ala
            340                 345                 350

Ser Ser Ser Ile Asp Met Thr Ala Ala Ala Ala Leu Gly Leu Leu Pro
        355                 360                 365
```

```
Gly Ser Pro Thr Arg His Phe Ala Pro Pro Pro Val Ser Pro Ser Ala
    370                 375                 380

Gly Ser Asn Gly Gly Ala Ala Ala Ala His Trp Leu Gln Gly Ser Arg
385                 390                 395                 400

Leu Arg Ser Ser Phe Asn Ala Arg Asp Ala Ala Val Asp Asp Leu Gly
                405                 410                 415

Met Leu Leu Glu Trp Glu Ser Gln Tyr Leu Gly Ala Leu Cys Leu Pro
            420                 425                 430

Pro Ser Ser Arg Pro Gln Pro Arg Leu Ser Ala Gly Leu Ser Ile Arg
        435                 440                 445

Pro Thr Ile Ala Pro Ser Asn Leu Glu Asp Met Tyr Ala Ser Asp Met
    450                 455                 460

Ala Met Ser Pro Arg Phe Pro Asn Asp Gln Gly His Ser Val Tyr Ser
465                 470                 475                 480

Pro Ala His Lys Ser Ala Leu Leu Asn Lys Leu His Gln Gln Lys Gly
                485                 490                 495

Leu Leu Ser Pro Val Asn Thr Asn Arg Met Tyr Ser Pro Arg Ala Leu
            500                 505                 510

Asp Pro Ser Ser Leu Ala His Ser Pro Phe Gly Gly Met Ser Pro Arg
        515                 520                 525

Ser Pro Arg Thr Met Glu Pro Thr Ser Pro Leu Ser Ala Arg Val Gly
    530                 535                 540

Ala Pro Ala Thr Gln Arg Pro Ser Val Gly Ser Pro Arg Asn Ser Ser
545                 550                 555                 560

Ala Trp Gly Thr Val Gly Ser Pro Met Gly Lys Val Asp Trp Gly Val
                565                 570                 575

Asp Ser Glu Glu Leu Val Arg Leu Arg Arg Pro Ala Gln Pro Gly Phe
            580                 585                 590

Gly Glu Asp Glu Thr Asp Val Ser Trp Val Gln Ser Leu Val Ser Asn
        595                 600                 605

Ala Glu Leu Asn Gly Lys Arg Gly Glu Val Gln Gly Met Pro Gly Thr
    610                 615                 620

Ser Ala Leu Met Asn Arg Pro Asp Leu Asn Asn Gln Gly Asp Leu Leu
```

625 630 635 640

Asp Gln Thr Val Ile Gly Ala Trp Leu Glu Gln Met His Leu Asp Gln
645 650 655

Lys

<210> 22
<211> 2223
<212> DNA
<213> Arabidopsis thaliana

<400> 22
ttcttcaaaa accccaacca cttcttctcc ccaaaaacct ccaaagtttc aatctttact 60

tctctctttt tctccaagtt atcttctttt ctaggaagag atatgtgcgg tgcaaagagc 120

aacctttgct catctaaaac cctaacagaa gtcgaattca tgaggcagaa atcagaagac 180

ggagcttccg ccacgtgtct cctcgaattc gccgcctgtg atgatctttc atcgtttaag 240

agagagatcg aagagaatcc atcggtggag attgatgagt cagggttttg gtattgcaga 300

cgggtcgggt ctaagaagat gggttttgaa gaaagaacac cacttatggt tgctgctatg 360

tatggaagca tggaagtgtt gaattacata attgccacag gaagatccga tgtgaacaga 420

gtttgcagtg acgagaaagt cactgctctt cactgtgcag tttctggctg ttctgtttct 480

atcgttgaga tcatcaagat cttgcttgat gcttctgctt cacctaattg tgttgacgct 540

aatgggaaca aaccggttga tttgttggct aaagattctc ggtttgttcc taaccagagt 600

agaaaggcgg ttgaggtttt actgaccggg attcatggtt cggttatgga agaagaggag 660

gaggaactga agagtgttgt gactaagtat ccagctgatg catcacttcc tgatattaac 720

gaaggtgttt atggaactga tgattttagg atgtttagct ttaaggttaa gccatgttct 780

agggcttatt cacatgattg gactgaatgt ccttttgttc atcctggtga gaatgcaagg 840

aggagagatc ctaggaagta tccttacact tgtgtgcctt gtcccgagtt tcgtaaaggg 900

tcttgtccta aaggagattc gtgtgagtac gcgcacggtg ttttcgagtc ttggcttcac 960

ccggcgcagt ataggacacg gctttgcaaa gatgagactg gttgtgctag gagagtttgt 1020

ttctttgctc atagacggga tgagttaaga ccggttaatg cttctactgg ttctgcaatg 1080

gtttcaccaa ggtcgtctaa tcagtctcct gagatgtctg ttatgtctcc tttgacgctg 1140

ggatcatcgc caatgaactc tcctatggct aatggtgttc ctttgtctcc aagaaatggt 1200

ggtttatggc agaacagagt taatagcctt acaccaccac cgttgcagct taatggtagc 1260

agattgaagt cgactttgag tgctagagat atggatatgg agatggaact taggtttcgc 1320

ggtttggata accggagact tggtgatctc aagccatcca acctcgaaga gactttcgga 1380

tcatatgact cagcttctgt gatgcaactt caatcaccaa gcaggcattc tcagatgaac 1440

cactatccgt cttcacctgt gaggcagcct cctcctcatg gattcgaatc ttcagcagcc 1500

atggcagctg cagtgatgaa tgcaagatcc tcagcgtttg cgaaacgcag cttgagtttc 1560

aaaccagctc cagtagcttc taatgtctcc gattggggat caccaaatgg gaagcttgag 1620

tggggaatgc aaagagatga gctgaacaag ttgaggagaa gtgcctcctt cggcattcat 1680

ggaaacaaca acaacagtgt gtcacgccct gctagagact acagtgacga gccagatgtg 1740

tcgtgggtga actcactggt gaaagagaat gcaccagaga gagtgaatga gagggttggg 1800

aatacggtga atggtgcagc gagtagagac aagtttaagc tgccgtcgtg ggcagagcaa 1860

atgtatatag accatgagca gcagattgtg gcataagaag cagaaagaaa gatgtgggat 1920

ttatattgct tttgtcttct gggcctctct acacagaatc taacaaatct ggcaataatt 1980

ctttgatttg tgtttgaccc atagtttggt tactagtata tgttttttta tgttcttttt 2040

ttctttgtca ttctcttgtc cttcgtgaca ctatgtaatg attaaaagca aataattgat 2100

gcatgagttc aaatgttctt tgaaggatcc atcttattag ctttgtaatt gttgtgatat 2160

cttaatctta ttggttacgt atttcaagtg ctttagaaaa aatgggccta agagattttg 2220

ggg 2223

<210> 23
<211> 597
<212> PRT
<213> Arabidopsis thaliana

<400> 23

Met Cys Gly Ala Lys Ser Asn Leu Cys Ser Ser Lys Thr Leu Thr Glu
1               5                   10                  15

Val Glu Phe Met Arg Gln Lys Ser Glu Asp Gly Ala Ser Ala Thr Cys
            20                  25                  30

Leu Leu Glu Phe Ala Ala Cys Asp Asp Leu Ser Ser Phe Lys Arg Glu
        35                  40                  45

Ile Glu Glu Asn Pro Ser Val Glu Ile Asp Glu Ser Gly Phe Trp Tyr
    50                  55                  60

Cys Arg Arg Val Gly Ser Lys Lys Met Gly Phe Glu Glu Arg Thr Pro
65                  70                  75                  80

Leu Met Val Ala Ala Met Tyr Gly Ser Met Glu Val Leu Asn Tyr Ile
                85                  90                  95

Ile Ala Thr Gly Arg Ser Asp Val Asn Arg Val Cys Ser Asp Glu Lys
            100                 105                 110

Val Thr Ala Leu His Cys Ala Val Ser Gly Cys Ser Val Ser Ile Val

115 120 125

Glu Ile Ile Lys Ile Leu Leu Asp Ala Ser Ala Ser Pro Asn Cys Val
130 135 140

Asp Ala Asn Gly Asn Lys Pro Val Asp Leu Leu Ala Lys Asp Ser Arg
145 150 155 160

Phe Val Pro Asn Gln Ser Arg Lys Ala Val Glu Val Leu Leu Thr Gly
165 170 175

Ile His Gly Ser Val Met Glu Glu Glu Glu Glu Glu Leu Lys Ser Val
180 185 190

Val Thr Lys Tyr Pro Ala Asp Ala Ser Leu Pro Asp Ile Asn Glu Gly
195 200 205

Val Tyr Gly Thr Asp Asp Phe Arg Met Phe Ser Phe Lys Val Lys Pro
210 215 220

Cys Ser Arg Ala Tyr Ser His Asp Trp Thr Glu Cys Pro Phe Val His
225 230 235 240

Pro Gly Glu Asn Ala Arg Arg Arg Asp Pro Arg Lys Tyr Pro Tyr Thr
245 250 255

Cys Val Pro Cys Pro Glu Phe Arg Lys Gly Ser Cys Pro Lys Gly Asp
260 265 270

Ser Cys Glu Tyr Ala His Gly Val Phe Glu Ser Trp Leu His Pro Ala
275 280 285

Gln Tyr Arg Thr Arg Leu Cys Lys Asp Glu Thr Gly Cys Ala Arg Arg
290 295 300

Val Cys Phe Phe Ala His Arg Arg Asp Glu Leu Arg Pro Val Asn Ala
305 310 315 320

Ser Thr Gly Ser Ala Met Val Ser Pro Arg Ser Ser Asn Gln Ser Pro
325 330 335

Glu Met Ser Val Met Ser Pro Leu Thr Leu Gly Ser Ser Pro Met Asn
340 345 350

Ser Pro Met Ala Asn Gly Val Pro Leu Ser Pro Arg Asn Gly Gly Leu
355 360 365

Trp Gln Asn Arg Val Asn Ser Leu Thr Pro Pro Pro Leu Gln Leu Asn
370 375 380

Gly Ser Arg Leu Lys Ser Thr Leu Ser Ala Arg Asp Met Asp Met Glu
385 390 395 400

Met Glu Leu Arg Phe Arg Gly Leu Asp Asn Arg Arg Leu Gly Asp Leu
405 410 415

Lys Pro Ser Asn Leu Glu Glu Thr Phe Gly Ser Tyr Asp Ser Ala Ser
420 425 430

Val Met Gln Leu Gln Ser Pro Ser Arg His Ser Gln Met Asn His Tyr
435 440 445

Pro Ser Ser Pro Val Arg Gln Pro Pro Pro His Gly Phe Glu Ser Ser
450 455 460

Ala Ala Met Ala Ala Ala Val Met Asn Ala Arg Ser Ser Ala Phe Ala
465 470 475 480

Lys Arg Ser Leu Ser Phe Lys Pro Ala Pro Val Ala Ser Asn Val Ser
485 490 495

Asp Trp Gly Ser Pro Asn Gly Lys Leu Glu Trp Gly Met Gln Arg Asp
500 505 510

Glu Leu Asn Lys Leu Arg Arg Ser Ala Ser Phe Gly Ile His Gly Asn
515 520 525

Asn Asn Asn Ser Val Ser Arg Pro Ala Arg Asp Tyr Ser Asp Glu Pro
530 535 540

Asp Val Ser Trp Val Asn Ser Leu Val Lys Glu Asn Ala Pro Glu Arg
545 550 555 560

Val Asn Glu Arg Val Gly Asn Thr Val Asn Gly Ala Ala Ser Arg Asp
565 570 575

Lys Phe Lys Leu Pro Ser Trp Ala Glu Gln Met Tyr Ile Asp His Glu
580 585 590

Gln Gln Ile Val Ala
595

<210> 24
<211> 1761
<212> DNA
<213> Arabidopsis thaliana

<400> 24
atggaaaaag atagtattat gtgcagtgga ccaaagagca atctctgctc ttcaagaacc 60

ttaacagaaa tcgaatcaag gcaaaaggaa gaagaaacaa tgcttctcct cgaattcgct 120
gcttgtgatg atcttgactc gttcaagaga gaggttgaag agaaagggct tgatttggat 180
gagtcagggt tatggtattg cagacgtgtc ggttctaaga agatgggtct tgaagaaaga 240
acacctttaa tggttgcagc tatgtatgga agcataaagg ttttgacttt catcgtttcc 300
actggaaaat ctgatgtgaa cagagcttgt ggtgaagaga gagttactcc gcttcactgt 360
gctgttgctg gctgttctgt gaatatgatt gaagtcatca atgtcttgct tgatgcttct 420
gctttggtta actctgttga tgctaatggg aatcaacctt tggatgtgtt tgttcgagtt 480
tcgaggtttg tggctagtcc gaggaggaaa gcggttgagt tgttgctgag aggaggaggt 540
gttggaggat tgatcgatga ggcggttgaa gaagagatca agattgtctc taagtatcca 600
gctgatgctt ctttaccgga tataaacgaa ggggtttatg gaagtgatga gtttaggatg 660
tatagcttta aggttaagcc atgttctagg gcttattctc atgattggac cgagtgtgct 720
tttgttcatc cgggagaaaa tgcgaggagg agagatccga ggaagtatcc ttacacttgt 780
gtcccctgtc ccgagttccg taaaggatca tgcccgaaag gagattcttg cgagtatgct 840
cacggggttt tcgagtcgtg gcttcacccc gcgcagtata aaacccggct ttgtaaagat 900
gaaacgggtt gtgcaaggaa agtttgtttc tttgctcata aacgcgaaga gatgagacct 960
gttaatgctt caactggctc tgccgtggct cagtctccgt ttagcagctt ggagatgatg 1020
ccagggttgt ctcctcttgc ttattcttca ggagtttcga ctcctccggt ttctccaatg 1080
gctaatggtg ttccttcctc tccaagaaac ggcggatcat ggcagaacag agtcaatacc 1140
cttactccac cggctttgca gctcaatggt ggaagcagat tgaagtccac actgagcgct 1200
agagatatcg atatggagat ggagatggaa ttgagactcc gcggttttgg caacaatgtg 1260
gaagagacgt tcgggtctta tgtttcctct ccaagtagga attctcaaat gggtcaaaac 1320
atgaaccaac attatccatc ttccccggtg agacaaccgc catctcaaca cgggttcgaa 1380
tcttcagcag ctgcagcggt tgcagtgatg aaagcgagat caaccgcctt tgcgaaacgt 1440
agcttgagct tcaaaccagc tactcaagca gcaccacagt cgaatctctc ggattgggga 1500
tctccaaacg ggaagctgga atggggaatg aaaggagaag agctgaataa gatgagaaga 1560
agtgtttcct ttggaatcca tggaaacaac aacaataacg cagctagaga ctacagggac 1620
gagccagatg tgtcatgggt taactcttta gttaaagaca gtactgtggt gtctgagaga 1680
agctttggaa tgaatgagag ggttcggata atgtcgtggg ctgagcaaat gtacagagag 1740
aaggagcaga ctgtggtgta a 1761

<210> 25
<211> 586
<212> PRT
<213> Arabidopsis thaliana

<400> 25

```
Met Glu Lys Asp Ser Ile Met Cys Ser Gly Pro Lys Ser Asn Leu Cys
1               5                   10                  15

Ser Ser Arg Thr Leu Thr Glu Ile Glu Ser Arg Gln Lys Glu Glu Glu
            20                  25                  30

Thr Met Leu Leu Leu Glu Phe Ala Ala Cys Asp Asp Leu Asp Ser Phe
        35                  40                  45

Lys Arg Glu Val Glu Glu Lys Gly Leu Asp Leu Asp Glu Ser Gly Leu
    50                  55                  60

Trp Tyr Cys Arg Arg Val Gly Ser Lys Lys Met Gly Leu Glu Glu Arg
65                  70                  75                  80

Thr Pro Leu Met Val Ala Ala Met Tyr Gly Ser Ile Lys Val Leu Thr
                85                  90                  95

Phe Ile Val Ser Thr Gly Lys Ser Asp Val Asn Arg Ala Cys Gly Glu
            100                 105                 110

Glu Arg Val Thr Pro Leu His Cys Ala Val Ala Gly Cys Ser Val Asn
        115                 120                 125

Met Ile Glu Val Ile Asn Val Leu Leu Asp Ala Ser Ala Leu Val Asn
    130                 135                 140

Ser Val Asp Ala Asn Gly Asn Gln Pro Leu Asp Val Phe Val Arg Val
145                 150                 155                 160

Ser Arg Phe Val Ala Ser Pro Arg Arg Lys Ala Val Glu Leu Leu Leu
                165                 170                 175

Arg Gly Gly Gly Val Gly Gly Leu Ile Asp Glu Ala Val Glu Glu Glu
            180                 185                 190

Ile Lys Ile Val Ser Lys Tyr Pro Ala Asp Ala Ser Leu Pro Asp Ile
        195                 200                 205

Asn Glu Gly Val Tyr Gly Ser Asp Glu Phe Arg Met Tyr Ser Phe Lys
    210                 215                 220

Val Lys Pro Cys Ser Arg Ala Tyr Ser His Asp Trp Thr Glu Cys Ala
225                 230                 235                 240

Phe Val His Pro Gly Glu Asn Ala Arg Arg Arg Asp Pro Arg Lys Tyr
                245                 250                 255
```

```
Pro Tyr Thr Cys Val Pro Cys Pro Glu Phe Arg Lys Gly Ser Cys Pro
            260                 265                 270

Lys Gly Asp Ser Cys Glu Tyr Ala His Gly Val Phe Glu Ser Trp Leu
        275                 280                 285

His Pro Ala Gln Tyr Lys Thr Arg Leu Cys Lys Asp Glu Thr Gly Cys
    290                 295                 300

Ala Arg Lys Val Cys Phe Phe Ala His Lys Arg Glu Glu Met Arg Pro
305                 310                 315                 320

Val Asn Ala Ser Thr Gly Ser Ala Val Ala Gln Ser Pro Phe Ser Ser
                325                 330                 335

Leu Glu Met Met Pro Gly Leu Ser Pro Leu Ala Tyr Ser Ser Gly Val
            340                 345                 350

Ser Thr Pro Pro Val Ser Pro Met Ala Asn Gly Val Pro Ser Ser Pro
        355                 360                 365

Arg Asn Gly Gly Ser Trp Gln Asn Arg Val Asn Thr Leu Thr Pro Pro
    370                 375                 380

Ala Leu Gln Leu Asn Gly Gly Ser Arg Leu Lys Ser Thr Leu Ser Ala
385                 390                 395                 400

Arg Asp Ile Asp Met Glu Met Glu Met Glu Leu Arg Leu Arg Gly Phe
                405                 410                 415

Gly Asn Asn Val Glu Glu Thr Phe Gly Ser Tyr Val Ser Ser Pro Ser
            420                 425                 430

Arg Asn Ser Gln Met Gly Gln Asn Met Asn Gln His Tyr Pro Ser Ser
        435                 440                 445

Pro Val Arg Gln Pro Pro Ser Gln His Gly Phe Glu Ser Ser Ala Ala
    450                 455                 460

Ala Ala Val Ala Val Met Lys Ala Arg Ser Thr Ala Phe Ala Lys Arg
465                 470                 475                 480

Ser Leu Ser Phe Lys Pro Ala Thr Gln Ala Ala Pro Gln Ser Asn Leu
                485                 490                 495

Ser Asp Trp Gly Ser Pro Asn Gly Lys Leu Glu Trp Gly Met Lys Gly
            500                 505                 510
```

Glu Glu Leu Asn Lys Met Arg Arg Ser Val Ser Phe Gly Ile His Gly
515 520 525

Asn Asn Asn Asn Asn Ala Ala Arg Asp Tyr Arg Asp Glu Pro Asp Val
530 535 540

Ser Trp Val Asn Ser Leu Val Lys Asp Ser Thr Val Val Ser Glu Arg
545 550 555 560

Ser Phe Gly Met Asn Glu Arg Val Arg Ile Met Ser Trp Ala Glu Gln
565 570 575

Met Tyr Arg Glu Lys Glu Gln Thr Val Val
580 585

<210> 26
<211> 2709
<212> DNA
<213> Eucalyptus grandis

<400> 26
cttctgaaag ctttttgact taagacgaga gagaaggaga gaaggtcccc ctcctcgtcc 60

tcgtcccccc gtggattttg aagaagaaaa gtcgcacctt ccttctcctt tcccactcct 120

ccctctgctc gaagcttttc tcttccgcag aattacataa aaacctcgac tttgcgcatc 180

attccgattc acctcacacc ttcactttcc cactcgaggt ctccccccctc ttttcctagc 240

tctttccctt tccctccctc tctctcgaga atcgccgcat ttggaggagc tccaatctgc 300

tttgctttgc tttgctctct tcttgctcgg ttccccctca taaggagtcg attatgtgca 360

acggttcttc gaagggtaaa cttttcccct cgagtatggg catggagggc gaattccaca 420

acaaggatgg cgaagcaccc cgtaaatgct ctgccttgct tgaattggca gcctcggacg 480

atctctcgtc gttcaaaagt gaagtggaag agaagggctg cgacgttgat gaggccagct 540

tttggtatgg taggagaatc gggtcgaaga agatgggttt tgaagagagg actccattga 600

tgatctctgc tttgtttgga agcaccaagg tcttgaaata cataatcgag accgccagag 660

ctgatgtcaa caggtcttgt gggtccgaca aggtggccgc cctccattgc gcagccgcgg 720

gtgggtccag ttcttcactt gaaattgtga agctcttgat tgaggcctca gcggatatta 780

attctgtaga tggcaatgga aataggccca tcgacgtgct tgccccggca gggaagtctc 840

gctgcaattc cagaaataag tttgttagat cgttgctgaa aggtgaaaac tatgtcgtgg 900

aaggtgacca atcctttgac atagaaggag aggagaagct agtcgctctt ccaaaggagg 960

gaggcgagaa gaaagagtat cctgttgatg tctctctacc tgacataaac aatgggttct 1020

acagtaccga tgagttccgg atgtatgctt tcaaggtgaa gccttgctcg agggcttact 1080

cccacgactg gaccgagtgc ccgtttgtgc accctgggga gaacgcgagg aggagggacc 1140

cacgcaagta cccttacagc tgtgtccctt gtcctgagtt tcgcaagggt tcgtgcgtaa 1200

gggggggatgc ttgtgagtat gctcatggag tctttgagtc gtggcttcac ccagcgcaat 1260

accgaacccg gctgtgcaag gatgaaactg gttgtactcg caaagtttgc ttctttgctc 1320

acaagtccga agaattgcgt cccgtgtatg cttccacagg ttctgctatg ccctcaccca 1380

agtccttttc agctaatgcc ctagacatga caaccctgag ccccttatcc cttaattcac 1440

catctctgcc tttgcctgct acttccacgc cccccatgtc acctttggct gcctcatctt 1500

cacccaaggg catgaacttg tggcataaca aaattaacct gaccccacca agcctgcagc 1560

ttcctggcag ccggctgaag acggctatga gtgcgcggga cttcgatttt gagttggaat 1620

ttcttgggct ggaaaagcaa gcttctcagc ggcagcaact gatagaagag atttctcgtc 1680

tctcatcgcc ctctcatatg tggaactcgg aatttggcag aaccgcagag ctgaagccca 1740

ctaaccttga tgatgcgttt ggatctcttg acacttctct tttgtctccg ttgcaggggt 1800

cgtcgatgaa aacatcgact cctacccagt tgcaatcccc cacagggctt aaaatttcga 1860

atttgaacca actccgtgcg agctacccgt ctagcagctt gtcgtcctct cctgtgagga 1920

agacctcttc ttttgggttc gactcatcca gtgcagttgc tgcagcagtc atgaactcac 1980

ggtctgctgc tatgacgaag cggagccaga gcttcattga ccgtggagca gtgggtcaac 2040

ggtctggact cattggacct gctaattctg ctcctaggat gtccaacctt tcggactggg 2100

gctcgcctga tgggaagttg gattggggtg ttcaagggga cgagctcaac aagcttagga 2160

agtccgcttc cttcggcttt agaaacaaca gtatggcgaa cccaaacaac gtggcgtctc 2220

ccagtgctga tgagccggac gtgtcgtggg ttggttcatt ggtgaaggat gtggctccgc 2280

ccgaagggta tccacagtat ctgtacatag aacaggagca gatggtggca taactaaagc 2340

gaagagcacc acacgaactc tctcctgatg gcttaagatg acttgtttga cattctttat 2400

attcttacaa acagcgcgtt cttaggagtt agctggagga aagaaggaaa cggtattgag 2460

tttgagattc aggctcttag ctggacagcg aaaatttggg gaaggaagag aatttggttt 2520

cttgcccaac ttagataatg atgcttttga aggcttaaaa gaaagatgaa ggcaaacatt 2580

cttttgttag tattgtatta ttgttttaat ttttcatccc ctctgtcggg gtgtggtggg 2640

tgtcgatgtt tctttcatca gtaaaatata taatgaggtt tactcatcta ttttctacta 2700

aaaaaaaaa 2709

<210> 27
<211> 659
<212> PRT
<213> Eucalyptus grandis

<400> 27

Met Cys Asn Gly Ser Ser Lys Gly Lys Leu Phe Pro Ser Ser Met Gly
1 5 10 15

```
Met Glu Gly Glu Phe His Asn Lys Asp Gly Glu Ala Pro Arg Lys Cys
            20                  25                  30

Ser Ala Leu Leu Glu Leu Ala Ala Ser Asp Asp Leu Ser Ser Phe Lys
        35                  40                  45

Ser Glu Val Glu Glu Lys Gly Cys Asp Val Asp Glu Ala Ser Phe Trp
    50                  55                  60

Tyr Gly Arg Arg Ile Gly Ser Lys Lys Met Gly Phe Glu Glu Arg Thr
65                  70                  75                  80

Pro Leu Met Ile Ser Ala Leu Phe Gly Ser Thr Lys Val Leu Lys Tyr
                85                  90                  95

Ile Ile Glu Thr Ala Arg Ala Asp Val Asn Arg Ser Cys Gly Ser Asp
            100                 105                 110

Lys Val Ala Ala Leu His Cys Ala Ala Ala Gly Gly Ser Ser Ser Ser
        115                 120                 125

Leu Glu Ile Val Lys Leu Leu Ile Glu Ala Ser Ala Asp Ile Asn Ser
    130                 135                 140

Val Asp Gly Asn Gly Asn Arg Pro Ile Asp Val Leu Ala Pro Ala Gly
145                 150                 155                 160

Lys Ser Arg Cys Asn Ser Arg Asn Lys Phe Val Arg Ser Leu Leu Lys
                165                 170                 175

Gly Glu Asn Tyr Val Val Glu Gly Asp Gln Ser Phe Asp Ile Glu Gly
            180                 185                 190

Glu Glu Lys Leu Val Ala Leu Pro Lys Glu Gly Gly Glu Lys Lys Glu
        195                 200                 205

Tyr Pro Val Asp Val Ser Leu Pro Asp Ile Asn Asn Gly Phe Tyr Ser
    210                 215                 220

Thr Asp Glu Phe Arg Met Tyr Ala Phe Lys Val Lys Pro Cys Ser Arg
225                 230                 235                 240

Ala Tyr Ser His Asp Trp Thr Glu Cys Pro Phe Val His Pro Gly Glu
                245                 250                 255

Asn Ala Arg Arg Arg Asp Pro Arg Lys Tyr Pro Tyr Ser Cys Val Pro
            260                 265                 270

Cys Pro Glu Phe Arg Lys Gly Ser Cys Val Arg Gly Asp Ala Cys Glu
```

275 280 285

Tyr Ala His Gly Val Phe Glu Ser Trp Leu His Pro Ala Gln Tyr Arg
290 295 300

Thr Arg Leu Cys Lys Asp Glu Thr Gly Cys Thr Arg Lys Val Cys Phe
305 310 315 320

Phe Ala His Lys Ser Glu Glu Leu Arg Pro Val Tyr Ala Ser Thr Gly
325 330 335

Ser Ala Met Pro Ser Pro Lys Ser Phe Ser Ala Asn Ala Leu Asp Met
340 345 350

Thr Thr Leu Ser Pro Leu Ser Leu Asn Ser Pro Ser Leu Pro Leu Pro
355 360 365

Ala Thr Ser Thr Pro Pro Met Ser Pro Leu Ala Ala Ser Ser Ser Pro
370 375 380

Lys Gly Met Asn Leu Trp His Asn Lys Ile Asn Leu Thr Pro Pro Ser
385 390 395 400

Leu Gln Leu Pro Gly Ser Arg Leu Lys Thr Ala Met Ser Ala Arg Asp
405 410 415

Phe Asp Phe Glu Leu Glu Phe Leu Gly Leu Glu Lys Gln Ala Ser Gln
420 425 430

Arg Gln Gln Leu Ile Glu Glu Ile Ser Arg Leu Ser Ser Pro Ser His
435 440 445

Met Trp Asn Ser Glu Phe Gly Arg Thr Ala Glu Leu Lys Pro Thr Asn
450 455 460

Leu Asp Asp Ala Phe Gly Ser Leu Asp Thr Ser Leu Leu Ser Pro Leu
465 470 475 480

Gln Gly Ser Ser Met Lys Thr Ser Thr Pro Thr Gln Leu Gln Ser Pro
485 490 495

Thr Gly Leu Lys Ile Ser Asn Leu Asn Gln Leu Arg Ala Ser Tyr Pro
500 505 510

Ser Ser Ser Leu Ser Ser Ser Pro Val Arg Lys Thr Ser Ser Phe Gly
515 520 525

Phe Asp Ser Ser Ser Ala Val Ala Ala Ala Val Met Asn Ser Arg Ser
530 535 540

Ala Ala Met Thr Lys Arg Ser Gln Ser Phe Ile Asp Arg Gly Ala Val
545 550 555 560

Gly Gln Arg Ser Gly Leu Ile Gly Pro Ala Asn Ser Ala Pro Arg Met
565 570 575

Ser Asn Leu Ser Asp Trp Gly Ser Pro Asp Gly Lys Leu Asp Trp Gly
580 585 590

Val Gln Gly Asp Glu Leu Asn Lys Leu Arg Lys Ser Ala Ser Phe Gly
595 600 605

Phe Arg Asn Asn Ser Met Ala Asn Pro Asn Asn Val Ala Ser Pro Ser
610 615 620

Ala Asp Glu Pro Asp Val Ser Trp Val Gly Ser Leu Val Lys Asp Val
625 630 635 640

Ala Pro Pro Glu Gly Tyr Pro Gln Tyr Leu Tyr Ile Glu Gln Glu Gln
645 650 655

Met Val Ala

<210> 28
<211> 2518
<212> DNA
<213> Eucalyptus grandis

<400> 28
tctccttcga gtttctttct tcactagaat tcgctcccga gtctgttgtt gctcgtgtag 60

ttttgcttac tccgtccttc gtttagctcg ctgaccagcg cggagctagg agcggtcgct 120

aaaggattac tcgtacaaaa cgtaaactca gctctgccaa ttttcccatg gagggggaat 180

cttacttcga gaaagatgaa aaatattcta attgctcaat cttgctcgaa ttatctgctt 240

cggacgatct cccagctttt gaaaggaaag cgaaagagaa gggctgtaac attgatggtg 300

ctagcttctg gtacggtaga agaattggct caaggaagat gggtcttgaa gagaggactc 360

ctctcatggt ggcttccttg tttggaagct ctagggttgt gaagtacatt ctcgaatctg 420

gcaaagtcga tgtaaatagg gcttgtggtt cggacaaggt cactgccctt cactgtgctg 480

ttgccagtgg ctctgcttct gcggtggagg ttgtcaagct cttgcttcac gcatctgccg 540

atgctaattg cattgatggc aatggaaaga agccaattga tgtgatagcc cttccattaa 600

agtcacgcgg cgattcaagg aggaagctga tggagctgtt gctgaaaggc gataattctg 660

atggggaatt tgaatcccac gaggagaagc cgattgccgc accgcaagca tccaaagagg 720

gaagcgaaaa gaaagagtat caatttcctg ttgatatctc tctgcctgac ataaatgttg 780

ggatttacag tactgatgag ttcagaatgt atgctttcaa agtaaagcct tgctcgcggg 840

catactccca tgactggaca gagtgcccat ttgttcatcc tggcgagaat gcgaggaggc 900

gggaccctcg caagtacccc tacagctgcg tcccttgccc tgaatttcgg aagggatctt 960

gccaaaaggg tgactcctgt gagtacgcgc acggcgtatt tgagtcgtgg cttcatcctg 1020

cacagtatag aacaagactg tgcaaggatg agactggatg tgctcgcaaa gtttgtttct 1080

ttgctcacaa gcccgaagaa ttaaggcctg tctatgcttc gacgggatca gctatgcctt 1140

ccccaaaatc ctactcatca agtgggctgg acatgtccac attgagtcct ctctcaatca 1200

gttctccgtc agcatcgttg cctgttactt caacagcacc catgtctcct cttgcagcct 1260

cgtcatctcc gatgtctgtg aacatgtggc agagcaaggc taacaagctc tccccgccaa 1320

tgctgcagct ctcaggtagt aggctgaaga ctgctttgag tgctagggac ttggacctgg 1380

agatggaatt gcgtggtcta gagagtcaga tggccactca acagcatcag ttgatggaag 1440

agatatctcg tctctcctca ccatcatcct gctttagtag taggattggg gaagtgaaac 1500

ccactaacct cgatgacgtt tttgggtctc cggatcctgc tttgctgcct caattgcagg 1560

ggctgtcaag accttcaaca ccaagccagt tgcaatctcc aactgggctt cagatgcgcc 1620

agaatgcaac ccagtttcgt ggggcgtacc agagcaatgc aaatgcattg tcatctccag 1680

caatgaagca ggcaccttct tatgggtttg actcatctag tgcagttgca gcagcggtga 1740

tgaattcgag gtcagccgct tttgcgaagc ggagtcagag ttttatcgac aggggaatgg 1800

cgtgccctgg aattgccaat tcttccccta tgatgtcttc agctatgtcg agctggagct 1860

cacctcatgg gaaattggat tggggcgtcc aaggagatga gttgaatagg ctgaggaaag 1920

ctgcttcctt taagatgaga agcagcaccg gagcaggtgc taatactgtc tcggcagcag 1980

ccatggctga tgagccagat atttcttggg tcagttcatt ggttaaggac gtgccttctg 2040

cggaggacgc gatgttcgct gcagagaaag gacagcgcac ttatgggaaa gacatccgcg 2100

aaaggattac cccatgggtg gagcagctgt acagagaagt gccacggatg gcgatgtaag 2160

attgccactg caagtcggat gccttagtat gctgactaat tgatattctt tgcatttgtt 2220

ttgaggcatt tggtagccat tagatacgag aaaaggccaa gcagcaggtg gtgtcttggc 2280

aaggaatagg atgcacatag tctgttatcg agtagaatag acttgggaac aatggttata 2340

gccaaatgtt aaaagttatg atattctttt ccaattcttt ctcttcctca tagtaggttt 2400

ctcaccaagt cttttagtga gagcctgcgg gatgtactat atgtttccct tatgtaacgt 2460

ctcttcgttg aaagaaatgg ctttataata taaagcatca agttttttaa aaaaaaa 2518

<210> 29
<211> 663
<212> PRT
<213> Eucalyptus grandis

<400> 29

Met Glu Gly Glu Ser Tyr Phe Glu Lys Asp Glu Lys Tyr Ser Asn Cys
1 5 10 15

Ser Ile Leu Leu Glu Leu Ser Ala Ser Asp Asp Leu Pro Ala Phe Glu
20 25 30

Arg Lys Ala Lys Glu Lys Gly Cys Asn Ile Asp Gly Ala Ser Phe Trp
35 40 45

Tyr Gly Arg Arg Ile Gly Ser Arg Lys Met Gly Leu Glu Glu Arg Thr
50 55 60

Pro Leu Met Val Ala Ser Leu Phe Gly Ser Ser Arg Val Val Lys Tyr
65 70 75 80

Ile Leu Glu Ser Gly Lys Val Asp Val Asn Arg Ala Cys Gly Ser Asp
85 90 95

Lys Val Thr Ala Leu His Cys Ala Val Ala Ser Gly Ser Ala Ser Ala
100 105 110

Val Glu Val Val Lys Leu Leu Leu His Ala Ser Ala Asp Ala Asn Cys
115 120 125

Ile Asp Gly Asn Gly Lys Lys Pro Ile Asp Val Ile Ala Leu Pro Leu
130 135 140

Lys Ser Arg Gly Asp Ser Arg Arg Lys Leu Met Glu Leu Leu Leu Lys
145 150 155 160

Gly Asp Asn Ser Asp Gly Glu Phe Glu Ser His Glu Glu Lys Pro Ile
165 170 175

Ala Ala Pro Gln Ala Ser Lys Glu Gly Ser Glu Lys Lys Glu Tyr Gln
180 185 190

Phe Pro Val Asp Ile Ser Leu Pro Asp Ile Asn Val Gly Ile Tyr Ser
195 200 205

Thr Asp Glu Phe Arg Met Tyr Ala Phe Lys Val Lys Pro Cys Ser Arg
210 215 220

Ala Tyr Ser His Asp Trp Thr Glu Cys Pro Phe Val His Pro Gly Glu
225 230 235 240

Asn Ala Arg Arg Arg Asp Pro Arg Lys Tyr Pro Tyr Ser Cys Val Pro
245 250 255

```
Cys Pro Glu Phe Arg Lys Gly Ser Cys Gln Lys Gly Asp Ser Cys Glu
            260                 265                 270

Tyr Ala His Gly Val Phe Glu Ser Trp Leu His Pro Ala Gln Tyr Arg
        275                 280                 285

Thr Arg Leu Cys Lys Asp Glu Thr Gly Cys Ala Arg Lys Val Cys Phe
    290                 295                 300

Phe Ala His Lys Pro Glu Glu Leu Arg Pro Val Tyr Ala Ser Thr Gly
305                 310                 315                 320

Ser Ala Met Pro Ser Pro Lys Ser Tyr Ser Ser Ser Gly Leu Asp Met
                325                 330                 335

Ser Thr Leu Ser Pro Leu Ser Ile Ser Ser Pro Ser Ala Ser Leu Pro
            340                 345                 350

Val Thr Ser Thr Ala Pro Met Ser Pro Leu Ala Ala Ser Ser Ser Pro
        355                 360                 365

Met Ser Val Asn Met Trp Gln Ser Lys Ala Asn Lys Leu Ser Pro Pro
    370                 375                 380

Met Leu Gln Leu Ser Gly Ser Arg Leu Lys Thr Ala Leu Ser Ala Arg
385                 390                 395                 400

Asp Leu Asp Leu Glu Met Glu Leu Arg Gly Leu Glu Ser Gln Met Ala
                405                 410                 415

Thr Gln Gln His Gln Leu Met Glu Glu Ile Ser Arg Leu Ser Ser Pro
            420                 425                 430

Ser Ser Cys Phe Ser Ser Arg Ile Gly Glu Val Lys Pro Thr Asn Leu
        435                 440                 445

Asp Asp Val Phe Gly Ser Pro Asp Pro Ala Leu Leu Pro Gln Leu Gln
    450                 455                 460

Gly Leu Ser Arg Pro Ser Thr Pro Ser Gln Leu Gln Ser Pro Thr Gly
465                 470                 475                 480

Leu Gln Met Arg Gln Asn Ala Thr Gln Phe Arg Gly Ala Tyr Gln Ser
                485                 490                 495

Asn Ala Asn Ala Leu Ser Ser Pro Ala Met Lys Gln Ala Pro Ser Tyr
            500                 505                 510
```

Gly Phe Asp Ser Ser Ser Ala Val Ala Ala Ala Val Met Asn Ser Arg
515 520 525

Ser Ala Ala Phe Ala Lys Arg Ser Gln Ser Phe Ile Asp Arg Gly Met
530 535 540

Ala Cys Pro Gly Ile Ala Asn Ser Ser Pro Met Met Ser Ser Ala Met
545 550 555 560

Ser Ser Trp Ser Ser Pro His Gly Lys Leu Asp Trp Gly Val Gln Gly
565 570 575

Asp Glu Leu Asn Arg Leu Arg Lys Ala Ala Ser Phe Lys Met Arg Ser
580 585 590

Ser Thr Gly Ala Gly Ala Asn Thr Val Ser Ala Ala Ala Met Ala Asp
595 600 605

Glu Pro Asp Ile Ser Trp Val Ser Ser Leu Val Lys Asp Val Pro Ser
610 615 620

Ala Glu Asp Ala Met Phe Ala Ala Glu Lys Gly Gln Arg Thr Tyr Gly
625 630 635 640

Lys Asp Ile Arg Glu Arg Ile Thr Pro Trp Val Glu Gln Leu Tyr Arg
645 650 655

Glu Val Pro Arg Met Ala Met
660

<210> 30
<211> 2001
<212> DNA
<213> Triticum aestivum

<220>
<221> misc_feature
<222> (481)..(530)
<223> n is a, c, g, or t

<400> 30
cgaattccgg tcgacgattt ctcgatttcc ttctctataa cacaacgctc tcttctcttg 60

caaccaaagt acttgttcca gtgtctactc tactcaaaaa ggatttggga catcatgtgc 120

agtgattcga aaagtaaact ttcttcccca accctcgtcg tcatggagaa tagtaacatt 180

cagaagcaga atctggatgg tctctacaac tcggttttgc ttgaattgtc tgcatctgat 240

gattatgaag ctttcaaaag agaggtggag gaaaaaggct tagatgtgaa cgaggcaggc 300

ttttggtacg gtagaagaat tgggtcaaag aagatgggat ctgaaacgag gacccctctg 360

atgattgctt ctttgtttgg aagcgccaag gtgctcaatt atattcttct tcagaaagga 420

ggaggtgttg atgtgaacag ggtctgtggt tctgataggg ccactgctct ccattgtgct 480

nnnnnnnnnn nnnnnnnnnn nnnnnnnnnn nnnnnnnnnn nnnnnnnnnn tcccgggtcg 540

cgatttcgta ggagattcac agagagaaaa gaagcaatta gtgataataa gaaagaatac 600

cctgttgata tatcactgcc agacataaac aacggtgtat atggaacaga tgattttagg 660

atgtacaact tcaaggtgaa gccttgctca agggcttact cccatgactg gaccgagtgt 720

ccattcgttc acccagggga gaacgctagg aggagagacc cacggaaata cccttacagc 780

tgtgttcctt gccctgagtt ccgcaaaggg acctgccaga agggtgattc ctgtgagtat 840

gctcatggtg tttttgagtc ctggctgcat cctgcccaat accggacaag gctttgcaag 900

gatgagactg gctgcgctag aaaagtctgc ttctttgccc acaaacctga agagctacgc 960

cctgtgtatg cttccactgg gtcggctatg ccatcaccaa aatcatattc agctagtgga 1020

cttgacatga cagcgatgag tccattggct ctaagttcca catctttgcc taatgccccc 1080

ccgtttccag cctcacccta tcgtgcgccc tcgttcttct ctcagagtga agctgtgcag 1140

aacaaaataa accttactcc accatcgttg cagctccctg gtagccgact gaaggctgct 1200

ttgagtgcca gggatctgga gatggagatg gaactgctcg gtctagaaag ccctgctcgc 1260

caacaacagc agcagcagca acaattgatc gaagagattg ccaggatctc ttccccatct 1320

ttccggagca aggaattcaa taggattgtt gatttgaatc ctactaacct tgatgacctg 1380

ttagcatctg ctgacccttc tgtattttct caactacatg gactttctgt gcaaccttca 1440

acacccacac aaagtgggct tcagatgcgc caaaacatga accacctccg tgcgagttat 1500

ccatccaaca tcccttcctc tcctgtgagg aagccctcag cttttgggtt tgactcatca 1560

gctgctgtgg caactgcagt gatgaattct aggtctgctg ccttcgcaaa gcgaagccaa 1620

agtttcattg atcgtggagc tgcaacccac catcttgggc tgtcttcagc ttccaactct 1680

tcttgcaggg tatcctctac cctttcagat tggagttccc ctaccgggaa actggattgg 1740

ggtgtaaacg gagacaagct gaacaagctg aggaaatcta cttcctttgg attcagaaac 1800

agtggggtaa ctgcatcccc catagcacag cctgaatttg gtgctgagcc ggatgtctca 1860

tgggttcatt cattggttaa agatgttccc tccgagaggt ctgagatatt tggtgctgag 1920

aagcaacaat atgatctcag taaagagatg cttccaccat ggatggagca gctgtatata 1980

gagcaggagc agatggtagc a 2001

<210> 31
<211> 667
<212> PRT
<213> Triticum aestivum

<220>
<221> UNSURE
<222> (161)..(177)

<223> Xaa can be any naturally occurring amino acid

<400> 31

Arg Ile Pro Val Asp Asp Phe Ser Ile Ser Phe Ser Ile Thr Gln Arg
1 5 10 15

Ser Leu Leu Leu Gln Pro Lys Tyr Leu Phe Gln Cys Leu Leu Tyr Ser
20 25 30

Lys Arg Ile Trp Asp Ile Met Cys Ser Asp Ser Lys Ser Lys Leu Ser
35 40 45

Ser Pro Thr Leu Val Val Met Glu Asn Ser Asn Ile Gln Lys Gln Asn
50 55 60

Leu Asp Gly Leu Tyr Asn Ser Val Leu Leu Glu Leu Ser Ala Ser Asp
65 70 75 80

Asp Tyr Glu Ala Phe Lys Arg Glu Val Glu Glu Lys Gly Leu Asp Val
85 90 95

Asn Glu Ala Gly Phe Trp Tyr Gly Arg Arg Ile Gly Ser Lys Lys Met
100 105 110

Gly Ser Glu Thr Arg Thr Pro Leu Met Ile Ala Ser Leu Phe Gly Ser
115 120 125

Ala Lys Val Leu Asn Tyr Ile Leu Leu Gln Lys Gly Gly Gly Val Asp
130 135 140

Val Asn Arg Val Cys Gly Ser Asp Arg Ala Thr Ala Leu His Cys Ala
145 150 155 160

Xaa Xaa Xaa Xaa Xaa Xaa Xaa Xaa Xaa Xaa Xaa Xaa Xaa Xaa Xaa Xaa
165 170 175

Xaa Pro Gly Ser Arg Phe Arg Arg Arg Phe Thr Glu Arg Lys Glu Ala
180 185 190

Ile Ser Asp Asn Lys Lys Glu Tyr Pro Val Asp Ile Ser Leu Pro Asp
195 200 205

Ile Asn Asn Gly Val Tyr Gly Thr Asp Asp Phe Arg Met Tyr Asn Phe
210 215 220

Lys Val Lys Pro Cys Ser Arg Ala Tyr Ser His Asp Trp Thr Glu Cys
225 230 235 240

Pro Phe Val His Pro Gly Glu Asn Ala Arg Arg Arg Asp Pro Arg Lys

```
                245                 250                 255

Tyr Pro Tyr Ser Cys Val Pro Cys Pro Glu Phe Arg Lys Gly Thr Cys
            260                 265                 270

Gln Lys Gly Asp Ser Cys Glu Tyr Ala His Gly Val Phe Glu Ser Trp
        275                 280                 285

Leu His Pro Ala Gln Tyr Arg Thr Arg Leu Cys Lys Asp Glu Thr Gly
    290                 295                 300

Cys Ala Arg Lys Val Cys Phe Phe Ala His Lys Pro Glu Glu Leu Arg
305                 310                 315                 320

Pro Val Tyr Ala Ser Thr Gly Ser Ala Met Pro Ser Pro Lys Ser Tyr
                325                 330                 335

Ser Ala Ser Gly Leu Asp Met Thr Ala Met Ser Pro Leu Ala Leu Ser
            340                 345                 350

Ser Thr Ser Leu Pro Asn Ala Pro Pro Phe Pro Ala Ser Pro Tyr Arg
        355                 360                 365

Ala Pro Ser Phe Phe Ser Gln Ser Glu Ala Val Gln Asn Lys Ile Asn
    370                 375                 380

Leu Thr Pro Pro Ser Leu Gln Leu Pro Gly Ser Arg Leu Lys Ala Ala
385                 390                 395                 400

Leu Ser Ala Arg Asp Leu Glu Met Glu Met Glu Leu Leu Gly Leu Glu
                405                 410                 415

Ser Pro Ala Arg Gln Gln Gln Gln Gln Gln Gln Gln Leu Ile Glu Glu
            420                 425                 430

Ile Ala Arg Ile Ser Ser Pro Ser Phe Arg Ser Lys Glu Phe Asn Arg
        435                 440                 445

Ile Val Asp Leu Asn Pro Thr Asn Leu Asp Asp Leu Leu Ala Ser Ala
    450                 455                 460

Asp Pro Ser Val Phe Ser Gln Leu His Gly Leu Ser Val Gln Pro Ser
465                 470                 475                 480

Thr Pro Thr Gln Ser Gly Leu Gln Met Arg Gln Asn Met Asn His Leu
                485                 490                 495

Arg Ala Ser Tyr Pro Ser Asn Ile Pro Ser Ser Pro Val Arg Lys Pro
            500                 505                 510
```

Ser Ala Phe Gly Phe Asp Ser Ser Ala Ala Val Ala Thr Ala Val Met
515 520 525

Asn Ser Arg Ser Ala Ala Phe Ala Lys Arg Ser Gln Ser Phe Ile Asp
530 535 540

Arg Gly Ala Ala Thr His His Leu Gly Leu Ser Ser Ala Ser Asn Ser
545 550 555 560

Ser Cys Arg Val Ser Ser Thr Leu Ser Asp Trp Ser Ser Pro Thr Gly
565 570 575

Lys Leu Asp Trp Gly Val Asn Gly Asp Lys Leu Asn Lys Leu Arg Lys
580 585 590

Ser Thr Ser Phe Gly Phe Arg Asn Ser Gly Val Thr Ala Ser Pro Ile
595 600 605

Ala Gln Pro Glu Phe Gly Ala Glu Pro Asp Val Ser Trp Val His Ser
610 615 620

Leu Val Lys Asp Val Pro Ser Glu Arg Ser Glu Ile Phe Gly Ala Glu
625 630 635 640

Lys Gln Gln Tyr Asp Leu Ser Lys Glu Met Leu Pro Pro Trp Met Glu
645 650 655

Gln Leu Tyr Ile Glu Gln Glu Gln Met Val Ala
660 665

<210> 32
<211> 2683
<212> DNA
<213> Eucalyptus grandis

<400> 32
gcaaaggtcg atcacttcct ccctagaaag cgagtgtgga gttgaagctt gataaccaga 60

ggccgcctct cgtctcgtct cgcccgcctg cgcttgctct gctctccgcg tgccaaggga 120

gtgttcctag gtgctgaatc tttccatgtg tagcggttca aaagggaagg gagagtgaat 180

tcgagaagca gaggatgtcg gcccgtcagt tctcgatcct gctcgagtta tctgctgcgg 240

atgatctgac gaactttaag aaagcagttg aggaagacgg ctacgatatt gatgagtcga 300

gcttgtggta tggtaggagg atcgggtcga agaagattgg gcttgaagag agaactcccc 360

tcatgattgc cgcgatgttc ggcagtatgt ccgtgctgga ttatattatc aagtctggcc 420

gggccaatgt aaacaaggcg tgtggttcag atggtgctac cgcgcttcac tgtgctgcgg 480

ctggtggctc ggtacaatct cctgaggtgg tcaagctgtt gcttgattct tcagcgaatg 540

ctaactccat tgatgcgaat gggaaacgag cgggagactt gatttctgag gtctctggtt 600
cgcccttcaa ttcgagaagg aagactttgg atgtcatgtt gactggaggt gggactgttg 660
agtttgttga ggaaacttac aatctgcctg agaatctggg tagtcaaatt gaaggaaacg 720
aacaaagaga gagtccaacg gcccgcgctt ccaaggatgg ttctgaaaag aaagagtatc 780
ctgtcgacct ttctcttccg gacatcaaca atggaatata tagcacagat gagtttagga 840
tgtattcttt caaagtgaag ccttgctcga gagcttactc tcatgactgg actgagtgtc 900
catttgttca ccctggggag aatgcaagac ggcgtgaccc acggaaatat cactacagct 960
gtgtgccttg ccctgagttc cgcaaggggt catgcaggca aggggatggc tgcgagtatg 1020
ctcatggtat atttgagtgc tggcttcacc cagctcaata tcgcacccgt ctctgtaagg 1080
atgagattgg atgcaccaga aaagtctgtt tctttgccca caaacatgaa gagcttcgtc 1140
cattgtatgc atcaactggt tcggcgcttc cttctccaag atcattttcg cccgttgctg 1200
cttctctaga catgggatca ctgagccctc tctctctcgg ttcttcttca gtccggatac 1260
cgccaacttc aacaccacct atgactccat caggggcctc ttctcccctt ggtgggtcga 1320
tgtggaaaag ccaaattaat agcactccgc ctggcttgca gcttccaggt agcaggttga 1380
gaagcgcatt gagtgctaga gacatggatt tagatgttga cttgatcgat ctagaaaata 1440
attatcgttt gcagaagcag ttgctcgaac actttcctga tctgtcctct cctcgtggtt 1500
ggaacaactc ttcatccacc acgtcggctt tccctgagta ttcaggtgac atgactggag 1560
aaataagtag gttaggagta aaaccaaata atctcgagga tagtttcagg tcattggacc 1620
tgaccctctt gtctcagtta caagggctgt cacttgatgg tgcaatatcc cagctgcaat 1680
ctcctactgg aatgaagatt cggcagaaca tgacccagca gctctactca aactatactg 1740
acaagctttc ctcgtcacct agggcaatgc catcatttgg aaccgatcct tccagagctt 1800
cagcagcagc cactctgagt tccaggtcat tggcatttgc aaaaaggagc cacagcttca 1860
ttgagcggag tacagtgaac agtcagtctg gatattcagc aggtgctgct tctccaactg 1920
caaggatgtc ttcccagaat gactggggct cgcccgatgg caaactagac tggggcattc 1980
aaggggagga gctgaacaag ctgaggaaat ctgcatcatt cgggctcagg agcagcagca 2040
accgcttcca tgcgtctgca gattctgcga cagcaactgt aggggaccca gacatgccct 2100
ggattcagtc cttggcaaag gaagccccgt cacaaaaccc tggcaatttt ggagcagagc 2160
atcagcagca gcagcagcag cagcagcagc agcagtatca tcttaattct ggaggtactg 2220
agctgcttcc agcttgggtg gagcagttgt acgcggatca ggagcagatg gtcgcctgag 2280
atcaacattg gcttcttatc taaccactat tagtcatttc gttattgctt taattttttt 2340
tcttctgagt ctagtattaa tgtctaggat tcgaacgaac tggaaaatta aatctagagg 2400
gaagatggga agaaaagagc aggatggaag gtttctgctc ggtccgagat ttctcatagt 2460

ctattataga ctatcgtatt tctcgttctt ttccgtccca atgttcttga tttggttctc 2520

agcatgtttt ctggatgagg cttacaaact atgtaatctt gtcttgctaa aagaatcaga 2580

gctgcacctg caccaaaggt tgtgatacta ccgcttattg atgatgatga taataataat 2640

aattcggaca tttagtacca agtccgatgt ctcaaaaaaa aaa 2683

<210> 33
<211> 694
<212> PRT
<213> Eucalyptus grandis

<400> 33

Met Ser Ala Arg Gln Phe Ser Ile Leu Leu Glu Leu Ser Ala Ala Asp
1 5 10 15

Asp Leu Thr Asn Phe Lys Lys Ala Val Glu Glu Asp Gly Tyr Asp Ile
20 25 30

Asp Glu Ser Ser Leu Trp Tyr Gly Arg Arg Ile Gly Ser Lys Lys Ile
35 40 45

Gly Leu Glu Glu Arg Thr Pro Leu Met Ile Ala Ala Met Phe Gly Ser
50 55 60

Met Ser Val Leu Asp Tyr Ile Ile Lys Ser Gly Arg Ala Asn Val Asn
65 70 75 80

Lys Ala Cys Gly Ser Asp Gly Ala Thr Ala Leu His Cys Ala Ala Ala
85 90 95

Gly Gly Ser Val Gln Ser Pro Glu Val Val Lys Leu Leu Leu Asp Ser
100 105 110

Ser Ala Asn Ala Asn Ser Ile Asp Ala Asn Gly Lys Arg Ala Gly Asp
115 120 125

Leu Ile Ser Glu Val Ser Gly Ser Pro Phe Asn Ser Arg Arg Lys Thr
130 135 140

Leu Asp Val Met Leu Thr Gly Gly Gly Thr Val Glu Phe Val Glu Glu
145 150 155 160

Thr Tyr Asn Leu Pro Glu Asn Leu Gly Ser Gln Ile Glu Gly Asn Glu
165 170 175

Gln Arg Glu Ser Pro Thr Ala Arg Ala Ser Lys Asp Gly Ser Glu Lys
180 185 190

Lys Glu Tyr Pro Val Asp Leu Ser Leu Pro Asp Ile Asn Asn Gly Ile

195 200 205

Tyr Ser Thr Asp Glu Phe Arg Met Tyr Ser Phe Lys Val Lys Pro Cys
210 215 220

Ser Arg Ala Tyr Ser His Asp Trp Thr Glu Cys Pro Phe Val His Pro
225 230 235 240

Gly Glu Asn Ala Arg Arg Arg Asp Pro Arg Lys Tyr His Tyr Ser Cys
245 250 255

Val Pro Cys Pro Glu Phe Arg Lys Gly Ser Cys Arg Gln Gly Asp Gly
260 265 270

Cys Glu Tyr Ala His Gly Ile Phe Glu Cys Trp Leu His Pro Ala Gln
275 280 285

Tyr Arg Thr Arg Leu Cys Lys Asp Glu Ile Gly Cys Thr Arg Lys Val
290 295 300

Cys Phe Phe Ala His Lys His Glu Glu Leu Arg Pro Leu Tyr Ala Ser
305 310 315 320

Thr Gly Ser Ala Leu Pro Ser Pro Arg Ser Phe Ser Pro Val Ala Ala
325 330 335

Ser Leu Asp Met Gly Ser Leu Ser Pro Leu Ser Leu Gly Ser Ser Ser
340 345 350

Val Arg Ile Pro Pro Thr Ser Thr Pro Pro Met Thr Pro Ser Gly Ala
355 360 365

Ser Ser Pro Leu Gly Gly Ser Met Trp Lys Ser Gln Ile Asn Ser Thr
370 375 380

Pro Pro Gly Leu Gln Leu Pro Gly Ser Arg Leu Arg Ser Ala Leu Ser
385 390 395 400

Ala Arg Asp Met Asp Leu Asp Val Asp Leu Ile Asp Leu Glu Asn Asn
405 410 415

Tyr Arg Leu Gln Lys Gln Leu Leu Glu His Phe Pro Asp Leu Ser Ser
420 425 430

Pro Arg Gly Trp Asn Asn Ser Ser Ser Thr Thr Ser Ala Phe Pro Glu
435 440 445

Tyr Ser Gly Asp Met Thr Gly Glu Ile Ser Arg Leu Gly Val Lys Pro
450 455 460

```
Asn Asn Leu Glu Asp Ser Phe Arg Ser Leu Asp Leu Thr Leu Leu Ser
465                 470                 475                 480

Gln Leu Gln Gly Leu Ser Leu Asp Gly Ala Ile Ser Gln Leu Gln Ser
                485                 490                 495

Pro Thr Gly Met Lys Ile Arg Gln Asn Met Thr Gln Gln Leu Tyr Ser
            500                 505                 510

Asn Tyr Thr Asp Lys Leu Ser Ser Ser Pro Arg Ala Met Pro Ser Phe
        515                 520                 525

Gly Thr Asp Pro Ser Arg Ala Ser Ala Ala Ala Thr Leu Ser Ser Arg
    530                 535                 540

Ser Leu Ala Phe Ala Lys Arg Ser His Ser Phe Ile Glu Arg Ser Thr
545                 550                 555                 560

Val Asn Ser Gln Ser Gly Tyr Ser Ala Gly Ala Ala Ser Pro Thr Ala
                565                 570                 575

Arg Met Ser Ser Gln Asn Asp Trp Gly Ser Pro Asp Gly Lys Leu Asp
            580                 585                 590

Trp Gly Ile Gln Gly Glu Glu Leu Asn Lys Leu Arg Lys Ser Ala Ser
        595                 600                 605

Phe Gly Leu Arg Ser Ser Ser Asn Arg Phe His Ala Ser Ala Asp Ser
    610                 615                 620

Ala Thr Ala Thr Val Gly Asp Pro Asp Met Pro Trp Ile Gln Ser Leu
625                 630                 635                 640

Ala Lys Glu Ala Pro Ser Gln Asn Pro Gly Asn Phe Gly Ala Glu His
                645                 650                 655

Gln Gln Gln Gln Gln Gln Gln Gln Gln Gln Gln Tyr His Leu Asn Ser
            660                 665                 670

Gly Gly Thr Glu Leu Leu Pro Ala Trp Val Glu Gln Leu Tyr Ala Asp
        675                 680                 685

Gln Glu Gln Met Val Ala
    690
```

<210> 34
<211> 2499
<212> DNA

<213>  Arabidopsis thaliana

<400>  34
atttgacct taagaagaaa gtgacaagga gaggaagaag aagaaaaaaa acataatttg     60
aggaagaaga aaaaaaattc ggatttgttt tttcaataaa ttgactaatt gagtactcgt    120
ttaaaggaag tgaagagcgg ttttttggta gtggtggtcg agaaaagaga gagtttgtct    180
ctgtgactca gagtgaaatc aatagagcgg gaaaagattg ttgctttttt ttgccatggg    240
agttgatgag ctgtctcacc tcaaattctc tcttctgcta gaatcatcag cctgcaatga    300
tttgtccggt tttaagtctc tagttgaaga agaaggtctt gagagcattg atggctctgg    360
tttgtggtat gggaggagat taggatcaaa gaagatgggt tttgaggaga ggacgcctct    420
tatgattgct gccttgtttg gaagcaaaga ggttgttgat tacatcatta gtactggtct    480
tgttgacgtg aaccgctctt gtggctctga tggtgccacg gctcttcact gtgcggtctc    540
tggcttgtct gccaatagcc ttgagattgt tactcttctg ctgaagggct ctgcgaatcc    600
ggattcttgt gatgcttatg gtaacaagcc tggagatgtg attttccctt gtttgagtcc    660
ggtttttagc gcgaggatga aggttttgga gcgtttgttg aaaggaaatg atgatttgaa    720
tgaagttaat gggcaagaag aaagcgagcc agaggttgag gttgaggttg aggtttcgcc    780
tcctcggggg tctgagagga aggagtatcc ggttgatcca acgcttcctg atatcaagaa    840
cggtgtatat gggacggatg agttccggat gtatgctttc aagatcaagc cgtgctctag    900
agcatactct cacgactgga cggaatgtcc ctttgttcat ccgggtgaga acgcaaggag    960
gcgtgatccg aggaagtacc attatagttg tgtcccttgt cctgaattcc ggaaggggtc   1020
ttgttccaga ggtgatactt gcgagtatgc tcatggtatc tttgagtgct ggcttcaccc   1080
ggctcagtac cggactcgtc tctgcaagga cgagacgaat tgctcgagaa gagtttgttt   1140
ctttgcccac aaacccgagg agctgcgtcc tttgtaccct tcaactggat caggtgttcc   1200
gtccccgcgg tcttccttct catcttgcaa ttcctcgacc gctttcgaca tgggaccgat   1260
tagtccgctt cctatcggag caacaaccac acctcctttg agtcctaacg gtgtatcctc   1320
tccaataggt ggaggaaaaa cgtggatgaa ctggcctaac ataacccctc ctgcattgca   1380
gcttccaggg agcagattga aatctgcatt gaatgcaaga gaaatcgatt tctctgaaga   1440
gatgcaaagt cttacttctc caactacatg gaacaacacg ccaatgtcat ctccattctc   1500
cggaaagggc atgaacaggc ttgcaggagg agcaatgagc ccggtgaata gtctcagtga   1560
tatgtttggg acagaggata atacatcggg tttgcagatc cgacgcagcg tcattaaccc   1620
gcagctgcat tccaacagtc tttcttcatc acctgtggga gccaattctc tgttttcgat   1680
ggattcctcc gcagtcttgg cttcaagagc ggctgaattt gctaaacagc gaagccaaag   1740
cttcatagaa cgcaacaacg gactgaatca ccatcccgca atctcttcca tgactacaac   1800
ttgtttaaac gattggggct cattggatgg gaagcttgac tggagcgtcc aaggagacga   1860

gctacagaag ctcagaaaat ccacttcttt ccgtctcaga gccggtggca tggaatcaag 1920

actgcctaac gaagggactg ggctcgaaga gccagatgtc tcatgggtgg agccgctggt 1980

gaaagagcca caggagacaa gactagctcc ggtttggatg gagcaatcat acatggagac 2040

agaacagacc gtggcttgaa tcaaaagttt tgaactttca ttaaccgttc cacaagaagc 2100

aaagtcagaa agattccgag aggtcgatgc taatctattt cattttattt gtttaatgct 2160

ttgttatttt tctttagaat aaaaagaaaa aattcttagg ggacaaaaga gagttcgttt 2220

gtctctctct ctgtctccaa agaaaaacag aggtgaaaaa aggtttcaaa acctaagaaa 2280

ccttgaatta cctcacctca cttccttgat tctttactat tcacaatgag taatcgattt 2340

tttttttct tggtaacact ctcacgctga atatatatgt tttttagtaa taatataatt 2400

ggaatacaga aatgtattta cacttgtgaa gttagggaaa gtgttgtaat tgtttcttct 2460

aagagttgat ctaagatgtt tgagactata tcttcgctt 2499

<210> 35
<211> 607
<212> PRT
<213> Arabidopsis thaliana

<400> 35

Met Gly Val Asp Glu Leu Ser His Leu Lys Phe Ser Leu Leu Leu Glu
1 5 10 15

Ser Ser Ala Cys Asn Asp Leu Ser Gly Phe Lys Ser Leu Val Glu Glu
20 25 30

Glu Gly Leu Glu Ser Ile Asp Gly Ser Gly Leu Trp Tyr Gly Arg Arg
35 40 45

Leu Gly Ser Lys Lys Met Gly Phe Glu Glu Arg Thr Pro Leu Met Ile
50 55 60

Ala Ala Leu Phe Gly Ser Lys Glu Val Val Asp Tyr Ile Ile Ser Thr
65 70 75 80

Gly Leu Val Asp Val Asn Arg Ser Cys Gly Ser Asp Gly Ala Thr Ala
85 90 95

Leu His Cys Ala Val Ser Gly Leu Ser Ala Asn Ser Leu Glu Ile Val
100 105 110

Thr Leu Leu Leu Lys Gly Ser Ala Asn Pro Asp Ser Cys Asp Ala Tyr
115 120 125

Gly Asn Lys Pro Gly Asp Val Ile Phe Pro Cys Leu Ser Pro Val Phe
130 135 140

```
Ser Ala Arg Met Lys Val Leu Glu Arg Leu Leu Lys Gly Asn Asp Asp
145                 150                 155                 160

Leu Asn Glu Val Asn Gly Gln Glu Glu Ser Glu Pro Glu Val Glu Val
                165                 170                 175

Glu Val Glu Val Ser Pro Pro Arg Gly Ser Glu Arg Lys Glu Tyr Pro
            180                 185                 190

Val Asp Pro Thr Leu Pro Asp Ile Lys Asn Gly Val Tyr Gly Thr Asp
        195                 200                 205

Glu Phe Arg Met Tyr Ala Phe Lys Ile Lys Pro Cys Ser Arg Ala Tyr
    210                 215                 220

Ser His Asp Trp Thr Glu Cys Pro Phe Val His Pro Gly Glu Asn Ala
225                 230                 235                 240

Arg Arg Arg Asp Pro Arg Lys Tyr His Tyr Ser Cys Val Pro Cys Pro
                245                 250                 255

Glu Phe Arg Lys Gly Ser Cys Ser Arg Gly Asp Thr Cys Glu Tyr Ala
            260                 265                 270

His Gly Ile Phe Glu Cys Trp Leu His Pro Ala Gln Tyr Arg Thr Arg
        275                 280                 285

Leu Cys Lys Asp Glu Thr Asn Cys Ser Arg Arg Val Cys Phe Phe Ala
    290                 295                 300

His Lys Pro Glu Glu Leu Arg Pro Leu Tyr Pro Ser Thr Gly Ser Gly
305                 310                 315                 320

Val Pro Ser Pro Arg Ser Ser Phe Ser Ser Cys Asn Ser Ser Thr Ala
                325                 330                 335

Phe Asp Met Gly Pro Ile Ser Pro Leu Pro Ile Gly Ala Thr Thr Thr
            340                 345                 350

Pro Pro Leu Ser Pro Asn Gly Val Ser Ser Pro Ile Gly Gly Gly Lys
        355                 360                 365

Thr Trp Met Asn Trp Pro Asn Ile Thr Pro Pro Ala Leu Gln Leu Pro
    370                 375                 380

Gly Ser Arg Leu Lys Ser Ala Leu Asn Ala Arg Glu Ile Asp Phe Ser
385                 390                 395                 400
```

Glu Glu Met Gln Ser Leu Thr Ser Pro Thr Thr Trp Asn Asn Thr Pro
405 410 415

Met Ser Ser Pro Phe Ser Gly Lys Gly Met Asn Arg Leu Ala Gly Gly
420 425 430

Ala Met Ser Pro Val Asn Ser Leu Ser Asp Met Phe Gly Thr Glu Asp
435 440 445

Asn Thr Ser Gly Leu Gln Ile Arg Arg Ser Val Ile Asn Pro Gln Leu
450 455 460

His Ser Asn Ser Leu Ser Ser Ser Pro Val Gly Ala Asn Ser Leu Phe
465 470 475 480

Ser Met Asp Ser Ser Ala Val Leu Ala Ser Arg Ala Ala Glu Phe Ala
485 490 495

Lys Gln Arg Ser Gln Ser Phe Ile Glu Arg Asn Asn Gly Leu Asn His
500 505 510

His Pro Ala Ile Ser Ser Met Thr Thr Thr Cys Leu Asn Asp Trp Gly
515 520 525

Ser Leu Asp Gly Lys Leu Asp Trp Ser Val Gln Gly Asp Glu Leu Gln
530 535 540

Lys Leu Arg Lys Ser Thr Ser Phe Arg Leu Arg Ala Gly Gly Met Glu
545 550 555 560

Ser Arg Leu Pro Asn Glu Gly Thr Gly Leu Glu Glu Pro Asp Val Ser
565 570 575

Trp Val Glu Pro Leu Val Lys Glu Pro Gln Glu Thr Arg Leu Ala Pro
580 585 590

Val Trp Met Glu Gln Ser Tyr Met Glu Thr Glu Gln Thr Val Ala
595 600 605

<210> 36
<211> 1806
<212> DNA
<213> Oryza sativa

<400> 36
atgtgctctg ggccgcgcaa gccgtccaca ccgccgctgc cgcagcagca gaaggaggcg 60

acggtgatgg cggcgtcctt gcttcttgag ctggcggcag cggacgacgt ggcggcggtg 120

aggagggtcg tggaggagga gaaggtgtct cttggcgtgg ctgggttgtg gtatgggcct 180

tcggcgagcg gcgtggcgag gctcgggatg gagcggagga cggcggcgat ggtggcggcg 240

ctgtacggga gcacgggggt gcttgggtat gtcgtggcgg cagcgccggc ggaggccgcg 300

cgcgcgtcgg agacggatgg ggccacgccg ctgcacatgg cggctgccgg tggcgcggcg 360

aacgcggtcg cggccacgcg cctgttgctc gccgcggggg cgtcggtcga cgcgctctcg 420

gcttcggggc tccgcgccgg tgacctcctc ccgcgcgcca ccgcggcgga gaaggccatc 480

cggctgctgc tcaagtcgcc ggccgtgtcg ccgtcgtcgt cgccgaagaa gtcggcctcg 540

ccgccgtcgc cgccgccgcc gcaggaggcg aagaaggagt acccgcctga cctgacgctg 600

cccgacctca agagcggact gttcagcacc gacgagttcc gcatgtacag cttcaaggtg 660

aagccgtgct cccgcgccta ctcccatgac tggaccgagt gccccttcgt ccaccccggc 720

gagaacgcgc gccgccgcga ccctcgccgc tactcctaca gctgcgtgcc ttgcccggag 780

ttccgcaagg gcggctcgtg ccgcaagggc gacgcgtgcg agtacgccca tggcgtgttc 840

gagtgctggc tccacccggc gcagtacagg acgcgcctct gcaaggacga ggtcggctgc 900

gcgcgccgca tctgcttctt cgcccacaag cccgacgagc tccgcgccgt caacccctcc 960

gccgtgtccg tcggcatgca gcccaccgta tcgtcgccgc gctcctcgcc gcccaacggg 1020

ctcgacatgg cggcggcggc ggcggcgatg atgagccccg cctggccgtc gtccccagcg 1080

agccgcctca agacggcgct cggcgcgcgg gagctcgact tcgacctcga gatgctcgcg 1140

ctggaccagt accagcagaa gctgttcgac aaggtgtccg gcgcgccgtc gccgagggcg 1200

agctggggcg ccgcggcgaa cggcctcgcc accgcgtcgc cggcgagggc cgtgccggac 1260

tacaccgacc tgctcggctc cgtcgacccg gccatgctgt cccagctcca cgcgctgtcc 1320

ctcaagcagg ccggcgacat gcccgcgtac agctccatgg cggacaccac gcagatgcac 1380

atgccgacct cgccgatggt gggcggcgcg aacaccgcgt tcgggctgga ccactccatg 1440

gcgaaggcga tcatgagctc ccgcgcctcg gcgttcgcca agcgcagcca gagcttcatc 1500

gaccgcggag gccgcgcccc ggcggcgcgt tcgctcatgt cgccggcgac gaccggcgcg 1560

ccgtccattc tctcggactg gggctcgccg gacggcaagc tggactgggg cgtccagggc 1620

gacgagctgc acaagctccg caagtcggcg tcgttcgcgt tccgcggcca atccgccatg 1680

ccggtggcga cgcacgccgc ggcggcggag ccggacgtgt catgggtgaa ctctcttgtc 1740

aaggacggcc acgccgccgg cgacatattc gcgcagtggc cggagcagga gcagatggtg 1800

gcatga 1806

<210> 37
<211> 601
<212> PRT
<213> Oryza sativa

<400> 37

Met Cys Ser Gly Pro Arg Lys Pro Ser Thr Pro Pro Leu Pro Gln Gln
1 5 10 15

Gln Lys Glu Ala Thr Val Met Ala Ala Ser Leu Leu Leu Glu Leu Ala
20 25 30

Ala Ala Asp Asp Val Ala Ala Val Arg Arg Val Val Glu Glu Glu Lys
35 40 45

Val Ser Leu Gly Val Ala Gly Leu Trp Tyr Gly Pro Ser Ala Ser Gly
50 55 60

Val Ala Arg Leu Gly Met Glu Arg Arg Thr Ala Ala Met Val Ala Ala
65 70 75 80

Leu Tyr Gly Ser Thr Gly Val Leu Gly Tyr Val Val Ala Ala Ala Pro
85 90 95

Ala Glu Ala Ala Arg Ala Ser Glu Thr Asp Gly Ala Thr Pro Leu His
100 105 110

Met Ala Ala Ala Gly Gly Ala Ala Asn Ala Val Ala Ala Thr Arg Leu
115 120 125

Leu Leu Ala Ala Gly Ala Ser Val Asp Ala Leu Ser Ala Ser Gly Leu
130 135 140

Arg Ala Gly Asp Leu Leu Pro Arg Ala Thr Ala Ala Glu Lys Ala Ile
145 150 155 160

Arg Leu Leu Leu Lys Ser Pro Ala Val Ser Pro Ser Ser Ser Pro Lys
165 170 175

Lys Ser Ala Ser Pro Pro Ser Pro Pro Pro Pro Gln Glu Ala Lys Lys
180 185 190

Glu Tyr Pro Pro Asp Leu Thr Leu Pro Asp Leu Lys Ser Gly Leu Phe
195 200 205

Ser Thr Asp Glu Phe Arg Met Tyr Ser Phe Lys Val Lys Pro Cys Ser
210 215 220

Arg Ala Tyr Ser His Asp Trp Thr Glu Cys Pro Phe Val His Pro Gly
225 230 235 240

Glu Asn Ala Arg Arg Arg Asp Pro Arg Arg Tyr Ser Tyr Ser Cys Val
245 250 255

Pro Cys Pro Glu Phe Arg Lys Gly Gly Ser Cys Arg Lys Gly Asp Ala
260 265 270

Cys Glu Tyr Ala His Gly Val Phe Glu Cys Trp Leu His Pro Ala Gln
275 280 285

Tyr Arg Thr Arg Leu Cys Lys Asp Glu Val Gly Cys Ala Arg Arg Ile
290 295 300

Cys Phe Phe Ala His Lys Pro Asp Glu Leu Arg Ala Val Asn Pro Ser
305 310 315 320

Ala Val Ser Val Gly Met Gln Pro Thr Val Ser Ser Pro Arg Ser Ser
325 330 335

Pro Pro Asn Gly Leu Asp Met Ala Ala Ala Ala Ala Ala Met Met Ser
340 345 350

Pro Ala Trp Pro Ser Ser Pro Ala Ser Arg Leu Lys Thr Ala Leu Gly
355 360 365

Ala Arg Glu Leu Asp Phe Asp Leu Glu Met Leu Ala Leu Asp Gln Tyr
370 375 380

Gln Gln Lys Leu Phe Asp Lys Val Ser Gly Ala Pro Ser Pro Arg Ala
385 390 395 400

Ser Trp Gly Ala Ala Ala Asn Gly Leu Ala Thr Ala Ser Pro Ala Arg
405 410 415

Ala Val Pro Asp Tyr Thr Asp Leu Leu Gly Ser Val Asp Pro Ala Met
420 425 430

Leu Ser Gln Leu His Ala Leu Ser Leu Lys Gln Ala Gly Asp Met Pro
435 440 445

Ala Tyr Ser Ser Met Ala Asp Thr Thr Gln Met His Met Pro Thr Ser
450 455 460

Pro Met Val Gly Gly Ala Asn Thr Ala Phe Gly Leu Asp His Ser Met
465 470 475 480

Ala Lys Ala Ile Met Ser Ser Arg Ala Ser Ala Phe Ala Lys Arg Ser
485 490 495

Gln Ser Phe Ile Asp Arg Gly Gly Arg Ala Pro Ala Ala Arg Ser Leu
500 505 510

Met Ser Pro Ala Thr Thr Gly Ala Pro Ser Ile Leu Ser Asp Trp Gly
515 520 525

Ser Pro Asp Gly Lys Leu Asp Trp Gly Val Gln Gly Asp Glu Leu His
530 535 540

Lys Leu Arg Lys Ser Ala Ser Phe Ala Phe Arg Gly Gln Ser Ala Met
545 550 555 560

Pro Val Ala Thr His Ala Ala Ala Ala Glu Pro Asp Val Ser Trp Val
565 570 575

Asn Ser Leu Val Lys Asp Gly His Ala Ala Gly Asp Ile Phe Ala Gln
580 585 590

Trp Pro Glu Gln Glu Gln Met Val Ala
595 600

<210> 38
<211> 1692
<212> DNA
<213> Hordeum vulgare

<400> 38
cggcacgagg cacatccatc atctaacctc acctctcctc tcctcccctc tcctcctacc 60

aaacccaaaa ccaagcagag caagagcaag agcaagagca agagcaagca agcatgtgcc 120

ctggcctgcg caacctcgcc gccgccatgc caccctccgc ccacgaccac ccctcctcct 180

acctgctcga gctcgccgcc gacgacgacc tccccgcctt ccgccgcgcc gtccaggagg 240

acaacctctc cctcgacgcc gcatccccga ggtacgagcc atcccccaaa tcagaccaac 300

aacaacaaca cgccccagct cgcgctccac ctgcgcaccc ccgccatggt cgccgcgctc 360

tacggcagca ccaccgtcct ctcctacgtc ctctccatcg ccccctccga ggccgcccgc 420

gcctccgcat ccgacggcgc caccccgctc ctcctcgccc accagggccg cgcgccatcc 480

gcgccccacg ccgcacgcct cctcctcacc gacggcgcat catcgtcctc cctactcgcg 540

ccccaagctc accctctcaa ccaccaaaac caaaaccaaa acagccccac caagaaagac 600

tcgccgccgg actccaggag gaccaccacc aagaaggact actcctccgc ctccgactcc 660

cagacggagg acatcaacgc gggcgtcttc gccaccgacg acttccggat gtacagcttc 720

aaggtgaacc cgtgctcccg cgcctacacg cacgactgga ccgagtgccc cttcgcccac 780

cccggcgaga acgcgcgccg ccgcgacccg cgccgcgtgc catactcgtg cgtcccatgc 840

ccggacttcc gccgcgaccc ggccgcatgc cgcaagggcg acgcctgcga gtacgcgcac 900

ggcgtcttcg agtcatggct ccaccccgcg cagtaccgca ccaggctctg caaggacgag 960

gtcggatgcc cgcgccgcat ctgcttcttc gcgcacggcg cccgacagct acgcgccgtc 1020

aacccctccg ccgcatccat ggactcgcca tccccaactt cctcttcgcc gccgcgaacc 1080

tccaggccgg ccgcgctcac cgcgtcgctc agctcgcggg acctcgactt ggacgccgac 1140

aaccaggccc agtacgcgcg caggatgatg atggccaggg ccaactcccc gccggactac 1200

```
tcgcccgacc tcgtcgccgc ctacgtacag gcgctctcct ccctgcaaca gcagcagcat   1260

cagcagaacc agcaacagca gcatcagcag cagaaccagc accagcagca acatcagcag   1320

aaccagcacc agcagcatca gcagcaacat cagcagagca tggggatggg ggggctgagc   1380

gcccgcgccg ccgccttcac caaccgcagc cagaccttcg tgcaccgctc tccgtccccg   1440

gctccggcgc ggtcgttcaa gtctccggcg ccgtcgtcca tgctcgcgga ctgggggtcg   1500

ccggacggga agctggactg ggcgtgcag gccgcggagc tgcgcaagtc cacgtctttc   1560

ggagtcagaa gcagcagcag gccgcatcat gagacgacga gggcggagga caacatgtac   1620

ccgtcgtgga tgaaggacgg cagcgatatg ctgctggcgg cgcggtggtc ggacctggag   1680

cagatggtcg cc                                                        1692
```

```
<210>  39
<211>  564
<212>  PRT
<213>  Hordeum vulgare

<400>  39

Arg His Glu Ala His Pro Ser Ser Asn Leu Thr Ser Pro Leu Leu Pro
1               5                   10                  15

Ser Pro Pro Thr Lys Pro Lys Thr Lys Gln Ser Lys Ser Lys Ser Lys
            20                  25                  30

Ser Lys Ser Lys Gln Ala Cys Ala Leu Ala Cys Ala Thr Ser Pro Pro
        35                  40                  45

Pro Cys His Pro Pro Pro Thr Thr Thr Pro Pro Pro Thr Cys Ser Ser
    50                  55                  60

Ser Pro Pro Thr Thr Thr Ser Pro Pro Ser Ala Ala Pro Ser Arg Arg
65                  70                  75                  80

Thr Thr Ser Pro Ser Thr Pro His Pro Arg Gly Thr Ser His Pro Pro
                85                  90                  95

Asn Gln Thr Asn Asn Asn Asn Thr Pro Gln Leu Ala Leu His Leu Arg
            100                 105                 110

Thr Pro Ala Met Val Ala Ala Leu Tyr Gly Ser Thr Thr Val Leu Ser
        115                 120                 125

Tyr Val Leu Ser Ile Ala Pro Ser Glu Ala Ala Arg Ala Ser Ala Ser
    130                 135                 140

Asp Gly Ala Thr Pro Leu Leu Leu Ala His Gln Gly Arg Ala Pro Ser
145                 150                 155                 160
```

```
Ala Pro His Ala Ala Arg Leu Leu Leu Thr Asp Gly Ala Ser Ser Ser
                165                 170                 175

Ser Leu Leu Ala Pro Gln Ala His Pro Leu Asn His Gln Asn Gln Asn
            180                 185                 190

Gln Asn Ser Pro Thr Lys Lys Asp Ser Pro Pro Asp Ser Arg Arg Thr
        195                 200                 205

Thr Thr Lys Lys Asp Tyr Ser Ser Ala Ser Asp Ser Gln Thr Glu Asp
    210                 215                 220

Ile Asn Ala Gly Val Phe Ala Thr Asp Asp Phe Arg Met Tyr Ser Phe
225                 230                 235                 240

Lys Val Asn Pro Cys Ser Arg Ala Tyr Thr His Asp Trp Thr Glu Cys
                245                 250                 255

Pro Phe Ala His Pro Gly Glu Asn Ala Arg Arg Arg Asp Pro Arg Arg
            260                 265                 270

Val Pro Tyr Ser Cys Val Pro Cys Pro Asp Phe Arg Arg Asp Pro Ala
        275                 280                 285

Ala Cys Arg Lys Gly Asp Ala Cys Glu Tyr Ala His Gly Val Phe Glu
    290                 295                 300

Ser Trp Leu His Pro Ala Gln Tyr Arg Thr Arg Leu Cys Lys Asp Glu
305                 310                 315                 320

Val Gly Cys Pro Arg Arg Ile Cys Phe Phe Ala His Gly Ala Arg Gln
                325                 330                 335

Leu Arg Ala Val Asn Pro Ser Ala Ala Ser Met Asp Ser Pro Ser Pro
            340                 345                 350

Thr Ser Ser Ser Pro Pro Arg Thr Ser Arg Pro Ala Ala Leu Thr Ala
        355                 360                 365

Ser Leu Ser Ser Arg Asp Leu Asp Leu Asp Ala Asp Asn Gln Ala Gln
    370                 375                 380

Tyr Ala Arg Arg Met Met Met Ala Arg Ala Asn Ser Pro Pro Asp Tyr
385                 390                 395                 400

Ser Pro Asp Leu Val Ala Ala Tyr Val Gln Ala Leu Ser Ser Leu Gln
                405                 410                 415
```

Gln Gln Gln His Gln Gln Asn Gln Gln Gln Gln His Gln Gln Gln Asn
420 425 430

Gln His Gln Gln Gln His Gln Gln Asn Gln His Gln Gln His Gln Gln
435 440 445

Gln His Gln Gln Ser Met Gly Met Gly Gly Leu Ser Ala Arg Ala Ala
450 455 460

Ala Phe Thr Asn Arg Ser Gln Thr Phe Val His Arg Ser Pro Ser Pro
465 470 475 480

Ala Pro Ala Arg Ser Phe Lys Ser Pro Ala Pro Ser Ser Met Leu Ala
485 490 495

Asp Trp Gly Ser Pro Asp Gly Lys Leu Asp Trp Gly Val Gln Ala Ala
500 505 510

Glu Leu Arg Lys Ser Thr Ser Phe Gly Val Arg Ser Ser Ser Arg Pro
515 520 525

His His Glu Thr Thr Arg Ala Glu Asp Asn Met Tyr Pro Ser Trp Met
530 535 540

Lys Asp Gly Ser Asp Met Leu Leu Ala Ala Arg Trp Ser Asp Leu Glu
545 550 555 560

Gln Met Val Ala

<210> 40
<211> 3610
<212> DNA
<213> Pinus radiata

<400> 40
tgtttccagg cgggcactaa agcaagggag ggggtaggct ttactttctg ctctgcgcaa 60

agaacgttga aatcaatcgc cctggctggt ctggcgtgac tactagattc aatttcttca 120

tggccgtctt cacataccca ttctttaccg gttcagagct gtgatcttta tttttaacag 180

ccacaatcat ggtttgtgtt tccagtgtta tgatctgagt gaagttcgtt ctttttctcg 240

tgacccaggc ttgatactag gccggacctt tctgaggtgg aagagatcta tacatttgag 300

gcctattttg tgtagccatg tgtggaggcc cagaacattt gaagcctgcc agcccacacg 360

aaggagaaga taaagtcaaa atggccgaga atcagtctat caaagtgaag gaattgtctg 420

aatcttgttc aagtctacat gaactagctg ctaataatga ccttattggc tttaagaaag 480

caatggagga agaagggtca aagatagatg aggttaactt ttggtacggg aggcagaatg 540

gttctaatca gatggtcctg gagcaaagga ctccattgat ggttgctgca ctttatggca 600
gtgtagatgc gctgagttac atcttatcca tttatgtaac ttgtggagca gatgttaacc 660
aagcctgtgg gtcagataac tccactgcct tgcattgtgc ggctgtggga gggtctgcct 720
gtgcagttga aactgtaaaa ttgttacttc atgcaggcag tgatgtgaat cgcttggatg 780
cttatggcag aagaccagca gatgtgatta tggtttctcc taagctaacc gaaatcaagg 840
ccaagctaga agaaatgtta aacgcagctg gttcatgtca aacttctccg gcaaagttgc 900
ctaacatagt ttcagggcca cctgggtttg agtcaaaggg gatggagtcc atgtccccat 960
tgccattgtt gcctctttca ttgtctttag aagcatccaa taatagatca ggttgtgtga 1020
attctccaac atcttcgcca aagtccatgg aagcattaaa gggtttcggt gatgttaatg 1080
agaagaagga atatcctgtg gacccttctt ttccagacat aaagaatagc atctatacta 1140
cagatgaatt tcggatgttt tccttcaagg tgcggccatg ttcacgggca tattctcatg 1200
attggactga atgcccattt gtgcatcctg gtgaaaatgc cagaaggcgg gatccaagaa 1260
ggtatcatta tagctgtgtt ccttgcccag attttcggaa agggacttgt aggcgcagtg 1320
atgtttgtga atatgcacac ggtgtttttg agtgctggtt acatcctgct caatatagga 1380
cacggttgtg caaagatggg actaattgtt cacgtagagt ttgcttcttt gctcacacat 1440
ctgaggaact acgccctctc attgtctcta ctgggtctgc tgttccatcc ccaagggcat 1500
catcatctct ggacatgaca tctgtcatga gtcctcttgc ccctggttct ccctcttcag 1560
tttcaatgat gtcacccttc ctatcaaatc ctcagcaagg cagtgtgctt actccgccta 1620
tgtctccatc agcgtcctct gtaaatggat atggaggctg gccacagcct aatgtaccaa 1680
ccttacacct tcctggtagc aatgttcaaa ccagccgtct tagagcggaa cttaatgcca 1740
gagacatgcc tgttgaggat tctcctcgaa tttcagacta tgaagggcag caactcctga 1800
atgatttttc tccactgtcc acacaagcca ggctgaatgc tgctgctgct gttatatctg 1860
gtggcgggaa caccacaaca aggtctggaa aatacaagag tcacgggatc aatactgttg 1920
ctccaacgaa tcttgaagac ttgtttgcct ccgaggtaac atctcctaga gtagcagttc 1980
ttgaaccttc catcttttct cagatgagtc cccaaatgca agctcataag actgcccagg 2040
catatatgca gattcaaaac cagatgctgc ctcctataaa tacacaggca ttttcgcagg 2100
gaattacaca gatgcagcag gctgcaatag agcctcagag ccctggacat tctttgatgc 2160
aatcaccttt ccaatcttcc tcgtatgggt tgggatcccc tggtagaatg tcacctcgtt 2220
gtgtggatgt ggaacgtcat aatacatgtg ggtctccctt atcaccggct atggctgcaa 2280
cgataaattc aagaatggct atggctgctt ttgttcagag ggaaaaacgg agccatagtt 2340
cccgtgactt gggagctaat gtgaatccca gttcatggtc tgattggggc tcgcctacag 2400
gtaaagttga ctgggggggtt caaggagaag agttgagcaa attaagaaag tcggcttcat 2460
ttggtccccg cagttatgaa gaaccggatt tgtcttgggt tcaaacactg gtaaaggaaa 2520

ctacaccaga gggtaaagat ggaggaaatg taagctgttc tggggaaact ccacacaagg 2580

ggcaaataga aaatgttgat cattcagttt tgggtgcctg gattgaacag atgcagcttg 2640

atcagattgt agcttgagat taggattatt tatttggagt ggtggtaggg ataggctcat 2700

ttaaaattca atttctcatt ttttactatt tcttttataa aaattcccca ttatagttta 2760

ggaaatagtc tggttttcta cctattatca gaattacacc tgcaggaaat tttggaggaa 2820

agcatgcaaa aagtagatag ggatgttatt cctatcagca ggttgacaag ctgaaaatca 2880

cttgggtggt agaccagaga atgacactat tttttgttga catggcaact gaagatgctg 2940

ttttctttac ttatcattaa caaccctata tatatttgtt ttgaaagaac tgagcggaga 3000

aatgttgtca gttggttact ctgcgcaagg ccttggaaga aatccaagat gtggcatctt 3060

ggtgcatttt taatttatca agtgtgaaat ccataacagg tttcagtgag tgacttctga 3120

ggttgtatat ggaaaaacct atgatgttgg ctgtctactg ctatttttct gtgcctaaac 3180

tgtcaactaa agtttgcagg tggcaatttt gtggcagcat atttgcacat tgaagcggat 3240

ggtctgcacc tgctatagaa gttttcgagt ctgtagaatt tgatggtgca agatgatttt 3300

ctagttgata tatttggaag gctttgccaa agtagtggca tgtacatttt gcaaaaattt 3360

aaaggatggc aatccattgt tttgccatgt agcttcactt tattgattag gtggaaagga 3420

attttgagac acttcaattt gtgcatactt ttgttctgaa ctgcaaaatc agtctcttgt 3480

gatgtcctca aggctattat gctcagggat ttgcctaaaa ccataagtgg ccttagataa 3540

ggtaccattg tattaccttt tattgtttgg atattttatt tatgaaagtg aatttatttt 3600

aaaaaaaaaa 3610

<210> 41
<211> 779
<212> PRT
<213> Pinus radiata

<400> 41

Met Cys Gly Gly Pro Glu His Leu Lys Pro Ala Ser Pro His Glu Gly
1 5 10 15

Glu Asp Lys Val Lys Met Ala Glu Asn Gln Ser Ile Lys Val Lys Glu
20 25 30

Leu Ser Glu Ser Cys Ser Ser Leu His Glu Leu Ala Ala Asn Asn Asp
35 40 45

Leu Ile Gly Phe Lys Lys Ala Met Glu Glu Glu Gly Ser Lys Ile Asp
50 55 60

Glu Val Asn Phe Trp Tyr Gly Arg Gln Asn Gly Ser Asn Gln Met Val
65 70 75 80

Leu Glu Gln Arg Thr Pro Leu Met Val Ala Ala Leu Tyr Gly Ser Val
85 90 95

Asp Ala Leu Ser Tyr Ile Leu Ser Ile Tyr Val Thr Cys Gly Ala Asp
100 105 110

Val Asn Gln Ala Cys Gly Ser Asp Asn Ser Thr Ala Leu His Cys Ala
115 120 125

Ala Val Gly Gly Ser Ala Cys Ala Val Glu Thr Val Lys Leu Leu Leu
130 135 140

His Ala Gly Ser Asp Val Asn Arg Leu Asp Ala Tyr Gly Arg Arg Pro
145 150 155 160

Ala Asp Val Ile Met Val Ser Pro Lys Leu Thr Glu Ile Lys Ala Lys
165 170 175

Leu Glu Glu Met Leu Asn Ala Ala Gly Ser Cys Gln Thr Ser Pro Ala
180 185 190

Lys Leu Pro Asn Ile Val Ser Gly Pro Pro Gly Phe Glu Ser Lys Gly
195 200 205

Met Glu Ser Met Ser Pro Leu Pro Leu Leu Pro Leu Ser Leu Ser Leu
210 215 220

Glu Ala Ser Asn Asn Arg Ser Gly Cys Val Asn Ser Pro Thr Ser Ser
225 230 235 240

Pro Lys Ser Met Glu Ala Leu Lys Gly Phe Gly Asp Val Asn Glu Lys
245 250 255

Lys Glu Tyr Pro Val Asp Pro Ser Phe Pro Asp Ile Lys Asn Ser Ile
260 265 270

Tyr Thr Thr Asp Glu Phe Arg Met Phe Ser Phe Lys Val Arg Pro Cys
275 280 285

Ser Arg Ala Tyr Ser His Asp Trp Thr Glu Cys Pro Phe Val His Pro
290 295 300

Gly Glu Asn Ala Arg Arg Arg Asp Pro Arg Arg Tyr His Tyr Ser Cys
305 310 315 320

Val Pro Cys Pro Asp Phe Arg Lys Gly Thr Cys Arg Arg Ser Asp Val
325 330 335

```
Cys Glu Tyr Ala His Gly Val Phe Glu Cys Trp Leu His Pro Ala Gln
            340                 345                 350

Tyr Arg Thr Arg Leu Cys Lys Asp Gly Thr Asn Cys Ser Arg Arg Val
        355                 360                 365

Cys Phe Phe Ala His Thr Ser Glu Glu Leu Arg Pro Leu Ile Val Ser
    370                 375                 380

Thr Gly Ser Ala Val Pro Ser Pro Arg Ala Ser Ser Ser Leu Asp Met
385                 390                 395                 400

Thr Ser Val Met Ser Pro Leu Ala Pro Gly Ser Pro Ser Ser Val Ser
                405                 410                 415

Met Met Ser Pro Phe Leu Ser Asn Pro Gln Gln Gly Ser Val Leu Thr
            420                 425                 430

Pro Pro Met Ser Pro Ser Ala Ser Ser Val Asn Gly Tyr Gly Gly Trp
        435                 440                 445

Pro Gln Pro Asn Val Pro Thr Leu His Leu Pro Gly Ser Asn Val Gln
    450                 455                 460

Thr Ser Arg Leu Arg Ala Glu Leu Asn Ala Arg Asp Met Pro Val Glu
465                 470                 475                 480

Asp Ser Pro Arg Ile Ser Asp Tyr Glu Gly Gln Gln Leu Leu Asn Asp
                485                 490                 495

Phe Ser Pro Leu Ser Thr Gln Ala Arg Leu Asn Ala Ala Ala Ala Val
            500                 505                 510

Ile Ser Gly Gly Gly Asn Thr Thr Thr Arg Ser Gly Lys Tyr Lys Ser
        515                 520                 525

His Gly Ile Asn Thr Val Ala Pro Thr Asn Leu Glu Asp Leu Phe Ala
    530                 535                 540

Ser Glu Val Thr Ser Pro Arg Val Ala Val Leu Glu Pro Ser Ile Phe
545                 550                 555                 560

Ser Gln Met Ser Pro Gln Met Gln Ala His Lys Thr Ala Gln Ala Tyr
                565                 570                 575

Met Gln Ile Gln Asn Gln Met Leu Pro Pro Ile Asn Thr Gln Ala Phe
            580                 585                 590
```

```
Ser Gln Gly Ile Thr Gln Met Gln Gln Ala Ala Ile Glu Pro Gln Ser
        595                 600                 605

Pro Gly His Ser Leu Met Gln Ser Pro Phe Gln Ser Ser Ser Tyr Gly
    610                 615                 620

Leu Gly Ser Pro Gly Arg Met Ser Pro Arg Cys Val Asp Val Glu Arg
625                 630                 635                 640

His Asn Thr Cys Gly Ser Pro Leu Ser Pro Ala Met Ala Ala Thr Ile
                645                 650                 655

Asn Ser Arg Met Ala Met Ala Ala Phe Val Gln Arg Glu Lys Arg Ser
            660                 665                 670

His Ser Ser Arg Asp Leu Gly Ala Asn Val Asn Pro Ser Ser Trp Ser
        675                 680                 685

Asp Trp Gly Ser Pro Thr Gly Lys Val Asp Trp Gly Val Gln Gly Glu
    690                 695                 700

Glu Leu Ser Lys Leu Arg Lys Ser Ala Ser Phe Gly Pro Arg Ser Tyr
705                 710                 715                 720

Glu Glu Pro Asp Leu Ser Trp Val Gln Thr Leu Val Lys Glu Thr Thr
                725                 730                 735

Pro Glu Gly Lys Asp Gly Gly Asn Val Ser Cys Ser Gly Glu Thr Pro
            740                 745                 750

His Lys Gly Gln Ile Glu Asn Val Asp His Ser Val Leu Gly Ala Trp
        755                 760                 765

Ile Glu Gln Met Gln Leu Asp Gln Ile Val Ala
    770                 775


<210>  42
<211>  3610
<212>  DNA
<213>  Pinus radiata

<400>  42
tgtttccagg cgggcactaa agcaagggag ggggtaggct ttactttctg ctctgcgcaa      60

agaacgttga aatcaatcgc cctggctggt ctggcgtgac tactagattc aatttcttca     120

tggccgtctt cacataccca ttctttaccg gttcagagct gtgatcttta tttttaacag     180

ccacaatcat ggtttgtgtt tccagtgtta tgatctgagt gaagttcgtt ctttttctcg     240

tgacccaggc ttgatactag gccggacctt tctgaggtgg aagagatcta tacatttgag     300

gcctattttg tgtagccatg tgtggaggcc cagaacattt gaagcctgcc agcccacacg     360
```

```
aaggagaaga taaagtcaaa atggccgaga atcagtctat caaagtgaag gaattgtctg    420
aatcttgttc aagtctacat gaactagctg ctaataatga ccttattggc tttaagaaag    480
caatggagga agaagggtca aagatagatg aggttaactt ttggtacggg aggcagaatg    540
gttctaatca gatggtcctg gagcaaagga ctccattgat ggttgctgca ctttatggca    600
gtgtagatgc gctgagttac atcttatcca tttatgtaac ttgtggagca gatgttaacc    660
aagcctgtgg gtcagataac tccactgcct tgcattgtgc ggctgtggga gggtctgcct    720
gtgcagttga aactgtaaaa ttgttacttc atgcaggcag tgatgtgaat cgcttggatg    780
cttatggcag aagaccagca gatgtgatta tggtttctcc taagctaacc gaaatcaagg    840
ccaagctaga agaaatgtta aacgcagctg gttcatgtca aacttctccg gcaaagttgc    900
ctaacatagt ttcagggcca cctgggtttg agtcaaaggg gatggagtcc atgtccccat    960
tgccattgtt gcctctttca ttgtctttag aagcatccaa taatagatca ggttgtgtga   1020
attctccaac atcttcgcca aagtccatgg aagcattaaa gggtttcggt gatgttaatg   1080
agaagaagga atatcctgtg gacccttctt ttccagacat aaagaatagc atctatacta   1140
cagatgaatt tcggatgttt tccttcaagg tgcggccatg ttcacgggca tattctcatg   1200
attggactga atgcccattt gtgcatcctg gtgaaaatgc cagaaggcgg gatccaagaa   1260
ggtatcatta tagctgtgtt ccttgcccag attttcggaa agggacttgt aggcgcagtg   1320
atgtttgtga atatgcacac ggtgtttttg agtgctggtt acatcctgct caatatagga   1380
cacggttgtg caaagatggg actaattgtt cacgtagagt ttgcttcttt gctcacacat   1440
ctgaggaact acgccctctc attgtctcta ctgggtctgc tgttccatcc ccaagggcat   1500
catcatctct ggacatgaca tctgtcatga gtcctcttgc ccctggttct ccctcttcag   1560
tttcaatgat gtcacccttc ctatcaaatc ctcagcaagg cagtgtgctt actccgccta   1620
tgtctccatc agcgtcctct gtaaatggat atggaggctg gccacagcct aatgtaccaa   1680
ccttacacct tcctggtagc aatgttcaaa ccagccgtct tagagcggaa cttaatgcca   1740
gagacatgcc tgttgaggat tctcctcgaa tttcagacta tgaagggcag caactcctga   1800
atgatttttc tccactgtcc acacaagcca ggctgaatgc tgctgctgct gttatatctg   1860
gtggcgggaa caccacaaca aggtctggaa aatacaagag tcacgggatc aatactgttg   1920
ctccaacgaa tcttgaagac ttgtttgcct ccgaggtaac atctcctaga gtagcagttc   1980
ttgaaccttc catcttttct cagatgagtc cccaaatgca agctcataag actgcccagg   2040
catatatgca gattcaaaac cagatgctgc ctcctataaa tacacaggca ttttcgcagg   2100
gaattacaca gatgcagcag gctgcaatag agcctcagag ccctggacat tctttgatgc   2160
aatcaccttt ccaatcttcc tcgtatgggt tgggatcccc tggtagaatg tcacctcgtt   2220
gtgtggatgt ggaacgtcat aatacatgtg ggtctccctt atcaccggct atggctgcaa   2280
```

cgataaattc aagaatggct atggctgctt ttgttcagag ggaaaaacgg agccatagtt 2340

cccgtgactt gggagctaat gtgaatccca gttcatggtc tgattggggc tcgcctacag 2400

gtaaagttga ctggggggtt caaggagaag agttgagcaa attaagaaag tcggcttcat 2460

ttggtccccg cagttatgaa gaaccggatt tgtcttgggt tcaaacactg gtaaaggaaa 2520

ctacaccaga gggtaaagat ggaggaaatg taagctgttc tggggaaact ccacacaagg 2580

ggcaaataga aaatgttgat cattcagttt tgggtgcctg gattgaacag atgcagcttg 2640

atcagattgt agcttgagat taggattatt tatttggagt ggtggtaggg ataggctcat 2700

ttaaaattca atttctcatt ttttactatt tcttttataa aaattcccca ttatagttta 2760

ggaaatagtc tggttttcta cctattatca gaattacacc tgcaggaaat tttggaggaa 2820

agcatgcaaa aagtagatag ggatgttatt cctatcagca ggttgacaag ctgaaaatca 2880

cttgggtggt agaccagaga atgacactat tttttgttga catggcaact gaagatgctg 2940

ttttctttac ttatcattaa caaccctata tatatttgtt ttgaaagaac tgagcggaga 3000

aatgttgtca gttggttact ctgcgcaagg ccttggaaga aatccaagat gtggcatctt 3060

ggtgcatttt taatttatca agtgtgaaat ccataacagg tttcagtgag tgacttctga 3120

ggttgtatat ggaaaaacct atgatgttgg ctgtctactg ctatttttct gtgcctaaac 3180

tgtcaactaa agtttgcagg tggcaatttt gtggcagcat atttgcacat tgaagcggat 3240

ggtctgcacc tgctatagaa gttttcgagt ctgtagaatt tgatggtgca agatgatttt 3300

ctagttgata tatttggaag gctttgccaa agtagtggca tgtacatttt gcaaaaattt 3360

aaaggatggc aatccattgt tttgccatgt agcttcactt tattgattag gtggaaagga 3420

attttgagac acttcaattt gtgcatactt ttgttctgaa ctgcaaaatc agtctcttgt 3480

gatgtcctca aggctattat gctcagggat ttgcctaaaa ccataagtgg ccttagataa 3540

ggtaccattg tattaccttt tattgtttgg atattttatt tatgaaagtg aatttatttt 3600

aaaaaaaaaa 3610

<210> 43
<211> 749
<212> PRT
<213> Pinus radiata

<400> 43

Met Lys Glu Met Ala Glu Tyr Cys Ser Pro Ala Leu Leu Glu Leu Ala
1 5 10 15

Ala Asn Asn Asp Leu Ser Gly Phe Lys Gln Ala Val Glu Glu Gly Gly
20 25 30

Ser Ser Val Asn Glu Arg Gly Leu Trp Tyr Gly Arg Gln Ile Gly Ser
35 40 45

```
Gly Gln Lys Met Val Leu Glu Gln Arg Thr Pro Leu Met Val Ala Ala
    50                  55                  60

Leu Tyr Gly Ser Leu Asp Val Leu Ser Tyr Met Leu Ser Gly Gly Arg
65                  70                  75                  80

Val Asp Val Asn Gln Ser Cys Gly Ser Asp Met Ser Thr Ala Leu His
                85                  90                  95

Cys Ala Ala Ala Gly Gly Ser Ile Leu Ala Ile Glu Thr Val Gly Met
            100                 105                 110

Leu Ile Lys Ala Gly Ala Asp Val Asn Phe Met Asn Ala Gly Gly Arg
        115                 120                 125

Lys Pro Ala Asp Val Ile Met Val Ser Pro Lys Leu Ala His Phe Lys
    130                 135                 140

Asn Val Leu Glu Asp Leu Leu Ile Met Gly Ser Asn Ser Pro Met Lys
145                 150                 155                 160

Ile Pro Cys Arg Val Ser Gly Ser Gly Phe Tyr Leu Pro Glu Gly Gly
                165                 170                 175

Gly Cys Phe Phe Asp Glu His Gly Cys Val Val Ser Val Pro Thr Ser
            180                 185                 190

Ser Pro Leu Phe Ser Ser Pro Asp Ala Thr Ser Pro Ala Thr Val Asn
        195                 200                 205

Ser Pro Leu Ser Ser Pro Pro Thr Ser Leu Asp Thr Pro Lys Asn Leu
    210                 215                 220

Cys Asp Cys Gly Gln Lys Lys Glu Phe Ala Val Asp Ser Ser Leu Pro
225                 230                 235                 240

Asp Ile Lys Asn Ser Ile Tyr Ser Thr Asp Glu Phe Arg Met Tyr Ser
                245                 250                 255

Phe Lys Val Arg Pro Cys Ser Arg Ala Tyr Ser His Asp Trp Thr Glu
            260                 265                 270

Cys Pro Phe Val His Pro Gly Glu Asn Ala Arg Arg Arg Asp Pro Arg
        275                 280                 285

Lys Tyr His Tyr Ser Cys Val Pro Cys Pro Asp Phe Arg Lys Gly Ala
    290                 295                 300
```

Cys Arg Arg Gly Asp Val Cys Glu Tyr Ala His Gly Val Phe Glu Cys
305                 310                 315                 320

Trp Leu His Pro Ala Gln Tyr Arg Thr Arg Leu Cys Lys Asp Gly Thr
                325                 330                 335

Asn Cys Ser Arg Arg Val Cys Phe Phe Ala His Thr Pro Glu Glu Leu
            340                 345                 350

Arg Pro Leu Tyr Pro Pro Ala Cys Ser Ser Met Leu Ser Gln Arg Thr
        355                 360                 365

Thr Met Thr Ser Ser Asp Lys Met Ala Val Met His Pro Leu Ala Pro
    370                 375                 380

Gly Ser Ala Ser Ser Val Leu Met Met Ser Ser Ser Asn Ser Ser Gln
385                 390                 395                 400

Ser Ser Phe Pro Asn Ser Pro Val Ser Pro Leu Ser Ser Ala Asn Thr
                405                 410                 415

Ser Ser His Ser Ser Phe Gly Gly Gly Ser Trp Ala His Pro Asn Leu
            420                 425                 430

Pro Thr Leu His Leu Ser Asn Gly Ala Leu Gln Ala Ser Arg Leu Arg
        435                 440                 445

Thr Ala Val Asn Ala Arg Asp Met His Pro Asp Cys Ser Ile Glu Ser
    450                 455                 460

Gly Asp Tyr Glu Gly Gln Leu Leu Asn Glu Phe Ala Tyr Leu Ser Thr
465                 470                 475                 480

Gln Ala Arg Gly Asn Gly Pro Met Ala Thr Val Ser Ser Ser Gly Asn
                485                 490                 495

Thr Pro Cys Arg Pro Arg Lys Phe Arg Ala His Asn Val Ala Pro Thr
            500                 505                 510

Asn Leu Glu Asp Leu Phe Ala Ser Glu Val Phe Ser Pro Lys Met Thr
        515                 520                 525

Ala Ser Glu Ser Ala Phe Leu Ser Glu Ile Gln Ser His Lys Ser Ala
    530                 535                 540

Gln Leu Ser Pro Gln Leu Gln Ser Gln Met Leu Ser Ser Phe Asn Thr
545                 550                 555                 560

Gln Val Tyr Pro Gln Gly Ser Thr Gln Gly Gln Met His Met Gln His

```
                565                 570                 575

Gly Gly Val Asp Cys Gln Ser Pro Ser Val Phe Leu Ser Pro Pro Pro
            580                 585                 590

Val Gln Leu Ala Ser Tyr Ser Leu Ser Ser Leu Gly Pro Leu Ser Ser
        595                 600                 605

Leu Thr Gly Glu Leu Glu Arg Gln Asn Ser Asn Gly Ser Pro Leu Ser
    610                 615                 620

Pro Ile Met Ser Thr Ala Ala Asp Ser Arg Ala Val Ala Phe Ser Gln
625                 630                 635                 640

Arg Asp Lys Gly Ser Ser Arg Ser Gly Asp Leu Gly Gly Ala Thr Thr
                645                 650                 655

Trp Ser Glu Trp Gly Ser Pro Thr Gly Lys Val Asn Trp Gly Ile Arg
            660                 665                 670

Gly Glu Glu Leu Gln Lys Phe Arg Lys Ser Ala Ser Phe Gly Ile Arg
        675                 680                 685

Ser Ser Asp Glu Pro Asp Leu Ser Trp Val Gln Lys Leu Phe Lys Glu
    690                 695                 700

Ala Pro Met Glu Ser Met Asp Arg Gly Thr Met Gly Arg Ser Met Asp
705                 710                 715                 720

Ile Ala Asn Ser Val Gln Met Glu Ala Thr Asp Leu Gly Gly Trp Ile
                725                 730                 735

Ser Gln Ile Asn Pro Asp Gln Val Ala Pro Leu Thr Leu
            740                 745


<210>  44
<211>  711
<212>  PRT
<213>  Glycine max

<400>  44

Lys Ser Ala Asn Asp Lys Glu Met Lys Ser Leu Thr Val Asn Thr Glu
1               5                   10                  15

Asp Ser Phe Ser Ser Leu Leu Glu Leu Ala Ser Asn Asn Asp Ile Glu
            20                  25                  30

Gly Phe Lys Val Leu Leu Glu Lys Asp Ser Ser Ser Ile Asn Glu Val
        35                  40                  45
```

Gly Leu Trp Tyr Gly Arg Gln Asn Gly Ser Lys Gln Phe Val Leu Glu
    50                  55                  60

His Arg Thr Pro Leu Met Val Ala Ala Thr Tyr Gly Ser Ile Asp Val
65                  70                  75                  80

Met Lys Ile Ile Leu Leu Cys Pro Glu Ala Asp Val Asn Phe Ala Cys
                85                  90                  95

Gly Ala Asn Lys Thr Thr Ala Leu His Cys Ala Ala Ser Gly Gly Ser
            100                 105                 110

Ala Asn Ala Val Asp Ala Val Lys Ile Leu Leu Ser Ala Gly Ala Asp
        115                 120                 125

Val Asn Gly Val Asp Ala Asn Gly Asn Arg Pro Ile Asp Val Ile Ala
    130                 135                 140

Val Pro Pro Lys Leu Gln Gly Ala Lys Ala Val Leu Glu Glu Leu Leu
145                 150                 155                 160

Ser Asp Ser Ala Ser Glu Gly Ser Ile Gly Glu Phe Ser Val Pro Val
                165                 170                 175

Ser Val Asn Thr Ser Ser Leu Gly Ser Pro Gly His Ser Ser Asn Gly
            180                 185                 190

Met Pro Tyr Thr Pro Ser Ser Ser Pro Pro Ser Pro Val Val Ala Lys
        195                 200                 205

Phe Thr Asp Ala Ala Val Cys Ser Leu Ser Glu Lys Lys Glu Tyr Pro
    210                 215                 220

Ile Asp Pro Ser Leu Pro Asp Ile Lys Asn Ser Ile Tyr Ala Thr Asp
225                 230                 235                 240

Glu Phe Arg Met Phe Ser Phe Lys Val Arg Pro Cys Ser Arg Ala Tyr
                245                 250                 255

Ser His Asp Trp Thr Glu Cys Pro Phe Val His Pro Gly Glu Asn Ala
            260                 265                 270

Arg Arg Arg Asp Pro Arg Lys Phe His Tyr Ser Cys Val Pro Cys Pro
        275                 280                 285

Asp Phe Arg Lys Gly Ala Cys Arg Arg Gly Asp Met Cys Glu Tyr Ala
    290                 295                 300

His Gly Val Phe Glu Cys Trp Leu His Pro Ala Gln Tyr Arg Thr Arg
305 310 315 320

Leu Cys Lys Asp Gly Thr Ser Cys Asn Arg Arg Val Cys Phe Phe Ala
325 330 335

His Thr Ala Glu Glu Leu Arg Pro Leu Tyr Val Ser Thr Gly Ser Ala
340 345 350

Val Pro Ser Pro Arg Ser Ser Ala Ser Ala Pro Asn Val Met Asp Met
355 360 365

Ala Ala Ala Met Ser Leu Leu Pro Gly Ser Pro Ser Ser Val Ser Ser
370 375 380

Met Ser Pro Ser His Phe Gly Gln Pro Met Ser Pro Ser Ala Asn Gly
385 390 395 400

Met Ser Leu Ser Ser Ala Trp Ala Gln Pro Asn Val Ser Ala Leu His
405 410 415

Leu Pro Gly Ser Asn Leu Gln Ser Ser Arg Leu Arg Ser Ser Leu Ser
420 425 430

Ala Arg Asp Met Pro Pro Asp Asp Leu Asn Met Met Ser Asp Leu Asp
435 440 445

Gly Gln Gln Gln His Pro Leu Asn Asp Leu Ser Cys Tyr Leu Gln Pro
450 455 460

Arg Pro Gly Ala Gly Ser Val Ser Arg Ser Gly Arg Ser Lys Ile Leu
465 470 475 480

Thr Pro Ser Asn Leu Glu Asp Leu Phe Ser Ala Glu Ile Ser Ser Ser
485 490 495

Pro Arg Tyr Ser Asp Pro Ala Ala Gly Ser Val Phe Ser Pro Thr His
500 505 510

Lys Ser Ala Val Leu Asn Gln Phe Gln Gln Leu Gln Ser Met Leu Ser
515 520 525

Pro Ile Asn Thr Asn Leu Leu Ser Pro Lys Asn Val Glu His Pro Leu
530 535 540

Leu Gln Ala Ser Phe Gly Val Ser Pro Ser Gly Arg Met Ser Pro Arg
545 550 555 560

Ser Val Glu Pro Ile Ser Pro Met Ser Ser Arg Ile Ser Ala Phe Ala

565 570 575

Gln Arg Glu Lys Gln Gln Gln Gln Gln Gln Gln Leu Arg Ser Leu Ser
580 585 590

Ser Arg Asp Leu Gly Ala Asn Ser Pro Ala Ser Leu Val Gly Ser Pro
595 600 605

Ala Asn Pro Trp Ser Lys Trp Gly Ser Pro Asn Gly Lys Ala Asp Trp
610 615 620

Ser Val Asn Gly Asp Thr Leu Gly Arg Gln Met Arg Arg Ser Ser Ser
625 630 635 640

Phe Glu Leu Lys Asn Asn Gly Glu Glu Pro Asp Leu Ser Trp Val Gln
645 650 655

Ser Leu Val Lys Glu Ser Pro Pro Glu Met Ile Lys Glu Lys Phe Ala
660 665 670

Ser Pro Met Pro Thr Ala Ser Ala Asp Gly Pro Asn Ser Asn Ser Gln
675 680 685

Ile Glu Ser Ile Asp His Ser Val Leu Gly Ala Trp Leu Glu Gln Met
690 695 700

Gln Leu Asp Gln Leu Val Val
705 710

<210> 45
<211> 643
<212> PRT
<213> Glycine max

<400> 45

Arg Gly Ser Gly Phe Pro Gly Arg Pro Thr Arg Pro Arg Ser Gly Arg
1 5 10 15

Thr Arg Gly Arg Thr Arg Gly Val Asn Phe Ala Cys Gly Ala Asn Lys
20 25 30

Thr Thr Ala Leu His Cys Ala Ala Ser Gly Ala Ser Thr Lys Ala Val
35 40 45

Asp Ala Val Lys Leu Leu Leu Ser Ala Gly Ala Asp Val Asn Cys Val
50 55 60

Asp Ala Asn Gly Asn Arg Pro Ile Asp Val Ile Ala Val Pro Pro Lys
65 70 75 80

```
Leu Gln Gly Ala Lys Ala Val Leu Glu Glu Leu Leu Ser Asp Asn Ala
                85                  90                  95

Ser Asp Val Ser Val Gly Glu Phe Ser Val Pro Val Ser Val Asn Ser
            100                 105                 110

Ser Ser Pro Gly Ser Pro Ala His Ser Ser Asn Gly Met Pro Tyr Thr
        115                 120                 125

Pro Ser Val Ser Pro Pro Ser Pro Val Ala Ala Lys Phe Thr Asp Ala
    130                 135                 140

Ala Ile Cys Ser Leu Ser Glu Lys Ala Arg Glu Tyr Pro Ile Asp Pro
145                 150                 155                 160

Ser Leu Pro Asp Ile Lys Asn Ser Ile Tyr Ala Thr Asp Glu Phe Arg
                165                 170                 175

Met Phe Ser Phe Lys Val Arg Pro Cys Ser Arg Ala Tyr Ser His Asp
            180                 185                 190

Trp Thr Glu Cys Pro Phe Val His Pro Gly Glu Asn Ala Arg Arg Arg
        195                 200                 205

Asp Pro Arg Lys Phe His Tyr Ser Cys Val Pro Cys Pro Asp Phe Arg
    210                 215                 220

Lys Gly Ala Cys Arg Arg Gly Asp Met Cys Glu Tyr Ala His Gly Val
225                 230                 235                 240

Phe Glu Cys Trp Leu His Pro Ala Gln Tyr Arg Thr Arg Leu Cys Lys
                245                 250                 255

Asp Gly Thr Ser Cys Asn Arg Arg Val Cys Phe Phe Ala His Thr Ala
            260                 265                 270

Glu Glu Leu Arg Pro Leu Tyr Val Ser Thr Gly Ser Ala Ala Pro Ser
        275                 280                 285

Pro Arg Ser Ser Ala Ser Gly Pro Asn Val Met Asp Met Ala Ala Ala
    290                 295                 300

Met Ser Leu Phe Pro Gly Ser Pro Ser Ser Gly Ser Ser Ile Ser Leu
305                 310                 315                 320

Ser Ile Ser Phe Ser Leu Asp Pro Met Ser Pro Ser Ala Asn Gly Met
                325                 330                 335
```

Pro Leu Ser Ser Ala Trp Ala Gln Pro Asn Val Pro Ala Leu His Leu
340 345 350

Pro Gly Ser Asn Leu Gln Ser Ser Arg Leu Arg Ser Ser Leu Ser Ala
355 360 365

Arg Asp Ile Pro Pro Glu Asp Leu Asn Met Met Ser Asp Leu Asp Gly
370 375 380

Gln Gln Gln His His Leu Asn Asp Leu Ser Cys Tyr Ile Gln Pro Arg
385 390 395 400

Pro Gly Ala Ser Ser Val Ser Arg Ser Gly Arg Ser Lys Thr Leu Thr
405 410 415

Pro Ser Asn Leu Glu Glu Leu Phe Ser Ala Glu Ile Ser Leu Ser Pro
420 425 430

Arg Tyr Ser Asp Pro Ala Ala Gly Ser Val Phe Ser Pro Thr His Lys
435 440 445

Ser Ala Val Leu Asn Gln Phe Gln Gln Leu Gln Ser Met Leu Ser Pro
450 455 460

Ile Asn Thr Asn Leu Leu Ser Pro Lys Asn Val Glu His Pro Leu Phe
465 470 475 480

Gln Ala Ser Phe Gly Val Ser Pro Ser Gly Arg Met Ser Pro Arg Ser
485 490 495

Val Glu Pro Ile Ser Pro Met Ser Ala Arg Leu Ser Ala Phe Ala Gln
500 505 510

Arg Glu Lys Gln Gln Gln Gln Leu Arg Ser Val Ser Ser Arg Asp Leu
515 520 525

Gly Ala Asn Ser Pro Ala Ser Leu Val Gly Ser Pro Ala Asn Pro Trp
530 535 540

Ser Lys Trp Gly Ser Pro Ile Gly Lys Ala Asp Trp Ser Val Asn Gly
545 550 555 560

Asp Ser Leu Gly Arg Gln Met Arg Arg Ser Ser Ser Phe Glu Arg Lys
565 570 575

Asn Asn Gly Glu Glu Pro Asp Leu Ser Trp Val Gln Ser Leu Val Lys
580 585 590

Glu Ser Pro Pro Glu Met Ile Lys Glu Lys Phe Ala Ser Pro Met Pro

595 600 605

Thr Ala Ser Ala Asp Gly Pro Asn Ser Asn Ser Gln Ile Glu Ser Ile
610 615 620

Asp His Ser Val Leu Gly Ala Trp Leu Glu Gln Met Gln Leu Asp Gln
625 630 635 640

Leu Val Val

<210> 46
<211> 669
<212> PRT
<213> Glycine max

<400> 46

Ser His Glu Met Asn His Leu Ser Leu Asp Thr Glu Asp Ser Leu Ala
1 5 10 15

Ser Leu Leu Leu Glu Leu Ala Ala Asn Asn Asp Val Ser Gly Phe Lys
20 25 30

Arg Leu Ile Glu Cys Glu Pro Ser Ser Ile Asp Glu Val Gly Leu Trp
35 40 45

Tyr Gly Arg His Lys Glu Ser Lys Lys Met Val Asn Glu Gln Arg Thr
50 55 60

Pro Leu Met Val Ala Ala Thr Tyr Gly Ser Ile Asp Val Met Thr Leu
65 70 75 80

Ile Leu Ser Leu Ser Glu Ala Asp Val Asn Arg Ser Ser Gly Leu Asp
85 90 95

Lys Ser Thr Ala Leu His Cys Ala Ala Ser Gly Gly Ser Glu Asn Ala
100 105 110

Val Asp Ala Val Lys Leu Leu Leu Glu Ala Gly Ala Asp Arg Asn Ser
115 120 125

Val Asp Ala Asn Gly Arg Arg Pro Gly Asp Val Ile Val Ser Pro Pro
130 135 140

Lys Leu Asp Tyr Val Lys Lys Ser Leu Glu Glu Leu Leu Gly Ser Asp
145 150 155 160

Asp Trp Ser Leu Leu Arg Val Met Arg Ser Thr Cys Asn Gly Cys Ser
165 170 175

```
Ala Glu Asp Leu Lys Met Lys Thr Asn Glu Val Ser Glu Lys Lys Glu
            180                 185                 190

Tyr Pro Val Asp Leu Ser Leu Pro Asp Ile Lys Asn Ser Ile Tyr Ser
        195                 200                 205

Ser Asp Glu Phe Arg Met Tyr Ser Phe Lys Val Arg Pro Cys Ser Arg
    210                 215                 220

Ala Tyr Ser His Asp Trp Thr Glu Cys Pro Phe Val His Pro Gly Glu
225                 230                 235                 240

Asn Ala Arg Arg Arg Asp Pro Arg Lys Phe His Tyr Ser Cys Val Pro
                245                 250                 255

Cys Pro Glu Phe Arg Lys Gly Ala Cys Arg Arg Gly Asp Met Cys Glu
            260                 265                 270

Tyr Ala His Gly Val Phe Glu Cys Trp Leu His Pro Ala Gln Tyr Arg
        275                 280                 285

Thr Arg Leu Cys Lys Asp Gly Thr Asn Cys Ala Arg Arg Val Cys Phe
    290                 295                 300

Phe Ala His Thr Asn Glu Glu Leu Arg Pro Leu Tyr Val Ser Thr Gly
305                 310                 315                 320

Ser Ala Val Pro Ser Pro Arg Ser Ser Ala Ser Ser Ala Met Asp Phe
                325                 330                 335

Val Ala Ala Ile Ser Pro Ser Ser Met Ser Val Met Ser Pro Ser Pro
            340                 345                 350

Phe Thr Pro Pro Met Ser Pro Ser Ser Ala Ser Ile Ala Trp Pro Gln
        355                 360                 365

Pro Asn Ile Pro Ala Leu His Leu Pro Gly Ser Asn Phe His Ser Ser
    370                 375                 380

Arg Leu Arg Ser Ser Leu Asn Ala Arg Asp Phe Ser Val Asp Asp Phe
385                 390                 395                 400

Asp Leu Leu Leu Pro Asp Tyr Asp His His His His Gln Gln Gln Gln
                405                 410                 415

Gln Gln Phe Leu Asn Glu Leu Ser Cys Leu Ser Pro His Ala Met Asn
            420                 425                 430
```

Cys Asn Thr Met Asn Arg Ser Gly Arg Met Lys Pro Leu Thr Pro Ser
435 440 445

Asn Leu Asp Asp Leu Phe Ser Ala Glu Ser Ser Ser Pro Arg Tyr Ala
450 455 460

Asp Pro Ala Leu Ala Ser Ala Val Phe Ser Pro Thr His Lys Ser Ala
465 470 475 480

Val Phe Asn Gln Phe Gln His Gln Gln Ser Met Leu Ala Pro Leu Asn
485 490 495

Thr Asn Phe Ala Ser Lys Asn Phe Glu His Pro Leu Leu Gln Ala Ser
500 505 510

Leu Gly Met Ser Pro Arg Asn Val Glu Pro Ile Ser Pro Met Gly Ser
515 520 525

Arg Ile Ser Met Leu Ala Gln Arg Glu Lys Gln Gln Phe Arg Ser Leu
530 535 540

Ser Phe Arg Glu Leu Gly Ser Asn Ser Ala Ala Ala Ser Ala Asp Ser
545 550 555 560

Trp Ser Lys Trp Gly Ser Pro Asn Val Lys Leu Asp Trp Pro Val Gly
565 570 575

Ala Gly Glu Val Gly Lys Leu Arg Arg Ser Ser Ser Phe Glu Leu Gly
580 585 590

Asn Asn Gly Glu Glu Pro Asp Leu Ser Trp Val Gln Ser Leu Val Lys
595 600 605

Glu Ser Pro Ala Glu Val Lys Asp Lys Leu Ala Thr Thr Val Ser Tyr
610 615 620

Val Ala Ala Ala Ala Ala Gly Ser Ser Ser Glu Gly Ser Asn Ile Ser
625 630 635 640

Thr Gln Met Glu Ser Val Val Asp His Ala Val Leu Gly Ala Trp Leu
645 650 655

Glu Gln Met Gln Leu Asp His Leu Val Ala Gln Gln Asn
660 665

<210> 47
<211> 580
<212> PRT
<213> Arabidopsis thaliana

<400> 47

Met Cys Ser Gly Pro Lys Ser Asn Leu Cys Ser Ser Arg Thr Leu Thr
1               5                   10                  15

Glu Ile Glu Ser Arg Gln Lys Glu Glu Glu Thr Met Leu Leu Leu Glu
            20                  25                  30

Phe Ala Ala Cys Asp Asp Leu Asp Ser Phe Lys Arg Glu Val Glu Glu
        35                  40                  45

Lys Gly Leu Asp Leu Asp Glu Ser Gly Leu Trp Tyr Cys Arg Arg Val
    50                  55                  60

Gly Ser Lys Lys Met Gly Leu Glu Glu Arg Thr Pro Leu Met Val Ala
65                  70                  75                  80

Ala Met Tyr Gly Ser Ile Lys Val Leu Thr Phe Ile Val Ser Thr Gly
                85                  90                  95

Lys Ser Asp Val Asn Arg Ala Cys Gly Glu Glu Arg Val Thr Pro Leu
            100                 105                 110

His Cys Ala Val Ala Gly Cys Ser Val Asn Met Ile Glu Val Ile Asn
        115                 120                 125

Val Leu Leu Asp Ala Ser Ala Leu Val Asn Ser Val Asp Ala Asn Gly
    130                 135                 140

Asn Gln Pro Leu Asp Val Phe Val Arg Val Ser Arg Phe Val Ala Ser
145                 150                 155                 160

Pro Arg Arg Lys Ala Val Glu Leu Leu Leu Arg Gly Gly Gly Val Gly
                165                 170                 175

Gly Leu Ile Asp Glu Ala Val Glu Glu Glu Ile Lys Ile Val Ser Lys
            180                 185                 190

Tyr Pro Ala Asp Ala Ser Leu Pro Asp Ile Asn Glu Gly Val Tyr Gly
        195                 200                 205

Ser Asp Glu Phe Arg Met Tyr Ser Phe Lys Val Lys Pro Cys Ser Arg
    210                 215                 220

Ala Tyr Ser His Asp Trp Thr Glu Cys Ala Phe Val His Pro Gly Glu
225                 230                 235                 240

Asn Ala Arg Arg Arg Asp Pro Arg Lys Tyr Pro Tyr Thr Cys Val Pro
                245                 250                 255

Cys Pro Glu Phe Arg Lys Gly Ser Cys Pro Lys Gly Asp Ser Cys Glu
260 265 270

Tyr Ala His Gly Val Phe Glu Ser Trp Leu His Pro Ala Gln Tyr Lys
275 280 285

Thr Arg Leu Cys Lys Asp Glu Thr Gly Cys Ala Arg Lys Val Cys Phe
290 295 300

Phe Ala His Lys Arg Glu Glu Met Arg Pro Val Asn Ala Ser Thr Gly
305 310 315 320

Ser Ala Val Ala Gln Ser Pro Phe Ser Ser Leu Glu Met Met Pro Gly
325 330 335

Leu Ser Pro Leu Ala Tyr Ser Ser Gly Val Ser Thr Pro Pro Val Ser
340 345 350

Pro Met Ala Asn Gly Val Pro Ser Ser Pro Arg Asn Gly Gly Ser Trp
355 360 365

Gln Asn Arg Val Asn Thr Leu Thr Pro Pro Ala Leu Gln Leu Asn Gly
370 375 380

Gly Ser Arg Leu Lys Ser Thr Leu Ser Ala Arg Asp Ile Asp Met Glu
385 390 395 400

Met Glu Met Glu Leu Arg Leu Arg Gly Phe Gly Asn Asn Val Glu Glu
405 410 415

Thr Phe Gly Ser Tyr Val Ser Ser Pro Ser Arg Asn Ser Gln Met Gly
420 425 430

Gln Asn Met Asn Gln His Tyr Pro Ser Ser Pro Val Arg Gln Pro Pro
435 440 445

Ser Gln His Gly Phe Glu Ser Ser Ala Ala Ala Ala Val Ala Val Met
450 455 460

Lys Ala Arg Ser Thr Ala Phe Ala Lys Arg Ser Leu Ser Phe Lys Pro
465 470 475 480

Ala Thr Gln Ala Ala Pro Gln Ser Asn Leu Ser Asp Trp Gly Ser Pro
485 490 495

Asn Gly Lys Leu Glu Trp Gly Met Lys Gly Glu Glu Leu Asn Lys Met
500 505 510

```
Arg Arg Ser Val Ser Phe Gly Ile His Gly Asn Asn Asn Asn Asn Ala
        515                 520                 525

Ala Arg Asp Tyr Arg Asp Glu Pro Asp Val Ser Trp Val Asn Ser Leu
    530                 535                 540

Val Lys Asp Ser Thr Val Val Ser Glu Arg Ser Phe Gly Met Asn Glu
545                 550                 555                 560

Arg Val Arg Ile Met Ser Trp Ala Glu Gln Met Tyr Arg Glu Lys Glu
                565                 570                 575

Gln Thr Val Val
            580


<210>  48
<211>  719
<212>  PRT
<213>  Arabidopsis thaliana

<400>  48

Met Cys Cys Gly Ser Asp Arg Leu Asn Gln Ile Val Ser Ser Arg Ser
1               5                   10                  15

Ser Leu Pro Ile Ser Phe Glu Glu Asp Asn Asn Leu Val Thr Asn Thr
            20                  25                  30

Asp Met Asn His Ile Thr Val Glu Thr Glu Asp Thr Phe Ala Ser Leu
        35                  40                  45

Leu Glu Leu Ala Ala Asn Asn Asp Val Glu Gly Val Arg Leu Ser Ile
    50                  55                  60

Glu Arg Asp Pro Ser Cys Val Asp Glu Ala Gly Leu Trp Tyr Gly Arg
65                  70                  75                  80

Gln Lys Gly Ser Lys Ala Met Val Asn Asp Tyr Arg Thr Pro Leu Met
                85                  90                  95

Val Ala Ala Thr Tyr Gly Ser Ile Asp Val Ile Lys Leu Ile Val Ser
            100                 105                 110

Leu Thr Asp Ala Asp Val Asn Arg Ala Cys Gly Asn Asp Gln Thr Thr
        115                 120                 125

Ala Leu His Cys Ala Ala Ser Gly Gly Ala Val Asn Ala Ile Gln Val
    130                 135                 140

Val Lys Leu Leu Leu Ala Ala Gly Ala Asp Leu Asn Leu Leu Asp Ala
145                 150                 155                 160
```

Glu Gly Gln Arg Ala Gly Asp Val Ile Val Val Pro Pro Lys Leu Glu
165 170 175

Gly Val Lys Leu Met Leu Gln Glu Leu Leu Ser Ala Asp Gly Ser Ser
180 185 190

Thr Ala Glu Arg Asn Leu Arg Val Val Thr Asn Val Pro Asn Arg Ser
195 200 205

Ser Ser Pro Cys His Ser Pro Thr Gly Glu Asn Gly Gly Ser Gly Ser
210 215 220

Gly Ser Pro Leu Gly Ser Pro Phe Lys Leu Lys Ser Thr Glu Phe Lys
225 230 235 240

Lys Glu Tyr Pro Val Asp Pro Ser Leu Pro Asp Ile Lys Asn Ser Ile
245 250 255

Tyr Ala Thr Asp Glu Phe Arg Met Tyr Ser Phe Lys Val Arg Pro Cys
260 265 270

Ser Arg Ala Tyr Ser His Asp Trp Thr Glu Cys Pro Phe Val His Pro
275 280 285

Gly Glu Asn Ala Arg Arg Arg Asp Pro Arg Lys Phe His Tyr Ser Cys
290 295 300

Val Pro Cys Pro Asp Phe Arg Lys Gly Ala Cys Arg Arg Gly Asp Met
305 310 315 320

Cys Glu Tyr Ala His Gly Val Phe Glu Cys Trp Leu His Pro Ala Gln
325 330 335

Tyr Arg Thr Arg Leu Cys Lys Asp Gly Thr Gly Cys Ala Arg Arg Val
340 345 350

Cys Phe Phe Ala His Thr Pro Glu Glu Leu Arg Pro Leu Tyr Ala Ser
355 360 365

Thr Gly Ser Ala Val Pro Ser Pro Arg Ser Asn Ala Asp Tyr Ala Ala
370 375 380

Ala Leu Ser Leu Leu Pro Gly Ser Pro Ser Gly Val Ser Val Met Ser
385 390 395 400

Pro Leu Ser Pro Ser Ala Ala Gly Asn Gly Met Ser His Ser Asn Met
405 410 415

Ala Trp Pro Gln Pro Asn Val Pro Ala Leu His Leu Pro Gly Ser Asn
420 425 430

Leu Gln Ser Ser Arg Leu Arg Ser Ser Leu Asn Ala Arg Asp Ile Pro
435 440 445

Thr Asp Glu Phe Asn Met Leu Ala Asp Tyr Glu Gln Gln Gln Leu Leu
450 455 460

Asn Glu Tyr Ser Asn Ala Leu Ser Arg Ser Gly Arg Met Lys Ser Met
465 470 475 480

Pro Pro Ser Asn Leu Glu Asp Leu Phe Ser Ala Glu Gly Ser Ser Ser
485 490 495

Pro Arg Phe Thr Asp Ser Ala Leu Ala Ser Ala Val Phe Ser Pro Thr
500 505 510

His Lys Ser Ala Val Phe Asn Gln Phe Gln Gln Gln Gln Gln Gln Gln
515 520 525

Gln Gln Gln Ser Met Leu Ser Pro Ile Asn Thr Ser Phe Ser Ser Pro
530 535 540

Lys Ser Val Asp His Ser Leu Phe Ser Gly Gly Gly Arg Met Ser Pro
545 550 555 560

Arg Asn Val Val Glu Pro Ile Ser Pro Met Ser Ala Arg Val Ser Met
565 570 575

Leu Ala Gln Cys Val Lys Gln Gln Gln Gln Gln Gln Gln Gln Gln Gln
580 585 590

Gln Gln Gln His Gln Phe Arg Ser Leu Ser Ser Arg Glu Leu Arg Thr
595 600 605

Asn Ser Ser Pro Ile Val Gly Ser Pro Val Asn Asn Asn Thr Trp Ser
610 615 620

Ser Lys Trp Gly Ser Ser Asn Gly Gln Pro Asp Trp Gly Met Ser Ser
625 630 635 640

Glu Ala Leu Gly Lys Leu Arg Ser Ser Ser Ser Phe Asp Gly Asp Glu
645 650 655

Pro Asp Val Ser Trp Val Gln Ser Leu Val Lys Glu Thr Pro Ala Glu
660 665 670

Ala Lys Glu Lys Ala Ala Thr Ser Ser Ser Gly Glu His Val Met Lys
675 680 685

Gln Pro Asn Pro Val Glu Pro Val Met Asp His Ala Gly Leu Glu Ala
690 695 700

Trp Ile Glu Gln Met Gln Leu Asp Gln Leu Val Ala Gln Gln Asn
705 710 715

<210> 49
<211> 686
<212> PRT
<213> Arabidopsis thaliana

<400> 49

Met Asn His Ile Thr Val Glu Thr Glu Asp Thr Phe Ala Ser Leu Leu
1 5 10 15

Glu Leu Ala Ala Asn Asn Asp Val Glu Gly Val Arg Leu Ser Ile Glu
20 25 30

Arg Asp Pro Ser Cys Val Asp Glu Ala Gly Leu Trp Tyr Gly Arg Gln
35 40 45

Lys Gly Ser Lys Ala Met Val Asn Asp Tyr Arg Thr Pro Leu Met Val
50 55 60

Ala Ala Thr Tyr Gly Ser Ile Asp Val Ile Lys Leu Ile Val Ser Leu
65 70 75 80

Thr Asp Ala Asp Val Asn Arg Ala Cys Gly Asn Asp Gln Thr Thr Ala
85 90 95

Leu His Cys Ala Ala Ser Gly Gly Ala Val Asn Ala Ile Gln Val Val
100 105 110

Lys Leu Leu Leu Ala Ala Gly Ala Asp Leu Asn Leu Leu Asp Ala Glu
115 120 125

Gly Gln Arg Ala Gly Asp Val Ile Val Val Pro Pro Lys Leu Glu Gly
130 135 140

Val Lys Leu Met Leu Gln Glu Leu Leu Ser Ala Asp Gly Ser Ser Thr
145 150 155 160

Ala Glu Arg Asn Leu Arg Val Val Thr Asn Val Pro Asn Arg Ser Ser
165 170 175

Ser Pro Cys His Ser Pro Thr Gly Glu Asn Gly Gly Ser Gly Ser Gly
180 185 190

Ser Pro Leu Gly Ser Pro Phe Lys Leu Lys Ser Thr Glu Phe Lys Lys
        195                 200                 205

Glu Tyr Pro Val Asp Pro Ser Leu Pro Asp Ile Lys Asn Ser Ile Tyr
    210                 215                 220

Ala Thr Asp Glu Phe Arg Met Tyr Ser Phe Lys Val Arg Pro Cys Ser
225                 230                 235                 240

Arg Ala Tyr Ser His Asp Trp Thr Glu Cys Pro Phe Val His Pro Gly
                245                 250                 255

Glu Asn Ala Arg Arg Arg Asp Pro Arg Lys Phe His Tyr Ser Cys Val
            260                 265                 270

Pro Cys Pro Asp Phe Arg Lys Gly Ala Cys Arg Arg Gly Asp Met Cys
        275                 280                 285

Glu Tyr Ala His Gly Val Phe Glu Cys Trp Leu His Pro Ala Gln Tyr
    290                 295                 300

Arg Thr Arg Leu Cys Lys Asp Gly Thr Gly Cys Ala Arg Arg Val Cys
305                 310                 315                 320

Phe Phe Ala His Thr Pro Glu Glu Leu Arg Pro Leu Tyr Ala Ser Thr
                325                 330                 335

Gly Ser Ala Val Pro Ser Pro Arg Ser Asn Ala Asp Tyr Ala Ala Ala
            340                 345                 350

Leu Ser Leu Leu Pro Gly Ser Pro Ser Gly Val Ser Val Met Ser Pro
        355                 360                 365

Leu Ser Pro Ser Ala Ala Gly Asn Gly Met Ser His Ser Asn Met Ala
    370                 375                 380

Trp Pro Gln Pro Asn Val Pro Ala Leu His Leu Pro Gly Ser Asn Leu
385                 390                 395                 400

Gln Ser Ser Arg Leu Arg Ser Ser Leu Asn Ala Arg Asp Ile Pro Thr
                405                 410                 415

Asp Glu Phe Asn Met Leu Ala Asp Tyr Glu Gln Gln Gln Leu Leu Asn
            420                 425                 430

Glu Tyr Ser Asn Ala Leu Ser Arg Ser Gly Arg Met Lys Ser Met Pro
        435                 440                 445

Pro Ser Asn Leu Glu Asp Leu Phe Ser Ala Glu Gly Ser Ser Ser Pro
    450                 455                 460

Arg Phe Thr Asp Ser Ala Leu Ala Ser Ala Val Phe Ser Pro Thr His
465                 470                 475                 480

Lys Ser Ala Val Phe Asn Gln Phe Gln Gln Gln Gln Gln Gln Gln Gln
                485                 490                 495

Gln Gln Ser Met Leu Ser Pro Ile Asn Thr Ser Phe Ser Ser Pro Lys
            500                 505                 510

Ser Val Asp His Ser Leu Phe Ser Gly Gly Gly Arg Met Ser Pro Arg
        515                 520                 525

Asn Val Val Glu Pro Ile Ser Pro Met Ser Ala Arg Val Ser Met Leu
    530                 535                 540

Ala Gln Cys Val Lys Gln Gln Gln Gln Gln Gln Gln Gln Gln Gln Gln
545                 550                 555                 560

Gln Gln His Gln Phe Arg Ser Leu Ser Ser Arg Glu Leu Arg Thr Asn
                565                 570                 575

Ser Ser Pro Ile Val Gly Ser Pro Val Asn Asn Asn Thr Trp Ser Ser
            580                 585                 590

Lys Trp Gly Ser Ser Asn Gly Gln Pro Asp Trp Gly Met Ser Ser Glu
        595                 600                 605

Ala Leu Gly Lys Leu Arg Ser Ser Ser Ser Phe Asp Gly Asp Glu Pro
    610                 615                 620

Asp Val Ser Trp Val Gln Ser Leu Val Lys Glu Thr Pro Ala Glu Ala
625                 630                 635                 640

Lys Glu Lys Ala Ala Thr Ser Ser Ser Gly Glu His Val Met Lys Gln
                645                 650                 655

Pro Asn Pro Val Glu Pro Val Met Asp His Ala Gly Leu Glu Ala Trp
            660                 665                 670

Ile Glu Gln Met Gln Leu Asp Gln Leu Val Ala Gln Gln Asn
        675                 680                 685

<210> 50
<211> 633
<212> PRT
<213> Arabidopsis thaliana

<400> 50

Met Val Asn Asp Tyr Arg Thr Pro Leu Met Val Ala Ala Thr Tyr Gly
1               5                   10                  15

Ser Ile Asp Val Ile Lys Leu Ile Val Ser Leu Thr Asp Ala Asp Val
            20                  25                  30

Asn Arg Ala Cys Gly Asn Asp Gln Thr Thr Ala Leu His Cys Ala Ala
        35                  40                  45

Ser Gly Gly Ala Val Asn Ala Ile Gln Val Val Lys Leu Leu Leu Ala
    50                  55                  60

Ala Gly Ala Asp Leu Asn Leu Leu Asp Ala Glu Gly Gln Arg Ala Gly
65                  70                  75                  80

Asp Val Ile Val Val Pro Pro Lys Leu Glu Gly Val Lys Leu Met Leu
                85                  90                  95

Gln Glu Leu Leu Ser Ala Asp Gly Ser Ser Thr Ala Glu Arg Asn Leu
            100                 105                 110

Arg Val Val Thr Asn Val Pro Asn Arg Ser Ser Ser Pro Cys His Ser
        115                 120                 125

Pro Thr Gly Glu Asn Gly Gly Ser Gly Ser Gly Ser Pro Leu Gly Ser
    130                 135                 140

Pro Phe Lys Leu Lys Ser Thr Glu Phe Lys Lys Glu Tyr Pro Val Asp
145                 150                 155                 160

Pro Ser Leu Pro Asp Ile Lys Asn Ser Ile Tyr Ala Thr Asp Glu Phe
                165                 170                 175

Arg Met Tyr Ser Phe Lys Val Arg Pro Cys Ser Arg Ala Tyr Ser His
            180                 185                 190

Asp Trp Thr Glu Cys Pro Phe Val His Pro Gly Glu Asn Ala Arg Arg
        195                 200                 205

Arg Asp Pro Arg Lys Phe His Tyr Ser Cys Val Pro Cys Pro Asp Phe
    210                 215                 220

Arg Lys Gly Ala Cys Arg Arg Gly Asp Met Cys Glu Tyr Ala His Gly
225                 230                 235                 240

Val Phe Glu Cys Trp Leu His Pro Ala Gln Tyr Arg Thr Arg Leu Cys
                245                 250                 255

```
Lys Asp Gly Thr Gly Cys Ala Arg Arg Val Cys Phe Phe Ala His Thr
            260                 265                 270

Pro Glu Glu Leu Arg Pro Leu Tyr Ala Ser Thr Gly Ser Ala Val Pro
        275                 280                 285

Ser Pro Arg Ser Asn Ala Asp Tyr Ala Ala Ala Leu Ser Leu Leu Pro
    290                 295                 300

Gly Ser Pro Ser Gly Val Ser Val Met Ser Pro Leu Ser Pro Ser Ala
305                 310                 315                 320

Ala Gly Asn Gly Met Ser His Ser Asn Met Ala Trp Pro Gln Pro Asn
                325                 330                 335

Val Pro Ala Leu His Leu Pro Gly Ser Asn Leu Gln Ser Ser Arg Leu
            340                 345                 350

Arg Ser Ser Leu Asn Ala Arg Asp Ile Pro Thr Asp Glu Phe Asn Met
        355                 360                 365

Leu Ala Asp Tyr Glu Gln Gln Gln Leu Leu Asn Glu Tyr Ser Asn Ala
    370                 375                 380

Leu Ser Arg Ser Gly Arg Met Lys Ser Met Pro Pro Ser Asn Leu Glu
385                 390                 395                 400

Asp Leu Phe Ser Ala Glu Gly Ser Ser Ser Pro Arg Phe Thr Asp Ser
                405                 410                 415

Ala Leu Ala Ser Ala Val Phe Ser Pro Thr His Lys Ser Ala Val Phe
            420                 425                 430

Asn Gln Phe Gln Gln Gln Gln Gln Gln Gln Gln Gln Gln Ser Met Leu
        435                 440                 445

Ser Pro Ile Asn Thr Ser Phe Ser Ser Pro Lys Ser Val Asp His Ser
    450                 455                 460

Leu Phe Ser Gly Gly Gly Arg Met Ser Pro Arg Asn Val Val Glu Pro
465                 470                 475                 480

Ile Ser Pro Met Ser Ala Arg Val Ser Met Leu Ala Gln Cys Val Lys
                485                 490                 495

Gln Gln Gln Gln Gln Gln Gln Gln Gln Gln Gln Gln Gln His Gln Phe
            500                 505                 510
```

Arg Ser Leu Ser Ser Arg Glu Leu Arg Thr Asn Ser Ser Pro Ile Val
        515                 520                 525

Gly Ser Pro Val Asn Asn Asn Thr Trp Ser Ser Lys Trp Gly Ser Ser
    530                 535                 540

Asn Gly Gln Pro Asp Trp Gly Met Ser Ser Glu Ala Leu Gly Lys Leu
545                 550                 555                 560

Arg Ser Ser Ser Ser Phe Asp Gly Asp Glu Pro Asp Val Ser Trp Val
                565                 570                 575

Gln Ser Leu Val Lys Glu Thr Pro Ala Glu Ala Lys Glu Lys Ala Ala
            580                 585                 590

Thr Ser Ser Ser Gly Glu His Val Met Lys Gln Pro Asn Pro Val Glu
        595                 600                 605

Pro Val Met Asp His Ala Gly Leu Glu Ala Trp Ile Glu Gln Met Gln
    610                 615                 620

Leu Asp Gln Leu Val Ala Gln Gln Asn
625                 630

<210> 51
<211> 678
<212> PRT
<213> Arabidopsis thaliana

<400> 51

Met Asn Asp Ala Ala Glu Trp Glu His Ser Phe Ser Ala Leu Leu Glu
1               5                   10                  15

Phe Ala Ala Asp Asn Asp Val Glu Gly Phe Arg Arg Gln Leu Ser Asp
            20                  25                  30

Val Ser Cys Ile Asn Gln Met Gly Leu Trp Tyr Arg Arg Gln Arg Phe
        35                  40                  45

Val Arg Arg Met Val Leu Glu Gln Arg Thr Pro Leu Met Val Ala Ser
    50                  55                  60

Leu Tyr Gly Ser Leu Asp Val Val Lys Phe Ile Leu Ser Phe Pro Glu
65                  70                  75                  80

Ala Glu Leu Asn Leu Ser Cys Gly Pro Asp Lys Ser Thr Ala Leu His
                85                  90                  95

Cys Ala Ala Ser Gly Ala Ser Val Asn Ser Leu Asp Val Val Lys Leu

```
            100                 105                 110

Leu Leu Ser Val Gly Ala Asp Pro Asn Ile Pro Asp Ala His Gly Asn
        115                 120                 125

Arg Pro Val Asp Val Leu Val Val Ser Pro His Ala Pro Gly Leu Arg
    130                 135                 140

Thr Ile Leu Glu Glu Ile Leu Lys Lys Asp Glu Ile Ile Ser Glu Asp
145                 150                 155                 160

Leu His Ala Ser Ser Ser Ser Leu Gly Ser Ser Phe Arg Ser Leu Ser
                165                 170                 175

Ser Ser Pro Asp Asn Gly Ser Ser Leu Leu Ser Leu Asp Ser Val Ser
            180                 185                 190

Ser Pro Thr Lys Pro His Gly Thr Asp Val Thr Phe Ala Ser Glu Lys
        195                 200                 205

Lys Glu Tyr Pro Ile Asp Pro Ser Leu Pro Asp Ile Lys Ser Gly Ile
    210                 215                 220

Tyr Ser Thr Asp Glu Phe Arg Met Phe Ser Phe Lys Ile Arg Pro Cys
225                 230                 235                 240

Ser Arg Ala Tyr Ser His Asp Trp Thr Glu Cys Pro Phe Ala His Pro
                245                 250                 255

Gly Glu Asn Ala Arg Arg Arg Asp Pro Arg Lys Phe His Tyr Thr Cys
            260                 265                 270

Val Pro Cys Pro Asp Phe Lys Lys Gly Ser Cys Lys Gln Gly Asp Met
        275                 280                 285

Cys Glu Tyr Ala His Gly Val Phe Glu Cys Trp Leu His Pro Ala Gln
    290                 295                 300

Tyr Arg Thr Arg Leu Cys Lys Asp Gly Met Gly Cys Asn Arg Arg Val
305                 310                 315                 320

Cys Phe Phe Ala His Ala Asn Glu Glu Leu Arg Pro Leu Tyr Pro Ser
                325                 330                 335

Thr Gly Ser Gly Leu Pro Ser Pro Arg Ala Ser Ser Ala Val Ser Ala
            340                 345                 350

Ser Thr Met Asp Met Ala Ser Val Leu Asn Met Leu Pro Gly Ser Pro
        355                 360                 365
```

```
Ser Ala Ala Gln His Ser Phe Thr Pro Pro Ile Ser Pro Ser Gly Asn
    370                 375                 380

Gly Ser Met Pro His Ser Ser Met Gly Trp Pro Gln Gln Asn Ile Pro
385                 390                 395                 400

Ala Leu Asn Leu Pro Gly Ser Asn Ile Gln Leu Ser Arg Leu Arg Ser
                405                 410                 415

Ser Leu Asn Ala Arg Asp Ile Pro Ser Glu Gln Leu Ser Met Leu His
            420                 425                 430

Glu Phe Glu Met Gln Arg Gln Leu Ala Gly Asp Met His Ser Pro Arg
        435                 440                 445

Phe Met Asn His Ser Ala Arg Pro Lys Thr Leu Asn Pro Ser Asn Leu
    450                 455                 460

Glu Glu Leu Phe Ser Ala Glu Val Ala Ser Pro Arg Phe Ser Asp Gln
465                 470                 475                 480

Leu Ala Val Ser Ser Val Leu Ser Pro Ser His Lys Ser Ala Leu Leu
                485                 490                 495

Asn Gln Leu Gln Asn Asn Lys Gln Ser Met Leu Ser Pro Ile Lys Thr
            500                 505                 510

Asn Leu Met Ser Ser Pro Lys Asn Val Glu Gln His Ser Leu Leu Gln
        515                 520                 525

Gln Ala Ser Ser Pro Arg Gly Gly Glu Pro Ile Ser Pro Met Asn Ala
    530                 535                 540

Arg Met Lys Gln Gln Leu His Ser Arg Ser Leu Ser Ser Arg Asp Phe
545                 550                 555                 560

Gly Ser Ser Leu Pro Arg Asp Leu Met Pro Thr Asp Ser Gly Ser Pro
                565                 570                 575

Leu Ser Pro Trp Ser Ser Trp Asp Gln Thr His Gly Ser Lys Val Asp
            580                 585                 590

Trp Ser Val Gln Ser Asp Glu Leu Gly Arg Leu Arg Lys Ser His Ser
        595                 600                 605

Leu Ala Asn Asn Pro Asn Arg Glu Ala Asp Val Ser Trp Ala Gln Gln
    610                 615                 620
```

Met Leu Lys Asp Ser Ser Ser Pro Arg Asn Gly Asn Arg Val Val Asn
625 630 635 640

Met Asn Gly Ala Arg Pro Leu Thr Gln Gly Gly Ser Ser Val Asn Pro
645 650 655

His Asn Ser Asp Thr Arg Glu Ser Asp Ile Leu Asp Ala Trp Leu Glu
660 665 670

Gln Leu His Leu Asp Arg
675

<210> 52
<211> 640
<212> PRT
<213> Arabidopsis thaliana

<400> 52

Met Gly Leu Trp Tyr Arg Arg Gln Arg Phe Val Arg Arg Met Val Leu
1 5 10 15

Glu Gln Arg Thr Pro Leu Met Val Ala Ser Leu Tyr Gly Ser Leu Asp
20 25 30

Val Val Lys Phe Ile Leu Ser Phe Pro Glu Ala Glu Leu Asn Leu Ser
35 40 45

Cys Gly Pro Asp Lys Ser Thr Ala Leu His Cys Ala Ala Ser Gly Ala
50 55 60

Ser Val Asn Ser Leu Asp Val Val Lys Leu Leu Leu Ser Val Gly Ala
65 70 75 80

Asp Pro Asn Ile Pro Asp Ala His Gly Asn Arg Pro Val Asp Val Leu
85 90 95

Val Val Ser Pro His Ala Pro Gly Leu Arg Thr Ile Leu Glu Glu Ile
100 105 110

Leu Lys Lys Asp Glu Ile Ile Ser Glu Asp Leu His Ala Ser Ser Ser
115 120 125

Ser Leu Gly Ser Ser Phe Arg Ser Leu Ser Ser Ser Pro Asp Asn Gly
130 135 140

Ser Ser Leu Leu Ser Leu Asp Ser Val Ser Ser Pro Thr Lys Pro His
145 150 155 160

Gly Thr Asp Val Thr Phe Ala Ser Glu Lys Lys Glu Tyr Pro Ile Asp

165 170 175

Pro Ser Leu Pro Asp Ile Lys Ser Gly Ile Tyr Ser Thr Asp Glu Phe
180 185 190

Arg Met Phe Ser Phe Lys Ile Arg Pro Cys Ser Arg Ala Tyr Ser His
195 200 205

Asp Trp Thr Glu Cys Pro Phe Ala His Pro Gly Glu Asn Ala Arg Arg
210 215 220

Arg Asp Pro Arg Lys Phe His Tyr Thr Cys Val Pro Cys Pro Asp Phe
225 230 235 240

Lys Lys Gly Ser Cys Lys Gln Gly Asp Met Cys Glu Tyr Ala His Gly
245 250 255

Val Phe Glu Cys Trp Leu His Pro Ala Gln Tyr Arg Thr Arg Leu Cys
260 265 270

Lys Asp Gly Met Gly Cys Asn Arg Arg Val Cys Phe Phe Ala His Ala
275 280 285

Asn Glu Glu Leu Arg Pro Leu Tyr Pro Ser Thr Gly Ser Gly Leu Pro
290 295 300

Ser Pro Arg Ala Ser Ser Ala Val Ser Ala Ser Thr Met Asp Met Ala
305 310 315 320

Ser Val Leu Asn Met Leu Pro Gly Ser Pro Ser Ala Ala Gln His Ser
325 330 335

Phe Thr Pro Pro Ile Ser Pro Ser Gly Asn Gly Ser Met Pro His Ser
340 345 350

Ser Met Gly Trp Pro Gln Gln Asn Ile Pro Ala Leu Asn Leu Pro Gly
355 360 365

Ser Asn Ile Gln Leu Ser Arg Leu Arg Ser Ser Leu Asn Ala Arg Asp
370 375 380

Ile Pro Ser Glu Gln Leu Ser Met Leu His Glu Phe Glu Met Gln Arg
385 390 395 400

Gln Leu Ala Gly Asp Met His Ser Pro Arg Phe Met Asn His Ser Ala
405 410 415

Arg Pro Lys Thr Leu Asn Pro Ser Asn Leu Glu Glu Leu Phe Ser Ala
420 425 430

Glu Val Ala Ser Pro Arg Phe Ser Asp Gln Leu Ala Val Ser Ser Val
435 440 445

Leu Ser Pro Ser His Lys Ser Ala Leu Leu Asn Gln Leu Gln Asn Asn
450 455 460

Lys Gln Ser Met Leu Ser Pro Ile Lys Thr Asn Leu Met Ser Ser Pro
465 470 475 480

Lys Asn Val Glu Gln His Ser Leu Leu Gln Gln Ala Ser Ser Pro Arg
485 490 495

Gly Gly Glu Pro Ile Ser Pro Met Asn Ala Arg Met Lys Gln Gln Leu
500 505 510

His Ser Arg Ser Leu Ser Ser Arg Asp Phe Gly Ser Ser Leu Pro Arg
515 520 525

Asp Leu Met Pro Thr Asp Ser Gly Ser Pro Leu Ser Pro Trp Ser Ser
530 535 540

Trp Asp Gln Thr His Gly Ser Lys Val Asp Trp Ser Val Gln Ser Asp
545 550 555 560

Glu Leu Gly Arg Leu Arg Lys Ser His Ser Leu Ala Asn Asn Pro Asn
565 570 575

Arg Glu Ala Asp Val Ser Trp Ala Gln Gln Met Leu Lys Asp Ser Ser
580 585 590

Ser Pro Arg Asn Gly Asn Arg Val Val Asn Met Asn Gly Ala Arg Pro
595 600 605

Leu Thr Gln Gly Gly Ser Ser Val Asn Pro His Asn Ser Asp Thr Arg
610 615 620

Glu Ser Asp Ile Leu Asp Ala Trp Leu Glu Gln Leu His Leu Asp Arg
625 630 635 640

<210> 53
<211> 2158
<212> DNA
<213> Oryza sativa

<400> 53
tgctccttct tcattgcaaa gaaagcacca ccttttaaag aatcctcctc acactccatt 60

cttcttaaaa aacccaccac aacacaattc ccacttgttt cttcatcatc acctacttca 120

atcaaaaaac attccaactt tcttctcaat ttcattccag gatagtatta tgtgcagtgg 180

accaaagagc aatctctgct cttcaagaac cttaacagaa atcgaatcaa ggcaaaagga 240
agaagaaaca atgcttctcc tcgaattcgc tgcttgtgat gatcttgact cgttcaagag 300
agaggttgaa gagaaagggc ttgatttgga tgagtcaggg ttatggtatt gcagacgtgt 360
cggttctaag aagatgggtc ttgaagaaag aacaccttta atggttgcag ctatgtatgg 420
aagcataaag gttttgactt tcatcgtttc cactggaaaa tctgatgtga acagagcttg 480
tggtgaagag agagttactc cgcttcactg tgctgttgct ggctgttctg tgaatatgat 540
tgaagtcatc aatgtcttgc ttgatgcttc tgctttggtt aactctgttg atgctaatgg 600
gaatcaacct ttggatgtgt ttgttcgagt ttcgaggttt gtggctagtc cgaggaggaa 660
agcggttgag ttgttgctga gaggaggagg tgttggagga ttgatcgatg aggcggttga 720
agaagagatc aagattgtct ctaagtatcc agctgatgct tctttaccgg atataaacga 780
aggggtttat ggaagtgatg agtttaggat gtatagcttt aaggttaagc catgttctag 840
ggcttattct catgattgga ccgagtgtgc ttttgttcat ccgggagaaa atgcgaggag 900
gagagatccg aggaagtatc cttacacttg tgtcccctgt cccgagttcc gtaaaggatc 960
atgcccgaaa ggagattctt gcgagtatgc tcacggggtt ttcgagtcgt ggcttcaccc 1020
cgcgcagtat aaaacccggc tttgtaaaga tgaaacgggt tgtgcaagga aagtttgttt 1080
ctttgctcat aaacgcgaag agatgagacc tgttaatgct tcaactggct ctgccgtggc 1140
tcagtctccg tttagcagct tggagatgat gccagggttg tctcctcttg cttattcttc 1200
aggagtttcg actcctccgg tttctccaat ggctaatggt gttccttcct ctccaagaaa 1260
cggcggatca tggcagaaca gagtcaatac ccttactcca ccggctttgc agctcaatgg 1320
tggaagcaga ttgaagtcca cactgagcgc tagagatatc gatatggaga tggagatgga 1380
attgagactc cgcggttttg gcaacaatgt ggaagagacg ttcgggtctt atgtttcctc 1440
tccaagtagg aattctcaaa tgggtcaaaa catgaaccaa cattatccat cttccccggt 1500
gagacaaccg ccatctcaac acgggttcga atcttcagca gctgcagcgg ttgcagtgat 1560
gaaagcgaga tcaaccgcct ttgcgaaacg tagcttgagc ttcaaaccag ctactcaagc 1620
agcaccacag tcgaatctct cggattgggg atctccaaac gggaagctgg aatggggaat 1680
gaaaggagaa gagctgaata agatgagaag aagtgtttcc tttggaatcc atggaaacaa 1740
caacaataac gcagctagag actacaggga cgagccagat gtgtcatggg ttaactcttt 1800
agttaaagac agtactgtgg tgtctgagag aagctttgga atgaatgaga gggttcggat 1860
aatgtcgtgg gctgagcaaa tgtacagaga gaaggagcag actgtggtgt aaacacacac 1920
aaagatggtt tcttatatat attgcttttg ggccatctct gcaaatttga ttctttaatt 1980
tttgtgactt tctttagttg ttactgttat tagtagtata tggtttgttg tcactacgag 2040
tctacgtgat gaaaagatag aagttaattg cattagtttc tatattcgtt tctcatcctc 2100

ttgtaattta tcaaaccatg aaatggctaa gcaatccaaa ccgaaaaaaa aaaaaaa 2158

<210> 54
<211> 2193
<212> DNA
<213> Oryza sativa

<400> 54
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag attttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctcctcctc ccatctataa attcctcccc ccttttcccc tctctatata 1020

ggaggcatcc aagccaagaa gagggagagc accaaggaca cgcgactagc agaagccgag 1080

cgaccgcctt cttcgatcca tatcttccgg tcgagttctt ggtcgatctc ttccctcctc 1140

cacctcctcc tcacagggta tgtgcccttc ggttgttctt ggatttattg ttctaggttg 1200

tgtagtacgg gcgttgatgt taggaaaggg gatctgtatc tgtgatgatt cctgttcttg 1260

gatttgggat agaggggttc ttgatgttgc atgttatcgg ttcggtttga ttagtagtat 1320

ggttttcaat cgtctggaga gctctatgga aatgaaatgg tttagggtac ggaatcttgc 1380

gattttgtga gtacctttttg tttgaggtaa aatcagagca ccggtgattt tgcttggtgt 1440

aataaaagta cggttgtttg gtcctcgatt ctggtagtga tgcttctcga tttgacgaag 1500

ctatcctttg tttattccct attgaacaaa aataatccaa ctttgaagac ggtcccgttg 1560

atgagattga atgattgatt cttaagcctg tccaaaattt cgcagctggc ttgtttagat 1620

acagtagtcc ccatcacgaa attcatggaa acagttataa tcctcaggaa caggggattc 1680

cctgttcttc cgatttgctt tagtcccaga attttttttc ccaaatatct taaaaagtca 1740

ctttctggtt cagttcaatg aattgattgc tacaaataat gcttttatag cgttatccta 1800

gctgtagttc agttaatagg taataccccct atagtttagt caggagaaga acttatccga 1860

tttctgatct ccatttttaa ttatatgaaa tgaactgtag cataagcagt attcatttgg 1920

attatttttt ttattagctc tcaccccttc attattctga gctgaaagtc tggcatgaac 1980

tgtcctcaat tttgttttca aattcacatc gattatctat gcattatcct cttgtatcta 2040

cctgtagaag tttctttttg gttattcctt gactgcttga ttacagaaag aaatttatga 2100

agctgtaatc gggatagtta tactgcttgt tcttatgatt catttccttt gtgcagttct 2160

tggtgtagct tgccactttc accagcaaag ttc 2193

<210> 55
<211> 1827
<212> DNA
<213> Oryza sativa

<400> 55
gcttgagtca tagggagaaa acaaatcgat catatttgac tcttttccct ccatctctct 60

taccggcaaa aaaagtagta ctggtttata tgtaaagtaa gattctttaa ttatgtgaga 120

tccggcttaa tgcttttctt ttgtcacata tactgcattg caacaattgc catatattca 180

cttctgccat cccattatat agcaactcaa gaatggattg atatatcccc tattactaat 240

ctagacatgt taaggctgag ttgggcagtc catcttccca acccaccacc ttcgtttttc 300

gcgcacatac ttttcaaact actaaatggt gtgtttttta aaaatatttt caatacaaaa 360

gttgctttaa aaaattatat tgatccattt ttttaaaaaa aatagctaat acttaattaa 420

tcacgtgtta aaagaccgct ccgttttgcg tgcaggaggg ataggttcac atcctgcatt 480

accgaacaca gcctaaatct tgttgtctag attcgtagta ctggatatat taaatcatgt 540

tctaagttac tatatactga gatgaataga ataagtaaaa ttagacccac cttaagtctt 600

gatgaagtta ctactagctg cgtttgggag gacttcccaa aaaaaaaagt attagccatt 660

agcacgtgat taattaagta ctagtttaaa aaacttaaaa aataaattaa tatgattctc 720

ttaagtaact ctcctataga aaacttttac aaaattacac cgtttaatag tttggaaaat 780

atgtcagtaa aaaataagag agtagaagtt atgaaagtta gaaaaagaat tgttttagta 840

gtatacagtt ataaactatt ccctctgttc taaaacataa gggattatgg atggattcga 900

catgtaccag taccatgaat cgaatccaga caagtttttt atgcatattt attctactat 960

aatatatcac atctgctcta aatatcttat atttcgaggt ggagactgtc gctatgtttt 1020

tctgcccgtt gctaagcaca cgccaccccc gatgcgggga cgcctctggc cttcttgcca 1080

cgataattga atggaacttc cacattcaga ttcgataggt gaccgtcgac tccaagtgct 1140

ttgcacaaaa caactccggc ctcccggcca ccagtcacac gactcacggc actaccaccc 1200

ctgactccct gaggcggacc tgccactgtt ctgcatgcga agctatctaa aattctgaag 1260

caaagaaagc acagcacatg ctccgggaca cgcgccaccc ggcggaaaag ggctcggtgt 1320

ggcgatctca cagccgcata tcgcatttca caagccgccc atctccaccg gcttcacgag 1380

gctcatcgcg gcacgaccgc gcacggaacg cacgcggccg acccgcgcgc ctcgatgcgc 1440

gagcccatcc gccgcgtcct ccctttgcct ttgccgctat cctctcggtc gtatcccgtt 1500

tctctgtctt ttgctccccg gcgcgcgcca gttcggagta ccagcgaaac ccggacacct 1560

ggtacacctc cgccggccac aacgcgtgtc cccctacgtg gccgcgcagc acatgcccat 1620

gcgcgacacg tgcacctcct catccaaact ctcaagtctc aacggtccta taaatgcacg 1680

gatagcctca agctgctcgt cacaaggcaa gaggcaagag gcaagagcat ccgtattaac 1740

cagccttttg agacttgaga gtgtgtgtga ctcgatccag cgtagtttca gttcgtgtgt 1800

tggtgagtga ttccagccaa gtttgcg 1827

<210> 56
<211> 2194
<212> DNA
<213> Oryza sativa

<400> 56
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag attttttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctccttctc ccatctataa attcctcccc ccttttcccc tctctatata 1020

ggaggcatcc aagccaagaa gagggagagc accaaggaca cgcgactagc agaagccgag 1080

cgaccgcctt ctcgatccat atcttccggt cgagttcttg gtcgatctct tccctcctcc 1140

acctcctcct cacagggtat gtgcctccct tcggttgttc ttggatttat tgttctaggt 1200

tgtgtagtac gggcgttgat gttaggaaag gggatctgta tctgtgatga ttcctgttct 1260

tggatttggg atagaggggt tcttgatgtt gcatgttatc ggttcggttt gattagtagt 1320

atggttttca atcgtctgga gagctctatg gaaatgaaat ggtttaggga tcggaatctt 1380

gcgattttgt gagtaccttt tgtttgaggt aaaatcagag caccggtgat tttgcttggt 1440

gtaataaagt acggttgttt ggtcctcgat tctggtagtg atgcttctcg atttgacgaa 1500

gctatccttt gtttattccc tattgaacaa aaataatcca actttgaaga cggtcccgtt 1560

gatgagattg aatgattgat tcttaagcct gtccaaaatt tcgcagctgg cttgtttaga 1620

tacagtagtc cccatcacga aattcatgga aacagttata atcctcagga acaggggatt 1680

ccctgttctt ccgatttgct ttagtcccag aatttttttt cccaaatatc ttaaaaagtc 1740

actttctggt tcagttcaat gaattgattg ctacaaataa tgcttttata gcgttatcct 1800

agctgtagtt cagttaatag gtaatacccc tatagtttag tcaggagaag aacttatccg 1860

atttctgatc tccattttta attatatgaa atgaactgta gcataagcag tattcatttg 1920

gattattttt tttattagct ctcacccctt cattattctg agctgaaagt ctggcatgaa 1980

ctgtcctcaa ttttgttttc aaattcacat cgattatcta tgcattatcc tcttgtatct 2040

acctgtagaa gtttcttttt ggttattcct tgactgcttg attacagaaa gaaatttatg 2100

aagctgtaat cgggatagtt atactgcttg ttcttatgat tcatttcctt tgtgcagttc 2160

ttggtgtagc ttgccacttt caccagcaaa gttc 2194

<210> 57
<211> 46
<212> PRT
<213> Artificial sequence

<220>
<223> SNH domain

<220>
<221> VARIANT
<222> (3)..(3)
<223> /replace = "Lys"

<220>
<221> UNSURE
<222> (4)..(4)
<223> Xaa can be any naturally occurring amino acid

<220>
<221> VARIANT
<222> (6)..(6)
<223> /replace = "Glu"

<220>
<221> VARIANT

<222> (7)..(7)
<223> /replace = "Asp"

<220>
<221> VARIANT
<222> (9)..(9)
<223> /replace = "Asn"

<220>
<221> UNSURE
<222> (10)..(10)
<223> Xaa can be any naturally occurring amino acid

<220>
<221> UNSURE
<222> (13)..(13)
<223> Xaa can be any naturally occurring amino acid

<220>
<221> VARIANT
<222> (14)..(14)
<223> /replace = "Ala" /replace = "Lys"

<220>
<221> VARIANT
<222> (16)..(16)
<223> /replace = "Val" /replace = "Met"

<220>
<221> VARIANT
<222> (17)..(17)
<223> /replace = "Asp" /replace = "Ser"

<220>
<221> VARIANT
<222> (18)..(18)
<223> /replace = "Asn"

<220>
<221> VARIANT
<222> (19)..(19)
<223> /replace = "Leu"

<220>
<221> UNSURE
<222> (21)..(21)
<223> Xaa can be any naturally occurring amino acid

<220>
<221> VARIANT
<222> (23)..(23)
<223> /replace = "Arg"

<220>
<221> UNSURE
<222> (24)..(25)
<223> Xaa can be any naturally occurring amino acid

<220>
<221> VARIANT
<222> (28)..(28)
<223> /replace = "Asp" /replace = "Ser"

<220>
<221> UNSURE

<222> (29)..(30)
<223> Xaa can be any naturally occurring amino acid

<220>
<221> VARIANT
<222> (32)..(32)
<223> /replace = "Ser" /replace = "Gln"

<220>
<221> UNSURE
<222> (33)..(33)
<223> Xaa can be any naturally occurring amino acid

<220>
<221> VARIANT
<222> (35)..(35)
<223> /replace = "His"

<220>
<221> UNSURE
<222> (36)..(36)
<223> Xaa can be any naturally occurring amino acid

<220>
<221> VARIANT
<222> (39)..(39)
<223> /replace = "Leu" /replace = "Val"

<220>
<221> VARIANT
<222> (43)..(43)
<223> /replace = "Thr"

<400> 57

```
Ile Gln Gln Xaa Leu Asp Glu Asn Lys Xaa Leu Ile Xaa Cys Ile Leu
1               5                   10                  15

Glu Ser Gln Asn Xaa Gly Lys Xaa Xaa Glu Cys Ala Xaa Xaa Gln Ala
            20                  25                  30

Xaa Leu Gln Xaa Asn Leu Met Tyr Leu Ala Ala Ile Ala Asp
        35                  40                  45
```

<210> 58
<211> 46
<212> PRT
<213> Arabidopsis thaliana

<400> 58

```
Ile Gln Gln Tyr Leu Asp Glu Asn Lys Ser Leu Ile Leu Lys Ile Val
1               5                   10                  15

Glu Ser Gln Asn Ser Gly Lys Leu Ser Glu Cys Ala Glu Asn Gln Ala
            20                  25                  30

Arg Leu Gln Arg Asn Leu Met Tyr Leu Ala Ala Ile Ala Asp
        35                  40                  45
```

<210> 59
<211> 633
<212> DNA
<213> Arabidopsis thaliana

<400> 59
atgcaacagc acctgatgca gatgcagccc atgatggctg gttactaccc cagcaatgtt 60

acctctgatc atatccaaca gtacttggac gaaaacaaat cgttgattct gaagattgtt 120

gagtctcaaa actctggaaa gcttagcgaa tgcgccgaga atcaagcaag gcttcaacgc 180

aacctaatgt acctagctgc aatagcagat tctcagcctc agccaccaag tgtgcatagc 240

cagtatggat ctgctggtgg tgggatgatt cagggagaag gagggtcaca ctatttgcag 300

cagcaacaag cgactcaaca gcaacagatg actcagcagt ctctaatggc ggctcgatct 360

tcaatgttgt atgctcagca acagcagcag cagcagcctt acgcgacgct tcagcatcag 420

caattgcacc atagccagct tggaatgagc tcgagcagcg gaggaggagg aagcagtggt 480

ctccatatcc ttcagggaga ggctggtggg tttcatgatt ttggccgtgg gaagccggaa 540

atgggaagtg gtggtggcgg tgaaggcaga ggaggaagtt caggggatgg tggagaaacc 600

ctttacttga aatcatcaga tgatgggaat tga 633

<210> 60
<211> 210
<212> PRT
<213> Arabidopsis thaliana

<400> 60

Met Gln Gln His Leu Met Gln Met Gln Pro Met Met Ala Gly Tyr Tyr
1 5 10 15

Pro Ser Asn Val Thr Ser Asp His Ile Gln Gln Tyr Leu Asp Glu Asn
20 25 30

Lys Ser Leu Ile Leu Lys Ile Val Glu Ser Gln Asn Ser Gly Lys Leu
35 40 45

Ser Glu Cys Ala Glu Asn Gln Ala Arg Leu Gln Arg Asn Leu Met Tyr
50 55 60

Leu Ala Ala Ile Ala Asp Ser Gln Pro Gln Pro Pro Ser Val His Ser
65 70 75 80

Gln Tyr Gly Ser Ala Gly Gly Gly Met Ile Gln Gly Glu Gly Gly Ser
85 90 95

His Tyr Leu Gln Gln Gln Gln Ala Thr Gln Gln Gln Gln Met Thr Gln
100 105 110

Gln Ser Leu Met Ala Ala Arg Ser Ser Met Leu Tyr Ala Gln Gln Gln
        115                 120                 125

Gln Gln Gln Gln Pro Tyr Ala Thr Leu Gln His Gln Gln Leu His His
    130                 135                 140

Ser Gln Leu Gly Met Ser Ser Ser Ser Gly Gly Gly Gly Ser Ser Gly
145                 150                 155                 160

Leu His Ile Leu Gln Gly Glu Ala Gly Gly Phe His Asp Phe Gly Arg
                165                 170                 175

Gly Lys Pro Glu Met Gly Ser Gly Gly Gly Gly Glu Gly Arg Gly Gly
            180                 185                 190

Ser Ser Gly Asp Gly Gly Glu Thr Leu Tyr Leu Lys Ser Ser Asp Asp
        195                 200                 205

Gly Asn
    210

<210> 61
<211> 588
<212> DNA
<213> Arabidopsis thaliana

<400> 61
atgcagcagc agcagtctcc gcaaatgttt ccgatggttc cgtcgattcc ccctgctaac 60

aacatcacta ccgaacagat ccaaaagtac cttgatgaga acaagaagct gattatggcc 120

atcatggaaa accagaatct cggtaaactt gctgagtgcg cccagtacca agctcttctc 180

cagaagaact tgatgtatct tgctgcaatt gctgatgctc aaccccccacc acctacgcca 240

ggaccttcac catctacagc tgtcgctgcc cagatggcaa caccgcattc tgggatgcaa 300

ccacctagct acttcatgca acacccacaa gcatcccctg cagggatttt cgctccaagg 360

ggtcctttac agtttggtag cccactccag tttcaggatc cgcaacagca gcagcagata 420

catcagcaag ctatgcaagg acacatgggg attagaccaa tgggtatgac caacaacggg 480

atgcagcatg cgatgcaaca accagaaacc ggtcttggag gaaacgtggg gcttagagga 540

ggaaagcaag atggagcaga tggacaagga aaagatgatg gcaagtga 588

<210> 62
<211> 195
<212> PRT
<213> Arabidopsis thaliana

<400> 62

Met Gln Gln Gln Gln Ser Pro Gln Met Phe Pro Met Val Pro Ser Ile
1               5                   10                  15

```
Pro Pro Ala Asn Asn Ile Thr Thr Glu Gln Ile Gln Lys Tyr Leu Asp
            20                  25                  30

Glu Asn Lys Lys Leu Ile Met Ala Ile Met Glu Asn Gln Asn Leu Gly
        35                  40                  45

Lys Leu Ala Glu Cys Ala Gln Tyr Gln Ala Leu Leu Gln Lys Asn Leu
    50                  55                  60

Met Tyr Leu Ala Ala Ile Ala Asp Ala Gln Pro Pro Pro Pro Thr Pro
65                  70                  75                  80

Gly Pro Ser Pro Ser Thr Ala Val Ala Ala Gln Met Ala Thr Pro His
                85                  90                  95

Ser Gly Met Gln Pro Pro Ser Tyr Phe Met Gln His Pro Gln Ala Ser
            100                 105                 110

Pro Ala Gly Ile Phe Ala Pro Arg Gly Pro Leu Gln Phe Gly Ser Pro
        115                 120                 125

Leu Gln Phe Gln Asp Pro Gln Gln Gln Gln Gln Ile His Gln Gln Ala
    130                 135                 140

Met Gln Gly His Met Gly Ile Arg Pro Met Gly Met Thr Asn Asn Gly
145                 150                 155                 160

Met Gln His Ala Met Gln Gln Pro Glu Thr Gly Leu Gly Gly Asn Val
                165                 170                 175

Gly Leu Arg Gly Gly Lys Gln Asp Gly Ala Asp Gly Gln Gly Lys Asp
            180                 185                 190

Asp Gly Lys
        195


<210>  63
<211>  672
<212>  DNA
<213>  Arabidopsis thaliana

<400>  63
atgcagcaat ctccacagat gattccgatg gttcttcctt catttccgcc caccaataat      60

atcaccaccg aacagatcca aaagtatctt gatgagaaca agaagctgat aatggcgatc     120

ttggaaaatc agaacctcgg taaacttgca gaatgtgctc agtatcaagc tcttctccag     180

aagaatttga tgtatctcgc tgcaattgcg gatgctcaac ctcagccacc agcagctaca     240

ctaacatcag gagccatgac tccccaagca atggctccta atccgtcatc aatgcagcca     300
```

ccaccaagct acttcatgca gcaacatcaa gctgtgggaa tggctcaaca aatacctcct 360

gggattttcc ctcctagagg tccattgcaa tttggtagcc cgcatcagtt tctggatccg 420

cagcaacagt tacatcaaca agctatgcaa gggcacatgg ggattagacc aatgggtttg 480

aataataaca acggactgca acatcaaatg caccaccatg aaactgctct tgccgcaaac 540

aatgcgggtc ctaacgatgc tagtggagga ggtaaaccgg atgggaccaa tatgagccag 600

agtggagctg atgggcaagg tggctcagcc gctagacatg gcggtggtga tgcaaaaact 660

gaaggaaaat ga 672

<210> 64
<211> 223
<212> PRT
<213> Arabidopsis thaliana

<400> 64

Met Gln Gln Ser Pro Gln Met Ile Pro Met Val Leu Pro Ser Phe Pro
1 5 10 15

Pro Thr Asn Asn Ile Thr Thr Glu Gln Ile Gln Lys Tyr Leu Asp Glu
20 25 30

Asn Lys Lys Leu Ile Met Ala Ile Leu Glu Asn Gln Asn Leu Gly Lys
35 40 45

Leu Ala Glu Cys Ala Gln Tyr Gln Ala Leu Leu Gln Lys Asn Leu Met
50 55 60

Tyr Leu Ala Ala Ile Ala Asp Ala Gln Pro Gln Pro Pro Ala Ala Thr
65 70 75 80

Leu Thr Ser Gly Ala Met Thr Pro Gln Ala Met Ala Pro Asn Pro Ser
85 90 95

Ser Met Gln Pro Pro Pro Ser Tyr Phe Met Gln Gln His Gln Ala Val
100 105 110

Gly Met Ala Gln Gln Ile Pro Pro Gly Ile Phe Pro Pro Arg Gly Pro
115 120 125

Leu Gln Phe Gly Ser Pro His Gln Phe Leu Asp Pro Gln Gln Gln Leu
130 135 140

His Gln Gln Ala Met Gln Gly His Met Gly Ile Arg Pro Met Gly Leu
145 150 155 160

Asn Asn Asn Asn Gly Leu Gln His Gln Met His His His Glu Thr Ala
165 170 175

Leu Ala Ala Asn Asn Ala Gly Pro Asn Asp Ala Ser Gly Gly Gly Lys
180 185 190

Pro Asp Gly Thr Asn Met Ser Gln Ser Gly Ala Asp Gly Gln Gly Gly
195 200 205

Ser Ala Ala Arg His Gly Gly Gly Asp Ala Lys Thr Glu Gly Lys
210 215 220

<210> 65
<211> 633
<212> DNA
<213> Aspergillus officinalis

<400> 65
atgcagcagc acctgatgca gatgcagccc atgatggcaa cctacggttc accgaatcag 60

gtcaccaccg atatcattca gcagtatctg gacgagaaca agcagttgat tctggctatt 120

cttgaaaacc aaaattcagg aaaagctgat gaatgtgctg agaatcaggc taagcttcag 180

aggaatctga tgtatcttgc agccattgcg gatagccagc cccaagttcc taccattgct 240

cagtatcctc ccaacgctgt tgctgctatg caatcgagtg ctcgctacat gcaacaacac 300

caagcagctc aacagatgac ccctcaatct ctcatggctg ctcgctcctc aatgctctac 360

tcacagtccc caatgtctgc actccagcag caacagcagc aagcagcaat gcatagccag 420

ctcgccatga gctccggagg caacaacagc agcaccggag gattcaccat tcttcatggt 480

gaagctagca taggaggcaa tggctcaatg aattctggtg gagtctttgg agattttgga 540

cggagcagcg gtgggaagca agagactggg agcgaagggc acgggacaga gactcctatg 600

tacctgaaag gctctgaaga agaaggaaac tga 633

<210> 66
<211> 210
<212> PRT
<213> Aspergillus officinalis

<400> 66

Met Gln Gln His Leu Met Gln Met Gln Pro Met Met Ala Thr Tyr Gly
1 5 10 15

Ser Pro Asn Gln Val Thr Thr Asp Ile Ile Gln Gln Tyr Leu Asp Glu
20 25 30

Asn Lys Gln Leu Ile Leu Ala Ile Leu Glu Asn Gln Asn Ser Gly Lys
35 40 45

Ala Asp Glu Cys Ala Glu Asn Gln Ala Lys Leu Gln Arg Asn Leu Met
50 55 60

```
Tyr Leu Ala Ala Ile Ala Asp Ser Gln Pro Gln Val Pro Thr Ile Ala
65                  70                  75                  80

Gln Tyr Pro Pro Asn Ala Val Ala Ala Met Gln Ser Ser Ala Arg Tyr
                85                  90                  95

Met Gln Gln His Gln Ala Ala Gln Gln Met Thr Pro Gln Ser Leu Met
            100                 105                 110

Ala Ala Arg Ser Ser Met Leu Tyr Ser Gln Ser Pro Met Ser Ala Leu
        115                 120                 125

Gln Gln Gln Gln Gln Gln Ala Ala Met His Ser Gln Leu Ala Met Ser
    130                 135                 140

Ser Gly Gly Asn Asn Ser Ser Thr Gly Gly Phe Thr Ile Leu His Gly
145                 150                 155                 160

Glu Ala Ser Ile Gly Gly Asn Gly Ser Met Asn Ser Gly Gly Val Phe
                165                 170                 175

Gly Asp Phe Gly Arg Ser Ser Gly Gly Lys Gln Glu Thr Gly Ser Glu
            180                 185                 190

Gly His Gly Thr Glu Thr Pro Met Tyr Leu Lys Gly Ser Glu Glu Glu
        195                 200                 205

Gly Asn
    210
```

<210> 67
<211> 591
<212> DNA
<213> Brassica napus

<400> 67

```
atgcagccca tgatggctgg ttactacccc agcaatgtca cctctgatca tatccagcag      60

tacttggatg agaacaagtc tttgattctg aagatagttg agtctcaaaa ctcaggaaag     120

ctcagcgagt gtgccgagaa tcaggcaagg cttcaacgca acctcatgta cttggctgca     180

atagcagatt ctcagcctca acctccaagc gtgcatagcc agtatggatc tgctggtggt     240

gggttgattc agggagaagg agcgtcacac tatttgcagc agcaacaggc gactcaacag     300

cagcagatga ctcagcagtc tcttatggca gctcgttctt caatgatgta tcagcagcag     360

caacagcctt atgcaacgct tcagcatcag cagttgcacc atagccagct tgggatgagc     420

tctagcagcg gaggaggaag cagtggtctc catatccttc agggagaggc tggtgggttt     480

catgaatttg gccgtgggaa gccggagatg ggaagtggtg aaggcagggg tggaagctca     540

ggggatggtg gagaaacact ctacttgaag tcatcagatg atgggaactg a              591
```

<210> 68
<211> 203
<212> PRT
<213> Brassica napus

<400> 68

```
Met Gln Gln His Leu Met Gln Met Gln Pro Met Met Ala Gly Tyr Tyr
1               5                   10                  15

Pro Ser Asn Val Thr Ser Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Ser Glu Cys Ala Glu Asn Gln Ala Arg Leu Gln Arg Asn Leu Met Tyr
    50                  55                  60

Leu Ala Ala Ile Ala Asp Ser Gln Pro Gln Pro Pro Ser Val His Ser
65                  70                  75                  80

Gln Tyr Gly Ser Ala Gly Gly Gly Leu Ile Gln Gly Glu Gly Ala Ser
                85                  90                  95

His Tyr Leu Gln Gln Gln Gln Ala Thr Gln Gln Gln Gln Met Thr Gln
            100                 105                 110

Gln Ser Leu Met Ala Ala Arg Ser Ser Met Met Tyr Gln Gln Gln Gln
        115                 120                 125

Gln Pro Tyr Ala Thr Leu Gln His Gln Gln Leu His His Ser Gln Leu
    130                 135                 140

Gly Met Ser Ser Ser Ser Gly Gly Gly Ser Ser Gly Leu His Ile Leu
145                 150                 155                 160

Gln Gly Glu Ala Gly Gly Phe His Glu Phe Gly Arg Gly Lys Pro Glu
                165                 170                 175

Met Gly Ser Gly Glu Gly Arg Gly Gly Ser Ser Gly Asp Gly Gly Glu
            180                 185                 190

Thr Leu Tyr Leu Lys Ser Ser Asp Asp Gly Asn
        195                 200
```

<210> 69
<211> 663
<212> DNA
<213> Citrus sinensis

<400> 69

```
atgcaacagc acctgatgca gatgcagccc atgatggcag cttattatcc caacaacgtc      60
actactgacc acattcaaca gtatctagat gagaacaaat cattgatttt gaagattgtt     120
gagagccaga attcagggaa actgagcgag tgtgcagaga accaggcaag attgcagcgg     180
aatctcatgt acctggctgc tattgctgat gctcaacccc aaccacctag cgttcatgcc     240
cagttctctt ctggtggcat tatgcagcca ggagctcact atatgcaaca ccagcaatct     300
cagccaatga caccacagtc acttatggct gcacgctcat ccatggtgta ctctcaacag     360
caattttcag tgcttcagca acagcaagcc ttgcatggtc agcttggcat gagctctggt     420
ggtagctcag gacttcacat gctgcaaagt gagggtagta ctgcaggagg tagtggttca     480
cttgggggtg gggattccc tgattttggc cgtggctcat ctggtgaagg cttgcactca     540
aggggaatgg ggagcaagca tgatataggc agttctggat ctgctgaagg acgaggaggg     600
agctcaggaa gccaagatgg aggcgaaact ctctacttga aggggctga tgatggaaat     660
taa                                                                   663
```

<210> 70
<211> 219
<212> PRT
<213> Citrus sinensis

<400> 70

```
Met Gln Gln His Leu Met Gln Met Gln Pro Met Met Ala Ala Tyr Tyr
1               5                   10                  15

Pro Asn Asn Val Thr Thr Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Ser Glu Cys Ala Glu Asn Gln Ala Arg Leu Gln Arg Asn Leu Met Tyr
    50                  55                  60

Leu Ala Ala Ile Ala Asp Ala Gln Pro Gln Pro Pro Ser Val His Ala
65                  70                  75                  80

Gln Phe Ser Ser Gly Gly Ile Met Gln Pro Gly Ala His Tyr Met Gln
                85                  90                  95

His Gln Gln Ser Gln Pro Met Thr Pro Gln Ser Leu Met Ala Ala Arg
            100                 105                 110

Ser Ser Met Val Tyr Ser Gln Gln Gln Phe Ser Val Leu Gln Gln Gln
        115                 120                 125
```

```
Gln Ala Leu His Gly Gln Leu Gly Met Ser Ser Gly Gly Ser Ser Gly
    130                 135                 140

Leu His Met Leu Gln Ser Glu Gly Ser Thr Ala Gly Gly Ser Gly Ser
145                 150                 155                 160

Leu Gly Gly Gly Gly Phe Pro Asp Phe Gly Arg Gly Ser Ser Gly Glu
                165                 170                 175

Gly Leu His Ser Arg Gly Met Gly Ser Lys His Asp Ile Gly Ser Ser
            180                 185                 190

Gly Ser Ala Glu Gly Arg Gly Gly Ser Ser Gly Ser Gln Asp Gly Gly
        195                 200                 205

Glu Thr Leu Tyr Leu Lys Gly Ala Asp Asp Gly
    210                 215


<210>  71
<211>  660
<212>  DNA
<213>  Gossypium arboreum


<220>
<221>  misc_feature
<222>  (309)..(309)
<223>  n is a, c, g, or t

<400>  71
atgcagcagc acctgatgca gatgcagccc atgatggcag cttattatcc caacaacgtc     60

actactgatc atattcaaca gtatctcgat gagaacaagt cattgatctt aaagattgtt    120

gagagccaga attctgggaa attgagtgaa tgtgctgaga accaagcaag gctgcagcga    180

aacctcatgt acctggctgc cattgcggat tctcaacccc aaccacccac cgtgcatgca    240

cagtttccat ctggtggtat catgcagcaa ggagctgggc actacatgca gcaccaacaa    300

gctcaacana tgacacaaca gtcgcttatg gctgctcggt cctcaatgtt gtattctcag    360

caaccatttt ctgcactgca acaacaacaa caacaaggct ttgcacagtc agcttggcat    420

gagctctggc gggagcacag gcctttcata tgctgcaaac tgaatctagt actgcagggg    480

gcagtgagac accttgggcc cgagggttgt cctgatttgg acgggggtct tttggagagg    540

catccctggt ggcaggccaa tggccggggg aacaaccaaa aatccgggga ggccggctca    600

cctaagggcc gggaggagcc cttggggcag gggggggtga tgggggggaac ctcttcttaa    660


<210>  72
<211>  219
<212>  PRT
<213>  Gossypium arboreum
```

<220>
<221>  UNSURE
<222>  (103)..(103)
<223>  Xaa can be any naturally occurring amino acid

<400>  72

```
Met Gln Gln His Leu Met Gln Met Gln Pro Met Met Ala Ala Tyr Tyr
1               5                   10                  15

Pro Asn Asn Val Thr Thr Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Ser Glu Cys Ala Glu Asn Gln Ala Arg Leu Gln Arg Asn Leu Met Tyr
    50                  55                  60

Leu Ala Ala Ile Ala Asp Ser Gln Pro Gln Pro Pro Thr Val His Ala
65                  70                  75                  80

Gln Phe Pro Ser Gly Gly Ile Met Gln Gln Gly Ala Gly His Tyr Met
                85                  90                  95

Gln His Gln Gln Ala Gln Xaa Met Thr Gln Gln Ser Leu Met Ala Ala
            100                 105                 110

Arg Ser Ser Met Leu Tyr Ser Gln Gln Pro Phe Ser Ala Leu Gln Gln
        115                 120                 125

Gln Gln Gln Gln Gly Phe Ala Gln Ser Ala Trp His Glu Leu Trp Arg
    130                 135                 140

Glu His Arg Pro Phe Ile Cys Cys Lys Leu Asn Leu Val Leu Gln Gly
145                 150                 155                 160

Ala Val Arg His Leu Gly Pro Glu Gly Cys Pro Asp Leu Asp Gly Gly
                165                 170                 175

Leu Leu Glu Arg His Pro Trp Trp Gln Ala Asn Gly Arg Gly Asn Asn
            180                 185                 190

Gln Lys Ser Gly Glu Ala Gly Ser Pro Lys Gly Arg Glu Glu Pro Leu
        195                 200                 205

Gly Gln Gly Gly Val Met Gly Gly Thr Ser Ser
    210                 215
```

<210>  73

<211> 636
<212> DNA
<213> Medicago truncul ata

<400> 73
atgcagcagc acctgatgca gatgcagccc atgatggcag cttactatcc taacaacgtc 60

actactgatc atattcaaca gtatcttgat gagaacaagt ccttgattct caagattgtt 120

gaaagccaga acactggcaa gctcaccgag tgtgctgaga accaatcaag gcttcagaga 180

aatctcatgt acctagctgc aatagctgat tctcaacccc aaccacctac tatgcctggc 240

cagtaccctt caagtggaat gatgcagcag ggaggacact acatgcaggc tcaacaagct 300

cagcagatga cacaacaaca attaatggct gcacgttcct ctcttatgta tgctcaacag 360

cttcaacagc agcaagcctt gcaaagccaa cttggtatga attccagtgg aagtcaaggc 420

cttcacatgt tgcatagtga aggggctaat gttggaggca attcatctct aggggctggt 480

tttcctgatt ttggccgtag ctcagccggt gatggtttgc acggcagtgg taagcaagac 540

attggaagca ctgatggccg cggtggaagc tctagtggtc actctggtga tggcggcgaa 600

acactttacc tgaaatcttc tggtgatggg aattag 636

<210> 74
<211> 211
<212> PRT
<213> Medicago truncul ata

<400> 74

Met Gln Gln His Leu Met Gln Met Gln Pro Met Met Ala Ala Tyr Tyr
1 5 10 15

Pro Asn Asn Val Thr Thr Asp His Ile Gln Gln Tyr Leu Asp Glu Asn
20 25 30

Lys Ser Leu Ile Leu Lys Ile Val Glu Ser Gln Asn Thr Gly Lys Leu
35 40 45

Thr Glu Cys Ala Glu Asn Gln Ser Arg Leu Gln Arg Asn Leu Met Tyr
50 55 60

Leu Ala Ala Ile Ala Asp Ser Gln Pro Gln Pro Pro Thr Met Pro Gly
65 70 75 80

Gln Tyr Pro Ser Ser Gly Met Met Gln Gln Gly Gly His Tyr Met Gln
85 90 95

Ala Gln Gln Ala Gln Gln Met Thr Gln Gln Gln Leu Met Ala Ala Arg
100 105 110

Ser Ser Leu Met Tyr Ala Gln Gln Leu Gln Gln Gln Gln Ala Leu Gln
115 120 125

```
Ser Gln Leu Gly Met Asn Ser Ser Gly Ser Gln Gly Leu His Met Leu
    130                 135                 140

His Ser Glu Gly Ala Asn Val Gly Gly Asn Ser Ser Leu Gly Ala Gly
145                 150                 155                 160

Phe Pro Asp Phe Gly Arg Ser Ser Ala Gly Asp Gly Leu His Gly Ser
                165                 170                 175

Gly Lys Gln Asp Ile Gly Ser Thr Asp Gly Arg Gly Gly Ser Ser Ser
            180                 185                 190

Gly His Ser Gly Asp Gly Gly Glu Thr Leu Tyr Leu Lys Ser Ser Gly
        195                 200                 205

Asp Gly Asn
    210

<210>  75
<211>  684
<212>  DNA
<213>  Oryza sativa

<400>  75
atgcagcagc aacacctgat gcagatgaac cagggcatga tgggggggata tgcttcccct      60

accaccgtca ccactgatct cattcagcag tatctggatg agaacaagca gctgatcctg     120

gccatccttg acaaccagaa caatgggaag gtggaagagt gcgctcggaa ccaagctaag     180

ctccagcaca atctcatgta cctcgccgcc atcgccgaca gccagccgcc gcagacggcc     240

gccatgtccc agtatccgtc gaacctgatg atgcagtccg ggcgaggta catgccgcag      300

cagtcggcgc agatgatggc gccgcagtcg ctgatggcgg cgaggtcttc gatgatgtac     360

gcgcagccgg cgctgtcgcc gctccagcag cagcagcagc agcaggcggc ggcggcgcac     420

gggcagctgg gcatgggctc gggggcaccc accagcgggt tcagcatcct ccacggcgag     480

gccagcatgg gcggcggcgg cggcggcggt ggcgccggta acagcatgat gaacgccggc     540

gtgttctccg acttcggacg cggcggcggc ggcggcggca aggaggggtc cacctcgctg     600

tccgtcgacg tccggggcgc caactccggc gcccagagcg gcgacgggga gtacctcaag     660

ggcaccgagg aggaaggcag ctag                                            684

<210>  76
<211>  227
<212>  PRT
<213>  Oryza sativa

<400>  76

Met Gln Gln Gln His Leu Met Gln Met Asn Gln Gly Met Met Gly Gly
```

```
1               5                   10                  15

Tyr Ala Ser Pro Thr Thr Val Thr Thr Asp Leu Ile Gln Gln Tyr Leu
            20                  25                  30

Asp Glu Asn Lys Gln Leu Ile Leu Ala Ile Leu Asp Asn Gln Asn Asn
        35                  40                  45

Gly Lys Val Glu Glu Cys Ala Arg Asn Gln Ala Lys Leu Gln His Asn
    50                  55                  60

Leu Met Tyr Leu Ala Ala Ile Ala Asp Ser Gln Pro Pro Gln Thr Ala
65                  70                  75                  80

Ala Met Ser Gln Tyr Pro Ser Asn Leu Met Met Gln Ser Gly Ala Arg
                85                  90                  95

Tyr Met Pro Gln Gln Ser Ala Gln Met Met Ala Pro Gln Ser Leu Met
            100                 105                 110

Ala Ala Arg Ser Ser Met Met Tyr Ala Gln Pro Ala Leu Ser Pro Leu
        115                 120                 125

Gln Gln Gln Gln Gln Gln Gln Ala Ala Ala Ala His Gly Gln Leu Gly
    130                 135                 140

Met Gly Ser Gly Gly Thr Thr Ser Gly Phe Ser Ile Leu His Gly Glu
145                 150                 155                 160

Ala Ser Met Gly Gly Gly Gly Gly Gly Gly Gly Ala Gly Asn Ser Met
                165                 170                 175

Met Asn Ala Gly Val Phe Ser Asp Phe Gly Arg Gly Gly Gly Gly Gly
            180                 185                 190

Gly Lys Glu Gly Ser Thr Ser Leu Ser Val Asp Val Arg Gly Ala Asn
        195                 200                 205

Ser Gly Ala Gln Ser Gly Asp Gly Glu Tyr Leu Lys Gly Thr Glu Glu
    210                 215                 220

Glu Gly Ser
225
```

<210> 77
<211> 558
<212> DNA
<213> Oryza sativa

<400> 77

```
atgcagcagc agccgatgcc gatgcccgcg caggcgccgc cgacggccgg aatcaccacc      60

gagcagatcc aaaagtatct ggatgaaaac aagcagctta ttttggctat tttggaaaat     120

cagaatctgg gaaagttggc agaatgtgct cagtatcaag cgcagcttca gaagaatctc     180

ttgtacttgg ctgcaattgc tgatactcaa ccgcagacca ctataagccg tccccagatg     240

gtgccgcatg gtgcatcgcc ggggttaggg gggcaataca tgtcgcaggt gccaatgttc     300

ccccccagga cccctctaac gccccagcag atgcaggagc agcagctgca gcaacagcaa     360

gcccagctgc tctcgttcgg cggtcagatg gttatgaggc ctggcgttgt gaatggcatt     420

cctcagcttc tgcaaggcga aatgcaccgc ggagcagatc accagaacgc tggcggggcc     480

acctcggagc cttccgagag ccacaggagc accggcaccg aaaatgacgg tggaagcgac     540

ttcggcgatc aatcctaa                                                   558


<210>  78
<211>  185
<212>  PRT
<213>  Oryza sativa

<400>  78

Met Gln Gln Gln Pro Met Pro Met Pro Ala Gln Ala Pro Pro Thr Ala
1               5                   10                  15


Gly Ile Thr Thr Glu Gln Ile Gln Lys Tyr Leu Asp Glu Asn Lys Gln
            20                  25                  30


Leu Ile Leu Ala Ile Leu Glu Asn Gln Asn Leu Gly Lys Leu Ala Glu
        35                  40                  45


Cys Ala Gln Tyr Gln Ala Gln Leu Gln Lys Asn Leu Leu Tyr Leu Ala
    50                  55                  60


Ala Ile Ala Asp Thr Gln Pro Gln Thr Thr Ile Ser Arg Pro Gln Met
65                  70                  75                  80


Val Pro His Gly Ala Ser Pro Gly Leu Gly Gly Gln Tyr Met Ser Gln
                85                  90                  95


Val Pro Met Phe Pro Pro Arg Thr Pro Leu Thr Pro Gln Gln Met Gln
            100                 105                 110


Glu Gln Gln Leu Gln Gln Gln Gln Ala Gln Leu Leu Ser Phe Gly Gly
        115                 120                 125


Gln Met Val Met Arg Pro Gly Val Val Asn Gly Ile Pro Gln Leu Leu
    130                 135                 140


Gln Gly Glu Met His Arg Gly Ala Asp His Gln Asn Ala Gly Gly Ala
```

145 150 155 160

Thr Ser Glu Pro Ser Glu Ser His Arg Ser Thr Gly Thr Glu Asn Asp
165 170 175

Gly Gly Ser Asp Phe Gly Asp Gln Ser
180 185

<210> 79
<211> 618
<212> DNA
<213> Oryza sativa

<400> 79
atgcagcagc agatggccat gccggcgggg gccgccgccg ccgcggtgcc gccggcggcc 60

ggcatcacca ccgagcagat ccaaaagtat ttggatgaaa ataaacagct aattttggcc 120

atcctggaaa atcaaaacct agggaagttg gctgaatgtg ctcagtacca agctcagctt 180

caaaagaatc tcttgtatct ggctgccatt gcagatgccc aaccacctca gaatccagga 240

agtcgccctc agatgatgca gcctggtgct accccaggtg ctgggcatta catgtcccaa 300

gtaccgatgt tccctccaag aactccctta accccacaac agatgcaaga gcagcagcag 360

cagcaactcc agcaacagca agctcaggct ctagccttcc ccggccagat gctaatgaga 420

ccaggtactg tcaatggcat gcaatctatc ccagttgctg accctgctcg cgcagccgat 480

cttcagacgg cagcaccggg ctcggtagat ggccgaggaa acaagcagga tgcaacctcg 540

gagccttccg ggaccgagag ccacaagagt gcgggagcag ataacgacgc aggcggtgac 600

atagcggaga agtcctga 618

<210> 80
<211> 205
<212> PRT
<213> Oryza sativa

<400> 80

Met Gln Gln Gln Met Ala Met Pro Ala Gly Ala Ala Ala Ala Ala Val
1 5 10 15

Pro Pro Ala Ala Gly Ile Thr Thr Glu Gln Ile Gln Lys Tyr Leu Asp
20 25 30

Glu Asn Lys Gln Leu Ile Leu Ala Ile Leu Glu Asn Gln Asn Leu Gly
35 40 45

Lys Leu Ala Glu Cys Ala Gln Tyr Gln Ala Gln Leu Gln Lys Asn Leu
50 55 60

Leu Tyr Leu Ala Ala Ile Ala Asp Ala Gln Pro Pro Gln Asn Pro Gly
65 70 75 80

Ser Arg Pro Gln Met Met Gln Pro Gly Ala Thr Pro Gly Ala Gly His
85 90 95

Tyr Met Ser Gln Val Pro Met Phe Pro Pro Arg Thr Pro Leu Thr Pro
100 105 110

Gln Gln Met Gln Glu Gln Gln Gln Gln Gln Leu Gln Gln Gln Gln Ala
115 120 125

Gln Ala Leu Ala Phe Pro Gly Gln Met Leu Met Arg Pro Gly Thr Val
130 135 140

Asn Gly Met Gln Ser Ile Pro Val Ala Asp Pro Ala Arg Ala Ala Asp
145 150 155 160

Leu Gln Thr Ala Ala Pro Gly Ser Val Asp Gly Arg Gly Asn Lys Gln
165 170 175

Asp Ala Thr Ser Glu Pro Ser Gly Thr Glu Ser His Lys Ser Ala Gly
180 185 190

Ala Asp Asn Asp Ala Gly Gly Asp Ile Ala Glu Lys Ser
195 200 205

<210> 81
<211> 540
<212> DNA
<213> Solanum tuberosum

<400> 81
atgcagcagc agcacctgat gcagatgcag cccatgatgg cagcctatta tcccaacaat 60

gtcactactg atcatattca acagttcctg gatgagaaca aatcacttat tctgaagatt 120

gttgagagcc agaactctgg gaaaataagt gaatgtgcag agtcccaagc taaacttcag 180

agaaatctta tgtaccttgc agctattgct gattcacagc cccagcctcc tagtatgcat 240

tcacagttag cttctggtgg gatgatgcag ggaggggcac attatatgca gcaacaacaa 300

gctcaacaac tcacaacgca atcgcttatg gctgcagcaa gatcctcctc ctcaatgctc 360

tatggacaac aacaacaaca acaacaacaa caactatcat cattgcaaca acagcaagca 420

gcctttcata gccagcaact cggaatgagc agctctggtg gaggaagcag tagtggactt 480

cacatgctac aaagcgaaaa cactcatagt gctagcactg gtggtgggtg gtttccctga 540

<210> 82
<211> 179
<212> PRT
<213> Solanum tuberosum

<400> 82

```
Met Gln Gln Gln His Leu Met Gln Met Gln Pro Met Met Ala Ala Tyr
1               5                   10                  15

Tyr Pro Asn Asn Val Thr Thr Asp His Ile Gln Gln Phe Leu Asp Glu
            20                  25                  30

Asn Lys Ser Leu Ile Leu Lys Ile Val Glu Ser Gln Asn Ser Gly Lys
        35                  40                  45

Ile Ser Glu Cys Ala Glu Ser Gln Ala Lys Leu Gln Arg Asn Leu Met
    50                  55                  60

Tyr Leu Ala Ala Ile Ala Asp Ser Gln Pro Gln Pro Pro Ser Met His
65                  70                  75                  80

Ser Gln Leu Ala Ser Gly Gly Met Met Gln Gly Gly Ala His Tyr Met
                85                  90                  95

Gln Gln Gln Gln Ala Gln Gln Leu Thr Thr Gln Ser Leu Met Ala Ala
            100                 105                 110

Ala Arg Ser Ser Ser Ser Met Leu Tyr Gly Gln Gln Gln Gln Gln Gln
        115                 120                 125

Gln Gln Gln Leu Ser Ser Leu Gln Gln Gln Gln Ala Ala Phe His Ser
    130                 135                 140

Gln Gln Leu Gly Met Ser Ser Ser Gly Gly Gly Ser Ser Ser Gly Leu
145                 150                 155                 160

His Met Leu Gln Ser Glu Asn Thr His Ser Ala Ser Thr Gly Gly Gly
                165                 170                 175

Trp Phe Pro
```

<210> 83
<211> 684
<212> DNA
<213> Zea mays

<400> 83

```
atgcagcagc aacacctgat gcagatgaac cagaacatga tgggggcta cacctctcct      60

gccgccgtga ccaccgatct catccagcag cacctggacg agaacaagca gctgatcctg    120

gccatcctcg acaaccagaa caatggcaag gcggaggagt gcgaacggca ccaagctaag    180

ctccagcaca acctcatgta cctggccgcc atcgctgaca gccagccgcc acagaccgcg    240

ccactatcac agtacccgtc caacctgatg atgcagccgg gccctcggta catgccaccg    300
```

```
cagtccgggc agatgatgaa cccgcagtcg ctgatggcgg cgcggtcctc catgatgtac     360

gcgcaccccgt ccctgtcgcc actccagcag cagcaggcgg cgcacggaca gctgggtatg     420

gctccagggg gcggcggtgg cggcacgacc agcgggttca gcatcctcca cggcgaggcc     480

agcatgggcg gtggtggtgc tggcgcaggc gccggcaaca acatgatgaa cgccggcatg     540

ttctcgggct ttggccgcag cggcagtggc gccaaggaag ggtcgacctc tctgtcggtt     600

gacgtccggg gtggaaccag ctccggcgcg cagagcgggg acggcgagta cctcaaagtc     660

ggcaccgagg aagaaggcag ttag                                             684
```

<210> 84
<211> 227
<212> PRT
<213> Zea mays

<400> 84

```
Met Gln Gln Gln His Leu Met Gln Met Asn Gln Asn Met Met Gly Gly
1               5                   10                  15

Tyr Thr Ser Pro Ala Ala Val Thr Thr Asp Leu Ile Gln Gln His Leu
            20                  25                  30

Asp Glu Asn Lys Gln Leu Ile Leu Ala Ile Leu Asp Asn Gln Asn Asn
        35                  40                  45

Gly Lys Ala Glu Glu Cys Glu Arg His Gln Ala Lys Leu Gln His Asn
    50                  55                  60

Leu Met Tyr Leu Ala Ala Ile Ala Asp Ser Gln Pro Pro Gln Thr Ala
65                  70                  75                  80

Pro Leu Ser Gln Tyr Pro Ser Asn Leu Met Met Gln Pro Gly Pro Arg
                85                  90                  95

Tyr Met Pro Pro Gln Ser Gly Gln Met Met Asn Pro Gln Ser Leu Met
            100                 105                 110

Ala Ala Arg Ser Ser Met Met Tyr Ala His Pro Ser Leu Ser Pro Leu
        115                 120                 125

Gln Gln Gln Gln Ala Ala His Gly Gln Leu Gly Met Ala Pro Gly Gly
    130                 135                 140

Gly Gly Gly Gly Thr Thr Ser Gly Phe Ser Ile Leu His Gly Glu Ala
145                 150                 155                 160

Ser Met Gly Gly Gly Gly Ala Gly Ala Gly Ala Gly Asn Asn Met Met
                165                 170                 175
```

Asn Ala Gly Met Phe Ser Gly Phe Gly Arg Ser Gly Ser Gly Ala Lys
180 185 190

Glu Gly Ser Thr Ser Leu Ser Val Asp Val Arg Gly Gly Thr Ser Ser
195 200 205

Gly Ala Gln Ser Gly Asp Gly Glu Tyr Leu Lys Val Gly Thr Glu Glu
210 215 220

Glu Gly Ser
225

<210> 85
<211> 549
<212> DNA
<213> Zea mays

<400> 85
atgcagcagc cgatgcacat gcagccacag gcgccggcga taaccccagc tgccggaatc 60

agcacggagc agatccaaaa gtatctggat gagaataagc agcttatttt ggctattttg 120

gaaaatcaga acctaggaaa attggcagaa tgtgctcagt atcaatcaca acttcagaag 180

aacctcttgt atctcgctgc aatcgcagat gctcaaccgc agactgctgt aagccgccct 240

cagatggcgc cgcctggtgg atcgcctgga gtagggcagt acatgtcaca ggtgcctatg 300

ttcccaccga ggacacctct tacaccccag cagatgcagg agcagcagct tcagcagcag 360

caggctcagt tgctaaactt cagtggccaa atggttgcta gaccaggcat ggtcaacggc 420

atggctcagt ccatgcaagc tcagctacca ccgggtgtga acaagcagga tgctggtggg 480

gtcgcctctg agccctcggg caccgagagc cacaggagca ctggtggtga cgatggtgga 540

agcgactag 549

<210> 86
<211> 182
<212> PRT
<213> Zea mays

<400> 86

Met Gln Gln Pro Met His Met Gln Pro Gln Ala Pro Ala Ile Thr Pro
1 5 10 15

Ala Ala Gly Ile Ser Thr Glu Gln Ile Gln Lys Tyr Leu Asp Glu Asn
20 25 30

Lys Gln Leu Ile Leu Ala Ile Leu Glu Asn Gln Asn Leu Gly Lys Leu
35 40 45

Ala Glu Cys Ala Gln Tyr Gln Ser Gln Leu Gln Lys Asn Leu Leu Tyr
50 55 60

Leu Ala Ala Ile Ala Asp Ala Gln Pro Gln Thr Ala Val Ser Arg Pro
65                  70                  75                  80

Gln Met Ala Pro Pro Gly Gly Ser Pro Gly Val Gly Gln Tyr Met Ser
                85                  90                  95

Gln Val Pro Met Phe Pro Pro Arg Thr Pro Leu Thr Pro Gln Gln Met
            100                 105                 110

Gln Glu Gln Gln Leu Gln Gln Gln Gln Ala Gln Leu Leu Asn Phe Ser
        115                 120                 125

Gly Gln Met Val Ala Arg Pro Gly Met Val Asn Gly Met Ala Gln Ser
    130                 135                 140

Met Gln Ala Gln Leu Pro Pro Gly Val Asn Lys Gln Asp Ala Gly Gly
145                 150                 155                 160

Val Ala Ser Glu Pro Ser Gly Thr Glu Ser His Arg Ser Thr Gly Gly
                165                 170                 175

Asp Asp Gly Gly Ser Asp
            180

<210>  87
<211>  1173
<212>  DNA
<213>  Homo sapiens

<400>  87
atgggcggca acatgtctgt ggctttcgcg gccccgaggc agcgaggcaa gggggagatc      60

actcccgctg cgattcagaa gatgttggat gacaataacc atcttattca gtgtataatg     120

gactctcaga ataaaggaaa gacctcagag tgttctcagt atcagcagat gttgcacaca     180

aacttggtat accttgctac aatagcagat tctaatcaaa atatgcagtc tcttttacca     240

gcaccaccca cacagaatat gcctatgggt cctggaggga tgaatcagag cggccctccc     300

ccacctccac gctctcacaa catgccttca gatggaatgg taggtggggg tcctcctgca     360

ccgcacatgc agaaccagat gaacggccag atgcctgggc ctaaccatat gcctatgcag     420

ggacctggac ccaatcaact caatatgaca aacagttcca tgaatatgcc ttcaagtagc     480

catggatcca tgggaggtta caaccattct gtgccatcat cacagagcat gccagtacag     540

aatcagatga caatgagtca gggacaacca atgggaaact atggtcccag accaaatatg     600

agtatgcagc caaaccaagg tccaatgatg catcagcagc ctccttctca gcaatacaat     660

atgccacagg gaggcggaca gcattaccaa ggacagcagc cacctatggg aatgatgggt     720

caagttaacc aaggcaatca tatgatgggt cagagacaga ttcctcccta tagacctcct     780

caacagggcc caccacagca gtactcaggc caggaagact attacgggga ccaatacagt 840

catggtggac aaggtcctcc agaaggcatg aaccagcaat attaccctga tggaaattca 900

cagtatggcc aacagcaaga tgcataccag ggaccacctc cacaacaggg atatccaccc 960

cagcagcagc agtacccagg gcagcaaggt tacccaggac agcagcaggg ctacggtcct 1020

tcacagggtg gtccaggtcc tcagtatcct aactacccac agggacaagg tcagcagtat 1080

ggaggatata gaccaacaca gcctggacca ccacagccac cccagcagag gccttatgga 1140

tatgaccagg gacagtatgg aaattaccag cag 1173

<210> 88
<211> 391
<212> PRT
<213> Homo sapiens

<400> 88

Met Gly Gly Asn Met Ser Val Ala Phe Ala Ala Pro Arg Gln Arg Gly
1 5 10 15

Lys Gly Glu Ile Thr Pro Ala Ala Ile Gln Lys Met Leu Asp Asp Asn
20 25 30

Asn His Leu Ile Gln Cys Ile Met Asp Ser Gln Asn Lys Gly Lys Thr
35 40 45

Ser Glu Cys Ser Gln Tyr Gln Gln Met Leu His Thr Asn Leu Val Tyr
50 55 60

Leu Ala Thr Ile Ala Asp Ser Asn Gln Asn Met Gln Ser Leu Leu Pro
65 70 75 80

Ala Pro Pro Thr Gln Asn Met Pro Met Gly Pro Gly Gly Met Asn Gln
85 90 95

Ser Gly Pro Pro Pro Pro Pro Arg Ser His Asn Met Pro Ser Asp Gly
100 105 110

Met Val Gly Gly Gly Pro Pro Ala Pro His Met Gln Asn Gln Met Asn
115 120 125

Gly Gln Met Pro Gly Pro Asn His Met Pro Met Gln Gly Pro Gly Pro
130 135 140

Asn Gln Leu Asn Met Thr Asn Ser Ser Met Asn Met Pro Ser Ser Ser
145 150 155 160

His Gly Ser Met Gly Gly Tyr Asn His Ser Val Pro Ser Ser Gln Ser
165 170 175

Met Pro Val Gln Asn Gln Met Thr Met Ser Gln Gly Gln Pro Met Gly
            180                 185                 190

Asn Tyr Gly Pro Arg Pro Asn Met Ser Met Gln Pro Asn Gln Gly Pro
        195                 200                 205

Met Met His Gln Gln Pro Pro Ser Gln Gln Tyr Asn Met Pro Gln Gly
    210                 215                 220

Gly Gly Gln His Tyr Gln Gly Gln Gln Pro Pro Met Gly Met Met Gly
225                 230                 235                 240

Gln Val Asn Gln Gly Asn His Met Met Gly Gln Arg Gln Ile Pro Pro
                245                 250                 255

Tyr Arg Pro Pro Gln Gln Gly Pro Pro Gln Gln Tyr Ser Gly Gln Glu
            260                 265                 270

Asp Tyr Tyr Gly Asp Gln Tyr Ser His Gly Gly Gln Gly Pro Pro Glu
        275                 280                 285

Gly Met Asn Gln Gln Tyr Tyr Pro Asp Gly Asn Ser Gln Tyr Gly Gln
    290                 295                 300

Gln Gln Asp Ala Tyr Gln Gly Pro Pro Pro Gln Gln Gly Tyr Pro Pro
305                 310                 315                 320

Gln Gln Gln Gln Tyr Pro Gly Gln Gln Gly Tyr Pro Gly Gln Gln Gln
                325                 330                 335

Gly Tyr Gly Pro Ser Gln Gly Gly Pro Gly Pro Gln Tyr Pro Asn Tyr
            340                 345                 350

Pro Gln Gly Gln Gly Gln Gln Tyr Gly Gly Tyr Arg Pro Thr Gln Pro
        355                 360                 365

Gly Pro Pro Gln Pro Pro Gln Gln Arg Pro Tyr Gly Tyr Asp Gln Gly
    370                 375                 380

Gln Tyr Gly Asn Tyr Gln Gln
385                 390

<210> 89
<211> 627
<212> DNA
<213> Allium cepa

<400> 89
atgcagcagc cgcagccagc gatgggaacc atgggctcgg tgccacctac tagcatcacc 60

```
accgaacaga ttcaaaggta cttggatgag aacaaacagt taatattggc aattttggat     120

aatcaaaatt taggaagact gaatgagtgt gctcaatatc aagctcagct tcaaaagaat     180

ctgctttacc tggcagcaat agctgatgct cagcctcagt ctcctgcggt gcgtctgcag     240

atgatgcctc aaggtgcagc tgccacgcct caagctggaa accaatttat gcagcagcag     300

agccctaatt tccctcccaa aacaggaatg caatttactc ctcaacaagt acaagaattg     360

cagcagcaac agctacaaca tcagccacat atgatgcctc catttcaagg tcaaatgggt     420

atgagaccta tgaatggaat gcaggcagca atgcatgcag attcatctct tgcttataac     480

actaacaata agcaagatgc aggaaacgca gcttatgaaa atactgctgc caacacagat     540

ggttccattc aaaagaaaac agcaaatgat gatttagacc cttctgcagc aaaccctaga     600

aggtctgaag atgccaaatc atcatga                                         627


<210>  90
<211>  208
<212>  PRT
<213>  Allium cepa

<400>  90

Met Gln Gln Pro Gln Pro Ala Met Gly Thr Met Gly Ser Val Pro Pro
1               5                   10                  15


Thr Ser Ile Thr Thr Glu Gln Ile Gln Arg Tyr Leu Asp Glu Asn Lys
            20                  25                  30


Gln Leu Ile Leu Ala Ile Leu Asp Asn Gln Asn Leu Gly Arg Leu Asn
        35                  40                  45


Glu Cys Ala Gln Tyr Gln Ala Gln Leu Gln Lys Asn Leu Leu Tyr Leu
    50                  55                  60


Ala Ala Ile Ala Asp Ala Gln Pro Gln Ser Pro Ala Val Arg Leu Gln
65                  70                  75                  80


Met Met Pro Gln Gly Ala Ala Ala Thr Pro Gln Ala Gly Asn Gln Phe
                85                  90                  95


Met Gln Gln Gln Ser Pro Asn Phe Pro Pro Lys Thr Gly Met Gln Phe
            100                 105                 110


Thr Pro Gln Gln Val Gln Glu Leu Gln Gln Gln Gln Leu Gln His Gln
        115                 120                 125


Pro His Met Met Pro Pro Phe Gln Gly Gln Met Gly Met Arg Pro Met
    130                 135                 140
```

Asn Gly Met Gln Ala Ala Met His Ala Asp Ser Ser Leu Ala Tyr Asn
145 150 155 160

Thr Asn Asn Lys Gln Asp Ala Gly Asn Ala Ala Tyr Glu Asn Thr Ala
165 170 175

Ala Asn Thr Asp Gly Ser Ile Gln Lys Lys Thr Ala Asn Asp Asp Leu
180 185 190

Asp Pro Ser Ala Ala Asn Pro Arg Arg Ser Glu Asp Ala Lys Ser Ser
195 200 205

<210> 91
<211> 633
<212> DNA
<213> Aquilegia formosa x Aquilegia pubescens

<400> 91
atgcaacaca tgcagatgca gcccatgatg ccaccttata gtgccaacag cgtcactact 60
gatcatatcc aacagtactt ggatgaaaat aaggcgttga ttctgaagat acttgagaac 120
caaaattcgg gaaaagttag tgaatgtgca gagaaccaag caagacttca acgaaatctt 180
atgtatctgg ctgcaattgc tgattctcaa ccacagcctc ccaatatgca tgctcagtac 240
tctaatgcgg gtataccacc tggtgcacat tacctacaac accaacaggc ccaacagatg 300
acacaacagt cgctcatggc tgctcgatca aatatgctgt atgctcagcc aatcacagga 360
atgcagcaac agcaagcaat gcatagccag cttggcatga gctctggtgg taacagtgga 420
ctccacatga tgcacaatga gggcagcatg ggaggtagtg gggcacttgg aagctattct 480
gattatggcc gtggcagtgg tggtggagta actatcgcta gcaaacaaga tggtggaagt 540
ggttctggtg aaggacgagg tggaaactct ggaggccaaa gtgcagatgg aggtgaatct 600
ctttacctga aaaacagtga cgaagggaac taa 633

<210> 92
<211> 210
<212> PRT
<213> Aquilegia formosa x Aquilegia pubescens

<400> 92

Met Gln His Met Gln Met Gln Pro Met Met Pro Pro Tyr Ser Ala Asn
1 5 10 15

Ser Val Thr Thr Asp His Ile Gln Gln Tyr Leu Asp Glu Asn Lys Ala
20 25 30

Leu Ile Leu Lys Ile Leu Glu Asn Gln Asn Ser Gly Lys Val Ser Glu
35 40 45

Cys Ala Glu Asn Gln Ala Arg Leu Gln Arg Asn Leu Met Tyr Leu Ala

50 55 60

Ala Ile Ala Asp Ser Gln Pro Gln Pro Pro Asn Met His Ala Gln Tyr
65 70 75 80

Ser Asn Ala Gly Ile Pro Pro Gly Ala His Tyr Leu Gln His Gln Gln
85 90 95

Ala Gln Gln Met Thr Gln Gln Ser Leu Met Ala Ala Arg Ser Asn Met
100 105 110

Leu Tyr Ala Gln Pro Ile Thr Gly Met Gln Gln Gln Gln Ala Met His
115 120 125

Ser Gln Leu Gly Met Ser Ser Gly Gly Asn Ser Gly Leu His Met Met
130 135 140

His Asn Glu Gly Ser Met Gly Gly Ser Gly Ala Leu Gly Ser Tyr Ser
145 150 155 160

Asp Tyr Gly Arg Gly Ser Gly Gly Gly Val Thr Ile Ala Ser Lys Gln
165 170 175

Asp Gly Gly Ser Gly Ser Gly Glu Gly Arg Gly Gly Asn Ser Gly Gly
180 185 190

Gln Ser Ala Asp Gly Gly Glu Ser Leu Tyr Leu Lys Asn Ser Asp Glu
195 200 205

Gly Asn
210

<210> 93
<211> 615
<212> DNA
<213> Brachypodium distachyon

<400> 93
atgcagcagg cgatgtccat gtccccgggg tcggccggcg cggtgccgcc tccggccggc 60

atcaccacag agcagatcca aaagtatttg gatgaaaata agcaacttat tttggccatc 120

ctggaaaatc agaacctagg aaagttgact gaatgtgctc agtatcaagc tcaacttcag 180

aagaatctct tgtatctggc tgccattgcg gatgcccaac caccacagaa ccctggaagt 240

cgcccccaga tggtgcagcc tggtggtatg ccaggtgcag ggcattacat gtcgcaagta 300

ccaatgttcc ctccaagaac ccctttaacc ccacaacaga tgcaagagca acagcaccag 360

cagcttcagc agcagcaagc acaggctctt gctttcccca gccagatggt catgagacca 420

ggtactgtga acggcatgca gcctatgcaa gctgatctcc aagcagcagc agcagcacct 480

ggcctggcag acagccgagg aagtaagcag gacgcagcgg tagctggggc catctcggaa 540

ccttctggca ccgagagtca caagagtaca ggagcggatc atgaggcagg tggcgatgta 600

gctgagcaat cctaa 615

<210> 94
<211> 204
<212> PRT
<213> Brachypodium distachyon

<400> 94

Met Gln Gln Ala Met Ser Met Ser Pro Gly Ser Ala Gly Ala Val Pro
1 5 10 15

Pro Pro Ala Gly Ile Thr Thr Glu Gln Ile Gln Lys Tyr Leu Asp Glu
20 25 30

Asn Lys Gln Leu Ile Leu Ala Ile Leu Glu Asn Gln Asn Leu Gly Lys
35 40 45

Leu Thr Glu Cys Ala Gln Tyr Gln Ala Gln Leu Gln Lys Asn Leu Leu
50 55 60

Tyr Leu Ala Ala Ile Ala Asp Ala Gln Pro Pro Gln Asn Pro Gly Ser
65 70 75 80

Arg Pro Gln Met Val Gln Pro Gly Gly Met Pro Gly Ala Gly His Tyr
85 90 95

Met Ser Gln Val Pro Met Phe Pro Pro Arg Thr Pro Leu Thr Pro Gln
100 105 110

Gln Met Gln Glu Gln Gln His Gln Gln Leu Gln Gln Gln Gln Ala Gln
115 120 125

Ala Leu Ala Phe Pro Ser Gln Met Val Met Arg Pro Gly Thr Val Asn
130 135 140

Gly Met Gln Pro Met Gln Ala Asp Leu Gln Ala Ala Ala Ala Ala Pro
145 150 155 160

Gly Leu Ala Asp Ser Arg Gly Ser Lys Gln Asp Ala Ala Val Ala Gly
165 170 175

Ala Ile Ser Glu Pro Ser Gly Thr Glu Ser His Lys Ser Thr Gly Ala
180 185 190

Asp His Glu Ala Gly Gly Asp Val Ala Glu Gln Ser
195 200

<210> 95
<211> 636
<212> DNA
<213> Brassica napus

<400> 95
atgcagcagc agcagcagca gcagcagcag cctccgcaaa tgtttccgat ggctccttcg 60

atgccgccaa ctaacatcac caccgaacag atccaaaagt accttgagga gaacaagaag 120

ctgataatgg caatcatgga aaatcagaat cttggcaagc ttgcagagtg tgcacagtac 180

caagctcttc tccagaagaa cttaatgtac ctcgctgcta ttgctgatgc tcaacctcct 240

ccatctaccg ctggagctac accaccacca gctatggctt cccagatggg ggcaccgcat 300

cctgggatgc aaccgccgag ctactttatg caacacccac aagcttcagg gatggctcaa 360

caagcaccac ccgctggtat cttccctccg agaggtcctt tgcagtttgg tagcccacac 420

cagcttcagg atccgcaaca gcagcatatg catcaacagg ctatgcaagg acacatgggg 480

atgcgaccaa tgggtatcaa caacaacaat gggatgcagc atcagatgca gcaacaacaa 540

ccagaaacct ctcttggagg aagcgctgca aacgtggggc ttagaggtgg aaagcaagat 600

ggagcagatg gacaaggaaa agatgatggc aaatga 636

<210> 96
<211> 203
<212> PRT
<213> Brassica napus

<400> 96

Met Gln Gln His Leu Met Gln Met Gln Pro Met Met Ala Gly Tyr Tyr
1               5                   10                  15

Pro Ser Asn Val Thr Ser Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Ser Glu Cys Ala Glu Asn Gln Ala Arg Leu Gln Arg Asn Leu Met Tyr
    50                  55                  60

Leu Ala Ala Ile Ala Asp Ser Gln Pro Gln Pro Pro Ser Val His Ser
65                  70                  75                  80

Gln Tyr Gly Ser Ala Gly Gly Gly Leu Ile Gln Gly Glu Gly Ala Ser
                85                  90                  95

His Tyr Leu Gln Gln Gln Gln Ala Thr Gln Gln Gln Gln Met Thr Gln
            100                 105                 110

```
Gln Ser Leu Met Ala Ala Arg Ser Ser Met Met Tyr Gln Gln Gln Gln
        115                 120                 125

Gln Pro Tyr Ala Thr Leu Gln His Gln Gln Leu His His Ser Gln Leu
    130                 135                 140

Gly Met Ser Ser Ser Ser Gly Gly Gly Ser Ser Gly Leu His Ile Leu
145                 150                 155                 160

Gln Gly Glu Ala Gly Gly Phe His Glu Phe Gly Arg Gly Lys Pro Glu
                165                 170                 175

Met Gly Ser Gly Glu Gly Arg Gly Gly Ser Ser Gly Asp Gly Gly Glu
            180                 185                 190

Thr Leu Tyr Leu Lys Ser Ser Asp Asp Gly Asn
        195                 200


<210>  97
<211>  636
<212>  DNA
<213>  Citrus sinensis

<400>  97
atgcagcagc caccgcaaat gatccctgtt atgccttcat ttccacccac caacatcacc      60

acagagcaga ttcaaaagta ccttgatgag aacaaaaagt tgattttggc aattttggac     120

aatcaaaatc ttggaaagct tacagaatgt gcccactatc aagctcagct tcaaaagaat     180

ttaatgtatt tagctgcaat tgctgatgca caaccacaag caccaacaat gcctcctcag     240

atggctccac atcctgcaat gcaagctagt gggtattaca tgcaacatcc tcaggcggca     300

gcaatggctc agcaacaagg aatctttccc caaaagatgc cattacaatt caataacccct    360

catcaactac aggatcctca acagcagcta caccaacatc aagccatgca agcacaaatg     420

ggaatgagac cgggtgccac taacaatggt atgcatccca tgcatgctga aagctctctt     480

ggaggtggca gcagtggagg accccccttca gcatcaggcc caggtgacat acgtggtgga    540

aataagcaag atgcctcgga ggctgggact actggtgctg atggccaggg cagttcggct     600

ggtgggcatg gtggggatgg agaggaggca aagtga                               636


<210>  98
<211>  211
<212>  PRT
<213>  Citrus sinensis

<400>  98

Met Gln Gln Pro Pro Gln Met Ile Pro Val Met Pro Ser Phe Pro Pro
1               5                   10                  15


Thr Asn Ile Thr Thr Glu Gln Ile Gln Lys Tyr Leu Asp Glu Asn Lys
```

20 25 30

Lys Leu Ile Leu Ala Ile Leu Asp Asn Gln Asn Leu Gly Lys Leu Thr
35 40 45

Glu Cys Ala His Tyr Gln Ala Gln Leu Gln Lys Asn Leu Met Tyr Leu
50 55 60

Ala Ala Ile Ala Asp Ala Gln Pro Gln Ala Pro Thr Met Pro Pro Gln
65 70 75 80

Met Ala Pro His Pro Ala Met Gln Ala Ser Gly Tyr Tyr Met Gln His
85 90 95

Pro Gln Ala Ala Ala Met Ala Gln Gln Gln Gly Ile Phe Pro Gln Lys
100 105 110

Met Pro Leu Gln Phe Asn Asn Pro His Gln Leu Gln Asp Pro Gln Gln
115 120 125

Gln Leu His Gln His Gln Ala Met Gln Ala Gln Met Gly Met Arg Pro
130 135 140

Gly Ala Thr Asn Asn Gly Met His Pro Met His Ala Glu Ser Ser Leu
145 150 155 160

Gly Gly Gly Ser Ser Gly Gly Pro Pro Ser Ala Ser Gly Pro Gly Asp
165 170 175

Ile Arg Gly Gly Asn Lys Gln Asp Ala Ser Glu Ala Gly Thr Thr Gly
180 185 190

Ala Asp Gly Gln Gly Ser Ser Ala Gly Gly His Gly Gly Asp Gly Glu
195 200 205

Glu Ala Lys
210

<210> 99
<211> 597
<212> DNA
<213> Euphorbia esula

<400> 99
atgcagcagc aaccgcagat gatgcctatg atgccttcat atccaccagc aaacattacc 60

acggagcaaa tccaaaagta tcttgatgaa aataaaaaat tgattttggc gatcttggat 120

aatcaaaatc ttggaaaact cgctgagtgt gcacagtatc aagccctgct gcaaaaaaat 180

ctgatgtatt tagccgcaat tgctgatgca caaccccaga ccccacccat gccacctcag 240

atgtccccac atccggctat gcaacaagga gcatattaca tgcaacatcc tcaggctgca 300

gcagcagcaa tggctcatca gtcgggtatt ttcccaccaa agatgtctcc gttacaattc 360

aataatcctc atcaaataca ggaccccag cagttacatc aagcagccct ccaagggcaa 420

atgggaatga ggcccatggg gcccaataac gggatgcatc cgatgcaccc cgaggcaaat 480

cttggaggat ctaatgatgg tcgtggagga aacaaacagg atgctccgga gacgggagca 540

tcgggaggtg atgggcaagg caattctggt ggtgatgggg ctgaagatgg gaaatga 597

<210> 100
<211> 198
<212> PRT
<213> Euphorbia esula

<400> 100

Met Gln Gln Gln Pro Gln Met Met Pro Met Met Pro Ser Tyr Pro Pro
1 5 10 15

Ala Asn Ile Thr Thr Glu Gln Ile Gln Lys Tyr Leu Asp Glu Asn Lys
20 25 30

Lys Leu Ile Leu Ala Ile Leu Asp Asn Gln Asn Leu Gly Lys Leu Ala
35 40 45

Glu Cys Ala Gln Tyr Gln Ala Leu Leu Gln Lys Asn Leu Met Tyr Leu
50 55 60

Ala Ala Ile Ala Asp Ala Gln Pro Gln Thr Pro Pro Met Pro Pro Gln
65 70 75 80

Met Ser Pro His Pro Ala Met Gln Gln Gly Ala Tyr Tyr Met Gln His
85 90 95

Pro Gln Ala Ala Ala Ala Ala Met Ala His Gln Ser Gly Ile Phe Pro
100 105 110

Pro Lys Met Ser Pro Leu Gln Phe Asn Asn Pro His Gln Ile Gln Asp
115 120 125

Pro Gln Gln Leu His Gln Ala Ala Leu Gln Gly Gln Met Gly Met Arg
130 135 140

Pro Met Gly Pro Asn Asn Gly Met His Pro Met His Pro Glu Ala Asn
145 150 155 160

Leu Gly Gly Ser Asn Asp Gly Arg Gly Gly Asn Lys Gln Asp Ala Pro
165 170 175

Glu Thr Gly Ala Ser Gly Gly Asp Gly Gln Gly Asn Ser Gly Gly Asp

180 185 190

Gly Ala Glu Asp Gly Lys
195

<210> 101
<211> 642
<212> DNA
<213> Glycine max

<400> 101
atgcagcaga caccgccaat gattcctatg atgccttctt tcccacctac gaacataacc 60

accgagcaga ttcaaaaata ccttgatgag aacaagaagc tgattctggc aatattggac 120

aatcaaaatc ttggaaaact tgcagaatgt gcccagtacc aagctcagct tcaaaagaat 180

ttgatgtatt tagctgcaat tgctgatgcc cagcctcaaa ccccggccat gcctccgcag 240

atggcaccgc accctgccat gcaaccagga ttctatatgc aacatcctca ggctgctgca 300

gcagcaatgg ctcagcagca gcaaggaatg ttcccccaga aaatgccatt gcaatttggc 360

aatccacatc aaatgcagga acaacaacag cagctacacc agcaggccat ccaaggtcaa 420

atgggactta gacctggaga tataaataat ggcatgcatc caatgcacag tgaggctgct 480

cttggaggtg gaaacagcgg tggtccacct tcggctactg gtccaaacga tgcacgtggt 540

ggaagcaagc aagatgcctc tgaggctgga acagctggtg gagacggcca aggcagctcc 600

gcggctgctc ataacagtgg agatggtgaa gaggcaaagt ga 642

<210> 102
<211> 213
<212> PRT
<213> Glycine max

<400> 102

Met Gln Gln Thr Pro Pro Met Ile Pro Met Met Pro Ser Phe Pro Pro
1 5 10 15

Thr Asn Ile Thr Thr Glu Gln Ile Gln Lys Tyr Leu Asp Glu Asn Lys
20 25 30

Lys Leu Ile Leu Ala Ile Leu Asp Asn Gln Asn Leu Gly Lys Leu Ala
35 40 45

Glu Cys Ala Gln Tyr Gln Ala Gln Leu Gln Lys Asn Leu Met Tyr Leu
50 55 60

Ala Ala Ile Ala Asp Ala Gln Pro Gln Thr Pro Ala Met Pro Pro Gln
65 70 75 80

Met Ala Pro His Pro Ala Met Gln Pro Gly Phe Tyr Met Gln His Pro
85 90 95

```
Gln Ala Ala Ala Ala Ala Met Ala Gln Gln Gln Gln Gly Met Phe Pro
            100                 105                 110

Gln Lys Met Pro Leu Gln Phe Gly Asn Pro His Gln Met Gln Glu Gln
        115                 120                 125

Gln Gln Gln Leu His Gln Gln Ala Ile Gln Gly Gln Met Gly Leu Arg
    130                 135                 140

Pro Gly Asp Ile Asn Asn Gly Met His Pro Met His Ser Glu Ala Ala
145                 150                 155                 160

Leu Gly Gly Gly Asn Ser Gly Gly Pro Pro Ser Ala Thr Gly Pro Asn
                165                 170                 175

Asp Ala Arg Gly Gly Ser Lys Gln Asp Ala Ser Glu Ala Gly Thr Ala
            180                 185                 190

Gly Gly Asp Gly Gln Gly Ser Ser Ala Ala Ala His Asn Ser Gly Asp
        195                 200                 205

Gly Glu Glu Ala Lys
    210


<210>  103
<211>  633
<212>  DNA
<213>  Glycine soya

<400>  103
atgcagcaga caccgcctat gattcctatg atgccttcgt tcccacctac gaacataacc      60

accgagcaga ttcaaaaata ccttgatgag aacaagaagc tgattctggc aatattggac     120

aatcaaaatc ttggaaaact tgcagaatgt gcccagtacc aagctcagct tcaaaagaat     180

ttgatgtatt tagctgcaat tgctgatgcc cagcctcaaa caccagccat gcctccacag     240

atggcaccac accctgccat gcaaccagga ttctatatgc aacatcctca ggctgcagca     300

gcagcaatgg ctcagcagca gcagcaagga atgttccccc agaaaatgcc attgcaattt     360

ggcaatccac atcaaatgca ggaacaacag cagcagctac accagcaagc catccaaggt     420

caaatgggac tgagacctgg aggaataaat aatggcatgc atccaatgca caatgagggc     480

ggcaacagcg gtggtccacc ctcggctacc ggtccgaacg acgcacgtgg tggaagcaag     540

caagatgctt ctgaggctgg aacagctggt ggagatggcc aaggcagctc tgcagctgct     600

cataacagtg gagatggtga agaggcaaag tga                                  633


<210>  104
<211>  210
```

<212> PRT
<213> Glycine soya

<400> 104

```
Met Gln Gln Thr Pro Pro Met Ile Pro Met Met Pro Ser Phe Pro Pro
1               5                   10                  15

Thr Asn Ile Thr Thr Glu Gln Ile Gln Lys Tyr Leu Asp Glu Asn Lys
            20                  25                  30

Lys Leu Ile Leu Ala Ile Leu Asp Asn Gln Asn Leu Gly Lys Leu Ala
        35                  40                  45

Glu Cys Ala Gln Tyr Gln Ala Gln Leu Gln Lys Asn Leu Met Tyr Leu
    50                  55                  60

Ala Ala Ile Ala Asp Ala Gln Pro Gln Thr Pro Ala Met Pro Pro Gln
65                  70                  75                  80

Met Ala Pro His Pro Ala Met Gln Pro Gly Phe Tyr Met Gln His Pro
                85                  90                  95

Gln Ala Ala Ala Ala Ala Met Ala Gln Gln Gln Gln Gln Gly Met Phe
            100                 105                 110

Pro Gln Lys Met Pro Leu Gln Phe Gly Asn Pro His Gln Met Gln Glu
        115                 120                 125

Gln Gln Gln Gln Leu His Gln Gln Ala Ile Gln Gly Gln Met Gly Leu
    130                 135                 140

Arg Pro Gly Gly Ile Asn Asn Gly Met His Pro Met His Asn Glu Gly
145                 150                 155                 160

Gly Asn Ser Gly Gly Pro Pro Ser Ala Thr Gly Pro Asn Asp Ala Arg
                165                 170                 175

Gly Gly Ser Lys Gln Asp Ala Ser Glu Ala Gly Thr Ala Gly Gly Asp
            180                 185                 190

Gly Gln Gly Ser Ser Ala Ala Ala His Asn Ser Gly Asp Gly Glu Glu
        195                 200                 205

Ala Lys
    210
```

<210> 105
<211> 690
<212> DNA
<213> Gossypium hirsutum

<400> 105
atgcagcagc acctgatgca gatgcagccc atgatggcag cttattatcc caacaacgtc 60

actactgatc atattcaaca gtatctcgat gagaacaagt cattgatctt aaagattgtt 120

gagagccaga attctgggaa attgagtgaa tgtgctgaga accaagcaag gctgcagcga 180

aacctcatgt acctggctgc cattgcggat tctcaacccc aaccacccac cgtgcatgca 240

cagtttccat ctggtggtat catgcagcca ggagctgggc actacatgca gcaccaacaa 300

gctcaacaaa tgacacaaca gtcgcttatg gctgctcggt cctcaatgtt gtattctcag 360

caaccatttt ctgcactgca acaacaacag cagcaagctt tgcacagtca gcttggcatg 420

agctctggcg gaagcacagg ccttcatatg ctgcaaactg aatctagtac tgcaggtggc 480

agtggagcac ttggggccgg agggtttcct gattttggac gtggttcttc tggagaaggc 540

atccatggtg gcaggccaat ggcaggtgga agcaagcaag atatcgggag tgccggctca 600

gctgaaggtc gtggaggaag ctctggtggt cagggtggtg gtgatggggg tgaaaccctt 660

tacttaaaag cagccgatga tgggaactga 690

<210> 106
<211> 229
<212> PRT
<213> Gossypium hirsutum

<400> 106

Met Gln Gln His Leu Met Gln Met Gln Pro Met Met Ala Ala Tyr Tyr
1 5 10 15

Pro Asn Asn Val Thr Thr Asp His Ile Gln Gln Tyr Leu Asp Glu Asn
20 25 30

Lys Ser Leu Ile Leu Lys Ile Val Glu Ser Gln Asn Ser Gly Lys Leu
35 40 45

Ser Glu Cys Ala Glu Asn Gln Ala Arg Leu Gln Arg Asn Leu Met Tyr
50 55 60

Leu Ala Ala Ile Ala Asp Ser Gln Pro Gln Pro Pro Thr Val His Ala
65 70 75 80

Gln Phe Pro Ser Gly Gly Ile Met Gln Pro Gly Ala Gly His Tyr Met
85 90 95

Gln His Gln Gln Ala Gln Gln Met Thr Gln Gln Ser Leu Met Ala Ala
100 105 110

Arg Ser Ser Met Leu Tyr Ser Gln Gln Pro Phe Ser Ala Leu Gln Gln
115 120 125

```
Gln Gln Gln Gln Ala Leu His Ser Gln Leu Gly Met Ser Ser Gly Gly
    130                 135                 140

Ser Thr Gly Leu His Met Leu Gln Thr Glu Ser Ser Thr Ala Gly Gly
145                 150                 155                 160

Ser Gly Ala Leu Gly Ala Gly Gly Phe Pro Asp Phe Gly Arg Gly Ser
                165                 170                 175

Ser Gly Glu Gly Ile His Gly Gly Arg Pro Met Ala Gly Gly Ser Lys
            180                 185                 190

Gln Asp Ile Gly Ser Ala Gly Ser Ala Glu Gly Arg Gly Gly Ser Ser
        195                 200                 205

Gly Gly Gln Gly Gly Gly Asp Gly Gly Glu Thr Leu Tyr Leu Lys Ala
    210                 215                 220

Ala Asp Asp Gly Asn
225


<210>  107
<211>  642
<212>  DNA
<213>  Gossypium hirsutum

<400>  107
atgccgcagc caccgcaaat gattcctgtg atgccttcat atccacctac taatatcact      60

actgaacaga ttcagaagta ccttgatgag aataagaagt tgattttggc aattttggac     120

aatcagaatc ttggaaaact cgctgaatgc gcccagtatc aagctcagct gcaaaagaat     180

ttgatgtatt tagctgcaat tgcggatgct caacctcaat caacgccagc aatgtcgcct     240

cagatggcac cgcatccagc aatgcaaccc ggaggatatt ttatgcaaca tcctcaagct     300

gctgcaatgt cacagcaacc tggcatgtac cctcaaaagg tgccattgca attcaatagt     360

ccgcatcaaa tgcaggaccc tcagcacctc ctatatcagc agcatcaaca agcaatgcaa     420

ggtcaaatgg gaatcaggcc tgggggaccc aataatagca tgcatcccat gcattcagag     480

gctagccttg gaggcggcag cagtggtggt ccccctcaac cttcaggccc aagtgatgga     540

cgtgctggaa acaagcaaga gggctccgaa gctggtggta atgggcaggg cagcacaact     600

ggtgggcatg gtggcggtga tggagcggat gaggcaaagt ga                        642


<210>  108
<211>  213
<212>  PRT
<213>  Gossypium hirsutum

<400>  108
```

```
Met Pro Gln Pro Pro Gln Met Ile Pro Val Met Pro Ser Tyr Pro Pro
1               5                   10                  15

Thr Asn Ile Thr Thr Glu Gln Ile Gln Lys Tyr Leu Asp Glu Asn Lys
            20                  25                  30

Lys Leu Ile Leu Ala Ile Leu Asp Asn Gln Asn Leu Gly Lys Leu Ala
        35                  40                  45

Glu Cys Ala Gln Tyr Gln Ala Gln Leu Gln Lys Asn Leu Met Tyr Leu
    50                  55                  60

Ala Ala Ile Ala Asp Ala Gln Pro Gln Ser Thr Pro Ala Met Ser Pro
65                  70                  75                  80

Gln Met Ala Pro His Pro Ala Met Gln Pro Gly Gly Tyr Phe Met Gln
                85                  90                  95

His Pro Gln Ala Ala Ala Met Ser Gln Gln Pro Gly Met Tyr Pro Gln
            100                 105                 110

Lys Val Pro Leu Gln Phe Asn Ser Pro His Gln Met Gln Asp Pro Gln
        115                 120                 125

His Leu Leu Tyr Gln Gln His Gln Gln Ala Met Gln Gly Gln Met Gly
    130                 135                 140

Ile Arg Pro Gly Gly Pro Asn Asn Ser Met His Pro Met His Ser Glu
145                 150                 155                 160

Ala Ser Leu Gly Gly Gly Ser Ser Gly Gly Pro Pro Gln Pro Ser Gly
                165                 170                 175

Pro Ser Asp Gly Arg Ala Gly Asn Lys Gln Glu Gly Ser Glu Ala Gly
            180                 185                 190

Gly Asn Gly Gln Gly Ser Thr Thr Gly Gly His Gly Gly Gly Asp Gly
        195                 200                 205

Ala Asp Glu Ala Lys
    210
```

<210> 109
<211> 561
<212> DNA
<213> Hordeum vulgare

<400> 109

```
atgcagcaag cgatgcccat gccgccggcg gcggcggcgc ctgggatgcc tccttctgcc      60

ggcctcagca ccgagcagat ccaaaagtac ctggatgaaa ataaacaact aattttggct     120
```

```
atcttggaaa atcagaacct gggaaagttg gcggaatgtg ctcagtatca agctcagctt     180

cagaagaatc ttttgtattt ggctgcgatt gctgatactc agccacagac ctctgtaagc     240

cgtcctcaga tggcaccacc tgctgcatcc ccaggggcag ggcattacat gtcacaggtg     300

ccaatgttcc ctccgaggac ccctctaacg cctcagcaga tgcaggagca gcaactacag     360

caacaacagg ctcagatgct tccgtttgct ggtcaaatgg ttgcgagacc cggggctgtc     420

aatggcattc cccaggcccc tcaagttgaa caaccagcct atgcagcagg tggggccagt     480

tccgagcctt ctggcaccga gagccacagg agcactggcg ccgataacga tggtgggagc     540

ggcttggctg accagtccta a                                               561


<210>  110
<211>  186
<212>  PRT
<213>  Hordeum vulgare

<400>  110

Met Gln Gln Ala Met Pro Met Pro Pro Ala Ala Ala Ala Pro Gly Met
1               5                   10                  15


Pro Pro Ser Ala Gly Leu Ser Thr Glu Gln Ile Gln Lys Tyr Leu Asp
            20                  25                  30


Glu Asn Lys Gln Leu Ile Leu Ala Ile Leu Glu Asn Gln Asn Leu Gly
        35                  40                  45


Lys Leu Ala Glu Cys Ala Gln Tyr Gln Ala Gln Leu Gln Lys Asn Leu
    50                  55                  60


Leu Tyr Leu Ala Ala Ile Ala Asp Thr Gln Pro Gln Thr Ser Val Ser
65                  70                  75                  80


Arg Pro Gln Met Ala Pro Pro Ala Ala Ser Pro Gly Ala Gly His Tyr
                85                  90                  95


Met Ser Gln Val Pro Met Phe Pro Pro Arg Thr Pro Leu Thr Pro Gln
            100                 105                 110


Gln Met Gln Glu Gln Gln Leu Gln Gln Gln Gln Ala Gln Met Leu Pro
        115                 120                 125


Phe Ala Gly Gln Met Val Ala Arg Pro Gly Ala Val Asn Gly Ile Pro
    130                 135                 140


Gln Ala Pro Gln Val Glu Gln Pro Ala Tyr Ala Ala Gly Gly Ala Ser
145                 150                 155                 160
```

Ser Glu Pro Ser Gly Thr Glu Ser His Arg Ser Thr Gly Ala Asp Asn
165 170 175

Asp Gly Gly Ser Gly Leu Ala Asp Gln Ser
180 185

<210> 111
<211> 555
<212> DNA
<213> Lactuca serriola

<220>
<221> misc_feature
<222> (253)..(253)
<223> n is a, c, g, or t

<400> 111
atgaagcagc cgatgatgcc gaatccaatg atgtcttctt cgtttcctcc tacaaacatc 60

accaccgatc agatccaaaa gttccttgat gaaaacaagc aactaattat agcaataatg 120

agcaacctaa atcttggaaa gcttgctgaa tgtgcccagt accaagctct actccaaaaa 180

aatttgatgt atctagcagc cattgcagat gctcaaccac ctacacctac accaacacta 240

aatatctctt atnagatggg cccggttcca catccaggga tgccacagca aggtggattt 300

tacatggcgc agcagcaccc tcaggcggct gtaatgacgg ctcagccacc ttctggtttt 360

ccacaaccga tgcctggtat gcaatttaac agcccacagg ctattcaagg gcagatgggc 420

gggaggtccg gtgggccgcc aagctcagcc gctagtgatg tctggagagg aagcatgcaa 480

gatggtggtg gtggtgctgc tgctgatggt ggtaaggatg gtcatgctgg cggtggacct 540

gaggaagcaa agtaa 555

<210> 112
<211> 184
<212> PRT
<213> Lactuca serriola

<220>
<221> UNSURE
<222> (85)..(85)
<223> Xaa can be any naturally occurring amino acid

<400> 112

Met Lys Gln Pro Met Met Pro Asn Pro Met Met Ser Ser Ser Phe Pro
1 5 10 15

Pro Thr Asn Ile Thr Thr Asp Gln Ile Gln Lys Phe Leu Asp Glu Asn
20 25 30

Lys Gln Leu Ile Ile Ala Ile Met Ser Asn Leu Asn Leu Gly Lys Leu
35 40 45

Ala Glu Cys Ala Gln Tyr Gln Ala Leu Leu Gln Lys Asn Leu Met Tyr
50 55 60

Leu Ala Ala Ile Ala Asp Ala Gln Pro Pro Thr Pro Thr Pro Thr Leu
65 70 75 80

Asn Ile Ser Tyr Xaa Met Gly Pro Val Pro His Pro Gly Met Pro Gln
85 90 95

Gln Gly Gly Phe Tyr Met Ala Gln Gln His Pro Gln Ala Ala Val Met
100 105 110

Thr Ala Gln Pro Pro Ser Gly Phe Pro Gln Pro Met Pro Gly Met Gln
115 120 125

Phe Asn Ser Pro Gln Ala Ile Gln Gly Gln Met Gly Gly Arg Ser Gly
130 135 140

Gly Pro Pro Ser Ser Ala Ala Ser Asp Val Trp Arg Gly Ser Met Gln
145 150 155 160

Asp Gly Gly Gly Gly Ala Ala Ala Asp Gly Gly Lys Asp Gly His Ala
165 170 175

Gly Gly Gly Pro Glu Glu Ala Lys
180

<210> 113
<211> 627
<212> DNA
<213> Lycopersicon esculentum

<400> 113
atgcagcagc acctgatgca gatgcagccc atgatggcag cttactatcc aacgaacgtc 60

actactgacc atattcaaca gtatttggat gaaaacaaat cactcattct gaagattgtt 120

gagagccaga actctgggaa actcagtgaa tgtgcggaga accaagctag gcttcagagg 180

aatctgatgt accttgctgc gattgctgat tcacaacctc aaccttctag catgcattct 240

cagttctctt ctggtgggat gatgcagcca gggacacaca gttacttgca gcagcagcag 300

cagcaacaac aagcgcaaca aatggcaaca caacaactca tggctgcaag atcctcgtcg 360

atgctctatg gacaacagca gcagcaatct cagttatcgc aatatcaaca aggcttgcat 420

agtagccaac tcggcatgag ttctggcagt ggcggaagca ctggacttca tcacatgctt 480

caaagtgaat catcacctca tggtggtggt ttctctcatg acttcggccg cgcaaataag 540

caagacattg ggagtagtat gtctgctgaa gggcgcggcg gaagttcagg tggtgagaat 600

ctttatctga aagcttctga ggattga 627

<210> 114
<211> 208
<212> PRT
<213> Lycopersicon esculentum

<400> 114

Met Gln Gln His Leu Met Gln Met Gln Pro Met Met Ala Ala Tyr Tyr
1               5                   10                  15

Pro Thr Asn Val Thr Thr Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Ser Glu Cys Ala Glu Asn Gln Ala Arg Leu Gln Arg Asn Leu Met Tyr
    50                  55                  60

Leu Ala Ala Ile Ala Asp Ser Gln Pro Gln Pro Ser Ser Met His Ser
65                  70                  75                  80

Gln Phe Ser Ser Gly Gly Met Met Gln Pro Gly Thr His Ser Tyr Leu
                85                  90                  95

Gln Gln Gln Gln Gln Gln Gln Gln Ala Gln Gln Met Ala Thr Gln Gln
            100                 105                 110

Leu Met Ala Ala Arg Ser Ser Ser Met Leu Tyr Gly Gln Gln Gln Gln
        115                 120                 125

Gln Ser Gln Leu Ser Gln Tyr Gln Gln Gly Leu His Ser Ser Gln Leu
    130                 135                 140

Gly Met Ser Ser Gly Ser Gly Gly Ser Thr Gly Leu His His Met Leu
145                 150                 155                 160

Gln Ser Glu Ser Ser Pro His Gly Gly Gly Phe Ser His Asp Phe Gly
                165                 170                 175

Arg Ala Asn Lys Gln Asp Ile Gly Ser Ser Met Ser Ala Glu Gly Arg
            180                 185                 190

Gly Gly Ser Ser Gly Gly Glu Asn Leu Tyr Leu Lys Ala Ser Glu Asp
        195                 200                 205

<210> 115
<211> 624
<212> DNA
<213> Malus domestica

<400> 115
atgcagcagc caccacaaat gatccccgtc atgccttcat ttcctcccac caacatcacc 60

accgaacaaa ttcagaagta ccttgatgac aacaaaaagt tgattctggc aatattggat 120

aatcaaaatc ttggaaaact tgctgagtgt gctcagtacc aggctctgct tcaaaagaat 180

ctgatgtatt tagcagcaat tgccgatgcg caaccacagg caccagctgc ccctccccag 240

atggccccac atcctgctat gcaacaggca ggatattaca tgcaacatcc tcaggcagca 300

gcaatggctc agcaacaggg tattttctcc ccaaagatgc cgatgcaatt caataacatg 360

catcaaatgc acgatccaca gcagcaccaa caagccatgc aagggcaaat gggaatgaga 420

cctggagggc ctaacggcat gccttccatg cttcatactg aggccacaca tggtggtggt 480

agtggcggcc caaattcagc tggagaccca aatgatgggc gtggaggaag caagcaagac 540

gcctctgagt ctggggcagg tggtgatggc caggggacct cagccggcgg gcgtggaact 600

ggtgatggag aggacggcaa gtga 624

<210> 116
<211> 207
<212> PRT
<213> Malus domestica

<400> 116

Met Gln Gln Pro Pro Gln Met Ile Pro Val Met Pro Ser Phe Pro Pro
1               5                   10                  15

Thr Asn Ile Thr Thr Glu Gln Ile Gln Lys Tyr Leu Asp Asp Asn Lys
            20                  25                  30

Lys Leu Ile Leu Ala Ile Leu Asp Asn Gln Asn Leu Gly Lys Leu Ala
        35                  40                  45

Glu Cys Ala Gln Tyr Gln Ala Leu Leu Gln Lys Asn Leu Met Tyr Leu
    50                  55                  60

Ala Ala Ile Ala Asp Ala Gln Pro Gln Ala Pro Ala Ala Pro Pro Gln
65                  70                  75                  80

Met Ala Pro His Pro Ala Met Gln Gln Ala Gly Tyr Tyr Met Gln His
                85                  90                  95

Pro Gln Ala Ala Ala Met Ala Gln Gln Gln Gly Ile Phe Ser Pro Lys
            100                 105                 110

Met Pro Met Gln Phe Asn Asn Met His Gln Met His Asp Pro Gln Gln
        115                 120                 125

His Gln Gln Ala Met Gln Gly Gln Met Gly Met Arg Pro Gly Gly Pro
    130                 135                 140

```
Asn Gly Met Pro Ser Met Leu His Thr Glu Ala Thr His Gly Gly Gly
145                 150                 155                 160

Ser Gly Gly Pro Asn Ser Ala Gly Asp Pro Asn Asp Gly Arg Gly Gly
                165                 170                 175

Ser Lys Gln Asp Ala Ser Glu Ser Gly Ala Gly Gly Asp Gly Gln Gly
            180                 185                 190

Thr Ser Ala Gly Gly Arg Gly Thr Gly Asp Gly Glu Asp Gly Lys
        195                 200                 205
```

<210> 117
<211> 639
<212> DNA
<213> Medicago trunculata

<400> 117

```
atgcagcaga cacctcaaat gattcctatg atgccttcat tcccacaaca aacaaacata      60

accactgagc agattcaaaa atatcttgat gagaacaaga agctgatcct ggcaatattg     120

gacaatcaaa atcttggaaa acttgcagaa tgtgcccagt accaagctca gcttcagaag     180

aatttgatgt atttagctgc aattgctgac gcgcagccac aaacaccggc cttgcctcca     240

cagatggccc cgcaccctgc gatgcaacaa ggattctata tgcaacatcc tcaggctgca     300

gcaatggctc agcaacaagg aatgttcccc caaaaaatgc caatgcagtt cggtaatccg     360

catcaaatgc aggatcagca gcatcagcag caacaacagc agctacatca gcaagctatg     420

caaggtcaaa tgggacttag acctggaggg ataaataacg gcatgcatcc aatgcacaac     480

gaggctgctc tcggaggtag cggcagtggt ggtcaaatga cgggcgtggt ggtggagcaa     540

gcaagatgct tcggagctgg gacagccggc ggtgatggtc aaggaacctc tgccgcagct     600

gcgcacaaca gtggagatgc ttcagaagaa ggaaagtaa                            639
```

<210> 118
<211> 213
<212> PRT
<213> Medicago trunculata

<400> 118

```
Met Gln Gln Thr Pro Gln Met Ile Pro Met Met Pro Ser Phe Pro Gln
1               5                   10                  15

Gln Thr Asn Ile Thr Thr Glu Gln Ile Gln Lys Tyr Leu Asp Glu Asn
            20                  25                  30

Lys Lys Leu Ile Leu Ala Ile Leu Asp Asn Gln Asn Leu Gly Lys Leu
        35                  40                  45
```

```
Ala Glu Cys Ala Gln Tyr Gln Ala Gln Leu Gln Lys Asn Leu Met Tyr
    50                  55                  60

Leu Ala Ala Ile Ala Asp Ala Gln Pro Gln Thr Pro Ala Leu Pro Pro
65                  70                  75                  80

Gln Met Ala Pro His Pro Ala Met Gln Gln Gly Phe Tyr Met Gln His
                85                  90                  95

Pro Gln Ala Ala Ala Met Ala Gln Gln Gln Gly Met Phe Pro Gln Lys
            100                 105                 110

Met Pro Met Gln Phe Gly Asn Pro His Gln Met Gln Asp Gln Gln His
        115                 120                 125

Gln Gln Gln Gln Gln Gln Leu His Gln Gln Ala Met Gln Gly Gln Met
    130                 135                 140

Gly Leu Arg Pro Gly Gly Ile Asn Asn Gly Met His Pro Met His Asn
145                 150                 155                 160

Glu Ala Ala Leu Gly Gly Ser Gly Ser Gly Gly Pro Asn Asp Gly Arg
                165                 170                 175

Gly Gly Gly Ser Lys Gln Asp Ala Ser Glu Ala Gly Thr Ala Gly Gly
            180                 185                 190

Asp Gly Gln Gly Thr Ser Ala Ala Ala Ala His Asn Ser Gly Asp Ala
        195                 200                 205

Ser Glu Glu Gly Lys
    210


<210>  119
<211>  624
<212>  DNA
<213>  Panicum virgatum

<400>  119
atgcagcagc agatgcccat gcagtcggcg cccccggcga ccggcatcac caccgagcag     60

atccaaaagt atttggatga aaataagcag cttattttgg ccatcctgga aaatcagaac    120

ttaggaaagt tggctgaatg tgctcagtat caagctcagc ttcaaaagaa tctcttgtac    180

ctggctgcga ttgcagatgc ccaaccccaa ccaccacaga accctgcaag tcgcccacag    240

atgatgcaac ctggcatggt accaggtgca gggcattaca tgtcccaagt accaatgttc    300

ccgccaagaa caccattaac cccgcaacag atgcaagaac agcagcagca gcagcagcag    360

cttcaacagc agcaagcaca ggctcttgct ttcccgggac agatggtcat gagacctacc    420
```

```
attaatggca tgcagcctat gcaagccgac cctgctgccg ccgccgccag cctacagcag    480

tcagcacctg gccctactga tgggcgagga ggcaagcaag atgcaactgc tggggtgagc    540

acagagcctt ctggcaccga gagccacaag agcacaaccg cagcagatca cgatgtgggc    600

actgatgtcg cggagaaatc ctaa                                           624


<210>  120
<211>  207
<212>  PRT
<213>  Panicum virgatum

<400>  120

Met Gln Gln Gln Met Pro Met Gln Ser Ala Pro Pro Ala Thr Gly Ile
1               5                   10                  15


Thr Thr Glu Gln Ile Gln Lys Tyr Leu Asp Glu Asn Lys Gln Leu Ile
            20                  25                  30


Leu Ala Ile Leu Glu Asn Gln Asn Leu Gly Lys Leu Ala Glu Cys Ala
        35                  40                  45


Gln Tyr Gln Ala Gln Leu Gln Lys Asn Leu Leu Tyr Leu Ala Ala Ile
    50                  55                  60


Ala Asp Ala Gln Pro Gln Pro Pro Gln Asn Pro Ala Ser Arg Pro Gln
65                  70                  75                  80


Met Met Gln Pro Gly Met Val Pro Gly Ala Gly His Tyr Met Ser Gln
                85                  90                  95


Val Pro Met Phe Pro Pro Arg Thr Pro Leu Thr Pro Gln Gln Met Gln
            100                 105                 110


Glu Gln Gln Gln Gln Gln Gln Gln Leu Gln Gln Gln Gln Ala Gln Ala
        115                 120                 125


Leu Ala Phe Pro Gly Gln Met Val Met Arg Pro Thr Ile Asn Gly Met
    130                 135                 140


Gln Pro Met Gln Ala Asp Pro Ala Ala Ala Ala Ala Ser Leu Gln Gln
145                 150                 155                 160


Ser Ala Pro Gly Pro Thr Asp Gly Arg Gly Gly Lys Gln Asp Ala Thr
                165                 170                 175


Ala Gly Val Ser Thr Glu Pro Ser Gly Thr Glu Ser His Lys Ser Thr
            180                 185                 190


Thr Ala Ala Asp His Asp Val Gly Thr Asp Val Ala Glu Lys Ser
```

195 200 205

<210> 121
<211> 747
<212> DNA
<213> Picea sitchensis

<400> 121
atgcagcagc atctcatgca aatgcagccc atgatggcgg catacgcctc caacaacatc 60

accactgatc acatccagaa gtacctggat gagaacaagc agttgattct ggcaattctg 120

gacaaccaaa atcttggaaa gctcaatgag tgtgctcagt accaagcaaa acttcagcag 180

aatttgatgt atctggctgc gattgctgat tctcaaccac aagcacaaac tgcacatgct 240

cagattcctc ctaatgcagt gatgcagtct ggtgggcatt acatgcagca ccagcaggca 300

cagcaacaag tgactcctca gtctctgatg gcagctagat cttccatgct gtattctcag 360

cagccgatgg ctgctttgca tcaagctcag caacaacagc agcagcagca tcagcagcaa 420

caacaatctc ttcacagcca gcttggcata aattctggag gaagcagtgg attgcatatg 480

ttgcatggtg agacaaacat gggatgtaat gggcctctct catctggggg cttccctgaa 540

tttgggcgtg ggtctgctac ctctgctgaa ggtatgcagg ccaacagggg cttcactata 600

gatcgtggtt caaataagca ggatggagta ggatcagaga atgcccatcc aggtgctggt 660

gatggaagag ggagttcaac tggagggcag aatgcagatg agtcagaacc atcatacctg 720

aaagcctccg aagaagaagg aaactag 747

<210> 122
<211> 248
<212> PRT
<213> Picea sitchensis

<400> 122

Met Gln Gln His Leu Met Gln Met Gln Pro Met Met Ala Ala Tyr Ala
1 5 10 15

Ser Asn Asn Ile Thr Thr Asp His Ile Gln Lys Tyr Leu Asp Glu Asn
20 25 30

Lys Gln Leu Ile Leu Ala Ile Leu Asp Asn Gln Asn Leu Gly Lys Leu
35 40 45

Asn Glu Cys Ala Gln Tyr Gln Ala Lys Leu Gln Gln Asn Leu Met Tyr
50 55 60

Leu Ala Ala Ile Ala Asp Ser Gln Pro Gln Ala Gln Thr Ala His Ala
65 70 75 80

Gln Ile Pro Pro Asn Ala Val Met Gln Ser Gly Gly His Tyr Met Gln
85 90 95

His Gln Gln Ala Gln Gln Gln Val Thr Pro Gln Ser Leu Met Ala Ala
100 105 110

Arg Ser Ser Met Leu Tyr Ser Gln Gln Pro Met Ala Ala Leu His Gln
115 120 125

Ala Gln Gln Gln Gln Gln Gln Gln His Gln Gln Gln Gln Gln Ser Leu
130 135 140

His Ser Gln Leu Gly Ile Asn Ser Gly Gly Ser Ser Gly Leu His Met
145 150 155 160

Leu His Gly Glu Thr Asn Met Gly Cys Asn Gly Pro Leu Ser Ser Gly
165 170 175

Gly Phe Pro Glu Phe Gly Arg Gly Ser Ala Thr Ser Ala Glu Gly Met
180 185 190

Gln Ala Asn Arg Gly Phe Thr Ile Asp Arg Gly Ser Asn Lys Gln Asp
195 200 205

Gly Val Gly Ser Glu Asn Ala His Pro Gly Ala Gly Asp Gly Arg Gly
210 215 220

Ser Ser Thr Gly Gly Gln Asn Ala Asp Glu Ser Glu Pro Ser Tyr Leu
225 230 235 240

Lys Ala Ser Glu Glu Glu Gly Asn
245

<210> 123
<211> 735
<212> DNA
<213> Pinus taeda

<400> 123
atgcagcagc acctcatgca aatgcagccc atgatggcgg cctacgcctc caacaatatc 60

accactgatc acatccagaa gtacctggat gagaacaagc agttgattct ggcaattttg 120

gacaaccaaa atctcggaaa gctcaatgag tgtgctcaat accaagcaaa acttcagcag 180

aatttgatgt atctggctgc tattgctgat tctcaacctc aagcacaaac tgcacatgct 240

cagattcctc caaatgcggt gatgcagtct ggtgggcatt acatgcagca tcaacaggca 300

cagcaacaag ttactcctca gtctctgatg gcagctagat cttccatact gtatgctcag 360

caacaacagc agcagcagca tcagcagcat cagcagcaac agcagcaaca acagtctctt 420

cacagccagc ttggcataaa ttctggagga agcagcggtt tgcatatgtt gcatggtgag 480

acaaacatgg gatgtaatgg gcctctgtca tctgggggat tccctgaatt tgggcgtggg 540

tctgctacct ctgctgatgg tatgcaggtg aacaggggct ttgctataga tcgtggttca 600

aacaagcagg atggagttgg atcagagaat gcccatgctg gtgctggtga tggaagaggg 660

agttcaactg gagggcagaa tgcagatgag tcagaaccat catacctgaa ggcctccgag 720

gaagaaggaa actag 735

<210> 124
<211> 244
<212> PRT
<213> Pinus taeda

<400> 124

```
Met Gln Gln His Leu Met Gln Met Gln Pro Met Met Ala Ala Tyr Ala
1               5                   10                  15

Ser Asn Asn Ile Thr Thr Asp His Ile Gln Lys Tyr Leu Asp Glu Asn
            20                  25                  30

Lys Gln Leu Ile Leu Ala Ile Leu Asp Asn Gln Asn Leu Gly Lys Leu
        35                  40                  45

Asn Glu Cys Ala Gln Tyr Gln Ala Lys Leu Gln Gln Asn Leu Met Tyr
    50                  55                  60

Leu Ala Ala Ile Ala Asp Ser Gln Pro Gln Ala Gln Thr Ala His Ala
65                  70                  75                  80

Gln Ile Pro Pro Asn Ala Val Met Gln Ser Gly Gly His Tyr Met Gln
                85                  90                  95

His Gln Gln Ala Gln Gln Gln Val Thr Pro Gln Ser Leu Met Ala Ala
            100                 105                 110

Arg Ser Ser Ile Leu Tyr Ala Gln Gln Gln Gln Gln Gln Gln His Gln
        115                 120                 125

Gln His Gln Gln Gln Gln Gln Gln Gln Gln Ser Leu His Ser Gln Leu
    130                 135                 140

Gly Ile Asn Ser Gly Gly Ser Ser Gly Leu His Met Leu His Gly Glu
145                 150                 155                 160

Thr Asn Met Gly Cys Asn Gly Pro Leu Ser Ser Gly Gly Phe Pro Glu
                165                 170                 175

Phe Gly Arg Gly Ser Ala Thr Ser Ala Asp Gly Met Gln Val Asn Arg
            180                 185                 190
```

```
Gly Phe Ala Ile Asp Arg Gly Ser Asn Lys Gln Asp Gly Val Gly Ser
        195                 200                 205

Glu Asn Ala His Ala Gly Ala Gly Asp Gly Arg Gly Ser Ser Thr Gly
    210                 215                 220

Gly Gln Asn Ala Asp Glu Ser Glu Pro Ser Tyr Leu Lys Ala Ser Glu
225                 230                 235                 240

Glu Glu Gly Asn
```

<210> 125
<211> 663
<212> DNA
<213> Populus tremula

<400> 125

```
atgcaacagc acctgatgca gatgcagccc atgatggcag cctattaccc cagcaacgtc      60

actactgatc atattcaaca gtatctggac gaaaacaagt cattgatttt gaagattgtt     120

gagagccaga attcagggaa actcagtgag tgtgcagaga accaagcaag actgcaacaa     180

aatctcatgt acttggctgc aattgctgat tgtcagcccc aaccacctac catgcatgcc     240

cagttccctt ccagcggcat tatgcagcca ggagcacatt acatgcagca tcaacaagct     300

caacagatga caccacaagc ccttatggct gcacgctctt ctatgctgca gtatgctcaa     360

cagccattct cagcgcttca acaacagcaa gccttacaca gccagctcgg catgagctct     420

ggtggaagcg caggacttca tatgatgcaa agcgaggcta acactgcagg aggcagtgga     480

gctcttggtg ctggacgatt tcctgatttt ggcatggatg cctccagtag aggaatcgca     540

agtgggagca agcaagatat tcggagtgca gggtctagtg aagggcgagg aggaagctct     600

ggaggccagg gtggtgatgg aggtgaaacc ctttacttga aatctgctga tgatgggaac     660

tga                                                                   663
```

<210> 126
<211> 220
<212> PRT
<213> Populus tremula

<400> 126

```
Met Gln Gln His Leu Met Gln Met Gln Pro Met Met Ala Ala Tyr Tyr
1               5                   10                  15

Pro Ser Asn Val Thr Thr Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45
```

```
Ser Glu Cys Ala Glu Asn Gln Ala Arg Leu Gln Gln Asn Leu Met Tyr
    50                  55                  60

Leu Ala Ala Ile Ala Asp Cys Gln Pro Gln Pro Pro Thr Met His Ala
65                  70                  75                  80

Gln Phe Pro Ser Ser Gly Ile Met Gln Pro Gly Ala His Tyr Met Gln
                85                  90                  95

His Gln Gln Ala Gln Gln Met Thr Pro Gln Ala Leu Met Ala Ala Arg
            100                 105                 110

Ser Ser Met Leu Gln Tyr Ala Gln Gln Pro Phe Ser Ala Leu Gln Gln
        115                 120                 125

Gln Gln Ala Leu His Ser Gln Leu Gly Met Ser Ser Gly Gly Ser Ala
    130                 135                 140

Gly Leu His Met Met Gln Ser Glu Ala Asn Thr Ala Gly Gly Ser Gly
145                 150                 155                 160

Ala Leu Gly Ala Gly Arg Phe Pro Asp Phe Gly Met Asp Ala Ser Ser
                165                 170                 175

Arg Gly Ile Ala Ser Gly Ser Lys Gln Asp Ile Arg Ser Ala Gly Ser
            180                 185                 190

Ser Glu Gly Arg Gly Gly Ser Ser Gly Gly Gln Gly Gly Asp Gly Gly
        195                 200                 205

Glu Thr Leu Tyr Leu Lys Ser Ala Asp Asp Gly Asn
    210                 215                 220


<210>  127
<211>  678
<212>  DNA
<213>  Saccharum officinarum

<400>  127
atgcagcagc aacacctgat gcagatgaac cagaacatga ttgggggcta cacctctcct      60

gccgctgtga caaccgatct catccagcag tacctggatg agaacaagca gctgatcctg     120

gccatcctcg acaaccagaa caatggcaag gtggaggagt gcgaacggca ccaagctaag     180

ctccagcaca acctcatgta cctggccgcc atcgccgaca gccagccacc acagactgca     240

ccactatcac aatacccgtc caacctgatg atgcagccgg gccctcggta catgccaccg     300

cagtccgggc agatgatgag cccgcagtcg ctaatggcgg cgcggtcctc catgatgtac     360

gcgcacccgt ccatgtcacc actccagcag cagcaggcag cgcacgggca gctgggcatg     420
```

gcttcagggg gcggcggtgg cacgaccagt gggttcaaca tcctccatgg cgaggccagt 480

atgggcggtg ctggtggcgc ttgtgccggc aacaacatga tgaacgccgg catgttctca 540

ggctttggcc gcagcggcag tggcgccaag gagggatcga cctcgctgtc ggttgacgtc 600

cgtggtggca ccagctccgg cgcgcaaagc ggggacggcg agtacctgaa agcaggcacc 660

gaggaagaag gcagttaa 678

<210> 128
<211> 225
<212> PRT
<213> Saccharum officinarum

<400> 128

Met Gln Gln Gln His Leu Met Gln Met Asn Gln Asn Met Ile Gly Gly
1 5 10 15

Tyr Thr Ser Pro Ala Ala Val Thr Thr Asp Leu Ile Gln Gln Tyr Leu
20 25 30

Asp Glu Asn Lys Gln Leu Ile Leu Ala Ile Leu Asp Asn Gln Asn Asn
35 40 45

Gly Lys Val Glu Glu Cys Glu Arg His Gln Ala Lys Leu Gln His Asn
50 55 60

Leu Met Tyr Leu Ala Ala Ile Ala Asp Ser Gln Pro Pro Gln Thr Ala
65 70 75 80

Pro Leu Ser Gln Tyr Pro Ser Asn Leu Met Met Gln Pro Gly Pro Arg
85 90 95

Tyr Met Pro Pro Gln Ser Gly Gln Met Met Ser Pro Gln Ser Leu Met
100 105 110

Ala Ala Arg Ser Ser Met Met Tyr Ala His Pro Ser Met Ser Pro Leu
115 120 125

Gln Gln Gln Gln Ala Ala His Gly Gln Leu Gly Met Ala Ser Gly Gly
130 135 140

Gly Gly Gly Thr Thr Ser Gly Phe Asn Ile Leu His Gly Glu Ala Ser
145 150 155 160

Met Gly Gly Ala Gly Gly Ala Cys Ala Gly Asn Asn Met Met Asn Ala
165 170 175

Gly Met Phe Ser Gly Phe Gly Arg Ser Gly Ser Gly Ala Lys Glu Gly
180 185 190

Ser Thr Ser Leu Ser Val Asp Val Arg Gly Gly Thr Ser Ser Gly Ala
195 200 205

Gln Ser Gly Asp Gly Glu Tyr Leu Lys Ala Gly Thr Glu Glu Glu Gly
210 215 220

Ser
225

<210> 129
<211> 561
<212> DNA
<213> Saccharum officinarum

<400> 129
atgcagcagc cgatgcccat gcagccgcag gcgccggaga tgaccccggc cgccggaatc 60

accacggagc agatccaaaa gtatctggat gagaataagc agcttatttt ggctattttg 120

gaaaatcaga acctaggaaa attggcagaa tgtgctcagt atcaatcaca acttcagaag 180

aacctcttgt atctcgctgc aatcgcagat gcccaaccac agactgctgt aagccgccct 240

cagatggcgc cgcctggtgc attgcctgga gtagggcagt acatgtcaca ggtgcctatg 300

ttcccaccga ggacacctct aacaccccag cagatgcagg agcagcaact tcagcagcag 360

caggctcagc tgctaaattt cagtggccta atggttgcta gacctggcat ggtcaacggc 420

atgcctcagt ccattcaagt tcagcaagct cagccaccac cagcagggaa caaacaggat 480

gctggtgggg tcgcctcgga gccctcgggc attgagaacc acaggagcac tggtggtgat 540

aatgatggtg gaagcgacta g 561

<210> 130
<211> 186
<212> PRT
<213> Saccharum officinarum

<400> 130

Met Gln Gln Pro Met Pro Met Gln Pro Gln Ala Pro Glu Met Thr Pro
1 5 10 15

Ala Ala Gly Ile Thr Thr Glu Gln Ile Gln Lys Tyr Leu Asp Glu Asn
20 25 30

Lys Gln Leu Ile Leu Ala Ile Leu Glu Asn Gln Asn Leu Gly Lys Leu
35 40 45

Ala Glu Cys Ala Gln Tyr Gln Ser Gln Leu Gln Lys Asn Leu Leu Tyr
50 55 60

Leu Ala Ala Ile Ala Asp Ala Gln Pro Gln Thr Ala Val Ser Arg Pro
65 70 75 80

```
Gln Met Ala Pro Pro Gly Ala Leu Pro Gly Val Gly Gln Tyr Met Ser
                85                  90                  95

Gln Val Pro Met Phe Pro Pro Arg Thr Pro Leu Thr Pro Gln Gln Met
            100                 105                 110

Gln Glu Gln Gln Leu Gln Gln Gln Gln Ala Gln Leu Leu Asn Phe Ser
        115                 120                 125

Gly Leu Met Val Ala Arg Pro Gly Met Val Asn Gly Met Pro Gln Ser
    130                 135                 140

Ile Gln Val Gln Gln Ala Gln Pro Pro Pro Ala Gly Asn Lys Gln Asp
145                 150                 155                 160

Ala Gly Gly Val Ala Ser Glu Pro Ser Gly Ile Glu Asn His Arg Ser
                165                 170                 175

Thr Gly Gly Asp Asn Asp Gly Gly Ser Asp
            180                 185
```

<210> 131
<211> 642
<212> DNA
<213> Saccharum officinarum

<400> 131

```
atgcagcagc agatgcccat gccgccggcg cccgctgcgg cggcggcgcc cccggcggcc      60

ggcatcacca ccgagcagat ccaaaagtat ttggacgaaa ataagcaact tattttggcc     120

atcctggaaa atcagaactt aggaaagttg gctgaatgtg ctcagtatca agctcaactt     180

caaaagaacc tcttgtacct ggctgcgatt gctgatgccc aaccccagcc accacaaaac     240

cctgcaggtc gccctcagat gatgcaacct ggtatagtgc caggtgcggg gcattacatg     300

tcacaagtac caatgttccc tccaagaact ccattaaccc cacagcagat gcaagagcag     360

cagcagcaac agcttcagca gcagcaagcg caggctctta cattccctgg acagatggtc     420

atgagaccag ctaccatcaa cggcatacag cagcctatgc aagctgaccc tgcccgggca     480

gcggagctgc aacaaccacc acctatccca gctgacgggc gagtaagcaa gcagcaggac     540

acaacggctg gcgtgagctc agagccttct gccaatgaga gccacaagac cacaactgga     600

gcagatagtg aggcaggtgg tgacgtggcg gagaaatcct aa                        642
```

<210> 132
<211> 213
<212> PRT
<213> Saccharum officinarum

<400> 132

```
Met Gln Gln Gln Met Pro Met Pro Pro Ala Pro Ala Ala Ala Ala Ala
1               5                   10                  15

Pro Pro Ala Ala Gly Ile Thr Thr Glu Gln Ile Gln Lys Tyr Leu Asp
            20                  25                  30

Glu Asn Lys Gln Leu Ile Leu Ala Ile Leu Glu Asn Gln Asn Leu Gly
        35                  40                  45

Lys Leu Ala Glu Cys Ala Gln Tyr Gln Ala Gln Leu Gln Lys Asn Leu
    50                  55                  60

Leu Tyr Leu Ala Ala Ile Ala Asp Ala Gln Pro Gln Pro Pro Gln Asn
65                  70                  75                  80

Pro Ala Gly Arg Pro Gln Met Met Gln Pro Gly Ile Val Pro Gly Ala
                85                  90                  95

Gly His Tyr Met Ser Gln Val Pro Met Phe Pro Pro Arg Thr Pro Leu
            100                 105                 110

Thr Pro Gln Gln Met Gln Glu Gln Gln Gln Gln Gln Leu Gln Gln Gln
        115                 120                 125

Gln Ala Gln Ala Leu Thr Phe Pro Gly Gln Met Val Met Arg Pro Ala
    130                 135                 140

Thr Ile Asn Gly Ile Gln Gln Pro Met Gln Ala Asp Pro Ala Arg Ala
145                 150                 155                 160

Ala Glu Leu Gln Gln Pro Pro Pro Ile Pro Ala Asp Gly Arg Val Ser
                165                 170                 175

Lys Gln Gln Asp Thr Thr Ala Gly Val Ser Ser Glu Pro Ser Ala Asn
            180                 185                 190

Glu Ser His Lys Thr Thr Thr Gly Ala Asp Ser Glu Ala Gly Gly Asp
        195                 200                 205

Val Ala Glu Lys Ser
    210
```

<210>  133
<211>  645
<212>  DNA
<213>  Solanum tuberosum

<400>  133
atgcagcagc acctgatgca gatgcagccc atgatggcag cttactatcc aacgaacgtc      60

actactgacc atattcaaca gtatttggat gagaacaaat cactcattct gaaaattgtt 120

gagagccaaa actcgggaaa actcagtgaa tgtgcagaga accaagctag gcttcagagg 180

aatctgatgt accttgctgc tattgctgat tcacaacctc agccttctag catgcattct 240

cagttctctt ctggtgggat gatgcagcca gggacacaca gttacctgca gcagcagcag 300

cagcaacaac aagcgcaaca aatggcaaca caacaactca tggctgcaag atcctcatca 360

atgctctatg gacaacaaca gcagcagcag cagcagtctc agttatcaca atttcaacaa 420

ggcttgcata gtagccaact tggcatgagt tctggcagtg gtggaagcac tggacttcat 480

cacatgcttc aaagtgaatc atcacctcat ggtggtggtt tctctcatga cttcggccgt 540

gcaaataagc aagacattgg gagtagtatg tctgctgaag ggcgcggcgg aagctcaggt 600

ggtgatggtg gtgagaatct ttatctgaaa gcttctgagg attga 645

<210> 134
<211> 214
<212> PRT
<213> Solanum tuberosum

<400> 134

Met Gln Gln His Leu Met Gln Met Gln Pro Met Met Ala Ala Tyr Tyr
1               5                   10                  15

Pro Thr Asn Val Thr Thr Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Ser Glu Cys Ala Glu Asn Gln Ala Arg Leu Gln Arg Asn Leu Met Tyr
    50                  55                  60

Leu Ala Ala Ile Ala Asp Ser Gln Pro Gln Pro Ser Ser Met His Ser
65                  70                  75                  80

Gln Phe Ser Ser Gly Gly Met Met Gln Pro Gly Thr His Ser Tyr Leu
                85                  90                  95

Gln Gln Gln Gln Gln Gln Gln Gln Ala Gln Gln Met Ala Thr Gln Gln
            100                 105                 110

Leu Met Ala Ala Arg Ser Ser Ser Met Leu Tyr Gly Gln Gln Gln Gln
        115                 120                 125

Gln Gln Gln Gln Ser Gln Leu Ser Gln Phe Gln Gln Gly Leu His Ser
    130                 135                 140

Ser Gln Leu Gly Met Ser Ser Gly Ser Gly Gly Ser Thr Gly Leu His

145 150 155 160

His Met Leu Gln Ser Glu Ser Ser Pro His Gly Gly Gly Phe Ser His
165 170 175

Asp Phe Gly Arg Ala Asn Lys Gln Asp Ile Gly Ser Ser Met Ser Ala
180 185 190

Glu Gly Arg Gly Gly Ser Ser Gly Gly Asp Gly Gly Glu Asn Leu Tyr
195 200 205

Leu Lys Ala Ser Glu Asp
210

<210> 135
<211> 645
<212> DNA
<213> Sorghum bicolor

<400> 135
atgcagcagc agatgcccat gccgccggcg cccgctgcgg cggcggcgac ggcgcccccg 60

gcggccggca tcaccaccga gcagatccag aagtatttgg acgaaaataa gcaacttatt 120

ttggccatcc tagaaaatca gaacttagga aagttggctg aatgtgctca gtatcaagct 180

caacttcaaa agaacctctt gtacctggct gcgattgctg atgcccaacc ccgaccaccg 240

caaaaccctg caggtcgccc tcagatgatg caacctggta tagtgccagg tgcagggcat 300

tacatgtcac aagtaccaat gttccctcca agaactccat taaccccaca gcaaatgcaa 360

gagcagcagc agcaacagct tcagcagcag caagcgcagg ctcttgcatt ccctgggcag 420

atggtcatga gaccagctac catcaacggc atgcagcagc ctatgcaggc tgaccctgcc 480

cgggcagcgg agctgcaaca gccagcatct gtcccagccg acgggcgagt aagcaagcag 540

gacacagcgg ctggggtgag ctcagagcct tctgccaatg agagccacaa gaccacaacc 600

ggagcagata gtgaggcagg tggagacgtg gcggagaaat cctaa 645

<210> 136
<211> 214
<212> PRT
<213> Sorghum bicolor

<400> 136

Met Gln Gln Gln Met Pro Met Pro Pro Ala Pro Ala Ala Ala Ala Ala
1 5 10 15

Thr Ala Pro Pro Ala Ala Gly Ile Thr Thr Glu Gln Ile Gln Lys Tyr
20 25 30

Leu Asp Glu Asn Lys Gln Leu Ile Leu Ala Ile Leu Glu Asn Gln Asn
35 40 45

Leu Gly Lys Leu Ala Glu Cys Ala Gln Tyr Gln Ala Gln Leu Gln Lys
    50                  55                  60

Asn Leu Leu Tyr Leu Ala Ala Ile Ala Asp Ala Gln Pro Arg Pro Pro
65                  70                  75                  80

Gln Asn Pro Ala Gly Arg Pro Gln Met Met Gln Pro Gly Ile Val Pro
                85                  90                  95

Gly Ala Gly His Tyr Met Ser Gln Val Pro Met Phe Pro Pro Arg Thr
            100                 105                 110

Pro Leu Thr Pro Gln Gln Met Gln Glu Gln Gln Gln Gln Gln Leu Gln
        115                 120                 125

Gln Gln Gln Ala Gln Ala Leu Ala Phe Pro Gly Gln Met Val Met Arg
    130                 135                 140

Pro Ala Thr Ile Asn Gly Met Gln Gln Pro Met Gln Ala Asp Pro Ala
145                 150                 155                 160

Arg Ala Ala Glu Leu Gln Gln Pro Ala Ser Val Pro Ala Asp Gly Arg
                165                 170                 175

Val Ser Lys Gln Asp Thr Ala Ala Gly Val Ser Ser Glu Pro Ser Ala
            180                 185                 190

Asn Glu Ser His Lys Thr Thr Thr Gly Ala Asp Ser Glu Ala Gly Gly
        195                 200                 205

Asp Val Ala Glu Lys Ser
    210

<210> 137
<211> 558
<212> DNA
<213> Triticum aestivum

<400> 137
atgcagcaag cgatgcccat gccgccggcg gcggcggcgc cggggatgcc tccgtctgct 60

ggcctcagca ccgagcagat ccaaaagtac ctggatgaaa ataagcaact aattttggct 120

atcttggaaa atcagaacct gggaaagttg gcggaatgtg ctcagtatca agctcagctt 180

cagaagaatc ttttgtattt ggctgcaatc gctgatactc agccacagac cactgtaagc 240

cgtcctcaga tggcaccacc tagtgcatcc ccaggggcag ggcattacat gtcacaggtg 300

ccaatgttcc ctccgaggac ccctctaacg cctcagcaga tgcaggagca gcaactacag 360

cagcaacagg ctcagatgct tccgtttgct ggtcaaatgg ttgcgagacc tggggctgtc 420

aatggcatgc ctcaggcccc tcaagttgaa ccagcctatg cagcaggtgg ggccagttct 480

gagccttctg gcactgagag ccacaggagc actggtgccg ataatgacgg ggggagcggc 540

tgggctgatc agtcctaa 558

<210> 138
<211> 185
<212> PRT
<213> Triticum aestivum

<400> 138

Met Gln Gln Ala Met Pro Met Pro Pro Ala Ala Ala Ala Pro Gly Met
1 5 10 15

Pro Pro Ser Ala Gly Leu Ser Thr Glu Gln Ile Gln Lys Tyr Leu Asp
20 25 30

Glu Asn Lys Gln Leu Ile Leu Ala Ile Leu Glu Asn Gln Asn Leu Gly
35 40 45

Lys Leu Ala Glu Cys Ala Gln Tyr Gln Ala Gln Leu Gln Lys Asn Leu
50 55 60

Leu Tyr Leu Ala Ala Ile Ala Asp Thr Gln Pro Gln Thr Thr Val Ser
65 70 75 80

Arg Pro Gln Met Ala Pro Pro Ser Ala Ser Pro Gly Ala Gly His Tyr
85 90 95

Met Ser Gln Val Pro Met Phe Pro Pro Arg Thr Pro Leu Thr Pro Gln
100 105 110

Gln Met Gln Glu Gln Gln Leu Gln Gln Gln Gln Ala Gln Met Leu Pro
115 120 125

Phe Ala Gly Gln Met Val Ala Arg Pro Gly Ala Val Asn Gly Met Pro
130 135 140

Gln Ala Pro Gln Val Glu Pro Ala Tyr Ala Ala Gly Gly Ala Ser Ser
145 150 155 160

Glu Pro Ser Gly Thr Glu Ser His Arg Ser Thr Gly Ala Asp Asn Asp
165 170 175

Gly Gly Ser Gly Trp Ala Asp Gln Ser
180 185

<210> 139
<211> 603

<212> DNA
<213> Triticum aestivum

<400> 139
atgcagcagg cgatgtcctt gcccccggga gcggtcggcg cggtgtcctc gccggccggc 60

atcaccaccg agcagatcca aaagtatttg gatgaaaata agcaacttat tttggccatc 120

cttgaaaatc agaacctagg aaagttggct gaatgtgctc agtatcaagc tcaactccaa 180

aagaatctct tgtatctagc tgctatcgcg gatgcccaac caccacagaa ccctacaagt 240

caccctcaga tggtgcagcc tggtagtatg caaggtgcag ggcattacat gtcacaagta 300

ccaatgttcc ctccaagaac gcctttaacc ccacagcaga tgcaagagca gcagcaccag 360

cagcttcagc agcagcaagc ccaggcccct tctttccccg cccaggtggt catgagacca 420

ggcaccgtca acggcatgca gcagcctatg caagcagccg gcgacctcca gccagcagca 480

gcacctggag ggagcaagca ggacgccgca gtggctgggg ccagctcgga accatctggc 540

accaagagcc acaagaacgc gggagcagag gaggtgggcg ctgatgtagc agaacaatcc 600

taa 603

<210> 140
<211> 200
<212> PRT
<213> Triticum aestivum

<400> 140

Met Gln Gln Ala Met Ser Leu Pro Pro Gly Ala Val Gly Ala Val Ser
1 5 10 15

Ser Pro Ala Gly Ile Thr Thr Glu Gln Ile Gln Lys Tyr Leu Asp Glu
20 25 30

Asn Lys Gln Leu Ile Leu Ala Ile Leu Glu Asn Gln Asn Leu Gly Lys
35 40 45

Leu Ala Glu Cys Ala Gln Tyr Gln Ala Gln Leu Gln Lys Asn Leu Leu
50 55 60

Tyr Leu Ala Ala Ile Ala Asp Ala Gln Pro Pro Gln Asn Pro Thr Ser
65 70 75 80

His Pro Gln Met Val Gln Pro Gly Ser Met Gln Gly Ala Gly His Tyr
85 90 95

Met Ser Gln Val Pro Met Phe Pro Pro Arg Thr Pro Leu Thr Pro Gln
100 105 110

Gln Met Gln Glu Gln Gln His Gln Gln Leu Gln Gln Gln Gln Ala Gln
115 120 125

```
Ala Leu Ser Phe Pro Ala Gln Val Val Met Arg Pro Gly Thr Val Asn
    130                 135                 140

Gly Met Gln Gln Pro Met Gln Ala Ala Gly Asp Leu Gln Pro Ala Ala
145                 150                 155                 160

Ala Pro Gly Gly Ser Lys Gln Asp Ala Ala Val Ala Gly Ala Ser Ser
                165                 170                 175

Glu Pro Ser Gly Thr Lys Ser His Lys Asn Ala Gly Ala Glu Glu Val
            180                 185                 190

Gly Ala Asp Val Ala Glu Gln Ser
        195                 200


<210>  141
<211>  672
<212>  DNA
<213>  Vitis vinifera

<400>  141
atgcagcagc acctgatgca gatgcagccc atgatggcag cctattaccc cagcaacgtc      60

accactgatc acattcagca gtatcttgat gaaaacaagt cattgattct gaagattgtt     120

gagagccaga attcaggaaa attgactgaa tgtgcagaga accaggcaag actacagaga     180

aacctcatgt acctggctgc aattgctgat tctcaacccc aaccacccac catgcatgct     240

cagttccctc ctagtggcat tgttcagcca ggagctcact acatgcaaca ccaacaagct     300

caacaaatga caccacagtc gctcctggct gcacgctcct ccatgctgta cacccaacaa     360

ccattttcgg ccctgcaaca acaacaagcc atccatagcc agcttggcat gggctctggt     420

ggaagtgcag gacttcacat gctgcaaagc gaggggagta atccaggagg caatggaaca     480

ctggggactg gtgggtttcc tgatttcagc cgtggaactt ctggagaagg cctgcaggct     540

gcaggcaggg gaatggctgg tgggagcaag caagatatgg gaaatgcaga agggcgagga     600

gggaactcag gaggtcaggg tggggatgga ggtgagactc tttacttgaa agctgctgaa     660

gatgggaatt ga                                                         672


<210>  142
<211>  223
<212>  PRT
<213>  Vitis vinifera

<400>  142

Met Gln Gln His Leu Met Gln Met Gln Pro Met Met Ala Ala Tyr Tyr
1               5                   10                  15

Pro Ser Asn Val Thr Thr Asp His Ile Gln Gln Tyr Leu Asp Glu Asn
            20                  25                  30
```

Lys Ser Leu Ile Leu Lys Ile Val Glu Ser Gln Asn Ser Gly Lys Leu
35 40 45

Thr Glu Cys Ala Glu Asn Gln Ala Arg Leu Gln Arg Asn Leu Met Tyr
50 55 60

Leu Ala Ala Ile Ala Asp Ser Gln Pro Gln Pro Pro Thr Met His Ala
65 70 75 80

Gln Phe Pro Pro Ser Gly Ile Val Gln Pro Gly Ala His Tyr Met Gln
85 90 95

His Gln Gln Ala Gln Gln Met Thr Pro Gln Ser Leu Leu Ala Ala Arg
100 105 110

Ser Ser Met Leu Tyr Thr Gln Gln Pro Phe Ser Ala Leu Gln Gln Gln
115 120 125

Gln Ala Ile His Ser Gln Leu Gly Met Gly Ser Gly Gly Ser Ala Gly
130 135 140

Leu His Met Leu Gln Ser Glu Gly Ser Asn Pro Gly Gly Asn Gly Thr
145 150 155 160

Leu Gly Thr Gly Gly Phe Pro Asp Phe Ser Arg Gly Thr Ser Gly Glu
165 170 175

Gly Leu Gln Ala Ala Gly Arg Gly Met Ala Gly Gly Ser Lys Gln Asp
180 185 190

Met Gly Asn Ala Glu Gly Arg Gly Gly Asn Ser Gly Gly Gln Gly Gly
195 200 205

Asp Gly Gly Glu Thr Leu Tyr Leu Lys Ala Ala Glu Asp Gly Asn
210 215 220

<210> 143
<211> 663
<212> DNA
<213> Zea mays

<400> 143
atgcagcagc agatgcccat gccgccggcg cccgctgccg ccgcggcggc ggcgcccccg 60

gcggcaggca tcactaccga gcagatccag aagtatttgg acgaaaataa gcaacttatt 120

ttggccatcc tggaaaatca gaacttaggg aagttggctg aatgtgctca gtatcaagct 180

caacttcaaa agaacctctt gtacctggct gcgattgctg atgcccaacc ccagcctccg 240

caaaaccctg caggtcgccc tcagatgatg cagcctggta tagtgccagg tgcggggcat 300

tacatgtcac aagtaccaat gttccctcca agaaccccat taaccccaca gcagatgcag 360

gagcagcagc aacaacaaca gtttcagcag cagcagcagc aagtgcaggc tcttacattt 420

cctggacaga tggtcatgag accaggcacc atcaacggca tgcagcagca gcagcctatg 480

caggctgacc ctgcccgggc agcagcggag ctgcagcagg cagcacctat cccagctgac 540

gggcgaggaa gcaagcagga caccgcgggt ggggcgagct cagagccttc tgccaatgag 600

agccacaaga gcgccaccgg agcagatacc gaggcaggtg gcgacgtggc cgagaaatcc 660

taa 663

<210> 144
<211> 220
<212> PRT
<213> Zea mays

<400> 144

```
Met Gln Gln Gln Met Pro Met Pro Pro Ala Pro Ala Ala Ala Ala Ala
1               5                   10                  15

Ala Ala Pro Pro Ala Ala Gly Ile Thr Thr Glu Gln Ile Gln Lys Tyr
            20                  25                  30

Leu Asp Glu Asn Lys Gln Leu Ile Leu Ala Ile Leu Glu Asn Gln Asn
        35                  40                  45

Leu Gly Lys Leu Ala Glu Cys Ala Gln Tyr Gln Ala Gln Leu Gln Lys
    50                  55                  60

Asn Leu Leu Tyr Leu Ala Ala Ile Ala Asp Ala Gln Pro Gln Pro Pro
65                  70                  75                  80

Gln Asn Pro Ala Gly Arg Pro Gln Met Met Gln Pro Gly Ile Val Pro
                85                  90                  95

Gly Ala Gly His Tyr Met Ser Gln Val Pro Met Phe Pro Pro Arg Thr
            100                 105                 110

Pro Leu Thr Pro Gln Gln Met Gln Glu Gln Gln Gln Gln Gln Gln Phe
        115                 120                 125

Gln Gln Gln Gln Gln Gln Val Gln Ala Leu Thr Phe Pro Gly Gln Met
    130                 135                 140

Val Met Arg Pro Gly Thr Ile Asn Gly Met Gln Gln Gln Gln Pro Met
145                 150                 155                 160

Gln Ala Asp Pro Ala Arg Ala Ala Ala Glu Leu Gln Gln Ala Ala Pro
                165                 170                 175
```

Ile Pro Ala Asp Gly Arg Gly Ser Lys Gln Asp Thr Ala Gly Gly Ala
180 185 190

Ser Ser Glu Pro Ser Ala Asn Glu Ser His Lys Ser Ala Thr Gly Ala
195 200 205

Asp Thr Glu Ala Gly Gly Asp Val Ala Glu Lys Ser
210 215 220

<210> 145
<211> 2193
<212> DNA
<213> Oryza sativa

<400> 145
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag attttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctcctcctc ccatctataa attcctcccc ccttttcccc tctctatata 1020

ggaggcatcc aagccaagaa gagggagagc accaaggaca cgcgactagc agaagccgag 1080

cgaccgcctt cttcgatcca tatcttccgg tcgagttctt ggtcgatctc ttccctcctc 1140

cacctcctcc tcacagggta tgtgcccttc ggttgttctt ggatttattg ttctaggttg 1200

tgtagtacgg gcgttgatgt taggaaaggg gatctgtatc tgtgatgatt cctgttcttg 1260

gatttgggat agaggggttc ttgatgttgc atgttatcgg ttcggtttga ttagtagtat 1320

ggttttcaat cgtctggaga gctctatgga aatgaaatgg tttagggtac ggaatcttgc 1380

```
gattttgtga gtacctttttg tttgaggtaa aatcagagca ccggtgattt tgcttggtgt   1440

aataaaagta cggttgtttg gtcctcgatt ctggtagtga tgcttctcga tttgacgaag   1500

ctatcctttg tttattccct attgaacaaa aataatccaa ctttgaagac ggtcccgttg   1560

atgagattga atgattgatt cttaagcctg tccaaaattt cgcagctggc ttgtttagat   1620

acagtagtcc ccatcacgaa attcatggaa acagttataa tcctcaggaa caggggattc   1680

cctgttcttc cgatttgctt tagtcccaga atttttttc ccaaatatct taaaaagtca   1740

ctttctggtt cagttcaatg aattgattgc tacaaataat gcttttatag cgttatccta   1800

gctgtagttc agttaatagg taatacccct atagtttagt caggagaaga acttatccga   1860

tttctgatct ccatttttaa ttatatgaaa tgaactgtag cataagcagt attcatttgg   1920

attatttttt ttattagctc tcaccccttc attattctga gctgaaagtc tggcatgaac   1980

tgtcctcaat tttgttttca aattcacatc gattatctat gcattatcct cttgtatcta   2040

cctgtagaag tttctttttg gttattcctt gactgcttga ttacagaaag aaatttatga   2100

agctgtaatc gggatagtta tactgcttgt tcttatgatt catttccttt gtgcagttct   2160

tggtgtagct tgccactttc accagcaaag ttc                                 2193


<210>  146
<211>  12
<212>  PRT
<213>  Artificial sequence

<220>
<223>  Box I


<220>
<221>  VARIANT
<222>  (3)..(3)
<223>  /replace = "Lys"

<220>
<221>  VARIANT
<222>  (4)..(4)
<223>  /replace = "Met" /replace = "Phe" /replace = "His"

<220>
<221>  VARIANT
<222>  (6)..(6)
<223>  /replace = "Glu"

<220>
<221>  VARIANT
<222>  (7)..(7)
<223>  /replace = "Asp"

<220>
<221>  VARIANT
<222>  (9)..(9)
<223>  /replace = "Asn"

<220>
```

<221> VARIANT
<222> (10)..(10)
<223> Xaa can be any naturally occurring amino acid

<400> 146

Ile Gln Gln Tyr Leu Asp Glu Asn Lys Xaa Leu Ile
1 5 10

<210> 147
<211> 10
<212> PRT
<213> Artificial sequence

<220>
<223> Box II

<220>
<221> VARIANT
<222> (3)..(3)
<223> /replace= "Leu" /replace= "Val"

<220>
<221> VARIANT
<222> (7)..(7)
<223> /replace= "Thr"

<400> 147

Asn Leu Met Tyr Leu Ala Ala Ile Ala Asp
1 5 10

<210> 148
<211> 53
<212> DNA
<213> Artificial sequence

<220>
<223> primer: prm06681

<400> 148
ggggacaagt ttgtacaaaa aagcaggctt aaacaatgca acagcacctg atg 53

<210> 149
<211> 50
<212> DNA
<213> Artificial sequence

<220>
<223> primer: prm06682

<400> 149
ggggaccact ttgtacaaga aagctgggtc atcattaaga ttccttgtgc 50

<210> 150
<211> 615
<212> DNA
<213> Brassica napus

<400> 150

atgcaacagc acctgatgca gatgcagccc atgatggctg gttactaccc cagcaatgtc 60

acctctgatc atattcagca gtacttggac gagaacaaat cgttgattct gaagatagtt 120

gaatctcaaa actcgggaaa gctcagcgag tgtgccgaga accaggcaag gcttcaacgc 180

aacttaatgt acttagctgc aattgcagat tctcagcctc aacctccaag catgcatagc 240

cagtatggaa ctgctggtgg tggtgggttg atgcagggag aaggagggtc acactatttg 300

caacagcaac aggcaattca acagcagcag agtcagcagt ctctaatggc ggctcgatct 360

tcaatgttgt atgctcagca gcagcaacag ccttatgcaa cgcttcagca gcagcaattg 420

caccatagcc agcttgggat gagctcaagc agcggaggag gaagcagcgg tctccatatg 480

ctacagggag aggctggtgg gtttcatgat tttggccgtg agaagttgga aatgggaagt 540

ggtgaaggca gaggaggaag ctcaggggat ggtggagaaa ccctttactt gaagtcatca 600

gatgatggga actga 615

<210> 151
<211> 204
<212> PRT
<213> Brassica napus

<400> 151

Met Gln Gln His Leu Met Gln Met Gln Pro Met Met Ala Gly Tyr Tyr
1 5 10 15

Pro Ser Asn Val Thr Ser Asp His Ile Gln Gln Tyr Leu Asp Glu Asn
20 25 30

Lys Ser Leu Ile Leu Lys Ile Val Glu Ser Gln Asn Ser Gly Lys Leu
35 40 45

Ser Glu Cys Ala Glu Asn Gln Ala Arg Leu Gln Arg Asn Leu Met Tyr
50 55 60

Leu Ala Ala Ile Ala Asp Ser Gln Pro Gln Pro Pro Ser Met His Ser
65 70 75 80

Gln Tyr Gly Thr Ala Gly Gly Gly Gly Leu Met Gln Gly Glu Gly Gly
85 90 95

Ser His Tyr Leu Gln Gln Gln Gln Ala Ile Gln Gln Gln Gln Ser Gln
100 105 110

Gln Ser Leu Met Ala Ala Arg Ser Ser Met Leu Tyr Ala Gln Gln Gln
115 120 125

Gln Gln Pro Tyr Ala Thr Leu Gln Gln Gln Gln Leu His His Ser Gln
130 135 140

Leu Gly Met Ser Ser Ser Ser Gly Gly Gly Ser Ser Gly Leu His Met
145 150 155 160

Leu Gln Gly Glu Ala Gly Gly Phe His Asp Phe Gly Arg Glu Lys Leu
165 170 175

Glu Met Gly Ser Gly Glu Gly Arg Gly Gly Ser Ser Gly Asp Gly Gly
180 185 190

Glu Thr Leu Tyr Leu Lys Ser Ser Asp Asp Gly Asn
195 200

<210> 152
<211> 639
<212> DNA
<213> Glycine max

<400> 152
atgcagcagc acctgatgca gatgcagccc atgatggctg cctactaccc caacaacgtc 60

accactgatc acattcaaca gtacctggat gagaacaagt ccttgattct gaagattgtt 120

gaaagccaga attctggcaa gctgagcgag tgtgccgaga accaatcaag gctgcagaga 180

aatctcatgt acctagctgc aatagctgat tctcaaccac aaccatctcc attggctggt 240

cagtatcctt ctagtggact tgtgcagcag ggagcacact acatgcaggc tcaacaggct 300

cagcagatgt cacaacaaca gctaatggct tcgcgctcct cgctcctgta ctcccaacag 360

cctttctcag tgcttcaaca gcagcaaggc atgcacagcc aacttggcat gagctccagt 420

ggaagtcaag gcctccacat gctgcaaagt gaagccacta atgttggagg caatgcaacc 480

ataggaaccg gaggagggtt tccggacttt gtacgcattg gtagtggcaa gcaagatatt 540

ggaatctctg gtgaaggcag aggaggaaac tctagtggcc actctggtga tggtggtgag 600

acacttaatt acctgaaagc tgctggtgat ggaaactga 639

<210> 153
<211> 212
<212> PRT
<213> Glycine max

<400> 153

Met Gln Gln His Leu Met Gln Met Gln Pro Met Met Ala Ala Tyr Tyr
1 5 10 15

Pro Asn Asn Val Thr Thr Asp His Ile Gln Gln Tyr Leu Asp Glu Asn
20 25 30

Lys Ser Leu Ile Leu Lys Ile Val Glu Ser Gln Asn Ser Gly Lys Leu
35 40 45

Ser Glu Cys Ala Glu Asn Gln Ser Arg Leu Gln Arg Asn Leu Met Tyr
50 55 60

Leu Ala Ala Ile Ala Asp Ser Gln Pro Gln Pro Ser Pro Leu Ala Gly
65 70 75 80

Gln Tyr Pro Ser Ser Gly Leu Val Gln Gln Gly Ala His Tyr Met Gln
85 90 95

Ala Gln Gln Ala Gln Gln Met Ser Gln Gln Gln Leu Met Ala Ser Arg
100 105 110

Ser Ser Leu Leu Tyr Ser Gln Gln Pro Phe Ser Val Leu Gln Gln Gln
115 120 125

Gln Gly Met His Ser Gln Leu Gly Met Ser Ser Ser Gly Ser Gln Gly
130 135 140

Leu His Met Leu Gln Ser Glu Ala Thr Asn Val Gly Gly Asn Ala Thr
145 150 155 160

Ile Gly Thr Gly Gly Gly Phe Pro Asp Phe Val Arg Ile Gly Ser Gly
165 170 175

Lys Gln Asp Ile Gly Ile Ser Gly Glu Gly Arg Gly Gly Asn Ser Ser
180 185 190

Gly His Ser Gly Asp Gly Gly Glu Thr Leu Asn Tyr Leu Lys Ala Ala
195 200 205

Gly Asp Gly Asn
210

<210> 154
<211> 1248
<212> DNA
<213> Chlamydomonas reinhardtii

<400> 154
atggccgcca ccatgctccg ctccagcacc cagtcgggca ttgccgctaa ggccggccgc 60

aaggaggctg tcagcgtccg cgcggtcgcc cagccccagc gccaggctgg tgctgccagc 120

gtcttctcct cgtcgtcgtc gggcgctgct gctcgccgcg gggtcgtcgc tcaggccacc 180

gctgttgcca cccccgcggc caagcctgcg gccaagacca gccagtatga gctgttcacg 240

ctcaccacct ggctgctgaa ggaggagatg aagggcacaa tcgatggcga gcttgtgacc 300

gtcatctcgt cggtgtcgct ggcctgcaag caaatcgcgt cgctggtgaa ccgcgctggt 360

atctccaacc tgaccggtgt ggctggcaac cagaacgtgc agggtgagga ccagaagaag 420

ctggacgtgg tgtccaacga ggtcttcaag aactgcctgg cctcctgcgg ccgcacgggt 480

```
gtgatcgcct ccgaggagga ggaccagccc gtggccgtgg aggagaccta ctcgggcaac     540
tacatcgtgg tgttcgaccc cctggacggc tcgtccaaca tcgacgccgg catctccgtc     600
ggctccatct tcggcatcta cgagcccagc gaggagtgcc ccattgacgc catggacgac     660
ccccagaaga tgatggagca gtgcgtcatg aacgtgtgcc agcccggctc gcgcctcaag     720
tgcgccggct actgcctgta cagcagcagc accatcatgg tgctgaccat cggcaacggt     780
gtgttcggct tcacgctgga ccccctggtc ggcgagttcg tgctgaccca ccccaacgtg     840
cagatccccg aggtgggcaa gatctacccg ttcaacgagg gcaactacgg cctgtgggac     900
gacagcgtta aggcttacat ggacagcctg aaggacccca agaagtggga cggcaagccc     960
tactcggccc gctacatcgg ctccctggtc ggtgacttcc accgcaccct gctgtacgga    1020
ggcatctacg gctaccccgg cgacgccaag aacaagaacg gcaagctccg cctgctgtac    1080
gagtgcgcgc ccatgtcgtt cattgccgag caggccggcg gcctcggctc caccggccag    1140
gagcgcgtgc tggacgtgaa ccccgagaag gtgcaccagc gcgtgccgct gttcatcggc    1200
tccaagaagg aggtcgagta cctggagtcc ttcaccaaga agcactaa                 1248
```

<210> 155
<211> 415
<212> PRT
<213> Chlamydomonas reinhardtii

<400> 155

```
Met Ala Ala Thr Met Leu Arg Ser Ser Thr Gln Ser Gly Ile Ala Ala
1               5                   10                  15

Lys Ala Gly Arg Lys Glu Ala Val Ser Val Arg Ala Val Ala Gln Pro
            20                  25                  30

Gln Arg Gln Ala Gly Ala Ala Ser Val Phe Ser Ser Ser Ser Ser Gly
        35                  40                  45

Ala Ala Ala Arg Arg Gly Val Val Ala Gln Ala Thr Ala Val Ala Thr
    50                  55                  60

Pro Ala Ala Lys Pro Ala Ala Lys Thr Ser Gln Tyr Glu Leu Phe Thr
65                  70                  75                  80

Leu Thr Thr Trp Leu Leu Lys Glu Glu Met Lys Gly Thr Ile Asp Gly
                85                  90                  95

Glu Leu Val Thr Val Ile Ser Ser Val Ser Leu Ala Cys Lys Gln Ile
            100                 105                 110

Ala Ser Leu Val Asn Arg Ala Gly Ile Ser Asn Leu Thr Gly Val Ala
        115                 120                 125
```

```
Gly Asn Gln Asn Val Gln Gly Glu Asp Gln Lys Lys Leu Asp Val Val
    130                 135                 140

Ser Asn Glu Val Phe Lys Asn Cys Leu Ala Ser Cys Gly Arg Thr Gly
145                 150                 155                 160

Val Ile Ala Ser Glu Glu Glu Asp Gln Pro Val Ala Val Glu Glu Thr
                165                 170                 175

Tyr Ser Gly Asn Tyr Ile Val Val Phe Asp Pro Leu Asp Gly Ser Ser
            180                 185                 190

Asn Ile Asp Ala Gly Ile Ser Val Gly Ser Ile Phe Gly Ile Tyr Glu
        195                 200                 205

Pro Ser Glu Glu Cys Pro Ile Asp Ala Met Asp Asp Pro Gln Lys Met
    210                 215                 220

Met Glu Gln Cys Val Met Asn Val Cys Gln Pro Gly Ser Arg Leu Lys
225                 230                 235                 240

Cys Ala Gly Tyr Cys Leu Tyr Ser Ser Ser Thr Ile Met Val Leu Thr
                245                 250                 255

Ile Gly Asn Gly Val Phe Gly Phe Thr Leu Asp Pro Leu Val Gly Glu
            260                 265                 270

Phe Val Leu Thr His Pro Asn Val Gln Ile Pro Glu Val Gly Lys Ile
        275                 280                 285

Tyr Pro Phe Asn Glu Gly Asn Tyr Gly Leu Trp Asp Asp Ser Val Lys
    290                 295                 300

Ala Tyr Met Asp Ser Leu Lys Asp Pro Lys Lys Trp Asp Gly Lys Pro
305                 310                 315                 320

Tyr Ser Ala Arg Tyr Ile Gly Ser Leu Val Gly Asp Phe His Arg Thr
                325                 330                 335

Leu Leu Tyr Gly Gly Ile Tyr Gly Tyr Pro Gly Asp Ala Lys Asn Lys
            340                 345                 350

Asn Gly Lys Leu Arg Leu Leu Tyr Glu Cys Ala Pro Met Ser Phe Ile
        355                 360                 365

Ala Glu Gln Ala Gly Gly Leu Gly Ser Thr Gly Gln Glu Arg Val Leu
    370                 375                 380
```

Asp Val Asn Pro Glu Lys Val His Gln Arg Val Pro Leu Phe Ile Gly
385 390 395 400

Ser Lys Lys Glu Val Glu Tyr Leu Glu Ser Phe Thr Lys Lys His
405 410 415

<210> 156
<211> 1239
<212> DNA
<213> Bigelowiella natans

<400> 156
atggctgccg tgctctttcg cggaggtgtg ctgtcttcca ccatgactgc ggctcgcaca 60

ttcgcagcgc catctgtgca cacccgtggc atgcacatgt cagtcaagag cagtaaccct 120

ttctctcagg ctggtcgtcg tgctgctgtt agatccagtg tcgcaccctc ccctgtccaa 180

gactctgctg gtactatcgt tgatgacgga actgtcacat taacaagatt catgattgag 240

gaggcaatga agagcaagac tcctgggcag gaggacatgg ttcgtttgat ttcttccatc 300

tcagttgcat gcaaacgcat tgcatccatg gtgcaaactg caggaatctc cggatctaca 360

ggtctggctg aaggtggtgg atcagtcaac gttcaaggcg aggaacagaa gaaacttgat 420

gtcatttcta acgatgtact aaagtctgcc cttcgtcctt ctggaaaact tggagtaatt 480

gcctctgagg aggaagataa tccagttgtc gttgatgaac tttactccgg cgaatatgtt 540

gctactttcg atccgctcga tggatcttcc aatattgatg ctgcaatttc cactggaact 600

atcttcggag tgttcaaagc tccagaagag tgcttgatcg ggattctga taatctcagt 660

attgcagagc agcaatgttt ggaggcaaca cttcaacctg gaactaacct tgttgctgct 720

ggatactgca tgtattcgtc ctccaccatc cttgttctca ccaccggaga cgggctcaat 780

ggatttactc ttgacccttc cattggagag ttcatcctca cccatcctaa tatccagatt 840

cctagccgtg gaaagatcta ttctatgaac gaagcaaact acttcgattg ggaccctaag 900

cttcagacct acgttgataa ccttaagaag gcagaaggtc aaactggaga aaagtacagc 960

tctcgctaca tcggatccat ggtcggagat gtgcaccgta cccttctcta tggaggcatc 1020

ttcgcttacc ctggagataa gaagaacgtc aacggaaaac ttcgccttct gtacgaagct 1080

gctccaatgt cccttatctt cgaacaagcg ggtggaaaat ctattactgg acctggtgga 1140

cgtgtgttgg acttagttcc tgataaggtt caccagcgtt gtcctgtgtt cattggatcc 1200

cctgatgatg tggatgaagt tgaaaaggct ctcgcataa 1239

<210> 157
<211> 412
<212> PRT
<213> Bigelowiella natans

<400> 157

Met Ala Ala Val Leu Phe Arg Gly Gly Val Leu Ser Ser Thr Met Thr
1               5                   10                  15

Ala Ala Arg Thr Phe Ala Ala Pro Ser Val His Thr Arg Gly Met His
            20                  25                  30

Met Ser Val Lys Ser Ser Asn Pro Phe Ser Gln Ala Gly Arg Arg Ala
        35                  40                  45

Ala Val Arg Ser Ser Val Ala Pro Ser Pro Val Gln Asp Ser Ala Gly
    50                  55                  60

Thr Ile Val Asp Asp Gly Thr Val Thr Leu Thr Arg Phe Met Ile Glu
65                  70                  75                  80

Glu Ala Met Lys Ser Lys Thr Pro Gly Gln Glu Asp Met Val Arg Leu
                85                  90                  95

Ile Ser Ser Ile Ser Val Ala Cys Lys Arg Ile Ala Ser Met Val Gln
            100                 105                 110

Thr Ala Gly Ile Ser Gly Ser Thr Gly Leu Ala Glu Gly Gly Gly Ser
        115                 120                 125

Val Asn Val Gln Gly Glu Glu Gln Lys Lys Leu Asp Val Ile Ser Asn
    130                 135                 140

Asp Val Leu Lys Ser Ala Leu Arg Pro Ser Gly Lys Leu Gly Val Ile
145                 150                 155                 160

Ala Ser Glu Glu Glu Asp Asn Pro Val Val Val Asp Glu Leu Tyr Ser
                165                 170                 175

Gly Glu Tyr Val Ala Thr Phe Asp Pro Leu Asp Gly Ser Ser Asn Ile
            180                 185                 190

Asp Ala Ala Ile Ser Thr Gly Thr Ile Phe Gly Val Phe Lys Ala Pro
        195                 200                 205

Glu Glu Cys Leu Ile Gly Asp Ser Asp Asn Leu Ser Ile Ala Glu Gln
    210                 215                 220

Gln Cys Leu Glu Ala Thr Leu Gln Pro Gly Thr Asn Leu Val Ala Ala
225                 230                 235                 240

Gly Tyr Cys Met Tyr Ser Ser Ser Thr Ile Leu Val Leu Thr Thr Gly
                245                 250                 255

Asp Gly Leu Asn Gly Phe Thr Leu Asp Pro Ser Ile Gly Glu Phe Ile

260 265 270

Leu Thr His Pro Asn Ile Gln Ile Pro Ser Arg Gly Lys Ile Tyr Ser
275 280 285

Met Asn Glu Ala Asn Tyr Phe Asp Trp Asp Pro Lys Leu Gln Thr Tyr
290 295 300

Val Asp Asn Leu Lys Lys Ala Glu Gly Gln Thr Gly Glu Lys Tyr Ser
305 310 315 320

Ser Arg Tyr Ile Gly Ser Met Val Gly Asp Val His Arg Thr Leu Leu
325 330 335

Tyr Gly Gly Ile Phe Ala Tyr Pro Gly Asp Lys Lys Asn Val Asn Gly
340 345 350

Lys Leu Arg Leu Leu Tyr Glu Ala Ala Pro Met Ser Leu Ile Phe Glu
355 360 365

Gln Ala Gly Gly Lys Ser Ile Thr Gly Pro Gly Gly Arg Val Leu Asp
370 375 380

Leu Val Pro Asp Lys Val His Gln Arg Cys Pro Val Phe Ile Gly Ser
385 390 395 400

Pro Asp Asp Val Asp Glu Val Glu Lys Ala Leu Ala
405 410

<210> 158
<211> 1230
<212> DNA
<213> Aquilegia formosa x Aquilegia pubescens

<400> 158
atggttgcag cagctatccc tacttcttgt caactgctct tctccacctc ttcttcgacc 60

acttctcgtc tatatcctcc ttatcttgat gccaaaactc tcttctcatt tcctacaaac 120

aagagacatg taagcattgt tagaactccg gcaggtgtac ggtgtcaagc attaggagca 180

gaggtagtag tgaccaagag gagtgcattt gagatacaaa ctctaacagg atggttattg 240

aaacaagaac aaacaggggt tatagatgca gagttaacta tagttttgtc tagcatttca 300

atggcctgta agcagattgc ttcattggtg cagagggcaa gtatttccaa cttaactgga 360

gttcaaggag ctgttaatat tcaaggtgaa gatcagaaga agcttgatgt catctctaac 420

gaggtgttct ctaattgcct tagatcaagt ggaagaacag ggattatagc atcagaagaa 480

gaggatgtac cagttgcagt ggaggaaagc tactctggca actatattgt cgtttttgat 540

cctcttgacg ggtcatcaaa cattgatgcc gctgtgtcaa ctggatccat atttggaatt 600

```
tatagtccga acgatgagtg tcttactgaa gtcgatgata atgccacagt gcttcagcaa    660

gtggaacaga agtgcatcat caatgtgtgt caacctggca acaacttgtt ggcagctggc    720

tactgcatgt actcaagctc tgtaatcttt gtgctttcca ttggacaagg ggttttctca    780

tttaccttag accctatgta cggagaattc gtcttgacac aggaaaacat tcaaatacct    840

aaatcaggta aaatctactc attcaacgaa ggcaactacc aattatggga tgataagttg    900

aagaagtata tcgatgacct taaggaccct ggtcctagtg gcaaaccata ctcttccaga    960

tacattggaa gcttggttgg cgatttccac cggacccttc tttatggagg gatatatgga   1020

taccctaggg ataagaatag aaaaaatggg aagctaaggc tactgtatga gtgtgcacct   1080

atgagttatt tagttgaaca agcaggagga aaaggatcag atggacacca aagagtactc   1140

gatattgaac cggtggagat tcatcagcgt gttccacttt tcattggcag cgttgaagaa   1200

gttgagaaac tagagaagtt cttggcttga                                    1230
```

```
<210>  159
<211>  409
<212>  PRT
<213>  Aquilegia formosa x Aquilegia pubescens

<400>  159

Met Val Ala Ala Ala Ile Pro Thr Ser Cys Gln Leu Leu Phe Ser Thr
1               5                   10                  15

Ser Ser Ser Thr Thr Ser Arg Leu Tyr Pro Pro Tyr Leu Asp Ala Lys
            20                  25                  30

Thr Leu Phe Ser Phe Pro Thr Asn Lys Arg His Val Ser Ile Val Arg
        35                  40                  45

Thr Pro Ala Gly Val Arg Cys Gln Ala Leu Gly Ala Glu Val Val Val
    50                  55                  60

Thr Lys Arg Ser Ala Phe Glu Ile Gln Thr Leu Thr Gly Trp Leu Leu
65                  70                  75                  80

Lys Gln Glu Gln Thr Gly Val Ile Asp Ala Glu Leu Thr Ile Val Leu
                85                  90                  95

Ser Ser Ile Ser Met Ala Cys Lys Gln Ile Ala Ser Leu Val Gln Arg
            100                 105                 110

Ala Ser Ile Ser Asn Leu Thr Gly Val Gln Gly Ala Val Asn Ile Gln
        115                 120                 125

Gly Glu Asp Gln Lys Lys Leu Asp Val Ile Ser Asn Glu Val Phe Ser
    130                 135                 140
```

```
Asn Cys Leu Arg Ser Ser Gly Arg Thr Gly Ile Ile Ala Ser Glu Glu
145                 150                 155                 160

Glu Asp Val Pro Val Ala Val Glu Glu Ser Tyr Ser Gly Asn Tyr Ile
                165                 170                 175

Val Val Phe Asp Pro Leu Asp Gly Ser Ser Asn Ile Asp Ala Ala Val
            180                 185                 190

Ser Thr Gly Ser Ile Phe Gly Ile Tyr Ser Pro Asn Asp Glu Cys Leu
        195                 200                 205

Thr Glu Val Asp Asp Asn Ala Thr Val Leu Gln Gln Val Glu Gln Lys
    210                 215                 220

Cys Ile Ile Asn Val Cys Gln Pro Gly Asn Asn Leu Leu Ala Ala Gly
225                 230                 235                 240

Tyr Cys Met Tyr Ser Ser Ser Val Ile Phe Val Leu Ser Ile Gly Gln
                245                 250                 255

Gly Val Phe Ser Phe Thr Leu Asp Pro Met Tyr Gly Glu Phe Val Leu
            260                 265                 270

Thr Gln Glu Asn Ile Gln Ile Pro Lys Ser Gly Lys Ile Tyr Ser Phe
        275                 280                 285

Asn Glu Gly Asn Tyr Gln Leu Trp Asp Asp Lys Leu Lys Lys Tyr Ile
    290                 295                 300

Asp Asp Leu Lys Asp Pro Gly Pro Ser Gly Lys Pro Tyr Ser Ser Arg
305                 310                 315                 320

Tyr Ile Gly Ser Leu Val Gly Asp Phe His Arg Thr Leu Leu Tyr Gly
                325                 330                 335

Gly Ile Tyr Gly Tyr Pro Arg Asp Lys Asn Arg Lys Asn Gly Lys Leu
            340                 345                 350

Arg Leu Leu Tyr Glu Cys Ala Pro Met Ser Tyr Leu Val Glu Gln Ala
        355                 360                 365

Gly Gly Lys Gly Ser Asp Gly His Gln Arg Val Leu Asp Ile Glu Pro
    370                 375                 380

Val Glu Ile His Gln Arg Val Pro Leu Phe Ile Gly Ser Val Glu Glu
385                 390                 395                 400
```

Val Glu Lys Leu Glu Lys Phe Leu Ala
405

<210> 160
<211> 1254
<212> DNA
<213> Arabidopsis thaliana

<400> 160
atggcagcat cggccgcaac aacgacgtcg tctcaccttc ttctctcaag ctcacgtcac 60

gtggcttcat catcccagcc ttcaatcctt tcaccgagat ctctcttctc caacaacggg 120

aaacgagcac caactggagt gagaaaccat cagtatgcga gtggagtgag gtgtatggcc 180

gtagcggcgg atgcttctga gacgaaaacg gcggcgagga agaagagtgg atacgaactt 240

caaacgttga cgggctggtt gctgagacaa gagatgaaag gagagataga tgcagagctg 300

acgatagtga tgtcgagtat atcattggct tgtaagcaga tcgcttcact tgttcaacgt 360

gccggaatat ctaacctgac cggagttcaa ggcgccatta atattcaggg agaggatcag 420

aagaagcttg acgtcatctc taatgaggtg ttttccaact gtttgagatc aagtggaaga 480

acgggaatca tagcctcgga ggaagaggac gtgccagttg cggtggagga gagttactcc 540

ggcaactacg tcgtcgtgtt tgaccctctt gatggttcct ccaacattga cgctgccgtc 600

tctactggtt ctatcttcgg tatctatagc cccaatgacg aatgcattgt cgacgactcc 660

gacgatatct cagctcttgg gtcagaagaa caaaggtgta tagtaaacgt gtgccagcca 720

gggaacaact tgttagcagc cggctactgt atgtactcga gctcggtcat cttcgttctt 780

actctaggca aaggcgtttt ctccttcacg cttgatccaa tgtacggtga gtttgtcctc 840

acgcaagaaa acattgagat ccccaaagcc gggagaatct actctttcaa cgaagggaat 900

taccagatgt gggacgataa actaaagaag tacattgatg accttaagga ccctggtcca 960

actgggaagc cttactcggc aaggtacatt ggaagtttgg ttggagattt tcacaggact 1020

ttgttgtacg gtgggattta cgggtaccct cgtgacgcaa agagcaaaaa tggaaagctt 1080

aggcttttgt atgagtgtgc accaatgagt ttcattgttg aacaagctgg agggaaaggt 1140

tctgatggac attcgagagt actagatatc caaccgactg agatacatca gagggttcct 1200

ctatacattg gaagcacaga ggaagtagag aagttggaga agtacttggc ttga 1254

<210> 161
<211> 417
<212> PRT
<213> Arabidopsis thaliana

<400> 161

Met Ala Ala Ser Ala Ala Thr Thr Thr Ser Ser His Leu Leu Leu Ser
1 5 10 15

Ser Ser Arg His Val Ala Ser Ser Ser Gln Pro Ser Ile Leu Ser Pro

```
            20                  25                  30

Arg Ser Leu Phe Ser Asn Asn Gly Lys Arg Ala Pro Thr Gly Val Arg
        35                  40                  45

Asn His Gln Tyr Ala Ser Gly Val Arg Cys Met Ala Val Ala Ala Asp
    50                  55                  60

Ala Ser Glu Thr Lys Thr Ala Ala Arg Lys Lys Ser Gly Tyr Glu Leu
65                  70                  75                  80

Gln Thr Leu Thr Gly Trp Leu Leu Arg Gln Glu Met Lys Gly Glu Ile
                85                  90                  95

Asp Ala Glu Leu Thr Ile Val Met Ser Ser Ile Ser Leu Ala Cys Lys
            100                 105                 110

Gln Ile Ala Ser Leu Val Gln Arg Ala Gly Ile Ser Asn Leu Thr Gly
        115                 120                 125

Val Gln Gly Ala Ile Asn Ile Gln Gly Glu Asp Gln Lys Lys Leu Asp
    130                 135                 140

Val Ile Ser Asn Glu Val Phe Ser Asn Cys Leu Arg Ser Ser Gly Arg
145                 150                 155                 160

Thr Gly Ile Ile Ala Ser Glu Glu Glu Asp Val Pro Val Ala Val Glu
                165                 170                 175

Glu Ser Tyr Ser Gly Asn Tyr Val Val Val Phe Asp Pro Leu Asp Gly
            180                 185                 190

Ser Ser Asn Ile Asp Ala Ala Val Ser Thr Gly Ser Ile Phe Gly Ile
        195                 200                 205

Tyr Ser Pro Asn Asp Glu Cys Ile Val Asp Asp Ser Asp Asp Ile Ser
    210                 215                 220

Ala Leu Gly Ser Glu Glu Gln Arg Cys Ile Val Asn Val Cys Gln Pro
225                 230                 235                 240

Gly Asn Asn Leu Leu Ala Ala Gly Tyr Cys Met Tyr Ser Ser Ser Val
                245                 250                 255

Ile Phe Val Leu Thr Leu Gly Lys Gly Val Phe Ser Phe Thr Leu Asp
            260                 265                 270

Pro Met Tyr Gly Glu Phe Val Leu Thr Gln Glu Asn Ile Glu Ile Pro
        275                 280                 285
```

Lys Ala Gly Arg Ile Tyr Ser Phe Asn Glu Gly Asn Tyr Gln Met Trp
    290                 295                 300

Asp Asp Lys Leu Lys Lys Tyr Ile Asp Asp Leu Lys Asp Pro Gly Pro
305                 310                 315                 320

Thr Gly Lys Pro Tyr Ser Ala Arg Tyr Ile Gly Ser Leu Val Gly Asp
                325                 330                 335

Phe His Arg Thr Leu Leu Tyr Gly Gly Ile Tyr Gly Tyr Pro Arg Asp
            340                 345                 350

Ala Lys Ser Lys Asn Gly Lys Leu Arg Leu Leu Tyr Glu Cys Ala Pro
        355                 360                 365

Met Ser Phe Ile Val Glu Gln Ala Gly Gly Lys Gly Ser Asp Gly His
    370                 375                 380

Ser Arg Val Leu Asp Ile Gln Pro Thr Glu Ile His Gln Arg Val Pro
385                 390                 395                 400

Leu Tyr Ile Gly Ser Thr Glu Glu Val Glu Lys Leu Glu Lys Tyr Leu
                405                 410                 415

Ala

<210>  162
<211>  1254
<212>  DNA
<213>  Brassica napus

<400>  162
atggcagcaa ccgccgcgac aacgtcatcg tctcatcttc ttctctcaag ctctcgccac    60

gtggcagcct catctcagcc acaaatcctt ttccagagat ctctgttttc cggcgggaaa   120

cgatcggcag ctggaaaaaa ccatcatgct agtggtggag tgaggtgtat ggcggttgca   180

gcggatgctt cggcggaggc taagccggcg gcagcgagga agaagagtgg gtacgagctt   240

cagacgctga cgggttggtt gctgagacaa gagcagaaag gagagataga tacagagctg   300

acgatagtga tgtcgagcat agcgatggct tgtaagcaga tcgcttcgct tgtacagcgc   360

gccggaatct ctaatctgac cggcgttcaa ggagccgtta acattcaggg agaggatcag   420

aaaaagctcg acgtcgtctc taatgaggta ttttcaaact gtttgagatc aagtggaagg   480

acagggatca ttgcgtcaga ggaagaggac gtccccgtag cagttgaaga gagttactcc   540

ggaaactaca ttgtcgtctt tgatcctctc gacggttcct ccaacatcga cgctgcagtc   600

tccactggtt caatcttcgg tatctacagc cccaatgacg agtgtatcgt tgacgactcc   660

gacgatatct cctctcttgg ttcagaagaa caaaggtgta tagtaaacgt gtgtcaacca 720

gggaacaact tgctcgcagc tggctactgt atgtactcga gctcagtcat cttcgttctc 780

accttaggca agggcgtttt ctccttcaca ctcgacccaa tgtacggcga gttcgtcctc 840

actcaagaga acattgagat ccccaaagca gggaaaatct actctttcaa cgaagggaac 900

taccagatgt gggacgagaa actgaagaag tacattgatg atcttaagga ccctggtcca 960

agtgggaagc cttactctgc aaggtacatt ggtagtttgg tcggagactt tcacagaact 1020

ttgttgtacg gtgggattta cgggtaccct cgtgacgcca agagcaaaaa cggtaagctt 1080

aggcttttgt atgagtgtgc accgatgagt ttcatcgttg aacaagctgg aggaaaagga 1140

tcagatggcc accaaagagt actagatatc caacccaccg agatacatca gagggttcca 1200

ctttacattg gaagcaaaga agaagtagag aagctggaga agtacttggc ttga 1254

<210> 163
<211> 417
<212> PRT
<213> Brassica napus

<400> 163

Met Ala Ala Thr Ala Ala Thr Thr Ser Ser Ser His Leu Leu Leu Ser
1 5 10 15

Ser Ser Arg His Val Ala Ala Ser Ser Gln Pro Gln Ile Leu Phe Gln
20 25 30

Arg Ser Leu Phe Ser Gly Gly Lys Arg Ser Ala Ala Gly Lys Asn His
35 40 45

His Ala Ser Gly Gly Val Arg Cys Met Ala Val Ala Ala Asp Ala Ser
50 55 60

Ala Glu Ala Lys Pro Ala Ala Ala Arg Lys Lys Ser Gly Tyr Glu Leu
65 70 75 80

Gln Thr Leu Thr Gly Trp Leu Leu Arg Gln Glu Gln Lys Gly Glu Ile
85 90 95

Asp Thr Glu Leu Thr Ile Val Met Ser Ser Ile Ala Met Ala Cys Lys
100 105 110

Gln Ile Ala Ser Leu Val Gln Arg Ala Gly Ile Ser Asn Leu Thr Gly
115 120 125

Val Gln Gly Ala Val Asn Ile Gln Gly Glu Asp Gln Lys Lys Leu Asp
130 135 140

Val Val Ser Asn Glu Val Phe Ser Asn Cys Leu Arg Ser Ser Gly Arg
145 150 155 160

Thr Gly Ile Ile Ala Ser Glu Glu Glu Asp Val Pro Val Ala Val Glu
165 170 175

Glu Ser Tyr Ser Gly Asn Tyr Ile Val Val Phe Asp Pro Leu Asp Gly
180 185 190

Ser Ser Asn Ile Asp Ala Ala Val Ser Thr Gly Ser Ile Phe Gly Ile
195 200 205

Tyr Ser Pro Asn Asp Glu Cys Ile Val Asp Asp Ser Asp Asp Ile Ser
210 215 220

Ser Leu Gly Ser Glu Glu Gln Arg Cys Ile Val Asn Val Cys Gln Pro
225 230 235 240

Gly Asn Asn Leu Leu Ala Ala Gly Tyr Cys Met Tyr Ser Ser Ser Val
245 250 255

Ile Phe Val Leu Thr Leu Gly Lys Gly Val Phe Ser Phe Thr Leu Asp
260 265 270

Pro Met Tyr Gly Glu Phe Val Leu Thr Gln Glu Asn Ile Glu Ile Pro
275 280 285

Lys Ala Gly Lys Ile Tyr Ser Phe Asn Glu Gly Asn Tyr Gln Met Trp
290 295 300

Asp Glu Lys Leu Lys Lys Tyr Ile Asp Asp Leu Lys Asp Pro Gly Pro
305 310 315 320

Ser Gly Lys Pro Tyr Ser Ala Arg Tyr Ile Gly Ser Leu Val Gly Asp
325 330 335

Phe His Arg Thr Leu Leu Tyr Gly Gly Ile Tyr Gly Tyr Pro Arg Asp
340 345 350

Ala Lys Ser Lys Asn Gly Lys Leu Arg Leu Leu Tyr Glu Cys Ala Pro
355 360 365

Met Ser Phe Ile Val Glu Gln Ala Gly Gly Lys Gly Ser Asp Gly His
370 375 380

Gln Arg Val Leu Asp Ile Gln Pro Thr Glu Ile His Gln Arg Val Pro
385 390 395 400

Leu Tyr Ile Gly Ser Lys Glu Glu Val Glu Lys Leu Glu Lys Tyr Leu

405 410 415

Ala

<210> 164
<211> 1130
<212> DNA
<213> Cyanidioschyzon merolae

<400> 164
atggcgaagc aagcaccgca cgctttcgtt agccttgggg ctcctaagct gcgctcaaca 60

aacatatggg gaagggtatc ggtgtcggtc ttgccgctgg aaacaaggca tcaacgtcgc 120

tttggtgcag tgagactgcg cgcagcttta gacctaccca tgacactcgg acagtgggtt 180

ctccaacagg agaggcagca tccagagacc gctgatttgg ctcctttgat aacagccaca 240

gcgacagctg gcaagcagat tgcctcactg gtcgcccgag cgggcgtgag caacttgact 300

ggccttcagg gctcggtcaa cgtgcagggc gaggagcaga aaaagctgga cgtgttgacg 360

aacgaggtgc tcaagaacgc gctacgctca acagggaaac taggtgtctt ggcttcggaa 420

gaggaaaacg agccagtgtc gctcatggaa gaggcgtata caggtgactt tattgctgcg 480

tttgacccgt tggacggatc ctcgaatttg gatgcagcga tcgccacggg tacgattttt 540

ggtgtaatgc gcagcggcga gcactgtctg acgggacccg aggacgcgga tataagtcag 600

cgacaaatgc agtgcctcgt gcagacgctg caaccgggtg cgaatctggt ggcagcgggc 660

tacattctct actcctcatc ggtgattttc atgctgtcta ttggtcacgg agtgcagggt 720

ttttctctgg acccagcgat tggcgagttt gttttgacgc acccagatgt tcgtgttccg 780

gagcgtggca agatctactc cttcaacgag gcaaactcgg ataactggga ccctgttctt 840

caagaatata ttcgatcgat gaagaaaaag ggctattctt cgcgctacat aggatctctc 900

gttggcgatg tccatcgcac gctgatttac ggcggcgttt ttggataccc tggtgataag 960

aagaacccga acggcaagtt gcgtcttctg tacgagtgcg cacctatggc gtatctcatg 1020

gagcaggccg gcggtatagc gaccacggga aagcaaagaa ttctggatat tgttccgcga 1080

gatgtccatc agcgagagcc gttcatttgc ggaagcccca gggatgttga 1130

<210> 165
<211> 391
<212> PRT
<213> Cyanidioschyzon merolae

<400> 165

Met Ala Lys Gln Ala Pro His Ala Phe Val Ser Leu Gly Ala Pro Lys
1 5 10 15

Leu Arg Ser Thr Asn Ile Trp Gly Arg Val Ser Val Ser Val Leu Pro
20 25 30

```
Leu Glu Thr Arg His Gln Arg Arg Phe Gly Ala Val Arg Leu Arg Ala
        35                  40                  45

Ala Leu Asp Leu Pro Met Thr Leu Gly Gln Trp Val Leu Gln Gln Glu
    50                  55                  60

Arg Gln His Pro Glu Thr Ala Asp Leu Ala Pro Leu Ile Thr Ala Thr
65                  70                  75                  80

Ala Thr Ala Gly Lys Gln Ile Ala Ser Leu Val Ala Arg Ala Gly Val
                85                  90                  95

Ser Asn Leu Thr Gly Leu Gln Gly Ser Val Asn Val Gln Gly Glu Glu
            100                 105                 110

Gln Lys Lys Leu Asp Val Leu Thr Asn Glu Val Leu Lys Asn Ala Leu
        115                 120                 125

Arg Ser Thr Gly Lys Leu Gly Val Leu Ala Ser Glu Glu Glu Asn Glu
    130                 135                 140

Pro Val Ser Leu Met Glu Glu Ala Tyr Thr Gly Asp Phe Ile Ala Ala
145                 150                 155                 160

Phe Asp Pro Leu Asp Gly Ser Ser Asn Leu Asp Ala Ala Ile Ala Thr
                165                 170                 175

Gly Thr Ile Phe Gly Val Met Arg Ser Gly Glu His Cys Leu Thr Gly
            180                 185                 190

Pro Glu Asp Ala Asp Ile Ser Gln Arg Gln Met Gln Cys Leu Val Gln
        195                 200                 205

Thr Leu Gln Pro Gly Ala Asn Leu Val Ala Ala Gly Tyr Ile Leu Tyr
    210                 215                 220

Ser Ser Ser Val Ile Phe Met Leu Ser Ile Gly His Gly Val Gln Gly
225                 230                 235                 240

Phe Ser Leu Asp Pro Ala Ile Gly Glu Phe Val Leu Thr His Pro Asp
                245                 250                 255

Val Arg Val Pro Glu Arg Gly Lys Ile Tyr Ser Phe Asn Glu Ala Asn
            260                 265                 270

Ser Asp Asn Trp Asp Pro Val Leu Gln Glu Tyr Ile Arg Ser Met Lys
        275                 280                 285
```

Lys Lys Gly Tyr Ser Ser Arg Tyr Ile Gly Ser Leu Val Gly Asp Val
    290                 295                 300

His Arg Thr Leu Ile Tyr Gly Gly Val Phe Gly Tyr Pro Gly Asp Lys
305                 310                 315                 320

Lys Asn Pro Asn Gly Lys Leu Arg Leu Leu Tyr Glu Cys Ala Pro Met
                325                 330                 335

Ala Tyr Leu Met Glu Gln Ala Gly Gly Ile Ala Thr Thr Gly Lys Gln
            340                 345                 350

Arg Ile Leu Asp Ile Val Pro Arg Asp Val His Gln Arg Glu Pro Phe
        355                 360                 365

Ile Cys Gly Ser Pro Arg Asp Val Glu Asp Leu Leu Lys Ile Tyr Gln
    370                 375                 380

Gly Ser Met Ala Met Arg Gly
385                 390

<210> 166
<211> 1218
<212> DNA
<213> Glycine max

<400> 166
atggttgcaa tggcagcagc aacagcatcc acccagttga ttttctcaaa gccttgttcc 60

ccttcacgtc tatgcccctt ccaactatgt gtctttgaca ctaaacaagt gctatcaagt 120

ggcaggagaa ggcatgtggg gggttctgga gttaggtgca tggctgtggg ggaagcagca 180

accactggga caaagaagag aagtggatat gagcttcaaa cactcactag ctggttgctg 240

aagcaggagc aagctggggt gattgatgca gaactcacta ttgtgctgtc tagcatttcc 300

atggcatgca aacagattgc ttctttggtg caaagagcta acatttccaa cctcactggg 360

gttcaaggtg ctgtcaatgt tcaaggggaa gaccagaaaa agcttgatgt tgtttcaaat 420

gaggttttct caaactgctt gaggtcaagt gggaggacag ggataatagc atcagaggag 480

gaagatgtgc cagtggcagt agaagagagt tattctggaa actacattgt ggtgtttgac 540

ccacttgatg ggtcatccaa tattgatgct gcagcgtcaa ctgggtccaa tttttggata 600

tacagcccca atgatgagtg tcttgctgac attgatgatg accccaccct tgacacaaca 660

gaacaaagat gtattgtgaa cgtgtgccaa cctggaagca accttcttgc agctggttac 720

tgcatgtatt ctagctcaat aatctttgtt ctcacacttg gaaatggagt gtttgtgttt 780

acattggacc cgatgtatgg cgaattcgtt ttgactcagg aaaacctcca gatacctaga 840

gcaggcaaaa tctatgcatt caatgaaggt aattatcagt tgtgggatga gaagctaaag 900

aaatatattg atgatctcaa ggacccaggt caaagcggca agccttattc tgcaaggtac 960

attggtagct tggtaggaga tttccacagg acactgctat atggtggcat ttacgggtac 1020

cccagggaca agaaaagcaa gaatgggaaa ctaaggctcc tgtatgaatg tgctcctatt 1080

aacttcattg tagaacaagc tggtggaaaa ggtacagatg gccttcaagt actccggctt 1140

caagggacag agattcatca acgtgtgcca ctgtacattg ggaagaggt agagaaggtg 1200

gaaaagtact tggcttaa 1218

<210> 167
<211> 405
<212> PRT
<213> Glycine max

<400> 167

Met Val Ala Met Ala Ala Ala Thr Ala Ser Thr Gln Leu Ile Phe Ser
1 5 10 15

Lys Pro Cys Ser Pro Ser Arg Leu Cys Pro Phe Gln Leu Cys Val Phe
20 25 30

Asp Thr Lys Gln Val Leu Ser Ser Gly Arg Arg Arg His Val Gly Gly
35 40 45

Ser Gly Val Arg Cys Met Ala Val Gly Glu Ala Ala Thr Thr Gly Thr
50 55 60

Lys Lys Arg Ser Gly Tyr Glu Leu Gln Thr Leu Thr Ser Trp Leu Leu
65 70 75 80

Lys Gln Glu Gln Ala Gly Val Ile Asp Ala Glu Leu Thr Ile Val Leu
85 90 95

Ser Ser Ile Ser Met Ala Cys Lys Gln Ile Ala Ser Leu Val Gln Arg
100 105 110

Ala Asn Ile Ser Asn Leu Thr Gly Val Gln Gly Ala Val Asn Val Gln
115 120 125

Gly Glu Asp Gln Lys Lys Leu Asp Val Val Ser Asn Glu Val Phe Ser
130 135 140

Asn Cys Leu Arg Ser Ser Gly Arg Thr Gly Ile Ile Ala Ser Glu Glu
145 150 155 160

Glu Asp Val Pro Val Ala Val Glu Glu Ser Tyr Ser Gly Asn Tyr Ile
165 170 175

Val Val Phe Asp Pro Leu Asp Gly Ser Ser Asn Ile Asp Ala Ala Ala

            180                 185                 190

Ser Thr Gly Ser Asn Phe Trp Ile Tyr Ser Pro Asn Asp Glu Cys Leu
        195                 200                 205

Ala Asp Ile Asp Asp Asp Pro Thr Leu Asp Thr Thr Glu Gln Arg Cys
    210                 215                 220

Ile Val Asn Val Cys Gln Pro Gly Ser Asn Leu Leu Ala Ala Gly Tyr
225                 230                 235                 240

Cys Met Tyr Ser Ser Ser Ile Ile Phe Val Leu Thr Leu Gly Asn Gly
                245                 250                 255

Val Phe Val Phe Thr Leu Asp Pro Met Tyr Gly Glu Phe Val Leu Thr
            260                 265                 270

Gln Glu Asn Leu Gln Ile Pro Arg Ala Gly Lys Ile Tyr Ala Phe Asn
        275                 280                 285

Glu Gly Asn Tyr Gln Leu Trp Asp Glu Lys Leu Lys Lys Tyr Ile Asp
    290                 295                 300

Asp Leu Lys Asp Pro Gly Gln Ser Gly Lys Pro Tyr Ser Ala Arg Tyr
305                 310                 315                 320

Ile Gly Ser Leu Val Gly Asp Phe His Arg Thr Leu Leu Tyr Gly Gly
                325                 330                 335

Ile Tyr Gly Tyr Pro Arg Asp Lys Lys Ser Lys Asn Gly Lys Leu Arg
            340                 345                 350

Leu Leu Tyr Glu Cys Ala Pro Ile Asn Phe Ile Val Glu Gln Ala Gly
        355                 360                 365

Gly Lys Gly Thr Asp Gly Leu Gln Val Leu Arg Leu Gln Gly Thr Glu
    370                 375                 380

Ile His Gln Arg Val Pro Leu Tyr Ile Gly Glu Glu Val Glu Lys Val
385                 390                 395                 400

Glu Lys Tyr Leu Ala
                405

<210>  168
<211>  1212
<212>  DNA
<213>  Lycopersicon esculentum

<400>  168

```
atggcagcaa cagcaacaac ttcatattta agtgctctag acaaaaagac tccattttta      60
tttgccttgg acaaaaagac tccattttta tgcccaaaaa acagcacaaa gagaaggtca     120
tttaatggag gagttaagtg catggcaata gagacagctt caggtgttac acaaaccaag     180
aaaaagagtg gctatgagtt acaaacttta acaagttggc tattgagaca agaacaagct     240
ggagttattg atgctgaact taccatagta atttcaagta tttcaatggc ttgtaaacag     300
attgcttctt tggtccaaag agctggaatt tctaacctta ctggagttca aggtgctgtc     360
aatattcaag gagaagatca gaagaaactt gatgttgtct ctaatgaggt tttctcgaat     420
tgtctaagat caagtggaag gactgggatt atagcatcag aagaagagga tgtacctgtg     480
gcagtggaag agagttactc aggcaactac attgtggtgt ttgatcctct tgatggatca     540
tcaaacattg atgctgctgt atctaccggt tctatctttg gaatatacag cccgaatgat     600
gagtgcctag ctgatcttgg agatgattcc acgcttgaca atatagagca aaagtgcatc     660
gtgaatgtat gtcaaccagg gacaaacctt cttgcagcag gatactgcat gtactcaagc     720
tctgtgattt tcgtactcac cttgggaaat ggcgtttttt cctttaactt ggatccaatg     780
tatggagaat ttgttctgac tcaagaaaat gtccaaatac caaagtctgg aaagatctat     840
tcattcaatg aaggaaacta ccagctctgg gatgacaagt tgaaaaaata tatcgatgac     900
ttgaaagacc ctggtcctag tggcaagcct tactctgcaa ggtacattgg tagtttggta     960
ggtgacttcc atagaactct tctatatggt ggcatttatg gttatcctag agacagaaag    1020
agcaagaatg gaaagttgag gcttttgtac gaatgtgctc ccatgagctt cattgtggaa    1080
caagctggtg gcaaaggatc cgatggtcac caaagagttc tcgatatcca accaactgag    1140
atacatcaac gagttccatt gtacattgga agcacagaag aagttgaaaa attggagaag    1200
tacttgtctt aa                                                        1212
```

```
<210>  169
<211>  403
<212>  PRT
<213>  Lycopersicon esculentum

<400>  169

Met Ala Ala Thr Ala Thr Thr Ser Tyr Leu Ser Ala Leu Asp Lys Lys
1               5                   10                  15

Thr Pro Phe Leu Phe Ala Leu Asp Lys Lys Thr Pro Phe Leu Cys Pro
            20                  25                  30

Lys Asn Ser Thr Lys Arg Arg Ser Phe Asn Gly Gly Val Lys Cys Met
        35                  40                  45

Ala Ile Glu Thr Ala Ser Gly Val Thr Gln Thr Lys Lys Lys Ser Gly
    50                  55                  60
```

```
Tyr Glu Leu Gln Thr Leu Thr Ser Trp Leu Leu Arg Gln Glu Gln Ala
65                  70                  75                  80

Gly Val Ile Asp Ala Glu Leu Thr Ile Val Ile Ser Ser Ile Ser Met
                85                  90                  95

Ala Cys Lys Gln Ile Ala Ser Leu Val Gln Arg Ala Gly Ile Ser Asn
            100                 105                 110

Leu Thr Gly Val Gln Gly Ala Val Asn Ile Gln Gly Glu Asp Gln Lys
        115                 120                 125

Lys Leu Asp Val Val Ser Asn Glu Val Phe Ser Asn Cys Leu Arg Ser
    130                 135                 140

Ser Gly Arg Thr Gly Ile Ile Ala Ser Glu Glu Glu Asp Val Pro Val
145                 150                 155                 160

Ala Val Glu Glu Ser Tyr Ser Gly Asn Tyr Ile Val Val Phe Asp Pro
                165                 170                 175

Leu Asp Gly Ser Ser Asn Ile Asp Ala Ala Val Ser Thr Gly Ser Ile
            180                 185                 190

Phe Gly Ile Tyr Ser Pro Asn Asp Glu Cys Leu Ala Asp Leu Gly Asp
        195                 200                 205

Asp Ser Thr Leu Asp Asn Ile Glu Gln Lys Cys Ile Val Asn Val Cys
    210                 215                 220

Gln Pro Gly Thr Asn Leu Leu Ala Ala Gly Tyr Cys Met Tyr Ser Ser
225                 230                 235                 240

Ser Val Ile Phe Val Leu Thr Leu Gly Asn Gly Val Phe Ser Phe Asn
                245                 250                 255

Leu Asp Pro Met Tyr Gly Glu Phe Val Leu Thr Gln Glu Asn Val Gln
            260                 265                 270

Ile Pro Lys Ser Gly Lys Ile Tyr Ser Phe Asn Glu Gly Asn Tyr Gln
        275                 280                 285

Leu Trp Asp Asp Lys Leu Lys Lys Tyr Ile Asp Asp Leu Lys Asp Pro
    290                 295                 300

Gly Pro Ser Gly Lys Pro Tyr Ser Ala Arg Tyr Ile Gly Ser Leu Val
305                 310                 315                 320
```

Gly Asp Phe His Arg Thr Leu Leu Tyr Gly Gly Ile Tyr Gly Tyr Pro
325 330 335

Arg Asp Arg Lys Ser Lys Asn Gly Lys Leu Arg Leu Leu Tyr Glu Cys
340 345 350

Ala Pro Met Ser Phe Ile Val Glu Gln Ala Gly Gly Lys Gly Ser Asp
355 360 365

Gly His Gln Arg Val Leu Asp Ile Gln Pro Thr Glu Ile His Gln Arg
370 375 380

Val Pro Leu Tyr Ile Gly Ser Thr Glu Glu Val Glu Lys Leu Glu Lys
385 390 395 400

Tyr Leu Ser

<210> 170
<211> 1236
<212> DNA
<213> Medicago truncatula

<400> 170
atggttgcaa tggcagcagc aacagcatca tcacaattaa tattctcaaa accttgttct 60

ccctcacgtc tatgtccctt ccaactttgt gttttcgaca cgaaatcagt gttatcaagt 120

tcaagaagaa agcatgtgag tggctctgga ggagtgagat gcatggctgt tggtgaagta 180

gctgctgaga caaagaaaag aagtagttat gagcttataa cattgactag ttggttgttg 240

aagcaagaac aaacaggggt tattgatgct gaacttacta ttgttttgaa tagtatttcg 300

ttggcatgta aacagattgc ttctttggtt caaagagcta atatttctaa ccttactggt 360

gttcaaggtg ctgtaaatat tcaaggggag gatcagaaaa aacttgatgt tgtctcaaat 420

gaggtattct caaattgttt gaggtcaagt gggaggacag ggattatagc atcagaggaa 480

gaggatgtgc cagtggcagt agaagagagt tattcaggaa actacattgt ggtctttgat 540

ccacttgatg gttcatcgaa tattgatgct gcagtttcaa ctggttctat ttttgggatt 600

tacagcccca atgatgagtg tcttgctgac gtaggcaatg agtccgatga ccccacactt 660

ggcacagaag aacaacgatg cattgtgaat gtgtgccaac caggaagcaa ccttctagca 720

gccggttact gcatgtattc tagctcagta atcttcgttc taacaatcgg caaaggagtt 780

ttcgtattca cattagatcc aatgtatggg gaatttgttt tgactcaaga aaatctccaa 840

ataccaaaat cagggaaaat ttattctttc aatgaaggaa attacaagtt atgggatgac 900

aacttgaaga aatacatcga tgatctcaag gaaccgggtg ctaatggcaa accttattca 960

gcaaggtata ttggtagttt ggtaggtgat ttccatagga cactgctata tggtggcatt 1020

tatggttacc ctagggacaa gaaaagtaag aatggaaggc ttaggctttt atatgagtgt 1080

gctccaatga gtttcattgt agaacaggct ggtggaaaag gttcagatgg tcatcaaaga 1140

gtacttgaca ttcaacccac cgaaattcat caacgtgttc cactgtacat tgggagcaca 1200

gaggaagtgg agaaggttga aaagtacttg gcttaa 1236

<210> 171
<211> 411
<212> PRT
<213> Medicago truncatula

<400> 171

Met Val Ala Met Ala Ala Ala Thr Ala Ser Ser Gln Leu Ile Phe Ser
1 5 10 15

Lys Pro Cys Ser Pro Ser Arg Leu Cys Pro Phe Gln Leu Cys Val Phe
20 25 30

Asp Thr Lys Ser Val Leu Ser Ser Ser Arg Arg Lys His Val Ser Gly
35 40 45

Ser Gly Gly Val Arg Cys Met Ala Val Gly Glu Val Ala Ala Glu Thr
50 55 60

Lys Lys Arg Ser Ser Tyr Glu Leu Ile Thr Leu Thr Ser Trp Leu Leu
65 70 75 80

Lys Gln Glu Gln Thr Gly Val Ile Asp Ala Glu Leu Thr Ile Val Leu
85 90 95

Asn Ser Ile Ser Leu Ala Cys Lys Gln Ile Ala Ser Leu Val Gln Arg
100 105 110

Ala Asn Ile Ser Asn Leu Thr Gly Val Gln Gly Ala Val Asn Ile Gln
115 120 125

Gly Glu Asp Gln Lys Lys Leu Asp Val Val Ser Asn Glu Val Phe Ser
130 135 140

Asn Cys Leu Arg Ser Ser Gly Arg Thr Gly Ile Ile Ala Ser Glu Glu
145 150 155 160

Glu Asp Val Pro Val Ala Val Glu Glu Ser Tyr Ser Gly Asn Tyr Ile
165 170 175

Val Val Phe Asp Pro Leu Asp Gly Ser Ser Asn Ile Asp Ala Ala Val
180 185 190

Ser Thr Gly Ser Ile Phe Gly Ile Tyr Ser Pro Asn Asp Glu Cys Leu
195 200 205

```
Ala Asp Val Gly Asn Glu Ser Asp Asp Pro Thr Leu Gly Thr Glu Glu
    210                 215                 220

Gln Arg Cys Ile Val Asn Val Cys Gln Pro Gly Ser Asn Leu Leu Ala
225                 230                 235                 240

Ala Gly Tyr Cys Met Tyr Ser Ser Ser Val Ile Phe Val Leu Thr Ile
                245                 250                 255

Gly Lys Gly Val Phe Val Phe Thr Leu Asp Pro Met Tyr Gly Glu Phe
            260                 265                 270

Val Leu Thr Gln Glu Asn Leu Gln Ile Pro Lys Ser Gly Lys Ile Tyr
        275                 280                 285

Ser Phe Asn Glu Gly Asn Tyr Lys Leu Trp Asp Asp Asn Leu Lys Lys
    290                 295                 300

Tyr Ile Asp Asp Leu Lys Glu Pro Gly Ala Asn Gly Lys Pro Tyr Ser
305                 310                 315                 320

Ala Arg Tyr Ile Gly Ser Leu Val Gly Asp Phe His Arg Thr Leu Leu
                325                 330                 335

Tyr Gly Gly Ile Tyr Gly Tyr Pro Arg Asp Lys Lys Ser Lys Asn Gly
            340                 345                 350

Arg Leu Arg Leu Leu Tyr Glu Cys Ala Pro Met Ser Phe Ile Val Glu
        355                 360                 365

Gln Ala Gly Gly Lys Gly Ser Asp Gly His Gln Arg Val Leu Asp Ile
    370                 375                 380

Gln Pro Thr Glu Ile His Gln Arg Val Pro Leu Tyr Ile Gly Ser Thr
385                 390                 395                 400

Glu Glu Val Glu Lys Val Glu Lys Tyr Leu Ala
                405                 410


<210>  172
<211>  1236
<212>  DNA
<213>  Nicotiana tabacum

<400>  172
atggcagcat catcagcaac agcaacagca acaacttcat ttttatgtgc tttagacaaa      60

aagactccat ttttatgtac tctagacaaa aaaggtactc catttttatg cccaaaaggc     120

agcagcacaa caaagagaag gtcatttaat ggaggagtga agtgcatggc aatagagaca     180
```

acagcaggag ctacagagac caagaaaaga agtggctatg agttacaaac tttaacaagc 240

tggctattaa ggcaagaaca agctgggagt attgatgctg aacttaccat agtgatttca 300

agtatttcta tggcttgtaa gcagattgct tctttggttc agagagctgg aatttctaac 360

cttactggag ttcaaggtgc tgtcaatatt caaggagaag accagaagaa gcttgatgtt 420

gtctctaacg aggttttctc gaattgtcta aggtcgagtg gaaggactgg gattatagca 480

tcagaggaag aggatgtacc agtggcagtg gaagagagtt actcaggaaa ctacattgtg 540

gtgtttgacc ctcttgatgg atcatcaaac attgatgctg ctgtttctac tggttctatt 600

ttcggaatat acagcccaaa tgatgagtgc ctcgcagatc atggagatga ttccacgctt 660

gacaatgttg aacagaggtg tattgtgaat gtatgccaac cagggagcaa ccttcttgca 720

gcaggctact gcatgtactc aagctctgtg atttttgtgg tcaccttggg aaacggagtc 780

tttgccttca acttggatcc gatgtatgga gaattcgttc tgacccaaga aaacatccaa 840

ataccaaaat ctggaaagat ctattcgttc aacgaaggaa actaccagct ttgggatgac 900

aaactgaaga aatacatcga tgacttgaag gaccctggtc ctagcggcaa gccttactct 960

gctaggtaca ttggtagttt ggttggtgac ttccatagaa ctcttctata tggtggcatt 1020

tatggctatc ctagagataa aaagagtaag aatggaaagt tgaggctttt gtatgagtgt 1080

gcccctatga gcttcctcgt ggaacaagct ggtggcaaag gatccgatgg ccaccaaaga 1140

gttcttgata tccaaccaac tgagatacac cagcgagtcc cattgtacat tggaagcaca 1200

gaagaagttg aaaagttgga gaagtattta tcttaa 1236

<210> 173
<211> 411
<212> PRT
<213> Nicotiana tabacum

<400> 173

Met Ala Ala Ser Ser Ala Thr Ala Thr Ala Thr Thr Ser Phe Leu Cys
1 5 10 15

Ala Leu Asp Lys Lys Thr Pro Phe Leu Cys Thr Leu Asp Lys Lys Gly
20 25 30

Thr Pro Phe Leu Cys Pro Lys Gly Ser Ser Thr Thr Lys Arg Arg Ser
35 40 45

Phe Asn Gly Gly Val Lys Cys Met Ala Ile Glu Thr Thr Ala Gly Ala
50 55 60

Thr Glu Thr Lys Lys Arg Ser Gly Tyr Glu Leu Gln Thr Leu Thr Ser
65 70 75 80

Trp Leu Leu Arg Gln Glu Gln Ala Gly Ser Ile Asp Ala Glu Leu Thr
85 90 95

Ile Val Ile Ser Ser Ile Ser Met Ala Cys Lys Gln Ile Ala Ser Leu
100 105 110

Val Gln Arg Ala Gly Ile Ser Asn Leu Thr Gly Val Gln Gly Ala Val
115 120 125

Asn Ile Gln Gly Glu Asp Gln Lys Lys Leu Asp Val Val Ser Asn Glu
130 135 140

Val Phe Ser Asn Cys Leu Arg Ser Ser Gly Arg Thr Gly Ile Ile Ala
145 150 155 160

Ser Glu Glu Glu Asp Val Pro Val Ala Val Glu Glu Ser Tyr Ser Gly
165 170 175

Asn Tyr Ile Val Val Phe Asp Pro Leu Asp Gly Ser Ser Asn Ile Asp
180 185 190

Ala Ala Val Ser Thr Gly Ser Ile Phe Gly Ile Tyr Ser Pro Asn Asp
195 200 205

Glu Cys Leu Ala Asp His Gly Asp Asp Ser Thr Leu Asp Asn Val Glu
210 215 220

Gln Arg Cys Ile Val Asn Val Cys Gln Pro Gly Ser Asn Leu Leu Ala
225 230 235 240

Ala Gly Tyr Cys Met Tyr Ser Ser Ser Val Ile Phe Val Val Thr Leu
245 250 255

Gly Asn Gly Val Phe Ala Phe Asn Leu Asp Pro Met Tyr Gly Glu Phe
260 265 270

Val Leu Thr Gln Glu Asn Ile Gln Ile Pro Lys Ser Gly Lys Ile Tyr
275 280 285

Ser Phe Asn Glu Gly Asn Tyr Gln Leu Trp Asp Asp Lys Leu Lys Lys
290 295 300

Tyr Ile Asp Asp Leu Lys Asp Pro Gly Pro Ser Gly Lys Pro Tyr Ser
305 310 315 320

Ala Arg Tyr Ile Gly Ser Leu Val Gly Asp Phe His Arg Thr Leu Leu
325 330 335

Tyr Gly Gly Ile Tyr Gly Tyr Pro Arg Asp Lys Lys Ser Lys Asn Gly

340 345 350

Lys Leu Arg Leu Leu Tyr Glu Cys Ala Pro Met Ser Phe Leu Val Glu
355 360 365

Gln Ala Gly Gly Lys Gly Ser Asp Gly His Gln Arg Val Leu Asp Ile
370 375 380

Gln Pro Thr Glu Ile His Gln Arg Val Pro Leu Tyr Ile Gly Ser Thr
385 390 395 400

Glu Glu Val Glu Lys Leu Glu Lys Tyr Leu Ser
405 410

<210> 174
<211> 1221
<212> DNA
<213> Oryza sativa

<400> 174
atggccgccg cagccacgac atcctcccac ctgctcctgc tctcccgcca gcaggcggcg 60
gcctcgctcc aatgcggcct ctccttccgg aggcagcccg gcaggctcgc cggtgggtcg 120
tcggccccga gcgtgcggtg catggcggcc gtcgacacgg cctcggcgcc cgccgcgacg 180
gaggctagca agaagagcag ctacgagatc accacgctga cgacgtggct gctgaagcag 240
gagcaggccg ggaccatcga cggcgagatg accatcgtgc tggccagcat ctccacggcg 300
tgcaagcaga tcgcctcgct ggtgcagcgc gcgcccatct ccaacctcac cggcgtccag 360
ggcgccgtca acgtgcaggg cgaggaccag aaaaaactcg acgtcgtctc caacgaggtg 420
ttctccaact gcctcaaatc gagcgggcgc accggcgtga tcgcgtcgga ggaggaggac 480
gtgccggtgg ccgtggagga gagctactcg ggcaactaca tcgtggtgtt cgacccgctc 540
gacggctcct ccaacatcga cgccgccgtc tccaccggct ccatcttcgg catctacagc 600
cccaacgacg agtgcctagc cgacatcgcc gacgaccaaa atcttgacca ggtggagcag 660
aggtgcatcg tgagcgtgtg ccagccgggg agcaacctgc tcgccgccgg ctactgcatg 720
tactcgagct cggtcatctt cgtgctcacc atcgggacag ggtgtacgt gttcacgctg 780
gacccgatgt acggcgagtt cgtgctgacg caggagaagg tgcagatccc caaggcaggc 840
aagatctatg ccttcaacga gggcaactac gcgctctggg acgacaagct caagagctac 900
atggacagcc tcaaggagcc cgggccgtcc gggaagccat actccgcgcg ctacatcggc 960
agcctcgtcg gcgacttcca ccgcacgctg ctctacggcg gcatctacgg ctaccccagg 1020
gaccagaaga gcaagaacgg caagctgcgg ctgctgtacg agtgcgcgcc gatgagcttc 1080
atcgtcgagc aggccggcgg caagggctcc gatggccacc agaggatact tgacatcatg 1140
cctacggaga tccatcagag agtgccgctg tacatcggga gcgtggagga agtggagaag 1200

gtcgagaagt tcttggcttg a 1221

<210> 175
<211> 406
<212> PRT
<213> Oryza sativa

<400> 175

Met Ala Ala Ala Ala Thr Thr Ser Ser His Leu Leu Leu Leu Ser Arg
1 5 10 15

Gln Gln Ala Ala Ala Ser Leu Gln Cys Gly Leu Ser Phe Arg Arg Gln
20 25 30

Pro Gly Arg Leu Ala Gly Gly Ser Ser Ala Pro Ser Val Arg Cys Met
35 40 45

Ala Ala Val Asp Thr Ala Ser Ala Pro Ala Ala Thr Glu Ala Ser Lys
50 55 60

Lys Ser Ser Tyr Glu Ile Thr Thr Leu Thr Thr Trp Leu Leu Lys Gln
65 70 75 80

Glu Gln Ala Gly Thr Ile Asp Gly Glu Met Thr Ile Val Leu Ala Ser
85 90 95

Ile Ser Thr Ala Cys Lys Gln Ile Ala Ser Leu Val Gln Arg Ala Pro
100 105 110

Ile Ser Asn Leu Thr Gly Val Gln Gly Ala Val Asn Val Gln Gly Glu
115 120 125

Asp Gln Lys Lys Leu Asp Val Val Ser Asn Glu Val Phe Ser Asn Cys
130 135 140

Leu Lys Ser Ser Gly Arg Thr Gly Val Ile Ala Ser Glu Glu Glu Asp
145 150 155 160

Val Pro Val Ala Val Glu Glu Ser Tyr Ser Gly Asn Tyr Ile Val Val
165 170 175

Phe Asp Pro Leu Asp Gly Ser Ser Asn Ile Asp Ala Ala Val Ser Thr
180 185 190

Gly Ser Ile Phe Gly Ile Tyr Ser Pro Asn Asp Glu Cys Leu Ala Asp
195 200 205

Ile Ala Asp Asp Gln Asn Leu Asp Gln Val Glu Gln Arg Cys Ile Val
210 215 220

Ser Val Cys Gln Pro Gly Ser Asn Leu Leu Ala Ala Gly Tyr Cys Met
225 230 235 240

Tyr Ser Ser Ser Val Ile Phe Val Leu Thr Ile Gly Thr Gly Val Tyr
245 250 255

Val Phe Thr Leu Asp Pro Met Tyr Gly Glu Phe Val Leu Thr Gln Glu
260 265 270

Lys Val Gln Ile Pro Lys Ala Gly Lys Ile Tyr Ala Phe Asn Glu Gly
275 280 285

Asn Tyr Ala Leu Trp Asp Asp Lys Leu Lys Ser Tyr Met Asp Ser Leu
290 295 300

Lys Glu Pro Gly Pro Ser Gly Lys Pro Tyr Ser Ala Arg Tyr Ile Gly
305 310 315 320

Ser Leu Val Gly Asp Phe His Arg Thr Leu Leu Tyr Gly Gly Ile Tyr
325 330 335

Gly Tyr Pro Arg Asp Gln Lys Ser Lys Asn Gly Lys Leu Arg Leu Leu
340 345 350

Tyr Glu Cys Ala Pro Met Ser Phe Ile Val Glu Gln Ala Gly Gly Lys
355 360 365

Gly Ser Asp Gly His Gln Arg Ile Leu Asp Ile Met Pro Thr Glu Ile
370 375 380

His Gln Arg Val Pro Leu Tyr Ile Gly Ser Val Glu Glu Val Glu Lys
385 390 395 400

Val Glu Lys Phe Leu Ala
405

<210> 176
<211> 1143
<212> DNA
<213> Ostreococcus lucimarinus

<400> 176
atgtctgccg ccacctccgc ctgcgctcgc gcgatcgtcg cgacgaagaa gaccaccgcg 60

gcgcgccgcg cgggcaagtc ggcgacgcgg gcgacgccgc gatccgtcgc ggcgcgcgcg 120

gggggcgcgg gcacgccgtt cacgacgtgg atcttgcaac aagagatgga agaaaagatc 180

gacggcgaac tcgcggtcgt gttgtcgagc atcggcttgg cgtgcaagca aatcgcgagc 240

ttggtgcaac gcgccgggct tcaaggcatg acgggtttgg cgggcgagac gaacgtgcaa 300

```
ggtgaagacc aaaagaagct cgacgtcatc tccaacgatg tgttctgcga tgtcttgcgt    360
caaaccggcc gcacgggcgt gattgcgtcc gaggaagaag acgtgccggt cgccgtcgaa    420
gaaaccttcg gcggtaacta cgtcgtcgtc ttcgatccgc tcgatggctc ttccaacatc    480
gacgccgccg tttccacggg ttccatctgg ggcatctacg agtccgactc cacgtgcatc    540
ccggattttg gcaccgaaga tgcggccaag attgaagaga agtgcgtcat gaacgtgtgc    600
caaccgggga acaacttgtt gtgcgcgggc tactgcatgt actcctcttc caccatcctc    660
gtcttgaccc tcggtgaggg tgtgtacggt ttcaccctcg acccgaccgt cggcgagttc    720
atcatgtccc acgacaacat caaggttccg gaatctggta agatttactc cttcaacgaa    780
ggcaactacg acatgtggac tccgggcttg aagaagtaca tggactccct caagactggc    840
ggcgcggaac aaggcaccaa gccgtacagc gcccgttaca tcggttccct cgtcggtgac    900
ttccacagaa ccatcttgta cggaggcatc tacggctacc cgggcgactc caagaacccg    960
aacggtaagc ttcgcctctt gtacgagtgc gcgccgatgt ccttcatcgc cgaacaagcc   1020
ggcggtatgg gttccaccgg taaggagcgc gtccttgacg ttgtcccgga aaagtttcac   1080
caacgtgtgc cgttcttcac cggctccaag aaggaagtgc agtacttgga atccttcatg   1140
tag                                                                 1143
```

<210> 177
<211> 380
<212> PRT
<213> Ostreococcus lucimarinus

<400> 177

```
Met Ser Ala Ala Thr Ser Ala Cys Ala Arg Ala Ile Val Ala Thr Lys
1               5                   10                  15

Lys Thr Thr Ala Ala Arg Arg Ala Gly Lys Ser Ala Thr Arg Ala Thr
            20                  25                  30

Pro Arg Ser Val Ala Ala Arg Ala Gly Gly Ala Gly Thr Pro Phe Thr
        35                  40                  45

Thr Trp Ile Leu Gln Gln Glu Met Glu Glu Lys Ile Asp Gly Glu Leu
    50                  55                  60

Ala Val Val Leu Ser Ser Ile Gly Leu Ala Cys Lys Gln Ile Ala Ser
65                  70                  75                  80

Leu Val Gln Arg Ala Gly Leu Gln Gly Met Thr Gly Leu Ala Gly Glu
                85                  90                  95

Thr Asn Val Gln Gly Glu Asp Gln Lys Lys Leu Asp Val Ile Ser Asn
            100                 105                 110
```

Asp Val Phe Cys Asp Val Leu Arg Gln Thr Gly Arg Thr Gly Val Ile
115 120 125

Ala Ser Glu Glu Glu Asp Val Pro Val Ala Val Glu Glu Thr Phe Gly
130 135 140

Gly Asn Tyr Val Val Val Phe Asp Pro Leu Asp Gly Ser Ser Asn Ile
145 150 155 160

Asp Ala Ala Val Ser Thr Gly Ser Ile Trp Gly Ile Tyr Glu Ser Asp
165 170 175

Ser Thr Cys Ile Pro Asp Phe Gly Thr Glu Asp Ala Ala Lys Ile Glu
180 185 190

Glu Lys Cys Val Met Asn Val Cys Gln Pro Gly Asn Asn Leu Leu Cys
195 200 205

Ala Gly Tyr Cys Met Tyr Ser Ser Ser Thr Ile Leu Val Leu Thr Leu
210 215 220

Gly Glu Gly Val Tyr Gly Phe Thr Leu Asp Pro Thr Val Gly Glu Phe
225 230 235 240

Ile Met Ser His Asp Asn Ile Lys Val Pro Glu Ser Gly Lys Ile Tyr
245 250 255

Ser Phe Asn Glu Gly Asn Tyr Asp Met Trp Thr Pro Gly Leu Lys Lys
260 265 270

Tyr Met Asp Ser Leu Lys Thr Gly Gly Ala Glu Gln Gly Thr Lys Pro
275 280 285

Tyr Ser Ala Arg Tyr Ile Gly Ser Leu Val Gly Asp Phe His Arg Thr
290 295 300

Ile Leu Tyr Gly Gly Ile Tyr Gly Tyr Pro Gly Asp Ser Lys Asn Pro
305 310 315 320

Asn Gly Lys Leu Arg Leu Leu Tyr Glu Cys Ala Pro Met Ser Phe Ile
325 330 335

Ala Glu Gln Ala Gly Gly Met Gly Ser Thr Gly Lys Glu Arg Val Leu
340 345 350

Asp Val Val Pro Glu Lys Phe His Gln Arg Val Pro Phe Phe Thr Gly
355 360 365

Ser Lys Lys Glu Val Gln Tyr Leu Glu Ser Phe Met
370 375 380

<210> 178
<211> 1134
<212> DNA
<213> Ostreococcus tauri

<400> 178
atgtctgcgt gtatttctgc ctctgtgacg cgcgcgcgaa cggcgcgcgc gcccgcggct 60

cgccgcgcgg cgagtaaggc gcgggcgtcg ccgcgcgccg tcgcggcgcg cgcggggggc 120

gttggtacgc cgtacacgac gtggattctc cagcaagaga tgcaagagaa cattgatggt 180

gaattggcgg tggttttgtc ctccatcggt ctcgcgtgca agcaaatcgc gagcctcgtc 240

cagcgcgcgg gtctcgcggg catgactggt ttggcgggcg agcaaaacgt gcaaggcgag 300

gaccagaaga agctcgacgt catctccaac gatgttttct gcaacgtatt gcgccaatcc 360

ggccgcacgg gcgtcatcgc ctccgaagaa gaagacgttc cggtcgccgt cgaggaaacg 420

tacggcggta actacgtcgt cgtcttcgat ccgcttgatg gttcctccaa tatcgacgcc 480

gccgtctcca cgggatccat ctggggtatc tacgagtccg actcctcgtg tattcccgac 540

ttcggctccg atgactccgc caaggttgaa gagaagtgcg tcatgaacgt ttgccaaccg 600

ggcagcaact tgctctgcgc gggttactgc atgtactcct cgtccaccat cctcgtcatc 660

accatcggcc aaggcgtgtt cggttttacc ctcgacccga ccgtcggtga gttcatcatg 720

tcccacgaga acatcaaggt tccggactct ggcaagattt actctttcaa cgaaggcaac 780

tacgccatgt ggtccgacgg tttgaagaag tacatggact ccctcaagac gggcggcaag 840

gacggtggca agccgtacag cgcccgctac atcggttcgc tcgtcggtga cttccaccgc 900

acgatccttt acggaggcat ctacggttac ccgggtgacg cgaagaaccc gaacggtaag 960

ctccgcctcc tgtacgagtg cgcgccgatg tccatgatcg ctgaacaagc cggtggtaag 1020

ggctccaccg gtgtcgcgcg tgtcctggac atcgttccgg aaaaggttca ccagcgcgtg 1080

ccgttcttcg ttggctccaa gaacgaggtt gcctacttgg agtccttcat gtga 1134

<210> 179
<211> 377
<212> PRT
<213> Ostreococcus tauri

<400> 179

Met Ser Ala Cys Ile Ser Ala Ser Val Thr Arg Ala Arg Thr Ala Arg
1 5 10 15

Ala Pro Ala Ala Arg Arg Ala Ala Ser Lys Ala Arg Ala Ser Pro Arg
20 25 30

Ala Val Ala Ala Arg Ala Gly Gly Val Gly Thr Pro Tyr Thr Thr Trp

35 40 45

Ile Leu Gln Gln Glu Met Gln Glu Asn Ile Asp Gly Glu Leu Ala Val
50 55 60

Val Leu Ser Ser Ile Gly Leu Ala Cys Lys Gln Ile Ala Ser Leu Val
65 70 75 80

Gln Arg Ala Gly Leu Ala Gly Met Thr Gly Leu Ala Gly Glu Gln Asn
85 90 95

Val Gln Gly Glu Asp Gln Lys Lys Leu Asp Val Ile Ser Asn Asp Val
100 105 110

Phe Cys Asn Val Leu Arg Gln Ser Gly Arg Thr Gly Val Ile Ala Ser
115 120 125

Glu Glu Glu Asp Val Pro Val Ala Val Glu Glu Thr Tyr Gly Gly Asn
130 135 140

Tyr Val Val Val Phe Asp Pro Leu Asp Gly Ser Ser Asn Ile Asp Ala
145 150 155 160

Ala Val Ser Thr Gly Ser Ile Trp Gly Ile Tyr Glu Ser Asp Ser Ser
165 170 175

Cys Ile Pro Asp Phe Gly Ser Asp Asp Ser Ala Lys Val Glu Glu Lys
180 185 190

Cys Val Met Asn Val Cys Gln Pro Gly Ser Asn Leu Leu Cys Ala Gly
195 200 205

Tyr Cys Met Tyr Ser Ser Ser Thr Ile Leu Val Ile Thr Ile Gly Gln
210 215 220

Gly Val Phe Gly Phe Thr Leu Asp Pro Thr Val Gly Glu Phe Ile Met
225 230 235 240

Ser His Glu Asn Ile Lys Val Pro Asp Ser Gly Lys Ile Tyr Ser Phe
245 250 255

Asn Glu Gly Asn Tyr Ala Met Trp Ser Asp Gly Leu Lys Lys Tyr Met
260 265 270

Asp Ser Leu Lys Thr Gly Gly Lys Asp Gly Gly Lys Pro Tyr Ser Ala
275 280 285

Arg Tyr Ile Gly Ser Leu Val Gly Asp Phe His Arg Thr Ile Leu Tyr
290 295 300

Gly Gly Ile Tyr Gly Tyr Pro Gly Asp Ala Lys Asn Pro Asn Gly Lys
305 310 315 320

Leu Arg Leu Leu Tyr Glu Cys Ala Pro Met Ser Met Ile Ala Glu Gln
325 330 335

Ala Gly Gly Lys Gly Ser Thr Gly Val Ala Arg Val Leu Asp Ile Val
340 345 350

Pro Glu Lys Val His Gln Arg Val Pro Phe Phe Val Gly Ser Lys Asn
355 360 365

Glu Val Ala Tyr Leu Glu Ser Phe Met
370 375

<210> 180
<211> 1254
<212> DNA
<213> Phaeodactylum tricornutum

<400> 180
atgtttattt tgaagtcccc ggcgctttgg ttgcttcttt acccagttgt tgcttttacg 60

gcggcgaggg cgaactcgat tcgtccagcg gccgcgttat ctgtcttcga tctgtcgagc 120

gtggaggcag taccgtctag aaagaccaag gccccaattt tcgatgaagt ttgcgacaca 180

actggagtca ctctcaaacg cttcatgacc gaggtttctc tactgaatcc tgaaatcgaa 240

gagctgacga cgctgtttgg tgcgattgaa accgcttgca aagccattgc aaatcttgtc 300

aagcgatcgc cgcttccctc cagtgacacg ttgggtcttc agggagaaat caacgttcaa 360

ggcgaagacc aaaagaaatt agatgtgatc gccaacgata ttttgaagcg agcactccgc 420

ttcacgggcc gcctcggagt cttagcctcg gaagaagagg atactcccgt cgatttgatg 480

ccaagggatc ctagtaccaa aaaagttcta atcgatgagg gagaaaagta tgtcgctgtc 540

ttcgatccgc tcgatggtag ctcaaacgtt gatgcaggca taccgacagg cacaataatt 600

gggatatacg agcacgacga aacttgcaag attgatcctg atgctttgga agaggatcgg 660

accaaacaag aaaacctatg cctcgcaaat actctgcagc ccggcaccaa cttggtagca 720

gcggcgtact gtttatattc ttcgtcaaca tttttggtgt tgacgctggg agctggaaca 780

tatggattca cgctagatga gactatcggt gaatttgtct tgagccatcc aaacattaag 840

attcctgaat gctcatccat tatgtcgttc aacgaggcaa atactcccag ctgggatcgt 900

ccgcttcaag acactttcgc aaagtggagg acagggacag gaaagagcgg caagaaattt 960

tcaagtcgct acattggttc tatggtaggg gatgtccatc ggacgctcct gtacggagga 1020

gtttttgggt atcctggcga caaaaagaat cccaacggga agctacgcct gctttatgaa 1080

ggagctccaa tgtcattcat catggaacag gcgggtggat tgtcgaccac tggcacgcag 1140

cgtgtcatgg aaatctcccc agatacggtc catcaacgtg tgccgattat catgggatcc 1200

agacaagatg tcgaagaggt catggacgcc tataaaaact ttggcatcga ataa 1254

<210> 181
<211> 417
<212> PRT
<213> Phaeodactylum tricornutum

<400> 181

Met Phe Ile Leu Lys Ser Pro Ala Leu Trp Leu Leu Leu Tyr Pro Val
1 5 10 15

Val Ala Phe Thr Ala Ala Arg Ala Asn Ser Ile Arg Pro Ala Ala Ala
20 25 30

Leu Ser Val Phe Asp Leu Ser Ser Val Glu Ala Val Pro Ser Arg Lys
35 40 45

Thr Lys Ala Pro Ile Phe Asp Glu Val Cys Asp Thr Thr Gly Val Thr
50 55 60

Leu Lys Arg Phe Met Thr Glu Val Ser Leu Leu Asn Pro Glu Ile Glu
65 70 75 80

Glu Leu Thr Thr Leu Phe Gly Ala Ile Glu Thr Ala Cys Lys Ala Ile
85 90 95

Ala Asn Leu Val Lys Arg Ser Pro Leu Pro Ser Ser Asp Thr Leu Gly
100 105 110

Leu Gln Gly Glu Ile Asn Val Gln Gly Glu Asp Gln Lys Lys Leu Asp
115 120 125

Val Ile Ala Asn Asp Ile Leu Lys Arg Ala Leu Arg Phe Thr Gly Arg
130 135 140

Leu Gly Val Leu Ala Ser Glu Glu Glu Asp Thr Pro Val Asp Leu Met
145 150 155 160

Pro Arg Asp Pro Ser Thr Lys Lys Val Leu Ile Asp Glu Gly Glu Lys
165 170 175

Tyr Val Ala Val Phe Asp Pro Leu Asp Gly Ser Ser Asn Val Asp Ala
180 185 190

Gly Ile Pro Thr Gly Thr Ile Ile Gly Ile Tyr Glu His Asp Glu Thr
195 200 205

Cys Lys Ile Asp Pro Asp Ala Leu Glu Glu Asp Arg Thr Lys Gln Glu
    210                 215                 220

Asn Leu Cys Leu Ala Asn Thr Leu Gln Pro Gly Thr Asn Leu Val Ala
225                 230                 235                 240

Ala Ala Tyr Cys Leu Tyr Ser Ser Ser Thr Phe Leu Val Leu Thr Leu
                245                 250                 255

Gly Ala Gly Thr Tyr Gly Phe Thr Leu Asp Glu Thr Ile Gly Glu Phe
            260                 265                 270

Val Leu Ser His Pro Asn Ile Lys Ile Pro Glu Cys Ser Ser Ile Met
        275                 280                 285

Ser Phe Asn Glu Ala Asn Thr Pro Ser Trp Asp Arg Pro Leu Gln Asp
    290                 295                 300

Thr Phe Ala Lys Trp Arg Thr Gly Thr Gly Lys Ser Gly Lys Lys Phe
305                 310                 315                 320

Ser Ser Arg Tyr Ile Gly Ser Met Val Gly Asp Val His Arg Thr Leu
                325                 330                 335

Leu Tyr Gly Gly Val Phe Gly Tyr Pro Gly Asp Lys Lys Asn Pro Asn
            340                 345                 350

Gly Lys Leu Arg Leu Leu Tyr Glu Gly Ala Pro Met Ser Phe Ile Met
        355                 360                 365

Glu Gln Ala Gly Gly Leu Ser Thr Thr Gly Thr Gln Arg Val Met Glu
    370                 375                 380

Ile Ser Pro Asp Thr Val His Gln Arg Val Pro Ile Ile Met Gly Ser
385                 390                 395                 400

Arg Gln Asp Val Glu Glu Val Met Asp Ala Tyr Lys Asn Phe Gly Ile
                405                 410                 415

Glu

<210> 182
<211> 1233
<212> DNA
<213> Pisum sativa

<400> 182
atggttgcaa tggcagcagc aacagcctca tctcagttaa tattctcaaa accttactct 60

ccttcacgtc tttgcccctt ccaactctgt gtctttgatg caaaatcagt gttatcaagt 120

```
tcaaggagaa agcatgtgaa tggctctggt gttagatgta tggctgtgaa ggaagcaact    180

agtgagacaa agaaaagaag tggatatgag attataacac tgactagttg gttgttgcag    240

caagaacaaa aagggattat tgatgcagaa cttactattg tactttctag tatttctatg    300

gcatgtaaac aaattgcttc tttggttcaa agagccaata tttctaacct cactggtact    360

caaggtgctg taaatattca aggggaagac cagaaaaaac ttgatgttat ctcaaatgag    420

gtattctcaa attgcttgag gtcaagtggg aggacaggga taattgcgtc ggaggaagag    480

gatgtcgcgg tggcagtaga agagagttat tcaggaaact acattgttgt atttgatcca    540

cttgatggtt catccaatct tgatgctgca gtctcaaccg gttccatttt cgggatttac    600

agccccaatg acgagtgtct tcctgatttt ggtgatgact ctgatgacaa cacacttggc    660

acagaagaac aaaggtgcat tgtgaatgtg tgtcaaccag gaagcaacct tctagcagct    720

ggctactgca tgtattctag ttcagtagct tttgttctta ccataggcaa aggagtgttt    780

gtattcacat tagatccatt gtacggagaa ttcgttttga ctcaagagaa tctccaaata    840

ccgaaatcag ggaaatcta ttctttcaat gaagggaatt acaagttgtg ggatgaaaac     900

ttgaagaaat atattgatga tcttaaggaa ccaggtccta gtggcaagcc ttattcagca    960

aggtatattg gtagtttggt tggtgatttt cacaggacac tgttatatgg tggcatttat   1020

ggataccta gggacaagaa aagtaagaat gggaagctta ggcttttata tgaatgtgct    1080

ccaatgagct tcattgttga acaggctggt ggaaaaggtt cagatggtca tcaaagagta   1140

cttgacattc aacccacaga aattcatcaa cgtgttccac tttacattgg gagcacagaa   1200

gaggtggaga aggttgaaaa gtacttagct taa                                1233
```

<210> 183
<211> 410
<212> PRT
<213> Pisum sativa

<400> 183

```
Met Val Ala Met Ala Ala Ala Thr Ala Ser Ser Gln Leu Ile Phe Ser
1               5                   10                  15

Lys Pro Tyr Ser Pro Ser Arg Leu Cys Pro Phe Gln Leu Cys Val Phe
            20                  25                  30

Asp Ala Lys Ser Val Leu Ser Ser Ser Arg Arg Lys His Val Asn Gly
        35                  40                  45

Ser Gly Val Arg Cys Met Ala Val Lys Glu Ala Thr Ser Glu Thr Lys
    50                  55                  60

Lys Arg Ser Gly Tyr Glu Ile Ile Thr Leu Thr Ser Trp Leu Leu Gln
65                  70                  75                  80
```

```
Gln Glu Gln Lys Gly Ile Ile Asp Ala Glu Leu Thr Ile Val Leu Ser
                85                  90                  95

Ser Ile Ser Met Ala Cys Lys Gln Ile Ala Ser Leu Val Gln Arg Ala
            100                 105                 110

Asn Ile Ser Asn Leu Thr Gly Thr Gln Gly Ala Val Asn Ile Gln Gly
        115                 120                 125

Glu Asp Gln Lys Lys Leu Asp Val Ile Ser Asn Glu Val Phe Ser Asn
    130                 135                 140

Cys Leu Arg Ser Ser Gly Arg Thr Gly Ile Ile Ala Ser Glu Glu Glu
145                 150                 155                 160

Asp Val Ala Val Ala Val Glu Glu Ser Tyr Ser Gly Asn Tyr Ile Val
                165                 170                 175

Val Phe Asp Pro Leu Asp Gly Ser Ser Asn Leu Asp Ala Ala Val Ser
            180                 185                 190

Thr Gly Ser Ile Phe Gly Ile Tyr Ser Pro Asn Asp Glu Cys Leu Pro
        195                 200                 205

Asp Phe Gly Asp Asp Ser Asp Asp Asn Thr Leu Gly Thr Glu Glu Gln
    210                 215                 220

Arg Cys Ile Val Asn Val Cys Gln Pro Gly Ser Asn Leu Leu Ala Ala
225                 230                 235                 240

Gly Tyr Cys Met Tyr Ser Ser Ser Val Ala Phe Val Leu Thr Ile Gly
                245                 250                 255

Lys Gly Val Phe Val Phe Thr Leu Asp Pro Leu Tyr Gly Glu Phe Val
            260                 265                 270

Leu Thr Gln Glu Asn Leu Gln Ile Pro Lys Ser Gly Glu Ile Tyr Ser
        275                 280                 285

Phe Asn Glu Gly Asn Tyr Lys Leu Trp Asp Glu Asn Leu Lys Lys Tyr
    290                 295                 300

Ile Asp Asp Leu Lys Glu Pro Gly Pro Ser Gly Lys Pro Tyr Ser Ala
305                 310                 315                 320

Arg Tyr Ile Gly Ser Leu Val Gly Asp Phe His Arg Thr Leu Leu Tyr
                325                 330                 335
```

```
Gly Gly Ile Tyr Gly Tyr Pro Arg Asp Lys Lys Ser Lys Asn Gly Lys
            340                 345                 350

Leu Arg Leu Leu Tyr Glu Cys Ala Pro Met Ser Phe Ile Val Glu Gln
        355                 360                 365

Ala Gly Gly Lys Gly Ser Asp Gly His Gln Arg Val Leu Asp Ile Gln
    370                 375                 380

Pro Thr Glu Ile His Gln Arg Val Pro Leu Tyr Ile Gly Ser Thr Glu
385                 390                 395                 400

Glu Val Glu Lys Val Glu Lys Tyr Leu Ala
                405                 410
```

<210> 184
<211> 1242
<212> DNA
<213> Poncirus trifoliata

<400> 184

```
atggttgcag cagcagcgac aacatcatca cagcttctct tttcaagctc tcactctttc     60
tctcggctct ctccttacca aatatgtgtc ttcgattcca aagcactcgt gtcgtcatgt    120
cccagcaacg ttatgaagag aagacatgtt ggtgttgctg ctggggttcg gtgcatggct    180
gttgggacaa catcggaggt tgcaaccaag aagagaagtt cgtatgagat tgaaacgctg    240
acaaactggc tgttgaagca agaacagtct ggcgttattg atgctgagct cactattgtg    300
ctttccagca tttcaacggc gtgcaagcag attgcttctt tggtgcaaag agctggcatt    360
tccaacttga ctggaattca gggtgctgtc aatgttcaag gcgaggacca gaagaagctc    420
gacgtcgttt caaatgaggt gttctcaaac tgtttgagat caagtgggcg aactgggatt    480
atagcatcag aggaagagga tgtaccagtg gcggtagaag agagctactc tggaaactat    540
attgtagttt ttgatccact tgatggatca tccaacattg atgctgcagt gtctactgga    600
tccatctttg gcatatacag cccaaatgat gagtgtcttg ccgatattgg tgatgattct    660
actctgggca atactgaaca aagatgtgta gtgaacgtgt gccagccagg aagcaacctt    720
cttgctgcag gctattgcat gtattcaagc tctgtaatct ttgtgataac tttaggcaac    780
ggagtctttg cattcaccct ggatcccatg tatggagaat ttgttttgac acaagagaac    840
attcagatac ccaaaaccgg aaagatctat gcttttaatg aaggcaacta ccagctttgg    900
gatgacaagt tgaagaagta cattgacgat cttaaggatc caggacccag cggcaagccc    960
tattctgcta ggtacattgg cagcttggtt ggtgatttcc atcgaactct gctctacggc   1020
ggcatttatg gctatccaag agacaagaag agcaagaatg gaaagctgag gctcttgtat   1080
gaatgtgcac caatgagctt catagtggaa caagcaggag gcaaaggatc tgacggccat   1140
```

caaagagtac ttgacattca acctactgag attcaccagc gtattccttt aaagattgga 1200

agccaggagg aagtggagaa actggagaag tatttggcct aa 1242

<210> 185
<211> 413
<212> PRT
<213> Poncirus trifoliata

<400> 185

Met Val Ala Ala Ala Ala Thr Thr Ser Ser Gln Leu Leu Phe Ser Ser
1 5 10 15

Ser His Ser Phe Ser Arg Leu Ser Pro Tyr Gln Ile Cys Val Phe Asp
20 25 30

Ser Lys Ala Leu Val Ser Ser Cys Pro Ser Asn Val Met Lys Arg Arg
35 40 45

His Val Gly Val Ala Ala Gly Val Arg Cys Met Ala Val Gly Thr Thr
50 55 60

Ser Glu Val Ala Thr Lys Lys Arg Ser Ser Tyr Glu Ile Glu Thr Leu
65 70 75 80

Thr Asn Trp Leu Leu Lys Gln Glu Gln Ser Gly Val Ile Asp Ala Glu
85 90 95

Leu Thr Ile Val Leu Ser Ser Ile Ser Thr Ala Cys Lys Gln Ile Ala
100 105 110

Ser Leu Val Gln Arg Ala Gly Ile Ser Asn Leu Thr Gly Ile Gln Gly
115 120 125

Ala Val Asn Val Gln Gly Glu Asp Gln Lys Lys Leu Asp Val Val Ser
130 135 140

Asn Glu Val Phe Ser Asn Cys Leu Arg Ser Ser Gly Arg Thr Gly Ile
145 150 155 160

Ile Ala Ser Glu Glu Glu Asp Val Pro Val Ala Val Glu Glu Ser Tyr
165 170 175

Ser Gly Asn Tyr Ile Val Val Phe Asp Pro Leu Asp Gly Ser Ser Asn
180 185 190

Ile Asp Ala Ala Val Ser Thr Gly Ser Ile Phe Gly Ile Tyr Ser Pro
195 200 205

Asn Asp Glu Cys Leu Ala Asp Ile Gly Asp Asp Ser Thr Leu Gly Asn

210 215 220

Thr Glu Gln Arg Cys Val Val Asn Val Cys Gln Pro Gly Ser Asn Leu
225 230 235 240

Leu Ala Ala Gly Tyr Cys Met Tyr Ser Ser Ser Val Ile Phe Val Ile
245 250 255

Thr Leu Gly Asn Gly Val Phe Ala Phe Thr Leu Asp Pro Met Tyr Gly
260 265 270

Glu Phe Val Leu Thr Gln Glu Asn Ile Gln Ile Pro Lys Thr Gly Lys
275 280 285

Ile Tyr Ala Phe Asn Glu Gly Asn Tyr Gln Leu Trp Asp Asp Lys Leu
290 295 300

Lys Lys Tyr Ile Asp Asp Leu Lys Asp Pro Gly Pro Ser Gly Lys Pro
305 310 315 320

Tyr Ser Ala Arg Tyr Ile Gly Ser Leu Val Gly Asp Phe His Arg Thr
325 330 335

Leu Leu Tyr Gly Gly Ile Tyr Gly Tyr Pro Arg Asp Lys Lys Ser Lys
340 345 350

Asn Gly Lys Leu Arg Leu Leu Tyr Glu Cys Ala Pro Met Ser Phe Ile
355 360 365

Val Glu Gln Ala Gly Gly Lys Gly Ser Asp Gly His Gln Arg Val Leu
370 375 380

Asp Ile Gln Pro Thr Glu Ile His Gln Arg Ile Pro Leu Lys Ile Gly
385 390 395 400

Ser Gln Glu Glu Val Glu Lys Leu Glu Lys Tyr Leu Ala
405 410

<210> 186
<211> 1245
<212> DNA
<213> Populus tremuloides

<400> 186
atggttgcac aagcagcagc aataacaact tcatcatcac atcttctctt ctcaacctca 60

cgctcactgt ctcgcccatc tccttcccag ttatgtgtct ttgactcaaa aacacttgtg 120

tcatacccca acagcactag tacttacaag aaaaaacgtg gtggtgatgg gctcaagtgc 180

atggctgtga gcacagcctc ggatgctaaa acaaagaaga gtacgtttga gattcaaaca 240

```
ctgactggtt ggctgttgaa gcaagaacaa gctggtgtta ttgatgctga gctcactatt     300
gttatatcaa gcatttcaat ggcatgtaag cagattgctt ctttggtgca aagagctagc     360
atttctaact taactggagt tcaaggttct gttaacgttc aaggagaaga tcagaagaag     420
cttgacgtgg tctctaatga ggtgttctct agctgcttga gatcaagtgg gaggacagga     480
atcatagcat cagaggaaga ggacgtgcca gtggcagtgg aggagagtta ctctggaaac     540
tatatagtgg tttttgaccc actcgatgga tcatccaaca ttgatgctgc agtgtctact     600
ggttccatct ttggaatata cagccccaat gacgaatgcc tggccgatat tggagatgac     660
tccactcttg atcaaacgga acagaggtgt attgtgaatg tgtgccagcc aggaaataac     720
ctccttgttg ctggctactg catgtattca agctcagtga tttttgtgct aactattgga     780
aaaggcgtgt tctctttcag cttggatcca atgtatggag agtttgtttt aactcaagaa     840
aacatccaga taccaaaggc tggaaagatt tattcattta atgaaggaaa ctaccagttg     900
tgggatgaca agctgaagaa gtacattgat gaccttaaag accctggtcc gagtggcaag     960
ccctactccg ctagatacat tggaagcttg gtcggtgact tccaccggac gctgctgtac    1020
ggtggcattt atgggtaccc cagggacaag aagagcaaga atgggaagct gaggcttctg    1080
tatgagtgtg caccgatgag ctttatagtg gaacaagctg gtgggaaagg atcagacggg    1140
catcagagag tactggatat cactcctact gagatacacc agcgtgttcc gctttacata    1200
gggagcgtgg aggaagtgga gaaattggag aagtatttgg cttga                   1245
```

<210> 187
<211> 414
<212> PRT
<213> Populus tremuloides

<400> 187

```
Met Val Ala Gln Ala Ala Ala Ile Thr Thr Ser Ser Ser His Leu Leu
1               5                   10                  15

Phe Ser Thr Ser Arg Ser Leu Ser Arg Pro Ser Pro Ser Gln Leu Cys
            20                  25                  30

Val Phe Asp Ser Lys Thr Leu Val Ser Tyr Pro Asn Ser Thr Ser Thr
        35                  40                  45

Tyr Lys Lys Lys Arg Gly Gly Asp Gly Leu Lys Cys Met Ala Val Ser
    50                  55                  60

Thr Ala Ser Asp Ala Lys Thr Lys Lys Ser Thr Phe Glu Ile Gln Thr
65                  70                  75                  80

Leu Thr Gly Trp Leu Leu Lys Gln Glu Gln Ala Gly Val Ile Asp Ala
                85                  90                  95
```

```
Glu Leu Thr Ile Val Ile Ser Ser Ile Ser Met Ala Cys Lys Gln Ile
            100                 105                 110

Ala Ser Leu Val Gln Arg Ala Ser Ile Ser Asn Leu Thr Gly Val Gln
        115                 120                 125

Gly Ser Val Asn Val Gln Gly Glu Asp Gln Lys Lys Leu Asp Val Val
    130                 135                 140

Ser Asn Glu Val Phe Ser Ser Cys Leu Arg Ser Ser Gly Arg Thr Gly
145                 150                 155                 160

Ile Ile Ala Ser Glu Glu Glu Asp Val Pro Val Ala Val Glu Glu Ser
                165                 170                 175

Tyr Ser Gly Asn Tyr Ile Val Val Phe Asp Pro Leu Asp Gly Ser Ser
            180                 185                 190

Asn Ile Asp Ala Ala Val Ser Thr Gly Ser Ile Phe Gly Ile Tyr Ser
        195                 200                 205

Pro Asn Asp Glu Cys Leu Ala Asp Ile Gly Asp Asp Ser Thr Leu Asp
    210                 215                 220

Gln Thr Glu Gln Arg Cys Ile Val Asn Val Cys Gln Pro Gly Asn Asn
225                 230                 235                 240

Leu Leu Val Ala Gly Tyr Cys Met Tyr Ser Ser Ser Val Ile Phe Val
                245                 250                 255

Leu Thr Ile Gly Lys Gly Val Phe Ser Phe Ser Leu Asp Pro Met Tyr
            260                 265                 270

Gly Glu Phe Val Leu Thr Gln Glu Asn Ile Gln Ile Pro Lys Ala Gly
        275                 280                 285

Lys Ile Tyr Ser Phe Asn Glu Gly Asn Tyr Gln Leu Trp Asp Asp Lys
    290                 295                 300

Leu Lys Lys Tyr Ile Asp Asp Leu Lys Asp Pro Gly Pro Ser Gly Lys
305                 310                 315                 320

Pro Tyr Ser Ala Arg Tyr Ile Gly Ser Leu Val Gly Asp Phe His Arg
                325                 330                 335

Thr Leu Leu Tyr Gly Gly Ile Tyr Gly Tyr Pro Arg Asp Lys Lys Ser
            340                 345                 350
```

```
Lys Asn Gly Lys Leu Arg Leu Leu Tyr Glu Cys Ala Pro Met Ser Phe
        355                 360                 365

Ile Val Glu Gln Ala Gly Gly Lys Gly Ser Asp Gly His Gln Arg Val
    370                 375                 380

Leu Asp Ile Thr Pro Thr Glu Ile His Gln Arg Val Pro Leu Tyr Ile
385                 390                 395                 400

Gly Ser Val Glu Glu Val Glu Lys Leu Glu Lys Tyr Leu Ala
                405                 410


<210>  188
<211>  1227
<212>  DNA
<213>  Solanum tuberosum

<400>  188
atggcagcat cagcagccac agcaacagca acaacttcat atttaagtgc tctagacaaa      60

aagactccat ttttatttgc cttagacaaa aagactccat ttttatgccc aaaaagcagc     120

acgaagagaa ggtcatttaa tggaggagta aagtgcatgg caatagagac aacttcaggt     180

tttacagcaa ccaagaaaag gagtggctat gagctgcaaa ctttaacaag ttggctatta     240

agacaagaac aagctggagt gattgatgct gaacttacca tagttatttc aagtatttca     300

atggcttgta aacagattgc ttccttagtc caaagagctg gaatttctaa ccttactgga     360

gttcaaggtg ctgtcaatat tcaaggagaa gatcagaaga aacttgatgt tgtctctaat     420

gaggttttct caaattgtct aagatcaagt ggaaggactg ggattatagc atcagaagaa     480

gaggatgtac ctgtggcagt ggaagagagt tactcaggca actacattgt ggtgtttgat     540

cctcttgatg gatcatcaaa cattgatgct gctgtgtcta ccggttctat ctttggaata     600

tacaacccga atgatgagtg cctcgctgat catggagatg attccacgct tgacaatata     660

gagcagaagt gcattgtgaa tgtatgtcaa ccagggacaa accttcttgc agcaggatac     720

tgcatgtact caagctctgt gatattcgta ctcaccttgg gaaatggcgt tttttccttt     780

aacttggatc cgatgtacgg agaatttgtt ctgactcaag aaaatgtcca aataccaaag     840

tctggaaaga tctattcatt caatgaagga aactaccagc tctgggatga caagttgaag     900

aaatatatcg atgacttgaa ggaccctggc cctagtggca agccttactc tgcaaggtac     960

attggtagtt tggttggtga cttccataga actcttctat atggtggcat ttatggttat    1020

cctagagacc aaaagagcaa gaatggaaag ttgaggcttt tgtacgagtg tgctcccatg    1080

agcttcattg tggaacaagc tggtggtaaa ggatccgatg gtcaccaaag agttctcgat    1140

atccaaccaa ctgaggtaca tcaacgagtt ccattgtaca ttggaagcac agaagaagtt    1200

gaaaaattgg agaagtactt gtcttaa                                        1227
```

<210> 189
<211> 408
<212> PRT
<213> Solanum tuberosum

<400> 189

```
Met Ala Ala Ser Ala Ala Thr Ala Thr Ala Thr Thr Ser Tyr Leu Ser
1               5                   10                  15

Ala Leu Asp Lys Lys Thr Pro Phe Leu Phe Ala Leu Asp Lys Lys Thr
            20                  25                  30

Pro Phe Leu Cys Pro Lys Ser Ser Thr Lys Arg Arg Ser Phe Asn Gly
        35                  40                  45

Gly Val Lys Cys Met Ala Ile Glu Thr Thr Ser Gly Phe Thr Ala Thr
    50                  55                  60

Lys Lys Arg Ser Gly Tyr Glu Leu Gln Thr Leu Thr Ser Trp Leu Leu
65                  70                  75                  80

Arg Gln Glu Gln Ala Gly Val Ile Asp Ala Glu Leu Thr Ile Val Ile
                85                  90                  95

Ser Ser Ile Ser Met Ala Cys Lys Gln Ile Ala Ser Leu Val Gln Arg
            100                 105                 110

Ala Gly Ile Ser Asn Leu Thr Gly Val Gln Gly Ala Val Asn Ile Gln
        115                 120                 125

Gly Glu Asp Gln Lys Lys Leu Asp Val Val Ser Asn Glu Val Phe Ser
    130                 135                 140

Asn Cys Leu Arg Ser Ser Gly Arg Thr Gly Ile Ile Ala Ser Glu Glu
145                 150                 155                 160

Glu Asp Val Pro Val Ala Val Glu Glu Ser Tyr Ser Gly Asn Tyr Ile
                165                 170                 175

Val Val Phe Asp Pro Leu Asp Gly Ser Ser Asn Ile Asp Ala Ala Val
            180                 185                 190

Ser Thr Gly Ser Ile Phe Gly Ile Tyr Asn Pro Asn Asp Glu Cys Leu
        195                 200                 205

Ala Asp His Gly Asp Asp Ser Thr Leu Asp Asn Ile Glu Gln Lys Cys
    210                 215                 220

Ile Val Asn Val Cys Gln Pro Gly Thr Asn Leu Leu Ala Ala Gly Tyr
225                 230                 235                 240
```

Cys Met Tyr Ser Ser Ser Val Ile Phe Val Leu Thr Leu Gly Asn Gly
245 250 255

Val Phe Ser Phe Asn Leu Asp Pro Met Tyr Gly Glu Phe Val Leu Thr
260 265 270

Gln Glu Asn Val Gln Ile Pro Lys Ser Gly Lys Ile Tyr Ser Phe Asn
275 280 285

Glu Gly Asn Tyr Gln Leu Trp Asp Asp Lys Leu Lys Lys Tyr Ile Asp
290 295 300

Asp Leu Lys Asp Pro Gly Pro Ser Gly Lys Pro Tyr Ser Ala Arg Tyr
305 310 315 320

Ile Gly Ser Leu Val Gly Asp Phe His Arg Thr Leu Leu Tyr Gly Gly
325 330 335

Ile Tyr Gly Tyr Pro Arg Asp Gln Lys Ser Lys Asn Gly Lys Leu Arg
340 345 350

Leu Leu Tyr Glu Cys Ala Pro Met Ser Phe Ile Val Glu Gln Ala Gly
355 360 365

Gly Lys Gly Ser Asp Gly His Gln Arg Val Leu Asp Ile Gln Pro Thr
370 375 380

Glu Val His Gln Arg Val Pro Leu Tyr Ile Gly Ser Thr Glu Glu Val
385 390 395 400

Glu Lys Leu Glu Lys Tyr Leu Ser
405

<210> 190
<211> 1248
<212> DNA
<213> Spinacia oleracea

<400> 190
atggcatcaa taggaccagc aacaacaact gcagtaaaac tgcgtagctc aatcttcaat 60

cctcagtcat ctactctttc cccgtctcaa caatgcatta catttacaaa gtcccttcac 120

tcgtttccta ctgccacccg acataatgtg gcttccgggg ttcgttgtat ggcagccgta 180

ggagaggcgg ctacagaaac aaaggcaagg actagaagta agtacgaaat tgaaacacta 240

acaggctggc tgcttaaaca agaaatggca ggtgttattg atgctgaact taccatcgtt 300

ctttctagca tttcattggc ttgtaaacaa attgcttcct tggttcaacg agctggtatt 360

tctaacttga ctggaattca aggtgctgtc aatatccaag gagaggatca gaagaaactt 420

gatgttgtct ccaatgaggt gttttcgagc tgcttgagat cgagtggaag aacaggaata 480

atagcatcag aagaagagga tgtaccagtg gcagtggaag agagttactc tggaaactat 540

attgttgtgt ttgatccact tgatggttca tccaacattg atgcagctgt ctccactggt 600

tccatctttg gcatttatag ccctaacgat gagtgcattg ttgactctga tcacgacgat 660

gagtcacagc taagtgcaga agaacagagg tgtgtagtga atgtatgtca accaggggat 720

aacctattag cagcagggta ttgtatgtac tcaagctctg ttatcttcgt acttacaatt 780

ggtaaaggtg tgtatgcatt cacattagat ccaatgtatg gtgaattcgt actcacttca 840

gagaaaatcc aaatcccaaa agctgggaag atctattcat tcaatgaagg taactacaaa 900

atgtgggatg ataaattgaa gaagtacatg gatgatctta aagagccagg agagtcacag 960

aaaccgtact cgtctcgtta catagggagt ttagttgggg actttcatag aacactttta 1020

tatggtggga tttatggtta cccaagagat gcaaagagta agaatgggaa attgaggctt 1080

ttgtatgaat gtgcacctat gagttttatt gttgaacaag ctggtggtaa aggttctgat 1140

ggtcatcaaa gaattcttga cattcaaccc accgagatac atcaacgtgt gccactgtac 1200

atcgggagtg tggaggaagt agagaaatta gagaagtact tagcataa 1248

<210> 191
<211> 415
<212> PRT
<213> Spinacia oleracea

<400> 191

Met Ala Ser Ile Gly Pro Ala Thr Thr Thr Ala Val Lys Leu Arg Ser
1               5                   10                  15

Ser Ile Phe Asn Pro Gln Ser Ser Thr Leu Ser Pro Ser Gln Gln Cys
            20                  25                  30

Ile Thr Phe Thr Lys Ser Leu His Ser Phe Pro Thr Ala Thr Arg His
        35                  40                  45

Asn Val Ala Ser Gly Val Arg Cys Met Ala Ala Val Gly Glu Ala Ala
    50                  55                  60

Thr Glu Thr Lys Ala Arg Thr Arg Ser Lys Tyr Glu Ile Glu Thr Leu
65                  70                  75                  80

Thr Gly Trp Leu Leu Lys Gln Glu Met Ala Gly Val Ile Asp Ala Glu
                85                  90                  95

Leu Thr Ile Val Leu Ser Ser Ile Ser Leu Ala Cys Lys Gln Ile Ala
            100                 105                 110

Ser Leu Val Gln Arg Ala Gly Ile Ser Asn Leu Thr Gly Ile Gln Gly
115 120 125

Ala Val Asn Ile Gln Gly Glu Asp Gln Lys Lys Leu Asp Val Val Ser
130 135 140

Asn Glu Val Phe Ser Ser Cys Leu Arg Ser Ser Gly Arg Thr Gly Ile
145 150 155 160

Ile Ala Ser Glu Glu Glu Asp Val Pro Val Ala Val Glu Glu Ser Tyr
165 170 175

Ser Gly Asn Tyr Ile Val Val Phe Asp Pro Leu Asp Gly Ser Ser Asn
180 185 190

Ile Asp Ala Ala Val Ser Thr Gly Ser Ile Phe Gly Ile Tyr Ser Pro
195 200 205

Asn Asp Glu Cys Ile Val Asp Ser Asp His Asp Asp Glu Ser Gln Leu
210 215 220

Ser Ala Glu Glu Gln Arg Cys Val Val Asn Val Cys Gln Pro Gly Asp
225 230 235 240

Asn Leu Leu Ala Ala Gly Tyr Cys Met Tyr Ser Ser Ser Val Ile Phe
245 250 255

Val Leu Thr Ile Gly Lys Gly Val Tyr Ala Phe Thr Leu Asp Pro Met
260 265 270

Tyr Gly Glu Phe Val Leu Thr Ser Glu Lys Ile Gln Ile Pro Lys Ala
275 280 285

Gly Lys Ile Tyr Ser Phe Asn Glu Gly Asn Tyr Lys Met Trp Asp Asp
290 295 300

Lys Leu Lys Lys Tyr Met Asp Asp Leu Lys Glu Pro Gly Glu Ser Gln
305 310 315 320

Lys Pro Tyr Ser Ser Arg Tyr Ile Gly Ser Leu Val Gly Asp Phe His
325 330 335

Arg Thr Leu Leu Tyr Gly Gly Ile Tyr Gly Tyr Pro Arg Asp Ala Lys
340 345 350

Ser Lys Asn Gly Lys Leu Arg Leu Leu Tyr Glu Cys Ala Pro Met Ser
355 360 365

Phe Ile Val Glu Gln Ala Gly Gly Lys Gly Ser Asp Gly His Gln Arg

370 375 380

Ile Leu Asp Ile Gln Pro Thr Glu Ile His Gln Arg Val Pro Leu Tyr
385 390 395 400

Ile Gly Ser Val Glu Glu Val Glu Lys Leu Glu Lys Tyr Leu Ala
405 410 415

<210> 192
<211> 1230
<212> DNA
<213> Triticum aestivum

<400> 192
atggccgccg cgaccaccac cacctcccgc ccgcttctgc tgtcccgcca gcaggcggcg 60

gctagctccc tccaatgccg cctccccagg aggcccggaa gcagcctctt tgccggccag 120

ggccaggcgt cgactccgaa tgtgcggtgc atggcagtcg tggacacggc ctcggcgccg 180

gcgccggcgg cggctaggaa gaggagcagc tacgacatga tcacgctgac gacgtggctg 240

ctgaagcagg agcaggaggg ggtcatcgac aacgagatga ccatcgtgct gtccagcata 300

tccacggcgt gcaagcagat cgcctcgttg gtgcagcgcg cgcccatctc caacctcacc 360

ggcgtccagg gcgccaccaa cgtgcagggc gaggaccaga agaagctcga cgtcatctcc 420

aacgaggtgt tctcgaactg cctgaggtgg agtggccgca ccggcgtgat cgcatcggag 480

gaggaggacg tgccggtggc ggtggaggag agctactcgg gcaactacat cgtggtgttc 540

gacccgctcg acggctcctc caacatcgac gccgccgtct ccaccggctc catcttcggc 600

atctacagcc catccgacga gtgccacatt ggcgacgacg caacccttga cgaagtgacg 660

cagatgtgca tagtgaacgt gtgccagcca gggagcaacc tgctcgccgc cggctactgc 720

atgtactcga gctcggtcat cttcgtgctc accatcggca ccggggtgta cgtgttcacg 780

ctggacccga tgtacggcga gttcgtgctg acgcaggaga aggtgcagat cccaaagtcg 840

ggcaagatct actccttcaa cgagggcaac tacgcgctct gggacgacaa gctcaagaag 900

tacatggaca gcctcaagga gcccggcacc tccggcaagc cctactccgc gcgctacatc 960

ggcagcctcg tcggcgactt ccaccgcacc atgctctacg gcggcatcta cgggtacccc 1020

agcgaccaga agagcaagaa cggcaagctg cggctgctct acgagtgcgc gcccatgagc 1080

ttcatcgccg agcaggccgg cggcaaaggc tccgacggcc accagagggt actcgacatc 1140

atgcccacag cggtccatca gagagtgcct ctgtacgtcg ggagcgtgga ggaagtggag 1200

aaggtggaga aattcttgtc ttcagagtag 1230

<210> 193
<211> 409
<212> PRT
<213> Triticum aestivum

<400> 193

Met Ala Ala Ala Thr Thr Thr Thr Ser Arg Pro Leu Leu Leu Ser Arg
1 5 10 15

Gln Gln Ala Ala Ala Ser Ser Leu Gln Cys Arg Leu Pro Arg Arg Pro
20 25 30

Gly Ser Ser Leu Phe Ala Gly Gln Gly Gln Ala Ser Thr Pro Asn Val
35 40 45

Arg Cys Met Ala Val Val Asp Thr Ala Ser Ala Pro Ala Pro Ala Ala
50 55 60

Ala Arg Lys Arg Ser Ser Tyr Asp Met Ile Thr Leu Thr Thr Trp Leu
65 70 75 80

Leu Lys Gln Glu Gln Glu Gly Val Ile Asp Asn Glu Met Thr Ile Val
85 90 95

Leu Ser Ser Ile Ser Thr Ala Cys Lys Gln Ile Ala Ser Leu Val Gln
100 105 110

Arg Ala Pro Ile Ser Asn Leu Thr Gly Val Gln Gly Ala Thr Asn Val
115 120 125

Gln Gly Glu Asp Gln Lys Lys Leu Asp Val Ile Ser Asn Glu Val Phe
130 135 140

Ser Asn Cys Leu Arg Trp Ser Gly Arg Thr Gly Val Ile Ala Ser Glu
145 150 155 160

Glu Glu Asp Val Pro Val Ala Val Glu Glu Ser Tyr Ser Gly Asn Tyr
165 170 175

Ile Val Val Phe Asp Pro Leu Asp Gly Ser Ser Asn Ile Asp Ala Ala
180 185 190

Val Ser Thr Gly Ser Ile Phe Gly Ile Tyr Ser Pro Ser Asp Glu Cys
195 200 205

His Ile Gly Asp Asp Ala Thr Leu Asp Glu Val Thr Gln Met Cys Ile
210 215 220

Val Asn Val Cys Gln Pro Gly Ser Asn Leu Leu Ala Ala Gly Tyr Cys
225 230 235 240

Met Tyr Ser Ser Ser Val Ile Phe Val Leu Thr Ile Gly Thr Gly Val
245 250 255

Tyr Val Phe Thr Leu Asp Pro Met Tyr Gly Glu Phe Val Leu Thr Gln
260 265 270

Glu Lys Val Gln Ile Pro Lys Ser Gly Lys Ile Tyr Ser Phe Asn Glu
275 280 285

Gly Asn Tyr Ala Leu Trp Asp Asp Lys Leu Lys Lys Tyr Met Asp Ser
290 295 300

Leu Lys Glu Pro Gly Thr Ser Gly Lys Pro Tyr Ser Ala Arg Tyr Ile
305 310 315 320

Gly Ser Leu Val Gly Asp Phe His Arg Thr Met Leu Tyr Gly Gly Ile
325 330 335

Tyr Gly Tyr Pro Ser Asp Gln Lys Ser Lys Asn Gly Lys Leu Arg Leu
340 345 350

Leu Tyr Glu Cys Ala Pro Met Ser Phe Ile Ala Glu Gln Ala Gly Gly
355 360 365

Lys Gly Ser Asp Gly His Gln Arg Val Leu Asp Ile Met Pro Thr Ala
370 375 380

Val His Gln Arg Val Pro Leu Tyr Val Gly Ser Val Glu Glu Val Glu
385 390 395 400

Lys Val Glu Lys Phe Leu Ser Ser Glu
405

<210> 194
<211> 1242
<212> DNA
<213> Zea mays

<400> 194
atggccgccg ccgccaccac ctcctcatcc tcccacttgc tcctcctctc ccgccagcag 60

gcggcctccc tacgatgccg cctctccttc ctcggccagc ccgccagaag gtccggcagg 120

gtcacggccc aggcgccggc cgctaaggac gtgcggtgca tggcggccgt ggacactgcg 180

gcgtccgcgg cggcggcgga gacgagcccc aagtcgagca gctacgagat cgtgacgctc 240

acgacgtggc tgctgcagca ggagcggacc ggcgcgatcg acaacgagat gaccatcgtg 300

ctggccagca tatccacggc gtgcaagcag atcgccgcgc tggtgcagcg cgcgcccatc 360

tccaacctca cgggcgttca gggcgccgtc aacgtgcagg gcgaggacca gaagaagctc 420

gatgtcgtct ccaacgaggt gttctccaac tgcctcaagt cgagcgggcg caccggcgtg 480

atcgcctcgg aggaggagga cgtgcccgta gcggtggagc agagctactc cggcaactac 540

```
atcgtcgtgt tcgaccctct cgacggctcc tccaacatcg acgccgccgt ctccactggc     600

tccatcttcg gcatctacaa ccccaacgac gagtgcctcg ccgacgtcga cgacaacgac     660

acccttgatt cggtggagca gaggtgcatc gtgaacgtgt gccagccggg gagcaacctg     720

ctggccgccg gctactgcat gtactcgagc tcggtgatct tcgtgctcac cgtcggcacc     780

ggggtgtacg tgttcacgct ggaccccatg tacggcgagt tcgtgctgac gcaggagaag     840

gtgcagatcc ccaaggcggg caagatctac gccttcaacg agggcaacta cgcgctctgg     900

gacgacaagc tgaagctgta catggacagc ctcaaggagc ccggcgactc ggggaagccc     960

tactccgcgc ggtacatcgg cagcctcgtc ggcgacttcc accgcactct gctctacgga    1020

gggatctacg ggtaccccag ggacaagaag agcaagaacg gcaagctgcg gcttctctac    1080

gagtgcgccc ccatgagctt catcgtcgag caggccggtg gcaagggctc tgacggccac    1140

cagagaattc ttgacatcac acctacagag atccaccaaa gagtgcctct gtacattggg    1200

agcgtggagg aagtggacaa ggtggagaaa ttcctggctt ga                       1242
```

```
<210>  195
<211>  413
<212>  PRT
<213>  Zea mays

<400>  195

Met Ala Ala Ala Ala Thr Thr Ser Ser Ser Ser His Leu Leu Leu Leu
1               5                   10                  15


Ser Arg Gln Gln Ala Ala Ser Leu Arg Cys Arg Leu Ser Phe Leu Gly
            20                  25                  30


Gln Pro Ala Arg Arg Ser Gly Arg Val Thr Ala Gln Ala Pro Ala Ala
        35                  40                  45


Lys Asp Val Arg Cys Met Ala Ala Val Asp Thr Ala Ala Ser Ala Ala
    50                  55                  60


Ala Ala Glu Thr Ser Pro Lys Ser Ser Ser Tyr Glu Ile Val Thr Leu
65                  70                  75                  80


Thr Thr Trp Leu Leu Gln Gln Glu Arg Thr Gly Ala Ile Asp Asn Glu
                85                  90                  95


Met Thr Ile Val Leu Ala Ser Ile Ser Thr Ala Cys Lys Gln Ile Ala
            100                 105                 110


Ala Leu Val Gln Arg Ala Pro Ile Ser Asn Leu Thr Gly Val Gln Gly
        115                 120                 125


Ala Val Asn Val Gln Gly Glu Asp Gln Lys Lys Leu Asp Val Val Ser
```

130 135 140

Asn Glu Val Phe Ser Asn Cys Leu Lys Ser Ser Gly Arg Thr Gly Val
145 150 155 160

Ile Ala Ser Glu Glu Glu Asp Val Pro Val Ala Val Glu Gln Ser Tyr
165 170 175

Ser Gly Asn Tyr Ile Val Val Phe Asp Pro Leu Asp Gly Ser Ser Asn
180 185 190

Ile Asp Ala Ala Val Ser Thr Gly Ser Ile Phe Gly Ile Tyr Asn Pro
195 200 205

Asn Asp Glu Cys Leu Ala Asp Val Asp Asp Asn Asp Thr Leu Asp Ser
210 215 220

Val Glu Gln Arg Cys Ile Val Asn Val Cys Gln Pro Gly Ser Asn Leu
225 230 235 240

Leu Ala Ala Gly Tyr Cys Met Tyr Ser Ser Ser Val Ile Phe Val Leu
245 250 255

Thr Val Gly Thr Gly Val Tyr Val Phe Thr Leu Asp Pro Met Tyr Gly
260 265 270

Glu Phe Val Leu Thr Gln Glu Lys Val Gln Ile Pro Lys Ala Gly Lys
275 280 285

Ile Tyr Ala Phe Asn Glu Gly Asn Tyr Ala Leu Trp Asp Asp Lys Leu
290 295 300

Lys Leu Tyr Met Asp Ser Leu Lys Glu Pro Gly Asp Ser Gly Lys Pro
305 310 315 320

Tyr Ser Ala Arg Tyr Ile Gly Ser Leu Val Gly Asp Phe His Arg Thr
325 330 335

Leu Leu Tyr Gly Gly Ile Tyr Gly Tyr Pro Arg Asp Lys Lys Ser Lys
340 345 350

Asn Gly Lys Leu Arg Leu Leu Tyr Glu Cys Ala Pro Met Ser Phe Ile
355 360 365

Val Glu Gln Ala Gly Gly Lys Gly Ser Asp Gly His Gln Arg Ile Leu
370 375 380

Asp Ile Thr Pro Thr Glu Ile His Gln Arg Val Pro Leu Tyr Ile Gly
385 390 395 400

```
Ser Val Glu Glu Val Asp Lys Val Glu Lys Phe Leu Ala
                405                 410


<210>  196
<211>  1266
<212>  DNA
<213>  Physicomitrella patens

<400>  196
atggcgacca cacaagcgat tctctctgcc acccttgcca tagccccggc ttccagctgc      60

gagacttcgt cacggagccc ggcgtccacc aaaacttgtc tctcagtggc aggatcgtcg     120

ctgcatggct cagtggccgg actcggagct gggaaacaga ttgtgagcgt gcagaggaag     180

agcgttgccg tgagggccgc cgttgcagct gagactgccg ctcccaagca gcaggcgaag     240

agccagtatg acatcactac cctgacgacg tggttgctga agaaagagca ggcgggcgtc     300

atcgatggcg agctcaccat tgtgctctcc agcatcgccc tggcttgcaa gcaaattgcg     360

tctctggtgc agagggctgg catctccaac atgactgggt tgcaaggagc tgctaacatt     420

caaggggagg accagaagaa gctagacgtt atttcgaacg aggtgttctc aagctgtctg     480

cgctcaagcg gacggacagg catcatcgct tctgaggaag aagacacccc ggttgcagtg     540

gaggagagct actctggcaa ctacattgtg gtgttcgacc ctcttgacgg ctcttccaac     600

atcgatgctg ctgtttccac ggggtcaatc tggggaatct acaagcccaa cgaggaatgc     660

ctgaccaatc tcggagagga gccaactatt gatgagatcg ccgaaaactg cgtcgtcaat     720

gtctgtcaac cagggagcaa tttgttgtcc gccgggtact gcatgtactc gagctccgtc     780

atcctcgttc tctcagtcgg tgatggagtc tacggcttca ctctggaccc tctctacgga     840

gaattcgtcc tctcgcacga caacatccaa attccaaaat ctggtaagat ctattccatg     900

aatgaaggca actacgccct ctgggatgac aacctcaaga agtacgtcga cagcttgaag     960

gaccccggtc ccagcggcaa gccctactcc gctcgttaca tcggcagtct ggtgggcgac    1020

ttccacagga caatgctgta tggtggcatc tatggctacc ccagggattc caagagcaag    1080

aacgggaagt tgaggttgct ttacgagtgt gctcccatga gctacctcgc tgaacaagca    1140

ggtgggaagg gctccgacgg tcaccagagg attctggaca tccaacctga gcaggttcac    1200

caacgtgtgc cattgtacgt tggaagcacg gaggaggtgg agaagttgga gaagttctta    1260

gcttaa                                                               1266


<210>  197
<211>  421
<212>  PRT
<213>  Physicomitrella patens

<400>  197

Met Ala Thr Thr Gln Ala Ile Leu Ser Ala Thr Leu Ala Ile Ala Pro
```

```
1               5                   10                  15

Ala Ser Ser Cys Glu Thr Ser Ser Arg Ser Pro Ala Ser Thr Lys Thr
            20                  25                  30

Cys Leu Ser Val Ala Gly Ser Ser Leu His Gly Ser Val Ala Gly Leu
        35                  40                  45

Gly Ala Gly Lys Gln Ile Val Ser Val Gln Arg Lys Ser Val Ala Val
    50                  55                  60

Arg Ala Ala Val Ala Ala Glu Thr Ala Ala Pro Lys Gln Gln Ala Lys
65                  70                  75                  80

Ser Gln Tyr Asp Ile Thr Thr Leu Thr Thr Trp Leu Leu Lys Lys Glu
                85                  90                  95

Gln Ala Gly Val Ile Asp Gly Glu Leu Thr Ile Val Leu Ser Ser Ile
            100                 105                 110

Ala Leu Ala Cys Lys Gln Ile Ala Ser Leu Val Gln Arg Ala Gly Ile
        115                 120                 125

Ser Asn Met Thr Gly Leu Gln Gly Ala Ala Asn Ile Gln Gly Glu Asp
    130                 135                 140

Gln Lys Lys Leu Asp Val Ile Ser Asn Glu Val Phe Ser Ser Cys Leu
145                 150                 155                 160

Arg Ser Ser Gly Arg Thr Gly Ile Ile Ala Ser Glu Glu Glu Asp Thr
                165                 170                 175

Pro Val Ala Val Glu Glu Ser Tyr Ser Gly Asn Tyr Ile Val Val Phe
            180                 185                 190

Asp Pro Leu Asp Gly Ser Ser Asn Ile Asp Ala Ala Val Ser Thr Gly
        195                 200                 205

Ser Ile Trp Gly Ile Tyr Lys Pro Asn Glu Glu Cys Leu Thr Asn Leu
    210                 215                 220

Gly Glu Glu Pro Thr Ile Asp Glu Ile Ala Glu Asn Cys Val Val Asn
225                 230                 235                 240

Val Cys Gln Pro Gly Ser Asn Leu Leu Ser Ala Gly Tyr Cys Met Tyr
                245                 250                 255

Ser Ser Ser Val Ile Leu Val Leu Ser Val Gly Asp Gly Val Tyr Gly
            260                 265                 270
```

```
Phe Thr Leu Asp Pro Leu Tyr Gly Glu Phe Val Leu Ser His Asp Asn
        275                 280                 285

Ile Gln Ile Pro Lys Ser Gly Lys Ile Tyr Ser Met Asn Glu Gly Asn
    290                 295                 300

Tyr Ala Leu Trp Asp Asp Asn Leu Lys Lys Tyr Val Asp Ser Leu Lys
305                 310                 315                 320

Asp Pro Gly Pro Ser Gly Lys Pro Tyr Ser Ala Arg Tyr Ile Gly Ser
                325                 330                 335

Leu Val Gly Asp Phe His Arg Thr Met Leu Tyr Gly Gly Ile Tyr Gly
            340                 345                 350

Tyr Pro Arg Asp Ser Lys Ser Lys Asn Gly Lys Leu Arg Leu Leu Tyr
        355                 360                 365

Glu Cys Ala Pro Met Ser Tyr Leu Ala Glu Gln Ala Gly Gly Lys Gly
    370                 375                 380

Ser Asp Gly His Gln Arg Ile Leu Asp Ile Gln Pro Glu Gln Val His
385                 390                 395                 400

Gln Arg Val Pro Leu Tyr Val Gly Ser Thr Glu Glu Val Glu Lys Leu
                405                 410                 415

Glu Lys Phe Leu Ala
            420


<210>  198
<211>  1263
<212>  DNA
<213>  Galderia sulphuraria

<400>  198
ggcacgagga aagatgtgca agtatacttt attcaagtcc tactatatct ttacaaagaa     60

gagtgtcaag aaataaccac tgcaaaagat atcttcctta taagatggat atccatcaaa    120

atgatagctg ttcaacccca acccaaaaag actcccgtag cagaatattt acaaacctca    180

caagacacac caacttcact cactcgttac ttgttggaag tagccaaaca aaataaggat    240

atgggagata tggtggcatt gataaacggt attcaatttg cttgcaaaaa gatagcttca    300

ttggttggta aagcaggggt cactgacttg atgggaatct atcaacaagg catagtcaac    360

gttcacggag aagaacagaa aaagttggat gttctttcta atgaagtatt gaagaacgct    420

ctcaaatatt cgggaaaaat ggctgtcata gcttcggaag aagaagacgt tcctatcatg    480

gtagaagaaa gttattccgg taactatgta gtcgtgtttg atccattaga tggttcttcc    540
```

aatttggatg ctggattgcc tactggaact atatttggag tgtttcaaca acaattctcg 600

tgtctcattc atgactatga ggagtccatc gataacatgg aattggcttg tttacaaaac 660

actttacaac caggacgtag attgattgcc gctggatatt gtatctattc ttcttctacc 720

atgttggtac tctcattggg taatggtctt cattgtttta ctttggatac ggaagttggt 780

gaatttgtat tgactcgtgc taacatccaa attccacaaa gaggtaatat ttattctttc 840

aacgagtcca acttttatca atgggataaa ggagttcaag attatataga gaggttaaaa 900

aaaggaaaca atcaaacaaa ttgtcgttat tccgcaagat atgttggctc catggttgct 960

gatgtacatc gtacaatatt gtatggcgga atatttggtt atccagcaga taaaaagaat 1020

gtctctggta aattgagatt ggtatatgaa tgtgcaccga tggcatattt ggttgaacaa 1080

gcaggaggta aagcaaccac gggaatagaa aatattttag atttgacacc aaaagatatt 1140

cacgaaagga agcctcttat attaggttct ccagcagata tcgaagaatt tctgcaagta 1200

tatggaatgt cacgagttga tagagaagat atgcatatgt gggataactt gagtagttta 1260

taa 1263

<210> 199
<211> 420
<212> PRT
<213> Galderia sulphuraria

<400> 199

Gly Thr Arg Lys Asp Val Gln Val Tyr Phe Ile Gln Val Leu Leu Tyr
1               5                   10                  15

Leu Tyr Lys Glu Glu Cys Gln Glu Ile Thr Thr Ala Lys Asp Ile Phe
            20                  25                  30

Leu Ile Arg Trp Ile Ser Ile Lys Met Ile Ala Val Gln Pro Gln Pro
        35                  40                  45

Lys Lys Thr Pro Val Ala Glu Tyr Leu Gln Thr Ser Gln Asp Thr Pro
    50                  55                  60

Thr Ser Leu Thr Arg Tyr Leu Leu Glu Val Ala Lys Gln Asn Lys Asp
65                  70                  75                  80

Met Gly Asp Met Val Ala Leu Ile Asn Gly Ile Gln Phe Ala Cys Lys
                85                  90                  95

Lys Ile Ala Ser Leu Val Gly Lys Ala Gly Val Thr Asp Leu Met Gly
            100                 105                 110

Ile Tyr Gln Gln Gly Ile Val Asn Val His Gly Glu Glu Gln Lys Lys
        115                 120                 125

Leu Asp Val Leu Ser Asn Glu Val Leu Lys Asn Ala Leu Lys Tyr Ser
130 135 140

Gly Lys Met Ala Val Ile Ala Ser Glu Glu Glu Asp Val Pro Ile Met
145 150 155 160

Val Glu Glu Ser Tyr Ser Gly Asn Tyr Val Val Val Phe Asp Pro Leu
165 170 175

Asp Gly Ser Ser Asn Leu Asp Ala Gly Leu Pro Thr Gly Thr Ile Phe
180 185 190

Gly Val Phe Gln Gln Gln Phe Ser Cys Leu Ile His Asp Tyr Glu Glu
195 200 205

Ser Ile Asp Asn Met Glu Leu Ala Cys Leu Gln Asn Thr Leu Gln Pro
210 215 220

Gly Arg Arg Leu Ile Ala Ala Gly Tyr Cys Ile Tyr Ser Ser Ser Thr
225 230 235 240

Met Leu Val Leu Ser Leu Gly Asn Gly Leu His Cys Phe Thr Leu Asp
245 250 255

Thr Glu Val Gly Glu Phe Val Leu Thr Arg Ala Asn Ile Gln Ile Pro
260 265 270

Gln Arg Gly Asn Ile Tyr Ser Phe Asn Glu Ser Asn Phe Tyr Gln Trp
275 280 285

Asp Lys Gly Val Gln Asp Tyr Ile Glu Arg Leu Lys Lys Gly Asn Asn
290 295 300

Gln Thr Asn Cys Arg Tyr Ser Ala Arg Tyr Val Gly Ser Met Val Ala
305 310 315 320

Asp Val His Arg Thr Ile Leu Tyr Gly Gly Ile Phe Gly Tyr Pro Ala
325 330 335

Asp Lys Lys Asn Val Ser Gly Lys Leu Arg Leu Val Tyr Glu Cys Ala
340 345 350

Pro Met Ala Tyr Leu Val Glu Gln Ala Gly Gly Lys Ala Thr Thr Gly
355 360 365

Ile Glu Asn Ile Leu Asp Leu Thr Pro Lys Asp Ile His Glu Arg Lys
370 375 380

Pro Leu Ile Leu Gly Ser Pro Ala Asp Ile Glu Glu Phe Leu Gln Val
385 390 395 400

Tyr Gly Met Ser Arg Val Asp Arg Glu Asp Met His Met Trp Asp Asn
405 410 415

Leu Ser Ser Leu
420

<210> 200
<211> 710
<212> DNA
<213> Marchantia polymorpha

<400> 200
gaggaggata ccccagtcgc cgtcgaagag agctactcag gaaactacgt cgttgtcttc 60

gatcctctcg atggatcctc caacatcgac gctgcggtat ctaccggatc catctttgga 120

atctacaggc ccactgagga gtgtcttgca gacatggacg acgactcaca gctcggtatg 180

gtggaacaaa actgcatcgt gaacgtatgc cagcccggta gcaacctcct atccgctggg 240

tactgcatgt actccagctc cgtcattttg gtgttgtcag tcggagacgg tgtctatgga 300

ttcacactgg atcccctgta cggtgaattc gtcatgaccc acgacaacat caagatccca 360

aagaagggat cgatctactc gttcaatgaa ggtaactacg cactctggga tgacaagctc 420

aagaagtaca tcgactcact gaaggacccc gaacccaccg gcaagcctta ctctgctcgt 480

tacatcggta gtctggtcgg tgacttccac agaaccatgc tctatggtgg catctacgga 540

taccctgccg acaagaaaag caagaacgga aagttgagac ttctgtacga gtgtgctcct 600

atgagctact tggcagagca ggccggagga aagggttctg atggttaccg cagagttctg 660

gaaatcgagc ctgagcaggt acatcagcga gtgccacttt tcgtcggaag 710

<210> 201
<211> 236
<212> PRT
<213> Marchantia polymorpha

<400> 201

Glu Glu Asp Thr Pro Val Ala Val Glu Glu Ser Tyr Ser Gly Asn Tyr
1 5 10 15

Val Val Val Phe Asp Pro Leu Asp Gly Ser Ser Asn Ile Asp Ala Ala
20 25 30

Val Ser Thr Gly Ser Ile Phe Gly Ile Tyr Arg Pro Thr Glu Glu Cys
35 40 45

Leu Ala Asp Met Asp Asp Asp Ser Gln Leu Gly Met Val Glu Gln Asn
50 55 60

```
Cys Ile Val Asn Val Cys Gln Pro Gly Ser Asn Leu Leu Ser Ala Gly
65                  70                  75                  80

Tyr Cys Met Tyr Ser Ser Ser Val Ile Leu Val Leu Ser Val Gly Asp
                85                  90                  95

Gly Val Tyr Gly Phe Thr Leu Asp Pro Leu Tyr Gly Glu Phe Val Met
            100                 105                 110

Thr His Asp Asn Ile Lys Ile Pro Lys Lys Gly Ser Ile Tyr Ser Phe
        115                 120                 125

Asn Glu Gly Asn Tyr Ala Leu Trp Asp Asp Lys Leu Lys Lys Tyr Ile
    130                 135                 140

Asp Ser Leu Lys Asp Pro Glu Pro Thr Gly Lys Pro Tyr Ser Ala Arg
145                 150                 155                 160

Tyr Ile Gly Ser Leu Val Gly Asp Phe His Arg Thr Met Leu Tyr Gly
                165                 170                 175

Gly Ile Tyr Gly Tyr Pro Ala Asp Lys Lys Ser Lys Asn Gly Lys Leu
            180                 185                 190

Arg Leu Leu Tyr Glu Cys Ala Pro Met Ser Tyr Leu Ala Glu Gln Ala
        195                 200                 205

Gly Gly Lys Gly Ser Asp Gly Tyr Arg Arg Val Leu Glu Ile Glu Pro
    210                 215                 220

Glu Gln Val His Gln Arg Val Pro Leu Phe Val Gly
225                 230                 235


<210>  202
<211>  765
<212>  DNA
<213>  Tagetes patula

<400>  202
atcattgcat cggaggaaga agacgtgccg gtggctgtgg aagagagtta ctccggaaac      60

tacattgtcg tgtttgatcc ccttgatggg tcatctaata ttgatgctgc tgtttcaacc     120

ggttctattt tcggaatcta tagccccaat gatgagtgtc tcgctgatat tagtgacgac     180

tccacgctag acagtgtgga acaaaaatgt attgtcaacg tatgccagcc cggaagcaat     240

ctactagcag ccggttactg tatgtactca agctccgtaa tcttcgtgct ctcgatcggt     300

actggtgtct acgcgttcac attagaccca atgtacggtg agtttgtact cactcaagaa     360

aagattcaaa tcccgaaatc ggggaagatt tactcgttta acgaaggaaa ctatcaacta     420
```

tgggacgata aattgaagaa gtacatggat gatctaaagg acccgggccc cacgggcaag 480

ccgtattcgg ctcgctacat tggtagcttg gttggtgatt ttcataggac attattgtac 540

ggagggattt acgggtaccc acgtgacaaa aagagcaaga acgggaagct tcggttgttg 600

tatgagtgtg caccgatgag ttacttggtt gaacaagcgg gtggaaaggg gtcggatgga 660

catcaacgag tgctcgacat tcaaccaacc gagattcatc agcgcgttcc gctatacatt 720

gggagcgtag aggaagtgga aaaattggag aagtatttgg cttga 765

<210> 203
<211> 254
<212> PRT
<213> Tagetes patula

<400> 203

Ile Ile Ala Ser Glu Glu Glu Asp Val Pro Val Ala Val Glu Glu Ser
1 5 10 15

Tyr Ser Gly Asn Tyr Ile Val Val Phe Asp Pro Leu Asp Gly Ser Ser
20 25 30

Asn Ile Asp Ala Ala Val Ser Thr Gly Ser Ile Phe Gly Ile Tyr Ser
35 40 45

Pro Asn Asp Glu Cys Leu Ala Asp Ile Ser Asp Asp Ser Thr Leu Asp
50 55 60

Ser Val Glu Gln Lys Cys Ile Val Asn Val Cys Gln Pro Gly Ser Asn
65 70 75 80

Leu Leu Ala Ala Gly Tyr Cys Met Tyr Ser Ser Ser Val Ile Phe Val
85 90 95

Leu Ser Ile Gly Thr Gly Val Tyr Ala Phe Thr Leu Asp Pro Met Tyr
100 105 110

Gly Glu Phe Val Leu Thr Gln Glu Lys Ile Gln Ile Pro Lys Ser Gly
115 120 125

Lys Ile Tyr Ser Phe Asn Glu Gly Asn Tyr Gln Leu Trp Asp Asp Lys
130 135 140

Leu Lys Lys Tyr Met Asp Asp Leu Lys Asp Pro Gly Pro Thr Gly Lys
145 150 155 160

Pro Tyr Ser Ala Arg Tyr Ile Gly Ser Leu Val Gly Asp Phe His Arg
165 170 175

```
Thr Leu Leu Tyr Gly Gly Ile Tyr Gly Tyr Pro Arg Asp Lys Lys Ser
            180                 185                 190

Lys Asn Gly Lys Leu Arg Leu Leu Tyr Glu Cys Ala Pro Met Ser Tyr
        195                 200                 205

Leu Val Glu Gln Ala Gly Gly Lys Gly Ser Asp Gly His Gln Arg Val
    210                 215                 220

Leu Asp Ile Gln Pro Thr Glu Ile His Gln Arg Val Pro Leu Tyr Ile
225                 230                 235                 240

Gly Ser Val Glu Glu Val Glu Lys Leu Glu Lys Tyr Leu Ala
                245                 250


<210>  204
<211>  795
<212>  DNA
<213>  Linum usitatissimum


<220>
<221>  misc_feature
<222>  (738)..(738)
<223>  n is a, c, g, or t

<400>  204
gaggaggaac aacgtcgccg gggttctgg tttcaaatgc tctgccgtcg ggacgcgccg     60

tcggaggcgg caacgacgac gaagaagagg agtagctacg agattctgac gctgacgacc    120

tggcttctgc agcaggaaca ggccggagtg atcgacgccg agctcacgat tgtgctctcc    180

agcatctcca cggcgtgtaa gcagatcgct tctctagttc agcgatccgg gatttctaac    240

ctcaccggcg tccagggcgc cgtcaatgtc cagggtgagg atcagaagaa gctcgacgtc    300

gtttcgaacg aggtgttttc aaattgcttg aggtcgagcg gcaggacggg gatcatagcg    360

tcggaggaag aagacgtgcc ggtcgccgtg gaggaaagct actccggtaa ctatatcgta    420

gtgttcgatc cacttgatgg atcgtcaaac atcgatgccg cagtctccac cggctcaatc    480

ttcggaatct acagtcccaa cgatgagtgc ctggccgaca tcggagacgg agacgagtca    540

aatctagata ctcaggagca gaagtgtgtg gtgagcgtgt gccagccggg gagcaaccta    600

ctcgccgccg gctactgcat gtactcaagc tcggtgatct tcgtcctcac gatcggaaac    660

ggcgttttcg ccttcaatct ggacccaatg tacggcgagt tggtgttgac tcaagagaac    720

attcagatcc cgaaagcngg aaagatctac tcattcaacg aaggggacta ccaaatgtgg    780

gatgagaaat tgaag                                                     795


<210>  205
<211>  265
<212>  PRT
<213>  Linum usitatissimum
```

<400> 205

Glu Glu Glu Gln Arg Arg Arg Gly Phe Trp Phe Gln Met Leu Cys Arg
1               5                   10                  15

Arg Asp Ala Pro Ser Glu Ala Ala Thr Thr Thr Lys Lys Arg Ser Ser
            20                  25                  30

Tyr Glu Ile Leu Thr Leu Thr Thr Trp Leu Leu Gln Gln Glu Gln Ala
        35                  40                  45

Gly Val Ile Asp Ala Glu Leu Thr Ile Val Leu Ser Ser Ile Ser Thr
    50                  55                  60

Ala Cys Lys Gln Ile Ala Ser Leu Val Gln Arg Ser Gly Ile Ser Asn
65                  70                  75                  80

Leu Thr Gly Val Gln Gly Ala Val Asn Val Gln Gly Glu Asp Gln Lys
                85                  90                  95

Lys Leu Asp Val Val Ser Asn Glu Val Phe Ser Asn Cys Leu Arg Ser
            100                 105                 110

Ser Gly Arg Thr Gly Ile Ile Ala Ser Glu Glu Glu Asp Val Pro Val
        115                 120                 125

Ala Val Glu Glu Ser Tyr Ser Gly Asn Tyr Ile Val Val Phe Asp Pro
    130                 135                 140

Leu Asp Gly Ser Ser Asn Ile Asp Ala Ala Val Ser Thr Gly Ser Ile
145                 150                 155                 160

Phe Gly Ile Tyr Ser Pro Asn Asp Glu Cys Leu Ala Asp Ile Gly Asp
                165                 170                 175

Gly Asp Glu Ser Asn Leu Asp Thr Gln Glu Gln Lys Cys Val Val Ser
            180                 185                 190

Val Cys Gln Pro Gly Ser Asn Leu Leu Ala Ala Gly Tyr Cys Met Tyr
        195                 200                 205

Ser Ser Ser Val Ile Phe Val Leu Thr Ile Gly Asn Gly Val Phe Ala
    210                 215                 220

Phe Asn Leu Asp Pro Met Tyr Gly Glu Leu Val Leu Thr Gln Glu Asn
225                 230                 235                 240

Ile Gln Ile Pro Lys Ala Gly Lys Ile Tyr Ser Phe Asn Glu Gly Asp
                245                 250                 255

```
Tyr Gln Met Trp Asp Glu Lys Leu Lys
            260                 265


<210>  206
<211>  50
<212>  DNA
<213>  Artificial sequence

<220>
<223>  primer: prm08448

<400>  206
ggggacaagt ttgtacaaaa aagcaggctt aaacaatggc cgccaccatg                50


<210>  207
<211>  50
<212>  DNA
<213>  Artificial sequence

<220>
<223>  primer: prm08449

<400>  207
ggggaccact ttgtacaaga aagctgggta gctgcttagt gcttcttggt                50


<210>  208
<211>  1167
<212>  DNA
<213>  Oryza sativa

<400>  208
atccaagcca agaagaggga gagcaccaag gacacgcgac tagcagaagc cgagcgaccg      60

ccttcttcga tccatatctt ccggtcgagt tcttggtcga tctcttccct cctccacctc     120

ctcctcacag ggtatgtgcc cttcggttgt tcttggattt attgttctag gttgtgtagt     180

acgggcgttg atgttaggaa aggggatctg tatctgtgat gattcctgtt cttggatttg     240

ggatagaggg gttcttgatg ttgcatgtta tcggttcggt ttgattagta gtatggtttt     300

caatcgtctg gagagctcta tggaaatgaa atggtttagg gtacggaatc ttgcgatttt     360

gtgagtacct tttgtttgag gtaaaatcag agcaccggtg attttgcttg gtgtaataaa     420

agtacggttg tttggtcctc gattctggta gtgatgcttc tcgatttgac gaagctatcc     480

tttgtttatt ccctattgaa caaaaataat ccaactttga agacggtccc gttgatgaga     540

ttgaatgatt gattcttaag cctgtccaaa atttcgcagc tggcttgttt agatacagta     600

gtccccatca cgaaattcat ggaaacagtt ataatcctca ggaacagggg attccctgtt     660

cttccgattt gctttagtcc cagaattttt tttcccaaat atcttaaaaa gtcactttct     720

ggttcagttc aatgaattga ttgctacaaa taatgctttt atagcgttat cctagctgta     780

gttcagttaa taggtaatac ccctatagtt tagtcaggag aagaacttat ccgatttctg     840

atctccattt ttaattatat gaaatgaact gtagcataag cagtattcat ttggattatt     900
```

tttttatta gctctcaccc cttcattatt ctgagctgaa agtctggcat gaactgtcct 960

caattttgtt ttcaaattca catcgattat ctatgcatta tcctcttgta tctacctgta 1020

gaagtttctt tttggttatt ccttgactgc ttgattacag aaagaaattt atgaagctgt 1080

aatcgggata gttatactgc ttgttcttat gattcatttc ctttgtgcag ttcttggtgt 1140

agcttgccac tttcaccagc aaagttc 1167

<210> 209
<211> 1629
<212> DNA
<213> Oryza sativa

<400> 209
atgatgggtt gcttcactgt cctgagatcc aagaagaaga agcctcttgc tctcaccaag 60

aaatcggttg atgcaaggga aagcacgtcc tcaagactcc cagagccaga agcgcatgtg 120

ccatcgttac aatctgctcc tcctagtttt aggaacaagg ctaaaatcca ccaatcggaa 180

aagaaagctt cttacagcag agcgcgcgtg ctgtctgctc cttccagcct aattgtggtt 240

gatcaggatg gtcttccata tgccgaattc gatgatcaag atgactccag ggcaaggga 300

ggttctataa agggccaccg tttctctaat ccactgcccc ttcctctccc atcaccagaa 360

ggaaaatcat tgaggaactt tggcagcttc aaagccatca atgcaagtgg accactcgat 420

gcttcaggcc ctctgccact tcctccaaag aagtgtgatg ggcttaagaa tttctcctat 480

gaggaacttt catcagcttg ccaatggttt tctggtgacc agtgtgtttc cgaaagtttg 540

acatcaacat catacaaggc gtcctttagg gatgatttta ccgacccaaa gaccattgaa 600

gcaatagtat ctcggttgct ctcctccact cagagtttga aagagtttaa aacacaagtg 660

aataccttgg catcacttca gcatcccaac ttatgtaaac taatcggctt tcacgcaaga 720

gaagaatcta atgaaaggat gttggtctat gagcgactcc atcatggcag cttagataaa 780

ctactctttg gaagatcgga tggtcgtttc atggactggt cagcacgttt gaaggttgct 840

cttggtgctg ctagaggcct ggctttccta catgatgaag ggccttttca ggccatgtac 900

aatgacttct caacctcaaa catccaaatt gacaaagatt tcactgcaaa gctatcagga 960

tatggatgtg ttggattcaa taccgaggag gaaatatcaa atgcatctgt ggctgctgca 1020

aacctctcag tggaaacctt ggagaaaggt gtactgactc ccaagagcaa cgtatggtgc 1080

tttggagttg tcctgctgga gctaataaca ggaaggaaga accttgatgt ccgttcctca 1140

aaagaagaac gcaatattgt caagtggagt aggcctttcc tcaccgatga tagtcgccta 1200

tcgttaatca tggactcccg tataaaagga cgcttcccta ccaaggctgc tcggattgta 1260

gcagatatca tattgagatg ccttaataaa gatccatcag agaggcctac catgagggcc 1320

gttgtggagt ccctagcaag cgtccaggac ataaaggttc catgtcgata tcctttgcaa 1380

gagccatctg ctgccccaag aaaggtgatg ttaaaatcta caagtctcaa tggcatcatt 1440

catcaccatc ctgtcgtaac cttctcaccg tcacctcctt cgcgaaacca acatttgctc 1500

tcgccaaggt catccacgtc tgcactgctt cctccaagga ccagctgtgc tctggatgac 1560

cctagagtaa gctctatcaa gaaatcgcct tcccctattt tacggagatc tggtgttgag 1620

ggtttttga 1629

<210> 210
<211> 542
<212> PRT
<213> Oryza sativa

<400> 210

Met Met Gly Cys Phe Thr Val Leu Arg Ser Lys Lys Lys Lys Pro Leu
1 5 10 15

Ala Leu Thr Lys Lys Ser Val Asp Ala Arg Glu Ser Thr Ser Ser Arg
20 25 30

Leu Pro Glu Pro Glu Ala His Val Pro Ser Leu Gln Ser Ala Pro Pro
35 40 45

Ser Phe Arg Asn Lys Ala Lys Ile His Gln Ser Glu Lys Lys Ala Ser
50 55 60

Tyr Ser Arg Ala Arg Val Leu Ser Ala Pro Ser Ser Leu Ile Val Val
65 70 75 80

Asp Gln Asp Gly Leu Pro Tyr Ala Glu Phe Asp Asp Gln Asp Asp Ser
85 90 95

Arg Gly Lys Gly Gly Ser Ile Lys Gly His Arg Phe Ser Asn Pro Leu
100 105 110

Pro Leu Pro Leu Pro Ser Pro Glu Gly Lys Ser Leu Arg Asn Phe Gly
115 120 125

Ser Phe Lys Ala Ile Asn Ala Ser Gly Pro Leu Asp Ala Ser Gly Pro
130 135 140

Leu Pro Leu Pro Pro Lys Lys Cys Asp Gly Leu Lys Asn Phe Ser Tyr
145 150 155 160

Glu Glu Leu Ser Ser Ala Cys Gln Trp Phe Ser Gly Asp Gln Cys Val
165 170 175

Ser Glu Ser Leu Thr Ser Thr Ser Tyr Lys Ala Ser Phe Arg Asp Asp
180 185 190

Phe Thr Asp Pro Lys Thr Ile Glu Ala Ile Val Ser Arg Leu Leu Ser
195 200 205

Ser Thr Gln Ser Leu Lys Glu Phe Lys Thr Gln Val Asn Thr Leu Ala
210 215 220

Ser Leu Gln His Pro Asn Leu Cys Lys Leu Ile Gly Phe His Ala Arg
225 230 235 240

Glu Glu Ser Asn Glu Arg Met Leu Val Tyr Glu Arg Leu His His Gly
245 250 255

Ser Leu Asp Lys Leu Leu Phe Gly Arg Ser Asp Gly Arg Phe Met Asp
260 265 270

Trp Ser Ala Arg Leu Lys Val Ala Leu Gly Ala Ala Arg Gly Leu Ala
275 280 285

Phe Leu His Asp Glu Gly Pro Phe Gln Ala Met Tyr Asn Asp Phe Ser
290 295 300

Thr Ser Asn Ile Gln Ile Asp Lys Asp Phe Thr Ala Lys Leu Ser Gly
305 310 315 320

Tyr Gly Cys Val Gly Phe Asn Thr Glu Glu Glu Ile Ser Asn Ala Ser
325 330 335

Val Ala Ala Ala Asn Leu Ser Val Glu Thr Leu Glu Lys Gly Val Leu
340 345 350

Thr Pro Lys Ser Asn Val Trp Cys Phe Gly Val Val Leu Leu Glu Leu
355 360 365

Ile Thr Gly Arg Lys Asn Leu Asp Val Arg Ser Ser Lys Glu Glu Arg
370 375 380

Asn Ile Val Lys Trp Ser Arg Pro Phe Leu Thr Asp Asp Ser Arg Leu
385 390 395 400

Ser Leu Ile Met Asp Ser Arg Ile Lys Gly Arg Phe Pro Thr Lys Ala
405 410 415

Ala Arg Ile Val Ala Asp Ile Ile Leu Arg Cys Leu Asn Lys Asp Pro
420 425 430

Ser Glu Arg Pro Thr Met Arg Ala Val Val Glu Ser Leu Ala Ser Val
435 440 445

Gln Asp Ile Lys Val Pro Cys Arg Tyr Pro Leu Gln Glu Pro Ser Ala

450 455 460

Ala Pro Arg Lys Val Met Leu Lys Ser Thr Ser Leu Asn Gly Ile Ile
465 470 475 480

His His His Pro Val Val Thr Phe Ser Pro Ser Pro Pro Ser Arg Asn
485 490 495

Gln His Leu Leu Ser Pro Arg Ser Ser Thr Ser Ala Leu Leu Pro Pro
500 505 510

Arg Thr Ser Cys Ala Leu Asp Asp Pro Arg Val Ser Ser Ile Lys Lys
515 520 525

Ser Pro Ser Pro Ile Leu Arg Arg Ser Gly Val Glu Gly Phe
530 535 540

<210> 211
<211> 1674
<212> DNA
<213> Arabidopsis thaliana

<400> 211
atggttttgg ggtgtttccc tttgaaaagc aagaagaaac gtggctctgt ttctatgaag 60

cggttggatc ttgaagaaag caagccaact gctttacctg agccaccaaa gattccaagt 120

cgtaatttac aatcaggtcc tccgagtttc agaaatcgtg tgaagccaat tcaatcaaac 180

aacggtggaa acggagagat gagtagccga gcaagagtca tgtttgctcc gtcaagcatc 240

cacggtgcag cggaacggga tttgcttgct ggtgtttacc acgacgagca agatgaacaa 300

ccaagagatc cacgtacttc tactaaagaa tctagccctc aaccacttcc gttaccgtca 360

ccaagaactg gttcttcatt gaagaattgg ggaagcttta agtcgtttaa cggaagcagc 420

ggtcggttat catcatccgc agctgtatct ggacctttac ctttgccacc tagcgggtca 480

gttaggagct tttcatatga tgaagtaatg gctgcgtgta acgctttttc ttcagaccga 540

tgtgtcatgg aaggtctttc atctgttatg tacatggctt cctttggtga tgaggcttcg 600

acctcaggtt taaagaaggt tgacgcaact gttgtacgac ttcacgtaac tactcagagt 660

attagggagt tcattaatga agtcaacaca ttggcgtcgc tgcaacacca gaacctttgt 720

aagctggtag gctatcatgc tcgtgacggt tctgacacaa gaatgttggt gtacgagagg 780

cttgctctgg gcagcttgga ccgtttactg catgggagat cagatgggcc tcctcttgat 840

tggaacacta gaatgaagat tgcactatgt gcagctcagg gtctaacctt cttgcacgaa 900

gaaggccctt ttcaggcaat gtacaatgaa ttttcgacgg caaatatcca agtcgataaa 960

gatttcagcg ccaagctatc aggatacggt tgtgcaggcc atgcgcctga gacagagaca 1020

tctaatagtt cggcacttgc taatctctct gtcgagactc tagagagagg gcttttgacc 1080

ccgaagagca atgtgtggag ctatggaata gttcttcttg agatgttaac gggtcggaaa 1140

aatatggacg ggtcttaccc gaaagaagag aggaacttag tgaaatggag cagagctttt 1200

ctagcagatg attgcaggct ctcgcttata atggatcctc agcttaaagg tcggtttccg 1260

gcaaaagcgg cgaggagcat agcagatata gcacagaaat gtctgcaggt ggagccatca 1320

gagcgtccaa ccatgagaaa catcgtggat caactcaaga tcatacagga catgaagtac 1380

tcgtgtaggt tcccgttaag agaacccgca ccagtcgcgg caaggaaaca tatgggaaga 1440

tcaagcagtc tcaacacgat tatttggacc ccggcatcag tgccaccaag gtcaagtttt 1500

tcaccgtcac ctccaccacg acgaccgtct gtttcaccca caaggggacg gacgctcgtg 1560

tttcccccag tgtttccgcc gcgagcgtgt tcatctttgg aggaaatggc tcgggaagag 1620

gttcgaagat cgtcttcagc cagtggtagg agaactagcc tcgaagggtt ttga 1674

<210> 212
<211> 557
<212> PRT
<213> Arabidopsis thaliana

<400> 212

Met Val Leu Gly Cys Phe Pro Leu Lys Ser Lys Lys Lys Arg Gly Ser
1 5 10 15

Val Ser Met Lys Arg Leu Asp Leu Glu Glu Ser Lys Pro Thr Ala Leu
20 25 30

Pro Glu Pro Pro Lys Ile Pro Ser Arg Asn Leu Gln Ser Gly Pro Pro
35 40 45

Ser Phe Arg Asn Arg Val Lys Pro Ile Gln Ser Asn Asn Gly Gly Asn
50 55 60

Gly Glu Met Ser Ser Arg Ala Arg Val Met Phe Ala Pro Ser Ser Ile
65 70 75 80

His Gly Ala Ala Glu Arg Asp Leu Leu Ala Gly Val Tyr His Asp Glu
85 90 95

Gln Asp Glu Gln Pro Arg Asp Pro Arg Thr Ser Thr Lys Glu Ser Ser
100 105 110

Pro Gln Pro Leu Pro Leu Pro Ser Pro Arg Thr Gly Ser Ser Leu Lys
115 120 125

Asn Trp Gly Ser Phe Lys Ser Phe Asn Gly Ser Ser Gly Arg Leu Ser
130 135 140

Ser Ser Ala Ala Val Ser Gly Pro Leu Pro Leu Pro Pro Ser Gly Ser

145 150 155 160

Val Arg Ser Phe Ser Tyr Asp Glu Val Met Ala Ala Cys Asn Ala Phe
165 170 175

Ser Ser Asp Arg Cys Val Met Glu Gly Leu Ser Ser Val Met Tyr Met
180 185 190

Ala Ser Phe Gly Asp Glu Ala Ser Thr Ser Gly Leu Lys Lys Val Asp
195 200 205

Ala Thr Val Val Arg Leu His Val Thr Thr Gln Ser Ile Arg Glu Phe
210 215 220

Ile Asn Glu Val Asn Thr Leu Ala Ser Leu Gln His Gln Asn Leu Cys
225 230 235 240

Lys Leu Val Gly Tyr His Ala Arg Asp Gly Ser Asp Thr Arg Met Leu
245 250 255

Val Tyr Glu Arg Leu Ala Leu Gly Ser Leu Asp Arg Leu Leu His Gly
260 265 270

Arg Ser Asp Gly Pro Pro Leu Asp Trp Asn Thr Arg Met Lys Ile Ala
275 280 285

Leu Cys Ala Ala Gln Gly Leu Thr Phe Leu His Glu Glu Gly Pro Phe
290 295 300

Gln Ala Met Tyr Asn Glu Phe Ser Thr Ala Asn Ile Gln Val Asp Lys
305 310 315 320

Asp Phe Ser Ala Lys Leu Ser Gly Tyr Gly Cys Ala Gly His Ala Pro
325 330 335

Glu Thr Glu Thr Ser Asn Ser Ser Ala Leu Ala Asn Leu Ser Val Glu
340 345 350

Thr Leu Glu Arg Gly Leu Leu Thr Pro Lys Ser Asn Val Trp Ser Tyr
355 360 365

Gly Ile Val Leu Leu Glu Met Leu Thr Gly Arg Lys Asn Met Asp Gly
370 375 380

Ser Tyr Pro Lys Glu Glu Arg Asn Leu Val Lys Trp Ser Arg Ala Phe
385 390 395 400

Leu Ala Asp Asp Cys Arg Leu Ser Leu Ile Met Asp Pro Gln Leu Lys
405 410 415

Gly Arg Phe Pro Ala Lys Ala Ala Arg Ser Ile Ala Asp Ile Ala Gln
420 425 430

Lys Cys Leu Gln Val Glu Pro Ser Glu Arg Pro Thr Met Arg Asn Ile
435 440 445

Val Asp Gln Leu Lys Ile Ile Gln Asp Met Lys Tyr Ser Cys Arg Phe
450 455 460

Pro Leu Arg Glu Pro Ala Pro Val Ala Ala Arg Lys His Met Gly Arg
465 470 475 480

Ser Ser Ser Leu Asn Thr Ile Ile Trp Thr Pro Ala Ser Val Pro Pro
485 490 495

Arg Ser Ser Phe Ser Pro Ser Pro Pro Pro Arg Arg Pro Ser Val Ser
500 505 510

Pro Thr Arg Gly Arg Thr Leu Val Phe Pro Pro Val Phe Pro Pro Arg
515 520 525

Ala Cys Ser Ser Leu Glu Glu Met Ala Arg Glu Glu Val Arg Arg Ser
530 535 540

Ser Ser Ala Ser Gly Arg Arg Thr Ser Leu Glu Gly Phe
545 550 555

<210> 213
<211> 2069
<212> DNA
<213> Sorghum bicolor

<400> 213
ctactggatt aacccccgc gtgcgttccc tccctctgtc agtctgtctc tctcttggcg 60

tcgactgggc gaccgtcgca gccgccctaa gccaggtacc cagaccatct tcgggccctt 120

cgccggcgac gagcaccacc aggaattttc ccagctgtag caatgatggg ttgcttcact 180

gttctcagat ccaagaagaa gaaaatccct tttgataatc ctcttcttcc aagcaagaaa 240

tcggttgatg caagggaaag tacgtcgtct agacttccgg aaccagaagt tcatgtgcca 300

tctttgcaat cagctctctc ctagttttag gaacaggact aagatatccc agtcgtcaaa 360

taaagtttca aacagcagag ctcgcgtgtt gtctgctccg tcaaccctta ttgtggttga 420

tcagtttggc tttccatatg ctgaataccg agatcaagac gactccagag ataaggaagg 480

ttcaacaaag gggcatcgct tttcaaatcc tttgcccctt cctcttcctt caccggaagg 540

acattctttt aggaactctg gtagcttcaa agctagcaac gtaagtgggc cattggagat 600

gtccagccct ctcccattgc ctccaaagaa atgtgatgga cttagaatct tttcttacga 660

ggaggttttg tctgcttgcc aatggttttc aagtgatcag tgtgtttcag aagcacttgg 720

ttcaacatca tacaaggcaa catttaggga tgaatttatt gatacaaaga ccactgaagc 780

aacggtagct cgattactcc cctccactca gagtttgaag gagtttaaaa cacaagcaac 840

tacattggca ttgcttcagc atcccaattt atgtaaactg attggctatt atgcaaaaga 900

agattctaat gaaaggatgt tggtctatga acggcttcat catgggagct tagataagct 960

actctttgga agaccagatg gtcgtttcat ggactggtct aaacgtttga aggttgccct 1020

tggtgcggcc agaggtcttg ctttcttgca tgatgaagga ccctttcagg ccatgtacag 1080

cgagttctca actttgaaca tccaaataga taaagatttc actgctaagc tttcaggata 1140

tggttgtgtt ggcttcaata ctgaggagat atctaatgca cctgcgtctg cagcaaacct 1200

ttcggtggag accttggaga agggtttact cactcccaag agcaatgtct ggagctttgg 1260

agttgtgtta ctggagctaa taactggaag gaagaacctt gatgccaatt cttctaaaga 1320

agaacgcaat attgtcaggt ggagtaggcc tttccttact gatgatagtc gcctatctct 1380

gatcatggac tcccgtataa aagggcgctt tcctactaag gctgctcgga tagtagcaga 1440

catcattctg aaatgcttgc ataatgatcc atccgagagg ccgaccatga gggatgttgt 1500

ggaggctcta gcaagagtcc aggagataaa ggttccgtgc agatatcctt tgcaagaacc 1560

ttctgctgca ccaagaaaag taatgctaaa atctacatcc ctcaatggaa ttgtgcctca 1620

tcatcctgtc ataaccttct ctccatcgcc gccttcgcat aaccagcact tgatttcacc 1680

aaggtcatca acatctgcct tgtttcatcc aaggacatgc tcttctactc tggatgatcc 1740

cggtgtaagc tctataaaga aaacacctcc tattatgcgt aggtctagcg ttgaaggctt 1800

ttgattgtcc atattgttct tttatttctt tttctcggat ttatttgttt ctttgttagc 1860

ctagtgattt tagctgtgtt ctgtattgta agacaagtgg ccttatgtag tgcccttgag 1920

tctcgagtaa taactaagca gagcaggatg aattgtaaat agtatcaggt tgtagatacc 1980

tttttgtgct ctaattgggt ggaagcagcg tgctgagtct gagtaggcct tgtaacctca 2040

taaataaact cgtttcccag tttctgtcc 2069

<210> 214
<211> 1360
<212> DNA
<213> Saccharum officinarum

<400> 214
agaaacactt ggttcaacat catacaaggc aacatttagg gatgaattta ttgatacaaa 60

gaccactgaa gcaacggtag ctcgattact cccctccact cagagtttga aggagtttaa 120

aacacaagcg accacattgg catcacttca gcatcccaat ttgtgtaaac tgattggcta 180

ttatgcaaaa gaagattcta atgaaaggat gttggtctat gaacggcttc atcatgggag 240

cttagataag ctactctttg gaagaccaga tggtcgtttc atggactggt ctaaacgttt 300

gaaggtttcc cttggtgctg cccgaggtct agctttcttg catgatgaag gaccctttca 360

ggccatgtac agtgagttct caactttgaa catccaaata gataaagatt tcactgctaa 420

gctttcagga tatggttgtg ttggcttcaa tactgaggag atatctaatg cacctgcgtc 480

tgcagcaaac ctttcggtgg agaccttgga gaagggttta ctcactccca agagcaacgt 540

ctggagcttt ggagttgtgt tactggagct aataactgga aggaagaacc ttgatgccaa 600

ttcttccaaa gaagaacgca atattgtcag gtggagtagg cctttcctta ctgatgatag 660

tcgcctatct ctgatcatgg actcccgtat aaaagggcgc tttccaacta aggctgctcg 720

gatagtagca gacatcattc tgaaatgctt gcataaagat ccatcagaga ggccgaccat 780

gagggatgtt gtggaggccc tagcaagagt ccaggaaata aaggttccat gcagatatcc 840

tctgcaagaa ccttttgctg caccaagaaa aataatgtta aaatctacat ccctcaatgg 900

aattgtgcct catcatcctg tcataacctt ctccccttcg ccgccttcac ataaccaaca 960

cttgatttca ccaaggtcat caacatctgc cttgtttcat ccaaggacat gctcttctac 1020

tctggatgat cccggtgtaa gctctataaa gaaaacacct cctattatgc gtaggtctag 1080

cgtcgaaggc ttttgattgt ccatattgtt cttttatttc cttttcttgg atttattagt 1140

ttctttggta gcctagtgat tctagctatg ttctgtattg caagacaagc ggccttaatg 1200

tagtgccctt gggtctagag taataactaa gcagagcagg atgaattgta aataggatca 1260

ggttgtagat accttttttgt gctctaattg ggtggaagca gcgtgctaag tgagccttgt 1320

aacctcccaa tgcaataaac tcgtttttca gtttttgttc 1360

<210> 215
<211> 2062
<212> DNA
<213> Medicago truncatula

<400> 215
acgaggctat tctctctctt tctctctcta catttctcaa cattcttcaa atttctctct 60

ccaaaacctt cacttcgcag ctttttcaat ctgctttctt tgactctgta acagtttttt 120

tcgtggaaga atcagaacca caaaaaagtt tcgattttta cccatttctt caacccgtga 180

tcgcttcact gtaattggca tgagcttcca tttctcgttc tgacaaaaac aggtatctat 240

aacatcctag ttctgtacat agaatggggt gttttactat attgaagaga aagaagaaaa 300

agcctgatca gattgtgtat gtaaaacgcg taagccctgg cgaagattca cctacagtac 360

tgcccgaacc ccaaactcat acccgctcac tccagtctgc gcctcctagt tttaagatca 420

gagtgaaacc cattcaacct agcaataaag ccactaacaa tagaatacga gcattgtctg 480

ctccatcgag tcttgatgat gcagaacaag atgcattggc caccattgag tatgaagaac 540

aagaagggtc aaaataccga actggatcat ggaaggagca gcgttcgcct agtccacaac 600

cattaccgct tccatctcct aagggtggtg gtacattgaa gactgttgga agctttaagt 660

tggggatagc tagtagtcct ttatatgctt ctggaccttt gccgcttcca ccaactgggt 720

cactgagaaa cttttcttat gatgaacttg ctgctgcttg cctcaatttc tcttcagatc 780

gatacatgtc agaatctctt tcatccacca tgtataaagc ttcctttggt gatgatacct 840

caacttcaag ttcaaagaag tttgaagcta ctgtcacacg ccttcgccca tcatctcagg 900

gcctgaagga attcataaat gaggttaata ctcttgcatc attgcagcat ccgaacctct 960

gtagattgct aggatttcac gcaggtgatg gttcagagca tagaatgttg gtttatgaga 1020

ggctatacca tggaagcttg gaccgcttat tgtatgggag atctgatggg ccatcaattg 1080

attggaatac aagaatgaaa attgcaatat gtgctgcact aggtctttct ttcttgcatg 1140

aagaagggcc ttttcaggca atgtataatg aattttcaac agctaacatt cagattgaca 1200

aagatttcag tgcaaagctt tcaggatatg gttgtgttgg acatgttccg aaggaagaga 1260

tttcaagcag ttcatctgcc gttggaaacc tatcgatgga gacactggag aaaggaatgc 1320

ttactccgaa aagcaatgta tggagtttcg gaattttcct tctagagcta cttacaggaa 1380

gaaagaatct cgatagcccg tcacccaaag gaagagagaa atttggtgaa gtggagcagg 1440

cctttcctgt ctgataatca tcgtttgtca atgatcatgg atcctcaact taaaggtcgc 1500

tttccttcta aagcggcgag tacaatagct aacattgcac aaagatgcct tcaaatggag 1560

ccatcagaac gaccaaccat gggaacagtt gttgagcagc tgaaaaagat acaagatttg 1620

aagcattcta gcagattccc gctgcaagaa cctgcacaaa tgtcgagatc accaagtctt 1680

aacggtatca accaccctgc accaaggccg agtttctctc catcaccatc atccagagcc 1740

ctggtatctg tctcacctcc aagatggtcg ggagtgtcaa ttcaacttcc acctcgcgcg 1800

ttttcttcaa ccctctattt ggaggagctt gataggcaag aaagccgcaa gtcagcttca 1860

gcctctagga aggctagtgt tgaaggattt tgattgtcga tagtgttcat tttttatttg 1920

ttattctttt tttggtgtag ttcaacagag atttatagga gataaagatt tgtaatcatc 1980

cctcagtgtg atgcagagag gatgctcttt tgtacatagt gcaaaggttg gtccccttgg 2040

ttcaattagt tactccattt tg 2062

<210> 216
<211> 2039
<212> DNA
<213> Zea mays

<400> 216
ctcccccggc gtgcgctccc tcctgtctct cttggcgacg actgggcaac ggtcgcagct 60

gtatgttcga gcccttcgcc gacgacgttc accaccagga attttctcag ctgtgccaat 120

gatgggttgc ttcactgttc tcagatccaa gaagaagaaa agccattttg ataatcctct 180

tgtcccaagc aagaaatcag ttgatgcaag ggaaagtacg tcctctagac ttccggagcc 240

agaagttcat gtgccatctt tgcaatcagc tcctcctagt tttaggaaca gggtcaagat 300

```
atccgagtca tcaaatgaag tttcaaacag aaacagcagg gctcgcgtgc tgtctgctcc    360
atcagccctt attgtggttg atcagtttgg ctttccatat tcggaatacc gagatcaaga    420
tgactccagg gataaggaag gtttgacaaa ggggcatcgc ttttcaaatc ctttgcccct    480
tcctcttcct tcacgcgaag gacattcttt taggaactct ggtagcttca aagccagcaa    540
cgtaagcggg ccattggaga tgtctggccc tctcccattg cctctaaaga aatgtgatgg    600
acttagaatc ttctcttatg aggagatttc atctgcctgc caacgatttt ctagtgatca    660
gtgtgtttca gaaacacttg gttcaacatc atacaaggca acatttagag atgaatttat    720
tgatacgagg accactgaag caacagtagc tcgattactc ccctccactc agagtttgaa    780
ggagtttaaa acgcaagcga ccacattggc atcgcttcag catcccaatt tatgtaaact    840
gattggctat tatgcaaaag aagattctaa tgaaaggatg ttggtctatg aacggcttta    900
tcatggaagc ttagataagc tactctttgg aagatcagat ggtcgtttca tggactggtc    960
taaacgtttg aaggttgccc tgggtgctgc cagaggtcta gctttcttgc atgatgaagg   1020
accctttcag gccatgtaca gcgagttctc aactttgaac atccaaatag ataaaggttt   1080
cactgctaag ctttcaggat atggttgtgt tggcttcaat accgaggaga tatctaatgc   1140
acctgtgtct gcagcaaacc tttcggtgga gaccttggag aagggtttac tcactcccaa   1200
gagcaacgtc tggagctttg gagtcgtgtt actggagcta ataactggaa ggaagaacct   1260
tgatcccaat tattccaaag aagaacgcaa tattgtcaac tggagtaggc ctttccttac   1320
tgatgacagt cgcttgtctc ttatcatgga ctcccgtata aaagggcgct ttcccactaa   1380
ggctgctcgg atagtagcag acatcatttt gaaatgcctg cgtaaggatc catcggagag   1440
gcctaccatg agggatgttg tggaggccct agcaagagtc caggaaataa aggttccgtg   1500
cagatatcct ctgcaagaac cttctgctgc accaagaaaa gtgatgttga aatctacatc   1560
cctcaatggg attgtgcctc accatcctgt cataaccttc tctccatcgc cgccttcaca   1620
taaccagcac ttgatttcac caaggtcgtc gacatctgcc ttgtttcatc caagggcatg   1680
ctcttctacc ctggacgatc cgagtgtaag ttctataaag aaaacaccac ctattatgcg   1740
aaggtctagc gtcgaaggct tttgattgtc catattgttc ctttatttcc ttttcttgga   1800
tttatttgtt tctttgttag tagtgtagcg gtttttagct gtgttctgta ttgtaagaca   1860
agtggcctca tatgtagtgc ccttgggtct ggagtaaagc agaacaggat gaattgtaaa   1920
taggaccagg ttgtagatac ctttttttg tgctcgaatt gggtggaagc agcgtgccga   1980
gtgggccttg taacctcata atgcaataaa ctccgtttcc cagtttctgt aaaaaaaa     2039
```

<210>  217
<211>  1479
<212>  DNA
<213>  Hordeum vulgare

<400> 217
tgccgaattc ggcacgagaa agaggtccga ggggcttaag aacttctcgt acgatgagat 60

ttccactgct tgccagtggt tttctggcga tcatcgtgtc tcagaaactc tgacttcgac 120

atcatacaag gcattgttca gggatgattt cgttgaacca aagaagatgg aagcaatagt 180

agccaggtta ctcccttcca atcagagttt caaagaattt aaggcacaag taaatacgtt 240

ggcatcactt caacatccca atttatgtaa actcatcggc tatcacgcaa gagaagaatc 300

taatgaaagg atgttggtct atgagcggct ccatcatggc agtttagaca ggttactctt 360

tggaagaccg gaaggtcgtt tcatggactg gtctacacgt ttgaaggttg cccttggtgc 420

tgctaaaggt ctagctttcc tacacgatga aggacccttt caggcaatgt atgatgactt 480

ctcaacgtca aacatccaaa tagagaaaga tttcactgca aagctgtcgg gatatggttg 540

tgttggcttc aattctgacg aggaaagatc caaggcatct gtggctgcaa acctctcaga 600

ggaaaccttg gagaaaggcg tactgactcc gaagagcaac gtatggagct ttggagttgt 660

cttgctcgag ctaataacag gacggaagaa cctcgatgta cgttccacca aagaagaacg 720

taatattgtc aagtggggta ggcctttcct caccgacgat agtcgtctat ccctcatcat 780

ggatccccgt ataaaaggac gctttcctac caaggctgct cgaactgtgg cagacataat 840

tctgaagtgc cttcaaagag atccatcaga aaggcctacg atgagggccg tcgtggaggc 900

cctaacaagc gttcaggaca taaaggtccc ctgccggtat cctcttcaag taccgtcccg 960

ccgcacccga ggaaagtgat gctaaagtct acgagcctca atggcattgt tcctcagcat 1020

cccgtcatga ccttctcgcc gtcgcctcct tcgcgcaacc agcacctggc gtcgccaaga 1080

tcatcgactt ccgcgcttct tcccccaagg acctgctctt ccatcatcac cctggattac 1140

cccagggtaa gctcggtcaa gaagtcgcct tccggtatcc tgcggagacc tggcgtcgag 1200

ggtttttgat tgtccataca tggtcggatt tcttctcttt gccttggatt tatttgttgt 1260

tttggttagc ctagcgagtg gtctcagtgc tatgtgatat gtatatgttg gaaagacatc 1320

tgaaaaaagg gcagagtgaa gtgtagatag tagaaccagc tttgtagata cttgtcgttc 1380

tctgattgga tgggggtcat ggaagcagtg tgtgttagag tggggcttgt aaccccatta 1440

ttatgcaata aacgtgggtt ggtcggtcgt atttgctcg 1479

<210> 218
<211> 769
<212> DNA
<213> Glycine max

<400> 218
gtaaattgct aggatttcat gcacgagagg gttcagaaca tagaatgttg gtttatgaaa 60

ggctatacca tggaagcttg gatcgcttat tgtatgggag atctgatggg ccatcaattg 120

attggaatac aagaatgaaa attgctatat gtgctgcaca aggtctaacc ttcttgcacg 180

aagaggggcc ttttcaggct atgtataatg agttctcaac agccaacata cagattgaca 240

aagatttcag cgcaaagctt tcaggatatg gttgtgttgg acatattccc gaagaagaga 300

tttcaagcag ctcatctgct gttggaaacc tatcaatgga aacactggag aaaggaatgc 360

tcactccaaa gagcaacgta tggagttttg gaatttttct tctggagcta cttactggaa 420

gaaagaatct tgatagccgt caccccaagg aagagaggaa tttagtcaag tggagccggc 480

ctttcttagc cgataactac cgtctgtcgt tgatcatgga tcctcaactc aaaggccgct 540

ttccttctaa agcagcaaga acaatagctg atattgcaca aagatgcctt caaaaggagc 600

catcagacag acctaccatg aggactgttg ttgagcatct caagataata caggatttga 660

aatattcgtg ccggttccct ctgcaagaac cagcatcaaa ctctggaaaa catatgtcaa 720

gatcaccaag tctcaatggc atcatctgcc ctgcaccaag ggtgagttt 769

<210> 219
<211> 1150
<212> DNA
<213> Solanum tuberosum

<400> 219
ttcttagctc atcgaaacgt taaaccttac aatacttcaa acatcgatca atacaatttg 60

ctgaagcacg caatttttca aggactgtat aaacaacaaa gatgggttgt ttcacagttt 120

taaaaagtaa gaagaagaag tctgaacaga gtattcacat caaacgtgtg aatcctcagg 180

aacattctcc tactgcattg cccgagcctc aagtgcagac acggtcgttg cagtcggcac 240

ctccaagttt tagaactaga gtaaaacctg tacagtcgag taatagagtt acaagcagta 300

gggcgcgggc actctctgcc ccatctagcc tggactcagc agaacaagat gtagcatcaa 360

atgaatgtga ggaacatgat gagttcaaga gtcgtattgg ttcaatcaag gagtaccaat 420

caccaagtcc tcagcctctt cctcttccat ctccacagag tgccgctgcc actctcaaga 480

ctatgggaag ctttaaagtt ggcaacgcca gcggtccgtt aaatgcctct ggacccctgc 540

cactgcctcc tacactgcct tcaacattgc cttctactgg agcactcagg aacttctcat 600

ttgaagaact tgctgctgcc tgccaccgct tctctcctga acggtgtatg tcagaaggtc 660

tctcttctgt tatttacaga gcttcttttg gagatgatgc aactggtaca aagaagcttg 720

aagccactgt aacccggctt catccttctt cgcaggggtt gaaggaattt gtgactgagg 780

tgaacacact agcttctttg caacatccat cactttgtaa actgattggt ttccatgcca 840

cggggaaggt tccgagcaca gaatgttggt ttatgaaagg ctttttcatg gaagcttaga 900

ccggcttttg tttgggaggt ccgatggtcc cccgatagac tggaatgctc gaacgaaaat 960

tgctttatgt gctgctcaag gtctcacatt cctgcatgag gaaggacctt ttcaggcaat 1020

gttccatgaa ttttccactg caaatataca aattgataag gattgcagtg caaagctctc 1080

gggatatgga tgcattactc ctataccaga gacagacata tcatgcagtt cagctgccct 1140

ggcaatctct 1150

<210> 220
<211> 910
<212> DNA
<213> Lycopersicon esculentum

<400> 220
catggaagct tacaccggct tttatttggg aggtcggatg gtcccccaat agactggaat 60

gctcgaataa aaattgcttt atgtgctgct caaggtctca cattcctgca tgaggaagga 120

ccttttcagg caatgttcca tgaattttcc actgcaaata tacaaattga taaggattgc 180

agtgcaaagc tctcgggata tggatgcatt actcatatac aagagacaga catatcatgc 240

agttcagctg ccctggcaaa tctctctgag gagactctgg agcgaggatt ggtaactcca 300

aagagtaatg tttggagttt tgggattgtt cttcttgagc tgctgactgg ccggaagaat 360

ttaagcagtc ggcatccaaa ggaagagagg aatttagtga agtggagcaa gctttttcta 420

gctgatgaca gtagattatc gctaatcatg gaccctcagt taaaaggtcg gttccctgcc 480

aaagcagcaa gaactgtggc tgatattgct caaagatgtc tgcaaaagga tccatctgaa 540

aggcccacca tgagaactgt agtggagcaa ctcaagactg tacaagttat gaagtaccct 600

tctcggtttc ctcttcaaga gccaagggca gttggtgtaa aacacatgtc aaagtgtccg 660

agcctaaatg gaattattac cccaacatca agattgagct tctccccttc accaccaacc 720

caccctatat ctatttctcc gacaaggaca gctgctccac tgctgtctct accttcatgt 780

tcctccatcc tctctatgga ggactttgat cgattggaaa atcgaaggcc atcatcttca 840

tctgttcgga ggtctagtgt cgaaggattt tgatcgattg tagagcactt ttttcttcaa 900

attgcttttc 910

<210> 221
<211> 681
<212> DNA
<213> Lycopersicon esculentum

<400> 221
gagaggcttt ttcatggaag cttagacaga cttttgttcg gaagatcaga tggcccctct 60

atagattgga atgcgagaac caaaattgcc ctatgtgctg cacaaggtct tacatttcta 120

catgaggagg gaccttttca ggcaatgttc caagaatttt caactggaaa tatacaaatc 180

gacaaggatt ttagtgcaaa gctctcgggg tatggatgca ttacgaatat acaagagacg 240

gagatatctt gcaattcaat cgccctggcg aatctctcac aggagacact ggagagaggg 300

ttgataactc ctaagagcaa tgtttggagt ttcgggattg ttcttttaga actgctcact 360

ggccggaaga atcttgatgg tcggtattca aaggaagaga ggaatctagt caagtggagt 420

aggcctttcc ttgctgatga tggtagatta tcgcttatca tggatcctca gcttaaagga 480

cggtttcccg caaaagcagc ccgtacagtg gctgatattg cccaaagatg cctgcaaaag 540

gatccatctg aaaggcccac catgagaact atcttggacc aactcaaatc cgtacaagtg 600

atgaagtgcc cttcacggtt tcctctgcaa gaaccagcgg ttgttggtgg taaacacatg 660

tcaaagtctc caagcatgaa t 681

<210> 222
<211> 741
<212> DNA
<213> Triticum aestivum

<400> 222
tttcctccca aggctgctag gactgtggca gacatcattc tgaagtgcct tcatagggat 60

ccatccgaaa ggcctacgat gagggccgtc gtggagtctc tagcaagcgt ccaggacata 120

aaggtcccct gccggtatcc tcttcaagta ccgtccgccg caccgaggaa agtgatgcta 180

aaatccacga gtctcaacgg cattgttcct cagcatcctg tcatgacctt ctcgccgtcg 240

cctccttcgc gcaaccagca cctggtgtca ccgagatcat cgacgtctgc gctgcttccc 300

ccaaggaact gctcttccac catcaccctg gactacccca gggtaagctc ggtcaagaaa 360

tcgcccccca acatcatgcg gagacctggc gtcgagggtt tttgattgtc catatagtgt 420

cggattttct tctctttgcc ttgatttatt tgttgttttg gttagcctag cgagtgatct 480

cagtgctatg ggatatacta tgttgcaagg acatgtgaaa aagggcagag tgaagcgtag 540

atagtagaac cagcttgtag atacttgtcg ttctctgatt ggatgggggt catggaagaa 600

gtgtgtgttg agtggggctt gtaaccccac tatgcaataa acgtgggttg gttggtcggt 660

cggtatttgc tcgccgtcaa acatttcagc tgtttctttc ttctcttgaa tgctaagttt 720

tgttgctgct agtcgtggaa t 741

<210> 223
<211> 2039
<212> DNA
<213> Zea mays

<400> 223
ctcccccggc gtgcgctccc tcctgtctct cttggcgacg actgggcaac ggtcgcagct 60

gtatgttcga gcccttcgcc gacgacgttc accaccagga attttctcag ctgtgccaat 120

gatgggttgc ttcactgttc tcagatccaa gaagaagaaa agccattttg ataatcctct 180

tgtcccaagc aagaaatcag ttgatgcaag ggaaagtacg tcctctagac ttccggagcc 240

agaagttcat gtgccatctt tgcaatcagc tcctcctagt tttaggaaca gggtcaagat 300

atccgagtca tcaaatgaag tttcaaacag aaacagcagg gctcgcgtgc tgtctgctcc 360

atcagccctt attgtggttg atcagtttgg ctttccatat tcggaatacc gagatcaaga 420

tgactccagg gataaggaag gtttgacaaa ggggcatcgc ttttcaaatc ctttgcccct 480

tcctcttcct tcacgcgaag gacattcttt taggaactct ggtagcttca aagccagcaa 540

cgtaagcggg ccattggaga tgtctggccc tctcccattg cctctaaaga aatgtgatgg 600

acttagaatc ttctcttatg aggagatttc atctgcctgc caacgatttt ctagtgatca 660
gtgtgtttca gaaacacttg gttcaacatc atacaaggca acatttagag atgaatttat 720
tgatacgagg accactgaag caacagtagc tcgattactc ccctccactc agagtttgaa 780
ggagtttaaa acgcaagcga ccacattggc atcgcttcag catcccaatt tatgtaaact 840
gattggctat tatgcaaaag aagattctaa tgaaaggatg ttggtctatg aacggcttta 900
tcatggaagc ttagataagc tactctttgg aagatcagat ggtcgtttca tggactggtc 960
taaacgtttg aaggttgccc tgggtgctgc cagaggtcta gctttcttgc atgatgaagg 1020
acccttcag gccatgtaca gcgagttctc aactttgaac atccaaatag ataaaggttt 1080
cactgctaag ctttcaggat atggttgtgt tggcttcaat accgaggaga tatctaatgc 1140
acctgtgtct gcagcaaacc tttcggtgga gaccttggag aagggtttac tcactcccaa 1200
gagcaacgtc tggagctttg gagtcgtgtt actggagcta ataactggaa ggaagaacct 1260
tgatcccaat tattccaaag aagaacgcaa tattgtcaac tggagtaggc ctttccttac 1320
tgatgacagt cgcttgtctc ttatcatgga ctcccgtata aaagggcgct ttcccactaa 1380
ggctgctcgg atagtagcag acatcatttt gaaatgcctg cgtaaggatc catcggagag 1440
gcctaccatg agggatgttg tggaggccct agcaagagtc caggaaataa aggttccgtg 1500
cagatatcct ctgcaagaac cttctgctgc accaagaaaa gtgatgttga aatctacatc 1560
cctcaatggg attgtgcctc accatcctgt cataaccttc tctccatcgc cgccttcaca 1620
taaccagcac ttgatttcac caaggtcgtc gacatctgcc ttgtttcatc caagggcatg 1680
ctcttctacc ctggacgatc cgagtgtaag ttctataaag aaaacaccac ctattatgcg 1740
aaggtctagc gtcgaaggct tttgattgtc catattgttc ctttatttcc ttttcttgga 1800
tttatttgtt tctttgttag tagtgtagcg gtttttagct gtgttctgta ttgtaagaca 1860
agtggcctca tatgtagtgc ccttgggtct ggagtaaagc agaacaggat gaattgtaaa 1920
taggaccagg ttgtagatac ctttttttg tgctcgaatt gggtggaagc agcgtgccga 1980
gtgggccttg taacctcata atgcaataaa ctccgtttcc cagtttctgt aaaaaaaaa 2039

<210> 224
<211> 768
<212> DNA
<213> Lactuca sativa

<400> 224
accgactgat ggccgctagt cggggaagaa cacgaagaat ccaaaacccg tggcagcggc 60
ggaggcggag gcgggttgcc gcctgtcccg cagccgctgc cgcttcctgc accgcattca 120
cgccaacttc gaaaaccatc gcggaatgct tttaaagatt gagtggcggt gagttgggca 180
tgctgccagc ctcttaatac ttccggaccc tctgccactt ccgccatcgg gaaccaccca 240
tcttccgcca atgattccgg catctttacc gacatctgga acacttaaga acttcacata 300

ttgaagaaat cgcagctgct tgccacaaca ttttcaccct gacagatgcg tgtctgaagg 360

tctttcttct gttatgtata gagcttcttt tggagaagat acttctaatt taaagaatct 420

tcaagccact gttaccagtc tccatccttc aactcagggt ttgaaggaat ttgtgagtga 480

agtgaacacg ctagcatcat tgcaacaccc ttatctctgt aaatagattg gtttccatgc 540

gcgtgaggga tctgatagaa gaatgttggt ttacgagagg ctttttcatg gaagcttaga 600

tcggctttta tatggtacaa cagatggccc acctattgat tgcaatgcaa gaatgaaagt 660

cgcactctgt gctgctcaag ggcttacttt tttgcatgag gaaggaccat ttcaggcgat 720

gtttcatgaa ttttccactg ctaatataca aattgataaa gattttag 768

<210> 225
<211> 1637
<212> DNA
<213> Gossypium spp.

<400> 225
ctttttcctc tcaaacctct ctcacactga taaacttttc tctccattac ctttctttga 60

cttgcaaagt tgttctcctc aaactaacca aacaagggat tctgggtttt cagctttctc 120

tgttttccta ctctctttcg cctttgcatg acctgcttac atagaaacat aaaagtccga 180

aatacaattt aggtggtttg tatataagat ggggtgtttt acagttttga gaagcaacaa 240

gaaaaagtcc aaacagtcag tttttgttaa acacattgct cataaagagc atattcctac 300

catgctgcct gagccccaaa ttcagacacg atcactgcaa tctgcacccc caagttttat 360

aaccagagta aaaccaattc aatctaataa caaggcaagc tgcaatagga cacgtgcatt 420

atctgctccg tcaagtcttg atgctgctga gcaagatgac cttgcgtcag ttgaatttga 480

agaacaagaa gagttgaaga gtcgtgttgg tttagtcaag gaacagaagt catcaagtcc 540

acagcctctt ccacttccat ctccccacag tactgcgctg aagacaatgg gaagttttaa 600

agcagggaat gttagtggcc ctctttttgc ttcgggacca ttacccctgc ctccctctgg 660

aacactacgt aacttcgcct acgaagaaat tgcagctgct tgccatcatt tctcttctga 720

tcgatgcaca tctgagggtc tatcttctgt tatgtacaag gcatccttcg gagatgacac 780

atcaagttca aagaagtttg aagctactgt tactcgcctt cacccatcca ctcagggttt 840

aagggaattt ataaacgaag taaacactct tgcatcattg caacatccaa atctctgtaa 900

attgcttggg taccatgcac gtgataattc agaacaacga atgttggtct atgagaggtt 960

atttcatgga agcttagacc ggcttttata cgggagatcg gatggaccgc cacttgattg 1020

gaatactcgc atgaaaattg ctttatgttc tgcacagggt cttactttct tgcatgagga 1080

aggaccattt caggcaatgt acaatgaatt ttcgactgcc aacatacaga tcgacaagga 1140

tttcagtgca aagctctcag gatatgggtg cgttggtcat atcccagaga cagaagagat 1200

ctccagtaat tcagttgctg tggcaaatat atccgtagag actctggaga gagggcgact 1260

aactccaaag agcaatgttt ggagttttgg gatcattcta cttgaattac tcactggccg 1320

gaagaacctt gacaaccgtt atcccaagga agagaggaac ctagtgaagt ggagccggcc 1380

attcctagcc gacaattgta gattgtcact catcatggat cctcagctca aaggtcgctt 1440

tcctatgaaa gctgcccgta cggtggctga cattgcacaa aggtgtctcc agatggaccc 1500

atcagagagg ccaaccatga gaaccatcgt tgagcatctc aaaatcatcc aagacctgaa 1560

atactcttgt cggtttcctc tgcaagatcc agcagcaatt gctggaaaac agatgtcaag 1620

gtcaccgagt ctcaacg 1637

<210> 226
<211> 2193
<212> DNA
<213> Oryza sativa

<400> 226
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag atttttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctcctcctc ccatctataa attcctcccc ccttttcccc tctctatata 1020

ggaggcatcc aagccaagaa gagggagagc accaaggaca cgcgactagc agaagccgag 1080

cgaccgcctt cttcgatcca tatcttccgg tcgagttctt ggtcgatctc ttccctcctc 1140

cacctcctcc tcacagggta tgtgcccttc ggttgttctt ggatttattg ttctaggttg 1200

tgtagtacgg gcgttgatgt taggaaaggg gatctgtatc tgtgatgatt cctgttcttg 1260

gatttgggat agaggggttc ttgatgttgc atgttatcgg ttcggtttga ttagtagtat 1320

ggttttcaat cgtctggaga gctctatgga aatgaaatgg tttagggtac ggaatcttgc 1380

gattttgtga gtacctttg tttgaggtaa aatcagagca ccggtgattt tgcttggtgt 1440

aataaaagta cggttgtttg gtcctcgatt ctggtagtga tgcttctcga tttgacgaag 1500

ctatcctttg tttattccct attgaacaaa aataatccaa ctttgaagac ggtcccgttg 1560

atgagattga atgattgatt cttaagcctg tccaaaattt cgcagctggc ttgtttagat 1620

acagtagtcc ccatcacgaa attcatggaa acagttataa tcctcaggaa caggggattc 1680

cctgttcttc cgatttgctt tagtcccaga attttttttc ccaaatatct taaaaagtca 1740

ctttctggtt cagttcaatg aattgattgc tacaaataat gcttttatag cgttatccta 1800

gctgtagttc agttaatagg taatacccct atagtttagt caggagaaga acttatccga 1860

tttctgatct ccatttttaa ttatatgaaa tgaactgtag cataagcagt attcatttgg 1920

attatttttt ttattagctc tcaccccttc attattctga gctgaaagtc tggcatgaac 1980

tgtcctcaat tttgttttca aattcacatc gattatctat gcattatcct cttgtatcta 2040

cctgtagaag tttctttttg gttattcctt gactgcttga ttacagaaag aaatttatga 2100

agctgtaatc gggatagtta tactgcttgt tcttatgatt catttccttt gtgcagttct 2160

tggtgtagct tgccactttc accagcaaag ttc 2193

<210> 227
<211> 56
<212> DNA
<213> Artificial sequence

<220>
<223> primer: prm06729

<400> 227
ggggacaagt ttgtacaaaa aagcaggctt aaacaatgat gggttgcttc actgtc 56

<210> 228
<211> 50
<212> DNA
<213> Artificial sequence

<220>
<223> primer: prm06730

<400> 228
ggggaccact ttgtacaaga aagctgggta tggacaatca aaaaccctca 50

<210> 229
<211> 1176
<212> DNA
<213> Arabidopsis thaliana

<400> 229
cttcaactgc tgcgtctagc tgtccttcct tcttcatttg cttcttcttc tctagctcag 60

cgcggatcgc tgcagtagta ccctgacgta gcctttcttc ttcctttact ttctcatctt 120

```
ctatctctca aaagaaaagc agacaacttt atttgcaaaa acagagtttt tttttcttat     180
cttgagaaag ttcaacagaa gatgatgttc gagaaagacg atctgggtct aagcttaggc     240
ttgaattttc caaagaaaca gatcaatctc aaatcaaatc catctgtttc tgttactcct     300
tcttcttctt cttttggatt attcagaaga tcttcatgga acgagagttt tacttcttca     360
gttccaaact cagattcgtc acaaaaagaa acaagaactt tcatccgagg aatcgacgtg     420
aacagaccac cgtctacagc ggaatacggc gacgaagacg ctggagtatc ttcacctaac     480
agtacagtct caagctctac agggaaaaga agcgagagag aagaagacac agatccacaa     540
ggctcaagag gaatcagtga cgatgaagat ggtgataact ccaggaaaaa gcttagactt     600
tccaaagatc aatctgctat tcttgaagag accttcaaag atcacagtac tctcaatccg     660
aagcagaagc aagcattggc taaacaatta gggttacgag caagacaagt ggaagtttgg     720
tttcagaaca gacgagcaag aacaaagctg aagcaaacgg aggtagactg cgagttctta     780
cggagatgct gcgagaatct aacggaagag aaccgtcggc tacaaaaaga agtaacggaa     840
ttgagagtac ttaagctctc tcctcagttc tacatgcaca tgagcccacc cactactttg     900
accatgtgcc cttcatgtga acacgtgtcg gtcccgccac cacaacctca ggctgctacg     960
tcagcgcacc accggtcgtt gccggtcaat gcgtgggctc ctgctacgag gatatctcac    1020
ggcttgactt ttgacgctct tcgtcctagg tcctaagtct tttacttgc aaccaaaggg    1080
cattttggtc gttttttaag tttcatggac cagatatgca tgtagttgtt aacatgtatg    1140
tattttctta gaaagaaaga aaaacagatt aatatt                                1176
```

<210> 230
<211> 284
<212> PRT
<213> Arabidopsis thaliana

<400> 230

```
Met Met Phe Glu Lys Asp Asp Leu Gly Leu Ser Leu Gly Leu Asn Phe
1               5                   10                  15

Pro Lys Lys Gln Ile Asn Leu Lys Ser Asn Pro Ser Val Ser Val Thr
            20                  25                  30

Pro Ser Ser Ser Ser Phe Gly Leu Phe Arg Arg Ser Ser Trp Asn Glu
        35                  40                  45

Ser Phe Thr Ser Ser Val Pro Asn Ser Asp Ser Ser Gln Lys Glu Thr
    50                  55                  60

Arg Thr Phe Ile Arg Gly Ile Asp Val Asn Arg Pro Pro Ser Thr Ala
65                  70                  75                  80
```

Glu Tyr Gly Asp Glu Asp Ala Gly Val Ser Ser Pro Asn Ser Thr Val
85 90 95

Ser Ser Ser Thr Gly Lys Arg Ser Glu Arg Glu Glu Asp Thr Asp Pro
100 105 110

Gln Gly Ser Arg Gly Ile Ser Asp Asp Glu Asp Gly Asp Asn Ser Arg
115 120 125

Lys Lys Leu Arg Leu Ser Lys Asp Gln Ser Ala Ile Leu Glu Glu Thr
130 135 140

Phe Lys Asp His Ser Thr Leu Asn Pro Lys Gln Lys Gln Ala Leu Ala
145 150 155 160

Lys Gln Leu Gly Leu Arg Ala Arg Gln Val Glu Val Trp Phe Gln Asn
165 170 175

Arg Arg Ala Arg Thr Lys Leu Lys Gln Thr Glu Val Asp Cys Glu Phe
180 185 190

Leu Arg Arg Cys Cys Glu Asn Leu Thr Glu Glu Asn Arg Arg Leu Gln
195 200 205

Lys Glu Val Thr Glu Leu Arg Ala Leu Lys Leu Ser Pro Gln Phe Tyr
210 215 220

Met His Met Ser Pro Pro Thr Thr Leu Thr Met Cys Pro Ser Cys Glu
225 230 235 240

His Val Ser Val Pro Pro Pro Gln Pro Gln Ala Ala Thr Ser Ala His
245 250 255

His Arg Ser Leu Pro Val Asn Ala Trp Ala Pro Ala Thr Arg Ile Ser
260 265 270

His Gly Leu Thr Phe Asp Ala Leu Arg Pro Arg Ser
275 280

<210> 231
<211> 3171
<212> DNA
<213> Arabidopsis thaliana

<400> 231
gacaatccga aattaggtaa ccatgttcat tgaatttatc gtttcaacac attcgaatca 60

ctgagtccta cgtttcagtt taattccctg atgcacattt tgaattcggt ttgcgtattg 120

gtcttcgagt tttccgtcgg cggccatgga cgcgttcaag gaggagatag aaatcgggag 180

ctcggtggag tctttaatgg agctattgga ttcgcagaag gtgcttttc atagccagat 240

cgatcagctc caagatgtcg tcgttgcgca atgcaaactt accggcgtta atccccttgc 300
gcaagaaatg gctgctggtg ctttgtccat taaaattgga aagcggccaa gagacttgtt 360
gaatcctaag gctgttaagt atctacaagc agttttcgca attaaagatg ctattagtaa 420
gagggaatct cgggagataa gtgctttatt tggcatcaca gtcgcccagg ttcgagaatt 480
ttttgttact caaaagacaa gagtgaggaa acaggtgagg ctttcaaggg agaaagtagt 540
tatgtccaat acgcatgctt tacaagatga tggtgttccg gaaaataaca atgccacaaa 600
tcatgttgaa cccgttccct tgaactctat acatccggag gcatgttcta taagctgggg 660
tgaaggtgaa acagtggcac ttattccacc tgaagatatt ccacctgaca tcagcgattc 720
agacaaatac tttgttgaga atatattttc tctgctgcgt aaagaggaaa cattttcagg 780
ccaggtgaaa ctaatggagt ggatcatgca gatacaagat gcttctgtgc tgatctggtt 840
tttatcaaaa ggagggggttt tgatacttac aacatggtta agtcaagctg ctagtgaaga 900
gcaaacaagt gtcttacttc ttatcctgaa ggttctttgt catttacctc tccacaaagc 960
atctcctgaa aatatgtctg ccatattaca aagcgttaat ggacttcgtt tctataggat 1020
atcagacata tcaaacaggg caaaaggttt gttatcaagg tggaccaagt tatttgcgaa 1080
aatccaagct atgaagaaac aaaatcgtaa cagttcgcaa attgattcgc agagtcaatt 1140
gcttctgaaa cagagtattg ctgaaatcat gggtgatagt agcaatcctg aagatattct 1200
tagtctctca aatggaaagt cagagaatgt caggaggatt gaatcgtcac agggtccaaa 1260
actgttgctt acttctgcag atgattccac caagaaacac atgcttggtt caaatccatc 1320
gtataacaaa gaacgcagga aagtacagat ggtggaacaa ccaggccaaa aagctgctgg 1380
aaagagtccg cagacagtaa gaataggaac ttcaggtcga agccgcccaa tgtctgctga 1440
tgatattcag aaagcaaaga tgcgtgccct ttatatgcag agcaagaaca gtaaaaagga 1500
tcctttacca agtgccattg gtgattcgaa aatcgttgct cctgagaagc ccttggctct 1560
tcattcagcc aaggattctc cacctattca gaacaatgaa gctaagactg aagacacacc 1620
tgtactctcg actgttcagc ccgtcaatgg attttcaact attcagcccg tcaatggacc 1680
ttcagctgtt cagcccgtca atggaccttt ggctgttcag cctgtcaatg gaccttcggc 1740
tcttcagccc gtcaatggac cttcggccgt aattgtcccg gtacaagctg atgaaattaa 1800
aaaaccttca acacctccta aaagcatttc tagtaaggtg ggagttatga tgaaaatgag 1860
ttcacaaact attctcaaga attgcaagag aaaacagatt gattggcatg taccaccagg 1920
aatggaactt gacgaactct ggagagtagc cgctggtggt aatagcaagg aggctgatgt 1980
tcagagaaac agaaaccggc gagaaagaga aacaacatat cagtctcttc aaactatacc 2040
gttgaaccct aaagaaccat gggataggga aatggactat gatgacagtt tgacccctga 2100
aattccatct caacagccac cagaagaaag tttaacggaa ccacaggatt cacttgatga 2160

acgaagaatt gctgctggtg ctgccacaac ctcttcatct ctaagcagtc ctgaacctga 2220

tctcgagtta ttagctgcgt tacttaagaa cccagatctt gtttatgcac taacttcggg 2280

aaaacccagt aatttagccg gccaagatat ggtaaaactg cttgatgtga ttaagactgg 2340

tgcaccaaac tcaagcagta gctcaaataa acaggttgaa gaaagggtcg aagtttccct 2400

tccatctccc actccatcaa ctaatcctgg aatgagtgga tggggacaag aagggattcg 2460

gaatccattt tcaaggcaaa accaagttgg tactgcagtt gctagatcgg gtacacagct 2520

tcgtgttggt tcaatgcaat ggcatcaaac aaatgaacaa tcaatcccac gacatgctcc 2580

atcagcatac agtaactcga tcacattggc tcacacagaa agagaacagc aacaatatat 2640

gcaaccaaaa cttcatcaca atttacattt tcaacaacaa caacaacaac caatctcaac 2700

aacctcgtat gcagttaggg aaccagtagg acaaatggga acaggtacat cgagttcatg 2760

gaggagtcag cagagtcaga acagttacta ctcacatcaa gaaaacgaga ttgcatcggc 2820

ttcacaagtt acttcatacc aagggaatag ccagtacatg agtagcaatc caggatatga 2880

atcatggagt cctgataata gcccaagtag gaaccagctt aacatgaggg gacaacaaca 2940

acaagcatca aggaaacatg attcttctac tcatccatat tggaaccaaa acaaaagatg 3000

gcgttaaacc atatataaga ctatggttct ttggtcctca atatttgctt gtcatggttg 3060

aattagcatt atactctgtt cagacacaga ttttcatttt taactcacaa cagattcatc 3120

agagataata aactaaaaga aagaatgaat ggataaagtt attactgtct c 3171

<210> 232
<211> 953
<212> PRT
<213> Arabidopsis thaliana

<400> 232

Met Asp Ala Phe Lys Glu Glu Ile Glu Ile Gly Ser Ser Val Glu Ser
1 5 10 15

Leu Met Glu Leu Leu Asp Ser Gln Lys Val Leu Phe His Ser Gln Ile
20 25 30

Asp Gln Leu Gln Asp Val Val Val Ala Gln Cys Lys Leu Thr Gly Val
35 40 45

Asn Pro Leu Ala Gln Glu Met Ala Ala Gly Ala Leu Ser Ile Lys Ile
50 55 60

Gly Lys Arg Pro Arg Asp Leu Leu Asn Pro Lys Ala Val Lys Tyr Leu
65 70 75 80

Gln Ala Val Phe Ala Ile Lys Asp Ala Ile Ser Lys Arg Glu Ser Arg
85 90 95

Glu Ile Ser Ala Leu Phe Gly Ile Thr Val Ala Gln Val Arg Glu Phe
100 105 110

Phe Val Thr Gln Lys Thr Arg Val Arg Lys Gln Val Arg Leu Ser Arg
115 120 125

Glu Lys Val Val Met Ser Asn Thr His Ala Leu Gln Asp Asp Gly Val
130 135 140

Pro Glu Asn Asn Asn Ala Thr Asn His Val Glu Pro Val Pro Leu Asn
145 150 155 160

Ser Ile His Pro Glu Ala Cys Ser Ile Ser Trp Gly Glu Gly Glu Thr
165 170 175

Val Ala Leu Ile Pro Pro Glu Asp Ile Pro Pro Asp Ile Ser Asp Ser
180 185 190

Asp Lys Tyr Phe Val Glu Asn Ile Phe Ser Leu Leu Arg Lys Glu Glu
195 200 205

Thr Phe Ser Gly Gln Val Lys Leu Met Glu Trp Ile Met Gln Ile Gln
210 215 220

Asp Ala Ser Val Leu Ile Trp Phe Leu Ser Lys Gly Gly Val Leu Ile
225 230 235 240

Leu Thr Thr Trp Leu Ser Gln Ala Ala Ser Glu Glu Gln Thr Ser Val
245 250 255

Leu Leu Leu Ile Leu Lys Val Leu Cys His Leu Pro Leu His Lys Ala
260 265 270

Ser Pro Glu Asn Met Ser Ala Ile Leu Gln Ser Val Asn Gly Leu Arg
275 280 285

Phe Tyr Arg Ile Ser Asp Ile Ser Asn Arg Ala Lys Gly Leu Leu Ser
290 295 300

Arg Trp Thr Lys Leu Phe Ala Lys Ile Gln Ala Met Lys Lys Gln Asn
305 310 315 320

Arg Asn Ser Ser Gln Ile Asp Ser Gln Ser Gln Leu Leu Leu Lys Gln
325 330 335

Ser Ile Ala Glu Ile Met Gly Asp Ser Ser Asn Pro Glu Asp Ile Leu
340 345 350

Ser Leu Ser Asn Gly Lys Ser Glu Asn Val Arg Arg Ile Glu Ser Ser
355 360 365

Gln Gly Pro Lys Leu Leu Leu Thr Ser Ala Asp Asp Ser Thr Lys Lys
370 375 380

His Met Leu Gly Ser Asn Pro Ser Tyr Asn Lys Glu Arg Arg Lys Val
385 390 395 400

Gln Met Val Glu Gln Pro Gly Gln Lys Ala Ala Gly Lys Ser Pro Gln
405 410 415

Thr Val Arg Ile Gly Thr Ser Gly Arg Ser Arg Pro Met Ser Ala Asp
420 425 430

Asp Ile Gln Lys Ala Lys Met Arg Ala Leu Tyr Met Gln Ser Lys Asn
435 440 445

Ser Lys Lys Asp Pro Leu Pro Ser Ala Ile Gly Asp Ser Lys Ile Val
450 455 460

Ala Pro Glu Lys Pro Leu Ala Leu His Ser Ala Lys Asp Ser Pro Pro
465 470 475 480

Ile Gln Asn Asn Glu Ala Lys Thr Glu Asp Thr Pro Val Leu Ser Thr
485 490 495

Val Gln Pro Val Asn Gly Phe Ser Thr Ile Gln Pro Val Asn Gly Pro
500 505 510

Ser Ala Val Gln Pro Val Asn Gly Pro Leu Ala Val Gln Pro Val Asn
515 520 525

Gly Pro Ser Ala Leu Gln Pro Val Asn Gly Pro Ser Ala Val Ile Val
530 535 540

Pro Val Gln Ala Asp Glu Ile Lys Lys Pro Ser Thr Pro Pro Lys Ser
545 550 555 560

Ile Ser Ser Lys Val Gly Val Met Met Lys Met Ser Ser Gln Thr Ile
565 570 575

Leu Lys Asn Cys Lys Arg Lys Gln Ile Asp Trp His Val Pro Pro Gly
580 585 590

Met Glu Leu Asp Glu Leu Trp Arg Val Ala Ala Gly Gly Asn Ser Lys
595 600 605

Glu Ala Asp Val Gln Arg Asn Arg Asn Arg Arg Glu Arg Glu Thr Thr

610 615 620

Tyr Gln Ser Leu Gln Thr Ile Pro Leu Asn Pro Lys Glu Pro Trp Asp
625 630 635 640

Arg Glu Met Asp Tyr Asp Asp Ser Leu Thr Pro Glu Ile Pro Ser Gln
645 650 655

Gln Pro Pro Glu Glu Ser Leu Thr Glu Pro Gln Asp Ser Leu Asp Glu
660 665 670

Arg Arg Ile Ala Ala Gly Ala Ala Thr Thr Ser Ser Ser Leu Ser Ser
675 680 685

Pro Glu Pro Asp Leu Glu Leu Leu Ala Ala Leu Leu Lys Asn Pro Asp
690 695 700

Leu Val Tyr Ala Leu Thr Ser Gly Lys Pro Ser Asn Leu Ala Gly Gln
705 710 715 720

Asp Met Val Lys Leu Leu Asp Val Ile Lys Thr Gly Ala Pro Asn Ser
725 730 735

Ser Ser Ser Ser Asn Lys Gln Val Glu Glu Arg Val Glu Val Ser Leu
740 745 750

Pro Ser Pro Thr Pro Ser Thr Asn Pro Gly Met Ser Gly Trp Gly Gln
755 760 765

Glu Gly Ile Arg Asn Pro Phe Ser Arg Gln Asn Gln Val Gly Thr Ala
770 775 780

Val Ala Arg Ser Gly Thr Gln Leu Arg Val Gly Ser Met Gln Trp His
785 790 795 800

Gln Thr Asn Glu Gln Ser Ile Pro Arg His Ala Pro Ser Ala Tyr Ser
805 810 815

Asn Ser Ile Thr Leu Ala His Thr Glu Arg Glu Gln Gln Gln Tyr Met
820 825 830

Gln Pro Lys Leu His His Asn Leu His Phe Gln Gln Gln Gln Gln Gln
835 840 845

Pro Ile Ser Thr Thr Ser Tyr Ala Val Arg Glu Pro Val Gly Gln Met
850 855 860

Gly Thr Gly Thr Ser Ser Ser Trp Arg Ser Gln Gln Ser Gln Asn Ser
865 870 875 880

```
Tyr Tyr Ser His Gln Glu Asn Glu Ile Ala Ser Ala Ser Gln Val Thr
                885                 890                 895

Ser Tyr Gln Gly Asn Ser Gln Tyr Met Ser Ser Asn Pro Gly Tyr Glu
            900                 905                 910

Ser Trp Ser Pro Asp Asn Ser Pro Ser Arg Asn Gln Leu Asn Met Arg
        915                 920                 925

Gly Gln Gln Gln Gln Ala Ser Arg Lys His Asp Ser Ser Thr His Pro
    930                 935                 940

Tyr Trp Asn Gln Asn Lys Arg Trp Arg
945                 950


<210>  233
<211>  501
<212>  DNA
<213>  Oryza sativa

<400>  233
atggagcagc agcttcctct tcttgcacct gactctaagg ctgccacgtc gtccccttg      60

tgcctcacct tggacaaccc aacctccaca tccacctcgc cggcggtgcc gtcgtcggcg    120

ccgccgccgg cagccgcctt ggaaccttct agacaatctt tccatgagag ggaaacggat    180

gcaatcaaag ccaagatcat gtcgcacccc ctctacccgg ctctcctcag agccttcata    240

gattgccaga aggtcggagc tccgccggag gtcgtcggcc ggctttccgc cctcgccggc    300

gagctcgact cgcgtgcaga agacaggtac cttcaagggc agtcgtcaga cccggagctc    360

gacgagttta tggaaaccta cattgatatg ctggtgagct acaggcagga gctgacaaga    420

ccaattcaag aggccgacca gttcttcaga aacatggagg cacagatcga ctcgtttaca    480

ctagagatgt gcagtttctg a                                              501


<210>  234
<211>  166
<212>  PRT
<213>  Oryza sativa

<400>  234

Met Glu Gln Gln Leu Pro Leu Leu Ala Pro Asp Ser Lys Ala Ala Thr
1               5                   10                  15

Ser Ser Pro Leu Cys Leu Thr Leu Asp Asn Pro Thr Ser Thr Ser Thr
            20                  25                  30

Ser Pro Ala Val Pro Ser Ser Ala Pro Pro Pro Ala Ala Ala Leu Glu
        35                  40                  45
```

Pro Ser Arg Gln Ser Phe His Glu Arg Glu Thr Asp Ala Ile Lys Ala
    50                  55                  60

Lys Ile Met Ser His Pro Leu Tyr Pro Ala Leu Leu Arg Ala Phe Ile
65                  70                  75                  80

Asp Cys Gln Lys Val Gly Ala Pro Pro Glu Val Val Gly Arg Leu Ser
                85                  90                  95

Ala Leu Ala Gly Glu Leu Asp Ser Arg Ala Glu Asp Arg Tyr Leu Gln
            100                 105                 110

Gly Gln Ser Ser Asp Pro Glu Leu Asp Glu Phe Met Glu Thr Tyr Ile
        115                 120                 125

Asp Met Leu Val Ser Tyr Arg Gln Glu Leu Thr Arg Pro Ile Gln Glu
    130                 135                 140

Ala Asp Gln Phe Phe Arg Asn Met Glu Ala Gln Ile Asp Ser Phe Thr
145                 150                 155                 160

Leu Glu Met Cys Ser Phe
                165

<210> 235
<211> 879
<212> DNA
<213> Oryza sativa

<400> 235
atggctcagg aggacgtcgg tcacctgagc gacgccggcc tggcgctggg cctgtccctc 60

ggcgggggag gaggagggac gaccgacgcg gcggcggcgc accgtggcgg ctgccggcgg 120

ccgtcgccgt cgtcgcagtg cccgccgctg gagccgtcgc tgaccctgag cttgcccgac 180

gacgcggcgg ccggcgcggc cgcgaccgcg accgcgaccg cgtccggcgg gggcggccct 240

gcgcacagcg tgtcgtcgct gtccgtcggc gcggcggcgg cggcggccgt gaagagggag 300

cgcgcggagg aggccgacgg cgagagggtg tcgtcgacgg cggccgggcg tgacgacgac 360

gacgacggga gcacccgcaa gaagctccgg ctgaccaagg agcagtccgc gctcctggag 420

gaccgcttcc gggagcacag cacgctcaac ccgaagcaga aagtcgcttt agcgaagcaa 480

ctgaacctca ggccaaggca ggtggaggtc tggttccaaa acagaagagc aaggacaaag 540

ctgaagcaga cggaggtgga ctgcgagttc ctgaagcgct gctgcgagac gctcaccgag 600

gagaaccgtc ggctgcagcg cgagctgcag gagctccgcg cgctcaagtt cgccccgccg 660

ccgccgtcct cggcggccca ccagccgtcg ccggcgccac cggcgccgtt ctacatgcaa 720

ctcccggccg ccacgctcac catctgcccg tcctgcgagc gcgtcggcgg gcccgcgtcc 780

gccgccaagg tcgtcgccgc cgacgggacc aaggccggcc ccggccggac caccacccac 840

cacttcttca accccttcac ccactccgcc gcctgctga 879

<210> 236
<211> 292
<212> PRT
<213> Oryza sativa

<400> 236

Met Ala Gln Glu Asp Val Gly His Leu Ser Asp Ala Gly Leu Ala Leu
1 5 10 15

Gly Leu Ser Leu Gly Gly Gly Gly Gly Gly Thr Thr Asp Ala Ala Ala
20 25 30

Ala His Arg Gly Gly Cys Arg Arg Pro Ser Pro Ser Ser Gln Cys Pro
35 40 45

Pro Leu Glu Pro Ser Leu Thr Leu Ser Leu Pro Asp Asp Ala Ala Ala
50 55 60

Gly Ala Ala Ala Thr Ala Thr Ala Thr Ala Ser Gly Gly Gly Gly Pro
65 70 75 80

Ala His Ser Val Ser Ser Leu Ser Val Gly Ala Ala Ala Ala Ala Ala
85 90 95

Val Lys Arg Glu Arg Ala Glu Glu Ala Asp Gly Glu Arg Val Ser Ser
100 105 110

Thr Ala Ala Gly Arg Asp Asp Asp Asp Asp Gly Ser Thr Arg Lys Lys
115 120 125

Leu Arg Leu Thr Lys Glu Gln Ser Ala Leu Leu Glu Asp Arg Phe Arg
130 135 140

Glu His Ser Thr Leu Asn Pro Lys Gln Lys Val Ala Leu Ala Lys Gln
145 150 155 160

Leu Asn Leu Arg Pro Arg Gln Val Glu Val Trp Phe Gln Asn Arg Arg
165 170 175

Ala Arg Thr Lys Leu Lys Gln Thr Glu Val Asp Cys Glu Phe Leu Lys
180 185 190

Arg Cys Cys Glu Thr Leu Thr Glu Glu Asn Arg Arg Leu Gln Arg Glu
195 200 205

Leu Gln Glu Leu Arg Ala Leu Lys Phe Ala Pro Pro Pro Pro Ser Ser

210 215 220

Ala Ala His Gln Pro Ser Pro Ala Pro Pro Ala Pro Phe Tyr Met Gln
225 230 235 240

Leu Pro Ala Ala Thr Leu Thr Ile Cys Pro Ser Cys Glu Arg Val Gly
245 250 255

Gly Pro Ala Ser Ala Ala Lys Val Val Ala Ala Asp Gly Thr Lys Ala
260 265 270

Gly Pro Gly Arg Thr Thr Thr His His Phe Phe Asn Pro Phe Thr His
275 280 285

Ser Ala Ala Cys
290

<210> 237
<211> 744
<212> DNA
<213> Oryza sativa

<400> 237
atggcgcaag atgatgagga cgtgggtcta gctctgggcc tctccctggg ctccggcggc 60

cacaggcggc agagagaaag tagagacgaa gcgccgtcgt cggcggcggc gtccctgctg 120

acgctgaggc tcccggcaga gagcgggggg cagccgcagg tggtggtgaa gagggaggtg 180

gtgcgggcgg aagaggagga gtacgaatac gagtacgaga gggcgctcta ctcgtcgtcg 240

gctgcggcgg cggacgacga cgagggctgc aacagccgga agaagctgag gctgagcaag 300

gagcagtcgg ctctgctgga ggatcgcttc aaggagcata gcactctcaa ccctaagcaa 360

aaggttgctt tggcgaagca gctaaacctg aggccaaggc aagttgaggt gtggttccaa 420

aacagaagag ccaggacgaa gctgaagcag acggaggtgg attgcgagct tctgaagcgc 480

tgctgcgaga cgctgacgga ggagaaccga cgcctccatc gtgagctcca gcagctcagg 540

gctctgaccc actccaccgc cgccggcttc ttcatggcca ccaccctccc cgtccccgcc 600

gccacgctct ccatctgccc ctcctgcgag cgcctcgcca ccgccgccgc cgccggagct 660

tcccccaccg ccgccgccga ccgcaccaac aagcccaccg ccccgcactt gttcagccct 720

ttcgccaagt ccgccgcctg ctag 744

<210> 238
<211> 247
<212> PRT
<213> Oryza sativa

<400> 238

Met Ala Gln Asp Asp Glu Asp Val Gly Leu Ala Leu Gly Leu Ser Leu
1 5 10 15

```
Gly Ser Gly Gly His Arg Arg Gln Arg Glu Ser Arg Asp Glu Ala Pro
            20                  25                  30

Ser Ser Ala Ala Ala Ser Leu Leu Thr Leu Arg Leu Pro Ala Glu Ser
        35                  40                  45

Gly Gly Gln Pro Gln Val Val Val Lys Arg Glu Val Val Arg Ala Glu
    50                  55                  60

Glu Glu Glu Tyr Glu Tyr Glu Tyr Glu Arg Ala Leu Tyr Ser Ser Ser
65                  70                  75                  80

Ala Ala Ala Ala Asp Asp Asp Glu Gly Cys Asn Ser Arg Lys Lys Leu
                85                  90                  95

Arg Leu Ser Lys Glu Gln Ser Ala Leu Leu Glu Asp Arg Phe Lys Glu
            100                 105                 110

His Ser Thr Leu Asn Pro Lys Gln Lys Val Ala Leu Ala Lys Gln Leu
        115                 120                 125

Asn Leu Arg Pro Arg Gln Val Glu Val Trp Phe Gln Asn Arg Arg Ala
    130                 135                 140

Arg Thr Lys Leu Lys Gln Thr Glu Val Asp Cys Glu Leu Leu Lys Arg
145                 150                 155                 160

Cys Cys Glu Thr Leu Thr Glu Glu Asn Arg Arg Leu His Arg Glu Leu
                165                 170                 175

Gln Gln Leu Arg Ala Leu Thr His Ser Thr Ala Ala Gly Phe Phe Met
            180                 185                 190

Ala Thr Thr Leu Pro Val Pro Ala Ala Thr Leu Ser Ile Cys Pro Ser
        195                 200                 205

Cys Glu Arg Leu Ala Thr Ala Ala Ala Ala Gly Ala Ser Pro Thr Ala
    210                 215                 220

Ala Ala Asp Arg Thr Asn Lys Pro Thr Ala Pro His Leu Phe Ser Pro
225                 230                 235                 240

Phe Ala Lys Ser Ala Ala Cys
                245
```

<210> 239
<211> 1065
<212> DNA

<213> Oryza sativa

<400> 239
```
atggagctgg ggctgagctt gggggatgcg gtgaccgtgg cggatggcgg gaggctggag      60
ctggttcttg ggctcggggt tggggttggg gctggggtga ggagaggaga ggaggaggag     120
agggggagga gggaggatgt ggtgggagct gggaggtggg cggcgatggc ggcggcctcg     180
ccggagccgt cggtgcggct cagcctcgtg tcgagcctcg ggctccactg gccttccgag     240
accgggcgtt cggaggcggc ggcgcgtggg ttcgacgtga accgggcgcc gtcggtggcg     300
gcgggcgcgc cggggatgga ggacgacgag gagggcccgg gcgccgcgcc ggcgttgtcg     360
tcgtcgccca acgacagcgg gggatccttc ccgctggacc tctccgggca aggcctccgt     420
ggccacgccg aggcggcggc gcagggtggc ggcggcggcg gcggcggcga gcggtcgtcc     480
tcgcgcgcga gcgacgacga cgagggcgcg tccgcgcgca agaagctgcg actctccaag     540
gagcagtcgg cgttcctcga ggagagcttc aaggagcaca gcacgctgaa tcccaagcag     600
aaggtggcgc tggcgaagca gctcaacctc cggccgcggc aagtggaggt ctggttccag     660
aaccgccgag ccaggacgaa gctgaagcag acggaggtgg actgcgagta cctgaagcgc     720
tgctgcgaga cgctcaccga ggagaaccgg cggctgcaca aggagctcgc cgagctgcgc     780
gcgctcaaga cggcgcgccc cttctacatg cacctcccgg ccacgaccct ctccatgtgc     840
ccctcctgcg agcgtgtcgc ctccaacccg gccaccgctt cgacctccgc ccccgccgcg     900
gccacgtccc cggcggcggc gccgaccgcg gccgcaagaa ccgccgtcgc gtcgcccgag     960
ccgcaccggc cgtcgtcctt cgccgcgctg ttcgcggcac ccctcggctt cccgctgacc    1020
gccgcccagc cgcggccgcc gccgccggcg agcaactgcc tgtaa                    1065
```

<210> 240
<211> 354
<212> PRT
<213> Oryza sativa

<400> 240

```
Met Glu Leu Gly Leu Ser Leu Gly Asp Ala Val Thr Val Ala Asp Gly
1               5                   10                  15

Gly Arg Leu Glu Leu Val Leu Gly Leu Gly Val Gly Val Gly Ala Gly
            20                  25                  30

Val Arg Arg Gly Glu Glu Glu Glu Arg Gly Arg Arg Glu Asp Val Val
        35                  40                  45

Gly Ala Gly Arg Trp Ala Ala Met Ala Ala Ala Ser Pro Glu Pro Ser
    50                  55                  60

Val Arg Leu Ser Leu Val Ser Ser Leu Gly Leu His Trp Pro Ser Glu
65                  70                  75                  80
```

Thr Gly Arg Ser Glu Ala Ala Ala Arg Gly Phe Asp Val Asn Arg Ala
85 90 95

Pro Ser Val Ala Ala Gly Ala Pro Gly Met Glu Asp Asp Glu Glu Gly
100 105 110

Pro Gly Ala Ala Pro Ala Leu Ser Ser Ser Pro Asn Asp Ser Gly Gly
115 120 125

Ser Phe Pro Leu Asp Leu Ser Gly Gln Gly Leu Arg Gly His Ala Glu
130 135 140

Ala Ala Ala Gln Gly Gly Gly Gly Gly Gly Gly Gly Glu Arg Ser Ser
145 150 155 160

Ser Arg Ala Ser Asp Asp Asp Glu Gly Ala Ser Ala Arg Lys Lys Leu
165 170 175

Arg Leu Ser Lys Glu Gln Ser Ala Phe Leu Glu Glu Ser Phe Lys Glu
180 185 190

His Ser Thr Leu Asn Pro Lys Gln Lys Val Ala Leu Ala Lys Gln Leu
195 200 205

Asn Leu Arg Pro Arg Gln Val Glu Val Trp Phe Gln Asn Arg Arg Ala
210 215 220

Arg Thr Lys Leu Lys Gln Thr Glu Val Asp Cys Glu Tyr Leu Lys Arg
225 230 235 240

Cys Cys Glu Thr Leu Thr Glu Glu Asn Arg Arg Leu His Lys Glu Leu
245 250 255

Ala Glu Leu Arg Ala Leu Lys Thr Ala Arg Pro Phe Tyr Met His Leu
260 265 270

Pro Ala Thr Thr Leu Ser Met Cys Pro Ser Cys Glu Arg Val Ala Ser
275 280 285

Asn Pro Ala Thr Ala Ser Thr Ser Ala Pro Ala Ala Ala Thr Ser Pro
290 295 300

Ala Ala Ala Pro Thr Ala Ala Ala Arg Thr Ala Val Ala Ser Pro Glu
305 310 315 320

Pro His Arg Pro Ser Ser Phe Ala Ala Leu Phe Ala Ala Pro Leu Gly
325 330 335

Phe Pro Leu Thr Ala Ala Gln Pro Arg Pro Pro Pro Pro Ala Ser Asn
340 345 350

Cys Leu

<210> 241
<211> 1089
<212> DNA
<213> Oryza sativa

<400> 241
atggagctgg ggttgagctt gggggaggct atggcggatg ctgggaggga gctggttctt 60

gggcttggga tggggaggag ggaggaggcg gcggaggcgg ggaggaggga tcatgaggtg 120

aggagggagc tggagttcgg gtcgatgtcg agcaggtgcg gtggttcttc gccggagccg 180

acggtgcggc tcacgcttct gcccatggtg cccggccttg gcctcccatg gccgccgccg 240

ccgccgccgt cgtccgagag caggcatttg gaggcgtcga cgcggggatt cgacgtgaac 300

cgcccgccgt cgtccggcgg cggcggcggc ggcggcggcg cggaggagga gcaggacgac 360

gtggccgggg cggcactctc gtcgtccccc aacaacagcg ccggatcctt cccgatggat 420

gacttctccg ggcacggcct cggcggcaac gacgcggccc ctggcggtgg cggcggcgac 480

cgctcgtgct cccgcgccag cgacgaggac gacggcggct ccgcgcgcaa gaagctccgc 540

ctctccaagg agcagtccgc gttcctcgag gagagcttca aggagcacag caccctaaac 600

cccaagcaga agctggcgct ggcgaagcag ctcaacctcc ggccgcgcca ggtggaggtg 660

tggttccaga accgccgcgc caggacgaag ctgaagcaga cggaggtgga ctgcgagtac 720

ctgaagcggt gctgcgagac gctgacggag gagaaccggc ggctgcagaa ggagctcgcc 780

gagctccggg cgctcaagac ggtgcacccc ttctacatgc acctcccggc gacgacactc 840

tccatgtgcc cctcctgcga gcgcgtcgcc tccaactccg ccccggccac cgcctcctcc 900

gccgcaacat cgtcgacggc cgcgccgccc gcggcaccct catccggcgg cattgcggcc 960

acctcctcct ccgccgccgc cgccgcggcg ccggaccaca ggccgtcgtc gttcgccgcg 1020

ctgttctcgt cgccgcgtgg cttcccgcta tccgtagccc cgcaggcgca gccgccgacg 1080

agctcgtga 1089

<210> 242
<211> 362
<212> PRT
<213> Oryza sativa

<400> 242

Met Glu Leu Gly Leu Ser Leu Gly Glu Ala Met Ala Asp Ala Gly Arg
1 5 10 15

Glu Leu Val Leu Gly Leu Gly Met Gly Arg Arg Glu Glu Ala Ala Glu
20 25 30

Ala Gly Arg Arg Asp His Glu Val Arg Arg Glu Leu Glu Phe Gly Ser
35 40 45

Met Ser Ser Arg Cys Gly Gly Ser Ser Pro Glu Pro Thr Val Arg Leu
50 55 60

Thr Leu Leu Pro Met Val Pro Gly Leu Gly Leu Pro Trp Pro Pro Pro
65 70 75 80

Pro Pro Pro Ser Ser Glu Ser Arg His Leu Glu Ala Ser Thr Arg Gly
85 90 95

Phe Asp Val Asn Arg Pro Pro Ser Ser Gly Gly Gly Gly Gly Gly Gly
100 105 110

Gly Ala Glu Glu Glu Gln Asp Asp Val Ala Gly Ala Ala Leu Ser Ser
115 120 125

Ser Pro Asn Asn Ser Ala Gly Ser Phe Pro Met Asp Asp Phe Ser Gly
130 135 140

His Gly Leu Gly Gly Asn Asp Ala Ala Pro Gly Gly Gly Gly Gly Asp
145 150 155 160

Arg Ser Cys Ser Arg Ala Ser Asp Glu Asp Asp Gly Gly Ser Ala Arg
165 170 175

Lys Lys Leu Arg Leu Ser Lys Glu Gln Ser Ala Phe Leu Glu Glu Ser
180 185 190

Phe Lys Glu His Ser Thr Leu Asn Pro Lys Gln Lys Leu Ala Leu Ala
195 200 205

Lys Gln Leu Asn Leu Arg Pro Arg Gln Val Glu Val Trp Phe Gln Asn
210 215 220

Arg Arg Ala Arg Thr Lys Leu Lys Gln Thr Glu Val Asp Cys Glu Tyr
225 230 235 240

Leu Lys Arg Cys Cys Glu Thr Leu Thr Glu Glu Asn Arg Arg Leu Gln
245 250 255

Lys Glu Leu Ala Glu Leu Arg Ala Leu Lys Thr Val His Pro Phe Tyr
260 265 270

Met His Leu Pro Ala Thr Thr Leu Ser Met Cys Pro Ser Cys Glu Arg

275 280 285

Val Ala Ser Asn Ser Ala Pro Ala Thr Ala Ser Ser Ala Ala Thr Ser
290 295 300

Ser Thr Ala Ala Pro Pro Ala Ala Pro Ser Ser Gly Gly Ile Ala Ala
305 310 315 320

Thr Ser Ser Ser Ala Ala Ala Ala Ala Ala Pro Asp His Arg Pro Ser
325 330 335

Ser Phe Ala Ala Leu Phe Ser Ser Pro Arg Gly Phe Pro Leu Ser Val
340 345 350

Ala Pro Gln Ala Gln Pro Pro Thr Ser Ser
355 360

<210> 243
<211> 771
<212> DNA
<213> Oryza sativa

<400> 243
atggagaggc aaggcttgga tcttggcctg agcctcgggc taggcttgac gacggcggcg 60

acatggccgg ctgctgggtt ctgtctgaac tccggcatgg cggagcagga agtgatcagg 120

cgtgatgatg tggttgcggc gacggcggcg gaggatgaga ggttcgcgtg ctcacccggc 180

agcccggtgt cgagcggcag cgggaagcga ggcagcggca gcggcagcgg cgacgaggtc 240

gacgacgccg gctgcgacgt cggcggcggc ggcgcgcgca agaagctgcg gttgtccaag 300

gaccaggccg ccgtcctcga ggagtgcttc aagacgcacc acaccctcac tccgaagcag 360

aaggtggcgc tggcgaagag cttgaacctg cggccgcggc aggtggaggt gtggttccag 420

aaccgccgcg cgaggacgaa gctgaagcag acggaggtgg actgcgagca cctcaagcgg 480

tggtgcgacc agctcgccga cgacaaccgc cgcctccaca aggagctcgc cgagctcagg 540

gcgctcaagg ccacgcccac accgcccgcc gccgcgccgc cattgaccac cctcacaatg 600

tgcctctcct gcaagcgcgt cgccaatgcc ggcgtgccct cgccggcggc ggcgatattc 660

cccggccacc cccagttctt gtgcggattc agagatcacg ccggagcagc gtcgtcgtcg 720

tacggcggcg catcatctgg actcgcgaag gcggtcaggg cggcgaggta g 771

<210> 244
<211> 256
<212> PRT
<213> Oryza sativa

<400> 244

Met Glu Arg Gln Gly Leu Asp Leu Gly Leu Ser Leu Gly Leu Gly Leu
1 5 10 15

Thr Thr Ala Ala Thr Trp Pro Ala Ala Gly Phe Cys Leu Asn Ser Gly
20 25 30

Met Ala Glu Gln Glu Val Ile Arg Arg Asp Asp Val Val Ala Ala Thr
35 40 45

Ala Ala Glu Asp Glu Arg Phe Ala Cys Ser Pro Gly Ser Pro Val Ser
50 55 60

Ser Gly Ser Gly Lys Arg Gly Ser Gly Ser Gly Ser Gly Asp Glu Val
65 70 75 80

Asp Asp Ala Gly Cys Asp Val Gly Gly Gly Gly Ala Arg Lys Lys Leu
85 90 95

Arg Leu Ser Lys Asp Gln Ala Ala Val Leu Glu Glu Cys Phe Lys Thr
100 105 110

His His Thr Leu Thr Pro Lys Gln Lys Val Ala Leu Ala Lys Ser Leu
115 120 125

Asn Leu Arg Pro Arg Gln Val Glu Val Trp Phe Gln Asn Arg Arg Ala
130 135 140

Arg Thr Lys Leu Lys Gln Thr Glu Val Asp Cys Glu His Leu Lys Arg
145 150 155 160

Trp Cys Asp Gln Leu Ala Asp Asp Asn Arg Arg Leu His Lys Glu Leu
165 170 175

Ala Glu Leu Arg Ala Leu Lys Ala Thr Pro Thr Pro Pro Ala Ala Ala
180 185 190

Pro Pro Leu Thr Thr Leu Thr Met Cys Leu Ser Cys Lys Arg Val Ala
195 200 205

Asn Ala Gly Val Pro Ser Pro Ala Ala Ala Ile Phe Pro Gly His Pro
210 215 220

Gln Phe Leu Cys Gly Phe Arg Asp His Ala Gly Ala Ala Ser Ser Ser
225 230 235 240

Tyr Gly Gly Ala Ser Ser Gly Leu Ala Lys Ala Val Arg Ala Ala Arg
245 250 255

<210> 245
<211> 927
<212> DNA

<213> Oryza sativa

<400> 245
atgatggatc tcggcctcag cctcggcctc ggcctcgcct cgcagggcag cctcacctcc 60

tccaccacca ccacctcctc ccccggcgcc ggatcatcct ccccgtgggc cgccgcgctc 120

aactccatcg tcggcgacgt gaggcgggat caggccgcgg cgcatgctgc cgcggcggtg 180

ggggttgggg ttgggggcga ggagatgtac caggggaggg cgtccacgtc gccggacagc 240

gcggcggcgc tgtcgagcgc gagcgggaag agggagaggg agctggagcg gtcgggatcc 300

ggggttgacg acgacgacgg cgcggacggc gccggcgggc ggaagaagct caggctgtcc 360

aaggaccagg ccgccgtgct cgaggagtgc ttcaagacgc actccactct caaccccaag 420

cagaaggtgg cgctggcgaa caggctgggg ctgcggccgc ggcaggtgga ggtgtggttc 480

cagaaccgga gggcgaggac gaagctgaag cagacggagg tggactgcga gtacctgaag 540

cggtggtgcg agcgcctcgc cgacgagaac aagcgcctcg agaaggagct cgccgacctc 600

agggcgctca aggccgcgcc ctcgccggcg tccgcgtccg cgatgcagcc ctcctcctcc 660

gccgccgcca cgctcaccat gtgcccctcc tgccgccgcg tcgccaccgc cggcgcgccg 720

caccagccta accaccaaca atgccatccc aaatctaaca ccaccatctc ctcctcctcc 780

accgccgccg ccgccgtcgc cgtcgccggc ggcaacgtgc tgcccagcca ctgccagttc 840

ttcccggccg ccgccgccgc cgccgaccgg acaagccaga gcacgtggaa cgccgccgcg 900

ccgctcgtca ccagagagct cttctag 927

<210> 246
<211> 308
<212> PRT
<213> Oryza sativa

<400> 246

Met Met Asp Leu Gly Leu Ser Leu Gly Leu Gly Leu Ala Ser Gln Gly
1 5 10 15

Ser Leu Thr Ser Ser Thr Thr Thr Thr Ser Ser Pro Gly Ala Gly Ser
20 25 30

Ser Ser Pro Trp Ala Ala Ala Leu Asn Ser Ile Val Gly Asp Val Arg
35 40 45

Arg Asp Gln Ala Ala Ala His Ala Ala Ala Ala Val Gly Val Gly Val
50 55 60

Gly Gly Glu Glu Met Tyr Gln Gly Arg Ala Ser Thr Ser Pro Asp Ser
65 70 75 80

Ala Ala Ala Leu Ser Ser Ala Ser Gly Lys Arg Glu Arg Glu Leu Glu
85 90 95

Arg Ser Gly Ser Gly Val Asp Asp Asp Asp Gly Ala Asp Gly Ala Gly
            100                 105                 110

Gly Arg Lys Lys Leu Arg Leu Ser Lys Asp Gln Ala Ala Val Leu Glu
        115                 120                 125

Glu Cys Phe Lys Thr His Ser Thr Leu Asn Pro Lys Gln Lys Val Ala
    130                 135                 140

Leu Ala Asn Arg Leu Gly Leu Arg Pro Arg Gln Val Glu Val Trp Phe
145                 150                 155                 160

Gln Asn Arg Arg Ala Arg Thr Lys Leu Lys Gln Thr Glu Val Asp Cys
                165                 170                 175

Glu Tyr Leu Lys Arg Trp Cys Glu Arg Leu Ala Asp Glu Asn Lys Arg
            180                 185                 190

Leu Glu Lys Glu Leu Ala Asp Leu Arg Ala Leu Lys Ala Ala Pro Ser
        195                 200                 205

Pro Ala Ser Ala Ser Ala Met Gln Pro Ser Ser Ser Ala Ala Ala Thr
    210                 215                 220

Leu Thr Met Cys Pro Ser Cys Arg Arg Val Ala Thr Ala Gly Ala Pro
225                 230                 235                 240

His Gln Pro Asn His Gln Gln Cys His Pro Lys Ser Asn Thr Thr Ile
                245                 250                 255

Ser Ser Ser Ser Thr Ala Ala Ala Ala Val Ala Val Ala Gly Gly Asn
            260                 265                 270

Val Leu Pro Ser His Cys Gln Phe Phe Pro Ala Ala Ala Ala Ala Ala
        275                 280                 285

Asp Arg Thr Ser Gln Ser Thr Trp Asn Ala Ala Ala Pro Leu Val Thr
    290                 295                 300

Arg Glu Leu Phe
305

<210> 247
<211> 936
<212> DNA
<213> Oryza sativa

<400> 247
atggagatga tggttcatgg gaggagagac gagcagtatg gcgggctcag gctcgggctt 60

gggcttgggc tcagcctcgg cgtcgccggt ggtgcagccg acgacgagca gccgccgccg 120

cgccgtggtg ccgccccgcc gccgcagcag cagctgtgcg gctggaacgg cggcggtctc 180

ttctcctcgt cttcctccga tcatcggggg aggtcggcga tgatggcgtg ccacgacgtc 240

atcgagatgc cgttcctgcg gggggatcgac gtgaaccgtg cgccggcggc agagacgacc 300

acgacgacgg cgaggggggcc cagctgcagc gaggaagacg aggagcccgg cgcgtcctcc 360

cccaacagca cgctctccag cctcagcggc aagcgcggcg caccatctgc cgccaccgcc 420

gccgccgccg ccgccagcga cgacgaggac tccggcggcg gatcccgcaa gaagctccgc 480

ctctccaagg accaagccgc cgtcctcgag gacaccttca aagagcacaa caccctcaat 540

cccaagcaga aggcggcgct ggcgaggcag ctgaatctga agccgcggca ggtggaggtg 600

tggttccaga acaggagggc gaggacgaag ctgaagcaga cggaggtgga ctgcgagctg 660

ctcaagcgct gctgcgagac gctcaccgac gagaaccgcc gcctccaccg cgagctccag 720

gagctccgcg ccctcaagct cgccaccgcc gccgccgcgc cgcaccacct ctacggcgcc 780

cgcgtcccgc cgcccaccac cctcaccatg tgcccctcct gcgagcgcgt cgcctccgca 840

gccaccacca cccgcaacaa ctccggcgcc gcccccgcgc ggccggtgcc cacccgcccg 900

tggccgccgg cggcggcgca gaggtcgtcg gcgtag 936

<210> 248
<211> 311
<212> PRT
<213> Oryza sativa

<400> 248

Met Glu Met Met Val His Gly Arg Arg Asp Glu Gln Tyr Gly Gly Leu
1 5 10 15

Arg Leu Gly Leu Gly Leu Gly Leu Ser Leu Gly Val Ala Gly Gly Ala
20 25 30

Ala Asp Asp Glu Gln Pro Pro Pro Arg Arg Gly Ala Ala Pro Pro Pro
35 40 45

Gln Gln Gln Leu Cys Gly Trp Asn Gly Gly Gly Leu Phe Ser Ser Ser
50 55 60

Ser Ser Asp His Arg Gly Arg Ser Ala Met Met Ala Cys His Asp Val
65 70 75 80

Ile Glu Met Pro Phe Leu Arg Gly Ile Asp Val Asn Arg Ala Pro Ala
85 90 95

Ala Glu Thr Thr Thr Thr Thr Ala Arg Gly Pro Ser Cys Ser Glu Glu
100 105 110

Asp Glu Glu Pro Gly Ala Ser Ser Pro Asn Ser Thr Leu Ser Ser Leu
115 120 125

Ser Gly Lys Arg Gly Ala Pro Ser Ala Ala Thr Ala Ala Ala Ala Ala
130 135 140

Ala Ser Asp Asp Glu Asp Ser Gly Gly Gly Ser Arg Lys Lys Leu Arg
145 150 155 160

Leu Ser Lys Asp Gln Ala Ala Val Leu Glu Asp Thr Phe Lys Glu His
165 170 175

Asn Thr Leu Asn Pro Lys Gln Lys Ala Ala Leu Ala Arg Gln Leu Asn
180 185 190

Leu Lys Pro Arg Gln Val Glu Val Trp Phe Gln Asn Arg Arg Ala Arg
195 200 205

Thr Lys Leu Lys Gln Thr Glu Val Asp Cys Glu Leu Leu Lys Arg Cys
210 215 220

Cys Glu Thr Leu Thr Asp Glu Asn Arg Arg Leu His Arg Glu Leu Gln
225 230 235 240

Glu Leu Arg Ala Leu Lys Leu Ala Thr Ala Ala Ala Ala Pro His His
245 250 255

Leu Tyr Gly Ala Arg Val Pro Pro Pro Thr Thr Leu Thr Met Cys Pro
260 265 270

Ser Cys Glu Arg Val Ala Ser Ala Ala Thr Thr Thr Arg Asn Asn Ser
275 280 285

Gly Ala Ala Pro Ala Arg Pro Val Pro Thr Arg Pro Trp Pro Pro Ala
290 295 300

Ala Ala Gln Arg Ser Ser Ala
305 310

<210> 249
<211> 1674
<212> DNA
<213> Arabidopsis thaliana

<400> 249
aaaatggaaa actcagacac tgattcagaa gttttctttt ggtttcagaa ccaaaaccaa 60

aaccatagcc ataagtttcc ttcttcttgt tttcctccga gctctcactc tgctttttat 120

ggatctagct caatgatcaa cacagagact gcaactatgg acgaagaaga cgtatgtgag 180

agctacatga tgcgtgagat aaccaagaaa cggaagctaa caccgatcca attgcgttta 240

ctagaggaga gtttcgaaga agagaaaaga ctcgaaccag acaggaaact ctggctagcc 300

gagaagctag ggctgcagcc aagccaagtc gctgtctggt tccaaaacag aagagctaga 360

tacaagacca aacagctcga acacgactgt gactctctta aagctagcta tgctaagctc 420

aagactgact gggacattct ctttgtacaa aaccaaaccc tcaagagcaa ggtacagttt 480

cttaatagat taacaagcca ctattttcaa gagtctgttc aaaattttga tgacacattc 540

aaacaggtcg atcttctcaa agagaagctg aaaatgcaag agaatcttga aactcagtct 600

attgagagga aaagacttgg ggaagaaggt agttcggtga aaagcgataa cacgcagtac 660

agtgaagaag aaggtttgga gaatcaatac agtttcccgg agcttgcagt tctcggattt 720

tattatgatc caaccttaac tgcttcaaat ctaagacaag aacctttgaa agtcacgtgt 780

gcggatcaga tgactcagat ccaaatatct gacgtcacag aacctgcgag ctccgcacat 840

aaaaaaattg aagtgacaca gcgaagttcg agtatgagcc gcaagagaga taaaccctac 900

acaaaccgtc acacaccggc gcgaatttcg aaacgccggc gaccatgggc gccttcgtca 960

tcggagcacg atgagattat cgacaaaccg atcaccaaac ctccgccgcc accggcgttg 1020

gtagttatgg gacttcccgc caactgttca gttctggagc taaaatctcg attcgagatc 1080

tacggttcaa tctcccgaat ccgaatccat aaagacggaa ttggctccgt ttcgtatcga 1140

accgccgagt cagcagaagc cgccattgct ggtagtcacg agccttcttt cggtatctcc 1200

atcgattcca aaaagttgga ggtggtttgg gcgacggatc cattggtgaa gtggaaggaa 1260

ggtgtgacgg cgggagaagg aaaggagaga acgtcgtcgt tttcgtcgaa gcttttacga 1320

cctgtgatgc ctttgagaaa acatgggaga agcagtaggt tagcgtcagc tattgttaat 1380

ccgagaagtg ataatactaa aggaattagt ggagatggag ggatatcatc tccggcgacg 1440

acaagtgaag ttaaacagag aaatatagta acctacgacg atatcgttta acaccattct 1500

actacaatta tagtaacttt gattctttta catattttgg caattttatg agtttagcta 1560

atcctgttgg taatctcagt tgtcgaatgt tgtgaaatga gttgagtttt gtttatttgt 1620

atgattgtaa cttataagaa agagaaggat tccaaaatgg gtagaagatt caaa 1674

<210> 250
<211> 495
<212> PRT
<213> Arabidopsis thaliana

<400> 250

Met Glu Asn Ser Asp Thr Asp Ser Glu Val Phe Phe Trp Phe Gln Asn
1 5 10 15

Gln Asn Gln Asn His Ser His Lys Phe Pro Ser Ser Cys Phe Pro Pro
20 25 30

Ser Ser His Ser Ala Phe Tyr Gly Ser Ser Ser Met Ile Asn Thr Glu
35 40 45

Thr Ala Thr Met Asp Glu Glu Asp Val Cys Glu Ser Tyr Met Met Arg
50 55 60

Glu Ile Thr Lys Lys Arg Lys Leu Thr Pro Ile Gln Leu Arg Leu Leu
65 70 75 80

Glu Glu Ser Phe Glu Glu Glu Lys Arg Leu Glu Pro Asp Arg Lys Leu
85 90 95

Trp Leu Ala Glu Lys Leu Gly Leu Gln Pro Ser Gln Val Ala Val Trp
100 105 110

Phe Gln Asn Arg Arg Ala Arg Tyr Lys Thr Lys Gln Leu Glu His Asp
115 120 125

Cys Asp Ser Leu Lys Ala Ser Tyr Ala Lys Leu Lys Thr Asp Trp Asp
130 135 140

Ile Leu Phe Val Gln Asn Gln Thr Leu Lys Ser Lys Val Gln Phe Leu
145 150 155 160

Asn Arg Leu Thr Ser His Tyr Phe Gln Glu Ser Val Gln Asn Phe Asp
165 170 175

Asp Thr Phe Lys Gln Val Asp Leu Leu Lys Glu Lys Leu Lys Met Gln
180 185 190

Glu Asn Leu Glu Thr Gln Ser Ile Glu Arg Lys Arg Leu Gly Glu Glu
195 200 205

Gly Ser Ser Val Lys Ser Asp Asn Thr Gln Tyr Ser Glu Glu Glu Gly
210 215 220

Leu Glu Asn Gln Tyr Ser Phe Pro Glu Leu Ala Val Leu Gly Phe Tyr
225 230 235 240

Tyr Asp Pro Thr Leu Thr Ala Ser Asn Leu Arg Gln Glu Pro Leu Lys
245 250 255

Val Thr Cys Ala Asp Gln Met Thr Gln Ile Gln Ile Ser Asp Val Thr
260 265 270

Glu Pro Ala Ser Ser Ala His Lys Lys Ile Glu Val Thr Gln Arg Ser
275 280 285

```
Ser Ser Met Ser Arg Lys Arg Asp Lys Pro Tyr Thr Asn Arg His Thr
    290                 295                 300

Pro Ala Arg Ile Ser Lys Arg Arg Arg Pro Trp Ala Pro Ser Ser Ser
305                 310                 315                 320

Glu His Asp Glu Ile Ile Asp Lys Pro Ile Thr Lys Pro Pro Pro Pro
                325                 330                 335

Pro Ala Leu Val Val Met Gly Leu Pro Ala Asn Cys Ser Val Leu Glu
            340                 345                 350

Leu Lys Ser Arg Phe Glu Ile Tyr Gly Ser Ile Ser Arg Ile Arg Ile
        355                 360                 365

His Lys Asp Gly Ile Gly Ser Val Ser Tyr Arg Thr Ala Glu Ser Ala
    370                 375                 380

Glu Ala Ala Ile Ala Gly Ser His Glu Pro Ser Phe Gly Ile Ser Ile
385                 390                 395                 400

Asp Ser Lys Lys Leu Glu Val Val Trp Ala Thr Asp Pro Leu Val Lys
                405                 410                 415

Trp Lys Glu Gly Val Thr Ala Gly Glu Gly Lys Glu Arg Thr Ser Ser
            420                 425                 430

Phe Ser Ser Lys Leu Leu Arg Pro Val Met Pro Leu Arg Lys His Gly
        435                 440                 445

Arg Ser Ser Arg Leu Ala Ser Ala Ile Val Asn Pro Arg Ser Asp Asn
    450                 455                 460

Thr Lys Gly Ile Ser Gly Asp Gly Gly Ile Ser Ser Pro Ala Thr Thr
465                 470                 475                 480

Ser Glu Val Lys Gln Arg Asn Ile Val Thr Tyr Asp Asp Ile Val
                485                 490                 495


<210>  251
<211>  1428
<212>  DNA
<213>  Arabidopsis thaliana

<400>  251
aaggccacaa cagatcttct tgttcctctc tctcaatctc tctctctaaa actcttattt      60

tcttcgtaag agatggaact agcgttgtct ctaggcgaca atactaagaa acagttctct     120

tttatggaga agaactcgaa gattaataat ccctctgtct cgtcgacatc tacttccgag     180
```

aaggatcttg ggttctgcat ggctttagat gttgcttttg gtggtcacag atcgttgtca 240

tcctcttcgt ctccgtcggt agaggatgag aagaagaaac cggcgcccag agcaaaaaaa 300

tctgacgaat ttagggtttc gtcttctgta gatccaccat tacagcttca gcttcacttc 360

cctaattggc tccctgagaa cagtaaaggt cgacaaggag gaagaatgcc cttaggagca 420

gctacggttg tggaggagga agaggaggag gaggaagcgg tgcctagtat gtcagtatcg 480

ccgccggata gtgtaacgtc gtcgtttcaa ttggactttg ggattaaaag ttatggttat 540

gagagaagaa gcaataagag agatattgat gatgaagtgg agagatcagc ttcaagagcc 600

agcaacgaag acaacgatga cgagaatgga tccactagga agaaacttag actctccaaa 660

gaccaatctg cttttcttga agacagcttc aaagaacaca gtacccttaa tcctgttcgt 720

gtcccattct ttacagtttt tatttattta aaatttgtct ttcttgaatt tattctattt 780

ttttagtacc aaattttagc cttaatgctt ttttttttct ttctaaaaca ttgcagaaac 840

agaagattgc attggcgaag cagttgaatc ttcgtcctcg tcaggttgaa gtctggtttc 900

aaaacagacg agccaggaca aagctgaagc aaacggaagt ggactgtgaa tacctaaaga 960

gatgctgtga gtcactaacc gaagaaaacc ggaggcttca aaaagaggtt aaagaattga 1020

gaaccttgaa gacttccaca cccttttaca tgcaacttcc ggccactact ctcactatgt 1080

gcccttcttg tgaacgtgtt gccacttcag cagcacagcc ctccacgtca gctgcccaca 1140

acctctgttt gtccacgtca tcattgattc cggttaagcc tcggccggcc aaacaagttt 1200

catgaaagca cctgcgaaat acagtttgag caaacggtcg agctagagtg gttttaaaag 1260

ttgtcttctt gtgtatatat ttattttact tttcatattt tattagagac cgctattttg 1320

aaagacgaat agattgatta tccggttagt gttttgtttt tcttagatag gaccggataa 1380

aaaacagatg gagcaaaagg gttgacatgt tttattgaat gatagagg 1428

<210> 252
<211> 237
<212> PRT
<213> Arabidopsis thaliana

<400> 252

Met Glu Leu Ala Leu Ser Leu Gly Asp Asn Thr Lys Lys Gln Phe Ser
1 5 10 15

Phe Met Glu Lys Asn Ser Lys Ile Asn Asn Pro Ser Val Ser Ser Thr
20 25 30

Ser Thr Ser Glu Lys Asp Leu Gly Phe Cys Met Ala Leu Asp Val Ala
35 40 45

Phe Gly Gly His Arg Ser Leu Ser Ser Ser Ser Ser Pro Ser Val Glu
50 55 60

Asp Glu Lys Lys Lys Pro Ala Pro Arg Ala Lys Lys Ser Asp Glu Phe
65 70 75 80

Arg Val Ser Ser Ser Val Asp Pro Pro Leu Gln Leu Gln Leu His Phe
85 90 95

Pro Asn Trp Leu Pro Glu Asn Ser Lys Gly Arg Gln Gly Gly Arg Met
100 105 110

Pro Leu Gly Ala Ala Thr Val Val Glu Glu Glu Glu Glu Glu Glu Glu
115 120 125

Ala Val Pro Ser Met Ser Val Ser Pro Pro Asp Ser Val Thr Ser Ser
130 135 140

Phe Gln Leu Asp Phe Gly Ile Lys Ser Tyr Gly Tyr Glu Arg Arg Ser
145 150 155 160

Asn Lys Arg Asp Ile Asp Asp Glu Val Glu Arg Ser Ala Ser Arg Ala
165 170 175

Ser Asn Glu Asp Asn Asp Asp Glu Asn Gly Ser Thr Arg Lys Lys Leu
180 185 190

Arg Leu Ser Lys Asp Gln Ser Ala Phe Leu Glu Asp Ser Phe Lys Glu
195 200 205

His Ser Thr Leu Asn Pro Val Arg Val Pro Phe Phe Thr Val Phe Ile
210 215 220

Tyr Leu Lys Phe Val Phe Leu Glu Phe Ile Leu Phe Phe
225 230 235

<210> 253
<211> 1167
<212> DNA
<213> Arabidopsis thaliana

<400> 253
tgcccccagc tagtcacata ctcatgattg caaaatctct ctctctctct gcctctctat 60

atattaacct ttcttcttcc tttactttct catcttctat ctctcaaaag aaaagcagac 120

aactttattt gcaaaaacag agtttttttt tcttatcttg agaaagttca acagaagatg 180

atgttcgaga aagacgatct gggtctaagc ttaggcttga attttccaaa gaaacagatc 240

aatctcaaat caaatccatc tgtttctgtt actccttctt cttcttcttt tggattattc 300

agaagatctt catggaacga gagttttact tcttcagttc caaactcaga ttcgtcacaa 360

aaagaaacaa gaactttcat ccgaggaatc gacgtgaaca gaccaccgtc tacagcggaa 420

```
tacggcgacg aagacgctgg agtatcttca cctaacagta cagtctcaag ctctacaggg     480

aaaagaagcg agagagaaga agacacagat ccacaaggct caagaggaat cagtgacgat     540

gaagatggtg ataactccag gaaaaagctt agactttcca aagatcaatc tgctattctt     600

gaagagacct tcaaagatca cagtactctc aatccgaagc agaagcaagc attggctaaa     660

caattagggt tacgagcaag acaagtggaa gtttggtttc agaacagacg agcaagaaca     720

aagctgaagc aaacggaggt agactgcgag ttcttacgga gatgctgcga gaatctaacg     780

gaagagaacc gtcggctaca aaaagaagta acggaattga gagcacttaa gctctctcct     840

cagttctaca tgcacatgag cccacccact actttgacca tgtgcccttc atgtgaacac     900

gtgtcggtcc cgccaccaca acctcaggct gctacgtcag cgcaccaccg gtcgttgccg     960

gtcaatgcgt gggctcctgc gacgaggata tctcacggct tgacttttga cgctcttcgt    1020

cctaggtcct aagtcttttt acttgcaacc aaagggcatt ttggtcgttt tttaagtttc    1080

atggaccaga tatgcatgta gttgttaaca tgtatgtatt ttcttagaaa gaaagaaaaa    1140

cagattaata tttttctagc ttaaacc                                        1167
```

```
<210>  254
<211>  284
<212>  PRT
<213>  Arabidopsis thaliana

<400>  254

Met Met Phe Glu Lys Asp Asp Leu Gly Leu Ser Leu Gly Leu Asn Phe
1               5                   10                  15

Pro Lys Lys Gln Ile Asn Leu Lys Ser Asn Pro Ser Val Ser Val Thr
            20                  25                  30

Pro Ser Ser Ser Ser Phe Gly Leu Phe Arg Arg Ser Ser Trp Asn Glu
        35                  40                  45

Ser Phe Thr Ser Ser Val Pro Asn Ser Asp Ser Ser Gln Lys Glu Thr
    50                  55                  60

Arg Thr Phe Ile Arg Gly Ile Asp Val Asn Arg Pro Pro Ser Thr Ala
65                  70                  75                  80

Glu Tyr Gly Asp Glu Asp Ala Gly Val Ser Ser Pro Asn Ser Thr Val
                85                  90                  95

Ser Ser Ser Thr Gly Lys Arg Ser Glu Arg Glu Glu Asp Thr Asp Pro
            100                 105                 110

Gln Gly Ser Arg Gly Ile Ser Asp Asp Glu Asp Gly Asp Asn Ser Arg
        115                 120                 125
```

Lys Lys Leu Arg Leu Ser Lys Asp Gln Ser Ala Ile Leu Glu Glu Thr
    130                 135                 140

Phe Lys Asp His Ser Thr Leu Asn Pro Lys Gln Lys Gln Ala Leu Ala
145                 150                 155                 160

Lys Gln Leu Gly Leu Arg Ala Arg Gln Val Glu Val Trp Phe Gln Asn
                165                 170                 175

Arg Arg Ala Arg Thr Lys Leu Lys Gln Thr Glu Val Asp Cys Glu Phe
            180                 185                 190

Leu Arg Arg Cys Cys Glu Asn Leu Thr Glu Glu Asn Arg Arg Leu Gln
        195                 200                 205

Lys Glu Val Thr Glu Leu Arg Ala Leu Lys Leu Ser Pro Gln Phe Tyr
    210                 215                 220

Met His Met Ser Pro Pro Thr Thr Leu Thr Met Cys Pro Ser Cys Glu
225                 230                 235                 240

His Val Ser Val Pro Pro Pro Gln Pro Gln Ala Ala Thr Ser Ala His
                245                 250                 255

His Arg Ser Leu Pro Val Asn Ala Trp Ala Pro Ala Thr Arg Ile Ser
            260                 265                 270

His Gly Leu Thr Phe Asp Ala Leu Arg Pro Arg Ser
        275                 280

<210> 255
<211> 1194
<212> DNA
<213> Arabidopsis thaliana

<400> 255
atttcacatc tctctctctc tataagaacc ctagaagaag gtttctcttg tcctccatac 60

acttagcaca actgataaat cttttgaggt aaaatcagct ttagatcaag gtttttctag 120

tcatctctac tcataaagat caaagctttt gctattctca ttttctacca agagacaata 180

tcatgatgat gggtaaagag gatttgggtt taagtcttag cttgggattt gcacaaaacc 240

atcctctcca gctaaatctt aaacccactt cttcaccaat gtccaatctc cagatgtttc 300

catggaacca aacccttgtt tcttcctcag atcaacaaaa gcaacagttt cttaggaaaa 360

tcgacgtgaa cagcttgcca acaacggtgg atttggaaga ggagacagga gtttcgtctc 420

caaacagtac gatctcgagc acagtgagtg gaaagaggag gagtactgaa agagaaggta 480

cctccggtgg tggttgcgga gatgaccttg acatcactct agatagatct tcctcacgtg 540

gaacctccga tgaagaggaa gattacggag gtgagacttg taggaagaag cttagactat 600

ccaaagatca atccgcagtt ctcgaagaca ctttcaaaga gcacaatact ctcaatccca 660

aacagaagct ggctttggct aagaagctag gtttaacagc aagacaagtg gaagtgtggt 720

tccaaaacag aagagcaagg acaaagttaa agcagaccga agtggattgc gagtatttga 780

aaagatgtgt tgagaaatta acggaagaga atcggcggct cgagaaagag gcagcggaac 840

taagagcatt aaagctttca ccgcggttgt atggtcagat gagtccaccg accacacttt 900

tgatgtgtcc atcgtgtgaa cgtgtggccg gaccatcctc atctaaccac aaccagcgat 960

ctgtctcatt gagtccatgg ctccaaatgg cccatgggtc aacctttgat gtgatgcgtc 1020

ctaggtctta actttaatgc tgcttctatg ggttgtgtgt gggtcattgt actttttaga 1080

ttattgactc tcagctaatg tatccttaaa agccttttc tacttttaaa tttactttaa 1140

tctaattaaa ttagttattc ttgtcttctt gataacaaac aaatttataa taat 1194

<210> 256
<211> 282
<212> PRT
<213> Arabidopsis thaliana

<400> 256

Met Met Met Gly Lys Glu Asp Leu Gly Leu Ser Leu Ser Leu Gly Phe
1 5 10 15

Ala Gln Asn His Pro Leu Gln Leu Asn Leu Lys Pro Thr Ser Ser Pro
20 25 30

Met Ser Asn Leu Gln Met Phe Pro Trp Asn Gln Thr Leu Val Ser Ser
35 40 45

Ser Asp Gln Gln Lys Gln Gln Phe Leu Arg Lys Ile Asp Val Asn Ser
50 55 60

Leu Pro Thr Thr Val Asp Leu Glu Glu Glu Thr Gly Val Ser Ser Pro
65 70 75 80

Asn Ser Thr Ile Ser Ser Thr Val Ser Gly Lys Arg Arg Ser Thr Glu
85 90 95

Arg Glu Gly Thr Ser Gly Gly Gly Cys Gly Asp Asp Leu Asp Ile Thr
100 105 110

Leu Asp Arg Ser Ser Ser Arg Gly Thr Ser Asp Glu Glu Glu Asp Tyr
115 120 125

Gly Gly Glu Thr Cys Arg Lys Lys Leu Arg Leu Ser Lys Asp Gln Ser
130 135 140

Ala Val Leu Glu Asp Thr Phe Lys Glu His Asn Thr Leu Asn Pro Lys
145 150 155 160

Gln Lys Leu Ala Leu Ala Lys Lys Leu Gly Leu Thr Ala Arg Gln Val
165 170 175

Glu Val Trp Phe Gln Asn Arg Arg Ala Arg Thr Lys Leu Lys Gln Thr
180 185 190

Glu Val Asp Cys Glu Tyr Leu Lys Arg Cys Val Glu Lys Leu Thr Glu
195 200 205

Glu Asn Arg Arg Leu Glu Lys Glu Ala Ala Glu Leu Arg Ala Leu Lys
210 215 220

Leu Ser Pro Arg Leu Tyr Gly Gln Met Ser Pro Pro Thr Thr Leu Leu
225 230 235 240

Met Cys Pro Ser Cys Glu Arg Val Ala Gly Pro Ser Ser Ser Asn His
245 250 255

Asn Gln Arg Ser Val Ser Leu Ser Pro Trp Leu Gln Met Ala His Gly
260 265 270

Ser Thr Phe Asp Val Met Arg Pro Arg Ser
275 280

<210> 257
<211> 1284
<212> DNA
<213> Arabidopsis thaliana

<400> 257
aataatgatg gattgtaatt agccacctga caaaatctct catcattcaa agtactatat 60

taatcccctc tcttcttcat taccatctca catctctctc tatttctctt ccacaaagag 120

tcctaacttc gagttgaaac aaacaccatt tctcatctct atctcagaaa gaacaaacca 180

tttcgtgttc tttctttctc tattctcata aggaaatata attcctgaaa ctgttgagtt 240

cttgtgaaag gaaataaaaa acatgatgat gggcaaagaa gatctaggtt tgagcctaag 300

cttagggttt tcacaaaatc acaatcctct tcagatgaat ctgaatccta actcttcatt 360

atcaaacaat ctccagagac tcccatggaa ccaaacattc gatcctacat cagatcttcg 420

caagatagac gtgaacagtt ttccatcaac ggttaactgc gaggaagaca caggagtttc 480

gtcaccaaac agtacgatct caagcaccat tagcgggaag agaagtgaga gagaaggaat 540

ctccggaacc ggcgttggct ccggcgacga tcacgacgag atcactccgg atcgagggta 600

ctcacgtgga acctcagatg aagaagaaga cgggggcgaa acgtcgagga agaagctcag 660

gttatcaaaa gatcagtctg cttttctcga agagactttc aaagaacaca acactctcaa 720

tcccaaacag aagctagctt tggctaagaa gctgaacttg acggcaagac aagtggaagt 780

gtggttccaa aacagaagag ctagaaccaa gttaaagcaa acggaggtag attgcgaata 840

cttgaaacgg tgcgtagaga agctaacgga agagaaccgg agacttcaga aagaggctat 900

ggagcttcga actctcaagc tgtctccaca attctacggt cagatgactc caccaactac 960

actcatcatg tgtccttcgt gcgagcgtgt gggtggccca tcatcatcga accatcacca 1020

caatcacagg cccgtttcta tcaatccgtg ggttgcttgt gctggtcagg tggctcatgg 1080

gctgaatttt gaagccttgc gtccacgatc gtgatttttt attttagtgg tgggaaaagg 1140

gtgttttggt atttttcgtt atcgttatat agtctatctg tgtggggtca ttgtaatttt 1200

ggatgattgg ccttctcatg aactagtcct atgtatgatg caaccttaaa aagatttaaa 1260

ttagcaaaaa ttagttacaa actt 1284

<210> 258
<211> 283
<212> PRT
<213> Arabidopsis thaliana

<400> 258

Met Met Met Gly Lys Glu Asp Leu Gly Leu Ser Leu Ser Leu Gly Phe
1 5 10 15

Ser Gln Asn His Asn Pro Leu Gln Met Asn Leu Asn Pro Asn Ser Ser
20 25 30

Leu Ser Asn Asn Leu Gln Arg Leu Pro Trp Asn Gln Thr Phe Asp Pro
35 40 45

Thr Ser Asp Leu Arg Lys Ile Asp Val Asn Ser Phe Pro Ser Thr Val
50 55 60

Asn Cys Glu Glu Asp Thr Gly Val Ser Ser Pro Asn Ser Thr Ile Ser
65 70 75 80

Ser Thr Ile Ser Gly Lys Arg Ser Glu Arg Glu Gly Ile Ser Gly Thr
85 90 95

Gly Val Gly Ser Gly Asp Asp His Asp Glu Ile Thr Pro Asp Arg Gly
100 105 110

Tyr Ser Arg Gly Thr Ser Asp Glu Glu Glu Asp Gly Gly Glu Thr Ser
115 120 125

Arg Lys Lys Leu Arg Leu Ser Lys Asp Gln Ser Ala Phe Leu Glu Glu
130 135 140

Thr Phe Lys Glu His Asn Thr Leu Asn Pro Lys Gln Lys Leu Ala Leu
145 150 155 160

Ala Lys Lys Leu Asn Leu Thr Ala Arg Gln Val Glu Val Trp Phe Gln
165 170 175

Asn Arg Arg Ala Arg Thr Lys Leu Lys Gln Thr Glu Val Asp Cys Glu
180 185 190

Tyr Leu Lys Arg Cys Val Glu Lys Leu Thr Glu Glu Asn Arg Arg Leu
195 200 205

Gln Lys Glu Ala Met Glu Leu Arg Thr Leu Lys Leu Ser Pro Gln Phe
210 215 220

Tyr Gly Gln Met Thr Pro Pro Thr Thr Leu Ile Met Cys Pro Ser Cys
225 230 235 240

Glu Arg Val Gly Gly Pro Ser Ser Ser Asn His His His Asn His Arg
245 250 255

Pro Val Ser Ile Asn Pro Trp Val Ala Cys Ala Gly Gln Val Ala His
260 265 270

Gly Leu Asn Phe Glu Ala Leu Arg Pro Arg Ser
275 280

<210> 259
<211> 957
<212> DNA
<213> Arabidopsis thaliana

<400> 259
atgggggaaa gagatgatgg gttgggtttg agtctaagct tgggaaatag tcaacaaaaa 60

gaaccatctc tgaggttgaa tcttatgccg ttgacaactt cttcttcttc ttcttcgttt 120

caacacatgc acaatcagaa taacaatagc catccccaga agattcataa catctcttgg 180

actcatctgt ttcaatcttc tgggattaaa cgtacaactg cagagagaaa ctccgacgcc 240

gggtcatttc taagaggttt caacgtgaac agagctcagt cttcggtggc ggtagtggac 300

ttggaagaag aagccgccgt cgtctcgtct ccaaacagcg ccgtttcgag tctgagtgga 360

aataaaaggg atcttgcggt ggcgagagga ggagatgaaa acgaggcgga gagagcttct 420

tgctcacgcg gagggggaag cggtggtagc gacgatgaag acggcggaaa cggcgacgga 480

tcaaggaaga aactacggtt atcgaaggat caagctcttg ttctcgagga gacttttaaa 540

gaacatagca ctcttaatcc gaagcaaaag ctggctctag caaaacagtt gaatctaagg 600

gcaagacaag ttgaagtgtg gtttcagaac cgtagggcaa ggacgaagct gaaacaaacg 660

gaggttgatt gtgagtattt aaagagatgt tgcgataatc tgaccgagga gaatcgacgg 720

ctgcagaaag aagtgtcgga gctgagggcg ttgaagttgt ctccacatct ctacatgcac 780

atgactcctc ctactactct caccatgtgc ccttcttgcg aacgtgtctc ctcctctgcc 840

gccactgtga ccgctgctcc ttccactact actactccta cggtggtggg gcggccaagt 900

ccacagcgat taactccttg gactgctatt tctctccagc aaaaatcagg tcgctag 957

<210> 260
<211> 318
<212> PRT
<213> Arabidopsis thaliana

<400> 260

Met Gly Glu Arg Asp Asp Gly Leu Gly Leu Ser Leu Ser Leu Gly Asn
1 5 10 15

Ser Gln Gln Lys Glu Pro Ser Leu Arg Leu Asn Leu Met Pro Leu Thr
20 25 30

Thr Ser Ser Ser Ser Ser Ser Phe Gln His Met His Asn Gln Asn Asn
35 40 45

Asn Ser His Pro Gln Lys Ile His Asn Ile Ser Trp Thr His Leu Phe
50 55 60

Gln Ser Ser Gly Ile Lys Arg Thr Thr Ala Glu Arg Asn Ser Asp Ala
65 70 75 80

Gly Ser Phe Leu Arg Gly Phe Asn Val Asn Arg Ala Gln Ser Ser Val
85 90 95

Ala Val Val Asp Leu Glu Glu Glu Ala Ala Val Val Ser Ser Pro Asn
100 105 110

Ser Ala Val Ser Ser Leu Ser Gly Asn Lys Arg Asp Leu Ala Val Ala
115 120 125

Arg Gly Gly Asp Glu Asn Glu Ala Glu Arg Ala Ser Cys Ser Arg Gly
130 135 140

Gly Gly Ser Gly Gly Ser Asp Asp Glu Asp Gly Gly Asn Gly Asp Gly
145 150 155 160

Ser Arg Lys Lys Leu Arg Leu Ser Lys Asp Gln Ala Leu Val Leu Glu
165 170 175

Glu Thr Phe Lys Glu His Ser Thr Leu Asn Pro Lys Gln Lys Leu Ala
180 185 190

Leu Ala Lys Gln Leu Asn Leu Arg Ala Arg Gln Val Glu Val Trp Phe
195 200 205

Gln Asn Arg Arg Ala Arg Thr Lys Leu Lys Gln Thr Glu Val Asp Cys
210 215 220

Glu Tyr Leu Lys Arg Cys Cys Asp Asn Leu Thr Glu Glu Asn Arg Arg
225 230 235 240

Leu Gln Lys Glu Val Ser Glu Leu Arg Ala Leu Lys Leu Ser Pro His
245 250 255

Leu Tyr Met His Met Thr Pro Pro Thr Thr Leu Thr Met Cys Pro Ser
260 265 270

Cys Glu Arg Val Ser Ser Ser Ala Ala Thr Val Thr Ala Ala Pro Ser
275 280 285

Thr Thr Thr Thr Pro Thr Val Val Gly Arg Pro Ser Pro Gln Arg Leu
290 295 300

Thr Pro Trp Thr Ala Ile Ser Leu Gln Gln Lys Ser Gly Arg
305 310 315

<210> 261
<211> 1357
<212> DNA
<213> Arabidopsis thaliana

<400> 261
ccacaacccc atatctcttt gtctgaaaaa actcttttgc atatataaat aaatataagc 60

catatctttt gaaactgtac tgctgcacaa gtgtagaggc agtggcacaa catctttaag 120

aaagagagag agaaagagtc atttattcat ttcctcgctt aaaaatcttg agcacccaga 180

cagaattctt ctttcttctt tctggggttg agaaaaatga gtgaaagaga tgatggattg 240

gggctaagtt tgagcttgag tttaggtttt aatcaaaagg acccgtcttc gaggttaaat 300

ccaatgcctc tggcttctta tgcatcttca tcacacatgc agcatatgca gcagagcaat 360

tataaccatc ctcaaaagat tcagaacact tggattaaca tgtttcagtc atcagagaga 420

aactcggaca tgagatcgtt tctccgggga atagacgtga acagagctcc atcgacggtg 480

gtggttgacg tggaggatga aggcgccgga gtttcgtctc cgaacagcac cgtctcaagc 540

gtgatgagcg ggaagaagag cgagcgagag ctaatggctg cggcaggtgc agttggagga 600

ggtagagtag aagataatga gattgagaga gcttcttgct cgctcggcgg tggtagcgac 660

gatgaagacg gtagcgggaa cggagatgac agttcgagga agaaactccg attgtctaaa 720

gaacaagctt tggttcttga agaaactttt aaagaacata gtacactcaa tccgaagcaa 780

aagatggctt tggctaagca attgaatctg aggacgagac aagttgaagt gtggttccaa 840

aaccgaaggg caaggacgaa gctgaagcaa acggaagtag actgtgaata tcttaagaga 900

tgttgcgaga atctaacgga tgagaatcgg agattgcaaa aggaagtgag tgagcttagg 960

gctttaaagc tttctccaca cttatacatg cacatgaaac ctcccactac tctcacaatg 1020

tgtccttctt gcgagcgagt cgctgttacg tcatcttcgt catcggtggc tcctcctgtg 1080

atgaattcat cgtctccgat gggtccgatg agtccgtggg ctgccatgcc tctacggcaa 1140

cgacctgctg ctggttctca ttagagttta attagtctaa agataatagg tttggtgatt 1200

tgtttttatt atattgttga acggacttgg aagttctttt caagagttgg tcccatgctc 1260

ttctttcctt tgttttattc agtttctatt tatagctgaa ttctggataa tggaaaatct 1320

actaatatta ttgtccaatt attagtttgg ggaaaga 1357

<210> 262
<211> 315
<212> PRT
<213> Arabidopsis thaliana

<400> 262

Met Ser Glu Arg Asp Asp Gly Leu Gly Leu Ser Leu Ser Leu Ser Leu
1 5 10 15

Gly Phe Asn Gln Lys Asp Pro Ser Ser Arg Leu Asn Pro Met Pro Leu
20 25 30

Ala Ser Tyr Ala Ser Ser Ser His Met Gln His Met Gln Gln Ser Asn
35 40 45

Tyr Asn His Pro Gln Lys Ile Gln Asn Thr Trp Ile Asn Met Phe Gln
50 55 60

Ser Ser Glu Arg Asn Ser Asp Met Arg Ser Phe Leu Arg Gly Ile Asp
65 70 75 80

Val Asn Arg Ala Pro Ser Thr Val Val Val Asp Val Glu Asp Glu Gly
85 90 95

Ala Gly Val Ser Ser Pro Asn Ser Thr Val Ser Ser Val Met Ser Gly
100 105 110

Lys Lys Ser Glu Arg Glu Leu Met Ala Ala Ala Gly Ala Val Gly Gly
115 120 125

Gly Arg Val Glu Asp Asn Glu Ile Glu Arg Ala Ser Cys Ser Leu Gly
130 135 140

Gly Gly Ser Asp Asp Glu Asp Gly Ser Gly Asn Gly Asp Asp Ser Ser

145 150 155 160

Arg Lys Lys Leu Arg Leu Ser Lys Glu Gln Ala Leu Val Leu Glu Glu
165 170 175

Thr Phe Lys Glu His Ser Thr Leu Asn Pro Lys Gln Lys Met Ala Leu
180 185 190

Ala Lys Gln Leu Asn Leu Arg Thr Arg Gln Val Glu Val Trp Phe Gln
195 200 205

Asn Arg Arg Ala Arg Thr Lys Leu Lys Gln Thr Glu Val Asp Cys Glu
210 215 220

Tyr Leu Lys Arg Cys Cys Glu Asn Leu Thr Asp Glu Asn Arg Arg Leu
225 230 235 240

Gln Lys Glu Val Ser Glu Leu Arg Ala Leu Lys Leu Ser Pro His Leu
245 250 255

Tyr Met His Met Lys Pro Pro Thr Thr Leu Thr Met Cys Pro Ser Cys
260 265 270

Glu Arg Val Ala Val Thr Ser Ser Ser Ser Ser Val Ala Pro Pro Val
275 280 285

Met Asn Ser Ser Ser Pro Met Gly Pro Met Ser Pro Trp Ala Ala Met
290 295 300

Pro Leu Arg Gln Arg Pro Ala Ala Gly Ser His
305 310 315

<210> 263
<211> 744
<212> DNA
<213> Oryza sativa

<400> 263
atgatggaga gggccgagga cctgcgcctg agcctcagtc tcagctcgcc gcttattgct 60

cctcgtactc accatgtcgc catgctgttc cacgctcctc cagagaaaag attcctggag 120

atgccgctgc tccctgctgc gaagcggagc gaggtcgtcg cggcagaaga ggagcgcgcg 180

ggcctgcgcg gcggcggcgg cagcgacgag gaggacggtg gctgcggcat cgacggctca 240

cgcaagaagc tccggctttc caaggaccag tccgccgtgc tcgaggacag cttccgggag 300

caccccaccc tcaaccccag gcagaaggca actttggcgc agcagctcgg gcttcggcct 360

cggcaggtcg aggtgtggtt tcagaacaga cgcgcaagga cgaagctgaa gcagacggag 420

gtggactgcg agttcctgaa gcgctgctgc gagacgctca cggaggagaa ccggaggctg 480

cagaaggagg tgcaggagct gcgagcgctc aagctcgtct cgccgcacct ctacatgaac 540

atgtccccgc ccaccacgct caccatgtgc ccctcctgcg agcgcgtctc caacaccaat 600

aacaactcca gcgccgccgc cgccgccgac cgccgcggca tcaggactac tactgccgca 660

ggcggcggca gcgtcgtcga caccgccgcc gacgggggca tcctctgcca ccgcccgatc 720

gccgtccggc cgcagcagtc atga 744

<210> 264
<211> 247
<212> PRT
<213> Oryza sativa

<400> 264

```
Met Met Glu Arg Ala Glu Asp Leu Arg Leu Ser Leu Ser Leu Ser Ser
1               5                   10                  15

Pro Leu Ile Ala Pro Arg Thr His His Val Ala Met Leu Phe His Ala
            20                  25                  30

Pro Pro Glu Lys Arg Phe Leu Glu Met Pro Leu Leu Pro Ala Ala Lys
        35                  40                  45

Arg Ser Glu Val Val Ala Ala Glu Glu Glu Arg Ala Gly Leu Arg Gly
    50                  55                  60

Gly Gly Gly Ser Asp Glu Glu Asp Gly Gly Cys Gly Ile Asp Gly Ser
65                  70                  75                  80

Arg Lys Lys Leu Arg Leu Ser Lys Asp Gln Ser Ala Val Leu Glu Asp
                85                  90                  95

Ser Phe Arg Glu His Pro Thr Leu Asn Pro Arg Gln Lys Ala Thr Leu
            100                 105                 110

Ala Gln Gln Leu Gly Leu Arg Pro Arg Gln Val Glu Val Trp Phe Gln
        115                 120                 125

Asn Arg Arg Ala Arg Thr Lys Leu Lys Gln Thr Glu Val Asp Cys Glu
    130                 135                 140

Phe Leu Lys Arg Cys Cys Glu Thr Leu Thr Glu Glu Asn Arg Arg Leu
145                 150                 155                 160

Gln Lys Glu Val Gln Glu Leu Arg Ala Leu Lys Leu Val Ser Pro His
                165                 170                 175

Leu Tyr Met Asn Met Ser Pro Pro Thr Thr Leu Thr Met Cys Pro Ser
            180                 185                 190
```

Cys Glu Arg Val Ser Asn Thr Asn Asn Asn Ser Ser Ala Ala Ala Ala
195 200 205

Ala Asp Arg Arg Gly Ile Arg Thr Thr Thr Ala Ala Gly Gly Gly Ser
210 215 220

Val Val Asp Thr Ala Ala Asp Gly Gly Ile Leu Cys His Arg Pro Ile
225 230 235 240

Ala Val Arg Pro Gln Gln Ser
245

<210> 265
<211> 846
<212> DNA
<213> Oryza sativa

<400> 265
atgaggtcgt acatggacgg cggcggcgcg gcggcgtacg aggaggagga ggaggaggtt 60

gaggacgacg acggcggcgg cggcggcggc ggcggcggcg gcggtggggg gctcggggag 120

aagaagcggc ggctggcggc ggagcaggtg cgggcgctgg agcggagctt cgaggcggac 180

aacaagctgg acccggagcg gaaggcccgg atcgcccgcg accttcgcct ccaccctcgc 240

caggtcgccg tctggttcca gaaccgccgc gcgaggtgga agaccaagca gatcgagcgc 300

gacttcgccg ccctccgctc ccgccacgac gccctccgcc tcgagtgcga cgccctccgc 360

cgcgacaagg acgccctcgc cgccgagatc gccgacctcc gggacagggt ggacggccag 420

atgtccgtca agctggaggc cgtggccgcg gacgaacacc agccgcctcc gccgccgccg 480

ccgccgccac tggcgtataa cagcaaggtg gtggacggct cgacggacag cgactcgagc 540

gcggtgttca acgaggaggc gtcgccgtac tccggcgcgg ccatcgacca ccaccaccac 600

caaactccgg cgagctacga cacggcgggg ttcacctcct tcttcgcgcc atccaccacg 660

ctcacctcgt ccctctcctt cccttccatg ttccacgcgt catcgcattt cgatggccac 720

caagaactcc tcgtcggcgg cggcggcgcc ggcgcagtgg ccgacgccga cctcggaggc 780

gccggattct tcgccggcga cgagcacgcc ggcggcctct cctggtacgg cgccgagggt 840

tggtag 846

<210> 266
<211> 281
<212> PRT
<213> Oryza sativa

<400> 266

Met Arg Ser Tyr Met Asp Gly Gly Gly Ala Ala Ala Tyr Glu Glu Glu
1 5 10 15

Glu Glu Glu Val Glu Asp Asp Asp Gly Gly Gly Gly Gly Gly Gly Gly
20 25 30

Gly Gly Gly Gly Gly Leu Gly Glu Lys Lys Arg Arg Leu Ala Ala Glu
35 40 45

Gln Val Arg Ala Leu Glu Arg Ser Phe Glu Ala Asp Asn Lys Leu Asp
50 55 60

Pro Glu Arg Lys Ala Arg Ile Ala Arg Asp Leu Arg Leu His Pro Arg
65 70 75 80

Gln Val Ala Val Trp Phe Gln Asn Arg Arg Ala Arg Trp Lys Thr Lys
85 90 95

Gln Ile Glu Arg Asp Phe Ala Ala Leu Arg Ser Arg His Asp Ala Leu
100 105 110

Arg Leu Glu Cys Asp Ala Leu Arg Arg Asp Lys Asp Ala Leu Ala Ala
115 120 125

Glu Ile Ala Asp Leu Arg Asp Arg Val Asp Gly Gln Met Ser Val Lys
130 135 140

Leu Glu Ala Val Ala Ala Asp Glu His Gln Pro Pro Pro Pro Pro Pro
145 150 155 160

Pro Pro Pro Leu Ala Tyr Asn Ser Lys Val Val Asp Gly Ser Thr Asp
165 170 175

Ser Asp Ser Ser Ala Val Phe Asn Glu Glu Ala Ser Pro Tyr Ser Gly
180 185 190

Ala Ala Ile Asp His His His His Gln Thr Pro Ala Ser Tyr Asp Thr
195 200 205

Ala Gly Phe Thr Ser Phe Phe Ala Pro Ser Thr Thr Leu Thr Ser Ser
210 215 220

Leu Ser Phe Pro Ser Met Phe His Ala Ser Ser His Phe Asp Gly His
225 230 235 240

Gln Glu Leu Leu Val Gly Gly Gly Gly Ala Gly Ala Val Ala Asp Ala
245 250 255

Asp Leu Gly Gly Ala Gly Phe Phe Ala Gly Asp Glu His Ala Gly Gly
260 265 270

Leu Ser Trp Tyr Gly Ala Glu Gly Trp

275 280

<210> 267
<211> 918
<212> DNA
<213> Oryza sativa

<400> 267
atgaagaggc ccagctgcag aggctcctcc atggccatca tccatgacac ctctgatcaa 60

caagaggaca acatgaggtc gtacatggac ggcggcggcg cggcggcgta cgaggaggag 120

gaggaggagg ttgaggacga cgacggcggc ggcggcggcg gcggcggcgg cggcggtggg 180

gggctcgggg agaagaagcg gcggctggcg gcggagcagg tgcgggcgct ggagcggagc 240

ttcgaggcgg acaacaagct ggacccggag cggaaggccc ggatcgcccg cgaccttcgc 300

ctccaccctc gccaggtcgc cgtctggttc cagaaccgcc gcgcgaggtg gaagaccaag 360

cagatcgagc gcgacttcgc cgccctccgc tcccgccacg acgccctccg cctcgagtgc 420

gacgccctcc gccgcgacaa ggacgccctc gccgccgaga tcgccgacct ccgggacagg 480

gtggacggcc agatgtccgt caagctggag gccgtggccg cggacgaaca ccagccgcct 540

ccgccgccgc cgccgccgcc actggcgtat aacagcaagg tggtggacgg ctcgacggac 600

agcgactcga gcgcggtgtt caacgaggag gcgtcgccgt actccggcgc ggccatcgac 660

caccaccacc accaaactcc ggcgagctac gacacggcgg ggttcacctc cttcttcgcg 720

ccatccacca cgctcacctc gtccctctcc ttcccttcca tgttccacgc gtcatcgcat 780

ttcgatggcc accaagaact cctcgtcggc ggcggcggcg ccggcgcagt ggccgacgcc 840

gacctcggag gcgccggatt cttcgccggc gacgagcacg ccggcggcct ctcctggtac 900

ggcgccgagg gttggtag 918

<210> 268
<211> 305
<212> PRT
<213> Oryza sativa

<400> 268

Met Lys Arg Pro Ser Cys Arg Gly Ser Ser Met Ala Ile Ile His Asp
1 5 10 15

Thr Ser Asp Gln Gln Glu Asp Asn Met Arg Ser Tyr Met Asp Gly Gly
20 25 30

Gly Ala Ala Ala Tyr Glu Glu Glu Glu Glu Glu Val Glu Asp Asp Asp
35 40 45

Gly Gly Gly Gly Gly Gly Gly Gly Gly Gly Gly Gly Gly Leu Gly Glu
50 55 60

Lys Lys Arg Arg Leu Ala Ala Glu Gln Val Arg Ala Leu Glu Arg Ser
65 70 75 80

Phe Glu Ala Asp Asn Lys Leu Asp Pro Glu Arg Lys Ala Arg Ile Ala
85 90 95

Arg Asp Leu Arg Leu His Pro Arg Gln Val Ala Val Trp Phe Gln Asn
100 105 110

Arg Arg Ala Arg Trp Lys Thr Lys Gln Ile Glu Arg Asp Phe Ala Ala
115 120 125

Leu Arg Ser Arg His Asp Ala Leu Arg Leu Glu Cys Asp Ala Leu Arg
130 135 140

Arg Asp Lys Asp Ala Leu Ala Ala Glu Ile Ala Asp Leu Arg Asp Arg
145 150 155 160

Val Asp Gly Gln Met Ser Val Lys Leu Glu Ala Val Ala Ala Asp Glu
165 170 175

His Gln Pro Pro Pro Pro Pro Pro Pro Pro Pro Leu Ala Tyr Asn Ser
180 185 190

Lys Val Val Asp Gly Ser Thr Asp Ser Asp Ser Ser Ala Val Phe Asn
195 200 205

Glu Glu Ala Ser Pro Tyr Ser Gly Ala Ala Ile Asp His His His His
210 215 220

Gln Thr Pro Ala Ser Tyr Asp Thr Ala Gly Phe Thr Ser Phe Phe Ala
225 230 235 240

Pro Ser Thr Thr Leu Thr Ser Ser Leu Ser Phe Pro Ser Met Phe His
245 250 255

Ala Ser Ser His Phe Asp Gly His Gln Glu Leu Leu Val Gly Gly Gly
260 265 270

Gly Ala Gly Ala Val Ala Asp Ala Asp Leu Gly Gly Ala Gly Phe Phe
275 280 285

Ala Gly Asp Glu His Ala Gly Gly Leu Ser Trp Tyr Gly Ala Glu Gly
290 295 300

Trp
305

<210> 269

<211> 936
<212> DNA
<213> Oryza sativa

<400> 269
atgaagaggc ccaccagcag cagccgaaaa tccaaaaaac aaggagagga cctggcgttc 60

tctgaggagg gcagcttgcc cgcggtgacc atggagcaga aagatgaagc cgagatggag 120

gaggtggacg aggaggagga ggaggaggtc gacgaagaca tggccggcgg gcacgcggcg 180

cagtcgccgt cgccgtcgtg cgggctgggc gagaagaagc ggcggctggc gctggagcag 240

gtgcgcgctc tggagcggag cttcgacacg gacaacaagc tggacccgga ccgcaaggcc 300

cgcatcgcgc gcgacctcgg cctgcagccg cgccaggtcg ccgtctggtt ccagaaccgc 360

cgcgcccggt ggaagacgaa gcagctcgag cgcgacttcg ccgccctccg cgcccgccac 420

gacgccctcc gcgccgactg cgacgccctg cgccgcgaca aggacgccct cgccgccgag 480

attcgggagc tgagggagaa gctgcccacc aagccggcgg acacggcggc atcggtgaag 540

gtagaagccg gcaatgacgc ggcggccggc gccgcggccg ccacggtgtg caaggacggc 600

tcgtcggacg acagcgactc cagcgtggtg ttcaacgacg aggcgtcgcc gtactccggc 660

gcggccttca ttggattcgg cccgtcgttc ttggtcgacg acgcgtcggc ggcaaccgtg 720

ggctgctcgt cgtcgctccc cgcgctcgag tccaaatggc acggtccgta ctccgacgac 780

tcgtgcaaag gcggcgtcta tggcttcacg gaggaatggc tcgctgcctg ctccggcgag 840

atggccggca acgacgccgc cggcttcttc tccgacgagc acgcctccaa cctcaacttc 900

ggttggtgcg cgagtggtaa cgagggttgg gaatga 936

<210> 270
<211> 311
<212> PRT
<213> Oryza sativa

<400> 270

Met Lys Arg Pro Thr Ser Ser Ser Arg Lys Ser Lys Lys Gln Gly Glu
1 5 10 15

Asp Leu Ala Phe Ser Glu Glu Gly Ser Leu Pro Ala Val Thr Met Glu
20 25 30

Gln Lys Asp Glu Ala Glu Met Glu Glu Val Asp Glu Glu Glu Glu Glu
35 40 45

Glu Val Asp Glu Asp Met Ala Gly Gly His Ala Ala Gln Ser Pro Ser
50 55 60

Pro Ser Cys Gly Leu Gly Glu Lys Lys Arg Arg Leu Ala Leu Glu Gln
65 70 75 80

```
Val Arg Ala Leu Glu Arg Ser Phe Asp Thr Asp Asn Lys Leu Asp Pro
                85                  90                  95

Asp Arg Lys Ala Arg Ile Ala Arg Asp Leu Gly Leu Gln Pro Arg Gln
            100                 105                 110

Val Ala Val Trp Phe Gln Asn Arg Arg Ala Arg Trp Lys Thr Lys Gln
        115                 120                 125

Leu Glu Arg Asp Phe Ala Ala Leu Arg Ala Arg His Asp Ala Leu Arg
    130                 135                 140

Ala Asp Cys Asp Ala Leu Arg Arg Asp Lys Asp Ala Leu Ala Ala Glu
145                 150                 155                 160

Ile Arg Glu Leu Arg Glu Lys Leu Pro Thr Lys Pro Ala Asp Thr Ala
                165                 170                 175

Ala Ser Val Lys Val Glu Ala Gly Asn Asp Ala Ala Ala Gly Ala Ala
            180                 185                 190

Ala Ala Thr Val Cys Lys Asp Gly Ser Ser Asp Asp Ser Asp Ser Ser
        195                 200                 205

Val Val Phe Asn Asp Glu Ala Ser Pro Tyr Ser Gly Ala Ala Phe Ile
    210                 215                 220

Gly Phe Gly Pro Ser Phe Leu Val Asp Asp Ala Ser Ala Ala Thr Val
225                 230                 235                 240

Gly Cys Ser Ser Ser Leu Pro Ala Leu Glu Ser Lys Trp His Gly Pro
                245                 250                 255

Tyr Ser Asp Asp Ser Cys Lys Gly Gly Val Tyr Gly Phe Thr Glu Glu
            260                 265                 270

Trp Leu Ala Ala Cys Ser Gly Glu Met Ala Gly Asn Asp Ala Ala Gly
        275                 280                 285

Phe Phe Ser Asp Glu His Ala Ser Asn Leu Asn Phe Gly Trp Cys Ala
    290                 295                 300

Ser Gly Asn Glu Gly Trp Glu
305                 310

<210>  271
<211>  810
<212>  DNA
<213>  Oryza sativa
```

<400> 271
atgagaagtc cagcagcgct tctcccggtg gttgcagatg gtggtggtgg tgttggggtg 60

gaggaggaga tggatgtgga cgaggacatg gcgatgtgcg gcggccgcgg cggcggcggc 120

ggggagaaga agcggcggct gagcgtggag caggtgcgcg cgctggagcg gagcttcgag 180

acggagaaca agctggagcc ggagcggaag gcgcggctgg cgcgcgacct cgggctgcag 240

ccgcgccagg tcgccgtctg gttccagaac cgccgcgcgc ggtggaagac caagcagctc 300

gagcgcgact acgccgcgct ccgccaatcc tacgacgcgc tccgcgccga ccacgacgcg 360

cttcgccgcg acaaggacgc cctcctcgcc gagatcaagg agctgaaggg gaagctcggc 420

gacgaggacg ccgcggcgag cttctcgtcg gtgaaggagg aggaggaccc ggcggcgtcc 480

gacgccgacc ccccggccac cggcgcgccg cagggctcgt ccgagagcga ctcgagcgcg 540

gtgctgaacg acgcggagat ccttccacac aagccagcgc cggcggccgc cgccgacgcc 600

gcggcctcgg aggagacgga ggcggtggtg accggcgccg cgctgctcca ccacgccgag 660

gtgttcttcc acgggcagct tctcaaggtg gacgacgacg aggcggcgtt cctaggcgac 720

gacggcgcgg cgtgcggcgg cttcttcgcc gacgagcatc tcccgtcgct gccgtggtgg 780

gccgagccca ccgagcaatg gacgacctag 810

<210> 272
<211> 269
<212> PRT
<213> Oryza sativa

<400> 272

Met Arg Ser Pro Ala Ala Leu Leu Pro Val Val Ala Asp Gly Gly Gly
1 5 10 15

Gly Val Gly Val Glu Glu Glu Met Asp Val Asp Glu Asp Met Ala Met
20 25 30

Cys Gly Gly Arg Gly Gly Gly Gly Gly Glu Lys Lys Arg Arg Leu Ser
35 40 45

Val Glu Gln Val Arg Ala Leu Glu Arg Ser Phe Glu Thr Glu Asn Lys
50 55 60

Leu Glu Pro Glu Arg Lys Ala Arg Leu Ala Arg Asp Leu Gly Leu Gln
65 70 75 80

Pro Arg Gln Val Ala Val Trp Phe Gln Asn Arg Arg Ala Arg Trp Lys
85 90 95

Thr Lys Gln Leu Glu Arg Asp Tyr Ala Ala Leu Arg Gln Ser Tyr Asp
100 105 110

```
Ala Leu Arg Ala Asp His Asp Ala Leu Arg Arg Asp Lys Asp Ala Leu
        115                 120                 125

Leu Ala Glu Ile Lys Glu Leu Lys Gly Lys Leu Gly Asp Glu Asp Ala
    130                 135                 140

Ala Ala Ser Phe Ser Ser Val Lys Glu Glu Glu Asp Pro Ala Ala Ser
145                 150                 155                 160

Asp Ala Asp Pro Pro Ala Thr Gly Ala Pro Gln Gly Ser Ser Glu Ser
                165                 170                 175

Asp Ser Ser Ala Val Leu Asn Asp Ala Glu Ile Leu Pro His Lys Pro
            180                 185                 190

Ala Pro Ala Ala Ala Ala Asp Ala Ala Ala Ser Glu Glu Thr Glu Ala
        195                 200                 205

Val Val Thr Gly Ala Ala Leu Leu His His Ala Glu Val Phe Phe His
    210                 215                 220

Gly Gln Leu Leu Lys Val Asp Asp Asp Glu Ala Ala Phe Leu Gly Asp
225                 230                 235                 240

Asp Gly Ala Ala Cys Gly Gly Phe Phe Ala Asp Glu His Leu Pro Ser
                245                 250                 255

Leu Pro Trp Trp Ala Glu Pro Thr Glu Gln Trp Thr Thr
            260                 265
```

<210> 273
<211> 834
<212> DNA
<213> Oryza sativa

<400> 273

```
atgaagcgac ccggcggtgc cggcggcggc ggaggcagcc catcgctcgt cacgatggct      60

aattctagtg atgatggata tggaggggtt gggatggagg cggaggggga cgtggaggag     120

gagatgatgg cgtgcggcgg cggcggggag aagaagcggc ggctgagcgt ggagcaggtt     180

cgcgcgctgg agcggagctt cgaggtggag aacaagcttg agcctgagcg gaaggcgcgg     240

ctggcgcgcg acctcggcct gcagccgcgc caggtcgccg tctggttcca gaaccgccgc     300

gcgcggtgga agaccaagca gctcgagcgc gactacgccg cgctccgcca ttcctacgac     360

tccctgcgcc tcgatcacga cgcgctccgc cgcgacaagg acgccctcct cgccgagatc     420

aaggagctga aggcgaagct cggggacgag gaggcggcgg cgagcttcac gtcggtgaag     480

gaggagccgg cggcctccga cgggccaccg gcggcgggat ttgggtcgtc cgacagcgac     540

tcaagcgcgg tgctgaacga cgtggacgcg gccggcgccg cgcccgcggc gacggacgcg     600
```

ctggctccgg aggcgtgcac gtttctcggc gcgccgcccg ccgcgggcgc gggcgcgggc 660

gcagcggcgg cggcgagcca cgaggaggtg ttcttccacg gcaatttcct caaggtggag 720

gaggacgaga cggggttcct cgacgacgac gagccgtgcg gcgggttctt cgccgacgat 780

cagccccgc cgctgtcgtc gtggtgggcc gagcccacgg agcactggaa ctga 834

<210> 274
<211> 277
<212> PRT
<213> Oryza sativa

<400> 274

Met Lys Arg Pro Gly Gly Ala Gly Gly Gly Gly Gly Ser Pro Ser Leu
1 5 10 15

Val Thr Met Ala Asn Ser Ser Asp Asp Gly Tyr Gly Gly Val Gly Met
20 25 30

Glu Ala Glu Gly Asp Val Glu Glu Glu Met Met Ala Cys Gly Gly Gly
35 40 45

Gly Glu Lys Lys Arg Arg Leu Ser Val Glu Gln Val Arg Ala Leu Glu
50 55 60

Arg Ser Phe Glu Val Glu Asn Lys Leu Glu Pro Glu Arg Lys Ala Arg
65 70 75 80

Leu Ala Arg Asp Leu Gly Leu Gln Pro Arg Gln Val Ala Val Trp Phe
85 90 95

Gln Asn Arg Arg Ala Arg Trp Lys Thr Lys Gln Leu Glu Arg Asp Tyr
100 105 110

Ala Ala Leu Arg His Ser Tyr Asp Ser Leu Arg Leu Asp His Asp Ala
115 120 125

Leu Arg Arg Asp Lys Asp Ala Leu Leu Ala Glu Ile Lys Glu Leu Lys
130 135 140

Ala Lys Leu Gly Asp Glu Glu Ala Ala Ala Ser Phe Thr Ser Val Lys
145 150 155 160

Glu Glu Pro Ala Ala Ser Asp Gly Pro Pro Ala Ala Gly Phe Gly Ser
165 170 175

Ser Asp Ser Asp Ser Ser Ala Val Leu Asn Asp Val Asp Ala Ala Gly
180 185 190

Ala Ala Pro Ala Ala Thr Asp Ala Leu Ala Pro Glu Ala Cys Thr Phe
195 200 205

Leu Gly Ala Pro Pro Ala Ala Gly Ala Gly Ala Gly Ala Ala Ala Ala
210 215 220

Ala Ser His Glu Glu Val Phe Phe His Gly Asn Phe Leu Lys Val Glu
225 230 235 240

Glu Asp Glu Thr Gly Phe Leu Asp Asp Asp Glu Pro Cys Gly Gly Phe
245 250 255

Phe Ala Asp Asp Gln Pro Pro Pro Leu Ser Ser Trp Trp Ala Glu Pro
260 265 270

Thr Glu His Trp Asn
275

<210> 275
<211> 375
<212> DNA
<213> Oryza sativa

<400> 275
atgtcatcct ctctctttct tcttcctcct ctctttcttt ctctcttctc tccccttcgg 60

ctagccgcag ggagcacggg ctggggcggt agtgggcggg gacaagagag gcggagggag 120

ctgaagcggc gccggcggcg gcacacgctc tccagcctca gcggcaagcg tggtgcgcca 180

tctgccgctg ccgccgccgt cggcggcagc gacgacgagg actccgacga cggatcccgc 240

aagaagctcc gcctctccaa ggaccaagcc gccgtcctcg aggacacctt caacaagcac 300

aacaccctca accccaagca gaaggcggcg ctggcgaggc agctgaatct gaagccgcgg 360

caggtggagg tgtag 375

<210> 276
<211> 124
<212> PRT
<213> Oryza sativa

<400> 276

Met Ser Ser Ser Leu Phe Leu Leu Pro Pro Leu Phe Leu Ser Leu Phe
1 5 10 15

Ser Pro Leu Arg Leu Ala Ala Gly Ser Thr Gly Trp Gly Gly Ser Gly
20 25 30

Arg Gly Gln Glu Arg Arg Arg Glu Leu Lys Arg Arg Arg Arg Arg His
35 40 45

Thr Leu Ser Ser Leu Ser Gly Lys Arg Gly Ala Pro Ser Ala Ala Ala

50 55 60

Ala Ala Val Gly Gly Ser Asp Asp Glu Asp Ser Asp Asp Gly Ser Arg
65 70 75 80

Lys Lys Leu Arg Leu Ser Lys Asp Gln Ala Ala Val Leu Glu Asp Thr
85 90 95

Phe Asn Lys His Asn Thr Leu Asn Pro Lys Gln Lys Ala Ala Leu Ala
100 105 110

Arg Gln Leu Asn Leu Lys Pro Arg Gln Val Glu Val
115 120

<210> 277
<211> 1767
<212> DNA
<213> Oryza sativa

<400> 277
atgttttatt caagtccctt ttgttcatct cctttgcaga ttccatttct gacccaaatt 60
ttggctattc ctcatgatga gtttgtctca aactggtgct ctgttaatct gccagtgata 120
gaagaagacg ctaatcttga ttatgatcca tttggtgcag ctgagctggc actggcagct 180
gctggtaata agttaactga agctaaagca aactattctt gcccttttcg ccctatcagt 240
atgccttcta tagcatatgc gcagacaaga acatcatgtg tggtgaaaat aatagcaaat 300
ttgcatgttt ttgtcccaaa catatgcgaa gagcaagaaa gagacctttt tcttcagaaa 360
tttcagaagt acttggtatc agggaatcct agatcatcag ttgatcatcc agcatcagct 420
gatctcaagg ccactacagt ttgcagaaac ttgggatctt tgtctgagta tgctagatcg 480
ttaattccta ataacttgtt aaacgaggaa gatgtgcaat tgttaagtga atttgcttat 540
aagttacaaa cttggtgtaa atcacatgtt ggacagagta catcccaggc agtaaagatt 600
gatccgtcat cagaaagcaa ggaagacttt aagccactgc agcatccctt gataccaagt 660
actgttgttc cagattccag tataaataac cttccgaaga acatggaaga gcctacacca 720
acaaacatgg aagagcctgc accaacaccc tcaacaaagc aagagggaaa tgccagggat 780
gagactccta gaagcactgt cgctttaaat ggtgggttcc tgcagaattc agtcggccag 840
gacttagtcc atcttggtgt ggcgagaact agttcaggct ttctaggggg aggtaccagt 900
acaagtacag gatccctgcg ctgcaaaatg gatcttgatc ctgcatccag cagtatggac 960
catttcaaaa caccagatag aaaagaaagt ggtcttcagg atgatgagaa aggagacact 1020
cacatgtatg atgagagaca acctaagaga aggaagcgaa ccattatgaa tgataggcaa 1080
ataaacgaaa tcgagaaggc tcttattgat gagcctgaga tgcataagaa tgctgcttta 1140
ctgcaggcat ggtcagagaa gttaagtggg cagggctcgg agattacgtc atctcagcta 1200

```
aagaattggc tgaacaacag aaaagctaag cttgctcgta ttgcgaaaga aagaggagta   1260

ctatctgagg gtgagaacgc agataagcca tctacgccag ctactcccca ccactgtgac   1320

tcctcagaaa gtgctggcga ggagagctac ttgccacctg cgagggtcat gagtgctcta   1380

ggcatatcca agggcagcag atttgtgagc ccagatggca atgagacaac atcacaggca   1440

gaattcaatc aaaatatcat gcttagccgc cctttcacaa gatcattctc atttgaacct   1500

ggccgtcttg tttcgctcat tgataatgat gggaaggagg ttggcagggg aaagatcttc   1560

caagttgagg ggagactgca agggaaggcc ctgacagata ctcgcgtttg catcgttgat   1620

gttattgagc tcaagattga gaaatggcgg gagctacctc atccctcgga agcatcagga   1680

agaacattcc aagaggcaga atcaaggaac ggtggtgtga tgagggtcgc gtgggatgtc   1740

atcagactat ctccagtggt tcagtaa                                       1767
```

<210> 278
<211> 588
<212> PRT
<213> Oryza sativa

<400> 278

```
Met Phe Tyr Ser Ser Pro Phe Cys Ser Ser Pro Leu Gln Ile Pro Phe
1               5                   10                  15

Leu Thr Gln Ile Leu Ala Ile Pro His Asp Glu Phe Val Ser Asn Trp
            20                  25                  30

Cys Ser Val Asn Leu Pro Val Ile Glu Glu Asp Ala Asn Leu Asp Tyr
        35                  40                  45

Asp Pro Phe Gly Ala Ala Glu Leu Ala Leu Ala Ala Ala Gly Asn Lys
    50                  55                  60

Leu Thr Glu Ala Lys Ala Asn Tyr Ser Cys Pro Phe Arg Pro Ile Ser
65                  70                  75                  80

Met Pro Ser Ile Ala Tyr Ala Gln Thr Arg Thr Ser Cys Val Val Lys
                85                  90                  95

Ile Ile Ala Asn Leu His Val Phe Val Pro Asn Ile Cys Glu Glu Gln
            100                 105                 110

Glu Arg Asp Leu Phe Leu Gln Lys Phe Gln Lys Tyr Leu Val Ser Gly
        115                 120                 125

Asn Pro Arg Ser Ser Val Asp His Pro Ala Ser Ala Asp Leu Lys Ala
    130                 135                 140

Thr Thr Val Cys Arg Asn Leu Gly Ser Leu Ser Glu Tyr Ala Arg Ser
```

145 150 155 160

Leu Ile Pro Asn Asn Leu Leu Asn Glu Glu Asp Val Gln Leu Leu Ser
165 170 175

Glu Phe Ala Tyr Lys Leu Gln Thr Trp Cys Lys Ser His Val Gly Gln
180 185 190

Ser Thr Ser Gln Ala Val Lys Ile Asp Pro Ser Ser Glu Ser Lys Glu
195 200 205

Asp Phe Lys Pro Leu Gln His Pro Leu Ile Pro Ser Thr Val Val Pro
210 215 220

Asp Ser Ser Ile Asn Asn Leu Pro Lys Asn Met Glu Glu Pro Thr Pro
225 230 235 240

Thr Asn Met Glu Glu Pro Ala Pro Thr Pro Ser Thr Lys Gln Glu Gly
245 250 255

Asn Ala Arg Asp Glu Thr Pro Arg Ser Thr Val Ala Leu Asn Gly Gly
260 265 270

Phe Leu Gln Asn Ser Val Gly Gln Asp Leu Val His Leu Gly Val Ala
275 280 285

Arg Thr Ser Ser Gly Phe Leu Gly Gly Gly Thr Ser Thr Ser Thr Gly
290 295 300

Ser Leu Arg Cys Lys Met Asp Leu Asp Pro Ala Ser Ser Ser Met Asp
305 310 315 320

His Phe Lys Thr Pro Asp Arg Lys Glu Ser Gly Leu Gln Asp Asp Glu
325 330 335

Lys Gly Asp Thr His Met Tyr Asp Glu Arg Gln Pro Lys Arg Arg Lys
340 345 350

Arg Thr Ile Met Asn Asp Arg Gln Ile Asn Glu Ile Glu Lys Ala Leu
355 360 365

Ile Asp Glu Pro Glu Met His Lys Asn Ala Ala Leu Leu Gln Ala Trp
370 375 380

Ser Glu Lys Leu Ser Gly Gln Gly Ser Glu Ile Thr Ser Ser Gln Leu
385 390 395 400

Lys Asn Trp Leu Asn Asn Arg Lys Ala Lys Leu Ala Arg Ile Ala Lys
405 410 415

Glu Arg Gly Val Leu Ser Glu Gly Glu Asn Ala Asp Lys Pro Ser Thr
420 425 430

Pro Ala Thr Pro His His Cys Asp Ser Ser Glu Ser Ala Gly Glu Glu
435 440 445

Ser Tyr Leu Pro Pro Ala Arg Val Met Ser Ala Leu Gly Ile Ser Lys
450 455 460

Gly Ser Arg Phe Val Ser Pro Asp Gly Asn Glu Thr Thr Ser Gln Ala
465 470 475 480

Glu Phe Asn Gln Asn Ile Met Leu Ser Arg Pro Phe Thr Arg Ser Phe
485 490 495

Ser Phe Glu Pro Gly Arg Leu Val Ser Leu Ile Asp Asn Asp Gly Lys
500 505 510

Glu Val Gly Arg Gly Lys Ile Phe Gln Val Glu Gly Arg Leu Gln Gly
515 520 525

Lys Ala Leu Thr Asp Thr Arg Val Cys Ile Val Asp Val Ile Glu Leu
530 535 540

Lys Ile Glu Lys Trp Arg Glu Leu Pro His Pro Ser Glu Ala Ser Gly
545 550 555 560

Arg Thr Phe Gln Glu Ala Glu Ser Arg Asn Gly Gly Val Met Arg Val
565 570 575

Ala Trp Asp Val Ile Arg Leu Ser Pro Val Val Gln
580 585

<210> 279
<211> 6
<212> PRT
<213> Artificial sequence

<220>
<223> motif I

<400> 279

Arg Lys Lys Leu Arg Leu
1 5

<210> 280
<211> 24
<212> PRT
<213> Artificial sequence

<220>
<223> motif II

<400> 280

Thr Lys Leu Lys Gln Thr Glu Val Asp Cys Glu Phe Leu Arg Arg Cys
1 5 10 15

Cys Glu Asn Leu Thr Glu Glu Asn
20

<210> 281
<211> 10
<212> PRT
<213> Artificial sequence

<220>
<223> motif III

<400> 281

Thr Leu Thr Met Cys Pro Ser Cys Glu Arg
1 5 10

<210> 282
<211> 2193
<212> DNA
<213> Oryza sativa

<400> 282
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag atttttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

```
aaccaagcat cctcctcctc ccatctataa attcctcccc ccttttcccc tctctatata   1020
ggaggcatcc aagccaagaa gagggagagc accaaggaca cgcgactagc agaagccgag   1080
cgaccgcctt cttcgatcca tatcttccgg tcgagttctt ggtcgatctc ttccctcctc   1140
cacctcctcc tcacagggta tgtgcccttc ggttgttctt ggatttattg ttctaggttg   1200
tgtagtacgg gcgttgatgt taggaaaggg gatctgtatc tgtgatgatt cctgttcttg   1260
gatttgggat agaggggttc ttgatgttgc atgttatcgg ttcggtttga ttagtagtat   1320
ggttttcaat cgtctggaga gctctatgga aatgaaatgg tttagggtac ggaatcttgc   1380
gattttgtga gtacctttg tttgaggtaa aatcagagca ccggtgattt tgcttggtgt   1440
aataaaagta cggttgtttg gtcctcgatt ctggtagtga tgcttctcga tttgacgaag   1500
ctatcctttg tttattccct attgaacaaa aataatccaa ctttgaagac ggtcccgttg   1560
atgagattga atgattgatt cttaagcctg tccaaaattt cgcagctggc ttgtttagat   1620
acagtagtcc ccatcacgaa attcatggaa acagttataa tcctcaggaa caggggattc   1680
cctgttcttc cgatttgctt tagtcccaga attttttttc ccaaatatct taaaaagtca   1740
ctttctggtt cagttcaatg aattgattgc tacaaataat gcttttatag cgttatccta   1800
gctgtagttc agttaatagg taatacccct atagtttagt caggagaaga acttatccga   1860
tttctgatct ccatttttaa ttatatgaaa tgaactgtag cataagcagt attcatttgg   1920
attatttttt ttattagctc tcacccccttc attattctga gctgaaagtc tggcatgaac   1980
tgtcctcaat tttgttttca aattcacatc gattatctat gcattatcct cttgtatcta   2040
cctgtagaag tttctttttg gttattcctt gactgcttga ttacagaaag aaatttatga   2100
agctgtaatc gggatagtta tactgcttgt tcttatgatt catttccttt gtgcagttct   2160
tggtgtagct tgccactttc accagcaaag ttc                                2193
```

<210> 283
<211> 58
<212> DNA
<213> Artificial sequence

<220>
<223> primer: p01294

<400> 283

```
ggggacaagt ttgtacaaaa aagcaggctt cacaatgatg ttcgagaaag acgatctg      58
```

<210> 284
<211> 52
<212> DNA
<213> Artificial sequence

<220>
<223> primer: p00402

<400> 284

```
ggggaccact ttgtacaaga aagctgggtt taggacctag gacgaagagc gt            52
```

<210> 285
<211> 622
<212> DNA
<213> Arabidopsis thaliana

<400> 285
atgagcagac ccggagattg gaactgcagg tcatgcagcc atctcaactt ccagcgccgt 60

gactcttgcc agcgatgcgg tgactctcgt tccggccccg gtggagttgg tggcttagac 120

tttggtaatt tcggtggcag agccatgtct gctttcggat tcaccaccgg ctccgacgtt 180

cgtcccggtg attggtactg caccgtggga aactgcggga cacacaactt cgccagtcgc 240

tccacctgct tcaaatgcgg cactttcaag gacgagaccg gcgctggagg cggaggtggt 300

ggcatcggcg gtccggccat gtttgacgcc gacattatgc ggtctagagt ccccggtaac 360

ggtggtcgct ctagctggaa atccggcgac tggatttgca ctaggattgg ttgcaatgag 420

cataactttg caagcagaat ggaatgcttc aggtgcaatg caccaaggga cttcagcaac 480

agaacctctt tctaagttat acaaactgct tttgaagtag ccttgtctac ccagctttct 540

tgtacaaagt tggcattata agaaagcatt gcttatcaat ttgttgcaac gaacaggtca 600

ctatcagtca aaataaaatc at 622

<210> 286
<211> 164
<212> PRT
<213> Arabidopsis thaliana

<400> 286

Met Ser Arg Pro Gly Asp Trp Asn Cys Arg Ser Cys Ser His Leu Asn
1 5 10 15

Phe Gln Arg Arg Asp Ser Cys Gln Arg Cys Gly Asp Ser Arg Ser Gly
20 25 30

Pro Gly Gly Val Gly Gly Leu Asp Phe Gly Asn Phe Gly Gly Arg Ala
35 40 45

Met Ser Ala Phe Gly Phe Thr Thr Gly Ser Asp Val Arg Pro Gly Asp
50 55 60

Trp Tyr Cys Thr Val Gly Asn Cys Gly Thr His Asn Phe Ala Ser Arg
65 70 75 80

Ser Thr Cys Phe Lys Cys Gly Thr Phe Lys Asp Glu Thr Gly Ala Gly
85 90 95

Gly Gly Gly Gly Gly Ile Gly Gly Pro Ala Met Phe Asp Ala Asp Ile
100 105 110

```
Met Arg Ser Arg Val Pro Gly Asn Gly Gly Arg Ser Ser Trp Lys Ser
        115                 120                 125

Gly Asp Trp Ile Cys Thr Arg Ile Gly Cys Asn Glu His Asn Phe Ala
    130                 135                 140

Ser Arg Met Glu Cys Phe Arg Cys Asn Ala Pro Arg Asp Phe Ser Asn
145                 150                 155                 160

Arg Thr Ser Phe
```

<210> 287
<211> 717
<212> DNA
<213> Gossypium hirsutum

<400> 287

```
atgagcaggc caggagattg gaactgcagg tcatgccaac acctcaactt ccaaaggagg     60

gacaactgcc aacgttgcgg tgaatctcga tacggtgtta gagtcggctc gacattcggg    120

tttaccgctg gctcggacgt tcgacctggt gactggtatt gcacggctgg aaactgcggc    180

acccacaatt tcgccagccg gtctacttgt ttcaattgcg gcgcgttcaa ggacgagtcg    240

gctggaggtt tcgacttgga catgtctcga tcaagagggt tcggaggtaa ccgatccggc    300

tggaaatcag gggattggat atgtaccagg ttagggtgca atgaacataa ttttgctagc    360

agaatggaat gtttcagatg cagtgctcca agagaattca acaatagaac ttcatattaa    420

atcacatgca tgctactata tccatttttg ggtgttttga ggcaattaat aggagattat    480

aatgagagag tgttactggc tttgatgatg aacaccacaa gcattttgtc atgtttcttt    540

tataagattc atggcaatgt agtacttttt cttgtgatga ttatttatgg tttcaattct    600

atggttttat tgtcttttat tttagagagt tttatttata tttttgtaat ggtgctttgg    660

ctttattaaa agaagatgat tctcttctgt cttttcaaaa aaaaaaaaaa aaaaaaa       717
```

<210> 288
<211> 139
<212> PRT
<213> Gossypium hirsutum

<400> 288

```
Met Ser Arg Pro Gly Asp Trp Asn Cys Arg Ser Cys Gln His Leu Asn
1               5                   10                  15

Phe Gln Arg Arg Asp Asn Cys Gln Arg Cys Gly Glu Ser Arg Tyr Gly
            20                  25                  30

Val Arg Val Gly Ser Thr Phe Gly Phe Thr Ala Gly Ser Asp Val Arg
        35                  40                  45
```

Pro Gly Asp Trp Tyr Cys Thr Ala Gly Asn Cys Gly Thr His Asn Phe
50 55 60

Ala Ser Arg Ser Thr Cys Phe Asn Cys Gly Ala Phe Lys Asp Glu Ser
65 70 75 80

Ala Gly Gly Phe Asp Leu Asp Met Ser Arg Ser Arg Gly Phe Gly Gly
85 90 95

Asn Arg Ser Gly Trp Lys Ser Gly Asp Trp Ile Cys Thr Arg Leu Gly
100 105 110

Cys Asn Glu His Asn Phe Ala Ser Arg Met Glu Cys Phe Arg Cys Ser
115 120 125

Ala Pro Arg Glu Phe Asn Asn Arg Thr Ser Tyr
130 135

<210> 289
<211> 937
<212> DNA
<213> Zea mays

<400> 289
cgggcacgac cagcaacaca acagccgagc tttgcttagg caagatgaac aggaagccag 60

gagactggga ctgcagggcg tgccagcacc tcaacttcag ccgccgagac atatgccagc 120

gctgcagcga gccacgtgga gttgctgatc gtggcagtgg cggcggcgga ggaggcgact 180

acgccagctt cggtggccgc ggtggctcct ccttcggcgg cggctttggc gctgctggct 240

ccgacgtccg ccctggtgac tggtactgct cctgcggcgc gcacaacttc gccagccgct 300

ccagctgctt caagtgctcc gcctacaagg aggaggccgc tgtgaacagt ggcgctggcg 360

gctttgatgg cgacatgtca cgctcacggg gctacggctt cggcagcggt gctgctgctg 420

ctgctggtgc tggcgctgcc cgtactacca accgccccgg ttggaagtcc ggagactgga 480

tctgcaccag atccggatgc aacgagcaca acttcgccag caggatggag tgcttcaggt 540

gcaacgcacc gcgggactct ggcactgagg tgtaggatcg agcaagttaa aaagtctgca 600

gcgccgaaga aagcgacgac aagaggagtc ctcatcacgt cgtaacgtaa gagagagagt 660

agtggatttg caacaaaaaa aaaaaaaaga cggccgcgat gctctgttac tagctagcta 720

gttttgttca accacccatg ccgtctcttc tcttttatta gatttggttt ggttctcata 780

gccctttaat taccatttgg gacctatgtt tgctgttctg tttcccgttc gtctcctccg 840

catgtttgcg cttggatcga gtcttgtgat gtaacccccc aaaaaacgct tgcttaacta 900

gtactgttgc ttcttaataa aaaaaaaaaa aaaaaaa 937

<210> 290
<211> 176
<212> PRT
<213> Zea mays

<400> 290

Met Asn Arg Lys Pro Gly Asp Trp Asp Cys Arg Ala Cys Gln His Leu
1 5 10 15

Asn Phe Ser Arg Arg Asp Ile Cys Gln Arg Cys Ser Glu Pro Arg Gly
20 25 30

Val Ala Asp Arg Gly Ser Gly Gly Gly Gly Gly Gly Asp Tyr Ala Ser
35 40 45

Phe Gly Gly Arg Gly Gly Ser Ser Phe Gly Gly Gly Phe Gly Ala Ala
50 55 60

Gly Ser Asp Val Arg Pro Gly Asp Trp Tyr Cys Ser Cys Gly Ala His
65 70 75 80

Asn Phe Ala Ser Arg Ser Ser Cys Phe Lys Cys Ser Ala Tyr Lys Glu
85 90 95

Glu Ala Ala Val Asn Ser Gly Ala Gly Gly Phe Asp Gly Asp Met Ser
100 105 110

Arg Ser Arg Gly Tyr Gly Phe Gly Ser Gly Ala Ala Ala Ala Ala Gly
115 120 125

Ala Gly Ala Ala Arg Thr Thr Asn Arg Pro Gly Trp Lys Ser Gly Asp
130 135 140

Trp Ile Cys Thr Arg Ser Gly Cys Asn Glu His Asn Phe Ala Ser Arg
145 150 155 160

Met Glu Cys Phe Arg Cys Asn Ala Pro Arg Asp Ser Gly Thr Glu Val
165 170 175

<210> 291
<211> 501
<212> DNA
<213> Oryza sativa

<400> 291
atgaacagga agccaggaga ctgggactgc agggcgtgcc agcacctcaa cttcagccgc 60

cgggacctat gccagcgctg cggcgagccg cgtggcgccg ctgatcgcgg cagcggtggt 120

ggcggtgact acgccaactt cggcggccgt ggtggttcct ccttcggtgg aggctttggc 180

actggctctg atgtccgccc aggtgactgg tactgcaact gcggcgcgca caacttcgcc 240

agccgctcca gctgcttcaa gtgcgctgct ttcaaggacg atgctgccgt caacagtggc 300

ggcgctggtg cctttgatgg tggggacatg tcgcgctcgc ggggctacgg cttcggcagc 360

ggcgccgtcc gcgccagccg ccctggctgg aagtctggcg actggatttg caccaggtct 420

ggatgcaatg agcacaactt cgccagcagg atggagtgct tcaggtgcaa cgcaccgcgg 480

gactccggca ctgaggtgta a 501

<210> 292
<211> 166
<212> PRT
<213> Oryza sativa

<400> 292

Met Asn Arg Lys Pro Gly Asp Trp Asp Cys Arg Ala Cys Gln His Leu
1 5 10 15

Asn Phe Ser Arg Arg Asp Leu Cys Gln Arg Cys Gly Glu Pro Arg Gly
20 25 30

Ala Ala Asp Arg Gly Ser Gly Gly Gly Gly Asp Tyr Ala Asn Phe Gly
35 40 45

Gly Arg Gly Gly Ser Ser Phe Gly Gly Gly Phe Gly Thr Gly Ser Asp
50 55 60

Val Arg Pro Gly Asp Trp Tyr Cys Asn Cys Gly Ala His Asn Phe Ala
65 70 75 80

Ser Arg Ser Ser Cys Phe Lys Cys Ala Ala Phe Lys Asp Asp Ala Ala
85 90 95

Val Asn Ser Gly Gly Ala Gly Ala Phe Asp Gly Gly Asp Met Ser Arg
100 105 110

Ser Arg Gly Tyr Gly Phe Gly Ser Gly Ala Val Arg Ala Ser Arg Pro
115 120 125

Gly Trp Lys Ser Gly Asp Trp Ile Cys Thr Arg Ser Gly Cys Asn Glu
130 135 140

His Asn Phe Ala Ser Arg Met Glu Cys Phe Arg Cys Asn Ala Pro Arg
145 150 155 160

Asp Ser Gly Thr Glu Val
165

<210> 293
<211> 859
<212> DNA

<213>  Arabidopsis thaliana

<400>  293
ataagctctt acactcattt caactctctt tttcgtttcc attaccctca gaagaagatg      60

aataggccgg gagattggaa ctgcagattg tgtagccacc tcaacttcca gaggagggat     120

tcatgccaac gttgtagaga gcctagaccg ggcgggatca gtaccgattt actcagcggt     180

tttggtggcc gtccggttag tagctccttc ggtttcaaca ccgggcccga tgtgcgaccc     240

ggggattggt attgcaacct tggggattgt gggacacata attttgccaa taggtccagt     300

tgtttcaagt gtggtgccgc aaaagatgag ttttcatgct caagtgctgc tgcaacaacc     360

gggtttatgg acatgaatgt tggtccgaga cgtggccttt ttggttttgg cggcagcagt     420

agtggtggtg gtggtacggg ccgttctcct tggaaatctg gagattggat ttgcccaagg     480

tcaggctgta acgaacataa cttcgcaagc aggtcagagt gtttcaggtg taacgcacca     540

aaggaacttg ccaccgaacc accctattag tcatttagtc ctcctacctt cttcatcatt     600

tccaatactc tggaagcatt agaggagagc agaaaggtga agaatcgaag caagataccg     660

accgccctta atctcttgat ttgtttaatt tcttagattt gactcgtttt atatctcgta     720

gtagtcagac ctatgttaga atgtaagcat gtcgagagtt ttccgaagca ttttgttctg     780

atatcggtct cctagctagt atgtaagttt gtttgaatgt attcttattt ctgataaagt     840

tgtttccttc tgattccgc                                                  859

<210>  294
<211>  170
<212>  PRT
<213>  Arabidopsis thaliana

<400>  294

Met Asn Arg Pro Gly Asp Trp Asn Cys Arg Leu Cys Ser His Leu Asn
1               5                   10                  15

Phe Gln Arg Arg Asp Ser Cys Gln Arg Cys Arg Glu Pro Arg Pro Gly
            20                  25                  30

Gly Ile Ser Thr Asp Leu Leu Ser Gly Phe Gly Gly Arg Pro Val Ser
        35                  40                  45

Ser Ser Phe Gly Phe Asn Thr Gly Pro Asp Val Arg Pro Gly Asp Trp
    50                  55                  60

Tyr Cys Asn Leu Gly Asp Cys Gly Thr His Asn Phe Ala Asn Arg Ser
65                  70                  75                  80

Ser Cys Phe Lys Cys Gly Ala Ala Lys Asp Glu Phe Ser Cys Ser Ser
                85                  90                  95

```
Ala Ala Ala Thr Thr Gly Phe Met Asp Met Asn Val Gly Pro Arg Arg
            100                 105                 110

Gly Leu Phe Gly Phe Gly Gly Ser Ser Ser Gly Gly Gly Gly Thr Gly
        115                 120                 125

Arg Ser Pro Trp Lys Ser Gly Asp Trp Ile Cys Pro Arg Ser Gly Cys
    130                 135                 140

Asn Glu His Asn Phe Ala Ser Arg Ser Glu Cys Phe Arg Cys Asn Ala
145                 150                 155                 160

Pro Lys Glu Leu Ala Thr Glu Pro Pro Tyr
                165                 170
```

```
<210>  295
<211>  834
<212>  DNA
<213>  Oryza sativa

<400>  295
cctagtacta agaacagcaa ccacctctga aagctttaca agttcgcagg ggcttaactg      60

cgaatgaaca tccagaggaa gccaggagac tggaactgca aatcgtgcca gcatctcaac     120

ttcagccgcc gggactactg ccagcgctgc cataccccac gccaggacct gccgcttggc     180

gatggttatg tcccaggtgg tgtgctgtcc tccctggaca ttcgcccggg cgactggtac     240

tgcaactgcg gctatcacaa ctttgctagc cgagcaagct gcttcaaatg tggcgccatt     300

gtgaaggacc ttccagcagg ccaaggtggt ggtgttgcca acggtgactt tgcccgtgcc     360

ctcgacagca gcgcagttcg tgctgggtgg aaggcgggtg actggatttg cacaaggcct     420

ggttgcaacg tccacaactt tgcaagtagg attgagtgct ataggtgcaa tgcacctagg     480

gaagcaggta atgtgaagta agaaaagact gacgcgacca aagatcgtga gacgtgacga     540

gctggccgag gatgaataaa gtgactgctg tcagttgtca cattcacagg ctgcgatcca     600

gaaactatgg atgggactat gtagtaacgt gctgatatat tattgctaag tgttactact     660

gcatggttgc aagggtggta gaagtacctt agcttccaag atcgttgtaa tgtgtgagtt     720

taattttggc gttttaagta ataagtctag tgattgcagg attgtacggg gtaatgtact     780

tgcattatcc attataatct taagcatcca atataattat gcaaaaaaaa aaaa           834
```

```
<210>  296
<211>  145
<212>  PRT
<213>  Oryza sativa

<400>  296

Met Asn Ile Gln Arg Lys Pro Gly Asp Trp Asn Cys Lys Ser Cys Gln
1               5                   10                  15
```

His Leu Asn Phe Ser Arg Arg Asp Tyr Cys Gln Arg Cys His Thr Pro
20 25 30

Arg Gln Asp Leu Pro Leu Gly Asp Gly Tyr Val Pro Gly Gly Val Leu
35 40 45

Ser Ser Leu Asp Ile Arg Pro Gly Asp Trp Tyr Cys Asn Cys Gly Tyr
50 55 60

His Asn Phe Ala Ser Arg Ala Ser Cys Phe Lys Cys Gly Ala Ile Val
65 70 75 80

Lys Asp Leu Pro Ala Gly Gln Gly Gly Gly Val Ala Asn Gly Asp Phe
85 90 95

Ala Arg Ala Leu Asp Ser Ser Ala Val Arg Ala Gly Trp Lys Ala Gly
100 105 110

Asp Trp Ile Cys Thr Arg Pro Gly Cys Asn Val His Asn Phe Ala Ser
115 120 125

Arg Ile Glu Cys Tyr Arg Cys Asn Ala Pro Arg Glu Ala Gly Asn Val
130 135 140

Lys
145

<210> 297
<211> 958
<212> DNA
<213> Oryza sativa

<400> 297
aagcctccat ccatccatcc attcatcaga gctcaagctc aagcaagcaa gcttgctagc 60

tagctgctga gcagcattcc agtctgctcc agctagctca gctctctctg ctctgctctt 120

tgctcgataa cgaccatcat cttcttcttc ttcttcttct tcttcttctt cttcttcttg 180

tacattctat ttgctcgata gatagataga tagatagata ggtagataga gatggagacg 240

aaggcggcgg cgatggcgat gaggaagccg ggggactgga gctgcaggtc gtgccagtac 300

gtgaacttct gcaagaggga ggcgtgccag cggtgcgggg aggcgaagct cggggtggag 360

cggacggact acgccgccat gggcggcggg tgggaggtga agcccggcga ctggtgctgc 420

cgctgctgcg ccgtcaacaa ctacgccagc cgcggcagct gcttcaagtg cggcgccgcc 480

aagaacgact ccgccgccgc cgtcgcccag ggctggggct tctccgtcgc ctcccaggcc 540

ggctggaaga acggcgactg gatctgcccc agaatggaat gcaacgtgca gaactacgct 600

aacagaaccg agtgcttccg gtgcaatttc cccagatact acgttgattg atctgacata 660

tatgatatat gccttgcact gagaagaaat gaaagggaaa taaaatttac gagatcgaag 720

atcgacggac aagattgatc ggccaccggt catgcccttc agggttttct tttttctatt 780

ttttttaga ataaccctcg ataacctgag ggtttttttt catttgtaag agcggtaacg 840

gtactaaaaa aacacaaaaa cggcacaaaa ttgtggctga tgactgatca gtcttcctct 900

ttttttccc aataaaaggc agataattaa gaaagcaaaa ttattaaaaa aaaaaaa 958

<210> 298
<211> 139
<212> PRT
<213> Oryza sativa

<400> 298

Met Glu Thr Lys Ala Ala Ala Met Ala Met Arg Lys Pro Gly Asp Trp
1 5 10 15

Ser Cys Arg Ser Cys Gln Tyr Val Asn Phe Cys Lys Arg Glu Ala Cys
20 25 30

Gln Arg Cys Gly Glu Ala Lys Leu Gly Val Glu Arg Thr Asp Tyr Ala
35 40 45

Ala Met Gly Gly Gly Trp Glu Val Lys Pro Gly Asp Trp Cys Cys Arg
50 55 60

Cys Cys Ala Val Asn Asn Tyr Ala Ser Arg Gly Ser Cys Phe Lys Cys
65 70 75 80

Gly Ala Ala Lys Asn Asp Ser Ala Ala Ala Val Ala Gln Gly Trp Gly
85 90 95

Phe Ser Val Ala Ser Gln Ala Gly Trp Lys Asn Gly Asp Trp Ile Cys
100 105 110

Pro Arg Met Glu Cys Asn Val Gln Asn Tyr Ala Asn Arg Thr Glu Cys
115 120 125

Phe Arg Cys Asn Phe Pro Arg Tyr Tyr Val Asp
130 135

<210> 299
<211> 519
<212> DNA
<213> Beta vulgaris

<400> 299
atgagtaggc caggtgattg gaattgtagg tcatgcagcc acttgaactt ccaaaggagg 60

gactcctgcc agcgctgtgg ggacgtacgt cctgacggcc gaggcggcgg agggggagga 120

ggagactttg gcagtagctt tggagggagg tcaggtgggt ccccctttgg tgggggtttt 180

```
gcagggcccg atgttaggcc cggtgattgg tattgtagca ttggcaactg tggggcccac    240

aactttgcca gcaggtctag ctgcttcaag tgtggggcct acaaagagga agctggctgt    300

ggtgatagca tgggccgttc acgtggagga ttttcctttg gtggcatcgg cggtggcggt    360

agcggtgccg ctaccggccg ctcaggctgg aaatccggtg actggatttg cactaggtcg    420

ggttgcaacg agcataactt cgctagtagg actgagtgct tcagatgcag ggaaccaagg    480

gactccggta atgcaatgct aaaggaagta ccatgctga                           519


<210>  300
<211>  172
<212>  PRT
<213>  Beta vulgaris

<400>  300

Met Ser Arg Pro Gly Asp Trp Asn Cys Arg Ser Cys Ser His Leu Asn
1               5                   10                  15


Phe Gln Arg Arg Asp Ser Cys Gln Arg Cys Gly Asp Val Arg Pro Asp
            20                  25                  30


Gly Arg Gly Gly Gly Gly Gly Gly Gly Asp Phe Gly Ser Ser Phe Gly
        35                  40                  45


Gly Arg Ser Gly Gly Ser Pro Phe Gly Gly Gly Phe Ala Gly Pro Asp
    50                  55                  60


Val Arg Pro Gly Asp Trp Tyr Cys Ser Ile Gly Asn Cys Gly Ala His
65                  70                  75                  80


Asn Phe Ala Ser Arg Ser Ser Cys Phe Lys Cys Gly Ala Tyr Lys Glu
                85                  90                  95


Glu Ala Gly Cys Gly Asp Ser Met Gly Arg Ser Arg Gly Gly Phe Ser
            100                 105                 110


Phe Gly Gly Ile Gly Gly Gly Gly Ser Gly Ala Ala Thr Gly Arg Ser
        115                 120                 125


Gly Trp Lys Ser Gly Asp Trp Ile Cys Thr Arg Ser Gly Cys Asn Glu
    130                 135                 140


His Asn Phe Ala Ser Arg Thr Glu Cys Phe Arg Cys Arg Glu Pro Arg
145                 150                 155                 160


Asp Ser Gly Asn Ala Met Leu Lys Glu Val Pro Cys
                165                 170
```

<210> 301
<211> 807
<212> DNA
<213> Arabidopsis thaliana

<400> 301
atgggagacg gaagagaagg agactgggaa tgtttaggat gcagaaacag gaattatgcg 60

tttagatcat tctgtaacag atgcaagcag cctcgtctta tcatggataa taatacttct 120

ccaaactcta agtggcttcc tcgtatcggc gattggatct gcactggttg tactaacaac 180

aattatgcat cacgagaaaa gtgcaagaaa tgtgggcaat ctaaggaagt agcagcattg 240

tcagcacttg ctatccctgg agcttctctt caaactcatc tccattactt cacccgtgga 300

cctgagtcac atgatcaacc tggttcttta ctcgcattct ctaacgctac aaatcaagct 360

tcggttcata aagaatggag gagtggtgac tggatttgca gatgtggttt tcacaattat 420

tcctctcgta tacagtgcaa aaagtgcaat gaaatagctc cactagccct tggtacaaag 480

agattagcat cagaagcttt ggctcatgaa tgggatagca aaagactgaa tcaaggatat 540

acaagcatgc aaacacagtc agcgatatat gcatcttttc ctggtatgag cctaggaagg 600

gtctcaaatt ggcaacttcc tctcccgttt ctacaacaac actcaacacc tgctttactt 660

ggaatgggag tgaaacaatg gcgtgatggc gactggatgt gtacaaattg caagaatcac 720

aattatgcat cacgagcaga gtgcaatagg tgcaagacta cacgagatat ccttgatcag 780

gacataactc caacagaaca atcttga 807

<210> 302
<211> 268
<212> PRT
<213> Arabidopsis thaliana

<400> 302

Met Gly Asp Gly Arg Glu Gly Asp Trp Glu Cys Leu Gly Cys Arg Asn
1 5 10 15

Arg Asn Tyr Ala Phe Arg Ser Phe Cys Asn Arg Cys Lys Gln Pro Arg
20 25 30

Leu Ile Met Asp Asn Asn Thr Ser Pro Asn Ser Lys Trp Leu Pro Arg
35 40 45

Ile Gly Asp Trp Ile Cys Thr Gly Cys Thr Asn Asn Asn Tyr Ala Ser
50 55 60

Arg Glu Lys Cys Lys Lys Cys Gly Gln Ser Lys Glu Val Ala Ala Leu
65 70 75 80

Ser Ala Leu Ala Ile Pro Gly Ala Ser Leu Gln Thr His Leu His Tyr
85 90 95

Phe Thr Arg Gly Pro Glu Ser His Asp Gln Pro Gly Ser Leu Leu Ala
100 105 110

Phe Ser Asn Ala Thr Asn Gln Ala Ser Val His Lys Glu Trp Arg Ser
115 120 125

Gly Asp Trp Ile Cys Arg Cys Gly Phe His Asn Tyr Ser Ser Arg Ile
130 135 140

Gln Cys Lys Lys Cys Asn Glu Ile Ala Pro Leu Ala Leu Gly Thr Lys
145 150 155 160

Arg Leu Ala Ser Glu Ala Leu Ala His Glu Trp Asp Ser Lys Arg Leu
165 170 175

Asn Gln Gly Tyr Thr Ser Met Gln Thr Gln Ser Ala Ile Tyr Ala Ser
180 185 190

Phe Pro Gly Met Ser Leu Gly Arg Val Ser Asn Trp Gln Leu Pro Leu
195 200 205

Pro Phe Leu Gln Gln His Ser Thr Pro Ala Leu Leu Gly Met Gly Val
210 215 220

Lys Gln Trp Arg Asp Gly Asp Trp Met Cys Thr Asn Cys Lys Asn His
225 230 235 240

Asn Tyr Ala Ser Arg Ala Glu Cys Asn Arg Cys Lys Thr Thr Arg Asp
245 250 255

Ile Leu Asp Gln Asp Ile Thr Pro Thr Glu Gln Ser
260 265

<210> 303
<211> 1264
<212> DNA
<213> Arabidopsis thaliana

<400> 303
ctatttctcc taccgtcgaa ctaaatccta gggcacaggt gaaatcgccg agtgacgtct 60

agccaccgca ccgcctcctt ctccggttat ctcgctacta atcagactat tccacagcta 120

tgtcacaggt agacaacaga aattcatcag cagccaagcg tgctagaact gacgggggc 180

gtagagaaga tgattggatc tgcccaagtt gtggcaatgt caacttttca ttcaggacaa 240

cttgcaatat gcgtaattgc actcagccta gacctgcaga tcataatgga aagtctgctc 300

ccaaacctat gcaacatcaa caaggtttct catcacccgg ggcatactta ggatctgggg 360

gtccccctcc agtatatatg ggcgggtcac catatggatc tcctctcttt aatggatcat 420

ctatgcctcc ttatgacgtc ccattttctg ggggttcgcc ttaccatttt aactataata 480

gccgaatgcc tgccggagct cattacagac cattacatat gtctggacca ccaccatacc 540

atggcggatc tatgatggga agtggtggta tgtatggaat gcctccacca atagacaggt 600

atggccttgg tatggcaatg ggtcctggtt ctgccgctgc catgatgcca agaccaaggt 660

tttacccaga tgaaaaatca caaaagagag attcaactcg cgataatgat tggacatgtc 720

cgaattgtgg taatgtaaac ttctcattca gaactgtatg taacatgagg aagtgcaaca 780

ctccaaagcc tggttctcag cagggtggaa gctcagataa aatatccaaa caaaatgcac 840

cggaagggag ctggaagtgt gataactgtg gaaatataaa ctacccattc aggagcaaat 900

gcaacaggca aaactgtgga gctgataagc ctggggatcg gtcgaatgga tctccgtccc 960

gtgcaccaga agagaacgat cagtgagtcg tagacatgtt tgggggttga gaagggtgag 1020

tctaatccag cgggtgtcgt aatgtcagtc aatgtctcgt caggtcaggt cgtccatgtt 1080

gctgcgtcgt cagtcaagtt gcttctatgc atttgtctct ttacttccct tgcgtggttg 1140

tggattgtat taataatgca aaaattgttt atttactttg tcgtcctcgt ggatcttgtc 1200

ttgctataga atcctcctcc aggtccatac tgttttacat cctaatctta agtaagttgg 1260

cacc 1264

<210> 304
<211> 288
<212> PRT
<213> Arabidopsis thaliana

<400> 304

Met Ser Gln Val Asp Asn Arg Asn Ser Ser Ala Ala Lys Arg Ala Arg
1 5 10 15

Thr Asp Gly Gly Arg Arg Glu Asp Asp Trp Ile Cys Pro Ser Cys Gly
20 25 30

Asn Val Asn Phe Ser Phe Arg Thr Thr Cys Asn Met Arg Asn Cys Thr
35 40 45

Gln Pro Arg Pro Ala Asp His Asn Gly Lys Ser Ala Pro Lys Pro Met
50 55 60

Gln His Gln Gln Gly Phe Ser Ser Pro Gly Ala Tyr Leu Gly Ser Gly
65 70 75 80

Gly Pro Pro Pro Val Tyr Met Gly Gly Ser Pro Tyr Gly Ser Pro Leu
85 90 95

Phe Asn Gly Ser Ser Met Pro Pro Tyr Asp Val Pro Phe Ser Gly Gly
100 105 110

```
Ser Pro Tyr His Phe Asn Tyr Asn Ser Arg Met Pro Ala Gly Ala His
        115                 120                 125

Tyr Arg Pro Leu His Met Ser Gly Pro Pro Pro Tyr His Gly Gly Ser
    130                 135                 140

Met Met Gly Ser Gly Gly Met Tyr Gly Met Pro Pro Pro Ile Asp Arg
145                 150                 155                 160

Tyr Gly Leu Gly Met Ala Met Gly Pro Gly Ser Ala Ala Ala Met Met
                165                 170                 175

Pro Arg Pro Arg Phe Tyr Pro Asp Glu Lys Ser Gln Lys Arg Asp Ser
            180                 185                 190

Thr Arg Asp Asn Asp Trp Thr Cys Pro Asn Cys Gly Asn Val Asn Phe
        195                 200                 205

Ser Phe Arg Thr Val Cys Asn Met Arg Lys Cys Asn Thr Pro Lys Pro
    210                 215                 220

Gly Ser Gln Gln Gly Gly Ser Ser Asp Lys Ile Ser Lys Gln Asn Ala
225                 230                 235                 240

Pro Glu Gly Ser Trp Lys Cys Asp Asn Cys Gly Asn Ile Asn Tyr Pro
                245                 250                 255

Phe Arg Ser Lys Cys Asn Arg Gln Asn Cys Gly Ala Asp Lys Pro Gly
            260                 265                 270

Asp Arg Ser Asn Gly Ser Pro Ser Arg Ala Pro Glu Glu Asn Asp Gln
        275                 280                 285


<210>  305
<211>  1853
<212>  DNA
<213>  Oryza sativa

<400>  305
cagtttccac tttcttccaa agccgaaaaa tcgagagaga aagagcaaaa ccctaaccgc      60

ggcgctccgc cccttccgc cgccggattc ctcctcctcc ttcgtccgcc ttcgccggcg      120

ccgccctgct ccgaaggagc ccaggccttg tcgccgcgtc gcttgccccg ccgccggttt     180

cagcagcagc cacccccacc accatgtctt ctcaggtcga caaccgtagc caatctgctg     240

gaaagcgtgc ccgcaccgac ggtgggcggc gtgaggacga ctgggtttgc cccagctgcc     300

agaacgtcaa cttcgccttc cgcaccacct gcaacatgcg caattgcaac caatccaggc     360

ccaccgacta tacgaaggat atgcagaaac ctatgcagac accgccgccc catttcccca     420
```

```
tgtcaggggg atacatgagc ccagggacgc cgccatccat gtacctcggg ggtggtgctc     480
ccccttatgg cacctccctc tatggcggac ctgctttacc acgttatggt attgctcagt     540
tccctggggg ttctggatat ccatatggct atggtggccg cctgccaatg gggagcccct     600
atgggccgcc gatgcatatg gcagggcctc catattctgc tggatccatg atgggaccag     660
gtggaatgta tgggatgccc atggacagat atagcttggg cttacctgct ggtcctggtc     720
caatgggcgc gagggctggt tcatattccg aggaaggatc ccagaagaag cctgcaggag     780
ctgggcgtga taatgattgg aaatgcccta attgcaacaa cattaacttt gcattccgga     840
cagtctgtaa catgagaaag tgcaatacac caagacctga aaaccagggg tccaaacctg     900
atggtgcaag aggtccaaaa ccaaagatgc cagaaggtag ttggaagtgc gagaagtgca     960
ataacataaa ctatccattc cgcacaaaat gtaaccgtcc aagttgcgag gctgaaaagc    1020
catttcagac aaacaatgct aatgagtcat ctgctgatca ggacaatcag ttgttgtcat    1080
gtaatattgt gaagctttta tcaaagctgc aactttcaca tgactttcaa gacaataatg    1140
agcaaacaaa ggtggatcct cccggtggcc ctgctggagt accgacaagt tcgcacgccc    1200
tgcgaatggc agctctcagt gagttgcaga acagtgttaa gagctgatac ccttttcatt    1260
ttccaaacaa ggtgagaact ttgatccatg tggatgtgct tctatcagga gcagctgaca    1320
gtattgcatg cagtgatctt gctaaggaag ttggagatgg gaaacatcgt gtgatggcca    1380
gccttgatgt aagaatgcaa atcagtctta ctccgtacca tatctgtgtg gagtgtgcac    1440
accggaagca tagtcatttt ttgaatggtc gattgcttct tccacagtaa agtctataat    1500
accagtgtgc aggtgttttt ttccctttct ttcagtgtga aggtgttttt ttaccccttt    1560
ctttcgttca ttcattcttt ttctttttcc ttgttgagac ggtatcctgg agcactttgt    1620
aatactctaa tgtgcttcgg aaagtagaat ataagttgtg cttatgggcg ggccaaaatg    1680
ctttggccgt tagttgcatg acagcctttc caacatgctg ctgcctagcc gtagttctcc    1740
caaaaatgaa accatcttga gatccgtttg cggttgaact gaaattagac ggtatctctg    1800
gtttcaacat catcctttaa accctggatg aaagatttgc aatattcctg cgg           1853
```

<210> 306
<211> 347
<212> PRT
<213> Oryza sativa

<400> 306

```
Met Ser Ser Gln Val Asp Asn Arg Ser Gln Ser Ala Gly Lys Arg Ala
1               5                   10                  15

Arg Thr Asp Gly Gly Arg Arg Glu Asp Asp Trp Val Cys Pro Ser Cys
            20                  25                  30

Gln Asn Val Asn Phe Ala Phe Arg Thr Thr Cys Asn Met Arg Asn Cys
```

35 40 45

Asn Gln Ser Arg Pro Thr Asp Tyr Thr Lys Asp Met Gln Lys Pro Met
50 55 60

Gln Thr Pro Pro Pro His Phe Pro Met Ser Gly Gly Tyr Met Ser Pro
65 70 75 80

Gly Thr Pro Pro Ser Met Tyr Leu Gly Gly Gly Ala Pro Pro Tyr Gly
85 90 95

Thr Ser Leu Tyr Gly Gly Pro Ala Leu Pro Arg Tyr Gly Ile Ala Gln
100 105 110

Phe Pro Gly Gly Ser Gly Tyr Pro Tyr Gly Tyr Gly Gly Arg Leu Pro
115 120 125

Met Gly Ser Pro Tyr Gly Pro Pro Met His Met Ala Gly Pro Pro Tyr
130 135 140

Ser Ala Gly Ser Met Met Gly Pro Gly Gly Met Tyr Gly Met Pro Met
145 150 155 160

Asp Arg Tyr Ser Leu Gly Leu Pro Ala Gly Pro Gly Pro Met Gly Ala
165 170 175

Arg Ala Gly Ser Tyr Ser Glu Glu Gly Ser Gln Lys Lys Pro Ala Gly
180 185 190

Ala Gly Arg Asp Asn Asp Trp Lys Cys Pro Asn Cys Asn Asn Ile Asn
195 200 205

Phe Ala Phe Arg Thr Val Cys Asn Met Arg Lys Cys Asn Thr Pro Arg
210 215 220

Pro Glu Asn Gln Gly Ser Lys Pro Asp Gly Ala Arg Gly Pro Lys Pro
225 230 235 240

Lys Met Pro Glu Gly Ser Trp Lys Cys Glu Lys Cys Asn Asn Ile Asn
245 250 255

Tyr Pro Phe Arg Thr Lys Cys Asn Arg Pro Ser Cys Glu Ala Glu Lys
260 265 270

Pro Phe Gln Thr Asn Asn Ala Asn Glu Ser Ser Ala Asp Gln Asp Asn
275 280 285

Gln Leu Leu Ser Cys Asn Ile Val Lys Leu Leu Ser Lys Leu Gln Leu
290 295 300

Ser His Asp Phe Gln Asp Asn Asn Glu Gln Thr Lys Val Asp Pro Pro
305 310 315 320

Gly Gly Pro Ala Gly Val Pro Thr Ser Ser His Ala Leu Arg Met Ala
325 330 335

Ala Leu Ser Glu Leu Gln Asn Ser Val Lys Ser
340 345

<210> 307
<211> 1078
<212> DNA
<213> Oryza sativa

<400> 307
ctggtctctc gctctcttcg ctcgcggcgg cttctccgcc tccgcctccg ccgccctctc 60

ctcctctcgc ctcgccgccg gcgccgccag ccaccgcgcg ggtcgggcgg gccgtatctt 120

cctttctgtt ccgatggctt ccgcgaaggt ggagaaccgc ggcggcggtg ggttcggttc 180

gaagaggtca cgcaacgacg tgtctgtaag ggagggggac tggacttgtc ctcagtgtgg 240

taatgtcaac ttcagtttta gaaatgtttg caaccgcgga gcctgtggtg caccccgtcc 300

atcaccgagt ctaagcccaa gagtgccacc tcctcctgct gctggatatg atcggccaca 360

tcttggatat gatcggccac atctgtttta tggtagtgct ggcaccccac ctcctattcc 420

tcttggatct ggtagctatg gtgcccccta tccacatctt ggcttgcggt atggatatgg 480

tccaccagta ggacctcctg cttcatatgg cctttttctt tcttatggtc aacctggacc 540

aatgggcagt ccgatgggag gcatgggcta tggccctgga cctgagctag gccgatatgg 600

ttatggtttt agaggatctc caatgccggt ttctagccca tggtctggtg gagcattagt 660

ggaaaataat gacagctctg cttcacgcaa gcgtcgtgga ggcccagatg gaatggctga 720

gaatgactgg atctgcccaa agtgtgagaa tgtcaacttt tccttcagaa acagttgcaa 780

tatgaagaaa tgtggagctc caaggccaag ccctggatct aatgctaccc catgtcgcaa 840

agacaaggac gctccggaag ggagctggac ctgcccggag tgcaacaacc tgaactaccc 900

tttccgcacg gcgtgcaatc ggaaaggctg cggaagcagc aggccggcag cggccacggc 960

gaactaggac cctactaact ttgcgccggc gattgggggc agagagagta ttgttgtatc 1020

ctagctatat acaagttaat ttaaactgta aacggctcaa atatattttc ttgtggcc 1078

<210> 308
<211> 277
<212> PRT
<213> Oryza sativa

<400> 308

Met Ala Ser Ala Lys Val Glu Asn Arg Gly Gly Gly Gly Phe Gly Ser

```
1               5                   10                  15

Lys Arg Ser Arg Asn Asp Val Ser Val Arg Glu Gly Asp Trp Thr Cys
            20                  25                  30

Pro Gln Cys Gly Asn Val Asn Phe Ser Phe Arg Asn Val Cys Asn Arg
        35                  40                  45

Gly Ala Cys Gly Ala Pro Arg Pro Ser Pro Ser Leu Ser Pro Arg Val
    50                  55                  60

Pro Pro Pro Pro Ala Ala Gly Tyr Asp Arg Pro His Leu Gly Tyr Asp
65                  70                  75                  80

Arg Pro His Leu Phe Tyr Gly Ser Ala Gly Thr Pro Pro Pro Ile Pro
                85                  90                  95

Leu Gly Ser Gly Ser Tyr Gly Ala Pro Tyr Pro His Leu Gly Leu Arg
            100                 105                 110

Tyr Gly Tyr Gly Pro Pro Val Gly Pro Pro Ala Ser Tyr Gly Leu Phe
        115                 120                 125

Ser Ser Tyr Gly Gln Pro Gly Pro Met Gly Ser Pro Met Gly Gly Met
    130                 135                 140

Gly Tyr Gly Pro Gly Pro Glu Leu Gly Arg Tyr Gly Tyr Gly Phe Arg
145                 150                 155                 160

Gly Ser Pro Met Pro Val Ser Ser Pro Trp Ser Gly Gly Ala Leu Val
                165                 170                 175

Glu Asn Asn Asp Ser Ser Ala Ser Arg Lys Arg Arg Gly Gly Pro Asp
            180                 185                 190

Gly Met Ala Glu Asn Asp Trp Ile Cys Pro Lys Cys Glu Asn Val Asn
        195                 200                 205

Phe Ser Phe Arg Asn Ser Cys Asn Met Lys Lys Cys Gly Ala Pro Arg
    210                 215                 220

Pro Ser Pro Gly Ser Asn Ala Thr Pro Cys Arg Lys Asp Lys Asp Ala
225                 230                 235                 240

Pro Glu Gly Ser Trp Thr Cys Pro Glu Cys Asn Asn Leu Asn Tyr Pro
                245                 250                 255

Phe Arg Thr Ala Cys Asn Arg Lys Gly Cys Gly Ser Ser Arg Pro Ala
            260                 265                 270
```

```
Ala Ala Thr Ala Asn
        275


<210>  309
<211>  662
<212>  DNA
<213>  Brassica rapa

<400>  309
ggtcttgttt tctttcaata aacacataat aagatagctt ccaagttcat caagaaataa      60

acgtaagcgc acgcatacaa taacctatcg aagatgagca gacccggaga ctggaactgt     120

agatcatgca cccacctcaa cttccagcgc cgtgattctt gccagcgatg cggtgactcc     180

cgtttgggtg caggtggagt cggtggctta gagtttggtg atttcggcgg cagaggtatg     240

tctgcttttg gattcaccac gggctccgac gttcgtccag gtgactggta ctgcacagtt     300

ggaaactgcg ggacacataa ctttgccagc cgctccacct gcttcaaatg cggcactttc     360

aaggacgaat ccctcggtgg gggcggcggc ggtggcgtag gcgtaaggcg gtccggtcat     420

gttgacgctg acgttatgcg gtctagagtc tccggcaacg gtggccgctc cagctggaaa     480

tccggtgatt ggatttgcac caggcttggt tgcaatgagc ataactttgc aagcagaatg     540

gagtgcttca gatgcaatgc accaagggac ttcagcatga gaacctcttt ctaagttaca     600

caataatgcc tttgaagtag ccttgtttcc aatctcttgg gcaccgtttc aatcaatgga     660

aa                                                                    662


<210>  310
<211>  166
<212>  PRT
<213>  Brassica rapa

<400>  310

Met Ser Arg Pro Gly Asp Trp Asn Cys Arg Ser Cys Thr His Leu Asn
1               5                   10                  15


Phe Gln Arg Arg Asp Ser Cys Gln Arg Cys Gly Asp Ser Arg Leu Gly
            20                  25                  30


Ala Gly Gly Val Gly Gly Leu Glu Phe Gly Asp Phe Gly Gly Arg Gly
        35                  40                  45


Met Ser Ala Phe Gly Phe Thr Thr Gly Ser Asp Val Arg Pro Gly Asp
    50                  55                  60


Trp Tyr Cys Thr Val Gly Asn Cys Gly Thr His Asn Phe Ala Ser Arg
65                  70                  75                  80


Ser Thr Cys Phe Lys Cys Gly Thr Phe Lys Asp Glu Ser Leu Gly Gly
```

                85                  90                  95

Gly Gly Gly Gly Gly Val Gly Val Arg Arg Ser Gly His Val Asp Ala
            100                 105                 110

Asp Val Met Arg Ser Arg Val Ser Gly Asn Gly Gly Arg Ser Ser Trp
        115                 120                 125

Lys Ser Gly Asp Trp Ile Cys Thr Arg Leu Gly Cys Asn Glu His Asn
    130                 135                 140

Phe Ala Ser Arg Met Glu Cys Phe Arg Cys Asn Ala Pro Arg Asp Phe
145                 150                 155                 160

Ser Met Arg Thr Ser Phe
                165

<210>  311
<211>  749
<212>  DNA
<213>  Euphorbia esula

<400>  311
attttccctt cttcacccaa atatctctca ccttttaaca agagaaaagc aaaaagaaga     60

tgagcagacc aggagattgg aactgcaggt cctgccagca tctcaacttc cagagaaggg    120

actcgtgcca gcgctgtggg gactccaggt ccgggactgg aggttcagga gctgactttg    180

gcgggtttgg aagccgggtt gggtcctcat tcgggttcag caccgggtct gatgttcgac    240

ccggtgactg gtactgtact gctggcaact gtggggccca caactttgcc agccgggcaa    300

gttgcttcaa atgtggagtt tataaggatg attctggggc ccctggcggg tttgattccg    360

atatcctccg ggcctctaga ggttttggta gtggcagcaa tcgctcttct tggaaatctg    420

gtgattggat ctgcactcgg tggggatgta atgaacacaa ctttgcaagc agaatggagt    480

gtttcaaatg cagtgcccct agggacctta gtaacagaac ttcatactag acattttgga    540

tggtgcagca gccaagaaag agaattaaga ttactacttt taaattttat tttattttta    600

ctatttgttt tgggtgttat tagagtggaa aacaaacatg gcttaaaaac ccatgtttca    660

tccctaattt aagctaagga agcaaacccc ttttggcatt ttgtaaggca gatttaggat    720

tgtagtactt aattaattag tgggttgtt                                      749

<210>  312
<211>  156
<212>  PRT
<213>  Euphorbia esula

<400>  312

Met Ser Arg Pro Gly Asp Trp Asn Cys Arg Ser Cys Gln His Leu Asn
1               5                   10                  15

```
Phe Gln Arg Arg Asp Ser Cys Gln Arg Cys Gly Asp Ser Arg Ser Gly
            20                  25                  30

Thr Gly Gly Ser Gly Ala Asp Phe Gly Gly Phe Gly Ser Arg Val Gly
        35                  40                  45

Ser Ser Phe Gly Phe Ser Thr Gly Ser Asp Val Arg Pro Gly Asp Trp
    50                  55                  60

Tyr Cys Thr Ala Gly Asn Cys Gly Ala His Asn Phe Ala Ser Arg Ala
65                  70                  75                  80

Ser Cys Phe Lys Cys Gly Val Tyr Lys Asp Asp Ser Gly Ala Pro Gly
                85                  90                  95

Gly Phe Asp Ser Asp Ile Leu Arg Ala Ser Arg Gly Phe Gly Ser Gly
            100                 105                 110

Ser Asn Arg Ser Ser Trp Lys Ser Gly Asp Trp Ile Cys Thr Arg Trp
        115                 120                 125

Gly Cys Asn Glu His Asn Phe Ala Ser Arg Met Glu Cys Phe Lys Cys
    130                 135                 140

Ser Ala Pro Arg Asp Leu Ser Asn Arg Thr Ser Tyr
145                 150                 155


<210>  313
<211>  646
<212>  DNA
<213>  Gossypium hirsutum

<400>  313
ctccattctc ttatttaact caaactcacc accttttcca tttttctaat taagaaaaga      60

aaagatgagc aggccaggag attggaactg caggtcgtgc caacacctaa acttccaaag     120

gagggactcc tgccaacgct gcggggaatt ccggtcgggt gatcacttcg gtagctacgg     180

tggtggcagg ggtggctcct cctttggatt cgccaccggc tccgacgtcc gacctggtga     240

ttggtactgc actgcgggaa actgcggtac ccacaatttc gccagtcgtt ccagctgctt     300

caaatgtggt gcattcaagg acgaccctgc cggaggtttc gacagcgacg ttccgcgttc     360

tagaggattt ggcggcggta atcgatccgg ctggaaatcc ggcgactgga tatgtaccag     420

gtcgggatgc aatgagcata actttgctag ccgaatggaa tgtttcagat gcagtgcccc     480

aagagacttc accgctagaa cttcatacta aatacaagct atccagtttt gggtgttgca     540

aaccaagaga gactcaaatg agagaaaaaa ttttacgggg ttagggttac ccgggtaaat     600

atatggcttg ggggggtgtg gcaccagggt tgaaaacacc agggtg                    646
```

<210> 314
<211> 148
<212> PRT
<213> Gossypium hirsutum

<400> 314

```
Met Ser Arg Pro Gly Asp Trp Asn Cys Arg Ser Cys Gln His Leu Asn
1               5                   10                  15

Phe Gln Arg Arg Asp Ser Cys Gln Arg Cys Gly Glu Phe Arg Ser Gly
            20                  25                  30

Asp His Phe Gly Ser Tyr Gly Gly Gly Arg Gly Gly Ser Ser Phe Gly
        35                  40                  45

Phe Ala Thr Gly Ser Asp Val Arg Pro Gly Asp Trp Tyr Cys Thr Ala
    50                  55                  60

Gly Asn Cys Gly Thr His Asn Phe Ala Ser Arg Ser Ser Cys Phe Lys
65                  70                  75                  80

Cys Gly Ala Phe Lys Asp Asp Pro Ala Gly Gly Phe Asp Ser Asp Val
                85                  90                  95

Pro Arg Ser Arg Gly Phe Gly Gly Gly Asn Arg Ser Gly Trp Lys Ser
            100                 105                 110

Gly Asp Trp Ile Cys Thr Arg Ser Gly Cys Asn Glu His Asn Phe Ala
        115                 120                 125

Ser Arg Met Glu Cys Phe Arg Cys Ser Ala Pro Arg Asp Phe Thr Ala
    130                 135                 140

Arg Thr Ser Tyr
145
```

<210> 315
<211> 613
<212> DNA
<213> Vitis vinifera

<400> 315

```
ggtcttcttc catctctgca cagttatcct gatatttcct tggaaaacat gagcaggcca      60

ggagattgga actgcaggtc atgccagcac atgaacttcc aaaggcgcga ttcctgccaa     120

cgctgcggtg acccaaaatc gggtgggggt gactttggaa gctttggtgg gaggggtgga     180

tcctcctttg ggttcacggg ctcggatgtc cgcccagggg actggtactg caatgcaggc     240

aactgtggag ctcacaactt tgctagccgc tctaactgct tcaagtgtgg tgcattcaaa     300
```

gatgagtctg ctgggggcta cgattccgac atgtcacgct cccgaggttt cgggttcggc 360

ggtggcagcg gccggtctgg gtggaaatcc ggtgattgga tatgcagcag gtctggatgc 420

aatgagcaca actttgctag cagaatggaa tgtttcagat gcaatgcccc gagggacttg 480

agtaacaaaa cttcatacta gacatatatc ttccattttt gggtactgca gccagccaag 540

agagaccaaa tcatagaata tctattaatc cttgtgttgt tattaatttc tttgctttgg 600

gtgctaggtt ctt 613

<210> 316
<211> 150
<212> PRT
<213> Vitis vinifera

<400> 316

Met Ser Arg Pro Gly Asp Trp Asn Cys Arg Ser Cys Gln His Met Asn
1 5 10 15

Phe Gln Arg Arg Asp Ser Cys Gln Arg Cys Gly Asp Pro Lys Ser Gly
20 25 30

Gly Gly Asp Phe Gly Ser Phe Gly Gly Arg Gly Gly Ser Ser Phe Gly
35 40 45

Phe Thr Gly Ser Asp Val Arg Pro Gly Asp Trp Tyr Cys Asn Ala Gly
50 55 60

Asn Cys Gly Ala His Asn Phe Ala Ser Arg Ser Asn Cys Phe Lys Cys
65 70 75 80

Gly Ala Phe Lys Asp Glu Ser Ala Gly Gly Tyr Asp Ser Asp Met Ser
85 90 95

Arg Ser Arg Gly Phe Gly Phe Gly Gly Gly Ser Gly Arg Ser Gly Trp
100 105 110

Lys Ser Gly Asp Trp Ile Cys Ser Arg Ser Gly Cys Asn Glu His Asn
115 120 125

Phe Ala Ser Arg Met Glu Cys Phe Arg Cys Asn Ala Pro Arg Asp Leu
130 135 140

Ser Asn Lys Thr Ser Tyr
145 150

<210> 317
<211> 844
<212> DNA
<213> Populus trichocarpa

<400> 317
tcagaccatt tccctttcat cttctagcta gcatagcttc tctcagaggt ttttgaaacc 60

cctttttgcc attcttctct cacttagtat acttagtctt ctctaatcaa ttcataagca 120

aatatgaaca ggccaggaga ctggaactgc aggtcatgcc aacacctcaa tttccagagg 180

cgtgactctt gccaacgttg tggggacccc aggtccgcag gtgattttgg gggtttcggt 240

gggcggggtg gctcatcact tgggttcacc gggtcggatg ttcgtcccgg tgattggtac 300

tgcactgccg gaaactgcgg ggcccacaac tttgctagcc gttctagttg cttcaaatgt 360

ggagtgtaca aggaaatgga ctccgccggg ggcttcgatt ctgatttttc tcgaactaga 420

gggtttggtg ggagcactgg aggtggcaat cgatctggat ggaaatccgg agactggatt 480

tgcactaggt ggggatgcaa cgaacataac tttgctagca gaatggagtg cttcaagtgc 540

aatgccccaa gagatcttag caacagaact tcatactaga tacaatttct ccatttcctg 600

ggactgcacc agccaagaac aaagacaaca tgattaaaaa atatatatca ctacttttca 660

ttttcttctt gatttctttt tgtattagct agctagggtc ttgaggcgag acatggttta 720

agaaccatgt ttactgcttt gagctatata taacccttgg cattttgtca ggcttcctgt 780

aggaagtata tcgttgtctt tgtgggcttt tgatctatta tattcattat tgtaaccaag 840

ggag 844

<210> 318
<211> 151
<212> PRT
<213> Populus trichocarpa

<400> 318

Met Asn Arg Pro Gly Asp Trp Asn Cys Arg Ser Cys Gln His Leu Asn
1 5 10 15

Phe Gln Arg Arg Asp Ser Cys Gln Arg Cys Gly Asp Pro Arg Ser Ala
20 25 30

Gly Asp Phe Gly Gly Phe Gly Gly Arg Gly Gly Ser Ser Leu Gly Phe
35 40 45

Thr Gly Ser Asp Val Arg Pro Gly Asp Trp Tyr Cys Thr Ala Gly Asn
50 55 60

Cys Gly Ala His Asn Phe Ala Ser Arg Ser Ser Cys Phe Lys Cys Gly
65 70 75 80

Val Tyr Lys Glu Met Asp Ser Ala Gly Gly Phe Asp Ser Asp Phe Ser
85 90 95

Arg Thr Arg Gly Phe Gly Gly Ser Thr Gly Gly Gly Asn Arg Ser Gly
100 105 110

Trp Lys Ser Gly Asp Trp Ile Cys Thr Arg Trp Gly Cys Asn Glu His
        115                 120                 125

Asn Phe Ala Ser Arg Met Glu Cys Phe Lys Cys Asn Ala Pro Arg Asp
    130                 135                 140

Leu Ser Asn Arg Thr Ser Tyr
145                 150

<210> 319
<211> 785
<212> DNA
<213> Gossypium hirsutum

<400> 319
ccttgcgtcc gctttcttct tcattgttcc tgtctttgct tactcacccc ttatatatct 60

gctttctcaa gtaggggaga aaatatgagc aggccaggag actggaattg caggtcatgc 120

caacacctca acttccaaag gagggactca tgccagcgct gtggagaacc aagacctggt 180

ggtggtgaca gaggcggcga ctatggaagc tttggtggca ggggtggctc atctttcggg 240

tttactggac ccgatgttag gcctggtgac tggtattgca ctgtgggcaa ctgcggtgct 300

cacaacttcg ccagcaggtc gagctgcttc aaatgtggtg cggccaaaga tgaatcatcc 360

ggaggatttg aaagtgacat cccacgtatg aggggttatg gttttagcac tggcagctct 420

agtcgctcta actggaaatc tggagactgg atttgcacca ggtcgggttg caatgaacac 480

aacttcgcca gcaggatgga atgtttcaga tgcaatgcac caagggactc cacccacaaa 540

tcttcatact aattaataaa attttcattt ttgggtactg cagtcgcaag agagagatca 600

gacaaggcaa tcccagatct tgctttcttt ttcttttgtt tgtttcttta ttttagatct 660

ttagatgaat catgtttgaa agacttcagt tgaagtactt aagctaatat caaagtacat 720

agggcatgca tgtaattgat gtatggtatc agcaatgtta tatcagtgtc tttgtgccaa 780

aaaaa 785

<210> 320
<211> 155
<212> PRT
<213> Gossypium hirsutum

<400> 320

Met Ser Arg Pro Gly Asp Trp Asn Cys Arg Ser Cys Gln His Leu Asn
1               5                   10                  15

Phe Gln Arg Arg Asp Ser Cys Gln Arg Cys Gly Glu Pro Arg Pro Gly
            20                  25                  30

Gly Gly Asp Arg Gly Gly Asp Tyr Gly Ser Phe Gly Gly Arg Gly Gly

35 40 45

Ser Ser Phe Gly Phe Thr Gly Pro Asp Val Arg Pro Gly Asp Trp Tyr
50 55 60

Cys Thr Val Gly Asn Cys Gly Ala His Asn Phe Ala Ser Arg Ser Ser
65 70 75 80

Cys Phe Lys Cys Gly Ala Ala Lys Asp Glu Ser Ser Gly Gly Phe Glu
85 90 95

Ser Asp Ile Pro Arg Met Arg Gly Tyr Gly Phe Ser Thr Gly Ser Ser
100 105 110

Ser Arg Ser Asn Trp Lys Ser Gly Asp Trp Ile Cys Thr Arg Ser Gly
115 120 125

Cys Asn Glu His Asn Phe Ala Ser Arg Met Glu Cys Phe Arg Cys Asn
130 135 140

Ala Pro Arg Asp Ser Thr His Lys Ser Ser Tyr
145 150 155

<210> 321
<211> 760
<212> DNA
<213> Lactuca serriola

<400> 321
gtattcggaa tcctcaaaga aacacacact tcctatccat cacaagaaag atgagcaggc 60
caggagattg gaactgcagg tcatgccagc acttgaactt ccagaggagg gactcttgcc 120
aaagatgtgg ggagacgagg tatggtggtg ggggtggtgt gtttggtggt agaggaagta 180
tcatcagccc ttcagcattt ggcttcacag gcccagatgt ccgaccgggt gattggtact 240
gcaatgttgg caactgcggg gctcacaact ttgctagccg ctcgagctgc ttcaagtgtg 300
gtgcgttcaa ggatgactta gcttgtagtg gtggtggtgg tggtggtggt ggcgtttttg 360
atggtgatat gtcacgtggc aggggtttcg ggtttggtgg aggaagtggt ggtggaggtg 420
gtggcagcag ccgttcaggg tggaagtccg gtgactggat atgcggcagg cctggttgca 480
atgagcacaa ctttgcaagc agaatggaat gttttaggtg caacgcacct cgggaatcgg 540
gtaacaagtc tccttattaa gcaggttgcg gcatttccag ttccgggtat catggacttg 600
ctatctacca aacaggaaga gagataagtg atcgatcaga cagaatcatg aagagcatcc 660
agttgatagg aaattctaaa ctgacccccct ttttgcccat atcataatat gcaatcctat 720
ctttgatttt ttttttcctt ttgttttatt tttttttttt 760

<210> 322

<211> 169
<212> PRT
<213> Lactuca serriola

<400> 322

Met Ser Arg Pro Gly Asp Trp Asn Cys Arg Ser Cys Gln His Leu Asn
1 5 10 15

Phe Gln Arg Arg Asp Ser Cys Gln Arg Cys Gly Glu Thr Arg Tyr Gly
20 25 30

Gly Gly Gly Gly Val Phe Gly Gly Arg Gly Ser Ile Ile Ser Pro Ser
35 40 45

Ala Phe Gly Phe Thr Gly Pro Asp Val Arg Pro Gly Asp Trp Tyr Cys
50 55 60

Asn Val Gly Asn Cys Gly Ala His Asn Phe Ala Ser Arg Ser Ser Cys
65 70 75 80

Phe Lys Cys Gly Ala Phe Lys Asp Asp Leu Ala Cys Ser Gly Gly Gly
85 90 95

Gly Gly Gly Gly Gly Val Phe Asp Gly Asp Met Ser Arg Gly Arg Gly
100 105 110

Phe Gly Phe Gly Gly Gly Ser Gly Gly Gly Gly Gly Gly Ser Ser Arg
115 120 125

Ser Gly Trp Lys Ser Gly Asp Trp Ile Cys Gly Arg Pro Gly Cys Asn
130 135 140

Glu His Asn Phe Ala Ser Arg Met Glu Cys Phe Arg Cys Asn Ala Pro
145 150 155 160

Arg Glu Ser Gly Asn Lys Ser Pro Tyr
165

<210> 323
<211> 517
<212> DNA
<213> Glycine max

<400> 323
tttctgcatc tctaatactt caactcactg tattatcttg agtaattttg cagagaagta 60

aaatatgagc aggccaggag actggaattg caggtcgtgc cagcacctga actttcagag 120

gagagactca tgccagcgat gtggggactc aaaatatgga gatagagttg ttgattttgg 180

tggttttgga ggaagaggag ggtcctcatt tggtttaact ggctcagatg ttcgccccgg 240

cgactggtac tgtgctgctg ctaactgtgg tgcacacaac tttgctagcc gctcaagctg 300

cttcaagtgt ggtgctttca aggatgactt ggctggagga ggctataaca gttctgacat 360

cttgcgctcc agagcttttg gtggcagtgg aagacctgga tggaaatctg gtgattggat 420

atgcagcaga tcaggatgca atgagcacaa ctttgctagc agaatggaat gttttaaatg 480

cagtgctccc agggacacgt actagaaaaa atgagtt 517

<210> 324
<211> 146
<212> PRT
<213> Glycine max

<400> 324

Met Ser Arg Pro Gly Asp Trp Asn Cys Arg Ser Cys Gln His Leu Asn
1 5 10 15

Phe Gln Arg Arg Asp Ser Cys Gln Arg Cys Gly Asp Ser Lys Tyr Gly
20 25 30

Asp Arg Val Val Asp Phe Gly Gly Phe Gly Gly Arg Gly Gly Ser Ser
35 40 45

Phe Gly Leu Thr Gly Ser Asp Val Arg Pro Gly Asp Trp Tyr Cys Ala
50 55 60

Ala Ala Asn Cys Gly Ala His Asn Phe Ala Ser Arg Ser Ser Cys Phe
65 70 75 80

Lys Cys Gly Ala Phe Lys Asp Asp Leu Ala Gly Gly Gly Tyr Asn Ser
85 90 95

Ser Asp Ile Leu Arg Ser Arg Ala Phe Gly Gly Ser Gly Arg Pro Gly
100 105 110

Trp Lys Ser Gly Asp Trp Ile Cys Ser Arg Ser Gly Cys Asn Glu His
115 120 125

Asn Phe Ala Ser Arg Met Glu Cys Phe Lys Cys Ser Ala Pro Arg Asp
130 135 140

Thr Tyr
145

<210> 325
<211> 889
<212> DNA
<213> Populus trichocarpa

<400> 325
gacaagttcc ttaaacaatc tattgctctt tcaactacta gctaggttac acttcatttc 60

tgtacgtgtt aattctccta tctttctcct ttctgctttc tcgagatgag cagaccagga 120

gattggaatt gcaggtcatg ccagcacttg aactttcaaa ggagggactc atgccagcgt 180

tgcggtgatc caaggcccgg agagagagat cactatggaa gtttcggtgg aagatcatca 240

gggggctcat tcggatttac gggccctgat gttaggcctg gtgattggta ttgcacggct 300

ggcaattgtg gagctcacaa ctttgctagt cgttcaagct gcttcaagtg tggtgtgtcc 360

aaggatgaat cctctggtgg tggacttgat gctgatatgt cacggatgag aggttatggc 420

ttcggcggag gcggaggcgg aggcagtggc tctagccgta attggaaatc cggagactgg 480

atttgcacca ggtccggttg caacgagcac aactttgcta gcaggactga atgctataga 540

tgcaatgcac caagagaatc tagcagcaac aagtcttcgt attaatcatc gatatcgata 600

gcatttttgt gtcctgcagt gctgcaagga gggaattaac gaagaaggct catgagaaat 660

cttggattca tctttttgct cttaattact tctatttttg ttcttttgga tagctgtact 720

cttaagctga gaagctttag aaggatcatg ggagtgaaat taagttcaag gagagctata 780

tatatatcat tagccagtaa tttgtcaggt tccctaaata tagacatgta gttctgtact 840

tggtatcaat gtctttgtgt tttgagacgg gtttaagccc ttgtcgttc 889

<210> 326
<211> 159
<212> PRT
<213> Populus trichocarpa

<400> 326

Met Ser Arg Pro Gly Asp Trp Asn Cys Arg Ser Cys Gln His Leu Asn
1               5                   10                  15

Phe Gln Arg Arg Asp Ser Cys Gln Arg Cys Gly Asp Pro Arg Pro Gly
            20                  25                  30

Glu Arg Asp His Tyr Gly Ser Phe Gly Gly Arg Ser Ser Gly Gly Ser
        35                  40                  45

Phe Gly Phe Thr Gly Pro Asp Val Arg Pro Gly Asp Trp Tyr Cys Thr
    50                  55                  60

Ala Gly Asn Cys Gly Ala His Asn Phe Ala Ser Arg Ser Ser Cys Phe
65                  70                  75                  80

Lys Cys Gly Val Ser Lys Asp Glu Ser Ser Gly Gly Gly Leu Asp Ala
                85                  90                  95

Asp Met Ser Arg Met Arg Gly Tyr Gly Phe Gly Gly Gly Gly Gly Gly
            100                 105                 110

Gly Ser Gly Ser Ser Arg Asn Trp Lys Ser Gly Asp Trp Ile Cys Thr

115 120 125

Arg Ser Gly Cys Asn Glu His Asn Phe Ala Ser Arg Thr Glu Cys Tyr
130 135 140

Arg Cys Asn Ala Pro Arg Glu Ser Ser Ser Asn Lys Ser Ser Tyr
145 150 155

<210> 327
<211> 723
<212> DNA
<213> Populus fremontii x Populus angustifolia

<400> 327
agggaaaaag aaagcctaac cctcctgcct gcctcaagct taccccatta tcgaggtcta 60

aaacaagtat aaaaacagcc ttagccccaa tacgtaaatc acaagttcct taaacaatct 120

attgctcttt caactactag gtttcccact tcatttctgt gcctactgat tctcctctct 180

ttcaactttc tgccttctca agctagctag agaagggaag atgagcagac caggagattg 240

gaattgcagg tcatgccaac acttgaactt ccagaggagg gactcgtgcc agcgttgtgg 300

ggacccaagg cccggagaga gagatcatta tggaagtttt ggtggaagat caggggggctc 360

gttcggattt acggggcctg atgttaggcc cggtgactgg tattgctcgg ttggcaactg 420

tggagctcac aactttgcta gtcgttcaag ctgcttcaag tgtggtatgt ccaaggatga 480

atcctctggt ggtgggcttg atgctgacat ttcatggatg agaggttatg gcttcggcgg 540

aggcagcgcc tctagccgct ctaattggaa atccggagac tggatttgca ccaggtcagg 600

ttgcaacgag cacaactttg ctagcaggac tgagtgttac agatgcaatg caccaagaga 660

atcaggcagc aacaagtctt cgtattaatc atcaacatcg atcgcatttt tgtgtactgc 720

agt 723

<210> 328
<211> 155
<212> PRT
<213> Populus fremontii x Populus angustifolia

<400> 328

Met Ser Arg Pro Gly Asp Trp Asn Cys Arg Ser Cys Gln His Leu Asn
1 5 10 15

Phe Gln Arg Arg Asp Ser Cys Gln Arg Cys Gly Asp Pro Arg Pro Gly
20 25 30

Glu Arg Asp His Tyr Gly Ser Phe Gly Gly Arg Ser Gly Gly Ser Phe
35 40 45

Gly Phe Thr Gly Pro Asp Val Arg Pro Gly Asp Trp Tyr Cys Ser Val
50 55 60

Gly Asn Cys Gly Ala His Asn Phe Ala Ser Arg Ser Ser Cys Phe Lys
65                  70                  75                  80

Cys Gly Met Ser Lys Asp Glu Ser Ser Gly Gly Gly Leu Asp Ala Asp
                85                  90                  95

Ile Ser Trp Met Arg Gly Tyr Gly Phe Gly Gly Gly Ser Ala Ser Ser
            100                 105                 110

Arg Ser Asn Trp Lys Ser Gly Asp Trp Ile Cys Thr Arg Ser Gly Cys
        115                 120                 125

Asn Glu His Asn Phe Ala Ser Arg Thr Glu Cys Tyr Arg Cys Asn Ala
    130                 135                 140

Pro Arg Glu Ser Gly Ser Asn Lys Ser Ser Tyr
145                 150                 155

<210> 329
<211> 920
<212> DNA
<213> Oryza sativa

<400> 329
cacctcacag cattttccac cttagagctc accaacacag cagctgatac ttgtttaggg 60

taagacaaga tgaacaggaa gccaggagac tgggactgca gggcgtgcca gcacctcaac 120

ttcagccgcc gggacctatg ccagcgctgc ggcgggccgc gtggcgccgc tgatcgcggc 180

agcggtggtg gcggtgacta cgccaacttc ggcggccgtg gtggttcctc cttcggtgga 240

ggctttggca ctggctctga tgtccgccca ggtgactggt actgcaactg cggcgcgcac 300

aacttcgcca gccgctccag ctgcttcaag tgcgctgctt tcaaggacga tgctgccgtc 360

aacagtggcg gcgctggtgc ctttgacggt ggggacatgt cgcgctcgcg gggctacggc 420

ttcggcagcg gcgccgtccg cgccagccgc cctggctgga agtctggcga ctggatttgc 480

accaggtctg gatgcaatga gcacaacttc gccagcagga tggagtgctt caggtgcaac 540

gcaccgcggg actccggcac tgaggtgtaa tttgccgtac gtgtccgatc gatctggatc 600

cgatgaggct tgcagcagtg acgacgagca gcagaagcag cgttaagagt tgtgatgtct 660

acataagaag aagaagaaag tagaatgcaa aagaaatctc cccatggttt tactagtttt 720

gtttcttccc gttttagatt tggttctgat tcccatttgg gaggacccgt cgacccctga 780

ttatctatgt tttacccgtt ttatttcctg tttctttcgg catgtttgct cttcgatcga 840

gtcgtgtaac ccgaaacgct tgcgcttgag aagtattatt attattaact agtatgttgc 900

ttcttaaaaa aaaaaaaaaa 920

<210> 330
<211> 166
<212> PRT
<213> Oryza sativa

<400> 330

Met Asn Arg Lys Pro Gly Asp Trp Asp Cys Arg Ala Cys Gln His Leu
1 5 10 15

Asn Phe Ser Arg Arg Asp Leu Cys Gln Arg Cys Gly Gly Pro Arg Gly
20 25 30

Ala Ala Asp Arg Gly Ser Gly Gly Gly Gly Asp Tyr Ala Asn Phe Gly
35 40 45

Gly Arg Gly Gly Ser Ser Phe Gly Gly Gly Phe Gly Thr Gly Ser Asp
50 55 60

Val Arg Pro Gly Asp Trp Tyr Cys Asn Cys Gly Ala His Asn Phe Ala
65 70 75 80

Ser Arg Ser Ser Cys Phe Lys Cys Ala Ala Phe Lys Asp Asp Ala Ala
85 90 95

Val Asn Ser Gly Gly Ala Gly Ala Phe Asp Gly Gly Asp Met Ser Arg
100 105 110

Ser Arg Gly Tyr Gly Phe Gly Ser Gly Ala Val Arg Ala Ser Arg Pro
115 120 125

Gly Trp Lys Ser Gly Asp Trp Ile Cys Thr Arg Ser Gly Cys Asn Glu
130 135 140

His Asn Phe Ala Ser Arg Met Glu Cys Phe Arg Cys Asn Ala Pro Arg
145 150 155 160

Asp Ser Gly Thr Glu Val
165

<210> 331
<211> 703
<212> DNA
<213> Panicum virgatum

<400> 331
ccacgcgtcc gcagaactcc ttgtcctagg ccccagaaac acaaacgacc gctctttgtt 60

taggcaagat gaacaggaag cctggagact gggattgcag ggcttgccaa cacctcaact 120

tcagtcggcg ggacctatgc cagcgctgtg gtgagccacg tggagctgct gaccgtggca 180

gcggcggtgg aggtgactat gccaacttcg gtggccgtgg tggctcctcc ttcggcggtg 240

gctttggtgc tggctctgat gtccgccctg gtgactggtt atgttcctgc ggcgcgcaca 300

acttcgccag ccgctccaac tgcttcaagt gctctgcctt caaggaggag gctgctgtca 360

acagtggtgc tggtggcttt gatggtgaca tgtcacgctc gcgctacggc ttcggtggcg 420

gtgctgcccg caccaaccgc cctggttgga agtctggaga ctggatctgc accaggtccg 480

gatgcaacga gcacaacttt gccagcagga tggagtgttt caggtgcaac gcaccacggg 540

actccggcac tgaggtgtag gatcggagct ggtgcttcga acggaccccg acgagcagaa 600

gcagcaagaa acctgataag aagagtagta gatttgcaaa aggcatcccg gatgctctat 660

tactgttttg ttccacccct accgcttcct tttagaattt ggt 703

<210> 332
<211> 163
<212> PRT
<213> Panicum virgatum

<400> 332

Met Asn Arg Lys Pro Gly Asp Trp Asp Cys Arg Ala Cys Gln His Leu
1 5 10 15

Asn Phe Ser Arg Arg Asp Leu Cys Gln Arg Cys Gly Glu Pro Arg Gly
20 25 30

Ala Ala Asp Arg Gly Ser Gly Gly Gly Gly Asp Tyr Ala Asn Phe Gly
35 40 45

Gly Arg Gly Gly Ser Ser Phe Gly Gly Gly Phe Gly Ala Gly Ser Asp
50 55 60

Val Arg Pro Gly Asp Trp Leu Cys Ser Cys Gly Ala His Asn Phe Ala
65 70 75 80

Ser Arg Ser Asn Cys Phe Lys Cys Ser Ala Phe Lys Glu Glu Ala Ala
85 90 95

Val Asn Ser Gly Ala Gly Gly Phe Asp Gly Asp Met Ser Arg Ser Arg
100 105 110

Tyr Gly Phe Gly Gly Gly Ala Ala Arg Thr Asn Arg Pro Gly Trp Lys
115 120 125

Ser Gly Asp Trp Ile Cys Thr Arg Ser Gly Cys Asn Glu His Asn Phe
130 135 140

Ala Ser Arg Met Glu Cys Phe Arg Cys Asn Ala Pro Arg Asp Ser Gly
145 150 155 160

Thr Glu Val

<210> 333
<211> 435
<212> DNA
<213> Medicago truncatula

<400> 333
atgaacagga aaatgagctg gtctggagga gattggatgt gtggtgcttg cgagcacata 60

aatttcaaga agagagaagc atgccaaaat tgtggatacc caaagtatgg aggccctgac 120

ccatcgacct atagatataa caggactgaa acgttggcag gggactggtt ttgcacttct 180

atgaactgtg gagctcacaa ctatgcaagc cgatcaaact gctatagatg tggtgcattt 240

aaagatcctt attcttctgg atatgggggt aacatggtgg gttctggagg atatggatca 300

gattgtagtt ctcccccagg atggaaaagt ggagactgga tttgccctag aattggctgt 360

ggaatccata attatgcaag caggacagag tgctacaaat gcaaaatgcc aagggattat 420

ggtggtgcag actga 435

<210> 334
<211> 144
<212> PRT
<213> Medicago truncatula

<400> 334

Met Asn Arg Lys Met Ser Trp Ser Gly Gly Asp Trp Met Cys Gly Ala
1 5 10 15

Cys Glu His Ile Asn Phe Lys Lys Arg Glu Ala Cys Gln Asn Cys Gly
20 25 30

Tyr Pro Lys Tyr Gly Gly Pro Asp Pro Ser Thr Tyr Arg Tyr Asn Arg
35 40 45

Thr Glu Thr Leu Ala Gly Asp Trp Phe Cys Thr Ser Met Asn Cys Gly
50 55 60

Ala His Asn Tyr Ala Ser Arg Ser Asn Cys Tyr Arg Cys Gly Ala Phe
65 70 75 80

Lys Asp Pro Tyr Ser Ser Gly Tyr Gly Gly Asn Met Val Gly Ser Gly
85 90 95

Gly Tyr Gly Ser Asp Cys Ser Ser Pro Pro Gly Trp Lys Ser Gly Asp
100 105 110

Trp Ile Cys Pro Arg Ile Gly Cys Gly Ile His Asn Tyr Ala Ser Arg
115 120 125

Thr Glu Cys Tyr Lys Cys Lys Met Pro Arg Asp Tyr Gly Gly Ala Asp
130 135 140

<210> 335
<211> 513
<212> DNA
<213> Medicago truncatula

<400> 335
atgagcagac caggagattg gaactgcagg acatgcaacc acctcaactt tcaaagaaga 60

gaatcttgcc aacgatgtgg ggagtcaaga atgacttctg gctgcggcgc cgttgatttt 120

ggtggctcct ttcttggtgg aagaggctct agctcccctt ttcctttcac caccggccct 180

gatgtccgtc ctggtgactg gtattgcact gttggaaact gtggagctca caactttgcc 240

agccgctcca gctgcttcaa atgtggtgcc cctaaggata ttgatacctt ctcctctgac 300

tcctcagaca tgccacgatt attgagatca ccatacggtt ttggagcagg cagcgctggt 360

ggcggtgcct ccactcgccc cggctggaaa tccggtgact ggatatgcac caggtctggg 420

tgtaacgagc ataacttcgc caatagaatg gaatgctacc gatgcaacgg tccaagggac 480

tctagtactg gaagatcttc ctatttatcg tga 513

<210> 336
<211> 170
<212> PRT
<213> Medicago truncatula

<400> 336

Met Ser Arg Pro Gly Asp Trp Asn Cys Arg Thr Cys Asn His Leu Asn
1 5 10 15

Phe Gln Arg Arg Glu Ser Cys Gln Arg Cys Gly Glu Ser Arg Met Thr
20 25 30

Ser Gly Cys Gly Ala Val Asp Phe Gly Gly Ser Phe Leu Gly Gly Arg
35 40 45

Gly Ser Ser Ser Pro Phe Pro Phe Thr Thr Gly Pro Asp Val Arg Pro
50 55 60

Gly Asp Trp Tyr Cys Thr Val Gly Asn Cys Gly Ala His Asn Phe Ala
65 70 75 80

Ser Arg Ser Ser Cys Phe Lys Cys Gly Ala Pro Lys Asp Ile Asp Thr
85 90 95

Phe Ser Ser Asp Ser Ser Asp Met Pro Arg Leu Leu Arg Ser Pro Tyr
100 105 110

Gly Phe Gly Ala Gly Ser Ala Gly Gly Gly Ala Ser Thr Arg Pro Gly

```
        115                 120                 125

Trp Lys Ser Gly Asp Trp Ile Cys Thr Arg Ser Gly Cys Asn Glu His
    130                 135                 140

Asn Phe Ala Asn Arg Met Glu Cys Tyr Arg Cys Asn Gly Pro Arg Asp
145                 150                 155                 160

Ser Ser Thr Gly Arg Ser Ser Tyr Leu Ser
                165                 170
```

<210> 337
<211> 826
<212> DNA
<213> Yucca filamentosa

<400> 337

```
ggcacaagct ctcacgcaac cccttgtctc cattttactc ttccttcctc tttcaaggga      60
cttgagaaga tgaacaggaa gccaggagac tggaactgca ggtcatgcca gcaccttaat     120
ttcagccgca gggactcgtg ccagcgctgc ggcgaccccc ggtcgatcgg cagcgagcga     180
tcggactacc cgggcttcgt cgggggccgc ggggggtcct cattcgggtt cagcggctcg     240
gacgtcaggc ccggggactg gtactgccag tgcggggccc acaacttcgc cagccgctcg     300
agctgcttca agtgcaatgc tttcaaggat gagtccgccg ctagcggcgg cctcgacggc     360
ggcgacatgc tgagatccag gggtttggc ttcggcggcg gcggcggcgc tcgcagtggc     420
tggaagtctg gtgactggat ttgcaacagg tctggctgca atgagcacaa cttcgctagc     480
aggatggaat gcttccgatg caatgcacct cgagattcgg gcactgaggt ttaaggagac     540
agccaagaga gaagtgacga gatgagatca gtgatgatga taccagtagt aatctcgagt     600
tattactagt tttgccacca gccccaactt tatctccttt tgatgttttt ttagagcccc     660
cttctaaacg gaggtgtctt ctctttttca tccgtttgtt tgcttttgaa agcttgcact     720
taccctttac acccccccgc ccccctccac cttgtagttt gtgttgagag tgtctttgta     780
agtctttgga gaagggctcc tttagcttct tgggggagtc ccttct                    826
```

<210> 338
<211> 154
<212> PRT
<213> Yucca filamentosa

<400> 338

```
Met Asn Arg Lys Pro Gly Asp Trp Asn Cys Arg Ser Cys Gln His Leu
1               5                   10                  15

Asn Phe Ser Arg Arg Asp Ser Cys Gln Arg Cys Gly Asp Pro Arg Ser
            20                  25                  30
```

```
Ile Gly Ser Glu Arg Ser Asp Tyr Pro Gly Phe Val Gly Gly Arg Gly
        35                  40                  45

Gly Ser Ser Phe Gly Phe Ser Gly Ser Asp Val Arg Pro Gly Asp Trp
    50                  55                  60

Tyr Cys Gln Cys Gly Ala His Asn Phe Ala Ser Arg Ser Ser Cys Phe
65                  70                  75                  80

Lys Cys Asn Ala Phe Lys Asp Glu Ser Ala Ala Ser Gly Gly Leu Asp
                85                  90                  95

Gly Gly Asp Met Leu Arg Ser Arg Gly Phe Gly Phe Gly Gly Gly Gly
            100                 105                 110

Gly Ala Arg Ser Gly Trp Lys Ser Gly Asp Trp Ile Cys Asn Arg Ser
        115                 120                 125

Gly Cys Asn Glu His Asn Phe Ala Ser Arg Met Glu Cys Phe Arg Cys
    130                 135                 140

Asn Ala Pro Arg Asp Ser Gly Thr Glu Val
145                 150
```

<210> 339
<211> 614
<212> DNA
<213> Hordeum vulgare

<400> 339

```
cggcacgagg cacttctcat cttggagctc agcagcacag catccaagct ttttcttcgg     60

caagatgaac aggaagccag gagactggga ctgcaggtcg tgccagcacc tcaacttcag    120

tcgccgggac ctatgccagc gctgcggtga gccacgtagt gccgctgacc gtggcagcgt    180

cggtggtgct cttggtggtg actacgccaa ctttggcggc cgtggcgggg gtggttcctc    240

atttggtgcc ggctttggtg ccggctctga cgtccgcccg ggtgactggt actgcacctg    300

tggagcgcac aacttcgcca gccgctccag ctgcttcaag tgtgctgctt tcaaggagga    360

agctgccgtc aatggtggcg ctggtggctt tgatggtgac atgtcacgct caaggggctt    420

tggctttggc gctgtcggtg gcatgggtgg cggcatggga gccggcgcag ccggtggtcg    480

tgccagtcgc cctggctgga agtctcgcga ctggatttgc accaggtctg gatgcaacga    540

gcacaacttc gccagcaggc aggagtgctt caggtgcaac gcgccgaggg actccggtag    600

cgccacacca tacg                                                      614
```

<210> 340
<211> 183
<212> PRT
<213> Hordeum vulgare

<400> 340

Met Asn Arg Lys Pro Gly Asp Trp Asp Cys Arg Ser Cys Gln His Leu
1               5                   10                  15

Asn Phe Ser Arg Arg Asp Leu Cys Gln Arg Cys Gly Glu Pro Arg Ser
            20                  25                  30

Ala Ala Asp Arg Gly Ser Val Gly Gly Ala Leu Gly Gly Asp Tyr Ala
        35                  40                  45

Asn Phe Gly Gly Arg Gly Gly Gly Gly Ser Ser Phe Gly Ala Gly Phe
    50                  55                  60

Gly Ala Gly Ser Asp Val Arg Pro Gly Asp Trp Tyr Cys Thr Cys Gly
65                  70                  75                  80

Ala His Asn Phe Ala Ser Arg Ser Ser Cys Phe Lys Cys Ala Ala Phe
                85                  90                  95

Lys Glu Glu Ala Ala Val Asn Gly Gly Ala Gly Gly Phe Asp Gly Asp
            100                 105                 110

Met Ser Arg Ser Arg Gly Phe Gly Phe Gly Ala Val Gly Gly Met Gly
        115                 120                 125

Gly Gly Met Gly Ala Gly Ala Ala Gly Gly Arg Ala Ser Arg Pro Gly
    130                 135                 140

Trp Lys Ser Arg Asp Trp Ile Cys Thr Arg Ser Gly Cys Asn Glu His
145                 150                 155                 160

Asn Phe Ala Ser Arg Gln Glu Cys Phe Arg Cys Asn Ala Pro Arg Asp
                165                 170                 175

Ser Gly Ser Ala Thr Pro Tyr
            180

<210> 341
<211> 54
<212> DNA
<213> Artificial sequence

<220>
<223> primer: prm5539

<400> 341
ggggacaagt ttgtacaaaa aagcaggctt aaacaatgag cagacccgga gatt 54

<210> 342
<211> 51

<212> DNA
<213> Artificial sequence

<220>
<223> primer: prm5540

<400> 342
ggggaccact ttgtacaaga aagctgggta gacaaggcta cttcaaaagc a 51

<210> 343
<211> 2193
<212> DNA
<213> Oryza sativa

<400> 343
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag attttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctcctcctc ccatctataa attcctcccc ccttttcccc tctctatata 1020

ggaggcatcc aagccaagaa gagggagagc accaaggaca cgcgactagc agaagccgag 1080

cgaccgcctt cttcgatcca tatcttccgg tcgagttctt ggtcgatctc ttccctcctc 1140

cacctcctcc tcacagggta tgtgcccttc ggttgttctt ggatttattg ttctaggttg 1200

tgtagtacgg gcgttgatgt taggaaaggg gatctgtatc tgtgatgatt cctgttcttg 1260

gatttgggat agaggggttc ttgatgttgc atgttatcgg ttcggtttga ttagtagtat 1320

ggttttcaat cgtctggaga gctctatgga aatgaaatgg tttagggtac ggaatcttgc 1380

gattttgtga gtaccttttg tttgaggtaa aatcagagca ccggtgattt tgcttggtgt 1440

aataaaagta cggttgtttg gtcctcgatt ctggtagtga tgcttctcga tttgacgaag 1500

ctatcctttg tttattccct attgaacaaa aataatccaa ctttgaagac ggtcccgttg 1560

atgagattga atgattgatt cttaagcctg tccaaaattt cgcagctggc ttgtttagat 1620

acagtagtcc ccatcacgaa attcatggaa acagttataa tcctcaggaa caggggattc 1680

cctgttcttc cgatttgctt tagtcccaga attttttttc ccaaatatct taaaaagtca 1740

ctttctggtt cagttcaatg aattgattgc tacaaataat gcttttatag cgttatccta 1800

gctgtagttc agttaatagg taatacccct atagtttagt caggagaaga acttatccga 1860

tttctgatct ccatttttaa ttatatgaaa tgaactgtag cataagcagt attcatttgg 1920

attatttttt ttattagctc tcacccttc attattctga gctgaaagtc tggcatgaac 1980

tgtcctcaat tttgttttca aattcacatc gattatctat gcattatcct cttgtatcta 2040

cctgtagaag tttctttttg gttattcctt gactgcttga ttacagaaag aaatttatga 2100

agctgtaatc gggatagtta tactgcttgt tcttatgatt catttccttt gtgcagttct 2160

tggtgtagct tgccactttc accagcaaag ttc 2193

<210> 344
<211> 3
<212> PRT
<213> Artificial sequence

<220>
<223> sequence motif 1

<220>
<221> VARIANT
<222> (1)..(1)
<223> /replace="Arg" /replace="Asp" /replace="Asn"

<400> 344

Gly Asp Trp
1

<210> 345
<211> 3
<212> PRT
<213> Artificial sequence

<220>
<223> sequence motif 2

<400> 345

Gly Ser Trp
1

<210> 346
<211> 5
<212> PRT
<213> Artificial sequence

```
<220>
<223>  sequence motif 3


<220>
<221>  VARIANT
<222>  (3)..(3)
<223>  /replace="Cys" /replace="Ser"

<220>
<221>  VARIANT
<222>  (4)..(4)
<223>  /replace="Lys"

<400>  346

Asn Phe Gln Arg Arg
1               5


<210>  347
<211>  5
<212>  PRT
<213>  Artificial sequence

<220>
<223>  sequence motif 4


<220>
<221>  VARIANT
<222>  (2)..(2)
<223>  /replace="Tyr"

<220>
<221>  VARIANT
<222>  (3)..(3)
<223>  /replace="Ser" /replace="Pro"

<220>
<221>  VARIANT
<222>  (4)..(4)
<223>  /replace="Ser" /replace="Phe"

<400>  347

Asn Phe Ala Asn Arg
1               5
```